# EUROPEAN PATENT APPLICATION

(11) **EP 2 505 649 A1**
(43) Date of publication of application: **03.10.2012**
(21) Application number: 12174138.3
(22) Date of filing: 07.05.2007
(51) Int. Cl.: C12N 15/113

(54) **Compounds and methods for modulating expression of GCGR**

(30) Priority: 05.05.2006 US 746631 P; 11.05.2006 US 747059 P; 23.06.2006 US 805660 P; 06.11.2006 US 864554 P; 27.01.2007 WO PCT/US2007/061183
(62) Divisional of application: 07811878.3
(71) Applicant: Isis Pharmaceuticals, Inc., Carlsbad, CA 92010 (US)
(72) Inventor: Bhanot, Sanjay, Carlsbad, CA 92009 (US); Geary, Richard S., Carlsbad, CA 92009 (US); McKay, Robert, Poway, CA 92064 (US); Monia, Brett P., Encinitas, CA 92024 (US); Seth, Punit P., San Marcos, CA 92078 (US); Siwkowski, Andrew M., Carlsbad, CA 92009 (US); Swayze, Eric E., Carlsbad, CA 92009 (US); Wancewicz, Edward, Poway, CA 92064 (US)
(74) Representative: Carpmaels & Ransford

(57) **Abstract**

The present disclosure describes short antisense compounds, including such compounds comprising chemically-modified high-affinity monomers 8-16 monomers in length. Certain such short antisense compound are useful for the reduction of target nucleic acids and/or proteins in cells, tissues, and animals with increased potency and improved therapeutic index. Thus, provided herein are short antisense compounds comprising high-affinity nucleotide modifications useful for reducing a target RNA *in vivo.* Such short antisense compounds are effective at lower doses than previously described antisense compounds, allowing for a reduction in toxicity and cost of treatment. In addition, the described short antisense compounds have greater potential for oral dosing.

SEQUENCE LISTING

<110> Isis Pharmaceuticals, Inc.

<120> COMPOUNDS AND METHODS FOR MODULATING
      EXPRESSION OF GCGR

<130> P059937EP

<150> EP 07811878.3
<151> 2007-05-07

<150> US 60/746,631
<151> 2006-05-05

<150> US 60/747,059
<151> 2006-05-11

<150> US 60/805,660
<151> 2006-06-23

<150> US 60/864,554
<151> 2006-11-06

<150> PCT/US2007/061183
<151> 2007-01-27

<160> 1576

<170> FastSEQ for Windows Version 4.0

<210> 1
<211> 14121
<212> DNA
<213> H. sapiens

<400> 1
attcccaccg ggacctgcgg ggctgagtgc ccttctcggt tgctgccgct gaggagcccg 60
cccagccagc cagggccgcg aggccgaggc caggccgcag cccaggagcc gccccaccgc 120
agctggcgat ggacccgccg aggcccgcgc tgctggcgct gctggcgctg cctgcgctgc 180
tgctgctgct gctggcgggc gccagggccg aagaggaaat gctggaaaat gtcagcctgg 240
tctgtccaaa agatgcgacc cgattcaagc acctccggaa gtacacatac aactatgagg 300
ctgagagttc cagtggagtc cctgggactg ctgattcaag aagtgccacc aggatcaact 360
gcaaggttga gctggaggtt ccccagctct gcagcttcat cctgaagacc agccagtgca 420
ccctgaaaga ggtgtatggc ttcaaccctg agggcaaagc cttgctgaag aaaaccaaga 480
actctgagga gtttgctgca gccatgtcca ggtatgagct caagctggcc attccagaag 540
ggaagcaggt tttcctttac ccggagaaag atgaacctac ttacatcctg aacatcaaga 600
ggggcatcat ttctgccctc ctggttcccc cagagacaga agaagccaag caagtgttgt 660
ttctggatac cgtgtatgga aactgctcca ctcactttac cgtcaagacg aggaagggca 720
atgtggcaac agaaatatcc actgaaagag acctggggca gtgtgatcgc ttcaagccca 780
tccgcacagg catcagccca cttgctctca tcaaaggcat gacccgcccc ttgtcaactc 840
tgatcagcag cagccagtcc tgtcagtaca cactggacgc taagaggaag catgtggcag 900
aagccatctg caaggagcaa cacctcttcc tgcctttctc ctacaacaat aagtatggga 960
tggtagcaca agtgacacag actttgaaac ttgaagacac accaaagatc aacagccgct 1020
tctttggtga aggtactaag aagatgggcc tcgcatttga gagcaccaaa tccacatcac 1080
ctccaaagca ggccgaagct gttttgaaga ctctccagga actgaaaaaa ctaaccatct 1140
ctgagcaaaa tatccagaga gctaatctct tcaataagct ggttactgag ctgagaggcc 1200
tcagtgatga agcagtcaca tctctcttgc cacagctgat tgaggtgtcc agccccatca 1260
ctttacaagc cttggttcag tgtggacagc ctcagtgctc cactcacatc ctccagtggc 1320
tgaaacgtgt gcatgccaac ccccttctga tagatgtggt cacctacctg gtggccctga 1380
tccccgagcc ctcagcacag cagctgcgag agatcttcaa catggcgagg gatcagcgca 1440
gccgagccac cttgtatgcg ctgagccacg cggtcaacaa ctatcataag acaaacccta 1500
cagggaccca ggagctgctg gacattgcta attacctgat ggaacagatt caagatgact 1560
gcactgggga tgaagattac acctatttga ttctgcgggt cattggaaat atgggccaaa 1620

ccatggagca gttaactcca gaactcaagt cttcaatcct caaatgtgtc caaagtacaa 1680
agccatcact gatgatccag aaagctgcca tccaggctct gcggaaaatg gagcctaaag 1740
acaaggacca ggaggttctt cttcagactt tccttgatga tgcttctccg ggagataagc 1800
gactggctgc ctatcttatg ttgatgagga gtccttcaca ggcagatatt aacaaaattg 1860
tccaaattct accatgggaa cagaatgagc aagtgaagaa ctttgtggct tcccatattg 1920
ccaatatctt gaactcagaa gaattggata tccaagatct gaaaaagtta gtgaaagaag 1980
ctctgaaaga atctcaactt ccaactgtca tggacttcag aaaattctct cggaactatc 2040
aactctacaa atctgtttct cttccatcac ttgacccagc ctcagccaaa atagaaggga 2100
atcttatatt tgatccaaat aactaccttc ctaaagaaag catgctgaaa actaccctca 2160
ctgcctttgg atttgcttca gctgacctca tcgagattgg cttggaagga aaaggctttg 2220
agccaacatt ggaagctctt tttgggaagc aaggattttt cccagacagt gtcaacaaag 2280
ctttgtactg ggttaatggt caagttcctg atggtgtctc taaggtctta gtggaccact 2340
ttggctatac caaagatgat aaacatgagc aggatatggt aaatggaata atgctcagtg 2400
ttgagaagct gattaaagat ttgaaatcca aagaagtccc ggaagccaga gcctacctcc 2460
gcatcttggg agaggagctt ggttttgcca gtctccatga cctccagctc ctgggaaagc 2520
tgcttctgat gggtgcccgc actctgcagg ggatccccca gatgattgga gaggtcatca 2580
ggaagggctc aaagaatgac ttttttcttc actacatctt catggagaat gcctttgaac 2640
tccccactgg agctggatta cagttgcaaa tatcttcatc tggagtcatt gctcccggag 2700
ccaaggctgg agtaaaactg gaagtagcca acatgcaggc tgaactggtg gcaaaaccct 2760
ccgtgtctgt ggagtttgtg acaaatatgg gcatcatcat tccggacttc gctaggagtg 2820
gggtccagat gaacaccaac ttcttccacg agtcgggtct ggaggctcat gttgccctaa 2880
aagctgggaa gctgaagttt atcattcctt ccccaaagag accagtcaag ctgctcagtg 2940
gaggcaacac attacatttg gtctctacca ccaaaacgga ggtgatccca cctctcattg 3000
agaacaggca gtcctggtca gtttgcaagc aagtctttcc tggcctgaat tactgcacct 3060
caggcgctta ctccaacgcc agctccacag actccgcctc ctactatccg ctgaccgggg 3120
acaccagatt agagctggaa ctgaggccta caggagagat tgagcagtat tctgtcagcg 3180
caacctatga gctccagaga gaggacagag ccttggtgga taccctgaag tttgtaactc 3240
aagcagaagg tgcgaagcag actgaggcta ccatgacatt caaatataat cggcagagta 3300
tgaccttgtc cagtgaagtc caaattccgg attttgatgt tgacctcgga acaatcctca 3360
gagttaatga tgaatctact gagggcaaaa cgtcttacag actcaccctg gacattcaga 3420
acaagaaaat tactgaggtc gccctcatgg gccacctaag ttgtgacaca aaggaagaaa 3480
gaaaaatcaa gggtgttatt tccatacccc gtttgcaagc agaagccaga agtgagatcc 3540
tcgcccactg gtcgcctgcc aaactgcttc tccaaatgga ctcatctgct acagcttatg 3600
gctccacagt ttccaagagg gtggcatggc attatgatga agagaagatt gaatttgaat 3660
ggaacacagg caccaatgta gataccaaaa aaatgacttc caatttccct gtggatctct 3720
ccgattatcc taagagcttg catatgtatg ctaatagact cctggatcac agagtccctg 3780
aaacagacat gactttccgg cacgtgggtt ccaaattaat agttgcaatg agctcatggc 3840
ttcagaaggc atctgggagt cttccttata cccagacttt gcaagaccac ctcaatagcc 3900
tgaaggagtt caacctccag aacatgggat tgccagactt ccacatccca gaaaacctct 3960
tcttaaaaag cgatggccgg gtcaaatata ccttgaacaa gaacagtttg aaaattgaga 4020
ttcctttgcc ttttggtggc aaatcctcca gagatctaaa gatgttagag actgttagga 4080
caccagccct ccacttcaag tctgtgggat tccatctgcc atctcgagag ttccaagtcc 4140
ctacttttac cattcccaag ttgtatcaac tgcaagtgcc tctcctgggt gttctagacc 4200
tctccacgaa tgtctacagc aacttgtaca actggtccgc ctcctacagt ggtggcaaca 4260
ccagcacaga ccatttcagc cttcgggctc gttaccacat gaaggctgac tctgtggttg 4320
acctgctttc ctacaatgtg caaggatctg gagaaacaac atatgaccac aagaatacgt 4380
tcacactatc atgtgatggg tctctacgcc acaaatttct agattcgaat atcaaattca 4440
gtcatgtaga aaaacttgga aacaacccag tctcaaaagg tttactaata ttcgatgcat 4500
ctagttcctg gggaccacag atgtctgctt cagttcattt ggactccaaa aagaaacagc 4560
atttgtttgt caaagaagtc aagattgatg ggcagttcag agtctcttcg ttctatgcta 4620
aaggcacata tggcctgtct tgtcagaggg atcctaacac tggccggctc aatggagagt 4680
ccaacctgag gtttaactcc tcctacctcc aaggcaccaa ccagataaca ggaagatatg 4740
aagatggaac cctctccctc acctccacct ctgatctgca aagtggcatc attaaaaata 4800
ctgcttccct aaagtatgag aactacgagc tgactttaaa atctgacacc aatgggaagt 4860
ataagaactt tgccacttct aacaagatgg atatgacctt ctctaagcaa aatgcactgc 4920
tgcgttctga atatcaggct gattacgagt cattgaggtt cttcagcctg ctttctggat 4980
cactaaattc ccatggtctt gagttaaatg ctgacatctt aggcactgac aaaattaata 5040
gtggtgctca caaggcgaca ctaaggattg gccaagatgg aatatctacc agtgcaacga 5100
ccaacttgaa gtgtagtctc ctggtgctgg agaatgagct gaatgcagag cttggcctct 5160
ctggggcatc tatgaaatta acaacaaatg gccgcttcag ggaacacaat gcaaaattca 5220
gtctggatgg gaaagccgcc ctcacagagc tatcactggg aagtgcttat caggccatga 5280
ttctgggtgt cgacagcaaa aacattttca acttcaaggt cagtcaagaa ggacttaagc 5340
tctcaaatga catgatgggc tcatatgctg aaatgaaatt tgaccacaca aacagtctga 5400
acattgcagg cttatcactg gacttctctt caaaacttga caacatttac agctctgaca 5460
agttttataa gcaaactgtt aatttacagc tacagcccta ttctctggta actactttaa 5520

acagtgacct gaaatacaat gctctggatc tcaccaacaa tgggaaacta cggctagaac 5580
ccctgaagct gcatgtggct ggtaacctaa aaggagccta ccaaaataat gaaataaaac 5640
acatctatgc catctcttct gctgccttat cagcaagcta taaagcagac actgttgcta 5700
aggttcaggg tgtggagttt agccatcggc tcaacacaga catcgctggg ctggcttcag 5760
ccattgacat gagcacaaac tataattcag actcactgca tttcagcaat gtcttccgtt 5820
ctgtaatggc cccgtttacc atgaccatcg atgcacatac aaatggcaat gggaaactcg 5880
ctctctgggg agaacatact gggcagctgt atagcaaatt cctgttgaaa gcagaacctc 5940
tggcatttac tttctctcat gattacaaag gctccacaag tcatcatctc gtgtctagga 6000
aaagcatcag tgcagctctt gaacacaaag tcagtgccct gcttactcca gctgagcaga 6060
caggcacctg gaaactcaag acccaattta acaacaatga atacagccag gacttggatg 6120
cttacaacac taaagataaa attggcgtgg agcttactgg acgaactctg gctgacctaa 6180
ctctactaga ctccccaatt aaagtgccac ttttactcag tgagcccatc aatatcattg 6240
atgctttaga gatgagagat gccgttgaga agccccaaga atttacaatt gttgcttttg 6300
taaagtatga taaaaaccaa gatgttcact ccattaacct cccatttttt gagaccttgc 6360
aagaatattt tgagaggaat cgacaaacca ttatagttgt agtggaaaac gtacagagaa 6420
acctgaagca catcaatatt gatcaatttg taagaaaata cagagcagcc ctgggaaaac 6480
tcccacagca agctaatgat tatctgaatt cattcaattg ggagagacaa gtttcacatg 6540
ccaaggagaa actgactgct ctcacaaaaa agtatagaat tacagaaaat gatatacaaa 6600
ttgcattaga tgatgccaaa atcaacttta atgaaaaact atctcaactg cagacatata 6660
tgatacaatt tgatcagtat attaaagata gttatgattt acatgatttg aaaatagcta 6720
ttgctaatat tattgatgaa atcattgaaa aattaaaaag tcttgatgag cactatcata 6780
tccgtgtaaa tttagtaaaa acaatccatg atctacattt gtttattgaa aatattgatt 6840
ttaacaaaag tggaagtagt actgcatcct ggattcaaaa tgtggatact aagtaccaaa 6900
tcagaatcca gatacaagaa aaactgcagc agcttaagag acacatacag aatatagaca 6960
tccagcacct agctggaaag ttaaaacaac acattgaggc tattgatgtt agagtgcttt 7020
tagatcaatt gggaactaca atttcatttg aaagaataaa tgatgttctt gagcatgtca 7080
aacactttgt tataaatctt attggggatt ttgaagtagc tgagaaaatc aatgccttca 7140
gagccaaagt ccatgagtta atcgagaggt atgaagtaga ccaacaaatc caggttttaa 7200
tggataaatt agtagagttg acccaccaat acaagttgaa ggagactatt cagaagctaa 7260
gcaatgtcct acaacaagtt aagataaaag attactttga gaaattggtt ggatttattg 7320
atgatgctgt gaagaagctt aatgaattat cttttaaaac attcattgaa gatgttaaca 7380
aattccttga catgttgata aagaaattaa agtcatttga ttaccaccag tttgtagatg 7440
aaaccaatga caaaatccgt gaggtgactc agagactcaa tggtgaaatt caggctctgg 7500
aactaccaca aaaagctgaa gcattaaaac tgtttttaga ggaaaccaag gccacagttg 7560
cagtgtatct ggaaagccta caggacacca aaataacctt aatcatcaat tggttacagg 7620
aggctttaag ttcagcatct ttggctcaca tgaaggccaa attccgagag actctagaag 7680
atacacgaga ccgaatgtat caaatggaca ttcagcagga acttcaacga tacctgtctc 7740
tggtaggcca ggtttatagc acacttgtca cctacatttc tgattggtgg actcttgctg 7800
ctaagaacct tactgacttt gcagagcaat attctatcca agattgggct aaacgtatga 7860
aagcattggt agagcaaggg ttcactgttc ctgaaatcaa gaccatcctt gggaccatgc 7920
ctgcctttga agtcagtctt caggctcttc agaaagctac cttccagaca cctgatttta 7980
tagtccccct aacagatttg aggattccat cagttcagat aaacttcaaa gacttaaaaa 8040
atataaaaat cccatccagg ttttccacac cagaatttac catccttaac accttccaca 8100
ttccttcctt tacaattgac tttgtcgaaa tgaaagtaaa gatcatcaga accattgacc 8160
agatgcagaa cagtgagctg cagtggcccg ttccagatat atatctcagg gatctgaagg 8220
tggaggacat tcctctagcg agaatcaccc tgccagactt ccgtttacca gaaatcgcaa 8280
ttccagaatt cataatccca actctcaacc ttaatgattt tcaagttcct gaccttcaca 8340
taccagaatt ccagcttccc cacatctcac acacaattga agtacctact tttggcaagc 8400
tatacagtat tctgaaaatc caatctcctc ttttcacatt agatgcaaat gctgacatag 8460
ggaatggaac cacctcagca aacgaagcag gtatcgcagc ttccatcact gccaaaggag 8520
agtccaaatt agaagttctc aattttgatt ttcaagcaaa tgcacaactc tcaaacccta 8580
agattaatcc gctggctctg aaggagtcag tgaagttctc cagcaagtac ctgagaacgg 8640
agcatgggag tgaaatgctg ttttttggaa atgctattga gggaaaatca aacacagtgg 8700
caagtttaca cacagaaaaa aatacactgg agcttagtaa tggagtgatt gtcaagataa 8760
acaatcagct taccctggat agcaacacta aatacttcca caaattgaac atccccaaac 8820
tggacttctc tagtcaggct gacctgcgca acgagatcaa gacactgttg aaagctggcc 8880
acatagcatg gacttcttct ggaaaagggt catggaaatg ggcctgcccc agattctcag 8940
atgagggaac acatgaatca caaattagtt tcaccataga aggacccctc acttcctttg 9000
gactgtccaa taagatcaat agcaaacacc taagagtaaa ccaaaacttg gtttatgaat 9060
ctggctccct caacttttct aaacttgaaa ttcaatcaca agtcgattcc cagcatgtgg 9120
gccacagtgt tctaactgct aaaggcatgg cactgtttgg agaagggaag gcagagttta 9180
ctgggaggca tgatgctcat ttaaatggaa aggttattgg aactttgaaa aattctcttt 9240
tcttttcagc ccagccattt gagatcacgg catccacaaa caatgaaggg aatttgaaag 9300
ttcgttttcc attaaggtta acagggaaga tagacttcct gaataactat gcactgtttc 9360
tgagtcccag tgcccagcaa gcaagttggc aagtaagtgc taggttcaat cagtataagt 9420

acaaccaaaa tttctctgct ggaaacaacg agaacattat ggaggcccat gtaggaataa 9480
atggagaagc aaatctggat ttcttaaaca ttcctttaac aattcctgaa atgcgtctac 9540
cttacacaat aatcacaact cctccactga aagatttctc tctatgggaa aaaacaggct 9600
tgaaggaatt cttgaaaacg acaaagcaat catttgattt aagtgtaaaa gctcagtata 9660
agaaaaacaa acacaggcat tccatcacaa atcctttggc tgtgctttgt gagtttatca 9720
gtcagagcat caaatccttt gacaggcatt ttgaaaaaaa cagaaacaat gcattagatt 9780
ttgtcaccaa atcctataat gaaacaaaaa ttaagtttga taagtacaaa gctgaaaaat 9840
ctcacgacga gctccccagg acctttcaaa ttcctggata cactgttcca gttgtcaatg 9900
ttgaagtgtc tccattcacc atagagatgt cggcattcgg ctatgtgttc ccaaaagcag 9960
tcagcatgcc tagtttctcc atcctaggtt ctgacgtccg tgtgccttca tacacattaa 10020
tcctgccatc attagagctg ccagtccttc atgtccctag aaatctcaag ctttctcttc 10080
cacatttcaa ggaattgtgt accataagcc atatttttat tcctgccatg ggcaatatta 10140
cctatgattt ctcctttaaa tcaagtgtca tcacactgaa taccaatgct gaacttttta 10200
accagtcaga tattgttgct catctccttt cttcatcttc atctgtcatt gatgcactgc 10260
agtacaaatt agagggcacc acaagattga caagaaaaag gggattgaag ttagccacag 10320
ctctgtctct gagcaacaaa tttgtggagg gtagtcataa cagtactgtg agcttaacca 10380
cgaaaaatat ggaagtgtca gtggcaaaaa ccacaaaagc cgaaattcca attttgagaa 10440
tgaatttcaa gcaagaactt aatggaaata ccaagtcaaa acctactgtc tcttcctcca 10500
tggaatttaa gtatgatttc aattcttcaa tgctgtactc taccgctaaa ggagcagttg 10560
accacaagct tagcttggaa agcctcacct cttacttttc cattgagtca tctaccaaag 10620
gagatgtcaa gggttcggtt ctttctcggg aatattcagg aactattgct agtgaggcca 10680
acacttactt gaattccaag agcacacggt cttcagtgaa gctgcagggc acttccaaaa 10740
ttgatgatat ctggaacctt gaagtaaaag aaaattttgc tggagaagcc acactccaac 10800
gcatatattc cctctgggag cacagtacga aaaaccactt acagctagag ggcctctttt 10860
tcaccaacgg agaacataca agcaaagcca ccctggaact ctctccatgg caaatgtcag 10920
ctcttgttca ggtccatgca agtcagccca gttccttcca tgatttccct gaccttggcc 10980
aggaagtggc cctgaatgct aacactaaga accagaagat cagatggaaa aatgaagtcc 11040
ggattcattc tgggtctttc cagagccagg tcgagctttc caatgaccaa gaaaaggcac 11100
accttgacat tgcaggatcc ttagaaggac acctaaggtt cctcaaaaat atcatcctac 11160
cagtctatga caagagctta tgggatttcc taaagctgga tgtaaccacc agcattggta 11220
ggagacagca tcttcgtgtt tcaactgcct ttgtgtacac caaaaacccc aatggctatt 11280
cattctccat ccctgtaaaa gttttggctg ataaattcat tactcctggg ctgaaactaa 11340
atgatctaaa ttcagttctt gtcatgccta cgttccatgt cccatttaca gatcttcagg 11400
ttccatcgtg caaacttgac ttcagagaaa tacaaatcta taagaagctg agaacttcat 11460
catttgccct caacctacca acactccccg aggtaaaatt ccctgaagtt gatgtgttaa 11520
caaaatattc tcaaccagaa gactccttga ttcccttttt tgagataacc gtgcctgaat 11580
ctcagttaac tgtgtcccag ttcacgcttc caaaaagtgt ttcagatggc attgctgctt 11640
tggatctaaa tgcagtagcc aacaagatcg cagactttga gttgcccacc atcatcgtgc 11700
ctgagcagac cattgagatt ccctccatta agttctctgt acctgctgga attgtcattc 11760
cttcctttca agcactgact gcacgctttg aggtagactc tcccgtgtat aatgccactt 11820
ggagtgccag tttgaaaaac aaagcagatt atgttgaaac agtcctggat tccacatgca 11880
gctcaaccgt acagttccta gaatatgaac taaatgtttt gggaacacac aaaatcgaag 11940
atggtacgtt agcctctaag actaaaggaa cacttgcaca ccgtgacttc agtgcagaat 12000
atgaagaaga tggcaaattt gaaggacttc aggaatggga aggaaaagcg cacctcaata 12060
tcaaaagccc agcgttcacc gatctccatc tgcgctacca gaaagacaag aaaggcatct 12120
ccacctcagc agcctcccca gccgtaggca ccgtgggcat ggatatggat gaagatgacg 12180
acttttctaa atggaacttc tactacagcc ctcagtcctc tccagataaa aaactcacca 12240
tattcaaaac tgagttgagg gtccgggaat ctgatgagga aactcagatc aaagttaatt 12300
gggaagaaga ggcagcttct ggcttgctaa cctctctgaa agacaacgtg cccaaggcca 12360
caggggtcct ttatgattat gtcaacaagt accactggga acacacaggg ctcaccctga 12420
gagaagtgtc ttcaaagctg agaagaaatc tgcagaacaa tgctgagtgg gtttatcaag 12480
gggccattag gcaaattgat gatatcgacg tgaggttcca gaaagcagcc agtggcacca 12540
ctgggaccta ccaagagtgg aaggacaagg cccagaatct gtaccaggaa ctgttgactc 12600
aggaaggcca agccagtttc cagggactca aggataacgt gtttgatggc ttggtacgag 12660
ttactcaaaa attccatatg aaagtcaagc atctgattga ctcactcatt gattttctga 12720
acttccccag attccagttt ccggggaaac ctgggatata cactagggag gaactttgca 12780
ctatgttcat aagggaggta gggacggtac tgtcccaggt atattcgaaa gtccataatg 12840
gttcagaaat actgttttcc tatttccaag acctagtgat tacacttcct ttcgagttaa 12900
ggaaacataa actaatagat gtaatctcga tgtataggga actgttgaaa gatttatcaa 12960
aagaagccca agaggtattt aaagccattc agtctctcaa gaccacagag gtgctacgta 13020
atcttcagga ccttttacaa ttcattttcc aactaataga agataacatt aaacagctga 13080
aagagatgaa atttacttat cttattaatt atatccaaga tgagatcaac acaatcttca 13140
atgattatat cccatatgtt tttaaattgt tgaaagaaaa cctatgcctt aatcttcata 13200
agttcaatga atttattcaa aacgagcttc aggaagcttc tcaagagtta cagcagatcc 13260
atcaatacat tatggccctt cgtgaagaat attttgatcc aagtatagtt ggctggacag 13320

tgaaatatta tgaacttgaa gaaaagatag tcagtctgat caagaacctg ttagttgctc 13380
ttaaggactt ccattctgaa tatattgtca gtgcctctaa ctttacttcc caactctcaa 13440
gtcaagttga gcaatttctg cacagaaata ttcaggaata tcttagcatc cttaccgatc 13500
cagatggaaa agggaaagag aagattgcag agctttctgc cactgctcag gaaataatta 13560
aaagccaggc cattgcgacg aagaaaataa tttctgatta ccaccagcag tttagatata 13620
aactgcaaga tttttcagac caactctctg attactatga aaaatttatt gctgaatcca 13680
aaagattgat tgacctgtcc attcaaaact accacacatt tctgatatac atcacggagt 13740
tactgaaaaa gctgcaatca accacagtca tgaaccccta catgaagctt gctccaggag 13800
aacttactat catcctctaa ttttttaaaa gaaatcttca tttattcttc ttttccaatt 13860
gaactttcac atagcacaga aaaaattcaa actgcctata ttgataaaac catacagtga 13920
gccagccttg cagtaggcag tagactataa gcagaagcac atatgaactg gacctgcacc 13980
aaagctggca ccagggctcg gaaggtctct gaactcagaa ggatggcatt ttttgcaagt 14040
taaagaaaat caggatctga gttattttgc taaacttggg ggaggaggaa caaataaatg 14100
gagtctttat tgtgtatcat a 14121<210>
2
<211> 13928
<212> DNA
<213> Mus musculus

<400> 2
atcagtgcct gcagtggatc aagtacctgc ctgagctccg cctccgaaga ccctgtagag 60
caagcagcag gggctaggcc cgtggccagg ccacagccag gaagccaccc caccatccat 120
ccgccatggg cccacgaaag cctgccctgc ggacgccgtt actgctgctg ttcctgctac 180
tgttcttgga caccagcgtc tgggctcaag atgaagtcct ggaaaactta agcttcagct 240
gtccaaaaga tgcaactcga ttcaagcacc tccgaaagta cgtgtacaac tatgaagctg 300
aaagttccag cggtgtccag ggcacagctg actccagaag cgccaccaag atcaactgta 360
aggtagagct ggaggtcccc caaatctgtg gtttcatcat gaggaccaac cagtgtaccc 420
ttaaagaggt gtatggcttc aaccctgagg gcaaggcctt gatgaagaaa accaagaact 480
ctgaagagtt tgcagctgcc atgtccaggt acgaactcaa gctggccatt cctgaaggga 540
aacaaattgt tctttaccct gacaaggatg aacctaaata tatcctgaac atcaagaggg 600
gcatcatctc tgctcttctg gttcccccag agacagaaga ggaccaacaa gagttgttcc 660
tggataccgt gtatggaaac tgctcaactc aggttaccgt gaattcaaga aagggaaccg 720
taccaacaga aatgtccaca gagagaaacc tgcagcaatg tgacggcttc cagcccatca 780
gtacaagtgt cagccctctc gctctcatca aaggcctggt ccaccccttg tcaactctta 840
tcagcagcag ccaaacttgc cagtacaccc tggatcctaa gaggaagcat gtgtctgaag 900
ctgtctgtga tgagcagcat cttttcctgc ctttctccta caagaataag tatgggatca 960
tgacacgtgt tacacagaaa ctgagtcttg aagacacacc taagatcaac agtcgcttct 1020
tcagtgaagg taccaaccgg atgggtctgg cctttgagag caccaagtcc acgtcatccc 1080
caaagcaggc tgatgctgtt ttgaagaccc ttcaagaact gaaaaaattg tccatctcag 1140
agcagaatgc tcagagagca aatctcttca ataaactggt tactgagctg agaggcctca 1200
ctggtgaagc aatcacatcc ctcttgccac agctgattga agtgtccagc cccatcactt 1260
tacaagcctt ggttcagtgt ggacagccac agtgctatac tcacatcctc cagtggctga 1320
aaactgagaa ggctcacccc ctcctggttg acattgtcac ctacctgatg gctctgatcc 1380
caaatccctc aacacagagg ctgcaggaaa tctttaatac tgccaaggag cagcagagcc 1440
gagccactct gtatgcactg agccacgcag ttaacagcta ttttgatgtg gaccattcaa 1500
ggagcccagt tctgcaggat atcgctggtt acctgttgaa acagatcgac aatgaatgca 1560
cgggcaatga agaccacacc ttcttgattc tgagggtcat tggaaatatg ggaagaacca 1620
tggaacaagt aatgccagcc ctcaagtcct cagtcctgag ctgtgtacga agtacaaaac 1680
catctctgct gattcagaaa gctgctctcc aggccctgag gaagatggaa ctggaagatg 1740
aggtccggac gatccttttt gatacatttg taaatggtgt cgctcccgtg gagaagagac 1800
tggctgccta tctcttgctg atgaagaacc cttcctcatc agatattaac aaaattgccc 1860
aacttctcca atgggaacag agtgagcagg tgaagaactt cgtggcatct cacattgcca 1920
acatcttgaa ctcggaagaa ctgtatgtcc aagatctgaa agttttgatc aaaaatgctc 1980
tggagaattc tcaatttcca acgatcatgg acttcagaaa attttcccga aactatcaga 2040
tttccaaatc tgcttctctc ccaatgttcg acccagtctc agtcaaaata gaagggaatc 2100
ttatatttga tccaagcagt tatcttccca gagaaagctt gctgaaaaca accctcacag 2160
tctttggact tgcttcactt gatctctttg agattggttt agaaggaaaa gggtttgagc 2220
caacactaga agctcttttt ggtaagcaag gattcttccc agacagtgtc aacaaggctt 2280
tgtattgggt caatggccga gttccagatg gtgtctccaa ggtcttggtg gaccactttg 2340
gctatactac agatggcaag catgaacagg acatggtgaa tggaatcatg cccattgtgg 2400
acaagttgat caaagatctg aaatctaaag aaattcctga agccagggcc tatctccgca 2460
tcctaggaaa agagctaagc tttgtcagac tccaagacct ccaagtcctg gggaagctgt 2520
tgctgagtgg tgcacaaact ttgcagggaa tcccccagat ggttgtacag gccatcagag 2580
aagggtcaaa gaatgacttg tttctccact acatcttcat ggacaatgcc tttgagctcc 2640
ccactggagc agggttacag ctgcaagtgt cctcgtctgg agtcttcacc cccgggatca 2700

aggctggtgt aagactggaa ttagccaaca tacaggcaga gctagtggca aagccctctg 2760
tgtccttgga gtttgtgaca aatatgggca tcatcatccc agacttcgct aagagcagtg 2820
tccagatgaa caccaacttc ttccacgagt caggcctgga ggcgcgagtg gccctgaagg 2880
ctgggcagct gaaggtcatc attccttctc caaagaggcc agtcaagctg ttcagtggca 2940
gcaacacact gcatctggtc tctaccacca aaacagaagt gatcccacct ctggttgaga 3000
acaggcagtc ctggtcaact tgcaagcctc tcttcactgg aatgaactac tgtaccacag 3060
gagcttactc caacgccagc tccacggagt ctgcctctta ctacccactg acaggggaca 3120
caaggtatga gctggagctg aggcccacgg gagaagtgga gcagtattct gccactgcaa 3180
cctatgaact cctaaaagag gacaagtctt tggttgacac attgaagttc ctagttcaag 3240
cagaaggagt gcagcagtct gaagctactg tactgttcaa atataatcgg agaagcagga 3300
ccttatctag tgaagtccta attccagggt ttgatgtcaa cttcgggaca atactaagag 3360
ttaatgatga atctgctaag gacaaaaaca cttacaaact catcctggac attcagaaca 3420
agaaaatcac tgaggtctct ctcgtgggcc acttgagtta tgataaaaag ggagatggca 3480
agatcaaagg tgttgtttcc ataccacgtt tgcaagcaga agccaggagt gaggtccaca 3540
cccactggtc ctccaccaaa ctgctcttcc aaatggactc atctgctaca gcttacggct 3600
caacaatttc caagagagtg acatggcgtt acgataatga gataatagaa tttgattgga 3660
acacgggaac caatgtggat accaaaaaag tggcctccaa tttccctgtg gatctttccc 3720
attatcctag aatgttgcat gagtatgcca atggtctcct ggatcacaga gtccctcaaa 3780
cagatgtgac ttttcgggac atgggttcca aattaattgt tgcaacaaac acatggcttc 3840
agatggcaac caggggtctt ccttaccccc aaactctaca ggatcacctc aatagcctct 3900
cagagttgaa cctcctgaaa atgggactgt ctgacttcca tattccagac aacctcttcc 3960
taaagactga tggcagagtc aaatacacaa tgaacaggaa caaaataaac attgacatcc 4020
ctttgccttt gggtggcaag tcttcaaaag acctcaagat gccagagagt gtgaggacac 4080
cagccctcaa cttcaagtct gtgggattcc atctgccatc tcgagaggtc caggtcccca 4140
cttttacaat ccccaagaca catcagcttc aagtgcctct cttgggtgtt ctagaccttt 4200
ccacaaatgt ctacagcaat ttgtacaact ggtcagcctc ctacactggt ggcaacacca 4260
gcagagacca cttcagcctt caggctcagt accgcatgaa gactgactct gtggttgacc 4320
tgttttccta cagtgtgcaa ggatctggag aaacaacata tgacagcaag aacacattta 4380
cattgtcctg tgatggatct ctacaccata aatttctaga ctcaaaattc aaagtcagcc 4440
acgtagaaaa atttggaaac agcccagtct caaaaggttt actaacattt gaaacatcta 4500
gtgccttggg accacagatg tctgctactg ttcacctaga ctcaaaaaag aaacaacatc 4560
tatacgtcaa agatatcaag gttgatggac agttcagagc ttcttcattt tatgctcaag 4620
gcaaatatgg cctgtcttgt gagagagatg ttacaactgg ccagctgagc ggcgaatcca 4680
acatgagatt taactccacc tacttccagg gcaccaacca gatcgtggga atgtaccagg 4740
atggagccct gtccatcacc tccacttctg acctgcaaga tggcatattc aagaacacag 4800
cttccttgaa atatgaaaac tatgagctga ctctgaaatc tgatagcagt gggcagtatg 4860
agaacttcgc tgcttccaac aagctggatg tgaccttctc tacgcaaagt gcactgctgc 4920
gttctgaaca ccaggccaat tacaagtccc tgaggcttgt cacccttctt tcaggatccc 4980
tcacttccca gggtgtagaa ttaaatgctg acatcttggg cacagacaaa attaatactg 5040
gtgctcacaa ggcaacacta aagattgcac gtgatggact atcaaccagt gcgaccacca 5100
acttgaagta cagccccctg ctgctggaga atgagttgaa tgcagagctt gggctctctg 5160
gggcatccat gaaattatca acaaacggcc gcttcaaaga acaccatgca aaattcagtc 5220
ttgatgggag agctgccctc acagaggtgt cactggggag catttaccag gccatgattc 5280
tgggtgcaga cagcaaaaac atcttcaact tcaaactcag ccgagaaggg ctgaggctgt 5340
ccaatgattt gatgggctcc tatgctgaga tgaaacttga ccacacacac agtctgaaca 5400
ttgcaggtct ctcactggac ttcttctcaa aaatggacaa tatttacagt ggagacaagt 5460
tctataagca gaattttaac ttacagctac agccctattc tttcataact actttaagca 5520
acgacctgag atatggtgct ctagatttga ccaacaatgg aaggtttcgg ctggagccac 5580
tgaagctgaa tgtgggtggc aactttaaag gaacctatca aaataatgag ctgaaacata 5640
tctataccat atcttatact gacctggtag tagcaagtta cagagcagac actgtggcta 5700
aggttcaggg tgtcgaattc agccataggc taaatgcaga cattgaagga ctgacttcct 5760
ctgttgatgt cactaccagc tacaattcag atccactgca ttttaacaat gttttccact 5820
tttctctggc accttttacc ttgggcatcg acacacatac aagtggtgat gggaaactgt 5880
ccttctgggg agaacacact gggcagctat atagtaagtt tctgttgaaa gcagaacctc 5940
tggcacttat tgtctctcat gactacaaag gatccacaag ccacagtctc ccgtacgaga 6000
gcagcatcag cacggctctt gaacacacag tcagtgcctt gctgacgcca gctgagcaga 6060
caagcacctg gaaattcaag accaaactga atgacaaagt atacagccag gactttgaag 6120
cctacaacac taaagacaaa atcggtgttg agcttagtgg acgggctgac ctctctgggc 6180
tgtattctcc aattaaacta ccgtttttct acagtgagcc tgtcaatgtc cttaatggct 6240
tagaggtaaa tgatgctgtt gacaagcccc aagaattcac aattattgct gtggtgaagt 6300
acgataagaa ccaggatgtt cacaccatca acctcccatt cttcaaaagc ctgccagact 6360
atttggagag aaatcgaaga ggaatgataa gtctactgga agccatgcga ggggaattgc 6420
aacgcctcag tgttgatcag tttgtgagga aatacagagc ggccctgagc agacttcctc 6480
agcagattca tcattatctg aatgcatctg actgggagag acaagtagct ggtgccaagg 6540
aaaaaataac ttctttcatg gaaaattata gaattacaga taatgatgta ctaattgcca 6600

tagatagtgc caaaatcaac ttcaatgaaa aactctctca acttgagaca tacgcgatac 6660
aatttgatca gtatattaaa gataattatg atccacatga cttaaaaaga actattgctg 6720
agattattga tcgaatcatt gaaaagttaa aaattcttga tgaacagtat catatccgtg 6780
taaatctagc aaaatcaatc cataatctct atttatttgt tgaaaacgtt gatcttaacc 6840
aagtcagtag tagtaacacc tcttggatcc aaaatgtgga ttccaattat caagtcagaa 6900
tccaaattca agaaaaacta cagcagctca ggacacaaat tcagaatata gacattcagc 6960
agcttgctgc agaggtaaaa cgacagatgg acgctattga tgtcacaatg catttagatc 7020
aattgagaac tgcaattcta ttccaaagaa taagtgacat tattgaccgt gtcaaatact 7080
ttgttatgaa tcttattgaa gattttaaag taactgagaa aatcaatact tttagagtta 7140
tagtccgtga gctaattgag aaatatgaag tagaccaaca catccaggtt ttaatggata 7200
aatcagtaga gttggcccac agatatagcc tgagcgagcc tcttcagaaa ctcagtaatg 7260
tgctacagcg aattgagata aaagattact atgagaaatt ggttgggttt attgatgata 7320
ctgttgagtg gcttaaagca ttgtctttca aaaataccat tgaagaacta aatagattga 7380
ctgacatgtt ggtgaagaag ttgaaagcat ttgattatca ccagtttgta gacaaaacca 7440
acagcaaaat ccgtgagatg actcagagaa tcaatgctga aatccaagct ctcaaacttc 7500
cacaaaaaat ggaagcatta aaactgttgg tagaagactt caaaaccaca gtctccaatt 7560
ccctggaaag actcaaggac accaaagtaa ctgtggtcat tgattggctg caggatattt 7620
tgactcaaat gaaagaccat ttccaagata ctctggaaga tgtaagagac cgaatttatc 7680
aaatggacat tcagagggaa ctggagcact tcttgtctct ggtaaaccaa gtttacagta 7740
cactggtcac ctatatgtct gactggtgga ctctgactgc taaaaacata acagactttg 7800
cagagcaata ttccatccaa aactgggctg agagtataaa agtactggtg gaacaaggat 7860
tcatagttcc tgaaatgcaa acatttctgt ggaccatgcc tgcttttgag gtcagtctcc 7920
gtgctctcca agaaggtaac tttcagaccc ctgtctttat agtccccttg acagatttga 7980
ggattccatc aattcggata aactttaaaa tgttaaagaa tataaaaatc ccattgagat 8040
tttccactcc agaattcact cttctcaaca ccttccatgt ccattccttt acaattgact 8100
tgctggaaat aaaagcaaag atcattagaa ctatcgacca aattttgagc agtgagctac 8160
agtggcctct tccagaaatg tatttgagag acctggatgt agtgaacatt cctcttgcaa 8220
gactgactct gccagacttc catgtaccag aaatcacaat tccagaattc acaatcccaa 8280
atgtcaatct caaagattta cacgttcctg atcttcacat accagaattc caacttcctc 8340
acctctcaca tacaattgaa atacctgctt ttggcaaact gcatagcatc cttaagatcc 8400
aatctcctct ctttatatta gatgctaatg ccaacataca gaatgtaaca acttcaggga 8460
acaaagcaga gattgtggct tctgtcactg ctaaaggaga gtcccaattt gaagctctca 8520
attttgattt tcaagcacaa gctcaattcc tggagttaaa tcctcatcct ccagtcctga 8580
aggaatccat gaacttctcc agtaagcatg tgagaatgga gcatgagggt gagatagtat 8640
ttgatggaaa ggccattgag gggaaatcag acacagtcgc aagtttacac acagagaaaa 8700
atgaagtaga gtttaataat ggtatgactg tcaaagtaaa caatcagctc acccttgaca 8760
gtcacacaaa gtacttccac aagttgagtg ttcctaggct ggacttctcc agtaaggctt 8820
ctcttaataa tgaaatcaag acactattag aagctggaca tgtggcattg acatcttcag 8880
ggacagggtc atggaactgg gcctgtccca acttctcgga tgaaggcata cattcgtccc 8940
aaattagctt tactgtggat ggtcccattg cttttgttgg actatccaat aacataaatg 9000
gcaaacactt acgggtcatc caaaaactga cttatgaatc tggcttcctc aactattcta 9060
agtttgaagt tgagtcaaaa gttgaatctc agcacgtggg ctccagcatt ctaacagcca 9120
atggtcgggc actgctcaag gacgcaaagg cagaaatgac tggtgagcac aatgccaact 9180
taaatggaaa agttattgga actttgaaaa attctctctt cttttcagca caaccatttg 9240
agattactgc atccacaaat aatgaaggaa atttgaaagt gggttttcca ctaaagctga 9300
ctgggaaaat agacttcctg aataactatg cattgtttct gagtccccgt gcccaacaag 9360
caagctggca agcgagtacc agattcaatc agtacaaata caatcaaaac ttttctgcta 9420
taaacaatga acacaacata gaagccagta taggaatgaa tggagatgcc aacctggatt 9480
tcttaaacat acctttaaca attcctgaaa ttaacttgcc ttacacggag ttcaaaactc 9540
ccttactgaa ggatttctcc atatgggaag aaacaggctt gaaagaattt ttgaagacaa 9600
caaagcaatc atttgatttg agtgtaaagg ctcaatataa aaagaacagt gacaagcatt 9660
ccattgttgt ccctctgggt atgttttatg aatttattct caacaatgtc aattcgtggg 9720
acagaaaatt tgagaaagtc agaaacaatg ctttacattt tcttaccacc tcctataatg 9780
aagcaaaaat taaggttgat aagtacaaaa ctgaaaattc ccttaatcag ccctctggga 9840
cctttcaaaa tcatggctac actatcccag ttgtcaacat tgaagtatct ccatttgctg 9900
tagagacact ggcttccagc catgtgatcc ccacagcaat aagcacccca agtgtcacaa 9960
tccctggtcc taacatcatg gtgccttcat acaagttagt gctgccaccc ctggagttgc 10020
cagttttcca tggtcctggg aatctattca agtttttcct cccagatttc aagggattca 10080
acactattga caatatttat attccagcca tgggcaactt tacctatgac ttttctttta 10140
aatcaagtgt catcacactg aataccaatg ctggacttta taaccaatca gatatcgttg 10200
cccatttcct ttcttcctct tcatttgtca ctgacgccct gcagtacaaa ttagagggaa 10260
catcacgtct gatgcgaaaa aggggattga aactagccac agctgtctct ctaactaaca 10320
aatttgtaaa gggcagtcat gacagcacca ttagtttaac caagaaaaac atggaagcat 10380
cagtgagaac aactgccaac ctccatgctc ccatattctc aatgaacttc aagcaggaac 10440
ttaatggaaa taccaagtca aaacccactg tttcatcatc cattgaacta aactatgact 10500

tcaattcctc aaagctgcac tctactgcaa caggaggcat tgatcacaag ttcagcttag 10560
aaagtctcac ttcctacttt tccattgagt cattcaccaa aggaaatatc aagagttcct 10620
tcctttctca ggaatattca ggaagtgttg ccaatgaagc caatgtatat ctgaattcca 10680
agggtactcg gtcttcagtg aggctacaag gagcttccaa agttgatggt atctggaacg 10740
ttgaagtagg agaaaatttt gctggagaag ccaccctcca acgcatctac accacatggg 10800
agcacaatat gaaaaaccat ttgcaggtat atagctactt cttcacaaaa ggaaagcaaa 10860
catgcagagc tactttggag ctctccccat ggaccatgtc aaccttgcta caggttcatg 10920
tgagtcaact cagttccctc cttgacctcc atcactttga ccaggaagtg atcctaaaag 10980
ctaacactaa gaaccagaag atcagctgga aaggtggggt ccaggttgaa tcacgggttc 11040
ttcagcacaa tgcacagttc tccaatgacc aagaagaaat acggcttgac cttgcaggat 11100
ccttagacgg acagctgtgg gaccttgaag ctatcttttt accagtatat ggcaagagct 11160
tgcaggaact cctacaaatg gatggaaagc gacagtatct tcaagcttca acttctcttc 11220
tatataccaa aaaccctaat ggctatctcc tctcactccc cgtgcaagaa ctggctgata 11280
gatttattat accagggata aaactaaatg acttcagtgg agtaaaaatc tataagaagt 11340
taagtacttc accatttgcc ctcaacctaa caatgctccc caaagtaaaa ttccctggga 11400
ttgatctgtt aacacagtac tctacaccag agggctcctc tgtccctatt tttgaggcaa 11460
ctatacctga aattcattta actgtatccc agtttacact tccaaagagc cttccagttg 11520
gcaacacagt ctttgatctg aataagttgg ccaacatgat tgccgatgtt gacctgccta 11580
gtgtcaccct gcctgagcag actattgtaa tcccaccctt ggagttctct gtacctgctg 11640
ggatttttat tcctttcttt ggagaactga ctgcacgtgc tgggatggct tctcccctgt 11700
ataatgtcac ttggagcgct ggttggaaaa ccaaagcaga tcatgttgaa acgttcctag 11760
attccatgtg cacttcaacc ttgcagtttc tggagtatgc tttaaaagtt gtagaaacac 11820
acaaaattga agaagatctg ttaacctata atatcaaagg aacacttcaa cactgtgact 11880
tcaatgtgga gtataatgaa gatggtctat ttaaaggact ttgggactgg cagggagagg 11940
ctcacctgga catcaccagc ccagcactga ctgactttca tctgtactac aaagaagaca 12000
agacaagtct gtctgcctca gcagcctcct cgaccatcgg cactgtgggt ctggattcga 12060
gcacagatga ccagagtgtg gagctgaatg tctacttcca cccacagtcc cctccagaga 12120
agaaactcag catattcaaa actgagtgga ggtacaagga gtctgatggt gaaaggtaca 12180
tcaaaattaa ttgggaagaa gaggcagctt ccagattgct aggctcccta aaaagcaatg 12240
tgcccaaggc ttctaaggct atttatgatt atgccaataa gtaccacctg gaatacgttt 12300
cttcagaact aagaaaaagt ctacaggtca atgctgaaca tgccagaagg atggttgatg 12360
aaatgaacat gagtttccag agagtagccc gtgataccta ccagaatctc tatgaggaga 12420
tgttggctca gaagagcctg agcatccctg agaatctcaa gaagagggtg ttagacagta 12480
tagtacatgt tactcagaag taccacatgg cagtcatgtg gctgatggac tcattcattc 12540
attttctgaa attcaataga gtccagttcc cagggtacgc tggaacatat actgtggacg 12600
aactctacac tatagtcatg aaggaaacca agaagtcact gtctcagctg tttaatgggt 12660
taggaaacct actttcctac gttcaaaacc aagtagagaa atcaagatta atcaatgaca 12720
taacatttaa atgtcctttt ttctcaaaac cttgtaaact aaaagatctc atattgattt 12780
tcagggagga gttaaacatt ttatcaaaca taggccaaca ggatatcaag tttacaacaa 12840
tactaagtag tcttcagggc tttttggaga gagttttaga catcatagaa gaacaaatta 12900
aatgcctaaa ggacaatgaa tctacttgtg ttgctgacca tatcaacatg gttttcaaaa 12960
tacaggtccc atatgctttt aaatccctaa gagaagacat atactttgtc ctcggtgagt 13020
tcaatgactt tcttcaatcc atacttcagg aggggtccta caagctacag caggtccatc 13080
agtatatgaa ggcccttcgt gaagagtatt ttgatccgag catggttggg tggacagtga 13140
aatattatga aatagaagaa aatatggttg agctgatcaa gaccctttta gtttccttta 13200
gggatgtcta ctctgaatat agtgtgacag ctgctgattt tgcttccaaa atgtcaactc 13260
aagttgaaca atttgtgtcc agggatatca gagagtatct tagcatgctt actgatataa 13320
atggaaagtg gatggaaaag attgcagagc tttctattgt ggcaaaggaa acaatgaaaa 13380
gctgggtcac tgccgtggcc aaaataatgt ctgattaccc ccagcagttc cactccaatc 13440
tgcaggattt ttcagaccaa ctctctagct actatgaaaa atttgttggt gagtccacaa 13500
gattgattga cctgtccatt caaaactacc acgtgtttct cagatacatc accgagttac 13560
tgagaaagct gcaggtggcc acagccaata atgtgagccc ctatataaag cttgctcaag 13620
gagagctgat gatcaccttc tgattcatct actaacaaat tcaaattaaa ccttcacata 13680
gtaggagact ttgtagacta ctataaagac catcctgagc cagacctgca gtcaacagca 13740
agagcaagaa gcacatagga actatacctg caaccaagct ggcataagaa ccaagacctt 13800
caaagcagcc tgaactcaag atgacatatt ttacaagtta gagtaaagtc aagagctgag 13860
ttgttttgtc caactcagga tggagggagg gagggaaggg gaaataaata aatacttcct 13920
tattgtgc 13928<210>
3
<211> 2273
<212> DNA
<213> H. sapiens

<400> 3
gggggcagat cctggggaga atggaggagc acacagaggc aggctcggca ccagagatgg 60

gggcccagaa ggccctgatt gacaatcctg ctgacatcct agtcattgct gcatatttcc 120
tgctggtcat tggcgttggc ttgtggtcca tgtgcagaac caacagaggc actgtgggcg 180
gctacttcct ggcaggacgc agcatggtgt ggtggccggt tggggcctct ctcttcgcca 240
gcaacatcgg cagtggccac tttgtgggcc tggcagggac tggcgctgca agtggcttgg 300
ctgttgctgg attcgagtgg aatgcgctct tcgtggtgct gctactgggc tggctgtttg 360
cacccgtgta cctgacagcg ggggtcatca cgatgccaca gtacctgcgc aagcgcttcg 420
gcggccgccg catccgcctc tacctgtctg tgctctccct tttcctgtac atcttcacca 480
agatctcagt ggacatgttc tccggagctg tattcatcca gcaggctctg ggctggaaca 540
tctatgcctc cgtcatcgcg cttctgggca tcaccatgat ttacacggtg acaggagggc 600
tggccgcgct gatgtacacg gacacggtac agaccttcgt cattctgggg ggcgcctgca 660
tcctcatggg ttacgccttc cacgaggtgg gcgggtattc gggtctcttc gacaaatacc 720
tgggagcagc gacttcgctg acggtgtccg aggatccagc cgtgggaaac atctccagct 780
tctgctatcg accccggccc gactcctacc acctgctccg gcaccccgtg accggggatc 840
tgccgtggcc cgcgctgctc ctcggactca caatcgtctc gggctggtac tggtgcagcg 900
accaggtcat cgtgcagcgc tgcctggccg ggaagagcct gacccacatc aaggcgggct 960
gcatcctgtg tgggtacctg aagctgacgc ccatgtttct catggtcatg ccaggcatga 1020
tcagccgcat tctgtaccca gacgaggtgg cgtgcgtggt gcctgaggtg tgcaggcgcg 1080
tgtgcggcac ggaggtgggc tgctccaaca tcgcctaccc gcggctcgtc gtgaagctca 1140
tgcccaacgg tctgcgcgga ctcatgctgg cggtcatgct ggccgcgctc atgtcctcgc 1200
tggcctccat cttcaacagc agcagcacgc tcttcaccat ggacatctac acgcgcctgc 1260
ggccacgcgc cggcgaccgc gagctgctgc tggtgggacg gctctgggtg gtgttcatcg 1320
tggtagtgtc ggtggcctgg cttcccgtgg tgcaggcggc acagggcggg cagctcttcg 1380
attacatcca ggcagtctct agctacctgg caccgcccgt gtccgccgtc ttcgtgctgg 1440
cgctcttcgt gccgcgcgtt aatgagcagg gcgccttctg gggactcatc gggggcctgc 1500
tgatgggcct ggcacgcctg attcccgagt tctccttcgg ctcgggcagc tgtgtgcagc 1560
cctcggcgtg cccagctttc ctctgcggcg tgcactacct ctacttcgcc attgtgctgt 1620
tcttctgctc tggcctcctc accctcacgg tctccctgtg caccgcgccc atccccagaa 1680
agcacctcca ccgcctggtc ttcagtctcc ggcatagcaa ggaggaacgg gaggacctgg 1740
atgctgatga gcagcaaggc tcctcactcc ctgtacagaa tgggtgccca gagagtgcca 1800
tggagatgaa tgagccccag gccccggcac caagcctctt ccgccagtgc ctgctctggt 1860
tttgtggaat gagcagaggt ggggtgggca gtcctccgcc ccttacccag gaggaggcag 1920
cggcagcagc caggcggctg gaggacatca gcgaggaccc gagctgggcc cgtgtggtca 1980
acctcaatgc cctgctcatg atggcagtgg ccgtgttcct ctggggcttc tatgcctaag 2040
accaactgcg ttggacacca taagccacag cctcacagga agtgggggtg aggagcctgc 2100
ggtgctcccc agaaaagggg aaggggcagt ggggtgagaa ggtcctggct ccccttctcc 2160
cggccttcct ctgcctgggg cccactgcat ctgattggca gtcacttccc atgagggcct 2220
ggcccacccg ctgcagttgc cctaaggaaa aataaagctg cctttcccct gta 2273

<210> 4
<211> 3636
<212> DNA
<213> H. sapiens

<400> 4
cagcgacgtc gaggcgctca tggttgcagg cgggcgccgc cgttcagttc agggtctgag 60
cctggaggag tgagccaggc agtgagactg gctcgggcgg gccgggacgc gtcgttgcag 120
cagcggctcc cagctcccag ccaggattcc gcgcgcccct tcacgcgccc tgctcctgaa 180
cttcagctcc tgcacagtcc tccccaccgc aaggctcaag gcgccgccgg cgtggaccgc 240
gcacggcctc taggtctcct cgccaggaca gcaacctctc ccctggccct catgggcacc 300
gtcagctcca ggcggtcctg gtggccgctg ccactgctgc tgctgctgct gctgctcctg 360
ggtcccgcgg gcgcccgtgc gcaggaggac gaggacggcg actacgagga gctggtgcta 420
gccttgcgtt ccgaggagga cggcctggcc gaagcacccg agcacggaac cacagccacc 480
ttccaccgct gcgccaagga tccgtggagg ttgcctggca cctacgtggt ggtgctgaag 540
gaggagaccc acctctcgca gtcagagcgc actgcccgcc gcctgcaggc ccaggctgcc 600
cgccggggat acctcaccaa gatcctgcat gtcttccatg gccttcttcc tggcttcctg 660
gtgaagatga gtggcgacct gctggagctg gccttgaagt tgccccatgt cgactacatc 720
gaggaggact cctctgtctt tgcccagagc atcccgtgga acctggagcg gattacccct 780
ccacggtacc gggcggatga ataccagccc cccgacggag gcagcctggt ggaggtgtat 840
ctcctagaca ccagcataca gagtgaccac cgggaaatcg agggcagggt catggtcacc 900
gacttcgaga atgtgcccga ggaggacggg acccgcttcc acagacaggc cagcaagtgt 960
gacagtcatg gcacccacct ggcaggggtg gtcagcggcc gggatgccgg cgtggccaag 1020
ggtgccagca tgcgcagcct gcgcgtgctc aactgccaag ggaagggcac ggttagcggc 1080
accctcatag gcctggagtt tattcggaaa agccagctgg tccagcctgt ggggccactg 1140
gtggtgctgc tgcccctggc gggtgggtac agccgcgtcc tcaacgccgc ctgccagcgc 1200
ctggcgaggg ctggggtcgt gctggtcacc gctgccggca acttccggga cgatgcctgc 1260

ctctactccc cagcctcagc tcccgaggtc atcacagttg gggccaccaa tgcccaagac 1320
cagccggtga ccctggggac tttggggacc aactttggcc gctgtgtgga cctctttgcc 1380
ccaggggagg acatcattgg tgcctccagc gactgcagca cctgctttgt gtcacagagt 1440
gggacatcac aggctgctgc ccacgtggct ggcattgcag ccatgatgct gtctgccgag 1500
ccggagctca ccctggccga gttgaggcag agactgatcc acttctctgc caaagatgtc 1560
atcaatgagg cctggttccc tgaggaccag cgggtactga cccccaacct ggtggccgcc 1620
ctgcccccca gcacccatgg ggcaggttgg cagctgtttt gcaggactgt atggtcagca 1680
cactcggggc ctacacggat ggccacagcc gtcgcccgct gcgccccaga tgaggagctg 1740
ctgagctgct ccagtttctc caggagtggg aagcggcggg gcgagcgcat ggaggcccaa 1800
gggggcaagc tggtctgccg ggcccacaac gcttttgggg gtgagggtgt ctacgccatt 1860
gccaggtgct gcctgctacc ccaggccaac tgcagcgtcc acacagctcc accagctgag 1920
gccagcatgg ggacccgtgt ccactgccac caacagggcc acgtcctcac aggctgcagc 1980
tcccactggg aggtggagga ccttggcacc cacaagccgc ctgtgctgag gccacgaggt 2040
cagcccaacc agtgcgtggg ccacagggag gccagcatcc acgcttcctg ctgccatgcc 2100
ccaggtctgg aatgcaaagt caaggagcat ggaatcccgg cccctcagga gcaggtgacc 2160
gtggcctgcg aggagggctg gaccctgact ggctgcagtg ccctccctgg gacctcccac 2220
gtcctggggg cctacgccgt agacaacacg tgtgtagtca ggagccggga cgtcagcact 2280
acaggcagca ccagcgaagg ggccgtgaca gccgttgcca tctgctgccg gagccggcac 2340
ctggcgcagg cctcccagga gctccagtga cagccccatc ccaggatggg tgtctgggga 2400
gggtcaaggg ctggggctga gctttaaaat ggttccgact tgtccctctc tcagccctcc 2460
atggcctggc acgaggggat ggggatgctt ccgcctttcc ggggctgctg gcctggccct 2520
tgagtggggc agcctccttg cctggaactc actcactctg ggtgcctcct ccccaggtgg 2580
aggtgccagg aagctccctc cctcactgtg gggcatttca ccattcaaac aggtcgagct 2640
gtgctcgggt gctgccagct gctcccaatg tgccgatgtc cgtgggcaga atgactttta 2700
ttgagctctt gttccgtgcc aggcattcaa tcctcaggtc tccaccaagg aggcaggatt 2760
cttcccatgg atagggaggg ggcggtaggg gctgcagggg acaaacatcg ttggggggtg 2820
agtgtgaaag gtgctgatgg ccctcatctc cagctaactg tggagaagcc cctgggggct 2880
ccctgattaa tggaggctta gctttctgga tggcatctag ccagaggctg gagacaggtg 2940
cgcccctggt ggtcacaggc tgtgccttgg tttcctgagc cacctttact ctgctctatg 3000
ccaggctgtg ctagcaacac ccaaaggtgg cctgcgggga gccatcacct aggactgact 3060
cggcagtgtg cagtggtgca tgcactgtct cagccaaccc gctccactac ccggcagggt 3120
acacattcgc acccctactt cacagaggaa gaaacctgga accagagggg gcgtgcctgc 3180
caagctcaca cagcaggaac tgagccagaa acgcagattg ggctggctct gaagccaagc 3240
ctcttcttac ttcacccggc tgggctcctc atttttacgg gtaacagtga ggctgggaag 3300
gggaacacag accaggaagc tcggtgagtg atggcagaac gatgcctgca ggcatggaac 3360
tttttccgtt atcacccagg cctgattcac tggcctggcg gagatgcttc taaggcatgg 3420
tcgggggaga gggccaacaa ctgtccctcc ttgagcacca gccccaccca agcaagcaga 3480
catttatctt ttgggtctgt cctctctgtt gcctttttac agccaacttt tctagacctg 3540
ttttgctttt gtaacttgaa gatatttatt ctgggttttg tagcattttt attaatatgg 3600
tgacttttta aaataaaaac aaacaaacgt tgtcct 3636

<210> 5
<211> 874
<212> DNA
<213> H. sapiens

<400> 5
ctgcagcgtc tggggtttcc gttgcagtcc tcggaaccag gacctcggcg tggcctagcg 60
agttatggcg acgaaggccg tgtgcgtgct gaagggcgac ggcccagtgc agggcatcat 120
caatttcgag cagaaggaaa gtaatggacc agtgaaggtg tggggaagca ttaaaggact 180
gactgaaggc ctgcatggat tccatgttca tgagtttgga gataatacag caggctgtac 240
cagtgcaggt cctcacttta atcctctatc cagaaaacac ggtgggccaa aggatgaaga 300
gaggcatgtt ggagacttgg gcaatgtgac tgctgacaaa gatggtgtgg ccgatgtgtc 360
tattgaagat tctgtgatct cactctcagg agaccattgc atcattggcc gcacactggt 420
ggtccatgaa aaagcagatg acttgggcaa aggtggaaat gaagaaagta caaagacagg 480
aaacgctgga agtcgtttgg cttgtggtgt aattgggatc gcccaataaa cattcccttg 540
gatgtagtct gaggcccctt aactcatctg ttatcctgct agctgtagaa atgtatcctg 600
ataaacatta aacactgtaa tcttaaaagt gtaattgtgt gactttttca gagttgcttt 660
aaagtacctg tagtgagaaa ctgatttatg atcacttgga agatttgtat agttttataa 720
aactcagtta aaatgtctgt ttcaatgacc tgtattttgc cagacttaaa tcacagatgg 780
gtattaaact tgtcagaatt tctttgtcat tcaagcctgt gaataaaaac cctgtatggc 840
acttattatg aggctattaa aagaatccaa attc 874

<210> 6
<211> 1631

<212> DNA
<213> H. sapiens

<400> 6
ggacttctag cccctgaact ttcagccgaa tacatctttt ccaaaggagt gaattcaggc 60
ccttgtatca ctggcagcag gacgtgacca tggagaagct gttgtgtttc ttggtcttga 120
ccagcctctc tcatgctttt ggccagacag acatgtcgag gaaggctttt gtgtttccca 180
aagagtcgga tacttcctat gtatccctca aagcaccgtt aacgaagcct ctcaaagcct 240
tcactgtgtg cctccacttc tacacggaac tgtcctcgac ccggggtaca gtattttctc 300
gtatgccacc aagagacaag acaatgagat tcttcatatt ttggtctaag gatataggat 360
acagttttac agtgggtggg tctgaaatat tattcgaggt tcctgaagtc acagtagctc 420
cagtacacat ttgtacaagc tgggagtccg cctcagggat cgtggagttc tgggtagatg 480
ggaagcccag ggtgaggaag agtctgaaga agggatacac tgtgggggca gaagcaagca 540
tcatcttggg gcaggagcag gattccttcg gtgggaactt tgaaggaagc cagtccctgg 600
tgggagacat tggaaatgtg aacatgtggg actttgtgct gtcaccagat gagattaaca 660
ccatctatct tggcgggccc ttcagtccta atgtcctgaa ctggcgggca ctgaagtatg 720
aagtgcaagg cgaagtgttc accaaacccc agctgtggcc ctgaggccca gctgtgggtc 780
ctgaaggtac ctcccggttt tttacaccgc atgggcccca cgtctctgtc tctggtacct 840
cccgcttttt tacactgcat ggttcccacg tctctgtctc tgggcctttg ttcccctata 900
tgcattgcag gcctgctcca ccctcctcag cgcctgagaa tggaggtaaa gtgtctggtc 960
tgggagctcg ttaactatgc tgggaaacgg tccaaaagaa tcagaatttg aggtgttttg 1020
ttttcatttt tatttcaagt tggacagatc ttggagataa tttcttacct cacatagatg 1080
agaaaactaa cacccagaaa ggagaaatga tgttataaaa aactcataag gcaagagctg 1140
agaaggaagc gctgatcttc tatttaattc cccacccatg acccccagaa agcaggagca 1200
ttgcccacat tcacagggct cttcagtatc agaatcagga cactggccag gtgtctggtt 1260
tgggtccaga gtgctcatca tcatgtcata gaactgctgg gcccaggtct cctgaaatgg 1320
gaagcccagc aataccacgc agtccctcca ctttctcaaa gcacactgga aaggccatta 1380
gaattgcccc agcagagcag atctgctttt tttccagagc aaaatgaagc actaggtata 1440
aatatgttgt tactgccaag aacttaaatg actggttttt gtttgcttgc agtgctttct 1500
taattttatg gctcttctgg gaaactcctc cccttttcca cacgaacctt gtggggctgt 1560
gaattctttc ttcatccccg cattcccaat atacccaggc cacaagagtg gacgtgaaca 1620
caggtgccgt g 1631

<210> 7
<211> 1898
<212> DNA
<213> Mus musculus

<400> 7
   cggacgcgtg ggcgaggacc gcgaggagcg cagccctagc cccggcgact gagcacacct 60
   gaggagaggt gcacacactc tgaggaccta ggtgtgcaac ctctgccaga tgtggggcgt 120
   ggctacccag aggcatgccc ctcacccagc tccactgtcc ccacctgctg ctgctgctgt 180
   tggtgctgtc atgtctgcca gaggcaccct ctgcccaggt aatggacttt ttgtttgaga 240
   agtggaagct ctatagtgac caatgccacc acaacctaag cctgctgccc ccacctactg 300
   agctggtctg taacagaacc ttcgacaagt actcctgctg gcctgacacc cctcccaaca 360
   ccactgccaa catttcctgc ccctggtacc taccttggta ccacaaagtg cagcaccgcc 420
   tagtgttcaa gaggtgtggg cccgatgggc agtgggttcg agggccacgg gggcagccgt 480
   ggcgcaacgc ctcccaatgt cagttggatg atgaagagat cgaggtccag aagggggtgg 540
   ccaagatgta tagcagccag caggtgatgt acaccgtggg ctacagtctg tccctggggg 600
   ccttgctcct tgcgctggtc atcctgctgg gcctcaggaa gctgcactgc acccgaaact 660
   acatccatgg gaacctgttt gcgtcctttg tgctcaaggc tggctctgtg ttggtcatcg 720
   attggctgct gaagacacgg tacagccaga agattggcga tgacctcagt gtgagcgtct 780
   ggctcagtga cggggcgatg gccggctgca gagtggccac agtgatcatg cagtacggca 840
   tcatagccaa ctattgctgg ttgctggtag agggcgtgta cctgtacagc ctgctgagcc 900
   ttgccacctt ctctgagagg agcttctttt ccctctacct gggcattggc tggggtgcgc 960
   ccctgctgtt tgtcatcccc tgggtggtgg tcaagtgtct gtttgagaat gttcagtgct 1020
   ggaccagcaa tgacaacatg ggattctggt ggatcctgcg tattcctgtc ttcctggcct 1080
   tactgatcaa ttttttcatc tttgtccaca tcattcacct tcttgtggcc aagctgcgtg 1140
   cccatcagat gcactatgct gactataagt tccggctggc caggtccacg ctgaccctca 1200
   tccctctgct gggggtccac gaggtggtct ttgcctttgt gactgacgag catgcccaag 1260
   gcaccctgcg ctccaccaag ctcttttttg acctgttcct cagctccttc cagggtctgc 1320
   tggtggctgt tctctactgt ttcctcaaca aggaggtgca ggcagagctg atgcggcgtt 1380
   ggaggcaatg gcaagaaggc aaagctcttc aggaggaaag gttggccagc agccatggca 1440
   gccacatggc cccagcaggg ccttgtcatg gtgatccctg tgagaaactt cagcttatga 1500
   gtgcaggcag cagcagtggg actggctgtg tgccctctat ggagacctcg ctggccagta 1560
   gtctcccaag gttggctgac agccccacct gaatctccac tggagcctag ccaggctgcg 1620
   ttcagaaagg gcctcagagg acaacccaga gccagatgcc cggccaaggc tgaagagaca 1680
   aagcagcaag acagcagctt gtactgtgca cactccccta acctgtccta gcctggcaca 1740
   ggccacagtg acagagtagg ggttggatat gatggagaag ccatgttatc tatgaactct 1800
   gagtgttccc atgtgtgttg acatggtccc tgtacccaga tatgtccttc agtaaaaagc 1860
   tcgagtggga aaaaaaaaaa aaaaaaaaaa aaaaaaaa 1898

<210> 8
<211> 106000
<212> DNA
<213> H. sapiens

<400> 8
aagcttatgg tttatgggtg ttacattcaa gacatttgta ggacacattc taaaatgcca 60
tccaatttca ggctctttcc agcagaaact gtggaatatt tttccgttca ttcagcattt 120
acttagtgcc tgctctgcca ggaattgaag agaaagccca aagacaggca gaccttacct 180
gagaggtagt gaactgacca ggatgactgt gggcagtaga cttgtttccc aaactagcct 240
caccatttct gtatttgcat atacgaggaa aggattagat atagggattc atgtcagcat 300
acaccccagg gacatttgtt tttagtgaaa ggtgccagtc ttcatccctg tacccagtac 360
acaaaccacg aagaagtatg ctcccgtcat tgtcaaagaa tcatagaatt ccaaatggag 420
ctagttttga tatccagatc tcacttcata tgaggaaact aggtccagta ttgtgagtaa 480
gaattaggac tcttcagatt ccctgggtat gaatctgact aacaactgtg tgaacttgac 540
caaattcata accctgtaaa ctctgtttcc tcacttttaa aatgggcaca acaaagtgat 600
gcatgtaaac tgcatagcac agtgtctggc acttaaaaag cactcctgaa gttattttta 660
gtgatgtgtt ttaagattag acaactcctt aatgccaaag gtttttactt gagaactctg 720
tctgtgtgcc atactacacg ctgttcataa gataagcctt tttcattaat tgatctcaaa 780
ctggcttcat tatgatctta actttatttc agttttattt ttaaaattta tttttaattt 840
ttatgggtat atagtaggca tatatattta tggggtacag gtcatgtttt aatgcaagca 900
tgcaattgtg ggggtgatat ataattgact ggggtgagat atctcattgt agttttgatt 960
tgcatttctc tgatgattaa ggatgttgaa catttcttca tacacctgtt ggccatttgt 1020
atgtcttttg agaaatgtct attcagatct tttgtccatt ttttaagttg gattgtttga 1080
ttttttcctg ttgtctgaac tctttatata ttctagttat taatcccttc tcagatgggt 1140
agcttgcaaa tattttcttc cattttgtgg gttgcttctt tgttgtttcc gttgctgtgc 1200
agaagttttt tagcttgatg tgatcccatt tgtccatttt tgcattggtt gcctgtgcat 1260
ttgaggtatt actaaagaaa tctttgccca taccagtgtc ctggagagct tcccaaatgt 1320
tttcttttag tatcctagtt tcaggtctta gatttagggc tttagtccat ttttatttga 1380
tttttatatg tggtgagaga taggggtcta gtttcattct gcctatggat atccagtttt 1440
cccagcacca tttattgaag agactgtcct ttccctagtg tatgttcttg gcacctttgc 1500
tgaaaatgag ttcactgtag gtgtatgaat ttgtttctgg gttctctagg tctgtgtatc 1560
tgtttttatg ctagaactat gttgtttggg ttattatagt tttgtagcat aatttgaagt 1620
cagataatgt aattcctcca gttttatttt ttttgttcag gatggctttg gctattccgg 1680
ggcttttgtg gttccatata aatcctatga tttttttttt ctatttctgt gaagaatgtc 1740
attgatattt attaataaag attgcattga atctgtagat tgctttgggt agtatggaca 1800
ttttaacaat attgattctt ccaatccatg agcatggact atctttcttt ttttgtgtgt 1860
cctcttcaat atttttcctc agtgttttat tgttttcatt gtagagctct ttcacttctt 1920
tcgttgagtt tattcctagg tgttttattt tatctgtagc tattgtaaat gagattactt 1980
tctgatttct tttttagatt gtcctctgtt ggcatctaga aatgccacag atttttgtat 2040
gttgattttg tatcctgtaa ctgtactgaa tttatctgtt ctaatatttt tttggtggag 2100
tctttaggct tttccaataa gatcatacag tctgcaaaca agaataattt gacttcttcc 2160
attccatttt ggattccctt tatatctttc tcttgtctga ttactctagg taggtcttcc 2220
agtacttcca gttgaataac agtgggcact cttgtcttgt tgtagatctt agaagaaagg 2280
ctttcagttt ttccccattc agtatgatac tagctgtcag tttgttgcag atggcataac 2340
tttcaaacta attgattata gttaggaagt ggatacttta acttgtggta ccattatcag 2400
atttatattt cggccataag cttgaagagg agctgaaaaa tgcatatgtg atgcatatgc 2460
ttcctatttg gctctcttct cccacccccc tgccctataa tccacacaag ttcctctctc 2520
agtcactcat caactacttg aacctctgag gaacttgggg ttaaggtaaa ttagaataaa 2580
actgtctgaa gaagagcaag cctttcatgt cttgagaaat tcttggggtt ttagaaataa 2640
cttcattgct ttttttctcc agttactttg gcttcttctt aaagagaata ctaacacttt 2700
gaacgtcata atactaaggt tctgcctctt caaataaaga ctttaaaaaa aaatggtttt 2760
tgtatgattc agtgtgaatt aaatcccaca gtgtaaagga ctttactttc ttaatgtaga 2820
ttttcaaata cacaattact gatgtttata agtagattta ttacaccaaa gcacctagca 2880
aattcttgaa tggatcaggt cttatttttc agtcttactt tgcaaattta agccaaataa 2940
ttaaggattt gttaaatatt tgtcttaata tcaagctttt gcatatcggg gccctctttt 3000
ataagcttta taagcaatct tttgttttct ctgcttgctc aaagtagcta tgtttgttgt 3060
atctgttagt atttgctcta taacaaacat actgggtgcc ttcccactta gatttggcaa 3120

ttatcactcc tgtaaatgag atattacata agataggaaa aagaacagta tctttccaag 3180
aagaatagta tccttccata ttaacagttt agagctgact gcttttaaaa tttagtggct 3240
ttaaaataac aaccatttat tattcttcat gagtctacaa atgaggtggg cagttctgct 3300
gatctggcca agctgaactt atctcagctg ggcacattca gcgtatctgc tgtcagttgg 3360
ctggttggct gtagcaatga atggtgaaag taggctgccc ttaacttttt cacacagtag 3420
cattagagtt acaaaagaac cagcagaacc atgcaaaact ctttaagacc taggcttgga 3480
acaactatat ttctaccaca ttctattggt caaagcaaat cacggggcta gtctagattc 3540
aagtgggtgg aggagctgca attacactgc aaaggagtgt gactgtaggg agaggtgttt 3600
ttttattttt atttttttgc gatttgtcac agtagttgta ggaatcaggt gtatttaaaa 3660
ttctgatcct tctgtgatat ccgaattgtt catgaacctt gcctctggtg gaaaggcaga 3720
atcattgtga cagaaggata aaatcttgga atttagagac taacaaaggt tcagattcca 3780
gctccatcac ttatttctgc aatcctgcag aagttaatct tcctgatagg cattcagtaa 3840
tgattgattc acctgaacct cagattcttt atgtatttta aagaaagggc taggtaaatg 3900
caaagcactt atgtaactgc ttttattatt gcaaacctgg ctcccacact ccattcaagg 3960
tgtaagactc agtgtcttcc ttgaattaaa aaggaagaga aagtgtgtta gggaaaggaa 4020
gagaaatatt tgactaattg tggccccaat aaagtgacca ctcactgggg gtatttccct 4080
gtaagaaaag aatggttgag gctcagagtt aagagataca aatccaaaag tctccttggg 4140
gtaggattcc ctgtgattca tgggttgaga ggtgtaacat tagacacagt cccagtctag 4200
attttttttt taaagaattg tggtccatcc tatacacact gggtgcctta atactatatg 4260
tggcaattat cactcctata aatcaggttt tacataagat aggaaaaaga acagtatcat 4320
tccacattaa caattgaaag atgactgctt ttaaaaaatt aaaagggcca tatagaaata 4380
aaatcacata aatttcttgt gttaaacata gttgtcatat tggatgagga ctaaacacct 4440
aaattcatcc aactagtagt aatagaaaag atgaaacaca cacacagtaa aactagatta 4500
atttaattta tacaaagggc cagatatctc agaattcaga cagtcagaga tgttgactag 4560
agttaatgcc tcttttagga gaggtaccag gtaagtgttc tcaaagaact ggaaactgag 4620
accaccacct ctggcattat ctgtttgtga acacaagcaa gtctgaattt ttccgcacca 4680
tagctacctt tcatgtaagc ttcttttctt agaagaaaag aaggtaacat ttgggtgtaa 4740
ttttttatta agggtgaaat ttagtgtaga gagtaaaggc atttggcata gaagccctta 4800
gttttttttg tttttaagtt gaactgccag cctttatgga ttgcagtctt cgctgttttg 4860
attgacattt cccaattcat tttgtattat ttattttttt aagagacagg gtctcactct 4920
gttacccagg ctggagtgca atggggcaaa cttggatcac tgcagccttg aactcctggg 4980
ctcaagcaat cctcccacct cagcctccca agtagcttgg actataggtg tgcaccacca 5040
tccttggcta attttttaaa tcttttgtag agacagggta gtgctctgtt gcccaggctg 5100
gtctcacatt cctggcctca gttgatgctc tggtctcagc cttccaaaat gctgggatta 5160
caagtgtgag ccactgcacc tggcccccaa tttcatcctt tacaaagact actttcaacc 5220
ataaatcaac ggaaacttca gctccctcag acatatttgg gatccaagga tattttccca 5280
aatgattaat gctaatttca tatcaataca tttttgcaaa acctacaaaa atggactagt 5340
aaagaaagac tcttaatttg ggaaagacag ttacttggag agaagagaaa cttaagaggc 5400
aggtcgagtt cagtgttcag aaatgagagg atcataaaga gatagccata aaaatgtttc 5460
tccctatatt gcctgctgat agggtgtatc agtgaaggtc ttactaagga ccttgtacct 5520
tttcagcgct gcactgcgtg ctcataggga ggaaagataa atcatgtgtt ttttctgacc 5580
tcaaaggagc ctgtatctgg ctagagagac atgatgcaga cacatgaaat aattaagaaa 5640
caattaactg tagcaggtgc tgaagaatat accaggaggt cagagaatgg tagagctagt 5700
gtgggcgaag gtatagccca gagcatcatc agatgattct tccttatgca aattcacatc 5760
tcctctgggt caagtatcat cctggcatgc agcagctcca taggtaatgc cctaaggcta 5820
gcctgaggca agttgcaaaa gccatcatat tgagtcatgg cctttttttg tgtgggggga 5880
ggggaatggc atccccttcc tgtctgccaa atcaaggaat acagtgccct cctaaacctg 5940
ctttgtttta gtggattgtt aaaaagaagt gaatgaattt atgcttcatt agggaaaggt 6000
tacagtggaa tactgaggag taaggggtat ttctatttaa caaatgacat aacttgaagg 6060
aatgaaatca taaggatgga atttcaggca ttaataaaaa gctgatgaga gatactttga 6120
gacaaaagag ccttcccagt gtaaccgaga tcacagcacc tacttcacat acacaggaaa 6180
ccagtcctat ctgtctctcc catagagcag tagctgcctt gtttttcctc cctcctccat 6240
cattcattct aaatctccag tcctccaccg caccttatcc aaaccctgat acccttaagt 6300
cacagatggt gaatcagtca aaagtagtat taaaaactag tggtacacag ctacacctgg 6360
aatgcagtaa gaaaaatacg gatttctgta catcatcttc cctccctgct cttaccccca 6420
tttaagagtt acagggtcag aacccaagag tgtgagtttt tgaaagtccc taaaaatttt 6480
ggatgatcac ctacatttag aaccactgca ctaagaagga caacaaatat gccaataaat 6540
tctgttgcca aggaggtgat tatgcaagct ggaaccctga taacatgagg agaatcccac 6600
aatagccaaa tagtccatgt actagttaca ttataataaa gccaaaagca gcaggcctac 6660
ctgactttct cctgaggtct atcatgagct tagagagaag gaacgtggac atacagaggt 6720
agctctagat ggagaagggc actaggtgtc atggaaagaa tcatgtgcaa gaagtaaaga 6780
ggtgctctga atgtcctagc cctgcttagg tgtctgtgtc ctcacatgag aatttatcca 6840
cagttctttc ccgctgtaac aatctttggt tccaactgca tttgtgagac agcaaaaagc 6900
tatggtccag tctccttcca ttgtatcatc tcatcaatgt atttctccca ctacccttgt 6960
gtgaaataca aacttttttg gcttattgtg attatgcaag gtgtatgcca actttttttt 7020

ttctccacat ctttcagctt tctgatgggt aaaaattttc cttattttgc tttagaaaaa 7080
ttctcattgg catagatcta atttcaggga gcctcccttg aaagctaaat aacattgaga 7140
attcatgaaa atataatgta gagcattatg cctgttagca tattagttta aatagaagtg 7200
gttcatgaaa atttttgaaa tgccagaccc tgtcctgtgt tttgtattct cccaaatact 7260
catccagata ctgttcagaa tgtaacatga ttattttgaa ataaagattt tcccctagtt 7320
tttaaaaaag ttactttata cattaaccct tatgttcctc tttgatcaat ttttccagta 7380
gtgtaaacag tcttcaggga agtagatttc ttacagaaat tgtcaagtgg ctctctgctg 7440
ttagcatggt tactaatctt ttggttactt ttcatatttt ttatactttc tggaagtgga 7500
caacttactt gtaaataaaa gtgcataatt tgtattaaaa atttttagta acaatctaat 7560
ttgtaaaata gatgtgagca gcatgaatgt gtgtgatatg cgtacatacg aattatgtct 7620
cttaaaaatg tatcacagac atctttccgt gtccaaacaa atctacctca ttctttctaa 7680
tagccatatg ggtataccat aatatattta actaggcccc tattaaaaga attttgactc 7740
ttttgtagct actatagtgt tgcagtgtgt atctgtgtat gtatctttgt gtgtgtatct 7800
ttgtacgagt gtacatatat tttccccttg gctatttcag attttttttt aggtttaaat 7860
cttaggaaag gttttgaaat tgtcttaagt attttcagaa gcattaaatc atggtttttt 7920
tacatttttc ttttagaagt tttatgtcat ctctatgagt agctttcagt aatttgttct 7980
gcataaaatt cccgaaaact tccatttaaa aataggtggc atgactagac tttctcagcc 8040
gaaagagtga ggtcccagga aggattttgg agaagctgtg ttcaaatata gctgctgacc 8100
tgatgtctgc ctagagtctg gcaaggtgat gtgttgaatc tagtgtctgc ctgcatgcca 8160
gcatcccttt actgatgaga tttgtggttt tcatcacttc atggtaatca tcccaagtta 8220
taagatggag tctctagaaa atcagtagag tatgaaggcc caagtaaaat acatgtgagt 8280
gcatgtatgt gtgcatacaa attacttctc ttaaaaacgt atcctgggca tttaaagaat 8340
gaggacctcc gaaggatttt gtggaagctg tgttcaagta cagctgctga gcgtatgtca 8400
gcctggagcc tggcaaggtg aagtgttgaa tctagtgtct tttgactcaa ctgttttttt 8460
tgactcactg tgctttgaag cccttgtcat ttgggctcat aaaatagatt tctgtatact 8520
gtctctcctc cctgccctcg cccccattta aaagtatagt ggcagaaccc aagaatcaga 8580
gttactaaaa actctctaga aaatttggat gatcacccac ctgatcatgt cttttttact 8640
cactatgttt tttttttttt tgagacagag tctcgctctg tcgcccaggc tggagtgcag 8700
tggcatgatc ttggctcact gcaagctccg cctccctggt tcacgccatt ctcctgcctc 8760
agcctcccat gtagctggga ctacaggccc tgccaccgcg cccggctaat tttttgtatt 8820
tttagtagag tcggggtttc actgtgttag ccaggatggt cccgatctcc tgacctcgtg 8880
atccacccgc ctcggcctcc caaagtgctg ggattacagg cgtgagccac cacacccggc 8940
cctttactca ctatgttttt aagcccttgt tttcatttgc tccactgtaa aacattcccc 9000
aagccaatct ggagctgagg caaattttta acaatttaaa atctggggaa tataaatatt 9060
ggataatgat catcctgaaa aaacaatgaa ggtagtagca taatacttta tatatcaata 9120
aaatggcaaa ataagacagt tgttgaagga cagaaagagt aactgaagtt aggagcttat 9180
cttaacacat tttttgtgtc ataccatagg catcatattt tttaaatttt ttttatttca 9240
tacacatagg aaaatatatg tgtgtaagaa ataataaaca cctctttgta cctaccaccc 9300
aacttaagga acagctcatt gctattccct ttggtgctcg ctggatgccc tttcccagtc 9360
acatccccct cccttcccat ctgcaggact atactagtaa attttgtatt ttttgcatta 9420
ttttgctttg ttttatgatt ttactaccta tctacatatc cctaaataat acattattta 9480
gtttcatatg ttttaacttt atgttgtgga atcacattaa atgtagtctt ttttttttat 9540
attatacttt aagttctagg gtacatgtgc acaacgtgca ggtttgttac gtaggtatac 9600
atgcgccatg ttggtttgct gcacccatca actcgtcatt tacactgggt atttctccta 9660
atgctatccc tcccctagcc ccccaccccc cgataaatgt agtctttata acttgttttt 9720
ttaactcaac attgtttgta agattcatcc atgtaagctg aagctttttt atagagatct 9780
ttgttaagcc ttttaatgaa tacagtacat acatttctct gttcccctgt tagtggacac 9840
ttggattgtt tccagagttt tgctgttttg aacaacgctg ctgtgaaaat gtctcctgaa 9900
acacatttat aagagttttt ttttccccaa gggaattata cctagaaatt gaataactag 9960
atcacaaggc atacacatct acaacttctg ctaggtaatg ccaaattgtt tccaaggagc 10020
gttagaagtg ttctcatcaa cttttactag tgctagtctt ttacatttgt ggcagtatgg 10080
tgggtgtgaa atatttatgt ttagtttttc ttggtgccat ttaataattt ttataaaaaa 10140
tatttagaag tcaaggcagt tttttgtttt tgtttttatt ttttgcttgt tttgttttaa 10200
tgcagacatt gagattacga cttggaataa acattggttg caaagttcct aaaaggaaaa 10260
ctttttttgg tattctggag cttttctggt actgaataaa ataagtatgt taaattatgc 10320
atgtgtagtt tagaagtcag agcaataatt gtgattgttg aacagaatgg cagtaaaaag 10380
tttctaaacg attgtactgt acaagggaca cttgttgtgg gtcagtttta gcctccccaa 10440
cttttatgtt aaaagttgca acaaggttta agggcttatg tttgataggc cagatggtga 10500
ccagctgtga taaaacacag ggaacccttg caaaggattt caaaatttat gcagtagtcc 10560
gccttatctg cagttttgct ttccaaggtt tcagttaccc gcagtcaact gtgttctgaa 10620
aatattaagt gaaaaattac agaaataaag aatcgaagag ttttaaattt tatgcttccc 10680
acccatccca cctgggatgt gaatcattcc tttgttcagc atctccatgc tgtaggtgct 10740
gcctgcccct tagtcacttg gtagccatcc aggttatcag attgactctt ctagtattac 10800
aacacttggc ttcaagtaat ccttatttta cttcatagtg gccccaaagt gcaggagtgg 10860
tgatcctggc aattcagata tgtcaaagag aagctgtaaa ttgcttccct taagtgaaag 10920

atgaaaattc tagacttata tataaagaaa agaaatcata tgctgagact gctaagatct 10980
atgataagaa tgaatctttt atacatgaaa ttgtgaagaa tgaaaaagaa atgcgtgctg 11040
gttttgctgt catatctcag actgcaaaag tttgcagcca atgtgtatga taagtgctta 11100
gttaaaagga aaaaggcatt taaggtaagt atatatagtg tttggtacta cctgtgattt 11160
caggcatcca ttgggggtct cctgagtata aggggagact actcttttag tgttaaatga 11220
acactaagga acagagatgg ggaagaggtt ggagaagatt agttcagcag tttgagtata 11280
ggtaaacagt tgtttgagaa agaagaaaaa tgtgattagt attttacctt agcaatagtg 11340
gcatagataa tgataaatta tagtcacaca gaactcttag tatttacaga acgttcacat 11400
ttgtgatccc atttaacaat aactctgaaa gaaaggtatc atctaccact gctttattga 11460
taaagatata aaaggtaaga gagatgaaac atattggcca atgataccca tctggtaaga 11520
gacagggatg gggtgggacc ccaaggctct tctcgccaag cccacggttt ttttgcttta 11580
tacttttttg cctcctgatc accatggctg cagtttctac tgtggacaat gtctgtcagc 11640
aagcattgat cccctgcctt cagcactctt acgtcttagc aaggactgga aagaaaaagc 11700
caggagttta cagtctgctg gagcaacaga aaagaatgat atgaaatatg aagagaccaa 11760
aatgatttat aataaggtgc tagactatgt agtaaaaatc tgctttagct gtaagtcaaa 11820
agcaagagca gtcttttcag aatggaatag aaatgttgga attaaaggaa ttttcaaagt 11880
tgtgaatttt tttcaagata aacatgtttt attttggtaa ttatggtatt actaatttga 11940
taaccttcag ggagccacct aatattatag aagatgtaca tataatgaca aaagcaaaca 12000
ttttattttt aaggaccaca atctaatcta aaacaaaatt tccccctttt ctggtctttg 12060
gttaattaag gacttattta aatatcaaag aaagacacat agaaaacatt tagtatattt 12120
ctatactttt attaatgtcc tccatacctt acacagatac ttgacttggc tatggtctag 12180
ataatccatg aaaatttaaa ggacagattt taacaacttt atgctaaatt gatagatctc 12240
taggatcaga ttgccatcac tctcagatgc gaagcttcca accacttata ggttcctgat 12300
atcttgcttt tatacagacc taatttctct tcctttaaac tttcttttcc tcagttgcta 12360
tttgattgaa atattgagtc attaaaaatt tccaagtggg aatttttgtg tttcttcatc 12420
tatcatgaag ctgctcaaat aagtaggtgt ttgaatagga gtagaaacag taataggctg 12480
aagccagacc aatacagctt cagctaaatg ccgaccttgc taaagtctgg gaggaccggt 12540
gtggtattct acaatgtaca agtctgtagc cggtgccctt aatatgttgg cttcatgtct 12600
catgactctc ttctgtaaat atgcagttta aaaaatacaa gttattctgc tgtagaagat 12660
acatttgcaa aattgatgta tcccctctaa gtaaagttgg ctaaacaata aggacatatt 12720
tataattaat gaatttgaga agaatgctga cgatatgcat tattctttga agttaacatt 12780
tttcaggtcc taaataaaca aaaagtaggt tacttctgtc tggagtgtat gcaaggggta 12840
ccatcttgtc cttggttcct ggctgctatt ccaaggtgct ataaagtcag ctaaagagag 12900
caatcataat acattgatag catccctcaa tgtgtttctg agctacttga gaatcttatt 12960
tttgaatagg tagcaggaaa ccatctttgc agggcagcat gggcaaaggg attggaggga 13020
ctattattat aaagatccac tgaactgctt cagtatcata atatcttaaa ctaaaggact 13080
ggaaagagcc agattccaat ttaatctgct cttctatgaa ttcttagctg ggttcattta 13140
aaaagaaaaa acttgaagat tgcaagattt tgaagacatc ttaaaatagg tgaactccaa 13200
ggtgcacttt aaacttgaga ctgataactg aatactcctt caccttttga tctgatattg 13260
tcaaaatgaa tgaggactta gtgctctagt aagtttggaa cagaatgata ttaatttatt 13320
ttctcatgat tgattctttt ttgcttttta atagattaaa cttcaccgta gaacagtttc 13380
tcaacctctg gactattgac atttttgatt ggataattct ttgctgtcag ggctgttctg 13440
tgtgttgcag gatagttagc aacatccctg acaatcacaa atgttacttt ctgtctctat 13500
ggatttgcct attctggaca tttcgtataa atagaatcat atatatgtgg cttcttgtac 13560
ctggcttatt tcacttaaca tgttttcaag gttcatccat attgtagcat gtaacagcac 13620
ttcattttct ttttatggct gagtaatatt ctgttatgtg gatatactac catattttgt 13680
ctatccactc cttagctgat ggtcttttag gttgtgtcca ttctttggct attataaata 13740
atgctgttaa gaacattcat atacaagttt ctgtgtagac atatatcttt atttctcttg 13800
tgtggatacc taggagtaga attactggat catatgataa ctctatgtgt taccttttga 13860
ggaactgcca aacatttttc tacagtggct gtatcatttt acactcccat cagcaatgta 13920
taagaattcc aatttctctg tccttgccta tatttattaa ctgtcttttc ttattagcca 13980
actgctgtgg ttcgaatgtt tgtcccctcc aaaactcatg ttggaacata atccccaatg 14040
tggcagtatt gagatgtgag gcctttaaga agtgcttggg tcatcagagg tctgccctca 14100
tgaataggct aatccattca tgagttaatg tactaatggg ttatcactgg attgggacta 14160
gtggctttat aagaagagga agagaactaa tctagtaagc tcagccttct cactatgtga 14220
ttgctgccct gtgtcacctt gggactctgc agagagtcct ccagcagcaa gaagttcttc 14280
atcagctgtg gccccttgac cttggacttc ccagcctcca gaaatgtaag aaatccattt 14340
ctttttttta ataaattaca cagtctcacg tattcagtta taccaacaga acacagacta 14400
agacaccatc ctattgggta tgggtatctc attgtgtttt ttatttgtgt ctcccaaatg 14460
actaacgatg ttgaacatct tttcatctgc tttttggaca tttgtgtatt ttctttgaag 14520
aaatgtcttt aacattcttt gcccatttta aaattaggtt gtctttttat tgttgagttg 14580
tcggtgtgtg tgtgtgtgtg tgtgtgtgtg tgtgtatcta gaatatatgt gtatgtatat 14640
atgcagatat attctaaaca ctagaccctt atgaaatata taatttgagg acaatttctc 14700
ccatttaaaa ggccatcttt tcacttcttg atagtgtcat ttgactcaca agtttttaat 14760
ttttatgaag tccaatttat tttttaattc tttgtttttg gcactgtatc tttaaaaagt 14820

tgcctgatct aaggtcaaac tgattttcac ctatgttttc atctaagaat tatagtttta 14880
gctcttacat ttaggccttt gatccatttt gaattaattt gtgtatatgg tgtgaagtag 14940
ggctctaact tattcttttg tgtaatgata cctagttgtc ccagcaccat ttgttgaaaa 15000
gattattctt tccccattga atggtcttga taccttgttg aaatcaactg accataaata 15060
tataggctta ttcctggact cacaattcta tgagtctgta tgtctaatct tatgccagta 15120
ccacactgtt ttgattatta catctttgta caaagttttg aaattgggaa atgtgagtct 15180
tccaactttg ttctttttta agattacttt gcctatattc cgtgttcgtt gcaaactcat 15240
atgaatttta aatcaactct ccatttctgg aagaaaaaaa gaggcaattg aagttcagat 15300
agggattgca ttgaacctgt agatcagttt ggggaatatt gccatcataa caattagtag 15360
gtcttccaac ccatgaatac aagacttctt tccatttctg tagatattta gtttctttca 15420
ttaatatttt gtagttttca atataaaagt cttgtacttc gattaaattt attcttgaat 15480
attttgggtt ttgatgcttt tatgaatttg ttttcttaat ttcactttaa gattgttcat 15540
tgctactgat tagtaatgca actgattttt gtgtgttgat ttttgtatcc tgcaacctag 15600
ctgaaatcat tgattagcat aatagagtat ttaatagatt taggatttct atatataaga 15660
tcatgtcatc tgcaattaga gataatttta cttcttccct ttcaatctgg acatttttta 15720
cttctttttc ttgcctagtt gccctagcta gaacctccag tgcagtgttg aatagcagtg 15780
gtgagaatga gcatctttgt gttggtcttc atcttgtggg gaaacctttc agtttaagtg 15840
tgttgttgtg gggttttcat agttgtcctt tatcagattg agaatgttcc tttctgttcc 15900
tagtttgttg agtgttttct ttttgattgt tttaatcagg aaagggcatt agattttgtc 15960
aaatgctttt tctgcagcta ttgagatttt tgtgtgtttt tctggtcttt tatggtttat 16020
cacattaatt gattttcata tgtcaaacaa accctgtgtt cttgggtttc atctcacttg 16080
gttatggttt ataatccttt ttatatactt gtagattcag tttgccagta ttttgttgag 16140
gatgcttgca tttatattta taagggatat tggtctgttg tagctgacca gtaagtatag 16200
taagctgtat agtttactaa gtgttccctc tgttttgggg gagactttga gaagaaggat 16260
tgttggtaat tgttctttaa acatttggta aaattcacta gtgaagccat ctggggtctt 16320
ctttggaagt tttttgatta ctaacttaat gtctttactt gtttgttata agtccattca 16380
gatttttttc tccttgagtc atttttgaca gttggttgag gaatttgttc atttcatgta 16440
gttatctaat tggttagtgt ataattattc atagtattcc tttataatct tatttttttg 16500
ctgtaaggtc agtcataatg ttcactcttt catttcggat tctggtaatt taagagtctt 16560
ctctcctttt ttttcttggt cagtctagct aaagtaaagt tttgtccgtt ttcaggggaa 16620
cagctttttt tttttttttg aggcagaatt tccatcttgt cacccagtct agagtgcagt 16680
ggtgcaatct cggctcattg cagcctccgc ttcccgggtt caagagattc tcctgcctca 16740
gcttgccaag tagctgggat tacaagcgcc caccaccacg cctggctaat tttttatatt 16800
tttagtagag acggggtttc accatgttgg gcaggctggt ctcgaactcc tgacctcagg 16860
tgatctgcct gccttggcct cccaaagtgc tgggattaca ggtgtgagct accgtgccca 16920
acccagcttt ggttattttt gttgacctac tctattgttt ttctcttctc tatttcactt 16980
atttctacac tggtctttat tattttcttc cttatgcttg ctttggactt agttcttctt 17040
tttctagtct cttaaggtgg ataattaagt tcctgatttg aattcttact tctttgtaag 17100
gtggtcatgt actgctatga atttccttct cagaaatgta tatgctttca ctgcatccct 17160
taagatttgg tatgttgtat ttttgttttc atttgtctca aggtatagtc ttctgatttc 17220
cattgtgatt tcttccccct ctaacccgtt tattatttag gaacttgttg atttccacat 17280
acctgtgaac tttccagatt tccttctttg ttaattctca gtgtcattcc attctggtcc 17340
gagaacatac tttgtatgat ttctatcttt taaaatttat ttggcttgtc ttatgaccta 17400
atacattgtc tatcctggag gatgtttcat gtacacttga gaagaatgtg tattctgctt 17460
ttgttgggta gagtgtttga caggtgtgtt ggtacatagt tctgttcaaa tctgtttcct 17520
tgcagatttc tatctagttg ttctgtctat tggaagtagg atattgaaat ctccaactaa 17580
tattgctgaa ttgtttattg ttttcttcag ttctgtcact ttttgcttta tatattttga 17640
aattctattg ttaggtacaa gtaagtttat gattattata tcttcttgat agattgattc 17700
ttttatcatt atacagtgcc ctataagaac aatttttatc ttaagtctat tggtctatat 17760
tagtatagcc acttcagctt tcttttgttt actgtttgca tggaatattt tcttctttta 17820
ctttctattt gtgttcttga gtctaaggtg aatctctgta gatagcaatt ggatctgcca 17880
atctttgctt tttatttggg gagtttaaac cattgacatt taatgtaatt attgatgagg 17940
aagattactt ctgatatttt gccatttgtt tcctttattt tgtgtctctt gttcttaaat 18000
tcttccatta ctaccttctt tcttttgtat tacatatttt ctagtgtaac gattttaatt 18060
tctttgtcat ttcttttgtt gtatgttttt agttattttc ttagtggttg ccacggagat 18120
tttattgtca ttttaacagc ctaggttggg cacagtggct catgcctgta atcccagcac 18180
tttgggagac tgaggcagga ggatagcttg agtccaggag ttcaagacca gcctgggcaa 18240
cttactgaga tactgtctct acaaaaaaat acaaaaatta gccaggcatg gtggtgtgtg 18300
cctgtagtcc cagatgcttg agaggctgag ttgggaggat agcttgagcc caggaggttg 18360
aggctgcagt gaactttgat cacaccactg cactccagcc tgggtaccag ggcaaaacta 18420
gcccaaagaa atgaaggaaa aaaaaaatct aatttagatt aatatcaact caacttcaac 18480
agtgtataaa aactttgcct ctgtatacct cttctgcttc cactctgtgc tgttattgtc 18540
atagattttc atctttctac actgtgtgtt tatcaatgta gatttaaaaa tattgcttag 18600
tagttgtctt tagaatccga tacggagaaa aggagatata aacaaaagat gcatttttac 18660
tgtcttgtat gtttacttat gtaattccct ttcctgatgt tgtatttcta aaggcaaagt 18720

agggttattg tgagtgtcct tttgtttcaa cctgaaagac tccttttagc atgtgttgga 18780
gatatgctaa tgatggactc tcacagtttt tgttatctgg gaatgtgtta atttatcctt 18840
catttttgaa ggatagtgtt ggcaggatac agaattcttg gttgacatgt aattctttca 18900
gcattatgaa tatgtcatcg tactgtcttc tgacctccat ggtttctgat aaggaatcaa 18960
ctgttaatct tattgaggat cacttgtttg taatgacttg cttgtcgtgc tgctttcaag 19020
attcattctt tgcctttagc ttttggtagt ttgattgtga tgcatttagg tgtgtacttt 19080
attagtctgt tctacttgga gtttgttgag ctttgtagat gtatttcatc agatgtgtca 19140
agttcttttg ccactatttt ttttttaaat aatctttttg cccctttccg ctccttctgt 19200
cactctgatt atttgtgtgt tgctttgttt ggtggtgtcc cagaagtctc tgagactctg 19260
tccagttttt tcctccccat tcttttttct ttcacttcct cagactggat gatctcaatt 19320
tgacctatct tcgagttcat ggattttctc ttctccaagt gacatctgtg agatgaattt 19380
ttttctagag aatttttcat ttcagttatt ctacttcaaa atttctcttt ggttcagttt 19440
tatcattgct atctttatat tattctcagt ttaatgagat actgttttat actttccttt 19500
agttctttag acatagttta tgtcactgaa tatatttaaa atagctgatt ttaagtcttt 19560
tttttttatt tttttggaga tggagtctcg ctctgtcacc caggctggag tgcagtggca 19620
cgatctcagc tcactgcaag ctccacctcc tgggttcacg caatgatttt aagtctttgt 19680
ctatgaagtc tagtatctgg gcttcctcag gcatagtttc tgttttcttt ctttcttttc 19740
ctgtgtactt cgtttctttg tataccttgt aattgttgtt gttaactgga cattttgaat 19800
attatagtgt aacaactctg gcagtcagac tgtctcccct ccccagtatt tgttgttggt 19860
gagtattgta gatgtttgtt tagtgacttt tcatggctaa ttctgtaaat tttatattct 19920
ttgaagattg tgggcaccct gaagtctctg tttgttagtt tagtggtcac ctaataatta 19980
acagagattt cattaaatgc ctagaagcaa aatatcttcc agtctttgcc catggcctct 20040
gtgtatgcat tagggcaggc cttgaactct tacccaggga gtttacaacc ctgccttagc 20100
ctttactacc agcttctgca gagcattaag gtcaacaggt ggtgagagtt tggagcctac 20160
tccatctttc ctgagcgtat acacagccct actcatgcat gtggccctct agatttccag 20220
gaatatgttg gaccctttca aagcccttac agactcccca gcttttcctc tcaatcttta 20280
gactagtgtg ttgttttctt caacagttat ctgtcaggca gcagcaaatt aagagattag 20340
cataaatgtt ttcaactcct ccacccgtca tgtgccccag ggaagcacta agccagttct 20400
aagttaggca aaataaagac aatccttttg aggtggtctt ccatggagtc accagacagg 20460
taaaccaaat aattaattac aagtctttgg ctggatacag tggctcacac ctgtaatccc 20520
ggcactttgg gaggctgagg caggtggatc acaaggtcag gagattgaga ccatcctggc 20580
taacacggtg aaaccctgtc tctactaaaa aatacgaaaa aataggtggc tgtggtggcg 20640
ggcgcctgta gtcccagcta ctcgggaggc tgaggcagga gaatggaatg aacccaggag 20700
gtggagcttg ccgtgagccg agatcacact actgcactcc agcctgggtg acagagcaag 20760
actccgtctc aacaaaaaaa aaaaaaaaca agtcttcatg aaagaggtcc attctgctgt 20820
ctttcatacc aggaatgtgg aatgtggact gttattttca tggctactgc taagctagga 20880
atcaagggat agatggggac tgggtaaaac accacagagt ttgctgttct taccaagaat 20940
aagctgggga agagggttgt ttttgttttt cagtaaaaat tccctgggct gcttcaagcc 21000
gttgattaat tttcaggttc cgaaaaagtt cagtttgaca gtttttgccc tttttatttg 21060
cttttatgga tatgtagaac ttgagttctt ttttccacca gttttgctga cattgtttta 21120
aaagcacttt ttgtaaaacc caaatgttgt ctctctcaag gctagccaat aattaaaaat 21180
actgttactc ccctttgatt ttggaaatga attcgtattg accaaaattc aatactagag 21240
gtctttcaag ctgttttacc atttatctaa actttagaat ctaatgattc ctgtacattg 21300
tctagcatac tggtggtcct caattgtcat aagttcaact ttggaacaaa tgaacttttt 21360
gtgtgcaagt ttccaattgt ttggaaatta cattgatgcc ccctccatca aactgttatt 21420
cgtgggacat ctaggaattt cttacagcag ctgacaaata tttcaagtca gtgcctggta 21480
gtactgtcca ccaggcaaca gcttcagtag tagagcgatc tttatctata aggcagtgtt 21540
tgagcaattg tttattagtg ttttcctaac tactcagaag aactatcagg ggttatagag 21600
gtagctcaga gagttgggtg caagtagaga aatccacccg gcttgcatta cacatcttat 21660
ttctagagaa gctttccttt gaagaaagag ttctaaggtt taaaaaatta ccttgaatgc 21720
cacttatatt gcattttaat tttattttag agaaatcaat ggaaagtaga aaaattaagg 21780
cactgatact agtgttaaga atgttggtta aagcttctgg caattaattt tttatttcct 21840
tttttaattt tattaaaatt taacaatttt cagtttatgc tgtaatccag accaaggttt 21900
caatctaatg aagttaatgc cagtgttgct gctacctatt ttgtctttag tcattcagcc 21960
atgcttccta cttatactga ataagctagc ttaatctaac aatcaaaaaa gaaagctgtt 22020
gcctaagtta agaaaaacag tttgaactgt tttcaaacta aatacccagt agactctcta 22080
gttgttgaca ggagaatgct taattcagaa ttgtcctgca gtagatcatt ttatctcatt 22140
cctgttcttc tataggatag cttatttgtt tgaaattgta tttaatatgt tgtgattttt 22200
gtgtgcttgt ttctattttt cactggatag actcaagata aaacctggta ccctgcagtg 22260
tagctatcag tttatagcag aggaaattta cattagaact tggctgtgta tttacatgta 22320
tctaacttgg aggtcactct gcttactgtt gatatatcag tcatattaga tgagtcccta 22380
atgagatacc agaaaccccg gaaacatcat taggtggaac agtgtcctta atgctttatt 22440
aagtgttata ggtaagacaa agcctagtac tatttgtggc atcaaggtta ggtgtttaaa 22500
gacctgtatt cttctattgt catgttgaaa ttgttccctt gatgtagcaa tagaaaattt 22560
tagattaggc ttaagttaat cagcaaacaa agataaaagt ctgatactat cctaaatatt 22620

ttgtgtttct aaataattta acagtgatcc aattagctac tcctgtagaa atgtaattga 22680
taaacttttc actctctttt aaattgccat cttgaatttt acctgttttt taaagctgtc 22740
tcaagtcctc tctaaaaaaa ggcagtcatt tataaattta gaaaagcttg atagcacaga 22800
aagtcacaga aaaatgtaaa catagtttaa aactgaattg tatacaagcc actagaagta 22860
cttttattaa gtttacaaat attagtagag tggaactcat gcatttaata tgtttgaaac 22920
ttttgatcaa atactgtgct atgaaaaaca ttttagataa ttattcttta atcatgtgtg 22980
tgtaaaatgt ggcttttttt gacaaccaag tagcttttct gtgtgccaaa ctgtgacttt 23040
aaaattttaa agtactcaac agagtaaaca aaccacaaat accacttaaa ctgtacacat 23100
ttgcacatgc atttcctata aatagtacat gggtttcaag tcttcacttt tgaaattcag 23160
aaatgggttt tttctccttc cagtagaaat aaaaacttga tttattttat ttatttattt 23220
attttatttt tgagacggag tctcgttctg tggcccaggc tatggtgcag gagggtgatc 23280
tcagctcact gcaacctctg cctcctgggt tcaagtgatt ctcctgcctc agcctgccga 23340
gtagctggga ttacaggtgc ctgccaccat gcccagctaa tttttgtatt tttagtagag 23400
atggggtttc tccatgttgg gcaggctggt ctcgaactcc tggcctcagg tgatctgtct 23460
gtctcagcct tccaaagtgc tggggattac aggtgtgagc caccgcatcc agctaaaaac 23520
ttgattttta aaaatccaaa tcgaagacag aattgtgtat tttagtacat ttattagcag 23580
ccttgacgct ataccatatg gctgtttatc atttaaacag cttgtaaaag caaacacttc 23640
aggattcatg agtggcagaa ggactgagta ctttgggaaa taagagagaa cttttgttga 23700
ggatggttga ggaagagtcc aagacaataa taggcagaat aagcaaaaat ctagagactc 23760
attgtaggca ctcaagtatg tatttgttag aatgaatggc tgaacttggt atattgagga 23820
acactgagaa agccatactg actggaagat agttcctaca agaaactggt gagacatatg 23880
ttacagtcta gattttggtg agccttgtta aagtttgggc tttattttta tacggggaga 23940
aagtttcaca ggggtttgga aatgaggctt ggagctgtta atggggacac agtgaggttt 24000
tagggtagtg gctttcaaac tgtttaaatc caaactttga tgataaccct gacataacta 24060
ttgtttataa cttccatttc agttgtattg gttttatcaa aacatcttca ttgatcttac 24120
tgattgcttc ctatgcagat taatattata aatttgaatg tacaaaggaa gctttagcag 24180
taaaatagca acttttatct gtcttacgta ttggaggttc tgcataagat ttaatttttt 24240
ttttttttga aatggagttt tgctcttgtt cacggggctg gagtgcaatg gtgtgatctc 24300
ggctcaccac aacctctgcc tcccgggttt aagtgattct cctggctcag cctcccaagt 24360
agctgggatt acaggcatgt gccaccatgc ccggctaatt ttgaatttta gtagagacgg 24420
ggtttctcca tgttggtcag gctggtctcg aactcctgac ctcaggtgat ccgcctgcct 24480
cagcctccca aagtgctggg attacaggcg tgagccaccg cgcccggcca agatttaatt 24540
ttttaaaaga aaatattttg ctaagggttt ggaaactctt gttttagcaa gaatggatta 24600
agactgatta aaactaaagg caaagaggag gctcttatgt ttggaattct ttgctaatat 24660
ttacacaata taattctctc cacaaatatt taatggtacc agatattaga tggttataat 24720
ggcaaaagtg ttcaaaggat gctatcatat tcatgattca tgatcaaaat gaacattata 24780
aggctatccc tcttcagaat taaatacgtt actcctgtgg aaaacttgct tttaatgtag 24840
aagttgtccc agagcctttc ttcctttctc atgtcctctt atgtccactg ctgagctaac 24900
atgggtctca ctgaatgatt aagaaaaaac atcttaggtg gggagttctg tatatagtaa 24960
atgtttaatt tattggggtg gtgaacggga agtgctgctg gcaagagagg atgggaagag 25020
aaatctaccc aaatccttac ccgctttaca gaacataaac ttcctattca gtagtacaca 25080
ataacttaac gatcaaggca tcttaacttt tctgttttca gatgaaagaa ctatcgtttg 25140
gcttgatcaa gtatttagta tttattcgtt cactcaagtg cttacgtttt tttgttatct 25200
cagggtttta cgttagttat taaccaaaag aactagtttt agttctggaa gtctaaaata 25260
tataagagaa ggtgaggagt aataagagaa gatgaaggga gactttcgga atggcctatg 25320
aacttctagt aactatacca ccttaaaata gacaaattac aatgcagtta tgaagatatg 25380
tatttttcag tgaagacaac taaaatgttt gcacagaatt ttctttttta ttgagtgtta 25440
gaaattctat tttggagata ctaccttgca caacataaaa agaaaaagtg agtgtggaat 25500
ctaggaatct acgtggctct aggaaatttt ttaagtgtgg aaactgaagg agagcaagag 25560
aaagggagca tggcattccc ctgtttgtag ttcatgaggt gggtttaaat tgccttttgc 25620
caatgcagct gcacactgag gattacagaa ttctttttaa atgtttgtag aattattttt 25680
cacttattag gtaaaacgtg tattttttga ttttctccaa tttcagcttt ctcatgttgc 25740
tatgctcaat tttgtatacc atatatagtt ttgttaaatt gacaaagtgg tgttttttgt 25800
tcttcttttt cccattggtt aaaatttaaa gagaaagtgg aagctagaaa tttatctaaa 25860
aaatgtaact ttccctgtaa ttattaaagt atcaaatcta aatttgaatt ttctttgtgc 25920
ataatctttt ttcaagctat ttaccatgtt gacaaacttg ctttcctgtg gcaaatacac 25980
tagcaatacg ttataaatat gtaactttca acctatttac agttgatgct tttttagccc 26040
tttggattta aaatacaagc actgaagagg tgaggaagta ccactgctgc ctcagcatta 26100
tttcgaaatt ctgtttataa actatacaat ttccaaggtc atgaatccag cacctttcca 26160
ggtactaact attgggacaa agatagaatt tgattttatt tatttaccta ttgactgaag 26220
tctaacttaa atcttgcacc tagtaagatc ttagaaataa cgtgtgtact ctgacctgta 26280
aactaatcct agtattctgt gtgtatattc tttctcattt gggctcttaa aaggaaaagt 26340
aacgtacatc tgatgatcat tagcactgag ctttttcagc aaaaagtata tgtttataaa 26400
gaagtatagg ataatttagt aatttaataa tgtgacaaca tttgcgtgtg tttttttttt 26460
tgagaaatac aaattgtgag aaacagaaaa gtaaaagaag cagcagcaga aatatcacta 26520

taggatcaaa agattgcagg aaccaaaact ccaaaattat tgggcataat gtactaaaaa 26580
cagggcagtg gaggaaaggg acagtccaga ctagctctga gggtccaaag aaagtattaa 26640
atattgttac tggagtgatt tgctctgcta tttgggcttg ggaattaagt gaaattgttg 26700
atatactaga cagatacttc ccacccattt ttctcttgat aatcagggtt cattttttct 26760
attttctatt tctctggatg ctccatttct taatattaat attaatatta agctctcagt 26820
ctttatgcta aaaattggtt atttaaaaca atttaaatca acttcagtct aattggctta 26880
agttcaaatc cattttaaga tcgatattgt gtcctttaaa aattttattt aaaagatatt 26940
taaactgatg agaggatact acccattcca ctgataaact attactgtaa gtttgtctat 27000
tgagggctag ttatttggtt taaaaatgct gagattatgg aaagtggatt ggaatatttt 27060
ggagcaatat taaaaacagt atctgtaaca atttaataaa cttataaatt cctctttctc 27120
tgttgatcta tcttgaaaag acactctatg tctctaggca ttccttctct gtggtgtgat 27180
tggtagacag ggagtaaaca acttactgta aatgggcacc atgccagttg gcttcaggca 27240
gcatcaagct tgtgactcac agtcagggtt aggaaaatgc cttttaactt gtttgtctct 27300
gcctctttta aacattaaag gcacaactgt actaattatt aagtatttca taaggtcttt 27360
tagggcttat aagatctttt aggaatggcc tggaagttat tagtactgtt tcattgaatc 27420
tgaatacctt taacatgata atgagaagtt tttaaagggt ggttttatag ttaaacggaa 27480
tttctcaaat tggcttgctc cttatgttga tttatttagg atcacatttg ggagtttctc 27540
tgccctactt tcaatgtatt taatttactg accatcacta tttgggggga aaatgttata 27600
tgatatttag aaaccaagag ttttggagtt tttcccccat tagatgtatt tatttattta 27660
tttattattt tttaaagaca gggtcttgct ctgtcaccca ggctggagca cagtggcatg 27720
atcctagctc actgtattct tgaactcctg ggctcagact gtcctcccac ctcagcccaa 27780
gtggctaagt atcaagtaag aatcacctgg caaattccaa ggctgtatac cagatttcct 27840
aaattagaat tttggggttg ggtatctgaa ttttagtaaa gccctccaaa tgtttctggt 27900
attgcttcta agaacaattg ataacataat agctgtggcc attatagggg tattctgtca 27960
tatttagata taggcatacc ttgttttatt gtacttccca aatattgcgt gtttattttg 28020
ttttgtttca cttacaaatt gaaggtttgt ggcaacccta tattaagcga gtctgtcagt 28080
gccatttttc caacagcttg tgctcatttt gtgtctctgt gtcacatttt ggtaattctc 28140
tcaatatatc aaactttttc atcatttttg tatctgttac gaccagtgat cagtgatctt 28200
tgattttttc tttttttttt tttttttgag acggactttt gctctgtcac ccaggctgga 28260
gtgcagtggt tcaatcttgg ctcacagcaa cctctgcctc ccaggttcaa gcaatcctcc 28320
tgcctcagcc tccccagtag ccgggcctac aggcgtgtgc caccacgcct ggctaatttt 28380
tgtattttta gtagagatgg ggattcccca tgttggccag gctggtctcg aactcctgac 28440
ctcaggtgat ccgctcacct tggcctccca aagtgctggg attaccgtgc cagcctgatg 28500
ttactatttt aattgttttc aggcaccata aacctcacct gtataaggca ccgtacttaa 28560
ttggtaaata ttgcgcatga tctgactgct cttccaactg gccattccct gtctgtctcc 28620
ctcttcctgg gactctcaaa tccctgagag acaataatat taaaattaag ctaattaata 28680
accctacagt ggcctctaag tgttgaagtg aaagagttgc atgtctctca ctttaaataa 28740
aaagctagaa gtggctaaac ttagtgagga aggcacatca aaagccaaga caggccaaaa 28800
gcaaggactc ttgtactaaa cagctaaatt gtgaatgcaa aggaaaagct cttgaaggaa 28860
ataactagtg ctactccagc aaacatgtga atgatcagaa agtgaaacag ccttcttgct 28920
gatacgaaga aagttttagt ggtctggaca gaagatcaaa ccattcacaa cattccttta 28980
agccaaagct taactctctt caattctatg aaggctgtga gaggtgagaa agctgcagaa 29040
gaaaaattgg aagctagcag aggtcggttg atgaggttta gggaaagaag ccagcgctgt 29100
aacataaaag tgtaaggtga agcagcaagt gctgatacag aaactgcagc aagttatgta 29160
gaagatctag ctaagattac taaataatag attttccatg tagatgaaaa agccttttgt 29220
tggaagaaga tgccatctag gactttcata gctagaaagg agtcaatgtc tggcttcaga 29280
ggacaggctg acattcttgt taggggctaa tgtagttggt gactttaagt tgaagccagg 29340
tctcatttac cactccaaaa atccgaagac ccttaagact tatgcttaat ctactctgct 29400
tgtactctag aaatgaaaca acaaagcctg gatgacagca catctgttta tagtatgctt 29460
cactgaatat tttaaggcca ctgtaaagac ctgttcaact gctcagaaat aaatgattac 29520
tttcaaaata ttgctgttca ttgacagtgc acctgggctc acccaagagc tctaatggaa 29580
ttgtacaaca agatggatgt tgttctcatg cctgccaaca catcatccat ttgtagccca 29640
tgaatcaggg agtgatttca agtttcaaat cagtacattt tgtaaggcta tagctgctat 29700
agacagtgat tgctctggtg gacctgggca aagtaaatca aaaaccttct gaaaaggatt 29760
ggccattcta gatgctatta agaatttgtg attcgcagga ggaggtcaaa ggatcaacat 29820
tagtagcagt ttgaaagaag ttgattccaa cagttataga tgaatttgag gggttcaaca 29880
cttcagttta ggaagtcact gcagatgtgg tagaaacagc aagagaacta gaattagaag 29940
tggagcccga aaatgtgacg gaattgctgc aatctcatga gaaaacgtga atggatgagg 30000
agttgcttct tatggacaaa tgagcaaata aatttttttc ttgagatgga atctactcct 30060
ggtgaagatt ctgtgaacct tgttgaaata acaacaaagg atttagaata ttacataaac 30120
ttaattggta aagcagcagc atggtttgag tggattcatt ccagttttga aagagtttct 30180
actgtgggta aaatgctatc aaacagcatc tcgtgctaca aagaaatctt ttatgaaaag 30240
aaaagtgaaa cttcattgtt gtctacttta agaaattgcc acagccaccc caccttcagc 30300
aaccacctct ctgatcagtc agcaggcatc aacactgaag caagaccctc cacaaggaaa 30360
aagattacaa ctcactgaaa gttcaaatga ttgttagcat ttttaagcaa tattttaaga 30420

ttaaggtaaa tacattttta aagacacaat gctattgcac acttaataga ctacagtata 30480
gtataaatat aacttttata tgtagtggga aaccaaaaaa ttcgtctgac ttgctttgtt 30540
gcaatattca ctttattgtg gtctagaacc gaacctgaaa tatctcagag gtatgcctgt 30600
attaatatta ttttgcaagt aaaaaaccca gcatataaaa aaaacgtaga atatgttgag 30660
agttcagtaa tatggatgaa aatgtttttc tctaactgaa gaacatgata aattataatt 30720
agggaaggat ataaaccaag aaaatatgtc tgagatagcc aattcttgca gttcataata 30780
tgaaaactca ttataccaat ctcagtaaga atacttttaa tagctgttat ttctttggga 30840
tatagaattt ataaagtaca cagtaatctt cttatgatca atcctaggat cactttacaa 30900
ccacttaccc catattacaa tgtagtacca agacaagcag accaaattat agaaggacaa 30960
agtttttgct aagcatattt tgtcatcagc ataccgcatt gtgtgtgcat gcatgtgtgt 31020
gtttgtgcat gtgtgtgatt gtataaaata ttagaaagcc accccagaaa agttaaatga 31080
ctaggaatgt tgtgaaggga ttaagctacc cctaaaatta tataacaaaa ctctcttcat 31140
ctattattag gtcatcttta gaacatcttc tcttaaattt gttataggtc tctctcatct 31200
gtttggatta aaattggtct gaaagcctaa aatggctttt tacctatata attatttccc 31260
aactagcttg tagtataggt gcaaagctat cacacttgct aggttagtga agtatgtaaa 31320
aactaccatc tttcaattag gaaccatcgg atagcttcta caggattgct ggggagaacc 31380
tttataaaga aagttatatc tttataaatt ttttgtcatt ttacttagct gagaatataa 31440
aataagttag ctaataatag agtagaaatg ttttctgtaa cagattaata ttgatcaaat 31500
gtgttattaa atgctaaaac accatttttt ttctctgtaa gccatgtgtt tcatgccaca 31560
acacaaaagg gacaattgtc tgtgttttat gacagttctg ttctgtcaga tgctgtttgt 31620
tcattttggt gaataaatga agagagccct ggacacatct ttttttcctc aacaaaagag 31680
gaaaattatt cttgtctgta tgtctataat cctgactctt tgaatggctt taattttttt 31740
aaagtcagca tttttttata aagataggtg tttggaatgt gggcgatatg gctggacagt 31800
tagattggga ccaaataatg gaaggctttg aacatcatgc taagaggttt gggttttact 31860
ctgaaggcag tagagaacca ttatgttttt aagccaggat tgacttgttc taagctgtac 31920
cttagaaata ttactctggc agttgtacat aggatgagct gtatgttgct ttgttttgtt 31980
tggggagaca gttctcgaag agagactaca tacgaaggca gttatatgag tcattactaa 32040
aggtctggca agaagtagta aaagcattaa ctggagtggt agcagtaggg aaggaaataa 32100
aaggatagat gtgggagtca tttggaaagt atgaggcaat tcattgacct tacagaatca 32160
ctggttttct gcttccactc cattcacatt gacctttcca aggttatcag tgacctgctt 32220
gtccttaaat tcagtgggca ctttccagta acctactgtt ggcaccagcc ctgtgctaga 32280
caccaggatc ctgtttgtaa aggcatctgc cagtggtttc tgtgacacaa ttctgtttct 32340
agttttcctc cttctacttc tctagcctct tggcaagttc ttctttcaga gtttctcaga 32400
gctttgtgct aggccctctt ctcattttct ccttctctaa gtgatcccat ccttttctgt 32460
tgcttcagtt accatttgtc cttatgcaaa ggacagccat atctactgta tctccagctc 32520
agatgtatct ctttgcctcc tgacccatat ttccaactat ctaactgggt atcttttctt 32580
ggatgagtta taggtctctc aaacacaaca tgtccagaat aattcattga cttattctaa 32640
ggcctgcttc ctctttctcc tgtagtccct atctcaggaa atatatggtg ctatcaaccc 32700
caaagcagaa atctggacat aatccctaac tacccttttc ccctctctgt gcacataatt 32760
tcagtcatta ggcctcatag attggactaa ataaatacct cgcaaaccct tctacttata 32820
ttcttaactg ctcctacctt aagccaggct accataattt tgtagctgga tgactgcatc 32880
atcatcttga ctggctccct tgtcatcttc aatctatatt ctatactgca gctagagctt 32940
tcaaacataa acatgtgatc agattagtcc cctctttaga acaccctagg gttctcactg 33000
tcctgagtac agtctaaggg tttaccatgg cttacagggt cttttatgat ttggtgagct 33060
ttttattgta taacctttct aaactgcctt tacttccctc tttcttggct ctgtgtcttt 33120
gcataatgct gttccctata cttcacctca cgtctaacct tcatctcctt ttcacttctc 33180
ctcttcctcc aaaatccagc tgaatatcac attgtcatgc aggcccattc ttgatctccc 33240
acgtttgggt tagatatccc tcttcagtac catcaccgca ccaggtgtgt cccctatcct 33300
agcatttgcc tcattgtatt acaactactg tgtactcgtc tctacagctc ctgctagtct 33360
aaaagttttg ggagagcaaa ggttcatgtt tgtgtttttc actgtggtat accccagtgc 33420
ctagtatatg ataagctctc aaaatatttg ttagatgtat gaagaaatga gaaagagaac 33480
aggaagaggg taagtttcaa gactaggaaa caaggctatg aaagctgcag gaaagcagca 33540
ggttaaaacc tagaagaaga gtttgtttta ggaaatactg tgttttaaac cactataact 33600
gaagcaaaaa cccaaggcct gggtgtggat agagtccact atctgataac agtggatact 33660
gatgcatggc agagttggag aggaagagag ccagattcca aaacagaagg ggtaaagtct 33720
tctaagaaga tagattatag taagaaggat taggggatag aaatatgagc ctgttccact 33780
catagatctc aaacatgaaa tgatgagtca tcatgaagag agtaggcaat tgtccagtga 33840
agaaggggat gctaaccctt cttaaccttg aatctctcag gtagaagcag ttagagaagg 33900
aacagccatc atcagatagt gttgtaagga aaatgatatc cttggggaaa cctgcatttt 33960
ggtaaagcaa agcaactaag aaagaatata ctaccactgt ttaacaatcg ccacaaaaag 34020
acagtaggat catctttgac ccccctcatc ctttctcagg aacttggagg actaagaaga 34080
gagaaatctg tagaagaggc ttctctctct gatcctccct ccacttcagt tttaccacat 34140
gtaatgcaac aataattaag aatttgtgta aaatttcacc aggttggcat gcatggagag 34200
aaaaattatt cagatgtttt cctttgtcaa taatacaagg agcatttgta gggaaaaata 34260
tttacaaata cagtaagacc tattctcttt ctatatttat gggaaaattt taagttgtgc 34320

ccttgtttca tgtgtgtttc tatttaaaga taccatactt aatatatatt gttgattcat 34380
taacattgaa ctcatggcta acagcactat aaatcatgtc tgatcaaaac ttatgataca 34440
tgtactttct tcgtaaggta catcatagtc ttctcgtaca tgggaactct aggtagtact 34500
tcaggactat gcatagaggc cattttaaac agcaaaattc ccaacaaaaa gcacaaaact 34560
caaaaaatgt gccactaaat ttaccatgaa aaggacactt gtttacagtt tgagagctaa 34620
aacaagaagg tggcgtgtca cttcgtttga cttcagctgg gaacatgcat atcagtcgac 34680
tcaaattttt tgctattctg tgcttatcca cgaatcgata ggaaagcaag tgtggatttg 34740
ggggttacaa ataaaatgta gcaaatgtgt aaacttgcag atgtggaatc tacaagtagt 34800
tagaatcaac tatgttagtc tgatcattaa atcagttttt taaagtacta ttgtaacacc 34860
ttataacctg ccccattcac tgagtgttgt agtttatagt ttcattgggc attttcagta 34920
gttttatctg aagtcacatt tcaaattttg taattgaagc tccaaagtat gctaccggaa 34980
acacgagctg atgctgtgag acaaaatcaa caggtaatcc accatcacaa ctgtgggcta 35040
gaatgctcaa gaaaccttgg aggcccagag agctgagatg aatactgaag aatcataggc 35100
aggtttactc tgtcaagctg cctgtatttt gagggtgtag tcctcaaacc aaaaagacac 35160
caaatgaaca aactcagatg gcctcactgg ggaacagaga ttgaaagctg acactggaat 35220
gtgtacttaa aaaaatgaga gcccgttttg gaaaggcaga ctgggcacag aatgtggaga 35280
gctatatttg ctaactgaag aaatttagac tttatcctct acaaaacaaa gctattggtt 35340
tttgaaggtt gcataaaagc tgcattttag cagcatatat tttggtagag ctgttacctg 35400
cctgaaaaca tcaatgtcat ttcacacaaa tgatacttat cccttggtgt ttgatctaaa 35460
tttctacaat gagaatgtga ttttatagtc tttactgggg aaggaagtag gtttttcagg 35520
ccgaaattct tgtgtagcaa aaattaacac ttaagttagc ccttggcaat ctccagttct 35580
ataatggtaa aatggatttc ccagaaagtc actctctatc cctttgaata gacattagaa 35640
ataacatgta ctttaagtgg gatttacaga ggaagggggc ctttaattct ttactagtgt 35700
gatgccctgt aaaaaaataa ctaacattag agttgaggcc tagaaatagc agcactgggt 35760
taaagtctgt tttcaagtgc aagtttttct ttttattcgt gtgtgtgtgt gtctgtgtgt 35820
gtttcacata gaaggaggaa atgccaattt cagttcttac aaatattaat gactgcaact 35880
tataaaaatg ttacagacta tattcttccc ttttgtaaca gatgagaaga ttttgaaatt 35940
tagtctctac tttttagttt ggtaagacaa tttgaataaa ctgcaataat tgcaaaagaa 36000
ttctgaatat ttgaacattt gacattttct atgtcaaata tacatttctt gtactatata 36060
aacattctag aaaagagaga caggcaggga ggaaagtgct cattaaaaag agcttcaccc 36120
tctctgaaaa gggatttcct ttacagtgct gtgtactaaa gcctgtgttg taaatcagaa 36180
agcactgagc acacatgttg ctgctttggt agcatcagaa gtcgattttc attagcctta 36240
taccattcac tatttctgcc aagcaatctt aaattataaa agaatcttat ttgattttgt 36300
gattctcttg ttttctgctc ataaagaaaa tatcctaaat tgaacaatgg catgctacgt 36360
ttttagtttt taagacagct aatgtgtaaa aagacattta aagtatagtt gtgttaagtt 36420
tttgaagttt acagttgttt caattttgct gctatacttt gttaacatat tttaggaata 36480
tttcatttta gtcacaacta ggatataaac attattttgg tggcgatctc cttgtaatca 36540
cgacatcaac caaatttggg aaattttgat ttgttagatt tataaatttt acagtaacac 36600
aaaagtctaa tttcctatat attttcaagg cccctatacc tttgtcaaaa taaagtatca 36660
atgaaaaatg aaaaaatcat aaactatgtt caggccaaac tgatactgac tttgttaaaa 36720
ggctagatag aaatctgttt tcctcttctg ttacatctcc tcttctggag accactctgt 36780
gtggactgaa ggtttgagat cctaggacct aggctagaac agattaggag attgtgctgt 36840
atgttaagtg gcagatacca tggaattcta agcctgttac gaaggaggag aagaagaggc 36900
acaatgaccc tgacacagcc cctgggttga ccacagcaga tatctcactt gagcaagtag 36960
atatcatctc aattgcttgc tgattatctc taacttgtca gtaacttact ttgataacct 37020
agatttagga gtctgacagc atgcagtgta tgcctcataa taatctgctg tttatgaaag 37080
tcataacatt gtatgtttag cataatggtg aagagcctgc catctggaat ggtctactta 37140
tttgggatcc acatacagta agctctcact taacatcatc agtaggttct tggaaactgt 37200
gaccttaagc aaaacaacct ctaatgaaac caattttacc acaggctaat tgatataaac 37260
aagagttaag ttcctgtggc atatttctgg tcacaaaaac atcactaaac ttctaaataa 37320
agacccaaaa cacttataat attaaccact gaaataaatg tgagctatat atatacattt 37380
aagaataata aaaacaaaaa ataattattt acccaatttt tggtgaacca gtgagtgata 37440
gtgatcatag tgatggtgga tgaaatcaag gaataaatat ttgcaaagtg aaaattgtaa 37500
gaagcacccc ctgtcaccac atagctcaga aataataatt agggcaggct tgctgagcat 37560
ttttaaactg cactgtttat tgtcatgcat ttgaatgatt atcgcagact ttatgaattt 37620
tcattttata ataatttgta ggccaggcac agtggctcac gtctgtaatc ccggcacttt 37680
gggaggccaa ggcaggcggg tcactggagg tcaggagttc aacaccagcc tgaccaacat 37740
ggggaatccc catctctact aaaaatacaa aaattagcca ggtgtggtgg tacacacctg 37800
taatcccagc tatttgggag gctgaggcag gagaattgct tgaacctggg aggtggaggt 37860
tgcagtaagc cgagattgtg cccctgcact ccggcctggt gacagagcta gactctgtct 37920
caaaaaacaa taataataat ttgtattcat tcatttccca atgtgttcat tccagttcag 37980
ggtccagggg gcctgcagct tatactcata gctcagagca actgacccta tagacaggac 38040
gccaccccat tgtagggtgc actcaaatgc acactcacac tcaaactggg acccttcaga 38100
catgccagtt accgtatcac acacagcttc gggatgtggg aggaaagcga agtatctgga 38160
gaaaaactac acagacatgg gaagaacgag ccaactctac acagacagtg gccctggaca 38220

gagctgggca ggcatcagtt tttttctttt tttttgtggg gggtgagggt ggggcatgga 38280
gtcttactct gtcacccagg ctggattgca gtgcagtggt gtgatctcag ctcactacaa 38340
cctccacctc ccgggttcaa gagtttctcc tgcctcagcc tcccaagtag ctgggattac 38400
aggcgcccgc caccacacct ggctaatttt tgtattttta gtagagacaa ggtttcacca 38460
tgttggccaa gctggtctgg aactcctgac ctcaggtgat ccacccgcct tggcctccca 38520
aagtgatggg attacaggcg tgagctaccg cgcccagtca gcatcatttt ttttttctca 38580
tcaacgttaa aacaatgttg aacaaaacat tattcaaaga cctgccgtat ggctattttc 38640
tagttgtgtg actttctttg ggaaagttag caaccctttc tgagcttaaa tgtcctcatt 38700
cataaaatgg ggctagtaat aatgcataag gtttttgtaa gaattagaat taataaagta 38760
cttagaccat aataactaat tagtattagt tgttgtcttt gctattattt tgatgtggtg 38820
gttgtttggt ttcacctgtg tactatcagg acatgctgaa ataaaattta agaattggct 38880
ttataatatt agaaaagcaa acttttgtac gatatgggta tgaaaaattg ttgggagtct 38940
actttttctc tcttacctaa tttgtcttag tctttttaaa gcttagattt tccaaatgag 39000
ccatagcaaa atataatgtt taaaaatgtt taaattctaa gcactatgtc atagttaaat 39060
aacttaaagg tgctacatct tatacagtcc aaaaggaaca taattagtaa aattctacaa 39120
tttagaaaaa aaaatagctg acagtgactg atttataaaa gtaaaatatc ttttgttaat 39180
actaatattc tttttataaa ttaattgatg acaaaaaatt gagtgaatga gatttgcagt 39240
tcatttatct atgatgctgg tttatttaat ctctataatt tgctgtattt gaaagagcat 39300
agtgatagag gtcatgataa aatctaggcc cagtgccaca agtaaatccc tgtaggaact 39360
ctcaaggttt tgatttcatc tctgaatggg aataacacct tccaagaata ttatgaagat 39420
taaaaagtta cgtatcataa atacacacag agtaacaata ctgggaatat tgcaacttgt 39480
aagaaagagg aagcatatgg catattctga tggttaggga tatggactct gtagctggga 39540
tgcctgaaag agaactctga ctccactaat ggctagttat atgaaattgt gcagataatt 39600
taacttctct gagtttgcat ttttctttgt ctatataatg gggataataa tagtacctac 39660
ctcacacata gtgttaattt ctattagtgg ttctcattaa gatagtattg ttgttcatcc 39720
ctggttgtta gccatcatgt atctgagtta gagagtcatt gattttagaa agtcccgagg 39780
agactatcag gtcaagcaac ctgcctcctg ctagacaatt agctttatcc atgagttacc 39840
aaagagggag ccgaaaccca gggaagctga aagagctgtt gattgtcacc ctgtgagttg 39900
gtgatagaaa gatatctgga atcccagtag ttgcccattt cctagttctg ggctctgcat 39960
tgcactagaa tactgtgcca ttctaaatat gaaaaggcag tatgaccatt gtgcttgtca 40020
ctttccattc cctagatgct atcttatatt tgtccttatg aaatttaacc tgtgactttc 40080
agatcactta gaaccttggt tggacagtgt tttctagtgt tatttagtat atttttttgt 40140
catcttctgt tgtctttggg ttcccctaaa agagctatac tctgggtgcc aggaaacttc 40200
acacatgact gtcttctctt cctcgacttc cctctctact tacctttcca gctcgtagca 40260
aatcagaaga cttctctgac acctctctat gtctaaaggt cctttgatat tctcacatgg 40320
cggcatgaat cacagtgtat tttaactggc cttttccttg tgtgtctcct acaatgagct 40380
gttgaagctt catgaaaaca caatctgttt tactcagggc agttataatt ccaattacaa 40440
agcacatttc ctggctcctg gctaggaact cgatcatttt tcgatgcttc cttgctcagg 40500
actttctgat tccttcttaa aacattttgg ggcatctcct tctcctggtt tttggaaaca 40560
tattctcata ctgctatgaa ggtttttact gacatttcca acttctctta aattgattca 40620
gcaaatgttt ttccataata aatgtcattg atatgtcatc aatatggaga gcaacaacag 40680
aatgcattga gtaaactcct cccctggagg tctgagaatc tagattccag ttctcacaga 40740
gccaccacct tggtgacctt ggacagtaga ccttctaagc ctcagtttcc ttatccctta 40800
agtggggata ttaatagaac ccattctcag agatgttgcc aagattaaaa taaccaagat 40860
aattcctgta gatgatttgg catagtgcct gccacgtact aagcaagagt tagcctccgt 40920
cattatagta tgatcataaa aaatgaacag actaaacgaa gtaaccagaa ggaaagaaat 40980
tttaattctt aaaatgtaat agtttcttgg tttttttttt tctgtgaaac acctgcatgg 41040
cacctttttg ttattcatac tgttttgact gtggctgtcg tagattcttg ttgaaagtct 41100
gagagactga gacttgtcat tttgaacatg gcatcagtgg aacagcttat gattcaataa 41160
ttgcatcatc ctggacaagc accagtagaa gtgagtcagg acatgtgata aaaagacatt 41220
cattttgccc ctcctccctc tctgtatttt ctttgctata aaattattga tgttaagccc 41280
atagtactaa tatttcagtt caattcataa taaaatttga gggcatttga atatattatc 41340
tgttgtaaat tataatttta tatttgacca cagagtattt gaagtgggtc ttttctttcc 41400
ccaaaattct attttaataa ctaaaaaata ttcttaggag aagtattatt taagaacagg 41460
tttatattaa ataacatcat ttcactttca actttctggt ggtcaaaaaa tatgctaata 41520
ctaattagga tatgatacac atgttctgtt agaacagttt tggcagttag aagacttctc 41580
ttcttgtgtt tgaaagggat gttacttggg gtagttatga gccatgtatc cagatgtcct 41640
gaaaggacca gtggtagatg tatttctatt tttgtctttt cttttttctt tctggcattc 41700
tagttgctga gtgactgact tttgttttca gctcttctca caatcaccat tgttctaata 41760
actttgctta aatagaatgt ctccttttgc tataagccat ggggccattt accgttaatt 41820
ttttaaagta ctgaaatgag aacctcataa attaaagaac actcctgatt ctgagttagc 41880
agatcctact aagccttttg cagatggaaa tttcctttaa attggtttgt tttcctttaa 41940
cattccatta tcctattgtt cattctttgg agctgtgatt tgtttaatat atttcaggct 42000
tcttaataaa tcaagtcatg taagttatta tttggatcat ttcgaaacta caacagctta 42060
tcaaacctct gaaagaagaa ttttgtgttt gcccacagac tgaagaactg attcagtttt 42120

attggctgag ctaccttcat tattcatatt taattcctgg tactgagggt gggaggaggg 42180
agaggagcag aaaagataca actattgggt actgggccta atatctgggt gatgaaataa 42240
tatgtacaac aagcccccgt gacatgtgtt tacctattta acgaaccctc acatgtatcc 42300
ccaagcctaa aagtttaaaa atatatattt ggtaaatcaa ttgatgtgtt ttaaaaaata 42360
tcgccttttg gccgggtgtg gtggcccatg tctgtaaccc cagcactttg ggaggccaag 42420
ccgggcggat cacgaggtca ggagttcaag accagcctgg ccaacatggt gaaaccctgt 42480
ctctactaaa aatacaaaaa atagctgggc gtggtggcgc gcacctgtaa tcccagctac 42540
tcgggaggct gaggcagggg aatctcttca acccaggagg cggaggttgc agtgagccaa 42600
gattgtgcca ttggactcca gcctgggcga cagagcgaga ctctgtctca aaaaaaaaaa 42660
aaaaaaaaaa aatcatcttt aaagagataa ctaacccttc cccagaaggc agggccaaag 42720
tctaaggttc ttccaggtcc tttgtattcc ctataaattt tagagtcagc ctgtcaattt 42780
ctatacacac acaaaaaaaa gcctgctggg attatgattg gtattgcatt gaaattaaat 42840
caatttgggt ataagagact tcaatttggg gattgagtct atattgagtc ttccaatcca 42900
ggaacactgt atatctctcc atttagtcag atatttagtt tatttcaaca atattttcag 42960
atctttagtt cctttcagca atattttctc atttttcctg taaagctctt gcacatcttt 43020
tgtcccatat ctattgtgta tatgtgtttt gctagttatt aaattatatt aatataaatt 43080
ttattttcca attgtttgtc gcatatatag aatgttttaa aaatattgtg tcctgtgacc 43140
atgctaaatt aactaattct agtcattatg tcttcattat ctttctcttg aattttcatt 43200
gtcttcccct tctgggactc cattcatatg taaggccatt tgatactgtc tctcaggtcc 43260
atgaagttct gttaattttt cttcattctt ctttttctct gtgttcttca actgaatgaa 43320
tgccattaat aatttggtat gtaatggctc acttaaactt ccttttgttt ttaagatatt 43380
tctactctca gctgtgtctg gaatccttta gtccggagcc ccaccaaccc tcagcctaga 43440
aggaaggagg agaaggatag ggtgaaagga aggggagagc ttctagcttc aggacagaga 43500
tcagaacaaa caacagagca gtcatcttgg ataaggaaac ttccctcaaa cctattactt 43560
atatcctcag aaataagaaa aataatgcat ttatcaaatt aaaggatttt gaaaaaggga 43620
acattcagag aataaaacta aactcttgaa agttaaaagg atgataacat aaatgaaaag 43680
ctcagttgaa ggattgaaag ataaaagtaa gaaaatatcc cagaaataag agcaaaaaga 43740
cagcaatgta aaatagggga gaagataaga gaattagaga accagcttag gagttctaga 43800
aagagaaaat gtagacaaca aaaggtaaga aatcatcaaa gactggagta ggggaggtca 43860
tgctatctgt ttctttttct attttttatt ttgagttaca ttttttttac tgtgaaacaa 43920
gcatatgtac atgagaatga acaaaacaaa tatgcagtca tgtattgctt aacaacagag 43980
ataggttctg agaaatgcat cattaggcga tgtcatcatt gtgcagacat catagagtga 44040
acttacacaa atctgaatgg tatgtcctac agtacacctg gaccatatgg tatagctgtt 44100
gcttccaggc cacaaactta cagcatgtta ctgtactgaa cactgcaggc acctctaata 44160
catcggtaag tatttatgta tctaaacata gaaaaggtac aataaaaata caatataaaa 44220
gaggaaaaaa atagtacacc tgtataggtg cttactgtga atagggcttc caggattgga 44280
agttgctgtg agtcattgag tagtgagtga atgtgaaggc ctaggacatt tattatatga 44340
agtctactgt agtgtaaact ctgtagactt aggctacact aaatttatag aaaaattttc 44400
ttcaataata aattaacctt agcctactgt aactttttta ctttgtaaac ttttaatttt 44460
tttaacattt tgactccttt ttagtaacac ttagcttaaa acacacacat tgtacagctg 44520
taaagaaaat tttatgtcct tcttctgtaa gcttttttcc atttttaaga tgtttttatt 44580
tttaaaactg ttactaaaaa ctaatacaca aacacacaca ttaacctagg cctatacaaa 44640
gtcagtgtca tcagtgttca accttcacat gttatcccac tggaaggcct tcaggggcaa 44700
taacaaacac agagctgtcg ttttctgtga taacagtgcc tttttctgat atacctactg 44760
aaagacctgg ctgagagtgt ttgacagtta acaaaaaaaa aaaaaggaca agaagtacac 44820
tctaaaataa tgaaaaaagt ataatacagt aaatacataa accaccaaca tagtcattta 44880
ttatcattat cgagtattat gtactgtaca cagttgtatt tgctgtactt ttctataact 44940
ggtagcacgg taggtttgtt tataccagca tcaccacaaa cataagcatg gtgttgtatt 45000
acaatgcaca gctgcagcta agtgatagga ctttttcagc tccattataa ttttatggga 45060
ccatcactat aaatgctgtc catcattgac tgaaatttat gtcgtgcatg accatacata 45120
caatttaatg aaaaataata ataataaagc tagcagtgtg taattaccaa ccagggcaag 45180
aaatagaata ttgccaatac cttggaggcc tccagtatga ccatataagt ttacaaatcc 45240
tattttgttc ctcctcccca gaggtaacca ctgccctgac aaatgtgatc gttgttttct 45300
tgtttttctt actacctata taaacatcct taaacaatat aactcagttt gtatattttg 45360
aattccatgt taatagaata tcatatgtat atgaatttta tgtgaataga atattatata 45420
tgtcattttg catcttgctt ttttcattca acattgtagg attcattcat gttgtagtgt 45480
acagctgtcg tttattcatt gctgtataga attatatcct cagagataag atatatggat 45540
gtttataaat cattccacta ttatgaacat ttgactagtt tgtagttttt atttaaccaa 45600
aaaaatgctg ctgccaacat tcttacacat tttactgtat atgcacatta atttatttac 45660
aagtataaat ttctttttga atacatatct attgatggag ttgctacatc ataggacatt 45720
cttgtctttg actttactgg ataataccaa actgtcttcc aaaatgatta catccttaaa 45780
ctcaggacac atcttattgt caaatgttta atttttgtca gtctgatggg tatgtaagtt 45840
attttattgt cgttttaatt tgcatttccc tgattactaa ttaagctgag taacttttca 45900
tatgtttatt ggccatttgg agttcctgta ttgtaaagta taagtttttt tgtccatttt 45960
tctagttttc tgtcctttta gttgaaatcc aaatttgcct aaatctgtta ttctctgagc 46020

acaagtaact tgggatgctt tcctttagat ttagcctaat tctttatcat tttgtcagct 46080
tgatggtgct tttaaggaga tatatatgtg tgtgtgcgca cacatgtgcg tgtgtgtata 46140
tatatatatg tatatgtatg tatgtatttt ttgagacagg gtctcactct gtcacccagg 46200
ctggagtgca gcggcacagt cttggctcac tgcagcctcc acctcctggg ttcaagcttt 46260
tccctgtctc agcaacccga gtagctagga ttacaggtat gccaccatac ccgctaattt 46320
ttgtatttaa tagaaacagg gtttcgccat gttgacaggc tggacttgaa ctcctcactt 46380
gaactcctca cgtcaagtga tctgcctgct ttagcctccc aaagtgctgg gattacaggc 46440
atgagctacc gcgcctggcc tggatatttt ttaaaaatat tttttatcta gcactttggt 46500
ttttggcagg caggttggca ctcatagtct gacctaccat ttctataaaa agaaacctgt 46560
aaatgttctt aaacagactt tgaaccagtc ttcctgattt tgaaccccta cctttacccc 46620
cagtttttga gcctttcaga attttttttc ataataatta ggttgcttct tagctttccc 46680
cactggtgac ttaacagatc ttaggaagcc aacaatcctt gtccatctgc tttctgtctt 46740
gtgaactgtt gctggtattg tctcttctct ttattcttag aggtgtatgc ttttaaaaac 46800
atatactggg tttgagaggg agctgaaata aaagcatgtg ttaaatatac catctttaac 46860
cagaactaca tttgactggt cattttattt tcaagctcac atacacttca aacagagata 46920
tggctaaagg aattatcatg tgaacaacag ccagggctct gaacatcaca gattatatca 46980
tcatacttga aatatttgaa attttgattc aaaatgagag ctttatagct atgtcctcaa 47040
tggactaagt gtttaagtac ttaacatcca aaacattctt actaatcaag agaagacaaa 47100
caccccaaca gagaaatagg caaattttat caatagccag ttcaccagat ttgttttctg 47160
ttagaagcga atatggggaa atacatgtgt ccatgttttg cctacttttc ctggagcagg 47220
taaggagagg cagtttaagg atccatgtga taaaccctaa agttgtccat cggctttcca 47280
gtcccttcta ggaatttaac ttagggaaat aatcagacat ttgcaaaggt gtgtacagtg 47340
gtatttataa tagtgaaaaa ccaaagaatg accaataacg ggagaatgga agttacagcc 47400
aaatacttta caactactaa agaatcatgt aaaatatcta ttgacatagg agttttatca 47460
aaatgtgaag tatacagatg aatagtacca cacataaaaa gcaaggtgca aattagccat 47520
ttatattgtt atccccaaaa taaatagatg cagttttttt aaaagatgca ggctatatat 47580
ggaagtgttt gctggttttc tgtcaaaaga atggcgactt tattttctaa tttaaacttt 47640
ttgctgtttt ctaaattgtc taaatagtta tagtttttat aatgtaaaag tatcttccaa 47700
tttagcttca tttgacaaat taccttttca ttctatctag ctatgtaatt ctaaatgaat 47760
ttacagcagt aatcttagag cagatgaatt tacaacaata atcttagagt agactacgga 47820
ttagatgtaa aaacatgagt tgggctttat ggttacagag agttttcctc agtgtgggga 47880
tcatagctgt attgagttta ttcagttttc ctttcccaca tgaatgaaaa atggggccag 47940
cctacaactg gaagggcctc ggcatgtacc actgtactgt gtatgatgtg atttcttgat 48000
gctagtaggg agagaatcaa attgcctcct attcaaacca agacccacaa atagcgtcaa 48060
ccagtcattt cagctactcc ctgcagtgtc aagaaggtgt gaacccctca tgttctctat 48120
tgcataccct tgtctaattc agtgtttctt cttcttttca ggttttggct ttatgctaca 48180
tttcagaaat cataataacc ttttctggta ttattttatt ctttttcgca ctgtgagaaa 48240
aattaaactt tcaagtggat gcttcttata aactatttat acccttttgc tcccttttgg 48300
gaggcaggga cagggacaga gttcctcctc aggctaacta agaaaactta ctgcttccaa 48360
tgtaatttaa aagatctccc tctttctatt gctctctgta ctcttaattc tttttttttt 48420
ttttcacagc agagacaagt gaacatttat ttttatgcct ttcttcctat gtgtatttca 48480
agtctttatc aaaacaaggc cccaggactc tccagattca attatgtcct tgggcttggt 48540
cgactgctgt aggagtctca gggagccttc tacaaatgct agagtgactc atttaccaac 48600
attaaaccct aggatacatg caacaaagca ggactccttc ctccatggaa tgtgccgatt 48660
tcagatgaca cagcacccaa tgtagaaaac gctggaattt ttccttggaa ctagactgtg 48720
atgagaggtg cttgacatga acataagcta ctgtcttttc tttttttttg agacagagtt 48780
tcgcttgttg cccaggctgg agtgcaatgg cgtgatctca gctcactgca acttccacct 48840
cccaggttca agcgattctc ctgcctcagc ctcctgagta gctgggatta caggcacgtg 48900
ccaccatgcc cggctaattt ttgtattttt agtagagatg gcatttctcc atgttggtca 48960
ggctggtctc gaactcccaa cctcaggtga tctgcctgcc tcagcctccc aaagtgttgg 49020
gattacaggc atgagccacc acgaccggcc agctactgtc ttttctttga cccttccttt 49080
ccagtttttg aagataaagc aggaaataat cttctctgaa gatacttgat aaaaattccc 49140
aaaacaacaa aacgcatgct tccacttcac tgataaaaaa tttaccgcag tttgtcacct 49200
aagagtatga caacagcaat aaaaagtaat ttcaaaaagt taagatttct tcagcaaaat 49260
agatgattca catcttcaag tcctttttga aatcagttat taatattatt ctttccccat 49320
ttccatctga atgactgcag caatagtttt ttgtttgttt gtttgtttgt ttgtttgttt 49380
tttgagatgg agtctcgctc tgtcgcccag ctggagtgca ctggcgcaat cttggctcac 49440
tgcagtctct gcctcctggg ttcaagcgat tttcctgcct tagcctctcg agtagctggg 49500
actacaggca cgtgccacca cacccagctc atttttgtat ttttagtaga gacagggttt 49560
caccatgttg gccaggatgg tctcaatctc ctgacctcat ggtctgcccg ccttggcctc 49620
ccaaagtgct gggattacag gcgtgagcca ccgcgcccgg ccagcaatac agtttttagt 49680
tactcgacat ctttaagcct ataactctta ggctatgcat agccccatgt cctaatcagg 49740
cattcactga tcccagcagg tctccatcta tttgtaccag cctcctcttt cctcccaatc 49800
tcaaggttac tcttaaatac tagtaaatgc aaaaagaact tgtaaagtgg caaggcatgg 49860
cctatcaaaa gtcagcccaa gggcagtttt cagccctgcc tcacctgggt ctagttcagc 49920

tgacggatga gctgattgat gcgttcaccc cgatagccag gtgtgcccat ctccttgagg 49980
aagcccactc ttatttttgg tagcatgatg ggccactgag aggtggaaag ggcgcaagaa 50040
ccatgagatc tcctggaaat gcttccctgg gaaggcaatt tcatgaatga ggtcttccaa 50100
gcaaatgaag ccaaacttcc ccaggtgctc ctcaatcact gtgttgtctg tcagagggat 50160
ggtcttattc ttgaccttgg cttgtccacg tttcaaaatg agttctcgga cagacttcag 50220
atttggaaat ccccaggtca cataaggttc cactatatgc agcattttta gattctaggg 50280
ggtaactttt acaaagatac cactaaaaat tttctttagg cgaagtcttg cagtggttct 50340
ctgcacccgt aaactcacgc catcaatcct ttcgatgcgt acaacaaagg ccaaggaatg 50400
tttatctggc aattccaagg catgaggttt cacttctagt cgtctgagac gcaccttgtc 50460
acgtttctgc cgccaggaat catgtaggaa tgattccagt cgcttaaacc tgagcccttt 50520
tcctttcttc tgtcttgctc tgccatcttt ctagtggtgc agctactcaa ttcttttttt 50580
aattataatt tttattttaa gttccagggt acatgtgcag gatttgcagg ttacataggt 50640
aaacatgtgg ccatggtggt ttgctgtacc tatcaactca tcaggtatta agcccggcat 50700
gcgttagcta tttttcctaa tgctgtcccg cccccccacc caacgggccc cagttacact 50760
cttaatcctt atagctcaga tgttatgatc cacagtgtgg ttcttacaga aagttatgga 50820
ttaaaaaaaa aaaaaaacac tcaaagtgcc cgaactttct taaaataatc ctggtacagc 50880
taaactcatg cactgactgt ccacctaata tttaacagtc tgtgttgtga tatattgttt 50940
taatgttctg aatgcttgtc agctttcagt attgaagatg tgaatcattt atcagcaatg 51000
acacatttag tctaaggttg tcagctattt atgctacaaa ttaatgactt gtccttaaaa 51060
tatcaatttt gtgattcatg ttttggcagg tggttagatg ttttgtgttc taattttaaa 51120
ctatggataa aggttttgtc ataatcattg ttttattggt tccttttctc ccctgcccac 51180
tccccaaaaa accctgcaat tcttttttgt taaactttta ttttaggttc agaggtacat 51240
gtgcaggttt gttatatagg caaattttgt gccacagggg tttgctgtac agattatttc 51300
atcacccagg aaataaacac agtacttgat ggataggttt ttagtcttca ttctcttccc 51360
accctcaagt aggccccagt gtctgtcctt cccttctttg tgtccctgtg tactcaatgt 51420
ttagttccta gttataactg agaagaacat gtggtatttg gttttctatt cctgtgttag 51480
tttgcttagg ataatggctg ccagctccat ccatgttgcc gcaaaggaca tgatttcatt 51540
ctttttatcg ctgtgtagaa ttccatggtg tatatgtacc acattttctt tatgcagtct 51600
tctgttgatg ggcttttagg ttgattctat gtctttgcta ttgtgagtag tactgcagtg 51660
aacatacaca tgcatgcgtc tttatggtag aatcatttat attcctctgg gtatataccc 51720
agtgatggga ttgctgggtc gaatggtagt tctgttttaa gttctttgag aaatcatcaa 51780
actgctttcc acaatggctg gattaattta cacttccacc aggagtgtat aagcatttcc 51840
ctttctctgc aacctcacca ggatctatta ttttctgact ttttaataat agctgttctg 51900
actggtgtga gatggtatcc cagcaccatt tattgaatag ggagtccttt ccccattact 51960
tgtttttgtt gactttgttg aagattggat ggttttaagt gtgtggtctt atttctgggc 52020
tctattctgt tgcattggtc tatgtgtctg ttttgtacca ataccatgct gttttggtta 52080
ctttagcctt gtagtagttt gaagtcgggt aatacggtgc ctccagcttt gttcttttgg 52140
cttaggattg ctttggctat ttgtgccctt ttttgattct atatgaattt taaaatagtt 52200
tttttctaat tctgtgatga atgtcattgg tattttgaga gcaatagcac tgaacccgct 52260
aattgctttg ggcagtatgg cgattttaac aatatcgatt ctttctatcc cctgcaattc 52320
tttgttgttg tatttaacta tttttacttg tgaagttttt tcagggatga ttttgttgaa 52380
agtgacaact ctaaaaatta tgttggtaat taaaatttta agtaatgact tttattttca 52440
gagattccac ttctcttaga ctttggagct gttaacagca gtgtccaatc tgcagtggta 52500
ctcagcagtt tctgtttcct gcatgcagaa ctgcttatat gaaaacacag ttttaaaaat 52560
gctttcttat ggctgacatt cacattctta ttcctttgga ttcttttcaa gagggatttg 52620
gtttgttaaa attaattttt gcaatacttt tatgaagata caaactctga caaagctttt 52680
aaaacaagtt tgagagaata cagtattgat ttcacttgta aatctgacga ttattttaga 52740
aaaaaggaaa atattattta ctattatttt gcttataaat gtttatcaat tttaaagctt 52800
ccacattgca catctcccac tacaacagta gctaccattt attctttctc aaaaaaagtg 52860
ctaagtgtgc ccttgaaatt tttacattgt gcagaatatc cctaaaattt taaaacaaaa 52920
attacatcat cacttgcttt aaatgtttct tctttattta acatacagtt tctaaaatgt 52980
tagcaaatag cattttagaa gagacacgtt acttttctaa tgaatgttct aaaatgaacc 53040
acagtaacct atacttactt agactgtgaa aaacaaaact tatattctat tgttaaattt 53100
tcaaaagtga aactacacga tagtttactt ggcacatcac tctgttattg tgaattgaca 53160
aatgtatatg tagacaaata tgtgaaaatc agagtacata tacattatat gcagcaccac 53220
aatacatttt ttagtatgtt ttgactgata tttaattata taatttacca agaggatctc 53280
accagaatgt agaaaagtat tgaattttag aacaattcac atatttaaaa aaaatgtagt 53340
cagccctttt atctgtatct ggagaatgca gggtaaagga ataatacatg agtattggta 53400
tttaaaaaaa ggtgttaatt tcttacctat gatacctgtt actttgggta tcatttaacc 53460
tttatttctg tgaaatagag gagttctaac atcctctaat tattataata ttgttctaat 53520
ttaatctatc ttaatctgtg atacagtttg aaaaccaagc ttttactatt ggcatgtgca 53580
aaaaaataaa gcagcagtag acttggaatc ttgaatgcaa atttagattt tgcctcttaa 53640
taaatgtata atatagtgtt ctgggaccaa ttctctaaca tttctgagtc ctagtttctg 53700
catctgtcaa atgggattag agatacctac tttcaggatg tgatatggtt tggctctgtg 53760
tccccaccca aatcttatct tgaattgtaa tccccatata ttgagggagg gacctggtgt 53820

gaggtgtttg gatcatggaa gtgatttcct ccatgctgtt cttgtgatag tgtgggagat 53880
cgcaaaacat ctgatggttt aaatatggca gtttcccctg tgctttctct ctctcctgcc 53940
accatgtaag actttccttg cttcctcttt gccttctgcc gtgattgtat gtttcttgag 54000
gcctccccag ctatgcagaa ctatgagtaa attaaacctc ccttataaat tacccagtct 54060
cagatattct ttatagtagt gtaaaaactg actaatacag agaattggta ctggcagggt 54120
tgggtactgc tataaagata atctgaaaat gcggaagtga ctttggaact gggtaacagg 54180
cagtggttag aacagtttgg agggctcaga agaaaactgg aagatatagg aaagtttgga 54240
acgtcgtaga gacttgtttt gaatactttt gaccaaaatg ctgatagtga cgtggacaat 54300
gaagtccagg ctgaaatggt cccagagatg aggaacttat tgggaactgg agcaaaggtt 54360
atttttgcta tgctttagca aaaagactgg cagcatttta cccctgccct agagaactga 54420
tgaactttga gatgatttag ggtatttggc agaagaaaat ttctaagcag caaagcatcc 54480
tagtggtgac ttggctgatt ctgaaagcgt tcagtcatgt gcattcacga agatatggtc 54540
tgaaattgga acttaggttt agaagtgaag cagaacataa aggtttggaa aatttgcagc 54600
ctgaccatgt agtagaaaag aaaaccccat tttctgggga ggaattcaag ccagctgcag 54660
aaatctgaat aagtaacaag gagtaataag taataataag taaaaagtaa taagtaataa 54720
gtaacaagga gccaaatgtt aataaccaag acaatggaga aaatgtctcc agggcatggc 54780
agagatcttc ggggcagccc ctcccatcac aggcctgaga actaggaggg aaaaatggtt 54840
tcctgctcag ggccttgctg ctctgtacag cctcacgaca tggtgccctg catccctgat 54900
gctccagctc cagctgtggc tgtaaggggc caagttacag ctcgcaccat tgcttcagag 54960
ggtgcaagcc ccaagctttg gcagctttca cgtggtgttg ggcctgcagg tgcgcagaag 55020
acaagagttg aggtttggga acctgtgcct atatttaaga ggatgtatag aaacgcctgg 55080
atgtccaggc agaagtctgc catggaggca gagccttcat ggagaacctc tgctagggca 55140
atgcggaagg gaaatatggg gttggatccc tcatacagag tccccactgg ggcactacct 55200
agtggagctg tgagaagagg gcctctgtcc tccaggcccc agaaaggtag attcaccgac 55260
agtttgcagt atacgtctgg aaaagccaca gaatgccagc ctgtgaaagc cacaggggta 55320
ccctgctgag ccacaggggc ggagctgccc aagggtatga aagcccaccc cttacttcag 55380
tgtgccctga atgtgagaca tggagtcaaa ggagattttg gagcttttag atttaagggc 55440
tgcccagctg ggtttcagat ttcatggggc ctgtggccct tggtttgacc agtttctccc 55500
atttggaaca ggaacattta cccaatgcct gttccctcat tgtatcttgg aagtaactaa 55560
cttgcttttg attttatagg ctcatacgtg gaagggactt gccatgtctc agatgagact 55620
ttggtcttgg acttttgagt taatgctgta ataagacttt gggggactgt tgtgaaggca 55680
taattggttt taaaatgtaa aaagacatgg gatttgagag ggagcaagtg caaaataata 55740
tggtttggct ctgtgtcccc acccaaatct aatcttgaat tgtaacccgc atgttttggg 55800
ggagggacct ggtgggaggc agttggatca tgggggggtt ttttccatgc tgttcttgtg 55860
atagggagtt ctcaggagag ttgatggttt aaatgtggca gtttcccttg tgctctttct 55920
ctctcctgct gccaggtgag acgtgtcttg cttcccctgc cccttccacc atgatcataa 55980
gtttcctgag gcctccccag ccatgcagaa ctgtgagtca attaaacctc ctttccgtat 56040
aaattaccca gtctcagata gtatctttat agcagtgtca gaatggacta atacaggata 56100
gtaatgaaga ttacagaata tgtagatgaa gaagtgctaa gtaaatagca gctattatta 56160
tgtagtcaaa ttgaatgtat acattgtggt acttcagtgt cctttaaatt gaataactag 56220
aaatttgttg gctttctcaa tctgctcaca tcagatgaca tgttaattta tgcctatact 56280
tttttctagt taatagatat aaatctattc actcaacttc tattgacaga actggtagtg 56340
tggcaagaca tctcatttct agttaaggct gtataatatt aagttcattt tacttaaatt 56400
aactatggtt tgggaaatgc ttttcatgtc atcatgtatg cccaatttga tactttagtg 56460
ggacagtata tttcagaaaa aaacaaatgc ttccccaaaa attccagggt tgaatacatt 56520
agtcagacat ataacaatgt acttcagagt tcctctaagg gcaaaaatcg tggtatgaat 56580
atacaaaaca ctcctattta tacttttgta tttttgaaat gtagtcttca tgttaattta 56640
gcatttcaat gaccagcatg acattatctt aataatttgg aatgccaata tgttcattta 56700
agacttaata tagtaagtat ctaaagaaaa aaatggaagt gactgaatgc ttttgtatct 56760
cttaattata atttgtgctc cattgtgata tgaaggatag aaggggcagg atagatagaa 56820
aacagaaatt aactttgatg tttaacctta ccttaagact gtctgttaag tgacccacat 56880
aatcttaaaa aactctgtca agcttaatgg atgctactct gcaggcccct gccaggcaac 56940
agtcacaagg ttatgaggtg catagatttt ggaattaggc agagctgaat tcagatccag 57000
gtgttgcctt ataatgcgac tttgggcaaa taaaaggccc aatttttgta ttcttatctg 57060
taaaatggac tcagtaaaaa ttatttgaga taatttattt gtgtactgta cctaggcatg 57120
cagcttgaca cacagaatta caagtcagta gtttccagta tgattattat tgtgaaagag 57180
atattttgtt tcacctactg aaaacttttt tcagtcttaa attttttatc taactggctg 57240
tattgcagat gtctgctata taacttttat ataattttaa aaactatttc ttttctcctt 57300
gatcttctag gggtaaggtt accaatgttt tcattattta ctaaatatag cagcccccac 57360
cccttattca tggaggatag gttccaaaac ccctagtgta tgcttgaaac cacagaccac 57420
agataatccc aaatcctata tgtatattgt ttttcctata catacatacc tatggttaat 57480
gtttaaccta ctaattagga agagtaaaag agtaatagta actaataata aaataaaaca 57540
attgtaacaa tattccagca tcactattct tgtgctttag ggccaccatt aagtaaaata 57600
agggttactt gaacacaagc actgtgatac tgtggcagtc caactggtaa cagagatagt 57660
gatgcggttt ggctgtgtcc tcaccagaat ctcaacgtga attgtatctc ccagaattcc 57720

tatgtgttgt gggagggacc cagggggagc taattgaatc acagggtctg gtctttccct 57780
tgctattctc gtgatagtta ataagtctca catgatctga tgggtttatc aggggtttcc 57840
ccttttgcct cttcctcatt tttcttttgc caccaccatg taagaagtac cttttgcctc 57900
ccgccatgat tctgaggcct ccccagccct gtggaactct aagtccaatt aaacctcttt 57960
ttgttcccag ttttgggtgt gtctttatca caagcatgaa aatggactaa tacagtaaat 58020
tggtaccagt agagtgggtg ttgctgaaaa gatacccaaa aatgtggaag cgactttgga 58080
actttggagg actcagaaga agacgggaaa atgtgggaaa gttaggaacc tcctagagac 58140
atgttgaatg gctttgacca acatgctgat agtgatatga acaataagat ccaggctgag 58200
gtggtctcag atggatatta ggaacttttt gggaactgga gcaaaggtta ctatgttatg 58260
ttttagcaaa aagactggca gcattttgcc tctgccctag agatttgtgg aactttgaac 58320
ttgagagaga tgatttaggg tatctggtgg aagaaatttc taagcagcaa agcactcaaa 58380
aggtgacttc ggtgctgtta aaagcattct gttttaaaag ggaaacagca taaaacttca 58440
gaaaatttgc agcctgacaa tgcagttgaa aagagaaacc cattttttga gaagaaatta 58500
aagctggctg cagatatttg cataagtagc aaggagccta atgttaatcc ccaagaccat 58560
ggggaaaatg tctccatggc catgtcagag accttcacag cagcccttcc catcacaggc 58620
ccagagaccc aggaggaaaa agtggtttcg tgggccaggc ccacggtcct catgctatgt 58680
gtaggctagg gactttgtgc cctgtgtccc agctgctcca gctgtggctg aaaggagcca 58740
atatagagct caggctgtga cttcagaggg tggaggcccc aagccttggc agcttccaca 58800
tggtgctgag cctgtgggta cacagaagtc aagaattgag gtttgggaac ctctgcctag 58860
attttagaag acgtatggaa acacctagat gcccaggcag aagtattact gcagggcagg 58920
gctgtcatgg agaacctttg ctagggcagt gcagaaggga aatgtgggat tggagccctc 58980
acacagaatc cctactgggg cactgcccag tggagctgtg ggaagagagc cgtcatcctc 59040
cagaccccag aatggtagat ccaccaacaa cttgcaccat gtacctggaa aagccacaga 59100
cactcaatgc cagcctgtga aagcagccgg gaggtaggct gcaaagtcac aggggcggag 59160
ctgcccaaga ccatgggaat ccatcttttg catcagcatg acctggatat gagacctgga 59220
gtcaaaggag atcattttgg ggctttaaaa tttgactaac tcactggatt tcagacttgc 59280
atgggccccg taaccccttt gttttggcca atttctccca tttggaacag ctgtatttaa 59340
cctgtgacac ccccctaccc cctgcccccc atccctccgg cccttgtatc tggaagtaac 59400
tagcttgctt ttgattttat aggctcatag gcagaagaga cttactagcc ttgtctcaga 59460
tgagactttg gactgtggac ttctgggtta atactgaaat aagctaagac tttgggggac 59520
tattgggaag gcatgattgg ttttgaaatg tgaggacatg agatttggag gggccagggg 59580
tggaatgata tggtttggct gtgtccccac cctaatctca acttgaattg tatgtcccag 59640
aattcccatg tgttgtggga gggacccggg ggtgggggtg cagtaattga atcatggggg 59700
ctggtctttc ctgtgctatt ctcatgatag tgaataagac tgacgagatc tcatgggttt 59760
atcaggggtt tccaaaactt ttgcctcttc ctcatttttc tcttgccacc accatgtaag 59820
aagtaccttt cacctcctgc catgattctg aggcttcccc agccatgtgg aactgtaagt 59880
ccaattaaac ctctttttct tcccagtttt aggtatatct ttatcagcag tgtgaaaaca 59940
actaatacag atggctagta agggactaac cggcagggag cgtctccagt gtggatatgc 60000
tggacaaagg gatgattcac gttccagggc ataagatttc attactcaga attgcacaga 60060
atttaaaact tattaattat ttctggaatt ttccacttaa tgttttcaaa ctgtggttga 60120
ctgcaggtac ctgaaactgt caaaagtgaa accacagata agtggggagt cctgtaccta 60180
agattattcc tttaaattgt ttcagtggat atgtagggac ctgagtgtga agtgagagca 60240
gcagcatcaa aacctgaggg aaatccagat agcaaaagaa acttgtctag tatactggca 60300
tgacagagaa accaaaaagt tctcaagtta atgtgagaat ctaagaatta aagaattaag 60360
cctttgcctt tgagggaagg aaaggggtaa tgtggcttta aatcaggttg agattggttc 60420
tgagggttcc ttttccttcc tttatattga tatgaatata gacacaactg ttctgcattt 60480
ccatttgttt ttataaatgt ctttttagga tttaggaact gctaattatg caatatgaga 60540
tatctgttag tttgaggaac atttgaaaat ttggtcaaat gacacagatc gtcacacagt 60600
tttaagacaa atgtttttac ctatttgacc tagtctggca atccctattt gggcaaaaat 60660
cttcatttgc aggtcatgat tggaggcagg cacagaaaaa aaattgccac cttttttgca 60720
ttatgtcatc aagacatcaa acttcagcct acaaagtaga aagtgttatt tctcaagttg 60780
aaggcctgga tatacctcag cttctcagtt ctgacacttt atcatagtgg aaaatgaaga 60840
agattgctta agaacactga tgttggtgtc agaaagacct gggtttgaac cctgacttta 60900
ctagttactt agatcacttt aggcaactca acttttctaa atcttgtttc ttcatctgta 60960
aatgctgaaa atagtaccca cctcttaggt ctgtggagag gattaaatga gataatctat 61020
acaaagaaag agcttgcata atagtgccaa gtaatggtga ggttatacct gtattctgat 61080
tataatctca taaatattta ccatgttagc tgtctcagag ttcttttgca aaacagataa 61140
agatagaaag tataaataag aaaaataagt gaacatatac tgaactttgt acaagatgct 61200
ggcgatatgg agagacccaa gacatgggcc ctacctaaaa gagattattg atagaaacag 61260
gatacatata catcaaaagg taacatagga tcatctgtgc aaagtgctat atggcagtgt 61320
tttaggaagt ctagaagctg tcatggatca ggaataccat ggtggacact tcaggcaggg 61380
aaaacagatc ttagcaaaag ctactcctat cataggtact tgataaatat ttgtagaatc 61440
caggatccct gtagtgataa agaaactaca tggattatgt aggggagtga taagacatat 61500
gactggaaaa ataaaaagac caaattatgg accatactga gcttgtacta taaacagtgg 61560
aggagccctt cagattttta atcatgttga gaaaagagtt ttagcagtgt gtgggggata 61620

gaatggaaag agaagccagt gccagaagga ctacttagta tcaaccattg cagtggttaa 61680
agcaagaggt gagagaaggc atgcattaga atggcagcgg tcagagtgga tgggaaggaa 61740
taggtcctga catagtgtta cagggagtaa taaataggat gtggaagatg ggttagaatt 61800
ggcaaaatct ctgcatgtaa gtctgggtta ctaaatatag tgagagaaat tcaaatctct 61860
ctttaagaat cgaataaaat atttagaaat aagttactgt tgtatttgag gtgaacacaa 61920
atggcatttc aaagatgctc gagatacctt gttggaaaaa gtcaataact gcactattgt 61980
ctccaacatg ttcttgcctt ctctgaagac atcatgttcc taattctgaa ttatgaacca 62040
tctattatcc ttgtatgctc ttatgtgtga ggaaccataa ggtgggaaca aaatccggtc 62100
ttcattctag aaataactat gcgatcaaaa agtttttagt ctttcttctt accatactgg 62160
ttcttggtat tctgtttacc attcaatgta ctattattgc ttctgcttaa aactcacatc 62220
ccctaatgca agcctgagca aacagaactg ataacacaca gcctgagaag ggagtgcttg 62280
gggtctcaag acttattctg tttttctcca tctttgacac ttggtttgaa gagcaaagaa 62340
ggatacagct gttaggaagt aagttaccca aacacagtga ccaaactgga ttaattcttc 62400
caatgagaaa gaaatacatt atttctgtga gacagattag actttaagta gcatagataa 62460
catgattata ttctctctac aaataaatac acaggaccta agaaaccctt tacagatcca 62520
agtgttttcc tctccacttt tccatcccca aacccatctt gcaagatatg gccagcttat 62580
ttggagttaa ttaaatcaag accttcgttt tacagacagg gaaaccaagc ccagagacac 62640
tgagtagtag gccactggtg tcttagaggt ctgaaaaatc ctttactgaa cattctcttg 62700
atctattaat gtataggttt tgttgctgta accctctccc caagaggagt gaatataaat 62760
gatgcagagt ttggatgaac tatcttaata agaacctaaa gttgaaacca atgcaaacct 62820
ctctcaataa atgcaaagca aagagaataa tcagtctttc tttggcttgt taaataagat 62880
aaaatgtgtt ctgctaaaac catttaacag aaatattgtg aaaggtttcc cctaaagcat 62940
ttttctattt gatttgaaaa ctattccata gcttattatc aaacaaatca gtaatccttt 63000
agctaatgca gagataaatg ggcagtcaga aaatataatc acctggtgtg tgcagctgag 63060
tatttacatt tttcctaatg aacaaagata agaaaagtgc aggtgacttt aatgtgtaaa 63120
aactaccttt tagtgctagc gctagaggga aaaagaaatt actggctcaa gccaatcctg 63180
tacttgataa ctaagccgta tagtccatgg cttggcttca gttctgtttt gaatctcttt 63240
ttggacttgt cttgaatgga ctgtttaggg ctgcttcagt agtgcagttg ttgcattttt 63300
aagcatagtt taggttttaa aatgtttctg gtcccttttt ttttttcttt tccactttat 63360
gttgcttaaa gctttatggc caggttttct catcctcagc attattgaca tttgaagctg 63420
gatacttctt tgtggtgggg gctgtcctgt gccttgtagg ctggttagca gcatccctcg 63480
cctcttctca cttagatgcc aatagcattt ccccaaccgt gataaccaaa agtgttttca 63540
gacactgcca aatgtctcct agagagcaaa attgctctct gttgagaact actgtgttac 63600
ggtgtttgga caaaaactga caagccaatg ggaatattct attggtagtt gtaaaaaatt 63660
aatccagtta tagcagctgt atttctggaa tttttttcca tattaacact tgctttctga 63720
ggtgataata tctttgtttt ttttctccca aatagatttc ttgcattaca ctgaaaaatt 63780
gctgattaat tcacttaaat tgaagactaa gccaatcatg tcatttgggt aatagtttac 63840
caactctgcc cctttctctg tcagggaagc ctctaattta gtaagcgata ctgtatcctt 63900
ttgtcaggta cattaccatt cctattagca atagggcaat tgagattgag aaagattaaa 63960
aggtcaccaa gctattacat tgtagaatta ggttatgaat tgtagcctat ctggtttaga 64020
atctttacct tactagtctc cataacaaca attcttccag tgtggtccat ggggccctgg 64080
gagtctcccc ttaaagggca gactattttc acagtaacac gtactttatt tgccatttca 64140
ttatgtcagc atttgcaata atggtacaaa agcaaagatg agtaaaactg ttggcatctt 64200
agtatacagt agttactgta ttcactgtca tgcacttaaa atctttgaag aagcaaaaaa 64260
attattaatt acattaaatt tcaaccctta aatacatgtg gtctttctca tgtcagtgtg 64320
acaaaatgag aaggtgcata atccacttat atcgcatata gcatttgata gttgtctcaa 64380
agaaaagtgt ataagattaa actgtgagtt aacctacttt ttttcatgga gtaccatgag 64440
agataaactc tggttttcag ccttgggtat ttggcgatgt tttcccaaaa atgactgaag 64500
taaacttagc actttaagga aaacaactta aagtatttgt tgccaattga taaaatatag 64560
gtttcaagca aaaatcagaa tttttgaaga cttgtatctg ccactgtgag cttgacaaat 64620
gtgactcttt tatattacat aatgaactat gtcaacattt gaaagatctg cataactcag 64680
tgaaccagta ttttccagat gactaatgca tgataataca aaatcatgca tgggtaaaag 64740
atacattcaa agtgcaagat agactgacat atttcaatgt aacaatcaaa agttcattga 64800
taacagtttt ggattccaca ttgcaatact aaaaccttta aaaaacgaaa ttgtccaatt 64860
ttggtgtagt aatcagaaaa ggcaatctat aattacctga acttaagttt ctggaggacc 64920
attaaccttc tacaggctca tggggaagac tgtagcactt ctctttccct aagatcctcc 64980
agaaaggaag aaggtaatcc ttgggggtag ggtagagacc tattgtgtga tgatcaccaa 65040
gtatgtaaca atgctttata taactctaat atatataatc cacacaaacc ccctaaaatg 65100
gcactaataa gggaatggac tcaaagaagt taagtcagct agccactgtc acagctatta 65160
gagcactgga actaggattt gaacccagat ttgtctgtat gtaaagctga ttctcttcgt 65220
aatagtactg agacacaaga ggcggctaca aaatattctg gtactccatc ctagaccaga 65280
gtttcaaggt tcgttatcat ttgtagcatg atactggatc ctcacagtgc ttgcctttca 65340
ttcaggtgcc aggaaacgtc tgcctgaatg aatgggtgta atttacctgc acattttaca 65400
tgcttctcta ggtgtgtgat taactcataa tccatccatg actttcaccc ataatcctcc 65460
ttgtagcaat tgctttgctt gcaacaaaac taagtagaca tatctagctt tatgcatggt 65520

tttctctctc tgaactctaa cataaactca gcctcaggaa ttattcggtt tctactacat 65580
ttgccattct gattgggaac caccagcatt caggtattca cctggaacaa ggcattttgt 65640
tccaagggtt cctcacttaa aagcaagcac cctagcaata gttcataatg gaacttctta 65700
acattctcag aatgtttggc acagctgtga gtgaacacac attgagcaat caataactat 65760
tacagataat gatgccctta agaccaggat attttagctt tcccattcaa agggggtgaa 65820
atatgcactc ttactatggt atacttttgg ttccttctgc catgtatcct taataaaaga 65880
tgtcaattcc atatggtttt ctcttgagtt ctaaccattt tgttgtaccc tagccctttt 65940
aacaatatca aacttgcaac tgaataccat ttagcattca tccatttttt ccaatggtgt 66000
tcattatagg ctatcttact cctcctattt gtatgacaaa aattggcttt tttcaccgat 66060
gtctatggta catctggcag ctttccatgt actcagttct tatctgatgt agcccagaac 66120
gactgcctga agggatgcca aaagcctgat tgaggttcca aattttcagc tactgtacta 66180
tcaatccatt tgttcatttt tactttccct tgtcatctgt agcttacagt tgagtggcct 66240
gaacatgttt tgcatacatt gtaatatcta agaatttggg aatacggtcc taggatttag 66300
acttaatact accttccatt tatataatac ttactcataa aatcttcagt gttcctgaaa 66360
aagaaaaagg aacatgtatt gagtgcctgc tagaagcagg aacttgtagt agattttcta 66420
tgtgttacct tattttcaca acacacacac aggtgatatc cttcccagtt tactgatgag 66480
gaaactcagg ggtcaaagta gtagatacct acccaaggta acagaagctg tgaagtggta 66540
cagctgggat ctaaaatatg tcagcttcac cgtagatagg ctccctgatg aaccacctgc 66600
cacggcccgt atgaccgcat ccaggggtga tgatgtcatt ttcacagggt tattgagagc 66660
taaaactacg aagtactata aactattatt taaaatataa atacatacta tatatgcata 66720
tgtgtgtata tataattaat ggggtaaaca ttacagaata ctgtcctaac ctttaaacaa 66780
tgcactcgtt ttctgtaaac taatatacaa acaactgttt ggtccctaaa aatagatgtc 66840
aggtgacaga gactggctga gcaagaatag gagtatcttc agaatagaag ccagaggagt 66900
ttttgcttcc ccaacacatt gtcgcaccat tcactgttcc aggaccttcc tacttctctg 66960
gaaaactctg gcccaaagca gctcctctac attagtcaca agtttccatt aatcaggggt 67020
ggcctgtgcc ggacctacag cagagtcatt tcaggttatt ctgttacagg ctttcgacgt 67080
gtagtcagtc cactcgccca aatctagcag ggaatgaatg ccttgtaata cggaagcatc 67140
tacaaattct tcttaacagt gttcagagaa caatgtgaaa ccctggggcc ttttcccaga 67200
attagggtgg tgggaatgct gtcctattga ctaagcctgt taggtaagca ggcagttggc 67260
aagattcagg aagcttcatt tgaagataga atttagggcg atcgtttgga tttactggct 67320
taattactta aggtaacatt tataaaagaa attgtcattc cattattatt accttttaac 67380
ttttattcct aaacggaaca ttagcaacaa actacattac ttgataaatg taatttctaa 67440
ccagattgat aactagaaaa aaattttaag ttactttgct ctgtgaatta gtttaaacat 67500
atttgtaatt gagacttact actgttattg gctgaaataa ataaaagcaa gagataataa 67560
agaataacag agacaacgaa cacccaattt aagtttattt ctaagttcca tcttttttag 67620
agaaaaggca aattaagaaa agtttagaga gaggtactag tatatttatg aacttgtata 67680
gatgataagc aaaacggact ttaatatgta gaattccaga atcaacaggt tgccagcatc 67740
catgtttttg aagatttgct taagaacaca accaaaaatg gaatgggcag tctctaatta 67800
caagcagaag gctacaaaat cattttagct gcataataca gttttggttc taaagtcagc 67860
acgtaagagg aaaattcctt aggaaaatac aacattgaaa accattgtgt catgtaatat 67920
gaaatgcaat aattaatttt tcctccagta atagaaagat cactgtttca ttggtttata 67980
aaaatatatc tttatcatta aatgtggcaa aatgttaaga cttggtgaat attggtgaaa 68040
agtatatatc cattgtacaa ttctttccaa ttttttttga gattgaaaat ttttaaaaca 68100
acaaattatc ttttaaacag ctaataatca ctagacctgc actctttgtg gtgagactat 68160
gaaaaatgtt agagacctag taagagaagc agattcacat ttctgtcttc ttcttcaagc 68220
caaacagtca tagagtggag tgggcagaat ggaactcact tttgaaagcc tagtgctttg 68280
tccaatctta ctgcaagcca gacaggaagg ttatagaaaa tgtttctgga tcagtcttct 68340
ctgagtcata tgaaattgtg gtttcagcca agatgacatt aggaattaga gacatgggac 68400
aaaaacttta agattgtaaa aaaattttga ctctagtagg aaacatgggt agaattgtaa 68460
tgacacttga ttgaatttta aaagatgcct gtataagatc ttaaaattag gaaaaaaatt 68520
atggcctaag caattaaagg cataggaggc atctttttgg gatgatggaa atatcctctc 68580
tcctgattgt gatagtagtt acatgaatat tcatttaaca aaaaccataa attatagact 68640
tagaaaacag taaatgttac tgtatgtgac accttaataa acgtgattat aaaaataaat 68700
cctaagcatc taaaaaaaaa aaaaaaaaga agaagaagtg aaccagaacc acaccattct 68760
attttggaga cacttcaaaa gaaatgacct cattcttaat tttgtttaaa gaagaatata 68820
acatgatttg aatatattta gctaggatat tttagtgcct gctagcactt gaagccagag 68880
ttcactgtga gcattctgac tatgaagtga gaagctaaga gaactgtatt ttgatattcc 68940
tttgacagtt aaatcataac actgttcttc cccttcttta gccccagcat gagaccagat 69000
gtaagctctc ctccatccag ctcctcaaca gcaacaacag gaccacctcc caaactctgc 69060
ctggtgtgct ctgatgaagc ttcaggatgt cattatggag tcttaacttg tggaagctgt 69120
aaagttttct tcaaaagagc agtggaaggt agtgtgtgtt ttgaagagtt tatttttcct 69180
ctacttggtt ttcatttctc agggtggatt ttgaaatttc cattatatgc aaagcccatg 69240
aaaggctaaa tatcagttaa gaggggagag gagggtggct cctaggtcct ctaatgggca 69300
ggaaagtatt taaaacgaca atacaaaaag atctagaata aaatagaaaa gtacaagttg 69360
atgtctggga gtttggtcag ggagcataag gtaacactat aagaaagtgc tatcatatga 69420

aatgatggtg ttaagtttgg gcataacata atgttcattg tattagaaac atggctttta 69480
acttccataa gctaataggt ttcaaagtca ccaactttac tggcctggca aaaatgagtc 69540
acagtgagaa ctgtgacagg aaaaaaaaaa gatattcatt tcatttctta ttcatttttt 69600
ttttctatta agccagggca ctgtgctaag tggtataaat accaataaga cctgatcctt 69660
accctctggg aagtcacact ccactgaagt gaaagatgag ttaacaatga caaggtacag 69720
agattataat atagatgagg gagagagaaa ctcggcctga ggaggtcagg aaaggtattt 69780
tagagaaact gatttcacta tataaatgtt gtattaacac aaatcttact ttgttatgga 69840
ttcagactgc tgacagggca acagcattat ctccctaaag aatgagaaat tcattccata 69900
gcaaatttat tagaagagag tctaaaatgt cctaatacta ccagtgactc ctctaggaaa 69960
aaaattgtca tataatttag ttatttctaa agcagtttga aagtagcttg gcctaaagct 70020
ctgattatat taatttttta aagaaacaat tattcattca ctgtatgagg attattatta 70080
tttgtctcat gttgtgtttg catatccatg agagttagat gagtcatttt cttttgtttt 70140
actttttaat acattagcaa attataaaat tactcatatt acaccacaaa gattacaagg 70200
atggcagctt tggccagtgt agtagtccca cctattgatt agagtcaaaa gtaaagccca 70260
gccctgcttt gtgcattgct cctaataaag tggatgttac ttaacacata cgcagaagac 70320
agaagcgtct tcgtgtcctc actttactcc tcactttctt aactgcttaa gtatttccac 70380
gatataaatg cagtgataat aataatacgg acagtccctg acttaacgat ttttcaactt 70440
ttatgatggt gggaaagtga tacgcattca gtatggctcc tcgacttaca atggggttgc 70500
ctccagataa acccattgtg aattgaaaat atcttacact tagcactcca ttcttaatac 70560
ctgctagaat tatagattat ccctcaaaat tggcatagta taatatgggt atcagcaagt 70620
tgttgcactt tattcagagc tttacactag gcaggggtgg gctttacttt tgactctaat 70680
caataggtgg gactactaca ctagccaaag ctggcatcct tgtggtctct gtggagtaac 70740
gtgagtagca ttataattta catcccccat aacaaatgat ccaagagagt atgtgatcaa 70800
tgcagcagaa ctattgtctt ttattatctg atttcacatg taacatgcca tcacttctgc 70860
catattttat tggccacaca gaccaatctt ggtaaaggac ggaaagggac tgcacaagac 70920
catgcattca aggaggcaga gatcactggg ggccatcttg ggaggctggc taccacaccc 70980
accataaata gaaaaccaga attatttgcc aaaaatagac tttaaccaca aaaatgaata 71040
ccatataaac aaaacaaagt cacaaaattt cagctgactt gaagactcat ctttctatta 71100
gttagaaagg gaatttacca agtagtagaa gacacaggaa ctccaaaata agatatctca 71160
ttgtcttatc agaagggttg acaggaaaat gggctgggca ctgtggctca aggaaaatgg 71220
gctgtgcact gtggctcaca cctattatcc cagcaatttg ggaggccaag atgggaggat 71280
tgcttgaggc ctggagtttg agaccagcct gagcaacata acgagacccc gtctctacag 71340
gaaaaaaaaa aaaaaaaaaa cgttatccag gcatcgcacc tgtagtctca gctactcagg 71400
aagctaaagc aggagattca ggctgcaaag agctatgaca caccactgta ctccagccta 71460
ggcaacgtag caagaacttg tctaaaaata aataaataaa tgagtcaagg aatgaatgaa 71520
tggattgaca ggaaatgact attagttgta cgtggccatg tgttatgaaa tagtgaatac 71580
tagttaaaac tcctcatttt atagataagg aacagataga tagacttgtc caacttcatg 71640
ctaataacca caaagggcta tttttaactt atgaaggtac attgcctctg atcctatagc 71700
tcagagtctt agctgtgcac aagacatacc tgggataaag aaatcaagat tggcgtaatg 71760
tgcacatcct gacatttcag ttggatataa acaaaacttt ggaatttttc atttttagca 71820
gtgggtgatt ttttttcttt ttttcttcca gtaactgtag gacagtgatt tagagattcc 71880
ttatagggta taactttttt gtattataac cacttcatca atagatgtat ctgttgatcg 71940
tacttttgat ttatagggga tagaattggg ttagtgcttc cattttctgt ccaagtaaag 72000
aagctaggat atttatagag tacaaaaaga aattgaaaca gctggtacag atatttggca 72060
ttggagagca gctctgaaca aaggtgaatt atagtctagt ggtcaatttt gtggcctgtt 72120
ctttacaaag aattgaacct gatacagtta accatctacc ccaaactatt atttgtttaa 72180
aacacaatct attggctggg cgtggtggct catgcctgta atcccagcac atcgggaggc 72240
cgaggcgggt ggatcacgag gtcctgagat cgagacaatc ctagccaaca tggtgaaacc 72300
ctgtctctac taaaaatata aaaattagcc aggcgtggtg gcgtgcacct gtaatcccag 72360
ctactcggga gtctgagcca ggagaattgc ttgaacctgg gaggcagagg ttgcagtgag 72420
gtcatgccac tacactacta cactcccagc ctgggcgaca gagcgagact ccatctcaaa 72480
aaaataaaaa taaaaaaaca taatctatca aactgtgtaa aacacagttt atcaaaaaag 72540
tagttaccct tggtgggtac tggctggaat tgggcagaaa gggggcctgt tggggtactg 72600
ttctgtttct tgatctgaga gctgattaca taaaggttct tggtttgtaa aaatttatta 72660
aatggttcac tgatttgtgt actttttta tatgtgaata ctgcaataag gtttttattt 72720
gcactgtttt cagtttgttg aacagaaaaa gggagactct ttttgttgtt tttgacctct 72780
cgacctcata atggcaatgt aggcaagaac attccctcaa ggcaatacct gtgggtgtct 72840
tggttatatt ccaccggaaa caaagacaga ggctgtcctt ataaaatatg tttgaagacc 72900
tgtgaaactt taatagtgcc ttttattcca tataggacag cacaattacc tatgtgctgg 72960
aaggaatgat tgcatcatcg ataaaattcg aagaaaaaac tgcccagcat gccgctatcg 73020
aaaatgtctt caggctggaa tgaacctgga aggtaatata aatatctgaa agcaattgtt 73080
tgtctctgta gcttataaaa atttatcatt ttacttttga agatacacgt aagcagatgt 73140
aattaatgta gtcagttcag tatatatatg cttgactagc ataatgttac tgcccaataa 73200
aaatggaaaa tttttttcat gaatatgtca tattgtttgt ttatccacca gttcttctta 73260
cacacactga attcagtaca gccagactat atacaaagaa aggaaattat gtaataatga 73320

aacttacaca acatgcagca actttattat tcttactcct tttttcagcc tcaaaactat 73380
tccctagggt tggaaatgtt tctgtatcag acatatttac atgtccattt ttctgtttgc 73440
cttttaaaag catacctttt acttggagat ctgtgtttta ttacagatct tcaagcgggg 73500
ggtggtggga aaaaaaaaac ctcaaggaag aactggatgg gttttgtttt ggttttcaag 73560
taaagaagaa acctgggccg ggtgcagtgg ctcacgcctg taatccccga agtttgtgag 73620
aatccttctg tctagttttt atgtgaagat attacctttt ccaccgtagg cctcaaagcg 73680
ctccaaatat ccacttgcag attctataaa atgagtgttt gaaaaactgc tcaatcaaaa 73740
gaaacgttca actccatgac ctgaatgcac acaacagtga gaagtttctg agaaagtttc 73800
ttggtctccc cgcactttgg gagaccaagg caggcggatc acgaggtcaa gagatcaaga 73860
tcatcctggc taacatggtg aaaccctgtc tctgctaaaa acacaaaaat tagcggagcg 73920
tggtggtgtc acctgtagtc ccagctactc aggaggctga ggcaggagaa tcacttgaac 73980
ccgggaggca gaggttgcag tgagccgaga tcacaccact gtactccagc ttggcgacag 74040
agcaagactc cgtcttggaa aaaaaaaaaa aaaaagaaac ctgaaactag ttataagtta 74100
gagtttcata tccctgttta tataacaagt tgtataatta acactgatct cagcattaaa 74160
aaattttcct ctgaaaaaag tttggaattc tgctgtggtt gaaattgcaa gttctgtgaa 74220
ggtagtggtg atctcataac acatatgctt agtatttatt gtgaaattag cacttttatt 74280
caacaaatat gcaccaacaa ggcagtcact aggtataaaa tgaataaaat agtgcctgta 74340
ttcaagtagt ttatctgcta gttaggttgc agagtcagtc acaaaatagc gtggcacacc 74400
atagagggca tagggccaca ggaacaagag gaaggtcacc taattctgtc ttggaagtca 74460
aggaagaagt aacattgaat tttaaatcta taagctgagt aggaattaga tagatgaaaa 74520
ataagggcag agacatgatc agatttgtat tttacaaaga ctaatcttac atggagagac 74580
caattaagtg aatatggcag tcctccagat aagagatggc agtactgaga gagaatggaa 74640
accatgtggt tccttttatg attatgatga ttattattat tttagagaca gagtctaact 74700
cttgtcaccc aggctggagt gcagtgacat gaacatggct cactgcagcc ttgaactcct 74760
agactcaagc catcttccca cccagtaggg ctacggatgt acactaccat gcccagctga 74820
ttttttttta atttttgttt taattttttg tagagacaaa ggggtcttgc tatgttccca 74880
ggctggtgtc taactcctgg ccttaagtga tcctcccaac gtggcctccc aaagtgctgg 74940
tattacaggt gtgagccact gcaactgacc tatgtggttc ttttgatagg agagactaat 75000
tgttggtgct atctagcaca cactgtgtgt agacatcttg ttaaatagaa aatagattta 75060
tgggtatgac tatgaagagt ctaattcccc aaaccacaca cacaactcta tctacgtttg 75120
accaggctat ttaaacttaa ctgcagagtg tcagcatgtt aaacattgat ttacataaaa 75180
tgatagctgc ccactttctt gtaaatgtta taaaaactgc agagattaac taaaaaatgc 75240
acacagaagt ttgctttcag ttccacaagg gtagtttatt tttgttataa aaacagtatt 75300
ccccactttc ttagatacca gatctctgcc cagattttac ccagtttcat cttgctgctc 75360
tctaatctcc tatgtatgta atatactttg accatttaaa tatgtattaa gacacttgag 75420
tttttagtgc cctttggttt attttctccg gtcccaatta tctctaatct tcattttttc 75480
attttaccta ttttatattt cgaaataggt tttgaatgaa gctcaaagga caaacccaaa 75540
taaaattctg tcgtatctct aatatattgt ggttgcttac ccagtaacat ttttaggtgc 75600
ttttctgaat acatataaag tttaagatct ttggagtttt aagtatataa tgtttttctg 75660
ggcaatttct ccctatccaa actatgaggg ccttctttca tcaaaagaaa aaagatatat 75720
caactacaaa gtaatgattt tgatggacta ggctacgaaa tctgtccatt ttttcctcct 75780
tcttacagtt taatagcaat tgcagtgccc tttgccctta ctgtactaga agacgacccc 75840
aggcagtgac tgacatctga tttttctatt aattatacca tcactgccat ttccagttga 75900
atcttttgtt ggacatcaga aatttttctt acatgaataa aatttaagca tacggttggg 75960
cgcggtggct catgcctgta atcccagcac tttgggaggc ctaggcaggt ggatcacgag 76020
gtcaggagat cgagactatc ctggctaaca cggtgaaacc ccgtctctac taaaaataca 76080
aaaaattagc caggcgtggt ggcgggcgcc tgtagtccca gctactcggg aggctgaggc 76140
aggagaatgg catcagccca ggagttggag cttgcagtga gccaagatcg cgccactgca 76200
ctccagcctg ggcgacagag cgagactccg tctcaaaaaa aaaaaaaaaa aaaaaaaaaa 76260
aaaaaaaaaa atttaagcat acaatttagg ctgcagtttc tcaaaatatt gtattaaaaa 76320
taaccaatta tatgctttta tagtcagtat aacgtatcca gttagtgtag aaattggcat 76380
ttgttgaaaa ctactacatg ttagtctttg atatacattc ttctactttt tggaccctga 76440
ttattaaaaa cacctttgaa tagggccatg atttacttta tatccatttt tatactacat 76500
agtggaagaa aattctgatt tgttatttcc tactatgata tgtaccgtgt ggcacatatc 76560
atataaatga tccaattcta cttgtagatg aattgaaaga aaggcttaaa aaagttctta 76620
gggtttgtgt gtgtggtttc actgtaaaac tatcattttt gtattgaact aacctcagta 76680
tacataaaat ctttatttgg cctggtatgt acgtatgcca ggaatctttg gcagacccta 76740
acacttacaa tacagatgag ccatgtgttt cacacttttt ttttaacaac cttcagaaat 76800
attctcttgt tcatcagagt gcttccccta agccaagcag tttcgatgat agccccagaa 76860
taactttgcc caagtctctc cataaatgta acttaggact ccaagtggtg tatttttata 76920
ctcttgcccc ataccaagta aatctcaaga tttattttaa gggagtggcc ttcactgctt 76980
aaagggccta gcatttaaga acagataaga tttttaatgg tgatcctaaa tgtttttttt 77040
taaaaaactt gcttgttttt ctcttgaaac taaatgtttt tattcacttc attttaagat 77100
atattgtaat caatccaaag tatggcttta tttttagtat aaacagtcaa atgaagctta 77160
gtcttgtggc attgtcagat ttataaccaa atattactga aactaatttt tttaagttca 77220

aaaacccaat ctagtagttt ctctcttatt ttcaactttt attttagatt ctaggggtac 77280
atgtacaggt ttgttactaa gatacattgt gtgatgccgg tgtttggagt atgattgaac 77340
ctttcatcta ggaagtaagc acagtaccta acaggtgctt tttaacctgt gcctcccttc 77400
ctctatcccc cctcttgtat ttcccagtgt ctgttcccat ctttatgtct atgtgtactc 77460
aatgtttagc tcccatttat aaatgagaac atggtatttg tttttctgca ttagttcatg 77520
taggatactg gccgcctgct acatccatgt tgctgcaaag gacgtgattt cattcttttt 77580
gtggccacat agtattccat ggcatataaa taccacattt tctttatcca gtccactgtt 77640
gatgggcacc tgggttggtt ccatgtcttt gctattgcaa accatgctgc agtgaacata 77700
tgggtacatg tgtctttttg atagaatgat ttatttttct ttgggtatat tcccagcaat 77760
aggattgcta ggttgaatgg tagttaaact cttaattctt tgaagaatct ccaaacttct 77820
ttccacagtg gtgtcattgt ggttttgact tgcatttctc tgatgattaa caatcagcat 77880
ttttccatat gtttgttggc cacacgtatg tctttttttg agaagtgtct gttcatgtcc 77940
tttgcccatt tttaatgggg ttgtttttgc ttgttaattt aagttccata taaactctgg 78000
atattagggc tttgtcagat gcatagtttg caaatatttt ctcccattct gtagattgtg 78060
atagtttctc ttgatttgca gaaactcttt agttaggtcc cattgtcaat ttttgttttt 78120
gttgcagttt cttttgggga ttagtcataa attctttccc aaggccaatg tcgagaaggt 78180
tatttcctag gttttcttct aggattttca tagtttgagg tcttacattt acatctttaa 78240
tccaccttac taatttttat atggcagtag gtaggggtcc agtttcattc ttctgcacat 78300
ggatagccag ttatcccagc accattaatg gaatagggag tcttttccct atggcttatt 78360
tttatcaact ttgtgtagat tacatggctg taggtgtgtg tctttatttc tggactctat 78420
tctgtaccat tgtgtgtggt ttttttttac cagtaccatg ctgtttcggt tactatagcc 78480
tgtagtatag tttgatttgg ggtaatgtga tgttgccaac tttgttcttt ttgcttagga 78540
ttgctttggc tatttggggc attttttggt tccataggaa ttttagaatg ctttttgcta 78600
attctgtgaa aaatgacatt gtagtttgat aggaatagtg ttgaatctat aaattgcttt 78660
gggtagtatg accattttaa ctatactgat tctaccagtc catgagcatg gaatgttatt 78720
ccatttgttt gtgtcatctt tgatttcttt cagcagtgtt ttgtagttct ccttgtaaaa 78780
attttaaact aacttagatg cattcctagg tattttactc tttttgtgac tgttacaaat 78840
gggattgcat tcttgatttg gctctcagct tgaacattac tggtgtatag aaatgctact 78900
gatttttgta cattgatttt aaatcctgaa cctttaccaa agttgtttat cagctccagg 78960
agccttttgg cagagtcttc agggttttct aggtatagaa tcataagtga aaagagatcg 79020
tttgattatt tattttccta tttggaagcc ttttatctct ttctcttacc tgattgttct 79080
gactaggatt tccagtacta tgttaaattg gaatggtgac attgggcatc cttgtcttat 79140
tgcattaagg ggaatgcttc cagcttttgc ccatttggta tgatgttggc tgttggtttg 79200
tcatacaggg ctctttatta ctttgaggta tgttccttca atacctagtt tggtgaaggt 79260
ttttatcatg aagagatgct ggattttatc gcaacttttt ctgcatctat tgagatgatc 79320
attatttttt ttgttatgtg gtgaatcaca tttattgatt tgcatatgtt gaacgagcct 79380
tgcatcccag aaataaagcc tacttgattg tggtgaatta actttttgat gtgcagctgg 79440
attcagtttg ctagtgtttt gttgaagatt tttgtatctg tgttcatcag ggatattggc 79500
ctgtagtttt gttgttgttg ttgtttctct accaggtttt ggtattagaa tgatgtttcc 79560
cttgtagaat aagttaggga tgaggccctc tttctagatt gcttttttag aatagtttta 79620
gtaggattag taccagctct tctttgtata tctggtagaa tttggctgtg aatccatctg 79680
gtcaagggct ttttttaatt ggtaggtttt ttattattga ttcaatttca gaactcgtta 79740
ttggtctgtt cagaatttca gtttcttcct ggttcaatct aggcaggttg tgtgtttcca 79800
tttccacata catacttact ccaaataatg gctttatata tacgggggtc agctgaaaac 79860
aaaaatgata ctttcatagt aaactccacc cgccccccca cccacataca cacacacata 79920
aaccctagat tttttaaagc ctttgttcca atttatccat ttcctctaga ttgtctactt 79980
tgtgtgcata gaggtgcttg taatagtgtg aagatctttt tcacttctgt ggaatctctt 80040
gtaatgtcat cttttacatt ttttattgtg cttatttggg tcttcactct ttttttcttt 80100
gttaatcttg ctagtggtct atcaatcttg tttatccttt caagtaacca acttttataa 80160
actaggtttt aagctaatta agatttctct actttcatta agaaggaagt agtgttacca 80220
cagactcatg aacacttctg tggagctcct gtattgactg ctaatcaact atatgctcca 80280
atgggtcagg aatttatata aagttgtatt aactaagttg ctttaaaata gtgattgctt 80340
aactaaatga ttcagttcag ttaactcctt cctgaagata ttttgaaaaa ttaattagta 80400
ttatttcttg ctctagtcag tacagcacag ttgggttcaa ttgtactttc tgagctgtat 80460
tgaaaaacat cagttttctc atttagaact atatataagt agtgagaaat taattacaaa 80520
ctgagtcata gaaaatgttt ttttttaatc ctccagcttg ttactctttc ttccttgttc 80580
taatgtggag taaagaaata tgcattccaa accatttaaa gttatgacta attgaggctg 80640
tcaaagtact gtttcagtgt attgatttgg cacatgtgtg ttctctttta cattgtcaac 80700
aaaagtacat tttatgattt tggatcaaga tttcactgag atacttctgg ttgtttaaag 80760
agtttcttta tgtattggtg tctttccttt ttaaaatttt atcactcctc tattaagttg 80820
tgatatccaa atttaaaata ttctaaaaac atgttctcct gcaagttgag gtaatgatag 80880
ttgttatgtg gtacttacta taatatatgc caggaactgt tctaagcatt ttacatattt 80940
aattctcaca acaaccctat gaggtaggga ctaatattgt cctcatttta cagaagggga 81000
aatgaagagt cagggagtaa cttgcacaga tatccagcta caacatggca gaaccaggac 81060
ttaaatccaa atatgctgat ttcaggtttc tgccctttag tcctatatca tactgtgcct 81120

ccaagagagc atggtaaact aattagcatg gttctatcat gattctgttt ctattttgaa 81180
ctattaataa aaatttttgc aattctcagt taccccattt agtatagaac acaataagaa 81240
tggaaccatt ctattctaac attgtacatt gagatatcgt tcccaccacc atatctgtcc 81300
tccatagact atatggtgtg tcattttaag gacagaggat ctaaaaatga tttttaaagg 81360
tgatttacat ttactcttcc ctttgcaaaa tggtttgcat ccctaataat ttagacaagt 81420
acatttcttc gtgatataaa ttacatttct tgcctttccc tggaattctg agtactttcc 81480
ctctgagaga acaatgtaat tcttatttat ttagtcacta aaataacttc aggagtatga 81540
ataagtctac taaaaagtct acaggatcca tgttgtagtt tgagtagatg gttccatacc 81600
aagtcaaggt aaaagataat ttatatataa tatgaaaatg gctgctttag gtttatagag 81660
taatcaatat aaatcttcct tataaaaggg aaatttccca cttataattt atgtaatgta 81720
aagtttttca tttcatcttc ccaaatgttt ttagtcccac gcagtattta tgttagtacc 81780
tatgtaaagg tgaaaagtga atttttctta ctggtagaac taatactatt tttagcatgt 81840
aatctgctgt catcttccta tctttataag tggctttgaa caagtgtaaa tagtgtaatt 81900
ctcttcatta tatatactac catgatttag attaatctta aaccacagtt tgtaatccgt 81960
tactccaagc ttagattttt ttttcagttt atagtaagag taatttgcct tatataacca 82020
atgaaattgt tgcatttaga gtgaaagtga gataaaaaaa taatttatag aagaatttac 82080
aaaagttatt tactcagatt gttttaacat accgttataa tactttgtat aaggaataac 82140
tctaatgaag tttctggcct atttgtaggc aaaattaatt gggaataggt tcctctggat 82200
cttttgcttt cagaaaaaaa aaagtttttt ctccttttcc atgtcacttt atcataattg 82260
ctaaataaaa tatttctccc atcttaatag ttttagaaag taaaaatact tcttgaataa 82320
actgtgtagc gcagaccttc ccattacagt tcatttctat gtattttttt aaatacccac 82380
agctcgaaaa acaaagaaaa aaataaaagg aattcagcag gccactacag gagtctcaca 82440
agaaacctct gaaaatcctg gtaacaaaac aatagttcct gcaacgttac cacaactcac 82500
ccctaccctg gtgtcactgt tggaggttat tgaacctgaa gtgttatatg caggatatga 82560
tagctctgtt ccagactcaa cttggaggat catgactacg ctcaacatgt taggagggcg 82620
gcaagtgatt gcagcagtga aatgggcaaa ggcaatacca ggtaagatgc aaaacataaa 82680
agagcaacta tataaacctt tgtgttttct tcagcaaaaa cactttggct tttatatcat 82740
cgtgagccca tggcttatct tgtttctctt agttctgggg actatgaagg ggagagtcag 82800
gtgaatacag gtgataggga gtttataata aaacatttac attactccct gcttttcaaa 82860
tcattatgca caggatggta atttcacata ggatgatgta atatcagaat tcaagttaca 82920
agactcactc aaaactcctt ttacactgaa gtttggggaa agaaaatgtt tttagttaat 82980
tccatttgtt ttccttcatt gtgccacttt taaaaatcag gttgtttgta agattggtaa 83040
acatcaagta tgttgattgt caaaatttgt actaaagtag aatgatttta acccttcact 83100
aaatgaaatg ctacacattg aatgtaattt taaagataat tttaaataaa agttacccta 83160
ttggaatttg gtgtggaatg gcagaggtca atgttagtgt cagctctgac tttaaagaca 83220
gggaattgac aagcctgtgt tcacgcaaat agttagggag agagcaagaa agtaacctga 83280
cctcctgtca tccttgtttt attaaggggg aaagaggtgt gaatagcagg gcaaatgttt 83340
tgcttaactc attgattaat acctcaagcc aagattcttt tctgtttttt aaaatcaata 83400
cataatagtt gtacatattt actgtacata tttatattta gggggtacat gtaataattt 83460
aataaaagca tacaacgtgt aaggatcaaa tcagagtaac tgggatatcc atcacctcaa 83520
acatttgttt ggggaacatt ccaaatcttc tcttttagct attttgaaat ataaagtaaa 83580
ttattgttaa ctatagtcat cctgttgtgc tactgaacac taaaacttat ttcttctaac 83640
tgtatttttg cacccgtcaa ccattcccgc ttcatcccca tcaccactat ctttcccggt 83700
cactggtaac cgccaagcca agaattttgg ctattttact atttagttca tgtttactta 83760
agcagacaga ggtgacaaaa ctggcttttt ttttttttac attaaaagct attaaaaagc 83820
acctaggggg ctgggtgcga tggctcacgc ctgtaatccc agcacttggg aagcccaggt 83880
gggtggatca gttgaggtca ggagttcgag accagcctgg ccagcatagc aaaaccccat 83940
ctctactaaa attacaaaaa ttagccgggc atggtggtat gaatctgtat tcctagctac 84000
ttgggaggct ggcactgaga atcacttgaa cccgggaggc ggaggttgca gtgagccgag 84060
atggcaccat tgcactccag cctgggtgac agagcaagac tttgtctcaa ttaaaaaaaa 84120
aaaaaaaaaa aaaaaacaca agagggtttg tgagtcttaa agtgtcagat gacagaagaa 84180
aactgtgtct acctagtatt taatttccat tttctgttag gggtgccctt gttttgacag 84240
ggctaattga tctcattgct ccttggcaat tcccacagag atgatcttct gaagagtgtt 84300
gcctcatacc tttatttctc ttaattcagg tttcaggaac ttacatctgg atgaccaaat 84360
gaccctactg cagtactcct ggatgtttct tatggcattt gctctggggt ggagatcata 84420
tagacaatca agtgcaaacc tgctgtgttt tgctcctgat ctgattatta atgagtaagt 84480
tgtatgtgtg tcattttccc tgtattcata gggtatcttt aaccagctga tgttttcctg 84540
attgactgct attgtgataa ttcaggactg aaacaatcct actaggtatc taggatctag 84600
gcaaactgga aatagagtta tgagtgcttg gggcaggaca agtgtaatgt aaagcaaatg 84660
tacatgtggc attattactg tcccaggaca tgtttgagga tatttaacag catatctgag 84720
gttagtaaag tctgtcgcaa gcaacaagga atcttactgt gatatcattt acataaccct 84780
attccagaaa gaaaaaggag catggtaaaa ctcatgtgga ttcagtgggg acaattgtag 84840
atgaggatat ctaggctgat ggggtgggac atatggaccc agacacaaga ggtatctctt 84900
tgcatggcaa ggctcaccca gtgtctgtgg tttaagaata tgggaacaaa tttgttttgt 84960
ttaactgaga gaagaccaag cctttaagat tttataaatc agctattctc ttatcctcta 85020

agcttattcc tgtgtctgcg aaatacttca ggtgtccatt tccccttacc tcattgcagt 85080
tgtttcctca ctcgttttct ccctccagtg taacgttcat catgttggct aatgtttgct 85140
tcctcaagca cagtctgact gcatcacata tctccccagt acacagattg tcttcagtat 85200
cttcccactg accctccagt acatattctg catgatttca gactttccag aatctgacct 85260
cacttcctct cccattgttt tccttcacac actcttcatt cccatccatc ctttccagca 85320
tactcttaga ctcttggtgt tcacatcacc agatacacag cagagaagtc acatcctagt 85380
tactctcact ttctaccttg tattactact tttcgtaccc ctagcttatt gctattagta 85440
caatgtaaac agggagttca cacacacata cccctggtct aagaagaata aaaaatgaag 85500
gagatttctg tttgtataga aaacagaagt caccttgact tttattgcca aaaagaggac 85560
tgttcaaact actgcatcac aatgtaacaa gattaggtag ttggatccaa ttttaaatta 85620
actggtaaat atatttagtt tctggggaaa ctgaagacat tattactcat cataatccta 85680
ccatgctgtt taaaaaatac catgttggca gtatttgttt tttagtcact ttctaatatg 85740
taatttgaag gcatttaagt ggaattaaaa gcataaacag atttgtatga aacaccaact 85800
tatcctggtt tataaaacta acctaattta gggtttttat tattagggca ttcagattta 85860
gctttaagca gtcacagcaa aatctaatca tgccacatac attccttaca taaagtggga 85920
tttataattt tttttcctca acagatttac attagtttca ttttcattaa gggatatgta 85980
cttcctattc ttgtgttctc atgctgctgc ctaaaagatg ggcagtcctc cacctttttc 86040
ttttcttttt tttttttttt tttttttgag acgagtctta ctctgtcacc caggctcaag 86100
tgcagtggtg tgatcttggc tcatggcaac ctctgcctcc agggttcaag tgattctctg 86160
cctcagcctc ccgaatagct gggattacag gcgcactcca ccacacttgg ctaatttttt 86220
gtatttttag tagagacggg gttttgccat attggccagg ctggtcttga actcctgacc 86280
tcaagtgatc cacccacttt ggcatcccaa agtgctggga ttacaggtgt gagccaccgc 86340
acccagccct ccaccctttt ttcttagccc actatgtttc catactgctc tggtgtctgt 86400
gacaggcaga tattgcatat cagaaagtat gcattcaagt tctgaccctc tatagagctg 86460
tcaaacagtc tctcatggtt gcccttaggt cagaacgttg tgggggaaaa aaaaattgtt 86520
gtttttacag ccaacaagaa tgagttttta cttattctac tacactataa ctttgttgaa 86580
attttcagtt atatgagtat aaccatgtac aagaaactaa aggaaaaaaa ggtgcctccc 86640
agaaaaggag tgctttacct actattaagg actagggagg tgcctcttcg gtaagagcag 86700
attttaaatt tgaagagcct ctgatcactt tggcagcata taagtcatgt ctaatttatt 86760
ttatataaag gaataaacca catattcagt agagaaaaat aataaccttt ctgttgttaa 86820
gtccaagacg actttctgtc agaaacttaa aaaaaaaaaa aaatcttgaa gcattttaaa 86880
agctgtgaac tgggcccagt ttcaggctct tagtgtcatt tcacaagtca ggaaacttta 86940
gagacctatt tgaaaatcat aggtatgtaa tgacttcaga atcataagca agaattggtt 87000
tagtaccttt agtttaaaga atattaaggc atatgcctgt cagaggcaga ttttgagcat 87060
cagaagtcta gaatcaagtt ctaggtctcg ccctctgcat aactgtgaac agtgtcacac 87120
atttttgtct ttaggatgga ctgctgtgaa aaaatttacc tttaaaaatc aagtgtgtag 87180
gacctaaaac tgtcgtctaa ttgaccgtat tcaaatgata aaccttgatt taaatgagca 87240
actagtaata agttctataa gaattctaac actttaatta aataataaaa taatacatgg 87300
catgcatgat agaaaataat atctccactg ttacattaga ttattcatta gtctatttaa 87360
acagccaaga tgcaggaagt ttaaggaaag ttctccaaaa ttctgatttt atagggaatt 87420
agcaataata ttattgcagt agttgttttt ctttatgagt tcatagtttt gcaaaacaaa 87480
acaaaaatgt gctttttggg gggaagtagc agtatttcta actaataccc tgctatttat 87540
ctttcacagg cagagaatga ctctaccctg catgtacgac caatgtaaac acatgctgta 87600
tgtttcctct gagttacaca ggcttcaggt atcttatgaa gagtatctct gtatgaaaac 87660
cttactgctt ctctcttcag gttggtagaa caccttttca ccttatgtca aaagcatgaa 87720
atatgaaggc ctagaaacaa aggttaattt atatacatag tactaataat tataccaagt 87780
ctactattat ttcctactag tcagatgatt tttatgaatg taaaatatta gaaaggcaca 87840
gtaagtgaca ccaagattaa taagacaaat aggtatggca gaaacagaga ggtatatgag 87900
ctgcataggg atctctgttg ataagaatct gtgtagactt ttttctcctt ccttcctttg 87960
atctttgatc atgggaagac atggaaaaag aaagctaact acagtgattt tgtctactac 88020
actgttattt ggttaaaaat tttagtttcc taatgagtat tagcatgtat gagaaattat 88080
gggagaaaaa ggcgcatcct agaaaaggtg tgcttaatta ctattgggga ttggttaaca 88140
tagcatggga gctggattgt cagagattca ttatctagaa aatggcaaca agagtttata 88200
aaacgaactt ctgtgagatt actttttagc tagcaaagac aaagatgtcc ttcagtaggt 88260
gaagtgataa actatgatac atccagatga tggaatacta ttgaggacta aaaagaaata 88320
agctgtcaag ccatgaaaac acatggaggg acgttaaatg catattacta agtgaaaaaa 88380
gctaatctga aagggctaca tactgtgtga ttctaactat ataacattcc ataaaaggca 88440
aaactgtgaa gacagcaaaa aaaaatcagc ggttgccagg gtttagaagg aagggaggga 88500
taaatgtgca gagcacagag gatttttagg gcagtgaaaa tacttcgtat gatactacaa 88560
tggtggaaac atgtcattat acatttatcc aaacccaaag aatgtccacc accaagagtg 88620
aaccctcaac tatggacttt gggtgatgat gtgtgggaca ggaggtatat gaaaaatctc 88680
tgtaccttcc tcccaatttt gctgtgaact taaaactgct ctaaaaaaag tcttttttaa 88740
aaaaagctct atgaactagt tggtattata aaccttaggc catttcaagt aaaaattaca 88800
tatcaatgtt tattaaatac tgagttaata gctgaatacc tctttcatat acaaataagt 88860
acatttgcaa ttttttaaaa agtcttaatt ccattagtaa ctgtggtttc atagttgcca 88920

aataactgta agctatggat gttgcacaag actgtgattt tatttaatca tttcatatct 88980
atttaaacat ttccaaagcg cacattcatc ttaatgtttt cacactattt ttgctcaaca 89040
aaaagttatt ttatgttaat ggatataaga agtattaata atatttcagt caaggcaaga 89100
gaacccgata aagatcattg ctagagacgt ttaatgttac ctgtagcggt acacttgtta 89160
aagaagtgat taagcagtta cataaaattc tgatcatagc tttgattgat accatgaagg 89220
tataattcag tgcctggata ctaacaactt tacttgttta aaaaaaaaaa aaaaagaatg 89280
gtttcaattg tatacatccc agactaattg agctatatga tttttttcat tgtaaataat 89340
atcacgagtt cttcttgtta aaaaataata gaatcataag gatggaaata tataccttaa 89400
gatatagact tctactatga tagactactg gaataggtat ataacctccc accaaaaatg 89460
ctagactaaa aaaattaaga actaagtgaa ggcaggaacc tacagagata agtggaactc 89520
aagccaactt gctctttgac ggcatttgta gaacctggta aattagtaag tttagtaagt 89580
tggggttttt ttaagtttat aatctttttt aaaatgattt caataggttt ttggggaaca 89640
ggtagtggta ggttacatga ataagttctt tagtggtgat ttctgggatt ttggtgcacc 89700
catcacccga gcagtgtaca ctgtacccaa tgtgtagtct ttcatccctc atcccctccc 89760
caaccctagt ccacaaagtc cataatatca ttctcatgcc tttgcatctt catagtttag 89820
ctcccactta gaagtgagaa catgcaatat ttggtttccc attcctgggt tacttcactt 89880
acaataatgg tttccagttc catccaggtt gctgcaaatg ccattatttt gttccttttt 89940
gtggctgagt agtattccat ggtatatata taccacattt tctttatcca ctcgttgatt 90000
gatgggcatt tggactggtt ctgtatattt agtaagttta aaaacaaggg atggaaatat 90060
aaatgcagtt gaaaaggcag tggatggatc taaaagcaga agaatacaat tgtttttaat 90120
gattgtgtat atgtttgtgt atataaacca caagggaaat ctgtaggtac tgaaaatcac 90180
aacaggaaaa tggcaacaaa gctatagaaa ctggaaaagc aatgactttt cttagatccc 90240
tcagagaatg gaggtcatag gacaaaccac cacttcaaaa tctagaagaa tagacaaata 90300
cagagaaaca gccaagatca gcttactggg aaaagatgcc actgaagcca ggaagactat 90360
ggcaatttgg gaaaagatgc cactgaagtc aggaagacta tggcaatttt gatgaattgc 90420
tggaggctga gtgaggacta gcttcagagt taaaaactcc cagggaccca gtcttagtgg 90480
gggtttcctg caatttcttg ggtttacccc acaaaatttc taacttccag aaactccaca 90540
aggttcttat ggtgaagatg caagaaaaat tccctccttt ttctggtagg agtagaggga 90600
aggtaaaatt tggaaatacg tagcagagtg ttcacaacaa aaggcctgcc ctgtaaggaa 90660
aactaattca acaggccctt atgtgacctg ggggaaaggc aaatagagga ttctagccct 90720
tccttagcct tcttgtctca tttctgaaag tcacagccca gggattcaga cccactaaaa 90780
aaaactgaga tttaatcata aagattaaaa aacaattccc ctccccctcc ccaacacctt 90840
accaccatat aaacagggct ccaggataaa ataacagtgg attacaactg agagagctgc 90900
aagacacaag ctgtttaagg agctcttagg aaacccaaaa acaacagaag aaaaagtaaa 90960
taaaaacaag gaaactagag gaaactgaag cctccagtac ctacaattat ggcaaacatt 91020
aaatacagcc cagctcctag ccagattagc atgaaacctc acactaaaag tctaattact 91080
tcagttttga tatatcaatc atgtccagct ttcagcaaaa aaactacaag gcatgctaaa 91140
aggcaagaaa aacccacggt ctgaagagac aaaacaagca tcagaagcag tcctcagata 91200
tgacacaaat atttcaatta tcagataggg aatttacaat acctatgatt agtaggttaa 91260
aggctccaat ggaaaaaagt agacaacatg caagaagtga tgtacgcaga gagatggaaa 91320
ctctaaaaat aaatgctaag gaatgctgta aggaaatgca gaatgatgtt gatgggctca 91380
tcagtagact gagcacagcc aagcaaagag tcagtgagct tgaagataga taggtcaaag 91440
gaaattcccc caaactcaaa tgcaatataa acatagtaga cattaatcca gctgtatcag 91500
taattacttt aaatttgaat gctctaagta caccaatcag ctattttttt aactaggagg 91560
tgaaaataaa gtttgccacc agatgctcac taaaaaatta ttagaggata tacttaggct 91620
aaagaaaagt aatcccagcc aggcgtggtg gctcacaccg gtaatcccaa cactttggga 91680
ggctgaggca ggcagatcac agagtcaaga gatcaagacc atcctggctt acgtggtaaa 91740
accccatctc tactagaaat acaaaactta gctgggggtg gtggtgcgcg cctgtagtcc 91800
cagctactca ggaggctgag gcaggagaat cacttgaacc tgggacgtag aggttgcaga 91860
gagccaagat agcaccactg cactccagcc tagtgacaga gggagactcc atcttagaaa 91920
aaaaataata aaagtaatcc catctttaag aaggactgaa gaataacaaa agtggtaaat 91980
aatatagata catttaaact gacatttact atgtatataa aataacaaca gtaacaattt 92040
ccttgagggc taaaaagtag aactaaagta agtttcaagg atgacaacta gaaatagggt 92100
atgcagggta tgcaaagtac caaaccattg gggaagagaa atacctaaga aaaacaatcc 92160
aaaagaatga aagacatgag aggagggaga aaaaaatgca taaacaaggg catgataaca 92220
ggaagtaaca gataaggtac attagtacag ctaaattcaa acacatcagt agtttagttt 92280
cattaaatat agagatgggg ccaggtgtag tggctcacac ctataatccc agcactttgg 92340
gaggctgtgg gcagatcact tgaggtcagg agttcgagac cagcctgacc aacatggcga 92400
aaccccgact ctactacaac tataaaaagc cgggtgtggt ggtgcatgcc tgttatccta 92460
gctactcggg aggctgaggc acaagaatca tttgaacctg ggagatggag gttgcagtga 92520
gccaagatcg tgccactctt ctccaaactg ggtgacagag ggacactgtc tcaaaaataa 92580
aataaatgta gagatggact gaatgctcca agctaatctg acaggatttt agaaataatc 92640
caaatttatg ctatttaaaa aaagctatat ctgaataaag atattgaaag gctgaagtaa 92700
aaggatctac tttgcatagt ataacccaag acatggccaa ctttttctgt aaagggccag 92760
atggtaaatg ttgttagctt tgcacagtct ctgtcacagc tactaaactc tgcccttgtg 92820

gcaggaacat agtcattgac ggtactcaaa tagaacaggc atggctgtgt tccaataaaa 92880
ctttatttac aaatacaggc tgcaagtagg atttggccca taggccaaag tttgctggcc 92940
cctatattga ccaaaacaaa accgaaggag ctacattatt accaagcaaa atagatgtta 93000
aggcaaaata ctccttaaag catttgttca ggaaaaataa ttgtaaatat atagtttcaa 93060
attacataat acaaaaattc atagaacaag aatacttaga taaatctagt aaaaataatg 93120
agattttact atacctttct tacaaattaa gcagacaaaa aaataaggat atggatgtac 93180
atttcatctc tcttgggtca atactgaggt gtgagatcac tgggacatag gttgagtgtg 93240
tgtttaaatt tatttttaaa attgccaaac ttttccgcaa ttgttaacat ttaccagaaa 93300
tgtatgagac ttcttaagat ccattctata tcctcctcag tacttggtac tgtcagcctc 93360
tttcatcgta ggtatactga tgattaaaaa tattaagcat cttttcatgg gcttattggc 93420
cacctatatt tcttatttgg tattgtgcct cttttaatct tttgcccatt ttttaactgg 93480
gttttaagaa ttgttcaaat attctcaatg tggccctttg ttaaatatat gttttgcatg 93540
ttttctttaa gtggattaca tttacagttt tcttaaaaaa atgtagagat gagcaaaagt 93600
gtataatttt gaagaaagct tcgtgtcttt gtttactaag aaagttttgc ttaatccagg 93660
gttaaaaaga ttttctacta tttgttttct tatagaaatt ctgtagtttc agctcacatg 93720
cttaagtata tgatgcaagg taagggacaa ggttcatttt cttccccaaa atccatatct 93780
ggttgctcca gaacttgact ctcttttccc tattgagtta cttggcaatt ttgtagaaaa 93840
tcagttgttt gtatatgtgt gggtctactt tcagactctt tttcttaccc aacgatctgt 93900
atttcttacc caatgatctg tatgcctata ttcatattga taacaccctg tcttgattac 93960
tgttgcatta cagtaaatct tgaaatttgg taatatgaat tctccaaatc tgttgttctt 94020
ttccaaactg ttgttttgga tattctagtt tccttgcatt tccacttcct tttttttttt 94080
tttttttgag atggagtctc actattgttg cccaggctgg agtgcagtgg catgatcttg 94140
gctcatcgca gcctcagcct ccccagcagt gggattgcag gcacccacca tcatgcttgg 94200
ctaatttttg tatttttagt agagacgggg tttcgccatg ttggccaggc tggtctcaaa 94260
ccctgacctc aggtgatcca cccacctcgg cctcccaaag tgctgggatt acaggcatga 94320
gccactgtgc ctggtcttcc acgtattttt taattagctt gacaatctct accaaaaagt 94380
cttttggggc tgggtgtggt agttcatgcc tgtaattcca ccactttgag aggccaaggc 94440
aggcagatcg cttaagccca ggagtttgag accagcctgg gcaaaatgtc gaaaccctgt 94500
cactacacaa aatagaaaaa attagccagg catggtagct tgtgcctgta gtcccagcta 94560
cccaggaggc tgaggaggga ggtcaaggct gcagtgagcc atgatcatgc cagtgcactc 94620
tagcctgggc aacagagtga gactctgtct caaaaacaca gtctgataga atttttatta 94680
ggatagcctt gaatctatag atccatttga aaataattaa catcttaaat ttccaatttc 94740
tggccgggcg ctatggctca cgcctgtaat tccagcacgt tgggaggccg aggtgggcag 94800
atcatcaagt caggagttcg agaccagcct gaccaacatg gtgaaaccct gtctctacta 94860
aaaatacaaa aaaattagcc aggcgtggtg gcacatgcct gtagtcccag ctactcagga 94920
ggctgaggca ggagaatcgc ttgaatctgg gaggcagagg ttgcagtaag ccgagattgt 94980
gccactgtac tccagcctgg gcaacagagt gaggctccgt ctccaaaaaa aaaaaaaaaa 95040
attccagttg ttgagaaaga ataggaattc cagctttgga ggagtgggga gaccatcaaa 95100
tcctctttcc aaaaatacta ctaaaatact actgagcaga gtatagttcc acaaatagtc 95160
ttctgtaaag agactcacag tacatatttg tctttgtagg ccatatagtc cctgttgcaa 95220
tttctcaatt ctacagctat aacaggaaag cagctatata cagtatgtga atgcttgtgt 95280
tctaatacaa atttatttgc aaaatcagga aaatggcttg aaatggttta agatctagtt 95340
ttctgactag atcatggtat ataatctttt ccatatatat tttgaatttg gtttgctaat 95400
attttgctga tcatttttat atctctcttt atgaaggatg ctgatctaca actttctttt 95460
cttgtgatat ctttttctgg ctttgctacc agggtagtac tagcctctta aaatgagttg 95520
agaagtattt tctgttttct taaagagttt atagagtatt gatcttattt attctttaaa 95580
tatttgatac atgttaccag tgaagccatc tgggtctgtg ttttctttca gggaagattt 95640
ttaattattt gcttatttgt tatatagatc tattcagaat ttatattttt ccttgacata 95700
gttttgtaat ttgtgtgttt ctatgaaatg agccattttg tctgagttgt ctaacttggg 95760
cataaagttg tttgtaatcc tttaagtttt gtaggatcca tagaggtgtc ccctccatta 95820
tagattttca taatttgtgc ctgatcatct ttttttcatg gtcagtctag ttaaaaattt 95880
atcaattttg ttggtcttta caaagaacca atttttagtt tcattgaaat ttttagtttc 95940
attgattttc tctttttgtt tcctatgtca ttgattatta tttcttcttt tctgcttgct 96000
tttcatttaa tttgttcctc tttttctagt ttaaggtaga agcttccatt gttagttgaa 96060
gaccttattt tcttatatag atgtttaaag ctatacattt tttgtatatt ttcattcatt 96120
tcattttcta atgtccttca tgattttttt cattgaccca tgtgtattgc ttaattttta 96180
tatatttggg gattttccat atctcttcct attcatttct aatttaattc cactgaggta 96240
ggaggtacat tgaaggactc taatattgaa tgactccaat aagtcttctg agactttttt 96300
aggcacttgc atatggtcta tcctgagtgt tccatgagtg cttgaaaaaa aacttactgt 96360
gctcttgtta agtagagttt tatgaacgtc agttaggtca agttgattga tagactaatt 96420
caagttttct gtatctttgc tgattttctg tctagttgtt ctagatccta caactttgtc 96480
tacatccttg ccagagcttg gtatggtttt tttattatcg ctatcctaga gagtatgtag 96540
ttgacccttg tgacttgcca tgcatttaat gactgcccat gttcatagca gcattattca 96600
taatagcaaa aaaaactttt atcatatgct tttgtgcctc aagatcatat atttttcgtt 96660
tttagtcact aatatggtat aatggtataa tatactgttt aatttctgag taattgacta 96720

gcctttcatt ccggggataa atcctatttg gttatgatat agtatccttt ttacatatag 96780
ctgaattcat tgtactaaaa ttttggtatt tttgcatcta aatccatgag ggatatattc 96840
tatagctttg gtgttatgat aatatggtat tatttctttc ttaaacgttt ggtaaaactc 96900
agcagtgaag ctgtcttggt ttgtttggag ccttttttgt agaaaggttt tcaagtacaa 96960
gttcatcaaa tgtttactga taatatgttt attcttgagt gagctttgtt ggtttacatc 97020
tttgaaggaa tttaactgtt tccttcaaat gttgaattta ttggtataaa gttaagttat 97080
tcataatatt cccataatat ccttctaatg gctccagtat ctctagtgtt attccctttc 97140
attcccgaca ttggtattta atatattctt gctttttttt ttttttttta atcagtctgg 97200
ctaaaagttt ttcagtttta ccaatgtttt catagaacca gcttggtctt gattttgttg 97260
ttgtttatgc atgttcttag ttattcgttt ctactcttta tcctttccat ttttcttgtg 97320
tttagggtag aagcatatat aattaattga gacctttctt ttctaatcaa agcttttaat 97380
gctgtaaatt ttctaagcac tgtcttcatt gcatcccaca cattttgata tgctgtgttt 97440
tcagtactag agatttttaa ttttatgata ccttatttaa tcatgatgcc ttatttaatc 97500
tatagcttat taaatgtcaa attctaaaca tttgggtttt tctccagata tgtttgttac 97560
tgacttctat tttaatctca tttttgtcag acagcattca ttgtatgact taatcctcct 97620
aaatgtattc agacttgttt tatgttctag attaatgttc tgtgtatact tgaaaagaat 97680
gcaagttctt gggtagactg tttcagaaat gtcagtcaaa tttaagtctt gtttattctt 97740
attgattctg agacaaaggt gtttataatg ttagatttgt ctgctatatc tctgacattg 97800
ccaaatatcc ccttggaggc aaaatctccc cctccctttt gagaaccact gatctatgta 97860
gccttttttc tgggactaat ttagccttgc ttctgagatg tggcccctag gtctctactg 97920
aatgcccggc atatttaatt agatctttct ttcctctatg gcctcaaggg atttcaccct 97980
aagtatgcac aaatttttat tcagccgaag actgtacaga tttctggagg cctttctttg 98040
tgtacctcct tcgtttccag tagtctgacc cataaattgt acagatttct ggaggccttt 98100
ctttgtgtac ctccttcgtt tccagtagtc tgacccataa attaaagctg ctttagcctc 98160
cccaaacttc aatctctttc tcctcaaccc agcaagattg ctagaccctg ggttcccttt 98220
cccttcactg cagtatgata attactttca agcacaaagg tttagaatta agatttctta 98280
ctcctgggct aggtatggct taccgtattt gtttctcttt tcctagggat cataatcatg 98340
tattgcttgt tgtccagttt tccagtagga ggggaattcc aggctgtact tacttcctgc 98400
agccaaaaga ggaagtaatg ttagtgattt caatattaaa acattaaaaa aaaatttaag 98460
atggatgaaa ttcttttata tgcatattga attgggcttc accatagtta tttttagaat 98520
taggactaac cggcagggaa aaaaactata cggcagggaa aaaaactata agccatcgct 98580
gttttacaat tttgcaataa ttagattttc tgtagtatag taatgtgtaa aattaaccca 98640
ttgttaatat agaatgccgt tatcactcct gattaagcgg tcttcatttt catgttaata 98700
ctgatgtctt gtaatgcttt ctggaatcaa acattttcat acatattcat tagtctaatt 98760
ctaatcataa tccaatgaaa aagagcagga aagatgctca aggaggttat attcaagtcc 98820
acatggcaag taagaaataa gactactcgg ctgggcatgg tgacttactg cctgaaatcc 98880
cagcactttg ggaggccaag gtgagcggaa ttgcttgaac ctgggaggcg gaagtggcag 98940
tgagctgaga tcatgccaat gcactccagc ctaggcaaca cagcaagact ctgtctcggg 99000
aaaaaaataa taataataag acttctagaa gctcctaaat ccatagcttt tcctctatac 99060
cagcatcttc taaaaatgtc agcagcagtg aagtttcagt ttgggaaata atgcatttcc 99120
cctctctgga gagtgcacag ttatatctcc aagaagtact gaaattcaga agtctgccta 99180
atatgtatta aacatttagc ttttctcaaa ctttgaccac caaatccttt gtctcgctct 99240
aactatagtt aacacagaat cagtgttccc aggagcacac tgtgaaaaat gtagcactct 99300
acaaaagtcc taatctccac aggattaagt gaaaccatga ttaaccctct gttccttgtc 99360
cttattagta ccattttctg aagagtaatg tatcccccca aaacttttat actagtttca 99420
ctaaccagaa tccatgtaca taaggaagga cagatatttg ctccctacta agacatatct 99480
attagctaca ttaaaaaaag tattgcatgc cgattttaaa gttataatta actggtgata 99540
tcacagatat tccaagatat aattgctgga ataaacactg ttgttgaagc cttctatcta 99600
tctcagtact agaattaaac tcaagtgcag aatggcagac aaagttaact aaaaatcact 99660
gtattatttc atttggtcct ccaaatagct ttgtgagcta aggaggagaa ggtgtatcat 99720
caccacttcc attttataga tgagaaatca agtgatttac tcaaggttaa gtcctccaat 99780
tctttgttat cctgcatttt ctcttggctg tagtttaatt aataatccta agaaaatgct 99840
tatattttag agtgcagtaa gagtacataa acaatgttaa atgcccatct tgcatgtata 99900
aaaagttata gcaagaaatc tggctgggaa tggtggctca cacctgtaat cctggcactt 99960
tgggaggccg aggcaggagg attgcttgag cccaggagtt taagaccagc ctgggcaaca 100020
tagggagatc ctgtctctac aaaaaaattt agccagacac agtggcttgt gtcctagcta 100080
ctcaggaggc tgaggtggga ggatcacttg agccaaggag gtcaaggctc cagtgagcta 100140
tgattatgcc actcagacat ggtggcttgt gcctacagtc ctagctactc aggaggctga 100200
ggtgggagga tcacttgagc caaggaggtc aaggctccag tgagctatga ttatgccact 100260
gcactccagc ctggatgaca cagtgagacc ctatctatct caaaaaaaaa aaaaaaagaa 100320
aagaaaagaa aaagaaaatc ctttaactga cttcatctta accttttagt tcctaaggat 100380
ggtctgaaga gccaagagct atttgatgaa attagaatga cctacatcaa agagctagga 100440
aaagccattg tcaagaggga aggaaactcc agccagaact ggcagcggtt ttatcaactg 100500
acaaaactct tggattctat gcatgaagta agtgtcaaac ataaagccaa atataagagt 100560
tttctgggac aaagtatgtt ttgattagtg aatataatta tataccagca gcgcccccac 100620

ccccgccccc agtttgtgga tgttggtgat agcttgagtt caacttatga acttcagttt 100680
tgtagacatt tttcctaagg ccaattatga aatatccttt cacctagtca tgtgtatata 100740
aaatcaccat gttattacag aatttagtaa cactgttttt aaaaagtatg attaatccat 100800
taaattagaa taatgcaccc ttcatatatt atggtactac agtgattcat gaaataattc 100860
tatataattc tacatacaat caaagaaata taaaatgtgt tttgtacgga agtgcttatt 100920
tttcatctgg ggaattccag tgagattggt atattctagg ccagataatt ttttcaaaat 100980
agaggacaac aaacatgaga tgttcccact gaccaatttg gaagcctgat cattaccata 101040
tcttctcttg caggtggttg aaaatctcct taactattgc ttccaaacat ttttggataa 101100
gaccatgagt attgaattcc ccgagatgtt agctgaaatc atcaccaatc agataccaaa 101160
atattcaaac ggaaatatca aaaaacttct gtttcatcaa aagtgactgc cttaataaga 101220
atggttgcct taaagaaagt cgaattaata gcttttattg tataaactat cagtttgtcc 101280
tgtagaggtt ttgttgtttt attttttatt gttttcatct gttgttttgt tttaaatacg 101340
cactacatgt ggtttataga gggccaagac ttggcaacag aagcagttga gtcgtcatca 101400
cttttcagtg atgggagagt agatggtgaa atttattagt taatatatcc cagaaattag 101460
aaaccttaat atgtggacgt aatctccaca gtcaaagaag gatggcacct aaaccaccag 101520
tgcccaaagt ctgtgtgatg aactttctct tcatactttt tttcacagtt ggctggatga 101580
aattttctag actttctgtt ggtgtatccc ccccctgtat agttaggata gcatttttga 101640
tttatgcatg gaaacctgaa aaaaagttta caagtgtata tcagaaaagg gaagttgtgc 101700
cttttatagc tattactgtc tggttttaac aatttccttt atatttagtg aactacgctt 101760
gctcattttt tcttacataa ttttttattc aagttattgt acagctgttt aagatgggca 101820
gctagttcgt agctttccca aataaactct aaacattaat caatcatctg tgtgaaaatg 101880
ggttggtgct tctaacctga tggcacttag ctatcagaag accacaaaaa ttgactcaaa 101940
tctccagtat tcttgtcaaa aaaaaaaaaa aaaaagctca tattttgtat atatctgctt 102000
cagtggagaa ttatataggt tgtgcaaatt aacagtccta actggtatag agcacctagt 102060
ccagtgacct gctgggtaaa ctgtggatga tggttgcaaa agactaattt aaaaaataac 102120
taccaagagg ccctgtctgt acctaacgcc ctatttttgc aatggctata tggcaagaaa 102180
gctggtaaac tatttgtctt tcaggacctt ttgaagtagt ttgtataact tcttaaaagt 102240
tgtgattcca gataaccagc tgtaacacag ctgagagact tttaatcaga caaagtaatt 102300
cctctcacta aactttaccc aaaaactaaa tctctaatat ggcaaaaatg gctagacacc 102360
cattttcaca ttcccatctg tcaccaattg gttaatcttt cctgatggta caggaaagct 102420
cagctactga tttttgtgat ttagaactgt atgtcagaca tccatgtttg taaaactaca 102480
catccctaat gtgtgccata gagtttaaca caagtcctgt gaatttcttc actgttgaaa 102540
attattttaa acaaaataga agctgtagta gccctttctg tgtgcacctt accaactttc 102600
tgtaaactca aaacttaaca tatttactaa gccacaagaa atttgatttc tattcaaggt 102660
ggccaaatta tttgtgtaat agaaaactga aaatctaata ttaaaaatat ggaacttcta 102720
atatattttt atatttagtt atagtttcag atatatatca tattggtatt cactaatctg 102780
ggaagggaag ggctactgca gctttacatg caatttatta aaatgattgt aaaatagctt 102840
gtatagtgta aaataagaat gattttagaa tgagattgtt ttatcatgac atgttatata 102900
ttttttgtag gggtcaaaga aatgctgatg gataacctat atgatttata gtttgtacat 102960
gcattcatac aggcagcgat ggtctcagaa accaaacagt ttgctctagg ggaagaggga 103020
gatggagact ggtcctgtgt gcagtgaagg ttgctgaggc tctgacccag tgagattaca 103080
gaggaagtta tcctctgcct cccattctga ccacccttct cattccaaca gtgagtctgt 103140
cagcgcaggt ttagtttact caatctcccc ttgcactaaa gtatgtaaag tatgtaaaca 103200
ggagacagga aggtggtgct tacatcctta aaggcaccat ctaatagcgg gttactttca 103260
catacagccc tcccccagca gttgaatgac aacagaagct tcagaagttt ggcaatagtt 103320
tgcatagagg taccagcaat atgtaaatag tgcagaatct cataggttgc caataataca 103380
ctaattcctt tctatcctac aacaagagtt tatttccaaa taaaatgagg acatgttttt 103440
gttttctttg aatgcttttt gaatgttatt tgttattttc agtattttgg agaaattatt 103500
taataaaaaa acaatcattt gctttttgaa tgctctctaa aagggaatgt aatattttaa 103560
gatggtgtgt aacccggctg gataaatttt tggtgcctaa gaaaactgct tgaatattct 103620
tatcaatgac agtgttaagt ttcaaaaaga gcttctaaaa cgtagattat cattccttta 103680
tagaatgtta tgtggttaaa accagaaagc acatctcaca cattaatctg attttcatcc 103740
caacaatctt ggcgctcaaa aaatagaact caatgagaaa aagaagatta tgtgcacttc 103800
gttgtcaata ataagtcaac tgatgctcat cgacaactat aggaggcttt tcattaaatg 103860
ggaaaagaag ctgtgccctt ttaggatacg tgggggaaaa gaaagtcatc ttaattatgt 103920
ttaattgtgg atttaagtgc tatatggtgg tgctgtttga aagcagattt atttcctatg 103980
tatgtgttat ctggccatcc caacccaaac tgttgaagtt tgtagtaact tcagtgagag 104040
ttggttactc acaacaaatc ctgaaaagta tttttagtgt ttgtaggtat tctgtgggat 104100
actatacaag cagaactgag gcacttagga cataacactt ttggggtata tatatccaaa 104160
tgcctaaaac tatgggagga aaccttggcc accccaaaag gaaaactaac atgatttgtg 104220
tctatgaagt gctggataat tagcatggga tgagctctgg gcatgccatg aaggaaagcc 104280
acgctccctt cagaattcag aggcagggag caattccagt ttcacctaag tctcataatt 104340
ttagttccct tttaaaaacc ctgaaaacta catcaccatg gaatgaaaaa tattgttata 104400
caatacattg atctgtcaaa cttccagaac catggtagcc ttcagtgaga tttccatctt 104460
ggctggtcac tccctgactg tagctgtagg tgaatgtgtt tttttgtgtg tgtgtctggt 104520

tttagtgtca gaagggaaat aaaagtgtaa ggaggacact ttaaaccctt tgggtggagt 104580
ttcgtaattt cccagactat tttcaagcaa cctggtccac ccaggattag tgaccaggtt 104640
ttcaggaaag gatttgcttc tctctagaaa atgtctgaaa ggattttatt ttctgatgaa 104700
aggctgtatg aaaataccct cctcaaataa cttgcttaac tacatataga ttcaagtgtg 104760
tcaatattct attttgtata ttaaatgcta tataatgggg acaaatctat attatactgt 104820
gtatggcatt attaagaagc tttttcatta ttttttatca cagtaatttt aaaatgtgta 104880
aaaattaaaa ccagtgactc ctgtttaaaa ataaaagttg tagtttttta ttcatgctga 104940
ataataatct gtagttaaaa aaaaagtgtc tttttaccta cgcagtgaaa tgtcagactg 105000
taaaaccttg tgtggaaatg tttaactttt attttttcat ttaaatttgc tgttctggta 105060
ttaccaaacc acacatttgt accgaattgg cagtaaatgt tagccattta cagcaatgcc 105120
aaatatggag aaacatcata ataaaaaaat ctgctttttc attatgtgac tccaacatgc 105180
ttttgtagaa cttgtacagt tccgattgtc caatctgatt tttgtttact gaaagtagag 105240
ttacccctgc ttcaggaacc ttaagataat atggtgggca tttaaatgtc agtgtggcaa 105300
tgttcgcctg ctaatatggc atagattcaa aataagctta accctggtgc caaagacctg 105360
aagattatcc catccatgcc tcaaatggtt gtgtgccaat tactgcaaag ggtactaagg 105420
gaaggagaaa ttcactcctg aggctgcttc aaatgtatgt ctttatcaca aaagatgaca 105480
ttttatgtaa gctaatgtta tctagtcaaa attcttagct tattttaaaa tcaactcttc 105540
aagaaaagga ataaacattt aatataaata tcatagcagt attgcacata gaatagaaag 105600
gtcgggcagg gtagtggaag tcagctattc tatacaatcc attcggtatt ttccaaaaca 105660
tttgatgttc aggccatatc caggaactgg atgacctaac aaacttctct gagtaccttt 105720
ttttccacaa gagatctcca tcactaagaa aaaaagcatt gtgatttaaa agccaaattt 105780
gccttatcca tcatcatgtg caccaagtat ttgctacctg cctactatat aatattgaag 105840
atacaacgtg aataagaaaa atactattgc taccctcaat cagagtatgt gattggaaaa 105900
gtgtataaca aacctttccc agtgtcttca ggtataatgc agagatacca gatacggcat 105960
caatgtgtat acacattatg gctgtaccat tcactttaag
106000<210> 9
<211> 2034
<212> DNA
<213> H. sapiens

<400> 9
ggatctggca gcgccgcgaa gacgagcggt caccggcgcc cgacccgagc gcgcccagag 60
gacggcgggg agccaagccg acccccgagc agcgccgcgc gggccctgag gctcaaaggg 120
gcagcttcag gggaggacac cccactggcc aggacgcccc aggctctgct gctctgccac 180
tcagctgccc tcggaggagc gtacacacac accaggactg cattgcccca gtgtgcagcc 240
cctgccagat gtgggaggca gctagctgcc cagaggcatg cccccctgcc agccacagcg 300
acccctgctg ctgttgctgc tgctgctggc ctgccagcca caggtcccct ccgctcaggt 360
gatggacttc ctgtttgaga agtggaagct ctacggtgac cagtgtcacc acaacctgag 420
cctgctgccc cctcccacgg agctggtgtg caacagaacc ttcgacaagt attcctgctg 480
gccggacacc cccgccaata ccacggccaa catctcctgc ccctggtacc tgccttggca 540
ccacaaagtg caacaccgct tcgtgttcaa gagatgcggg cccgacggtc agtgggtgcg 600
tggaccccgg gggcagcctt ggcgtgatgc ctcccagtgc cagatggatg gcgaggagat 660
tgaggtccag aaggaggtgg ccaagatgta cagcagcttc caggtgatgt acacagtggg 720
ctacagcctg tccctggggg ccctgctcct cgccttggcc atcctggggg gcctcagcaa 780
gctgcactgc acccgcaatg ccatccacgc gaatctgttt gcgtccttcg tgctgaaagc 840
cagctccgtg ctggtcattg atgggctgct caggacccgc tacagccaga aaattggcga 900
cgacctcagt gtcagcacct ggctcagtga tggagcggtg gctggctgcc gtgtggccgc 960
ggtgttcatg caatatggca tcgtggccaa ctactgctgg ctgctggtgg agggcctgta 1020
cctgcacaac ctgctgggcc tggccaccct ccccgagagg agcttcttca gcctctacct 1080
gggcatcggc tggggtgccc ccatgctgtt cgtcgtcccc tgggcagtgg tcaagtgtct 1140
gttcgagaac gtccagtgct ggaccagcaa tgacaacatg ggcttctggt ggatcctgcg 1200
gttccccgtc ttcctggcca tcctgatcaa cttcttcatc ttcgtccgca tcgttcagct 1260
gctcgtggcc aagctgcggg cacggcagat gcaccacaca gactacaagt tccggctggc 1320
caagtccacg ctgaccctca tccctctgct gggcgtccac gaagtggtct ttgccttcgt 1380
gacggacgag cacgcccagg gcaccctgcg ctccgccaag ctcttcttcg acctcttcct 1440
cagctccttc cagggcctgc tggtggctgt cctctactgc ttcctcaaca aggaggtgca 1500
gtcggagctg cggcggcgtt ggcaccgctg gcgcctgggc aaagtgctat gggaggagcg 1560
gaacaccagc aaccacaggg cctcatcttc gcccggccac ggccctccca gcaaggagct 1620
gcagtttggg aggggtggtg gcagccagga ttcatctgcg gagaccccct tggctggtgg 1680
cctccctaga ttggctgaga gccccttctg aaccctgctg ggaccccagc tagggctgga 1740
ctctggcacc cagaggcgtc gctggacaac ccagaactgg acgcccagct gaggctgggg 1800
gcgggggagc caacagcagc ccccacctac ccccaccccc cagtgtggct gtctgcgaga 1860
ttgggcctcc tctccctgca cctgccttgt ccctggtgca gaggtgagca gaggagtcca 1920
gggcgggagt gggggctgtg ccgtgaactg cgtgccagtg tccccacgta tgtcggcacg 1980
tcccatgtgc atggaaatgt cctccaacaa taaagagctc aagtggtcac cgtg 2034

<210> 10
<211> 2439
<212> DNA
<213> H. sapiens

<400> 10
ctccgggaac gccagcgccg cggctgccgc ctctgctggg gtctaggctg tttctctcgc 60
gccaccactg gccgccggcc gcagctccag gtgtcctagc cgcccagcct cgacgccgtc 120
ccgggacccc tgtgctctgc gcgaagccct ggccccgggg gccggggcat gggccagggg 180
cgcggggtga agcggcttcc cgcggggccg tgactgggcg ggcttcagcc atgaagaccc 240
tcatagccgc ctactccggg gtcctgcgcg gcgagcgtca ggccgaggct gaccggagcc 300
agcgctctca cggaggacct gcgctgtcgc gcgaggggtc tgggagatgg ggcactggat 360
ccagcatcct ctccgccctc caggacctct tctctgtcac ctggctcaat aggtccaagg 420
tggaaaagca gctacaggtc atctcagtgc tccagtgggt cctgtccttc cttgtactgg 480
gagtggcctg cagtgccatc ctcatgtaca tattctgcac tgattgctgg ctcatcgctg 540
tgctctactt cacttggctg gtgtttgact ggaacacacc caagaaaggt ggcaggaggt 600
cacagtgggt ccgaaactgg gctgtgtggc gctactttcg agactacttt cccatccagc 660
tggtgaagac acacaacctg ctgaccacca ggaactatat ctttggatac cacccccatg 720
gtatcatggg cctgggtgcc ttctgcaact tcagcacaga ggccacagaa gtgagcaaga 780
agttcccagg catacggcct tacctggcta cactggcagg caacttccga atgcctgtgt 840
tgagggagta cctgatgtct ggaggtatct gccctgtcag ccgggacacc atagactatt 900
tgctttcaaa gaatgggagt ggcaatgcta tcatcatcgt ggtcgggggt gcggctgagt 960
ctctgagctc catgcctggc aagaatgcag tcaccctgcg gaaccgcaag ggctttgtga 1020
aactggccct gcgtcatgga gctgacctgg ttcccatcta ctcctttgga gagaatgaag 1080
tgtacaagca ggtgatcttc gaggagggct cctggggccg atgggtccag aagaagttcc 1140
agaaatacat tggtttcgcc ccatgcatct tccatggtcg aggcctcttc tcctccgaca 1200
cctgggggct ggtgccctac tccaagccca tcaccactgt tgtgggagag cccatcacca 1260
tccccaagct ggagcaccca acccagcaag acatcgacct gtaccacacc atgtacatgg 1320
aggccctggt gaagctcttc gacaagcaca agaccaagtt cggcctcccg gagactgagg 1380
tcctggaggt gaactgagcc agccttcggg gccaactccc tggaggaacc agctgcaaat 1440
cacttttttg ctctgtaaat ttggaagtgt catgggtgtc tgtgggttat ttaaaagaaa 1500
ttataacaat tttgctaaac cattacaatg ttaggtcttt tttaagaagg aaaaagtcag 1560
tatttcaagt tctttcactt ccagcttgcc ctgttctagg tggtggctaa atctgggcct 1620
aatctgggtg gctcagctaa cctctcttct tcccttcctg aagtgacaaa ggaaactcag 1680
tcttcttggg gaagaaggat tgccattagt gacttggacc agttagatga ttcacttttt 1740
gcccctaggg atgagaggcg aaagccactt ctcatacaag cccctttatt gccactaccc 1800
cacgctcgtc tagtcctgaa actgcaggac cagtttctct gccaagggga ggagttggag 1860
agcacagttg ccccgttgtg tgagggcagt agtaggcatc tggaatgctc cagtttgatc 1920
tcccttctgc cacccctacc tcacccctag tcactcatat cggagcctgg actggcctcc 1980
aggatgagga tgggggtggc aatgacaccc tgcaggggaa aggactgccc cccatgcacc 2040
attgcaggga ggatgccgcc accatgagct aggtggagta actggttttt cttgggtggc 2100
tgatgacatg gatgcagcac agactcagcc ttggcctgga gcacatgctt actggtggcc 2160
tcagtttacc ttccccagat cctagattct ggatgtgagg aagagatccc tcttcagaag 2220
gggcctggcc ttctgagcag cagattagtt ccaaagcagg tggcccccga acccaagcct 2280
cacttttctg tgccttcctg agggggttgg gccggggagg aaacccaacc ctctcctgtg 2340
tgttctgtta tctcttgatg agatcattgc accatgtcag acttttgtat atgccttgaa 2400
aataaatgaa agtgagaatc caaaaaaaaa aaaaaaaaa 2439

<210> 11
<211> 3318
<212> DNA
<213> H. sapiens

<400> 11
gtgatgcgta gttccggctg ccggttgaca tgaagaagca gcagcggcta gggcggcggt 60
agctgcaggg gtcggggatt gcagcgggcc tcggggctaa gagcgcgacg cggcctagag 120
cggcagacgg cgcagtgggc cgagaaggag gcgcagcagc cgccctggcc cgtcatggag 180
atggaaaagg agttcgagca gatcgacaag tccgggagct gggcggccat ttaccaggat 240
atccgacatg aagccagtga cttcccatgt agagtggcca agcttcctaa gaacaaaaac 300
cgaaataggt acagagacgt cagtcccttt gaccatagtc ggattaaact acatcaagaa 360
gataatgact atatcaacgc tagtttgata aaaatggaag aagcccaaag gagttacatt 420
cttacccagg gccctttgcc taacacatgc ggtcactttt gggagatggt gtgggagcag 480
aaaagcaggg gtgtcgtcat gctcaacaga gtgatggaga aaggttcgtt aaaatgcgca 540
caatactggc cacaaaaaga agaaaaagag atgatctttg aagacacaaa tttgaaatta 600

acattgatct ctgaagatat caagtcatat tatacagtgc gacagctaga attggaaaac 660
cttacaaccc aagaaactcg agagatctta catttccact ataccacatg gcctgacttt 720
ggagtccctg aatcaccagc ctcattcttg aactttcttt tcaaagtccg agagtcaggg 780
tcactcagcc cggagcacgg gcccgttgtg gtgcactgca gtgcaggcat cggcaggtct 840
ggaaccttct gtctggctga tacctgcctc ttgctgatgg acaagaggaa agacccttct 900
tccgttgata tcaagaaagt gctgttagaa atgaggaagt ttcggatggg gctgatccag 960
acagccgacc agctgcgctt ctcctacctg gctgtgatcg aaggtgccaa attcatcatg 1020
ggggactctt ccgtgcagga tcagtggaag gagctttccc acgaggacct ggagccccca 1080
cccgagcata tccccccacc tccccggcca cccaaacgaa tcctggagcc acacaatggg 1140
aaatgcaggg agttcttccc aaatcaccag tgggtgaagg aagagaccca ggaggataaa 1200
gactgcccca tcaaggaaga aaaaggaagc cccttaaatg ccgcacccta cggcatcgaa 1260
agcatgagtc aagacactga agttagaagt cgggtcgtgg ggggaagtct tcgaggtgcc 1320
caggctgcct ccccagccaa aggggagccg tcactgcccg agaaggacga ggaccatgca 1380
ctgagttact ggaagccctt cctggtcaac atgtgcgtgg ctacggtcct cacggccggc 1440
gcttacctct gctacaggtt cctgttcaac agcaacacat agcctgaccc tcctccactc 1500
cacctccacc cactgtccgc ctctgcccgc agagcccacg cccgactagc aggcatgccg 1560
cggtaggtaa gggccgccgg accgcgtaga gagccgggcc ccggacggac gttggttctg 1620
cactaaaacc catcttcccc ggatgtgtgt ctcacccctc atccttttac tttttgcccc 1680
ttccactttg agtaccaaat ccacaagcca ttttttgagg agagtgaaag agagtaccat 1740
gctggcggcg cagagggaag gggcctacac ccgtcttggg gctcgcccca cccagggctc 1800
cctcctggag catcccaggc gggcggcacg ccaacagccc cccccttgaa tctgcaggga 1860
gcaactctcc actccatatt tatttaaaca attttttccc caaaggcatc catagtgcac 1920
tagcattttc ttgaaccaat aatgtattaa aattttttga tgtcagcctt gcatcaaggg 1980
ctttatcaaa aagtacaata ataaatcctc aggtagtact gggaatggaa ggctttgcca 2040
tgggcctgct gcgtcagacc agtactggga aggaggacgg ttgtaagcag ttgttattta 2100
gtgatattgt gggtaacgtg agaagataga acaatgctat aatatataat gaacacgtgg 2160
gtatttaata agaaacatga tgtgagatta ctttgtcccg cttattctcc tccctgttat 2220
ctgctagatc tagttctcaa tcactgctcc cccgtgtgta ttagaatgca tgtaaggtct 2280
tcttgtgtcc tgatgaaaaa tatgtgcttg aaatgagaaa ctttgatctc tgcttactaa 2340
tgtgccccat gtccaagtcc aacctgcctg tgcatgacct gatcattaca tggctgtggt 2400
tcctaagcct gttgctgaag tcattgtcgc tcagcaatag ggtgcagttt tccaggaata 2460
ggcatttgcc taattcctgg catgacactc tagtgacttc ctggtgaggc ccagcctgtc 2520
ctggtacagc agggtcttgc tgtaactcag acattccaag ggtatgggaa gccatattca 2580
cacctcacgc tctggacatg atttagggaa gcagggacac cccccgcccc ccacctttgg 2640
gatcagcctc cgccattcca agtcaacact cttcttgagc agaccgtgat ttggaagaga 2700
ggcacctgct ggaaaccaca cttcttgaaa cagcctgggt gacggtcctt taggcagcct 2760
gccgccgtct ctgtcccggt tcaccttgcc gagagaggcg cgtctgcccc accctcaaac 2820
cctgtggggc ctgatggtgc tcacgactct tcctgcaaag ggaactgaag acctccacat 2880
taagtggctt tttaacatga aaaacacggc agctgtagct cccgagctac tctcttgcca 2940
gcattttcac attttgcctt tctcgtggta gaagccagta cagagaaatt ctgtggtggg 3000
aacattcgag gtgtcaccct gcagagctat ggtgaggtgt ggataaggct taggtgccag 3060
gctgtaagca ttctgagctg ggcttgttgt ttttaagtcc tgtatatgta tgtagtagtt 3120
tgggtgtgta tatatagtag catttcaaaa tggacgtact ggtttaacct cctatccttg 3180
gagagcagct ggctctccac cttgttacac attatgttag agaggtagcg agctgctctg 3240
ctatatgcct taagccaata tttactcatc aggtcattat tttttacaat ggccatggaa 3300
taaaccattt ttacaaaa 3318

<210> 12
<211> 78001
<212> DNA
<213> H. sapiens

<400> 12
cagattccgg gtctcatccc tagaccaaag atatctgaat catttggcga tgggtccagg 60
aacctgcatt attcaacaca cttcccaggt gaccgtaaat gtcaaaagac aaaattacaa 120
caaacttaaa catcttaatt ggcttcattc atgattctag aatcgggcaa gacttcattc 180
ctcaaaatag cacaagtgtc ccaatgagct gagcagagga ggttggtttt atagacagaa 240
aagggctgaa aaaagcagaa acaaagaaca aaaagcagat tggtcatttc aaagttactt 300
tccttgtaag gcaggaacag ggaaacagaa caagagagaa ataactgatt ggtcgcatcg 360
ggttacttca ggttactttt tgttgtaagg attaaagcaa agggaacttc attatgttga 420
ttaaaacggt ctgcttggga aatcagggtg tgtatctctc ttctgatttt gtgaaaggtt 480
atcagtctga tgatgtagaa ctttagcatg agtgactcca ttttgatttt tagtctagtc 540
tgttgagacc ctaatgccag aacttttttt gtttgtttgt ttcgctcttg ttgcccaggc 600
tggagtgcag tggagctatc ttggctcact gcaacctctg cctccagggt tcaagcgatt 660
ctcctgcctc agcctcccaa gtagctggga ttacaggcat gcgccaccac gcccggctaa 720

tttttttttt tttttttttt tttagcagaa accgagtttc accatgttgg tcaggctggt 780
ctcggactct tgacctcagg tgatccacct gcgtcggtct cccaaagtgc tgggattaca 840
ggtgtgagcc accacaccca gccctttttt ttcaagacag ggtctctctc tgccacccaa 900
ggtggagtgc agtggcgcca aaacagctca ctgcagcttc cacctcctgg gctcaggtga 960
tcttcctgcc tcagcctccc cagcagctgg gccccaccac accggctaat tttttaactt 1020
ttagtagtga cgaggtctga ttctgttacc caggctggtc tggaactcct ggcctcaaga 1080
catccgcctg cctctgcctc ccaaagtgct gggattacag atgtaagcca ccgcgcctgg 1140
gctcctatga tttttattta acataatgca ccatggaatt tgtgctctgc ttagttcagt 1200
ctgagcagga gttccttgat acttcgggaa acactgaaaa tcattccatc cccatccatt 1260
cattcctgca gcacccaagt ggaaattctg cgtttcagac agggacacta cccttagaga 1320
gcagtgggct tccccagcag cgtagtgaaa catgatactc ctgagtttca tgaaaaaagg 1380
gcagacatct ggccagagct gggaggcagg aaatagagca cggtgccctc ctcccatact 1440
ccagcttgga ttactgaggc tggggcccag gccctgcagg aaaggaggtg catgactact 1500
ttaaggccac tcactctgtg actcaacggg ccgggtcggg gctggaactc aatgccctcc 1560
cgggcctgga gagcccacgc gccgtgggcg gggctcccgg ggtcgcctag gcaacaggcg 1620
cgcgccgcgc ccgagcccag agccccaaag cggaggaggg aacgcgcgct attagatatc 1680
tcgcggtgct ggggccactt cccctagcac cgcccccggc tcctccccgc ggaagtgctt 1740
gtcgaaattc tcgatcgctg attggtcctt ctgcttcagg ggcggagccc ctggcaggcg 1800
tgatgcgtag ttccggctgc cggttgacat gaagaagcag cagcggctag ggcggcggta 1860
gctgcagggg tcggggattg cagcgggcct cggggctaag agcgcgacgc ggcctagagc 1920
ggcagacggc gcagtgggcc gagaaggagg cgcagcagcc gccctggccc gtcatggaga 1980
tggaaaagga gttcgagcag atcgacaagt ccgggagctg ggcggccatt taccaggtgc 2040
gggagcgccc cggagcgtgg cgggcccttc gcttaggccg cttgaacatc ccctcagacc 2100
tccaggcccc agactccctc tgggtcttgc cctctgcctc gctcctactg cttgaggatt 2160
cgatgggaca gcgacgcact gcgtcccccc accctttgtc cccggggcgg gcgtgtttct 2220
cgccgcagcg tcggagcccc cttcgatccc ccacctccct tctgttctcc agctcgggtg 2280
atctctcaag ccgggggacc gccggtctgt gctctcaacg cgaatccctc gcaccccgac 2340
cccgccccct gcctgtccac tctttgtccc ctggggtgat ttagcacccc cactatttcc 2400
ttttctggag tggaccacct cagactctct tcctttgtct ccctggggga aaaggttact 2460
ccccccgtcc ctccttcaca tttcctttcc cctagtctca gtgtgcgtcg agtcccagag 2520
atgacagtcc cctttcccct ttctgttcat tcatttattg gataggagtt ggcaagctta 2580
ttctgtgcta ggcaccgctt aggcattgga ggtggtgttt gctaatcagg acaggcaaga 2640
tcctagcctt agtggggcct agagtcgaat agggcaatca aacacaaaag caaataattt 2700
cagatagtga caggtgctgt gaagagaacg acttcctaac ggggtacagg gtgactgcat 2760
agaaggccgg ctgtcttaga gaaggggatc agggaaggcc tgtcaaagga ggagacattt 2820
gctttgtgag ctgaaccaag aggagcagaa agccgtgaga atatggggct aaagaacctt 2880
ctagccagga ggcctgcggt acccactcca ttggggccat gatattattc tttcaggcag 2940
ggactcagga aggttaacgt tttaaccctc tctaaaatag catctttcct caatgagcag 3000
cttagtcttt ggtcgtggca gagatgacct tgtcttagga gtcatctcct tgtgtgttaa 3060
aaagttagga aaggagggtt tctcatatat ctataaaaca agtagttaaa aacacaaaga 3120
gctcttcctt tcacaagcag ctgaataaga tacatactcc caattaaatg tcattgcggg 3180
ggttgttaag attaactaaa accacacttg cacagtatct taaataagcg atatacagaa 3240
tagagagatt ttgttacttg tgtaaaggga gacagcagat gattctgttt tcagcttata 3300
ggctcaaaag gcaaattgtg agatccatca gctgtagtat taaaatctat tttgagctcc 3360
gcttagaaag gaaaaaaggt ttaagcagtt ctttggtatg cttgactaac aaaagccttt 3420
ttttttggca gccttgattt tcatgtggat ttacatcaag cttatttgac aggattcttt 3480
ttatttggac tgtagtgtgt atattagttt ctgctagact aatatttcta accactgtaa 3540
tctatatact aataagtatg attgatcagt atataaaatt tgtatgccat atctggtctc 3600
tgaattagct gaatgaattc cataagggac tttgagactg tgtagacaaa ttttctgcat 3660
cagtttaatg cagtagagtc taaaatgtct ttaaatgaaa attgttggtc tgaagtgttg 3720
gagttgatta tgatacaccc catcacagtg gaagcattgt ggagagaagt cttttccact 3780
gaaattgact gagttgacaa caagaaatac gtattgtaac ttagttctta gttgaatttt 3840
atttcttaca attttaagcc agagtgggtt gacctgtcac ccaagcatgg ttaaaattgt 3900
attcagcatg caactagcat ggagtgtgtc agtcttcaat tcatttcctt cattgttctt 3960
aagtttttct gccacaatta aaccccacaa gttagtcaag gtgttgagat tttcactgct 4020
tcttaatgga ttgccacatt ccctgaggta gtttcttttg gtcttagaga attgtcaggg 4080
ccagcttttc tcacctccac tgtatggata tttttctttt ctaagatctt gaaatcagaa 4140
gcttttctcc taagtgtaaa agtagctctt tgtcatacaa ctgtagcgtt ttctgaaaca 4200
gagttcagat gaccttgagt ctaaagtggc taactttcca aggtgtgtat cgctttacca 4260
aaaccattat ttttcaagga ttcaaagaat gtgtttacaa ttgatagaaa atggaagttt 4320
aaaaaaatta atactttata gcatgttgaa atgagggcag ccttatacaa agtcatactt 4380
tgagcttgcc tagcctattg tgatcagaga ataatgtaat ttttgcttac aacttggtaa 4440
gcaggtcagt tattctaact tattttctga ttagaacaaa aagatgtaaa aacttgaaaa 4500
ctattgggaa aagaacaaag agtgaagagg acttttgagt gctgaggaat gtggcagctt 4560
ggaaaacaaa ctttttaggc agagattctt tgctaggtca gtttgataaa gtgagcataa 4620

ccgtattttt aatctttaat gctaatgaat agcatagatg ctaataagca tctaggtcta 4680
taaaaagtca gctttgatag tgtatataga tggctttaaa cattgttttc tagcatttaa 4740
acactttcaa atcatccggt tgcttgattg ggcctagctg tctaagagga gagaatgagc 4800
ccagatgagg aaaagagatt gattttactg agctagaatg agaggagaga gggttgagtg 4860
aatgaaaaga atagctcatg tgctcccctc catctgtagt ttaagagggg ttgggtccgg 4920
tgttttgctt gttttctcgt ctgtaaattc tttgattctc tgacaccact cactatattt 4980
cattgtgaat gatttgattg tttcagataa aggggactgc aataatacct tgtgacatga 5040
aggcaagatt tattcatgtt agaggcaggc tttgtaaaat gggccactct tccaattgac 5100
atttgttttt atagctgttt tcattatgaa atacaatcta atgcctgact aggttaaaac 5160
catgttgtaa caatagttca ctaaaattcc ttactgatat acagcttatg ttgttatatt 5220
ccaaaaagat gaatattaaa atttgccaat aatgtttatt taaatactat tttcttcaga 5280
ggaaaaaaaa ctattttatg caaaggagaa agatctatac actatgactc acttcactta 5340
aaaaaaaaaa gactaacgga aatgacatgg agagactggg aagttctagt catcttgagt 5400
gacccattag atctaaatgt tcttgtttag ccctggtttg agtgaactaa atttaggtgt 5460
ctgatcagta ctttggaaat ggtgtaaatg cctttgtaat tgtctggact gatattagat 5520
taactgggag cacaagtaga aatagtgaag gaaagaactt tttgctattg ttatttgaca 5580
tcactggcat atttatagga atactttggt gtttttggaa gtaagtaaac caaccagtgg 5640
ttctaaaaag tcagctgggg gataatggta atgccgctgt ttcttagctg caagttatct 5700
gccgttactt ctcctccatt ttgcatttta tcttgaatag ctcctcaaaa cctattaaaa 5760
tacctggtat tgaataatgt aattgaatgt gtactgaatt tcacagtgga aatgaataag 5820
aaatttcctg tggaggtttt ttgacttagc tactgaaata acggcctttt gttgtgtgat 5880
tctttccctt ttctctttgt taaagaaaac tgtcttgtga tcttgtagat tacagaatcc 5940
ttttggcaat ttctgttcct agcactgctt tttctttctt tctttctttt aaatagaaat 6000
ggggttttgc tgtgttgccc aggttggtct tgaactcctg gcttcaagcg atcctcccac 6060
cttggcctcc tgaagttggg attgcaggcg tgagcaggta ctttttctga ggcctgcctg 6120
agcctatata tattttgcac aatttggcat tcctccctac agtgtttatg ctgatttgtt 6180
tctggtaaca actaatactg gcaaatcggc tgggcatgtt actttatgct gcccatattc 6240
aggaaaattg gaattctagc tgggtcattg ttcccagatg atgtagtttg gcaccagcca 6300
ttccatgttc acattttgag tatccaggag ggctggggac tttggagtag ttggtgattc 6360
cctctgccac atttcactgg ttggtcacta tggcatcctt tccaccacac tagtagtcta 6420
ggttctcaga tgttgcttat gagcctgcaa tggtttctag tttcacactg cagaaatgag 6480
tgaagccggt tacccgttaa tatggtccca tcatcactag agtaattcat tgttctaaaa 6540
ccagatctga gtctctcact cctctgcaac tacttctgat tctttcataa cacttgtaaa 6600
gtccaaactc ctctttagca tggcagccag cttccagtcc ttccctccta tgtggcttcc 6660
attctagcca gacaagaaag ggcagcgttc tccaaactca tcctcgccct tcattcctct 6720
ataccattgc tgagcacttt gttgaggatg cctctcccgt tcaatctagc ttgcatcttc 6780
cagctcgaat gtgtgcttcc ttgcaccaga gttttgttcc gtcacctgtg tgttttcata 6840
caagctggca catatctctt ctaaagccct gctgtcattg tagctgcgtc tttacaaaca 6900
tttttttttt aaatttttat aaagtcaagg tctcactata ttgcccaggc tggtctcaaa 6960
ctcctgggct caagtgatcc tcctgccttg gcctcccaga gtgctgggat tataggtatg 7020
agacactgtg cccagctgta gctgctactt tatatcccag gtctatctcc aatggagccc 7080
aagcttcctg aggccacctg ttgtatcttt ctcattcatc ttgaagtcct ctgctcctgg 7140
cacagagtag gtacctaaca agagttggga ttgaattgat ggtcagtact ttgctagcct 7200
gatggtataa agatgtacaa aacatgttcc tggctcccac tctagggggg caatgatgga 7260
aacaaataga ttagcccaca ttagtaccaa tagtagaggt cactctggga gaaggccccc 7320
accacatttt gagtcatggc ctaatgaggt aatttagtat tgcctgctgc agtggctttg 7380
gaagaaaggc tggcattctt agccagtaga agctgatacc actgatttgt ttcacagaag 7440
ctttaaatat aacaataaat ttgtgcttgg cctacggtga actttacagg caacttggag 7500
gtaatatgtt tgtctctcta agaattgttg aattcctctt ccctcatccc tcctgactgg 7560
ttctcacaag cctagcgggc ctttgcatgt ggttggttca taaaatactt tttgattttg 7620
ggatataaaa tatagttctc cataaaataa cgactgttac caagtctttg attttttttt 7680
tcaaactata aatggtaatg acattctttg gcctttgatc agaccaccct taggggcaag 7740
agagtagttt catgttttgc tttttctagt gtcccctgtg tctgggtata gttgcagtct 7800
cagctgtcat actaacagtg ctgagtgagt ccccttactt ctttgggttt tggtttctcc 7860
cttgtaaaaa tgatcctgga ctaactgatc attaagttca ggtcaagtaa taaaaatcct 7920
taatgtactc acaaatacaa tttaatgttc ctgaataatc cttgtaaaaa ctgcagcagt 7980
tactcagttt tgtaaggtgt ggttgggtac tattaggctc aaaagtttat aggagctttg 8040
tgagtatagt taacaactca aaagaatggg gtgttttttc ccgaggggca tgaaatgttt 8100
ttgataaata gagttcattt gacttggtaa tgtggaaaat gagtagccct gacacgtacg 8160
ctatgctttt gcagtttttc tctcaagtag caattgggtg gcttttcctg taaaagatag 8220
aggaactgat tcttgagaat ttacgaaagc ttcaacccta actaggtatg caaagaatag 8280
ttgcccttta tgttgtaatt ttaggaagaa acctacatct ggtctaagtt tcatttgaat 8340
aatatgatag tttacacatc tgccatattt gagaagaaag tacctaagtc tccagcattt 8400
tagaaataat gctttacttt gtgtagaaat ggtctttaga gtttaatagc tgctgccctc 8460
tcctttttca aagcagcttg acataatcat gagtatcttg ctgacagctt gtaaattttg 8520

attgtatgaa aactgaaaat aagaccattt cacatggaag attccctcct gccctgaaac 8580
agccaaagaa aactgtagcc atcaaatcta ttgatctctg ggctttggta caagtcacac 8640
tactacaaat aaaataatac caagtactta taaatgattt tcagtccttt taaagtttat 8700
ttttttaata ttttttttga gatggggtct tgctgtgtcg tccaggctgg agtgcagtgg 8760
cacaatcttg gctcactgca acctccacct cctgggctca agtgatcctc ccacctcagg 8820
ctcccaagta gctgagacta caggcatgtg ccatcacgcc cagctaattt ttgtattttt 8880
ttggagtaga gatgggattt tgctgtgttg cccaggctgg tcttgaactc ctgggcttaa 8940
gccatctgtc tgcctcaggc tcccaaagtg ttgggattac aggtgtgagc cactgtgccc 9000
ggcccagccc tttttttaag agaaaaacgt atgacatcgt tcgatttact gagtgcttat 9060
ggttttacta aggcagtaag gttttatgga taccctatgg taattagata gaattagtgc 9120
tctgaagtca gctctgtaat atggactcag agtaaacatg gcaaagggac acttaaggtc 9180
tgcattttct ctgggaaata aacgtattct ttactactct gaatctagtg ctgggaaatt 9240
ctaaatcctt cttgaggatt aaccacttga agtaaagttt tgggtcccaa gtaggcttgt 9300
gtccctgtct ccttctcttt acttttcaga tgtttcttcc tagagactga ggtatatttt 9360
acttttacag atgaagaagg aagcctcggc tgtgtttgtg gcttttgtgg gtgagcaaca 9420
tcacttgcaa agataagatg agcatagcaa aactaggctt tcaaaataat ttttaaaaat 9480
ttcttagtga ttagaaaagg aaaactcttc ccttgtctct gttaagaaac gtttttcgac 9540
ttttttcctt tcttaatgga tcttttattg gcacttctct tccttttgca gaatcttact 9600
taaaagtcac tacgttacat tacagcaaac agcttagcta atttttatcc agatgggccc 9660
cggttacagg attgtacact attgcgaatt tcttacagga aagtgaacat caagtaatta 9720
ttccaaatag agttctctta agaacgtgag ttacttaaaa atgtctaagg atgaagtcac 9780
ttctgaatat aacttcactc aagagaacaa ataagcaaac tgcatttagc ataacatggt 9840
aaattagctt taactctcct tgatgtttga acatttgtcg ctgttaacta ctgtttcact 9900
tttcaaatag tcagggctta gtttgcttct gtaaggataa agggaaaata cgccttcact 9960
gagtcataaa tatttttgtg gctaactttt gcacagagaa aagaggcctc taagaaggta 10020
cccagtgaat ttttttttcg gggcagggag agaatatgtc attttttggt ttgttgttgt 10080
tgttgtcatt gttttgcttt gttgttttta ctctgaactg aactgtatct tgacagcact 10140
tttgaattaa gagcattact cttattgttc tctactacct ggacgccacc tccctgttgc 10200
catagtgtta aggatcatgc tccgaggtgg ggtgaggcag aatggggcca agatcagaaa 10260
gttacattaa gctacatcag gtttatacaa gcataaaacc aaatttttgg agcagtcccc 10320
agaatacaac ctggtttagc cacacctaaa ggttgctctt gaatattcct tgagaatcca 10380
catccctaga atgctgggtt tcaatggccc ctttatgtac ctatcatggt gtcatttctg 10440
agcatttcta aatattcctt catgtcttac tgacagtttt tcttgaataa atcttaggaa 10500
tattagtgcc attatcagta ttttgtttgg tctgttcaca ccacaaataa ctacccaggt 10560
ctgctacttg cccctatttc tctacctgct aatgaaaatg cttttgaaag tttgagtaac 10620
agtattggag tgtgcacagt ggtattggta ggttctgtac tcatccttaa ccacttgttt 10680
tcatcctttg tgagcttgaa gtttctccaa aaaatttatc acaaaactta tcagacatag 10740
ttaatacact cagagagaga atcactgaaa aagtagatgt agtttaacaa acccagtgcc 10800
ttttttttac ccatgaatac atatttgtca actaaacctc attttgcaac ttgttccact 10860
actcgaatgg taacaaactt ttggtttccc aatagatttg gaagatgttg cttttgaaag 10920
taggaaatag atggctttag aagatggaag aatattttgt ttgaagtggg agcgtggtat 10980
gtccttagct gtctgtgaaa tgcagctgaa gatgggtgtg ggccttcatc tgcatttccc 11040
atcttcagtt tgaggaggta gttacccttc taaccactta agaactgcat ggtacatgct 11100
gttttattta cagggcaaaa ctgtgctccc gtagtttccc tggtgcttgc cttcacgtta 11160
acacagtgtc atcgtttggc agtgtttatg tgccagggtc catgttagaa ggaggaaagg 11220
tatagcgaag ttaaagggtg cagttggcct cccaccttta gttttgtaag tgcctttaaa 11280
gtttgatttt tgtaggttga tcataaggaa gtgataagta tgttaggtta tttgtggttt 11340
gagctaattt tagtctcttt ttacagcttg ctttgtatcc tttgccatta aaacatgctt 11400
tctagaaaga caacttttga atgtaggaca cagtctatat tctatacttg gctacatttc 11460
aaaaaatatt ttctcagtac tttggaagtt ggacagttgg aagcatagtg acagtattta 11520
aaaatctttg attccggccg ggcatggtgg ctcacgcctg taatcccagc actttgggag 11580
gccgaggtgg gtggatcact tgaggtccgg agttcaggac cagcctgacc aacatggtga 11640
aaccctgtct ctactaaaaa tacaaaatta gccgagcgtg gtggtacatg cctgtaatcc 11700
cagctactca ggaggctgag gcaggagaat cgcttgaatc tgggaggcgg aggttgcatt 11760
gagccgagat cataccattg cactacagcc tgggggacaa gagtgaaact ctgtctcaaa 11820
aaaaaaaaaa aaattaagtg atttctttgc tttgtgacac ttctactttt ccagcaagta 11880
aattatattc tttcatacag gtatgaaatt cttgttccaa gctagtggtt aaaaaggcac 11940
agttgatatt agaggatttg taaaagatta tgaccacgcc tgcaatgtac tgaagcaagg 12000
ctttgctggg ctgtgtatag gaaaccttcc ccagcctgtg cccttgcttg atagaacatt 12060
ttgctcctaa gggtaggtgc ctgtatctgt ctccagtact ggttagtttc acacagaaca 12120
gttgtgtttc agagctttag tctcaagctg ccctgctccc ctgaagcagc caccctgagc 12180
atgtgcactc acaggagggg acatgtgagg tcatggaaga agacgactca ggaagaagaa 12240
gacttgggtt tgggttctga ctctgccttt gactgttgtg ggattttgag gagttgcata 12300
caggatctgt aaaatgtagt cattagacta gactagacag ccatatagca ttacctagat 12360
gtaactttct acaaagacat ggtcacagga gaagaccaga gggtggggtg atctttctgg 12420

aaaaattggg gcttcatgcc ttactcatgc tagatatggt agcattatat ggctgtgcct 12480
gatcccccta atctaaaagt gggacagaac tttaaaattt catattaact caaattaaaa 12540
cttgaaaaaa acccattatt tccttaaaaa taataaaatg ccctgtgggg gcataagtca 12600
cattatattt taaaattcct gaatgccaca tggatgaatg tagttccttt tgaaattctt 12660
cttttgtcta aagaggaatg ttggattttg taattggact aaaaaatctt ccatttgaga 12720
gagaaacagt ctgctgcatg ttctaccctt gttcaggata aaacccacta atagctaaca 12780
tttattgaat tctgtgttgt gcctcaggca ctgtgcaaag tcctttacat gcaatgctgt 12840
ttattatata ctgtcaattg gtctataaca gcaggaaatg tttcaggagg acaatgaggt 12900
cccagaccct cagtcttctc ctgtgtcctg gattcagctt cacaatagca ctatggcagt 12960
gtggccactg cttcagcttc cacatacatg gctgtgaaga gagacagggg attgtgctaa 13020
gcctccccga tttattagga cataggagga gagagtttgt agtttttgac ctttgcctag 13080
ttttctaacc tctttcctag atgtcacaaa ttggccaccc acagtcatat tttgcttgct 13140
tcacgcaatg ctttttaaaa aagagaagag tttaatttgt gccattgttt ataaatgaat 13200
caggagaaat gacatgcaac tctggattct ggcctctctt gaaaaatctg aaaatcacac 13260
cgtctgagct tacactggca gtggtctgct ggactgaggg acacaactcc ttttggatgt 13320
acatgtgtgc gttgcagagt ttaccacagt cccacagtgg gtcacactgt ccttgtcggt 13380
gtacactacc tagcacttga gtttgcaacc cctaccccaa gctgagtttt ctcgtcaagc 13440
ttgatgttaa tgttatgtga tgcttggcct tgtaggtatt tggtatatta tcgttagata 13500
aaattgaagc aaagggctaa agggttggtg gcctgaggga gtgcccttga cagtaaagtc 13560
taggataaaa tcattggcca ggtactcctt cccttcccgc ccttcctctt ttctctttat 13620
cctcagcctc cttctgctat tttgaggaag ttagaagcca ccaccatttt ttcccacctc 13680
aggcaactga gtgtggctgt atttctgtcc catgttcagt tatttccagg aactattttt 13740
gatgaccaac ttgaagttac attgggtggg cctaatgggg gctgataaaa gaatgaggtg 13800
accaaatatg cttgcactga gacggctacg aagtaaggtt tttaatgact tgctttgtga 13860
cttggtcagg agtgatacca tttgtcatgt gtccaacttc atgactaaat ggttgctcta 13920
ccttatcctc atagctataa taaaataaaa taaatacata cattgcaggg aggaatgtat 13980
cttgttaaag gtctctccct tttagcaaca aaagtacata ttatgttgta gaacatgctt 14040
tttctttgat ccttcttgaa cacctattac tctatagagg tatgttgtgt atggcaaatt 14100
agaacaagca atagataagg atgattcttt accattataa cccagtcaag gtctttgtcc 14160
taagttttgt acctttctcc agagggaaag gtatttgtat ttatttattt attttgaggg 14220
cagagttttg ctcttgttgc ccaggctggg gtgcaatggc acgatctcag ctcactgtaa 14280
catccgcctc ccgagttcaa gtgattctcc tgcctcagcc tcccgagtag ctgggattac 14340
aggtgcctgc cacgatgccc ggctaatttt tttttttttt tttgtatttt tagtagagat 14400
ggggtttcat catgttggcc aggctggtct tgaactcctg acctcaggtg atccatccac 14460
ctcggcctcc caaagtgttg ggattacagg catcagccac tgcctccggc caggtatttg 14520
tatttttagt ctctatgcct taccgtctca gatcaggagg atttggtgat ttatcgaatg 14580
tgggggaagg ggaagaagag gaaacgggag gaatgttcca gattagggaa atagctagat 14640
ggaagatgca gcccctcatc aaggtgggga cacaggaaaa ggaacgtgtg caaagaagat 14700
ggtgatctgg ttgtgaccat gttgttagag gacgtccagg gaagcatctg gtaggtggtg 14760
gggtgtttaa atatagaaca ttcggagaat gctccgaagc ttcagagaac ccttcccaaa 14820
aggacaaaac cagctcagtg ttttagcact ccgggatcat atggcatgac agcatggctg 14880
ctttatactt ttttgtgtat gtgaaattaa aaccaaccac tcaggaccaa tttctctgaa 14940
gctttttgtc aatctttcat ttgcttttct cgtctagatt gtaagctcct tgcagccagt 15000
gtctgttgat tcagtcattc aaaaaataat acatgaacag ctactaggta ccaggctctg 15060
tgctgggcag ttgggatatg tggtgaggaa gacaaacttg gtccctgccc ttaggaagtt 15120
cagtagtcca gcagacaaag tggctgaata aagataatct cagttcacag tgataagagc 15180
tcttacaggc ctaggctcca ggtgctgtgg ggatgctcag gaaaaggtat ctaattggga 15240
ttgggagcag gcaaaacaaa taaaggatag tgtataaagg taatatctag ttgaagttct 15300
gaagggcaag gaggagtgag cctgtatatt ctctgagtct ctccctaatc tgggattgac 15360
ttcttgtccg tctctgttca tattaagtgt cacctaggct tgaaagggtg agatcatatt 15420
tcacttcctt cctctttggt cttaaccttt ctctgctacc ccctcacaca atgcatatgc 15480
attattctct tattgtatat atttttcctc tcttcctttt catgtttcct ctgccattac 15540
ttttaacctc gactgccata tggcctctaa acgcttccag aagggtagcc tagtggaggt 15600
tattccatca tggccttgag ctcatgcgac cagatagtga aggcatctgt gtaggtgtct 15660
tctccaggag ggtgatattt gtttcattgt aaattttgta gccctagaac accaacaaca 15720
gtgcacagta attagtaggc aggcagtaca ggattcattg aagtgaagtg ataactttta 15780
tccaagtatg tatgcagata atctttgatt tgtacaaaaa aaattatatt ttaatatgta 15840
aagatttttt aaaagaatct tcaagtttta gccttcccac taggaatata ttgaaaacat 15900
gtgcctagtt cactgacttg cagctgccac tatgagaata aaggtctcat ttagttgttg 15960
tgaattttaa gggatatttt caatgatgtt ggctggttta tcccattatg tggtcttttt 16020
tttttttttt tttttttttt gaggtggagt ctcgctctgt cacccaggct ggagtgcagt 16080
ggcgcaatct cgactcactg caacctccgc ctcccgggtt caagcgattc tgctgtctca 16140
gcctcctaag tagctgggat tacaggcgcc tgccactacg cccagctaat tttggtattt 16200
tttggtagag aagggtttca ccatgttggt caggctggtc tcgaactcct gacctcatga 16260
tccactcact tcagcctccc aaagtgctgg gattacaggc gtgagccacc atgcccagcc 16320

tatgtgctct tattagcaat tctcagtaca cagatagctt tgagtgattc tttcaagtca 16380
agtaccttat taaaaaactc aagtgtactg ataattatct tacttttaaa tggctaagtg 16440
ataagactga atttttaggt actgtaacac ttcagattac agattctgat atttttatgg 16500
ttatttatat ttatttattt ttgagatgga gttttgctct tgctgcctag gctggagtgc 16560
aatggcacga tctcggctca ctgcaacctc cgcctcccag gttcaagcga ttctcctgcc 16620
tcagcctcct gagtagctgg gattacagtc acccgccact acagccggct aatttttgtt 16680
atttttaata gagacaatgt ttcaccatgt tggccagggt ggtctcgcac ttctgacctc 16740
tggcgatccg cccgcctcgg cctcccaaag tgctgggatt acaggcgtga gccaccgcac 16800
ctggcctggt tacttaaatt taaatacaaa aattatgttg attaattctg aatgatttcc 16860
tgattgctcc ccgtttacca ttcacacatt tattaaattc ttcgcttgcc atatagaagc 16920
agtctctctg ccatatatgc catatagata acagaactag ctgtctgcaa accactgaaa 16980
ttgtgaaaac atctcccctt ttttcctgtt tctaattcta gctatgagga ttatatacag 17040
aagtagtcct ggatttgatt tttttttttt tttgatgatt gttttttgat agttgttgac 17100
tacaaatcat ttaaacgtct gaaaggggaa aggttttcct taaaaatgga tgacaaagga 17160
gaataaaaag gtattttgac tatttttttg aatgatgagt tttttttttc tctttcttgt 17220
tttcttttgg agtcatttat gtgtcactga gtggatacca tggaacatgt ggcagaagta 17280
gatatatggg gtaaaagaac catagttcat aagctccttg acagaatcac tgaagtgtag 17340
ccgttatatg gccactgtcg cagggggagg cagcagtttt gaagaagggg atgagtaata 17400
atgagtgata aaaaggcatc ctggatagaa gaccaaactc tgcagaagac cccagtttga 17460
ttatgctttt gttttctgat ttgcggagga gagtgaaaat gcctgagggg tgcgggggag 17520
cacatagggt gtatgtgtgt gtgtgtgcgc gtgcagattc tctctttcac tgtatgtatt 17580
tgtatgcatg tatgtatctt aggacttaag ctttctagtc aataaattgc catagtgggg 17640
aattgcttaa ttgcttgcct tctgttgttg tatttaattt aattttattt ttaatgattt 17700
ttttggtggg gtacagggtc ttaactatgt tgtccaggct ggtcttgaac tcctaaactc 17760
aagtgatcct cccgcctcgg gctcccaaaa tgctgggatt acaggtgtga gccaccatgc 17820
ccagcttagt tgtattttaa atgggcctgt ttgcagcatt ccctactccc cttagtttac 17880
ctggctcaca acctgtcttt ccatatcaag gcttctgtca cccctggccc atgtcagtgc 17940
atttgggcag cccacccagc atcatcacct catgtcccag ggaacttcct gttcctctct 18000
tccagctatt tccttccctg gcagttgaga tagtctctac ctttgaccta ctgttaagct 18060
cagaccttct gctctctagt tacagcctct gtgctgccag attccctcgc tcagttgctt 18120
tctctagttt gggttttctc ctttattcag atttccagct gtttctctcc tccccccacc 18180
gcagcctcct cacttccctc cttatgcatc tgagactgtg gtcagtcact ttagatgctg 18240
cctctccact gtacttgtgt ccatcttctt acctaccacc tctagccctg gagcaggctc 18300
ttcccctgtc tttgtcttcc tgggcccagg ctcctaagcg ctgctggaaa aaaaatcccc 18360
cagtattgag cccctagaaa tccagtcttt aatcccaaat ctgtctcccc cagcatctgg 18420
ccatcagatc taaagcttac ctgccatcct ttccacctca tttctctcac aggggaaaag 18480
gagcctttgc tcctagagtc tgcgctcctg accccttccc atctcacctg ttcaaggcat 18540
cttgcaataa ggggttggtg actctcgagg aatggatccc aggccctccc tattatcatc 18600
ttatgtatgc cagttcaacg ttctcagctt cctccagccg agacggcccc tccagccact 18660
gctttatact ctccttctct ggttgaaatt tttgaagtaa ataggtcact ctgcccatcg 18720
ttcatcttcc agtcactctg tgtgtttatc ttccagggaa gtgaggctct atgctaccaa 18780
gccactgaaa taattttttt ttttttccag actgagtctt gctctgtcac ccaggctgga 18840
gtgcagtgcc gcagtcttgg ctcactgcaa cctctgcctc ccggcttcag gcgattctcc 18900
tgccccagcc tcctgagtag ctgggattac aggtgcctgt catcacgcct ggctaatttt 18960
ttgtattttt ggtagagatg gggcttcacc atgttggcca ggcttgttgg catgttgacc 19020
atgttggcca ggctagcctc aagtgatcca cccgtcagcc tcccaaagtg ctgagattac 19080
aggtgtgagc caccgcacct ggcctgaaat aattcttgac aagatctgct tccttgttac 19140
taatacagtg gatattttgc atcctaattt taatgcagtt cagtgtggta gacctgtatt 19200
tgcatattga atattccctt ccctgtttta ataactctat tttttccttt tcttttatat 19260
ctcctgcttc tctagctagt cctagacctt actcatcggt gtcttctctg tttgttcctc 19320
aacttgagga gttcctacag ggtttaccca atctgctgct ttcatttagc ccttttgttc 19380
tttttgagcc atctcattca ctcacccagg atgtagcatc ggcccttgaa ttcagtgtgc 19440
acacatacac tgtgcactat gggacagcct tcagaggcac tttgttcctg aaattgtggt 19500
ggtctttgcc tctcatggag ccttgcatat gctgtttcct ctgcctggaa tatcctacct 19560
tttacttaac tgattctcgt tcttctttcc agtcacattt tgtacatttc ttctgggaag 19620
ctttctctga tttccccttt ccacaggtcc aagttaactg ccttgtctag gtcctcccat 19680
ggccctctga aggcctcctt tcatagcacc atgtctgagt atactgtaat aacacgcatt 19740
gctctgtaat agcctgttta cttacctatt gccaagtaat ctatcaagtc ttataaaggg 19800
cggggctgct tttgttctag tcatttgtat ctcttagtac ccaatatagt gtttggcata 19860
tagaaaatac ccaacaaggc cagtcgcagt ggctcatacc tgtaatccga gcactttggt 19920
aggctgaggt gggcggatca cttgaggtca ggagtttgag accagcctgg ccaacatggt 19980
gaaaccctgt ctctactaaa aatacaaaaa ttagccaggc gtggtggcgg gtgcctgtag 20040
tcccagctac ttgggaggct gaggcaggag aatcacttga actggggagg tggaggttgc 20100
agtgagctga gatcactcca ctgcactcca gcctgggtga cagagtgaga ctccatctta 20160
aaaaaaaaaa agactccatc ttaaaaaaaa aaaaaaagaa aaaagaaaga aaatacccaa 20220

taagtagttc ctgaatgaat agatgagaat gctgtttaga aggttcatga attggaaacc 20280
gtgattgcta gggaggcttt gagttgatgg tattgtgttg aaccatgtgt tacccaggat 20340
caatttagat tttacacttt gttttctctg ttccttttta tagtaatttt ctgtatgtgg 20400
tgttttcccc ccatgagatt gtataccatt tctcagcgag aactgtgtgt aatgcttggt 20460
ggctccctca tggtgccttg catggaattg gacttcgttt cagtggatct gatcccagtt 20520
atgttaatgc tcgatggagc taagtcttat ctcgaagcag tccatgtctt catcagctgg 20580
ccctgcctcc atgccctgca cagaccatgc cactctggag aggtagtttc cctgtggctt 20640
attagtctta tgttccagtg tgctggccaa gtatgagaga catcagtggt atgagagagt 20700
ctctctcatt caaacttcgt aggttttgta gctgggactg accagtgctg acaggaaata 20760
gaggcattta ttaaaagcca gagatttttc aagttgcagg aagcaaagct cttgttagct 20820
atgattttgt ggtgggtttg gtagtccaat ataaaagtaa aaactggatg acaatgggag 20880
gagcatgctt gggtctccaa agttagatca tttttcctaa gtaatttgtc tttaaacttt 20940
tactggtttg gaatttcctg agattttgat cttgccagaa agtttatagc aaaagttctg 21000
agcagatgac acttttgcgt ctgaaaccaa atcattgttt ttgtttttaa cttttttctt 21060
aatatattat ccttagttca gccctgaaga ttattctgtt atttgtggat ctcaactttc 21120
cccccatctc ctggatcttt gtgaaatgaa tggtattaat tgaatagaga aggaagatat 21180
aaacataaac ttagtcaaaa acttgttctt gactaggcaa gttgggcttt atagctttga 21240
gctgatgaca tgtctattct tgtgaaaaag ggatttttag tgttggtttg gcttcttgtt 21300
atatttgatt tattattatt atcattatca ttatttttga gacagagtct tgctctgtcg 21360
cccaggctgg agtgcagtgg ctcaatctcg gctcagtgca acctccgcct cccaggttca 21420
agcgattctc gtgcctcagc ctctggagta gctgggatta caggcgggtg ccactacacc 21480
tggctaatat ttgtattttt agtagagaca ggtttcacca tgttggctag gctggtcttg 21540
aactcctgac ctcaggtgat ccacctgcct tggcctccca aagtgctggg attacaggcc 21600
ttagccactg tgcctggctg attttttttt tttttttttt tttaggtttg ttttaactgg 21660
aactttacgt gaatgtaatt gaatttagaa taaaagcact taatttcaca gtgtgcagtg 21720
aactttctgt tacttatttt aacagtaaaa ccccttgcag taaatgactt ggagcaaaga 21780
ttgctttttt aaaaaatgtt ttaatttgtt tttcttttct tgagatggag tcttgctctg 21840
tcaccaggct ggagtatggt ggcgcgatct tggctcactg cagcctcccc gcctcctagg 21900
ttcaagcgaa tctcctgcct cagcctcctg agtagctggg actacaggca catgccacca 21960
tgcccagcta atttttgtat ttttagtaga gacagggttt caccatgttg gtcaggatgg 22020
tcttgatctc ttgaccccgt gatccaccct cctcggcctc ccaaagtgct gggattacaa 22080
ctgctgggat tacaagtgct gggattacaa gcgtgagcca ccacgcctgg ccaatttttt 22140
tttttttttt ctttttgaga cagagtttca ctctgtcacc caggctggag tgcagtgtca 22200
cagtcaaaac tcactggcag ccttaacctc ctgggctcga atgatcctcc tgcctcagcc 22260
tcccaagtaa ctgagactac aggcatgtac cactgtgccc agctaattgt ttttttattt 22320
tttatttttt gtagggacag ggtctcgcta ttttgcccag gctagtctac aactcttggg 22380
ctcaagcagt cctcctgcct tgacctccca aaatgttggg attacaggga caagccactg 22440
cacctggcca aggattgttt tttaagtgaa ctgagaccca gccttattag tggtcccaga 22500
gcagacctgg gacctgaagg gaaccctttt cttctggtcc agcgtctttc ctctgatggg 22560
ctactttcct ggagcctttg attgcctgtc atcagagtaa ctgagtttga acagagtagg 22620
tagttcctct ccagaccacc acactcacca gctttcattc tgcttctctc gtttagactg 22680
tggttctgaa tcctcagttc tatttactga gtgtttttaa acataaaaat gccttttaat 22740
gagattgaag gccagaggtg ggacagttga ggacaaagta gaaataaaac cttcaaggcg 22800
gggttgttgg tgggagtctt tttttgtttg tttgtttttt gagactgagt ctcgctctgt 22860
cacccaggct ggagtgcagt ggcacaatct cagctcactg caacctccgc ctcccgagtt 22920
caagctattc tcctgcctca gcctccttag tagctgggat ttcaggctcc cgccaccatg 22980
cccagctaat tttgtatttt ttggtagaaa cggggtttca ccatgttggc caggctggtc 23040
tcaaactcct gacctcaggt gatctgtctg cctcagcctc ccaaagtgct gggattacag 23100
gcgtgagcca ctgtgcctgg cagggagtct tatagaagct gtcgtggaca atgtgggaag 23160
tagtgagcct ttgtattcca gtatgctggg ctccactgtg cttgctctgg cccccggtcg 23220
ctctctgtgt gttattgagt ccccatccac ggccatactc ttcgtcctgc ttctctcctt 23280
accatcctct ccccgctagt ggtaccacgg ctaccactag caattactga catgtgggat 23340
cttagggcta cttccctata aggctgcagg gcatgtggtg ttggctacgc gcatggtaac 23400
catggtagcc ctgtggttct ccacatgtgc gccttgtgac ctgggattgg ctgcagacta 23460
gtaataaact gcgtcttctg gtatggaatc tgtctgtagt tgtactttct acctctgtat 23520
ttaaggggag atctgtaacc taccaatgcc agttgaagag gatggatgat agagatgtta 23580
acaaacagct gaaaaactaa ctacaatggc ctgcaaaata gaacagcagg tttttgtggc 23640
aaaactttgt gtccatgagt ttgtttttta aatatcctca tataatctgt tttaaatcga 23700
gaggctttgg gtaaaagcca tggctagtct tacatgtcat ggagtaccta gcttgtgagg 23760
ttcacagttt attatttaca gagtgtcccc ttaaatcttc tttgggtcgg ttcagcgaat 23820
gttgctcaga tggacttttt tggctgacat agagtcaaaa tggtaatcaa gcatgaaagt 23880
acagacagtc cttaacgcac aaatgtgtca tgcttgaaaa gttggaaagt tggttctctg 23940
gagctctgat tgtattgtcc tgtagaatcc gtgttgtgaa tggtggttaa atcccaaatg 24000
agtccgtaga acctatataa tctgcaatat acctgcagta ttccaattaa tatgtaattc 24060
ccccatagaa ctatgttaat gatttgtatg tatggtattt aatattatac ataataatga 24120

ttgtatgaat aaaaaacatt ctgggctcca tgtggatgat ggggtgtgtg tgtgtgtctg 24180
tctatgtgtg ggtgggtgtg tgttcataga tcccttttcc tgcaatcctg gcactggaat 24240
tggttttatc atttccaatt aagtttcatt cccatgaatt ttggagtaca gactgggtcc 24300
aggtatgcag ggcatagatt agagccctga gaaataggat taggctggaa ttgctgggtt 24360
ggagatcagt agcttccagg aacacttttt gggcctggct gtcttcatta tccccttttg 24420
ttttctcctg gggtctgcag gtattgccct gttttgttcc tctaatatca cttttttttt 24480
ttttctgctt ttgaccaggg tttttgcctc tggtctacaa ctgaatatcc tatcagactc 24540
tcctgatttt gaaataaata tatagttttt ttgaggtgtt ctagcgaatt tctaaatcta 24600
aatgttgtgg cagagttatt acatactaat tttgctatga gaggttgtag aatcccagat 24660
gactaatctt gtaaaccata cacgcatttc catctaattc tccattgtat atcatgttgc 24720
agaaaataac agcctctaga gtttacattg cctcctttga ctatatttct tatttaagat 24780
tagttttcag ataagacctt ttcatggcag tacataactg tacagagggc ttccaacttg 24840
tcttgggagc tctcatctct gggagacatc acattaccca ctgccccctg ccccccgccc 24900
ccagcctgga tgcactcagc ctgtacccca tttctgtcct cagccaaaca ctgctgaaat 24960
gcaagagctt tcaattgcta gccagtgaag atgcagacta agggatttcc atgtagaagc 25020
ccgctctttt cagctggctc gtcgagagct ggaggcccct tgcttgttca catgaggctt 25080
tttgtccctg acttggtggc tgctgtttca cttctcagca gaaagggaca cccttgcccc 25140
cccccagaaa ggaagatttg atgtaccact tccgaaaggt tcagtcgggc atcactgtaa 25200
ccaagaagat aggtcaggtg aggctggagg tggaacaggg ctgctcgcta gaactccaga 25260
ttgttccaca agtgccttct ggcagagaat gatggaagct tccgtgattt ttttttctcc 25320
ttaatagtta tgagcacaga agaggagcag attgtctggc tatagaagct gtcttatttt 25380
ttatttttgt ttttgagatg gagtctttct ctcttgccca ggctaaagtg caatggcgcg 25440
atctcggctc actgcaacct ccgcctcccg agttcaagcg attctcctgc ctcagcctcc 25500
tgagtagctg ggaattacag gcatgcgcca ccatgccaga ctgatttttg tattagagac 25560
agggtttcac catgttggtc agtctggttt cgaactcctg acctcaagat ctgcccacct 25620
cagcctccca aagtgttggg attacaggtg ttagccactg cacccggccg aagctgtcat 25680
attaaatagc actttctgct tttagcaaat ttaatccaaa tgagacttta gattttcttg 25740
ctctgactta ccagcagttc cttgaaacac atttaattat ttttgccaga aaatcactca 25800
agcacttacg ccattttttt accgtgaaaa tatgctgcat tattttaaaa tatattagaa 25860
gtcagtaacc ataagatttt atatgttttc taatgtattc tgtaagcttt ctgctgcttt 25920
tgtttggaag gtgtattttg taacgtagag gactgcttta tctgcttgta agcttgattt 25980
ttgtttttac tgtaattttt ttttcttttg ctgtattgag aaatacattg agtaattata 26040
aagtcagtgg catgtttata agttaatatt tgtatctatt ccttagttac tctaactcaa 26100
aacctaaagt aatcttcaac tctaatttac tctgacatcc agttgactgc caagtcctcc 26160
aacttaatcc ttatcctttt ttttttaaag agatgcagtc ttgctttgtc acccaggctg 26220
gagtgcagtg gtgcaatcat agcttactgt aacctcaaat tcctgggctc aaatgatcct 26280
cccacgtcag cctctggagt agctggggct acaggctctt gctaccatgc ccagctaact 26340
ttttattttt attttttata gagacagagt ctcactgttg ctcaggctgg ccttgaactc 26400
ctgccttcag gcggaactcc tgccttcagg cggtcctcct gcattggcct cccaaagtgc 26460
tggaattaca ggcccaattt tattcttggg atgtatgtct gaaactcttt ccttcacttc 26520
cttcccaagc cttagttcag gcccttctca tctgtggtct tcaaagtcgc cttcagctgg 26580
ttcaggtcct tcctttctgc tgtatctttc atgggaggac atgttatgta tcactgtcct 26640
acttgaaaac ttccattccc cattgatgag ggtgttacct ccagattcct aacacaggtg 26700
ctgaaggcat gcctggataa aggcactccc ttgatctcct ggccaggtcc ccgtacacct 26760
gcagcgcatg ctccacattc tgtctttact gatgctgtgt cttctgcctg cggagccacc 26820
caccattcta ttcacagccc ctgcctcagc ggagcacgtg cctccctctt cctacactga 26880
gctgtccttt ctattgaatc ccctcttttt tgtagtatgg gaaatatttt attatgaata 26940
ctcttttctc tgttgcctcc gtgaccacgt taactttgcc ctaattcgcc ttaggactcc 27000
atctgcttag gggaaagtta ggatttggtt acagaaagca agctgctaga aagaacagtg 27060
tttagcttct gacaggcaaa ataggatttt gcaacatgct tttccttttt aatgcttaga 27120
cattttatat gaattaatat ttttatttgg ttgcttatac attactttct ttttagctag 27180
aatgtgaacc ctataggaac atggggattg cctttcacat ctttgtatcc tcagtaccta 27240
atgttcagtc accctgtggt cttgtgtcgt atatacattt agccttcctt aattaaacca 27300
tatgtactgg tccccgtccc ccacccccaa atagagagaa agaaattcct tgaatactac 27360
attgccagta tcaaaccaca ccttgatatc ctctggggaa agggaggtat cagttgaaaa 27420
gagaaaagag gttaaaatct aggcattaaa atgtgtaagg cttagatgct ggcaatttaa 27480
ggtatgtttt cctgaggtta attttgattg tgtgcaaatt ttacctcata tctaactgta 27540
ggatttagtc accacataag atgggatacc tccataaatc cttcagaaat gtttgtgaaa 27600
ttaaataaag ccttattgaa gactcagctc ttgagagtca tctacctacc taacagttat 27660
tcttgaacag aagagtctta cttttcccta taaggcagtg tgatagccat ctgtatattc 27720
atataattta tgttggcgct tacttcattt aaaaatgtat tccgtgaatg cagttgccag 27780
gcggtgtgct gatcagaaac gtgtaccaat ggcctctttt ataattataa gaggaagacc 27840
aacctgaaac agtcacacaa atgattaatt ttaattgtgg aggagtgctg ggaaagaaaa 27900
ataaaagatg caatgcaagt gtttacaaag gagctttgag cttgtttgaa gtggtccttg 27960
ggcacttaag caaggcttaa agaatgatgt gattagaagt ggcttagcaa ttctaaagaa 28020

cacagggaag gcgtgtggcc agaacattgg tccctagagc acatcgcctc ctgacatacc 28080
atttccttaa gttaatgttt taccactata cataggccct cccctttgtt tacccagatt 28140
tttttaattt taaggatgtt tttaataact tagaatcctg taatttgttg aacagtcctg 28200
tattcccttt acttatattc cttgagattt tataaaatat tttttacatg tcccaagtct 28260
tgattatatc tttttacctc ttgttaagaa atacttactt ttctattttt atgctatatt 28320
tcatgtttac tgtagaaaac aaaaaaagta aaatttttct ttattcctat cactgcagct 28380
tataagcact ctaaacattt tgatctatat tttgccaatc atatatttta gttaaaattg 28440
ttgttgacat aattgtagat tcctgtgcag ttgaaagaaa taatacagag ctgagcgcgg 28500
tggctcacgc ctgtaatccc agcactttgg gaggccgagg caggcagatc atgaggtcag 28560
gagtttgaga ccagactggc caacatggcg aaaccctgtc tctactaaaa atacaaaaat 28620
tagctgggtg tggtggcggg cacctgtaat cccagctagt tgggaggctg aggcaggaga 28680
atcgtttgaa ctccggaggc agaggttgca gtgagccgag atggtaccat tccactccag 28740
cctgggcaac aagagcaaga ctgcatctca aaaataataa taataataat aaataaactt 28800
taaaaataaa acagagagat cccatgtgcg ctttgcctag ttcccccatc cactgcccat 28860
aacattttgc agaactgcag tacagtatca caaccacaat actgacattg atacagtctg 28920
ctcatcttat tcatatttcc ccagtgttac tcgtatccac gtgtgtatgc attgtgtttt 28980
caatactctt ttattataaa gctgttttta atgtgattca attctaggtt gttttgttct 29040
gccctcaaaa agcattccct ctcctaatca tatctccgtc atacccttgt atgttttctt 29100
taaacctgtt ttaagaaagc agctacctgt aagagaaatg agattgaaaa cagaattgcc 29160
aatctgcttg tactttataa gcctgttgat tgtttagata cggtttagcc agtttatagt 29220
taccctgggt gctgaaaggt atgctggatg atacctaacc aacagagaac cattgaatgc 29280
cgttcaaaat ggactgaagc atcagcaatg tctgaaaaag gcctgacagt aatgtacatg 29340
tcaaatggcc cgtaatttaa gcagagtaga gtaagtagaa gaataaacat ggggaaagtt 29400
ccagcaacag aggaggcttt gagcttttgc tcttcatctt gagtggatgt tgttctcagg 29460
tggtaatagg ccatcgagct ttctccactg gctgcctctc tggggaacaa ataaccgaaa 29520
agatactcag caccctggtt ggtacatagg tggtcagttg atttatactt cctggttttc 29580
agtgttgctt gaattttcta aatggaaaca cagtaccttt ataatcagaa aacaatcccg 29640
agttttgatt tgagggtgtt gtaaaaagtt aaaaaaaaaa aaacagaaat gtgaaaagga 29700
agttgtgtta gagtatttgg agttgagaaa gcatgaaaag gacagaagag aagctggttg 29760
tcaggttgca tggggtagct acaagcacac tgaccagaaa gtcagctgga aaaaaaatgt 29820
agaaacagga gataaaacgg ccaaggggct atacaagcaa acagcaagga cctgagaaga 29880
aaaactagtt aggtgtgact gtcagagtga tgtgtacagt gtgatccttt ctgtgtaaaa 29940
acaagcagta agaattcgct gtttacgttt gcgtgtgttt ggagaagagt ggggaagagt 30000
aggcactgcc agactgtgaa cactggttag gttattgtta tatctttgta ttatatacac 30060
tggacatgtt atttgtataa tatgagaaga aattttataa atcattaaat cttttggcat 30120
ttaggaacat ttgtgttttc taatagttgc ttctatacta ttatctttat tatatgccct 30180
tcatcttctc agtgtttggc tgttgttgtg attccctttt gtgagcagtg ttgaagttag 30240
ctaatattca tttcttctcc cttctttcac cctcctccag agtctgattt gaagtattcc 30300
tagctgctac ctataaaagc aataagcaag attgttttac ttttcacaaa ctcgtcctgt 30360
tctgtgcctc tgcctcggac atagctgtag tatagagtgt tgtctccctt acatccttct 30420
atcttagacc tactagtaaa tattaatgct cactctaagt tcttctcaat tctttttttt 30480
tttttttttt ttttttttga gaaagagttt cgctcttgtt gcccaggctg gagtgcaacg 30540
gcacgatttc ggctcaccgc aacctccacc ttctgggttt aagcgactct cctgcctcag 30600
cctcctgagt agctgggatt acagtcacgt gccaccaccc ctggcaaatt ttgtattttt 30660
agtagagaca aggtttcttc catgttggcc aggctggtct caaactcccg acctcaggtg 30720
atccacctgc ctcagccttc caaagtgctg ggattccagg cgtgagccac cgcgcccagc 30780
ctcttctctc aattcttcct gaagctcttt ctgcactaga ttcctcagga agggcttgtg 30840
ggaacaatct tctgtgaatc aacagtacat attcataata gtttgtcagc agcctattat 30900
tttaaggcca tttggtctgt atataaaaat gtttggatca cattttcttt ctttaaggta 30960
aatatgttat tctgttgtct tctggtataa agcattgctg taaatgtttg acagtctaat 31020
tatcttttgc ttataagtga cttagggttt tttgtctatg tgcccaaagg attttttccc 31080
tctttctctc tttttttttt tttttttttt tttttaaaca gacaggatct caccctgttg 31140
cccaggcttt agtgcagtga ggcagtcaga gcttactgaa gttttgaact cctgggcttg 31200
aggaacaaag gattttttta accttttaat tcaaagtctc atcatttatg caaccatgtc 31260
ttggtgttgg ctgttttggg ttgttctccc tcaaaaatcc atgtgctctt tcaatatgta 31320
gttttaaatc tttttttttt aatttcagga aaatcttgaa ttagagtttt ccgtttttcg 31380
tctggtacat tgcttgggtt tccttcttca ggaactcagc ctgttatgtg tatgtttgat 31440
cttctttgcc tgtcgtctgt ttctttcact tcctctcact tttttaaact tcatttatta 31500
aaaaaaaatt tttttttcga gacagagttt cgctcttgtt gcccaggctg gagtgcaatg 31560
gcgtgatctc ggctcactgc aacctccgcc tcccaggttc aagtgattct cctgcctcag 31620
tctcccaagt agctgggatt acaggcatgc gccaccacgc ccagctaatt ttttgtattt 31680
ttagtagaga cagggtttct ccatgttggt caggctggtc ttgaactcct gacctcgtga 31740
tctgcccgcc tcagcctccc aaagtgctgg gattacaggc gtgagccact gtgcccagcc 31800
ttattaaaaa ttttaaaaac atacatttaa acttaacaga aaaattatga gagagaaggg 31860
ggtggtgcca ggctttttta aacaaccagc tcttacatga actcatagag tgataactca 31920

ttaccatgag gacggcatca agccgttcat gaaggatctg gccccgtgac ccagacacct 31980
cctactaggt ccatttttaa cattggggat cacatttcaa cgtgagattt ggagggggca 32040
aaactacaaa ccatgtcact cagggattgg aggagcaagt accacctata ctttggactc 32100
aggtagaaag gcaaaatatc caggaaataa gctgctaccg tccagggttc agcagaggtg 32160
cccatcagcc tgccaagtac tcaagagtcc agcctctagg gagctaatca tcatggtgag 32220
ctcttcgagg cacagggagc tgggaagaca gtgcttgcca cccctgcctg aatagtgttt 32280
gcacagagag ttctgttgtg tcttgattgg gtcctcctgc cactgggaat gctgtggatt 32340
atactaggtc tctatctggc ttgtttcagg gctccatgtg aaaaccttct tgatatccta 32400
gccatccacc tgctcagtcc ctagtttgca aggaggctgt ggggagccta gattctgtgt 32460
cagatagaat gtactacatt ccgtctcagg aatgtaccac atcagaaaac agtgcgacct 32520
gcaggagaag tagaggtgaa gaggcacatt cttccgagaa atgtttctct caacacccag 32580
cattccctgg atatcagcag gaaattactc actgctagaa aatgccccat gagccttctg 32640
ttaaggaggt caagggagag aacagagaaa gttctcaaag ttgacttggt cactggtact 32700
ttcttatgcg gttcttattt tgtttgccat cgtcatcatc atgctatgtc tattttctca 32760
atccaaatcc actgctttca ccttggttct ttctgaccgg tttggcacac tcattcagta 32820
aatccttatg gagagcccaa tgtctgcata attgtgctgt gctgatgacc aagctagacc 32880
tacgagtgtc ggctcctttg agatgtacgg gacagctctt ctgtcatctc ttctgggaag 32940
cctctccagg cttggtgaac agtggcaaga tgtttaacag ttgtacatgt gtcccatgtt 33000
cctttctaag agcctgggca aaccagaccc ggtcgcaggt catcgtagta tggcgtgagc 33060
ttcctctctc ctttctgacc ttttgtgtga tggcaagaac ctgcagagtg acacaagcag 33120
caggcttctg aggttgctct agcctcagaa tggccgtccc ttctccaccc tggccctcat 33180
tgctgaggtt tcctttgaag caacagtgcc ggaacagact aggggaagca gcttggacat 33240
agctgtatga tttattacca cccattgagg ccaaccaaag tcggcaagga gaggtagcag 33300
gtcagtggtg cctggaagct tcctctttcc tttgcaccag atgtgactgc tctgcaatta 33360
ctcctaaatt tgctactctc gtttttacta gccaaccttg atgtttttcc cttcttcctg 33420
tagaatagac ttcccctctg atcagtactt tctactcaac actatttgtg gccacagtgg 33480
gaactcattg aggacaggga ccatgacatt actacctgac ccatcaacac ttggcataac 33540
ttgaaatgca aggacaaaaa ttggctgcaa gtacaatgtg gtcttcactc tgaaggtgat 33600
ccttaaaact tggctttggc atcatattgc cttaatatac ctaggggatt gggtaaaacc 33660
agttacttta aaagagtttt acaattctgg ccttctagct atcttgtctt cttaaacaag 33720
agcacaagat gaatgtatct tagtgaaatt ttatatggtt tgctttgagt aatcttgcga 33780
agattgattt ttagcacagt aggaaagaca cattctaata gtgatttttt tccccgagtt 33840
tatgtactgc tgttgcatga aaatctgact agatttaatg ttcctaaagt tctttgttca 33900
tcctgatttt tgcaggtcct agggaaagct ttgttttcct cttaacctaa cttagatgtt 33960
gtcatttcat gagctttgga ggaagagtgt atagccaatt gtgtaatgtc tttaaaggat 34020
attatctctg caatagttgt ttataaggcc taagttattc atgtaataat agtggccccg 34080
gatctgtttc tagcaatagg tatatggatt ttggttccta tatagttgta gttgtggctt 34140
tgagatattg agcaagccct tttaagaaag gatttggcat ccctcagcct tcaaaagctt 34200
ctcaaaattg atcatatgtt attagcaaag gtttactgcc tgcttccatt gtatagacaa 34260
tttatttttt atgtattccg ttctaagaag gcagatgacc aaaagatctt gcatctgttg 34320
cccaaggctt gtgactagag aggaaagaga taagaatact tttttaaaat cccattttac 34380
taaatatgtt gaggaagtgg taagatatat taatttgttg agatttttct gttatgccta 34440
ttatatgaaa taggtactct gaacatggct tcttaattaa atatatttga taaaatacaa 34500
cttgcttccc ctggagttta gaagtcagat aactgccatg gagagctatg ctttctttgt 34560
tttaaagatc tgcttatgaa catgataaac aggaacaatt taatgttttc aatattttct 34620
tgtattttac tgcaagttta tacacaacat aaatatgggg gaagggggaa atgtttatac 34680
cagagccatc ctgcccattc tttccttaca gaaggacaaa ggagcagtat ttattttaac 34740
tacaaaaata ctattgtagg ttttaaaaat tccgtatatt ttgatatctt gtgttcctct 34800
tgacctttaa tttgctaaat agttgcaaag aatgaaggta acctgcatca tcttcttaaa 34860
aaccaactct atctaattat aatagtttgt ctatctctga aaaatagtga tgtgttcatt 34920
ctgaaatcag aactaccgga tgcagctgca ttttgttact atttgaattt cgggagaggg 34980
aggaggatgc agcctttcga gctgctgaaa tacacaaaca caaagaagac accaagcata 35040
gtagaactgt gttaagctga ccaagccaga agaagcacct attctcagca tagtatgaga 35100
cgtaaaggca atataatggg catagttgaa gatggtagaa ggaaaataga ctctgatggt 35160
ttaatgttaa atgctttttt taaaaaagtg gtattccaat atcgaagaag aagactttct 35220
acttttagaa gcaataaagg aaattgcaga ggaaagggtc aataggttgg aatacataaa 35280
aattaaaaac ttttaaactt tttttttttg agacagagtc tcactctgtc acccaggctg 35340
gagtgcaatg gtgcaatctc ggctcgctac aacctccgct tcctgagttc aagcaattct 35400
cctgcctcag cctcccgagt agctgggatt acaggcatgg gccaccactc ctggctaata 35460
tttgtatttt tagtagagac agggtttcac catgttgtcc aggctgatct caaactcctg 35520
acctcgtgat ccgcctgcct cggcctccca aagtgctggg attacaggca tgagccaccg 35580
cgcctggact aaattgtttc agtattaatt ttttttaaaa caagatctta ctgttgccca 35640
ggctgaagta cagtggccca atcatggcta actgcagcct tgacttctgg gcctcaaggg 35700
atcctcccac ctcagcgtcc cgagtagctg ggaccacaga catgtaccac cacacccagc 35760
tacttgtttt atttttattt ttgtagagat gaggtttcac catgttgccc aggctggtct 35820

cgaactcctg ggcccaagca atcctcctcc cttggcctcc caaagtgctg gtattacagg 35880
tgtaagccat tgcgccctgc ctgatttttt aaatgtgcaa acagataagt tggaaaagtg 35940
atttccaata aagataaaga gttgatggtt ttaaaatacg taaagagctt atatgaatga 36000
gaaaaacact aacattccaa aagattagaa ggcaaaggac agaaagaaac aaatcactat 36060
gtctgggaag ggacatgaag gagcaggttc ccactgggcc agcggggctc aaacccactg 36120
gggacgtccg agagactgca agggccatgc cttcacattg ccgtacctga gaagcaagga 36180
gctggggtat ttatctcttt cacactttgg gaggctgagg tgggcggatc acctgaggtc 36240
aggagttcga gactagcctg gccaacacag tgaaaccccg tctctactaa aactagaaat 36300
aattagctgg gtgtggtggc acacacctgt aatcccagct acttggaagg ctgaggcatg 36360
agaattgctt gagcccagga ggtagaggct gcagtgagca taaattgcac cactgcactc 36420
cagcctgggt gaaactctgt ctcaaaaagt aataataatc atgataaata aaataacatt 36480
agattgttag cagaagtagc cacaggtttc tcccacctct ctgcaagttg ctgagtgtga 36540
ttcccatcaa gaggtacaat gtctttttat ttttatttta tttattttat ttatattgcc 36600
tatgttgtct aggctggttc caaactcctg agctcaagtg atccttctac gtcagccccc 36660
caaagtgttg ggattacagg catcagccac tgcacctggc ccagatactt tttcttgagt 36720
aggaatttcg agtcaccctg aacattgcat gccttcgtag tggggaagac aataggaaac 36780
cacaggctgt aggctaaaat gggttgtgtt tcttgtaacg tcatgacaag gcataaccca 36840
tcttggcata gtaaatagta agcactcact gaactgatga ttttaaatct ttgctgttta 36900
ttcagcaata tcctaaatta gcgctatgtt agtggagttg catctccctc atggattagt 36960
ctgaaaaaga tgagaaatct gtatgtagac caagttatcc ttaaactgct cataatgtat 37020
gatgcacgtg gttttacgtg tacagcctgg taccattgtt cttaggcaca tttcagtgcc 37080
agaactctta atacccagga agaagcaaaa agaaagatgg aggtgcagct agaggttgtg 37140
gcctttgaac gattcattct gccttaataa gagtggtctg gctgagctcg gtggctcaca 37200
cctgtaatcc cagcactttg ggaggccaag gcaggcagat cgcttgagcc caggagttca 37260
agaccagccc aggcagcata gcgagacccc ccctcccccc gtctctacaa aaaaatagaa 37320
acaatgagcc aggcatggtg gaacgtagtg cgtggtgcct gtagtctcag ctacccagtt 37380
ggctgaggtg ggaggatcac ctgagcccta gaagtcgagg cttcagtgag cccttattgt 37440
gccactgcac tccactctag gtgacagagc gagacaggtc ctgtctcgaa aagaaagaag 37500
aagaattaaa aaaagtgatt agatcccttg tgtttgggac acttgttggc agcagggatg 37560
gtagcgttta tgagggttgc atgtaacatc gcctagctca gacatctgtt tgactgtctt 37620
cccccctgaa gcgcaggctc tgtgagggca ggtcttttgt ctttcttgtt aatcttcata 37680
tgcttagtgc ttgccacata gttgatgctc agtcgatatt tggatgaatt gaagggatta 37740
atgcattgaa tctgaacctt gctttcttaa tgcatatggg gagttctttg gaaagccaca 37800
cagaggagct tggttgcctg cttcctctct tccccagatt gtctttttat tgttgtggct 37860
tcactgaagc actctcactt caaataattt tgggcattgg tcgtatttta ttctttgttc 37920
cttcttcatc cttacccctc agatggtatg tagaaaagta cactacatct agaaagtact 37980
ttataaactc atttggttga taataataca tatgcctttt ccttggtcct ggtagcagaa 38040
tcttgtgcca ctcttggaat acaaacgaaa ttcttaacca aagccagttt cattttgatg 38100
ttctattttc ctcccattca cactccaaat tgtgcaccaa agtatcatcc tagttttgtg 38160
aggatggttc tccatacttc agggtaggag tatcatgtgg attcctatga tacctttctc 38220
cctgggacca tggagggcag cagctggtga ttgatagtct gattcccggt gaggaaagct 38280
gtgagccttc cacttgcaga tgtctgccaa ctacatgtgt ccttagtcaa ctgtaccact 38340
gtcctccggc aaacagcaga agcccagggc ctgaagttct taagctgtca ttatggaaag 38400
cagaaggtaa acaaaacaga agtgaaagta gatttaattt tttagactgt tctcttacag 38460
gaatggtttt gtggttctca gcattttaaa aaaaatagtg gttccaatat gttttattga 38520
catcaattac tgtaagtctg attcattttc tgcctattga tttctaccca aggtgaaatt 38580
catgacattt aacagaaagc ataagtgatt ttttaaaagc agacactatt agggacggta 38640
aaaataagat ttaaagtcgg gacacttgaa aaagcaattt ttataccttt ggtaacgatt 38700
ctattctgat tctttgtata aataatataa acaaaggctc tagaagctta ctataatgaa 38760
gttggtgtgc tgtttctaaa ttctggttta aggcccaaat tcattttatc tgcattaact 38820
tttttttttt tgagagtctc gctctgtcac ccaggctaga gtgcaatggt atgatctcgg 38880
ctcactgcaa cctctgcctc ccgggttcaa gcgattctcc tgcctcagcc tcccgagtag 38940
ctgggattat aggtgtgcgc caccacgccc ggctaatttt tgtattttta gtagagacgg 39000
ggtttcacta tgctggtcag gctggtctca aactcctgac cttgtgatcc gcctgcctcg 39060
gcctcccaaa gtgctgggat tacaggcgtg agccactgca cccggccgtg ttaaaatttt 39120
tcagtggtag accactatgt caatatgttg ctttcactga caacagtatt ttcttaaaga 39180
taggataccc catttctaga tgaatctcat tctagctgga aaataatttt tcagttctga 39240
aactacatca ggcctcaggg aatcaaaact agctattagc cacacacata taaagtggct 39300
ttgctttata aacgatttag ggtcaccatc aatgacaatg gtcccttttt attgtatttt 39360
taagagtttc ttatcttaaa tggctgcata actgtagagt tttaaaaaaa ttaagtaaat 39420
gaccatgtta atgctctatt aagcttccaa acaatattgt aatttacttt gaagattttt 39480
ttttattctc aacatcctgc agcttgaccg tttgcctccg tgtctcagtg ctgcttattt 39540
tgaggtgtgg actggagtcc atctgtcccc cttgcctctg aactgctccg ttttgtgttt 39600
cgtaattctt catgctgcat cctgggcgca tttctctgta gtagctttca atttgctcat 39660
gctttgactg ggcttagtct agcgtttatc ctatctctta aggtttttta aaaaattttc 39720

atgattattc atttatttcc aggatttctc atttcttcag tcacatctcc ttgttctggt 39780
tttacttctt cctgttttta ttcataacat cttttttata cacgattcct tcatgtattt 39840
ctaatcttaa gtatatttaa ttgcttattt gattcttttt tttttttatt gagacagggt 39900
cttactctgc caccaggccg gagtgcagtg acatagtcat agctcactgc agcctcaact 39960
acttggactc aagcgacctt cccacctcag cctcccaggt agctaggaat acaggtgtga 40020
gagccgccac acccagctga tttgtcttac tatgttgccc aggctggtct tgaattcctg 40080
ggctcatgtg atctgccctt cttggcctcc tgaagtgctg agattatagg tgtgaaccac 40140
tgcacctggc caagtatgtt tatttattta ttctaatttg agagggagtc tcgctctgtc 40200
gtgcccaggc tgtagtgcag tggcacaatc ccagctcact gcaacctctg cctcctgggt 40260
tcatgcgatt ctcttgcctc agcctcctga gtacctgggg ttacagttgc gtgccaccac 40320
acctagctaa tttttgtgtt tttagtacag gcggggtttt accctgttgg ccaggctggt 40380
cttgaacttg tgacctgaag tgatccgccc gccttggcct cccaaagtgc tgggattaca 40440
ggcatgagcc accacgcttg gcccaagtat gtttattttt aaagtcccca acaagctata 40500
caataaattg catatggaat ggatttttgt tctagttgat ttgttggtta tcatttgtag 40560
aactaactag ttgtcttctg tgtttgatac cttgcttcta ggtcattttg agttgggagc 40620
cttttgtttt gtttttattc tcatgctgtt tttgagccta gctgtgcctt tatggttttc 40680
tctaaattta attgaccatt gttttatatt tggagcagtg ggtgtacatc agagtgtgaa 40740
agcagcccca ccctctccac cagaaggtct ccatgccagt ttcacgaagc atttttcatg 40800
ccctcattcc tgcccttatc ccttgatttg tggggagttt gtaaagcagt tgattgtttt 40860
ttttccacgt agttttccaa gtgcacataa ttgttctgtt agtgacttgt agctccatta 40920
tctattaacc ttgccccaga ccactgtaca agcggaccca acgcttcctc cagctgtggc 40980
agggacagtt acttggtatc ctgctgcctt ttcaatgctg accagttttg ccccttcctc 41040
ccctcaaccc ctgtctttca ttcaactatc accaaaccaa aagattctgg tttgcttttt 41100
agtatgtgtt cttattcagt acatagtcat tttaaaattt aaaccaaaac agacttggta 41160
ctgattagct taattttaag ctttttcttt attattaaac agtgtagttt atcttagcat 41220
ttcatattaa gtatatgatt tatttcatat tgcttatatg aatgtacaca taaatataat 41280
aaaaatattt tcctaaggtt tttgtagtaa attatatcgt ttcattaact ttcatatata 41340
gcattgcttt tgacctggaa gacattgaac ctctgatgat ttgtatattc ctcggagtat 41400
actttgttac atagaaattt tctcatttat aatgagattt gtgattaaca aaatttgttc 41460
aacatgcatt actttgaaga tctggtttct aaaattttat gctagttacc ccaccccccc 41520
ttctatatat atctccctat tcagcgacta ctgcaagagt tccaggaaat gtacactgtg 41580
tgttcactta ctgcatttta aatcattgcc tttactatat ttctgcattt cccttcaatc 41640
tagctctgtc tgtacatttc tgaaagccag tagcttccct gaagaaccag gtaacaaccc 41700
gaacaatcaa attagataac catttgtaga atggaggttc cgggagatct tagaagatgt 41760
gatgggtgct aagggacttt gtagttccct gaagttccag tgagtaaaag gtacccttgg 41820
aattttttat tccttcagac ttttaaaaca gagatcactt tcaaaaatta ctctttctgc 41880
tttgaatcca tgttttagta actattttga cactgtttgg tcagaaggct gtgtgggtca 41940
actgcaaata aataaaataa atgtgatttc agtaatttcc attttgtaac aagtaattga 42000
gaaaatagga ttggatcaga tatttgctta tacacattcc ctttcaggag cacttctgtt 42060
ctataaagaa tgttggtata ttgttaagga cacttcaagc tttgggaacc tttgaagtat 42120
ccattgattc agttaacaaa attatgttga gtgcctaccc tgggcctggg cctgtgttag 42180
gaggggacac taagatgaga gtccaaagca cttcttctca gactcctggc tgctaatggg 42240
ttgctgcctc tacttcttca cttagcagat agctttaaaa tgagtaatgc attttaccat 42300
ggagcccgta agagacattc acccagttgt ggaccgagga gaagggtgtt aaacccagat 42360
tgtgatgttt cacttgatga agtgcttaat ataaacatgg aaatatttcc gcaaggataa 42420
actggctttt atgcctgtgt gttttcagga gaaatagaaa tctctaatca aatattgcca 42480
gcttttcacc caagtttgac tttttgccta attgagtttg ggaggtgtct gaataatgga 42540
taatgagctt tcctgaataa atataaaaat taattaactc caggctctaa ttcattctgt 42600
taccagagtt ttgtaagcat gttacccctt tgtgttcatt gggagatcat ctgttacctt 42660
cttaaatgag tggggaagga tgggaaatga ggaagagcta taaaaactat tcaggtgaag 42720
aaggtttctg cccctccttg ccccttttaa aatctccagc tcagcagatg ctttgtttaa 42780
acttgatcaa gtgcttgtga atcttcctag cctagctaaa tcataacttt ggaaggactt 42840
gcttttttct ctcatgacaa tggtttacca cagaaatgat tcagatcact ttgtgtgcct 42900
gatgcctatg taaaatgata cagtgaaatg gaaaccattt acctgtaagc tttgggcaca 42960
cccaagcctg cttcaggagc acatgatcag gcgtgcactc tgggagagcc gtacacattt 43020
gacatctatg atgtgtggcg ttttattcta tcacatttct gaaatctaca ctaagagaaa 43080
ggaggctctt aaaaaaccac tgaggtgtgg actgggggaa ggagagatcc gtaaagaacc 43140
tgtttgttac ctgttgatac tatttcccat tggtaaaatt tctaatttag tgtgatccag 43200
ccctgaaatg ctgaggcaca cactgaatga ctcctgacat ctttagtgtt tttgttcagg 43260
ggactcttct gggaatctgt ttcatggcaa gtttattatt cccttttggt ttggctcatc 43320
agtttaccca gcagtcatct taatcggttt taaaggcttt tattttattt tgttttctct 43380
gtggaaattt tacacattca gtagattaga agtagttatt taatctttgg ttagcataat 43440
aaaagatctt ctagggacat tttttgcttg cagtggaagg ctagttaaat gtgttcatta 43500
gtcatgaatc tgctttttct atagctgttg gaaacgtagc tcccctgtga tacagttgta 43560
gaatacagaa atctcgtttt gctgttacgg tacggtagtc tacttacttt cttccaaacc 43620

attaatgtta tagttacctt taattgcgta ggtcctatca cccctcaatt ttaagactct 43680
aagcctggca ttttatctta caaaatgaaa tataaagact tgtactcaga gtatgtgtgt 43740
gttttccata taccattcta aagtagagaa agatgaggga ttcgccagaa actgatttct 43800
aataaattat ccagaaactg accccttctc acctcttctg ttactgtcac tgtggtttca 43860
gccacagcat cctttgctgc attgttacct tagtttcctg actgtatcct tccttacacc 43920
attgatccct gcaatcccat ctgcgcgtag cagccagaag ggatccactt actgctgtga 43980
tcagaaatcc tcagccaggt gcagtggctc atgcctgtaa tctcagcact atgggaggct 44040
gagactggag aattatttga gcccaggagt ttgagaccag cctcaaactg ggtaatataa 44100
tgagacctca tctctacaaa caggaaaaaa aaaatttttt tttttttttt aactagccag 44160
gtatagtgct aatatacctg ttctgggatc cagcatgctc tccctgacct gcagcttcat 44220
ctccaccact ttgcccctca ctcccaccac aatggctttc ttctcttcct cagacatgcc 44280
gtgcgtcctc ctacctggaa tattcccctc caaacattcc catggctcac tccctcacct 44340
tcatcagatc tctgttccag tgtcactttt actggaaggt cttttgtgac catcctactt 44400
attataaaaa aataatctgc ccaaccttct ccttttattt cctctacttg atttttcaat 44460
ttagtactta tcagctgaca tatattttgt ctctctgtct ctctctgtct ctcatagaag 44520
gtaaattcta taaaggaagg aatttttatg tttggttctt tgctgtagct ccaatattca 44580
aaacagtgcc tgacacacag taggcccttt atatttgttg aataaatgtt gacactctga 44640
tatctaattt ttgtctggtg actaatacga aaactataga gtgataataa aagcattacc 44700
ttagtagact ggaaagggat gagcgctagg atgaactttc tgcctggcga tcttgctgaa 44760
tttaggaggc agattggggt tcaaaggagg ctgaaatggc taggatttgc agagcagggt 44820
actaaggatg agcaggctat gacagaaaga actccagaaa tctgcaaagg gatcaccttg 44880
agtctggctg gatacagtgt acactttgta gggtgtctct tcatgagctt ggataaagaa 44940
caactgttgg ggagtggata attcccagca ctcattcaag cttgcatcgg ccagaacgga 45000
gagagacaga cctctgtaat acgtaggata tttggtagaa acattcaacc gaaaaccatc 45060
agatatgcaa aaagtaataa taataagtaa acaatgtgat gcatagctag aagaaaaatc 45120
agacattaga agcaagccca gaaatgacag atgataaatt agcagataag gacattaaaa 45180
cagctattat aaataactta gcagatttaa agaaaaacaa cataatgagg ataatggaag 45240
aaaaacaacc gaataccatt tctaaagaag aaaaatacaa tatctgaaat gagaatttag 45300
ctggatagga ttaatagttt aggcactgca gaagaaaaaa acagcatcta tatgagaata 45360
tacccaaggg aagtacagag aggaaaaaaa tgtggattgg ggggtgcctc agtgacatat 45420
ggaacaatat taaacaagtc tgcccccaaa atacttgaag gaataaggtt caagtttttt 45480
ccaggtttaa tgaaaactat aagcctacag attcaagcat ttcaacaaac cttcagcaaa 45540
ataaacaaaa ccacagtagg cctggcacac tgtctcatgc ctgcaatccc agcactttgg 45600
gagcctgagt caggaggatt gcttgagatc tgcttgggca acatagccag accctgtctc 45660
tacaaaaaat aaaatgaaat aaattagctg gatgtggagg tccacacctg taactctagc 45720
tagcctggag gctaagaagg gaggattgcc tgagcccagt agttcaaggc tggagtgagc 45780
taggactgca tcactgcact ccagcctagg caacagcaag accacatctc tctctctctc 45840
tctctctctc tctcaaaagg cagtgaaata acgacttatt tggggaaaaa ataaaggcag 45900
agaatttgtt gccagcagac tagcataaaa aaaaggaagt ccttgaaaca gaagagaaat 45960
gataaaagat ggaaatttgg atatatacta aagaatgagg attgctaaaa gtgacataca 46020
tagataaata tgaaatatat ttttatttta aaatttattt aaagcaaaaa taaaaataca 46080
tcatatttat aacatagaaa taaaaaatgt atgataatag cataaaggat aagtggacaa 46140
atgctgttgt cgtatttttg gtaaaatgca ctattatttg aaagtagacc atcgtgaatt 46200
cgatgcatat tgtaaaccaa atagaacact aaaaaatgaa aataaagaga tatggctaat 46260
gtgccaatgg tggagataag atagatgcaa aaaaagaaaa acattcaaaa gaaggcagag 46320
acagaggaaa aaaggaccaa agatcaaatg agtcaaatag aaagcagcta aactagcaat 46380
atggcagatt taaatctagc catgtcaata gttatattaa atgtaaatgt tctaaatacc 46440
tgaattaaag gatgaagatt gtcagattag attgaaaaag catgacccaa ctacatgctg 46500
tctgtaagaa attagaaaaa gaacaaatta aatccaaagt aagaagaaag gaaatagagt 46560
agaagttagt gaagtataaa acaaagagca aagaaaatca attaaatgaa aagctggttc 46620
tttgtaaaga tcagtaaaat tgataaattt ctagctaaac tggccaagaa aaaagaaaag 46680
acatacaaat taacagtatc aggaagaaaa acagagaatt caaaggagtg taatgcaaac 46740
tttatgctag taaatgcaat aagttagatg gtatggaaaa aaatgtgaac aatacaaagc 46800
agactgtggt tgcctttggt ggcagtagcg gggtgggagt ggaaggttga attgactgga 46860
accagaagca caagtgaact ttttggggtg atggaaatgt tttgtatctt ggttgcattg 46920
atagttaaat ggttgtagac attgcttaaa actcactgaa cacttaagtg ggtatgtttt 46980
attatttgta aaatatacct caaaagcagt tttaaaaatg tattcaagta catacttaag 47040
atctttgcat tttactctga gtatacctta attttaaaat ctgtttttta aaaagtatta 47100
tgtagatacc ttttattttc ccaatgtctt tattaaatga catctccacg ttttgcttct 47160
tacctctatt tttttttttt tatttctctg tctctcaggc atgcacacac acacaccaaa 47220
aaaagtacat atgcataatc cttttggctg aataaaatca gttgcaactg ttatttcggc 47280
ccttatttgc tccgggtaaa tattcgttag ctgagtggtt tatctgtatc agatatttct 47340
tacatcttca tccagtcaca ccagctggac tgaccagatt gtttttcact tcaagggcag 47400
aatttgtact cactgctgaa tgcttccaaa tgatacgtag aataacaaat ttaagactta 47460
gatttttact ttttcaggtc tttttttttt tttctgtgct gtatagcatt tccctgaaag 47520

cttaatctca tctgtaagtg atgcagtgga tgtgttacta ttggattaat ttatttactc 47580
ttaggtaggt ttgtaatctg tcatcatgct gttgtttttt tgtgtgggtt tgtttttggt 47640
tttgagacag ggtctcactc tgctgcccag gctggagagg ctagagtgca gtgatgtgtt 47700
tatgggtcac tgcagattca atctcctggg ctcaagtgat cttcctgcct caaccccttg 47760
tgtagatgga agcacaggtg cacgccacca cacccggcta tttttttaaa tgtattgtag 47820
agacgaggca tcattttttt gcccaaggct gatcttgaac tcctgggctc aaacaatcct 47880
cccacctcgg ctcccaaagt gctgggatta cagatgtgaa ccaccactcg agctccatca 47940
ttctgttatt agttgttctc tagtatgagt caaaaactct tacctgccct tttacagttt 48000
tataaataag taagcagaat agcagaatgt ggacattttt taaatccaaa ttgaatatgc 48060
acatgactca aggagtcaaa tagtaccgta atcggtttat gataaaatcc agtggtttgg 48120
ctgggtgtcg tggctcacac ttgtaatccc agcaccttgg gaggctgagg caggtggatc 48180
acctgaagtc aggagtttga gaccagtctg acctacatgg tgaaactact aaaatacaaa 48240
attagctggg catggtggtg catgcctgta atcccagcta cttgggaggc tgaggcagga 48300
gaattgcttc aacccgggag gcagaggttg tggtgagccg atatcgcatt atttcagaac 48360
aattttccac aagatcagtg agtgctgtcc aatagacata taatacaacc cacatacatg 48420
actttacatt ttcttgtagc catagtagaa aaggtcaaaa gaagcagatg aaattaatag 48480
cctgggcaac aagagcaaaa ccccatcttt taaaaaataa aataaaatat ggtggtttgc 48540
tgtccccacc tcagaccatt tctctggtct ttctcattga ccaccactcc caatctttgt 48600
tctgctgatt gattacagct tgtatatatc tccatatttc taagcaaaat gtttatcttt 48660
tttaaattta taaattcttt ttattatttt tcagagacag ggtcttaact ctgtcgccca 48720
ggctggagta cagtggcacc atcgtagctc actgtagcct cgaactcctg ggctcaagca 48780
gtcttcctgc ctccgcctct caggtagctg agactacgct acaggcacat accaccatgc 48840
ccagctcaaa atgtttatct tttgatacat tattcgagac cattattaag gtggatgatt 48900
tagttttctt aaacagccat cccctttctt ttcctcccct ctgcttcacc gcccccattt 48960
tcccaatgtt ttaccttttg gttaaatcag tactcattgt ttacattatt tgcctctgca 49020
catagtcaca gatagtattg tactgtactg tactgtgttt cttttttaaa cattatttct 49080
gttgttaata attgactttt taattttttt cctattttgt tttttaaaga gatggggtct 49140
tactatattg cccaggctag agttcagtgg ctcttcgcgg gcatgatccc actgctgatc 49200
agtacaggaa tttccacctg ctccatttcc aacctggacc agttcacccc ttcttaggca 49260
acctggtggt cccccattcc cgggaggtca gcatattgat gccaaactta gtgcggacac 49320
ccgatcggca taacgcatgc agcccaggac tcctgggctc aagcagtcct cccgggctca 49380
agcagtcctc ccacctaagc ctcccgcgta gctgagacta cagacacttg ccaccacacc 49440
aggttaattt ttgtgttttt tgtagaggtg gggttttgcc atgttgtcca gactcatctc 49500
aaacttctca gctcaagtga gcctcctgcc tcagcttccc aagtagctgg gattatagac 49560
gcatgccacc acaccccatg ataattgcct ttttttttaa tttgcataat tttctttgta 49620
gcttttgcta atgttcccat atcttcttat agccttacag aatgattttc cacaagatca 49680
gtgagtgctg tccagtagac atataataca acccacatac atgattttac ctttttttgt 49740
agccatagta aaaaaggtca aaagaagcag atgaaattaa tagtatcttt tacttaaccc 49800
agttcattca aaatgttatt tcaataaatg gtcaatattt aaaatacttg agatattttg 49860
cttttattta tttcttttgt tactaagtct tcaaaatcca atgtgtattt tacacttaca 49920
gaacatctct ttttagactg gccacatgta gctcagggtt actgtattgg acagagtggt 49980
ttcagtttca agtttttcct tggagacatc ctacttgaaa tttccattct ccatgtatct 50040
gggtggttgg tctatagact tgccactcac agctgtcatc ttgagacttt ctttgctttt 50100
cttctctatt ggatattcag tttcctggat ttcaggtctt ctcattttcc tctagtagtt 50160
ttgttaggtc atggttggta tggcatggtt gggatagcgt gttcacacag ctatctcgtg 50220
agtcatactc ctccaatcca gcctgctcgc ttcccgtgtc tgtcatgtag ttgtcaccct 50280
gctatctctc cctccagttt ttgcagaaat ttcctttgtc ttcactcttg gtcttcctct 50340
cccatccccc atgtatccta tatctttctc tttcttggtt tatttcatca ctcaggtgga 50400
aaagatgctc cagtggatta ctgggaaaag ggggagcatg gatgataaag gtattgagac 50460
cttacacgtc agggaatttt tttttttttt tttttttgag acggagtttt gctcttgtcc 50520
aggttggagt gcagtggcgc caactcggct cactgcaacc tccacctcct gggttcaagt 50580
gattctcctg cctcagcctc ctgagtagct gggattacag gtgcccgcca ccacgcccag 50640
ctaatttttt gtatttttaa tagagacgag gtttcactgt gttggccagg ctggtcttga 50700
actcctactt caggcaatcc acccacctcg gaatgttttt attgtccctt ctcatttcat 50760
gactgctggg ctaggtatag aattccagaa tcattgttct tagaatctcg aaggcattgc 50820
ttcattgctg gccagctttc agtgttcttg caaagtctga agctgtgcta atcacctcat 50880
cctttgaaag tgaactgttt tttcttccca gaaacttaca gaacattctc tttgtccgca 50940
gaattctggg attgcaatta ctgtgcctta gaatgggtct gtttttatca ttatgaagag 51000
tactggatgg gtcgggaggt tttcttgaat tacttcttga tgttttcttt ccttgtattt 51060
ttttgtttgc taattttcta tttttttttc ttggtttact ttcttgggca gggggatttc 51120
ttctacttat atttgattct tcagttgagc ttgtcatttt tgctatcttg tttttaagtt 51180
tcgagagaca tctttgtttt atataacatt ctgttcttaa tacatagatg caagatcttt 51240
tctttctgag tatattaata tgtatttgaa atctttctat tctctgcagt ttgtttcccc 51300
caagggtttt tttttttttc tggtttttgt tttttgtttt tatgttagag actttcctgt 51360
tatatctggt catcagtggt acctgcatgt ggtggagagt aggggcttat tggagtatga 51420

gaaccttgag caggtgtaag gagcctgtca acactgcgct ggcctcaggg cctctaggga 51480
ggctgccagt tgtgcattct gaggatacct tttggttgtg ccttttgtct ggtcagatta 51540
tctagagatg ctctgcctcc tacctggagg agaagggtct agctgccagc ggtgtgagtg 51600
tctcttgggg aaaaggactc gagttcctgg tgtttggctt gtgtatggcc gcttacccca 51660
tttttggtgg agcgctcaca tcttccactg tgccaacagt cttgctgcag ttcatagacc 51720
ttctggttta catttttcca gaaagtatgt ctttagattt ctgcagaagt ctgaggagca 51780
tggaaggagc ttggggaatg agatggcaat ccaggtcttc ccagatggct ctacctttat 51840
cccctgcagg gaatcccact cctccttcct gactgggagc acagccagag ccttgggagg 51900
aatctggagt ggaaatctcg ggcggtctgg ctttcttact gttcacttgt aattttgctt 51960
tctcacaact gccaaccact aatcagcctg atttccagct tccagaattc tattgctgtt 52020
gtctgctctc ctattcccac cgtaggggat ggggctgtct tttttttttt ttttaatttt 52080
ggtaaaacat acaaaacata aagtgttcca ttttagccat ttttaggtac acagttcagt 52140
ggcagtaagt acattcacgt tgtgtgtatt tgttttttta gtaataaaca atataaaatt 52200
ttttaagtaa taaaacacaa ataaaagatt gtttaatgtg attatcgtgg aattttaggt 52260
gtgatcagga gccatggtgt agtcttctgt tgaaacaggg tgataggatt tgtttaccac 52320
ctcctaggaa agcagttgga tagtttgttg gcataaaagt acattttatc tatttttaat 52380
aatcgtagct ttatagaaat tgcagttgga actcccaggc ctggcattca aggctctctg 52440
agatctgggc tacccaccca tgtcctccag ccgtctgtcg cacctcctac tgcccactca 52500
ctgttcctgg catgagatgt gatctccagc ccccatgcct ttgctgtgca gggtgttcca 52560
gagtgaattg tccctcctgt ctgtctctct gccctcttcc tcgtctttcc atcttcctgc 52620
cccacatcac tgcctcctac ccaaggcctg tgctcattcc tcctcggttt tcccccatgg 52680
cctggtacat acctctgaat tatcaccttg catttcccat attgcccggc tctctttgat 52740
gtctgtttct ttgctgggtc ttcctcagtg tctgacggtc agttaaatgt ctttattctt 52800
ttttgtagga tatccgacat gaagccagtg acttcccatg tagagtggcc aagcttccta 52860
agaacaaaaa ccgaaatagg tacagagacg tcagtccctg taagtatcca cgtggccggt 52920
accagtcttg ctcttccttt gctgcaggcc tttttagtca agactccttt cgcctcaggg 52980
tttagtataa taataaatca atgtagcaga ggtttatgac gcgattgttt cctatagtaa 53040
aggcattaga gacttatagt aatagctcat ttttccacca ttatagaagg gctcaggttt 53100
cagtttctgg aaaattcagt gaagttcaaa gcacttttct taagctttga ctgtttttgt 53160
gatgaatcat tttcctacca gctgaagcag agtatagcag gcataataaa accttttctg 53220
gatgactcag cagcagcgtc attagggcat gagcactgtg ttccgctgta atgaagcccc 53280
gcacaggcat tcggggtggg cactgtcgtc ccctgcgctg aatatgcaag gcagctctgt 53340
ctggagtccc caccgcctcc acccccgcca acctcatcat ttttctccct ctttcctgct 53400
gttagttctt cctaggattg tcagtgtgcc tgctggcctg tggcagccct gtccgccttc 53460
tgagtgattg gctgtcagtc tgccggtagc tgaaaagtaa ataacttaac atgttagaat 53520
ttgcataaag taaggaaaac tggagctgag tacaggactt gaactgcgcc atctcctcta 53580
ggccacagag gcctttttga cccccttcca ggtctttaga cattgtcagg cagtgagggg 53640
tcgtagctgc cagtgtctcc atggtagcgt gctctgccag ggatgcagaa gattctccag 53700
tcattcctcc agtgggcact tcctgcaggt cctgtgccca tggctgggag tggtggctgt 53760
cattgttctc tgccagaagg gttagcagtg catcctgacc tgacttatgt ggcgcccaga 53820
ttcctggaag gggtctaaaa atggacctag acttggtgta gaacgtgtgc ctcttggcct 53880
gccaccatgg ttccctgcct ggttttgtgt gtcagctctg ccgcttaaga actgagtggc 53940
ttcgggcaag ttgttctctc tcataggagt gtgtgaagat gaagcaacat aagctgctta 54000
gcccagcgcc cagtacctca cgcagacata agtgctcagt aaatgttgtc tgtggtgggg 54060
atggttgtca ccaacatctg aagtgcactt ctaggtcatc aggtgacatg attggcgcca 54120
acacatggta ctcttgattt agcacatctc agctgaggca cctcattgat atttgtttaa 54180
aaacaaaaac aaaaaacctt ggtgattctg ctgtgaagtc ctggccagaa acctccagac 54240
cgctgatcaa cacgcaacag aaccatcacc gttcacctct ttgacatggt gccaggatac 54300
cctggatctc tagcttttgc tatagttgct ctaattaggg aataatcttg tctttaatat 54360
tcctttgcta cattttttaa catttcttat ctaaatggtt ttatgaatca gttttacaga 54420
gaaaaaaaac cagtatttaa aatattcttc caggggctgg tccaagtaca gtagtgttta 54480
caactatgtg atcacaacca gttacagatt tctttgttcc ttctccatcc ccactgcttt 54540
acttgactag ccaaaaaaaa aaaaaaaaaa agttattcca gggaaacaat tctccaactt 54600
tttcactccc aatctcactc ctcttatctt cctcccgtac tcctatcctc ctcccgtact 54660
cctatcctcc tcccctactc ctatcctcca gtagaaacag tcatttgctg tgaaggttat 54720
gggggagaat gagtcaaggt agaaggtcac ctgctgccca gctcacagtg ctgctggtga 54780
tgacagcagt ccacagttac aggcacttgc tgaacgaggg gctctgtata cacctcagct 54840
cattgactct tcccacaacc ctcttgtcac ctaccattta gcaaatgaaa aaaccaaggc 54900
tctgaggtga gttgtttgcc cagagtcacc cagtgctgtt tgaacccact cacataacca 54960
accaatacca ttatgtaatt tttgaggtct tttatctctg tgatccactt aaaaattatc 55020
caagtatctt tatttgtact aagcctccat aatgagaaac agtgttccag atggtggcta 55080
gttttcaaag acatctctct ttggaattct tctttagaac aaaaagcccc agaccactta 55140
tccccattca tatccccttt ggacctaggg agaaggtact atttataggt gatcacctga 55200
gtttattgtc ccttgtgctg tgccagaaat aaaggtcccc acctgctctt attagctcta 55260
ctaacaggat aaggaaagtg gccctcagag agctactgct tttgtgacaa acaaatgata 55320

caagaaaaaa aaagtggctt tttaatttta gtgacctggg gcaggacttc caaatgaaag 55380
tttatttcta aaaactaaaa ggtaaattta atatactttc agtgtttggg cttaaattct 55440
ctttcaagtg tctttgtgat atgctctgaa ttttaaaaat ttagaatcat tgaagttcat 55500
tatacttgaa ctttaaaaaa aaaaaacaaa aacctcgtat aaaggtcaag gtatgacttc 55560
atgctgctgt gtacttaggt catttaatct tcaaaccact ggatagaggt taggttgaag 55620
ttcgatctta aatcctacct actgtagctc attgtaccag caacagctgt agggactagg 55680
tggaattcat ggtgggtttt gttccctttt aaagattgaa gccaccatat tttctgccct 55740
ctaaaagttt atgtcagcca ggcatgggtg gctcacactt gtaatcccag cactttgggg 55800
aggctgaggt gggtggatca cttgaggcca ggagttcgag accagcctgg ccaacatggt 55860
gaaaccccat ctctactaaa aatagaaaaa ttaggtgagc atggtggcct gcgcctgtaa 55920
tcccagctac tcgggaggct gaggcaggag aaacatttga atccgggaga tggaggctgc 55980
agtgagctga gaacatgcca ctgcactcca gcctgggtga cagagtgaga ctcttgactc 56040
aaaaaaaaaa gttatgcatc agagaacaga tcctttgatg ccctcctctg ccctgaaagg 56100
tttttggggg agagtaataa gtatcacaac aagatatgac ctgagaacag atttcccaga 56160
taggacatga tccatgtttt aatatggctt actgctgttg cttcatagtg tgaagcttca 56220
gacacttctg aaaacccttt cagaaaatcc cagtcgcccc atactgatga ctaatctcaa 56280
ctaaaacagg gcttcagcca gtgtgaatgc cactaatgcc accaactcac ctttgctttt 56340
ctgtagggtg tgcacctgta tgtacacatt cagcttttcc gggattaacc tctgagttct 56400
ggtttgtctt tcagttgacc atagtcggat taaactacat caagaagata atgactatat 56460
caacgctagt ttgataaaaa tggaagaagc ccaaaggagt tacattctta cccaggtaag 56520
cagattgtct gaattttcta tttaatgtca atttaagagt ttgagagtgc tgttatccac 56580
acctcaaata aaatctgcca catcctttag aaggtcagga tttcagcata ccaaaaagca 56640
gcaaggaagg gggaaaaatc atccttcaaa ggttcagttt ggttataagg aacgctaatc 56700
ttttctggga agcataagat gacattgctg gaaatgagag cttatagaaa acaacattaa 56760
aatgccagag ttgcctgtgt ggtctgttgg cagagacagc agagccatgg ctggaggagg 56820
gtctgtacct gtgttgcttc cagaagtatt tgtcgtagag cacttgtgat ggcaaatcta 56880
agaacgttag cagtagacca ggaatctctg tccagagcca ttcagagtag ctcagcatgg 56940
ttctcattct ttggccagaa gaaaggcatc attggatcat gtgaacaagc atgaaaaatg 57000
acttaaaatt tctgttggct tttggcatct ttatggaaac aaaatcctga aagtggttta 57060
ataattgagc ctcttgtaaa acactcagtg gcatgtgacc aaaagggtat ctgggaaaga 57120
ggataaaaag agtttctttt taattaatct tctcaagtct taacttgtta cctgtaagtt 57180
ggtctaaaaa gactgggttt cttattttgt ttttcatcat aatttttgtt tctcattcca 57240
tgtcagcttt cagtcttata tggctttagg ccacagggcg attttgaaca tttgtaattt 57300
tgcttaataa ttaggaaatt aaaattctgg ggaagacaga atgctctatg aagaaaggct 57360
gctttgagca aggagctagg tcagggcgcg ttcaactgag gcctttcttc actgcctttt 57420
tgtcttgtcc cagttcctcc ccatttatga ctaaaatcag cccagatgct tctcgtcatc 57480
tgggatgcag agcatcagcc cagctgtgtt cagtcctatg gggccattga gtaagttctt 57540
ggtgcatgga tacagggcag gcctttacca ggccctgagc ccctggtcct cccagcacct 57600
ctggggtatt taggggaggc tgatggggga gggggttgat aaggcgggag atgtctgggg 57660
atgaggttga ggcaaaagtg acttcttgag gactttgctt tttggagaag tcaaatttcc 57720
tacttcttga tttcagccct tcaactctgg tatggagtca ggaagccctt taaatacctg 57780
ttgtcgggtg tatcatgtca agtgttgcat tagcaaatga ccatgtatcc ttgtgctact 57840
gtcctgccta ccccgcatcc tagcgcttcc ttgggacatg agaagctctg tctggtttgt 57900
gaggtggcac tggggatgtt gagaaactgt ttacacagtt tccctttgcc ctggggattt 57960
actaaaggag tcgaggcagc ctgaccccaa agcatcaccc ctggacacta tgaccgaaac 58020
atttccccag tgcccaaacc aagaacaccc ttcccatttt tttttcagtg gtgttcatta 58080
tgtaataata caagtctctc ttctcatttt ttaaaagtca gaagtacaga agagcagaga 58140
ataatgtcca aggggccctc cttcacctcc cccgtgcagt gtcagctaag tgtggtgcgt 58200
gtccttgcag atcttagggg attgtgatcc ttcagaccat tctaaactgg ggtggtgctg 58260
ggagttaggg aaggcatgaa gggagtagtg gagagctgca gtgactgggg tcttcatgcc 58320
agggtggaga atgcaaggcc caggtggcca gccatgtgcc acgggatttc tggctgccaa 58380
gagctgttta tctgttcact ggggagggaa gagttaaatg tggtctgctt ttctccgagt 58440
cccttcagca cagggagtgc tgacttgtct tgttcaggta gtaagttcaa gatgagctca 58500
ggaaagaaag tgagaggaca ctgagggcta gtggttgagc caagtgtgat gggacttaaa 58560
gggagaagat ttaaagaata aggagcttat gggccgggga cggtggctta cgcctgtaat 58620
cccagcactt tgggaggctg aagcaggtgg atcacttggg tcaggagttc gaggccagcc 58680
tggccaacat ggtgaaaccc cgtctctact aaaaatacag aaattagctg ggtgtggtag 58740
tgtgcacctg taatcccagc tacttgggag gctgagacag gagaatcgct tgagcccagg 58800
aggcagaggt tgcagtgagc caggattgcg cccctgtact ccagcctggg tgatggagcg 58860
agactctgcc tcaaaaaaaa ttaaaaaaaa ataaagaggt taggtgaaaa tagatgagaa 58920
tggaaaccat gagaagaagt gatgctggcc aaggacatga caggttctga tgtggaggtg 58980
ataggcaatg tctcttccag ccactgctaa taattgagac aaactcaagg cattcatacc 59040
ctgtgtccag taaacatctg tgcccattgc caggtgagct ggattgaaat gggccagctg 59100
ctcagcagac accctcatgc cccagtgact ctgttcccct tgggccacct cattgaccat 59160
ttatgtttct acatctccta agtttgttgg gccaaggatg gaggctgtct gccgtcaggg 59220

tcctcattgc tgatggtagg aatagttgct gatgtttcat tggatgttgc tgtattctag 59280
ggactgtgct aagtacttta tagaaatgaa catacttcat tttcacagtt ttatgaatag 59340
ggactattat tagtcaagta agcgatgggg aaactggggc agggagcgat gaagtgactt 59400
gcgcaaggtc acaagatgat gtgattggaa ccaagagaag tgttgtggtt ggccacgccc 59460
ccacactgcc tctcatctgc accaaggagt tttgtcccat agcccaaggg ccttggggac 59520
gaatctcagt ggaggccctt agcgggcctg cctgagccag aaagcagaat cggcattttt 59580
ctgtccttgg ttggcccagc cctgaactga gatgcggaaa tcgcctttcg ctgcctggta 59640
gaaaatggag ctgcagttac tgaccaccag gcagagagag gtgggtccct gtcccagcct 59700
cagccaccac tctgcctaag ctgtggggac tgagggcgct gtcgttagct gactgcagaa 59760
ggtgagcaca cgctgtagca tgttatgttt cagatgtcac atgttgtgtt attgtgtctt 59820
tgcagggccc tttgcctaac acatgcggtc acttttggga gatggtgtgg gagcagaaaa 59880
gcaggggtgt cgtcatgctc aacagagtga tggagaaagg ttcggtaagt ctcggcttca 59940
tttgctgtgt atgtgatcat gcataccact ccatatagtt accattttcg tccagatttt 60000
taaattattt ttcttgcctt tgtatttcct ttacgtagta tttttattta aaaaaattaa 60060
aacagcagca tataaatgca tgttggttgt caaccagtta atgaagtgaa taaaagggag 60120
gaggcggaag aactgcacgg acctcttcgc ccccgccttc tcctgtgtgg tgcgtgtggc 60180
gctccgccca cctgtgctgc ctgtgcggct ctcatcacag tgtggagttg tgtgtggagt 60240
tatggagacc tgcttttatc ttgaaaagca agttcttagt gcatcttcat ggtgtctgat 60300
tttttggctg gtgagagtgt ggctacctct gcggagctgt gggagcggct gactagatga 60360
gatttgcctc cattcagtac ctagactctt gccctgccac acctcttcgg agtgagcatt 60420
gacttcagga tgtgtgtcat tctaagttcc tgcaactttt caaacacccc tcgggctagc 60480
gtgtggctgc acggtgtcca tttgtgcagg ccaccactcc tcttgcatct gggtctagcc 60540
acctctcctt cttgacttac catagttcat tttgtaccat gctttcagaa tgagctttct 60600
caaatccaag tctcaccacg gttcttccca gctgaaaacc cttgtgcggt tccctttgcc 60660
tcacaggata atacatggtg tggcttacgg aaccctgcag gtctggccct aggcccctgg 60720
acacagacct ctcaccactc ttggaacttt agccaggaca aagttttctg tttttagttt 60780
cttaccatgt tctctgggcc gaggagtccc agtgcccacg ttcatcccac ttgcaggcac 60840
ccctggacgg ctgcccccag ctccccaact gcctgcattc tcccctgccc tcctcactct 60900
gttggaatag ctgagaatag ccgatttctg ggcagccggc ctcctgtgta gactgtcctg 60960
tgtagactgt cctgtgtaga ttgtctgtgt agactgtcct gtgtagattg tctgtgtaga 61020
ctgtcctgtg tagactgtcc atgtaaactg tcctgtgtag attgtctgtg tagactgtcc 61080
tgtgtagact gtcctgtgta gattgtctgt gtagactgtc tctgtagacc gtcgtgtata 61140
gactgtcctg tgtagactgt ctctgtagac cgtcgtgtat agactgtcct gtgtagactg 61200
tctctgtaga ccgtcctgtg tagattgtct gtgtagacca tcctgtgtag accatcccat 61260
ttagaccatc tgcctgtgca ggcgcaggcc agtgttcagc agggccacag gctcctcggc 61320
ctccctgccc tcgctgctcc ccaacactgc caaccctgct gcggggtcca ggaggagatg 61380
ggctgaggat cgtggagacc agcaggagcg tgtggcccag gagcagggaa ctgggtgtcc 61440
ttgggccttg ccaggtccag gctcagctag gacacggctc tcacagctgt cctggttgcc 61500
tccggccaca gaagaaggtg agggctccag agaggccacc tttccaaaaa aagcacagtc 61560
atggccctag aatgtaaaaa atccaagtgt taagaaggaa cacatcaaag gaaacttcag 61620
cagtgaaaac ttgaagcatt aaccacgaag cctctgcctc caccacacac aaagaaacgg 61680
ctttagttac tcgcagaaag tcttcctctt aggacagcgc gtgtttaaaa tcataggggt 61740
ttggtttgtt ttgttttggg gttggggttt tttgggggtt ttttaccctt gcctactttt 61800
taaaaaatga aagtgtttat ttgcccaaca ataacagaca gggagcttgc ctaagtgttc 61860
tgttgatgat ataatgtatc ttgtcttaga aaaaaacttt ttcagtgaaa ggtggttttt 61920
aaattttttc ttccctcctt agtagcttga ttagtaaaat gtgaagttac aaatgtgaag 61980
caaaccccca cccttcacca ctagtcagca attttgagta aagaaacaaa gcatcaggtg 62040
ctcacagcac acactgtctt agagggaagg ggaagcctgg tggcctgtgg aagccttcag 62100
catagctcca tctgcaggct tctgaccctc agcactactg acacttgggc tggatcattg 62160
tctgctaggg atccgggcag ggagtggctg tgctgggcgc tgtaggaagt ttagcagcat 62220
ctctggcctc tatccaccag atgccagtag caccccctcc ccagatgtgg cagtcagatg 62280
tgtttctgtc tagactccag actttgtcca acgtcccctg gtaggccaaa ttgcccccgg 62340
ttgagaacca ccgctctaga tggtattgag ggttgggaat tttaaatcaa gacatttatt 62400
cagaaattac cagatatagt agcatttgct tcttatttat ttctttgttg ctaagtgttt 62460
ggcaaaacct ctttgctgtg agcacaaggt ttgctttagc aattgttgtc acattacagc 62520
aaggagtggt gtccagcgct gtagttatgt atttgagcag tgtccagtgc tgtagttatg 62580
tgttccagcc tcaccaggcc ctgtgcttca ttgtctccca ctcaagactg accacaaatg 62640
gcccacagat ccactgtgac aacctttccc tttgggttac tgtggtggca tcgagaacat 62700
ggctggttgg ctttgctgta gtttactgtg ataactgtgc cagcagtccc tgctttcctt 62760
tgttaagtat cccattccac tggaggatta cttgggcgtg cagattggca tgaaaagcaa 62820
tgtatggttt gagattgtta aagtttcttt gggatcaaca ttttcaattc tgtatcagca 62880
ttatccctcc cagagggctg gctgggagaa atcatgagaa gttacagtat cttatttgct 62940
cagctaatct aattataaat gatccacaca gcttgtggta aaaccagctt ttggggagtt 63000
ttcatttaat gcatacttgt cttctgattt ccttccttca ccaaatagtg taggatgctc 63060
cctcttattt ttggcaaaca tgcctgttat cttttgggac cctgggcttc ctggaaacca 63120

gttatgcaga agatgattgt gtgtgttaga ctggggtcat ccagatggct agagttctca 63180
ctggttctgt ttaaggattg actttagaca cctcagtgta ggctgcacca tggcgtaagg 63240
gttgggattg ttgtttagaa gggggaagta agcaaggtga gtttaattgg ccattgcaga 63300
atctcacccg tatctccctc ctgaaatcct cactaaagct gccgtttgct ttcaggtgct 63360
ttcatgcaca agacactgca ttttgtatca cagggtccat ataattcatt tttctctcgt 63420
acttagttct ctgtgttaag aattacttac ttagttctct gtgttaataa tttttggcga 63480
aaccaaatta cccgtcacag ggttactgta gatgtctttc ataggttttc caaacaccac 63540
ttgcccactt gtttgggaag gccccaagga ctgtttaaca tctgccttca tggtggaaac 63600
agcaactatg agagatgcta gcatgttggc actgccatgt tcctctggta ccagcccaag 63660
ataggactca atttgaggcc tggtgaagta ctgtgttcta ataaaaatcc atctactttt 63720
catggccgta tatatcaatg taatagggta actggaaatg tgatcttgtg ccttttaaaa 63780
attttgtgtg tttaaaacaa aaatttctat tggaaatgac agagcatagc ttgttgctgt 63840
agacacctga gagtccttaa aaataaatat tgggttattg acacttagtt gcatgacaga 63900
attcctcact tgtacagttc caaagtctta gtctttaccc agattacaga gggttattaa 63960
gcattaggtt tggttttgaa agtgagtgct tgctgtctgg aggtgagctt taagactcgt 64020
ctgccctgct tatgagatga ggaagggtgg cctcttcctc ctgcatttct gttcttcgct 64080
tccttctctg tctgctcact ctgtggaatg cccaccccag cacgggtggg gtggaacctg 64140
tcagatcagt ctcttgtttc tggggtcttg aggcattata agatctagtt gttagaagtg 64200
tgggattaat tcatcttttc acattcttct aagttcctgc ttttagctgc cacacccact 64260
ttggctaagt gggggtcttg ccatgtaatt agcgcctcca tgccaagtgg cagaattgct 64320
tcaatggtga cagattgtcc ccattcaaga gttcactttt ggcaactcat cattgatcca 64380
ggaaggtgac atggatgaaa ctggctaaga cttcagacag gcttgtgtcc agactcttga 64440
gaaagctctg ttggcttctg gtctggcact gtgaagtttg ctgtgatgct ggcaccacaa 64500
cctggtgttt cctaatttgt ttctcccaca ttttgctttg gttttgtctt ttgggcagct 64560
tccagctcca gtagagcagg accaataggc atttgtggtt ctatattcac cctcctcacg 64620
tgcttcctgg ctcctcattg cccccagatg atgccacagg tccctgggcc tgctgccagt 64680
cgtctgtgat ctgggcctct gctggcccct tctccagctg ctcttttcag cctcttattt 64740
gcagtcactg cctaggaaat cctagtcatc cttcaaaacc tgcctcttgc acagagcttt 64800
ctctgatctc tcttttctgt aaccttggct gacctgaaac atttccctct tctgaattcc 64860
tgctgcatgt ccgtagcatt tccccctcag ccctccccca tagtccacct tgtcactgct 64920
gggcacagca gtgtcttctg acagacagct ggccctgaag tggttccctt cacccacacc 64980
atcctttgcc ccagaggagg tattgagtgg gtcagtgcac gtgaactgcc agtgtcattt 65040
gccaaagagc tgttgacaca cgctgacatt tcttttgctg aaaatcataa gggctttgag 65100
cttccctctg tccaggcaca tggtcaggct gacccggtag ctctgcccct gctgacctgc 65160
catttttgtc cacaacagtt atccatgagc agaaacattt gtgtaactga ggcagaaact 65220
tagttcaagt aaaatgtcac taaattcgag tcagtttttg tcttagaccc taaatgaaac 65280
caaattttca taaatttctt tgttttaaag aaaaatttaa tgagctacat ttaaactgag 65340
aacatcagat agtgtctgag attatcaaaa tagaacatca aaagtatttt tctgaatgaa 65400
ctgaaccaaa ccagaatgaa agggcaagcc ctggggagcc tgtctccaag ccttctctga 65460
aagggagtct gtatttggtg ataactgctc agcctctcca aagggcctca cctgctgtct 65520
ctcccagttt tatttttaat tgcctgtgag ttttctgtgc agggtaaggc acctacattc 65580
tatgccagca gcctgatcag gtcctgggta atgtttgaaa tggctacaca gaggagtttc 65640
aaagcctttt gttcaatctg gcttcacctc gtagacggtg agaaagcgtc agagccctgc 65700
aggatcccgt tgccacgttt gaccggggag ccgatgggtt tggaagtctg agccctgtct 65760
gcacaacctg ccccggtcag cagcttcgtg cccccacccc catctcccca tgaggcaggc 65820
atctgtgctg accatggctt ccatgttcag aaacccccag gcctttgagt tatcatgaag 65880
cttgtgggat gtgctccaag cctcctgcca tagaaaaact gccatattgc tcacaataat 65940
tcactattat ttgtttcccc agttaaaatg cgcacaatac tggccacaaa aagaagaaaa 66000
agagatgatc tttgaagaca caaatttgaa attaacattg atctctgaag atatcaagtc 66060
atattataca gtgcgacagc tagaattgga aaaccttaca gtgagtatag cacacacttc 66120
agcacttcag gcggctactg gttcacatgc ctcttccttt atcccttggg tgatattacc 66180
taatgtcagt gttcctggct tttgtatacc ccgagcaaga tgtggtttgg gcactgtggt 66240
gagcggagct tacttgtgta cctaccaagt gcccagggag ggtggaggcc acagtgctct 66300
ctctgacctt taacaacagt taacaccagt tcttagggaa aggagagttt cttacccaaa 66360
agactggttc ctgcttgtgc agctgcagag ggactggagc ggcagcctgc aagtcccagt 66420
gaagcatgct gccttctttg tggtcctcag tcttcgagtc tgaagagagg gaagaagggg 66480
tataggggct cactccagtt tcatagctag tgaaagtttt ctgggccagg tcttgggttt 66540
ttttgttgtg ggaagagttt ataacaccag ctacttgctt ggtaaaagtt ggtcttggaa 66600
catggcaagg cattgtggca agcagcactg ccgctgaacg cgctgctcct ggggctttgg 66660
aataattccc ctggatccgt aacttggggg tgttcatgtc attctgggga acagtggagg 66720
gagtgcgcgg cagcacctgg gggcaccagt gaagagtggc cagccaccaa cctctagaac 66780
ctaactgggg tcgaatcctg gccccacctt actagctcat cacagtgtct ccgtttcctc 66840
ttctgtcaaa ctcaggtttt gcgagggttc tgggaggtcc tatacgggaa gggttagcag 66900
ttaccatggg tgtgtagcac gggctttatc tgaaggaaag gtggagccgt agggagacca 66960
tgtggagtgg ggctccaggg ctgtgtgggt ggggagggat ctgcttctgg gttaccccat 67020

gcctcccctt ctcaagtact actttttaat catcatggct cctgccattc atttcatagt 67080
tgatgtaagc caggtgcggt ggctcacgtc tttaatccca gcacttgggg aggctgaggc 67140
caggaggatc actcgaggcc aggagttcaa gaccagcttg ggcaacatag tgagaccccc 67200
gtctctacaa aaaaacaaaa acagttagtc agacatcgtg gtgctcccct atagtccagc 67260
tactcaggag gctgaggcag gaggattgct tgtgcccggg agttcaaggc tgcagtgagc 67320
tatgcttgca ccactgcact ctagcctggg tgacagagca agaccctgtc tcaaaaataa 67380
ataaataaaa aaaatagtag aagtaagatc tagaatgtag cacaggttac caggacgtag 67440
gcaaggggtt cgggctgcct ggctcttgag gatggtagca gtgcagctga tgtgagtgct 67500
ttctgccctc tggtggtgac cgcgccggag tcaccagccc tgccatagcc ctgatggggc 67560
agagggttct gagtacggtg gatggaggtg ctttctggaa gattctcagg agtaacatgg 67620
gcagtgtgtt ggaatgtgct agaggattta tgcagtagcc ttttaaaaga atgcttttta 67680
gcatttgcaa gcctgacatt aagagtgact tctgggaaac tatttgcttg ttgagggaaa 67740
ctgaatttca acagagcaga agagctgtgc gctttttgct tggcagagtg aatacagcca 67800
gctcagaggt tttgatgtta ggatctgttt gctccaacag actttgtttt taaaaggctt 67860
ttctcagcca tagctgtctg ttctagcaca aggctggaat gagttccttg tgaaagaggt 67920
gagcaggtgt gagggagggt gtcagtgggc ggtaacccac accttcaagg attaaaggaa 67980
aacttgcatt tggcatgctt gcttcttatt caattttaaa atacatttta acggccgggc 68040
acggtggcta acacctgtaa tcccagcact ttgaggggct gaggtgggtg gttcacgagg 68100
ccaggggttc aagaccagcc tggccaagat ggtgaaaccc catctctact aaaaatacaa 68160
aaaaaaaaaa aattagccgg gcgtggtggc gggcacctgt aatcccagct actcgggagg 68220
ctgaggcaga gaattgcttg aacccaggag gcggaggttg cattgagccg agatcatgcc 68280
actgcattcc agcctgggcg gcagagcaag actctgtctc aaaataataa taataatttt 68340
ttaaaaatac attttaagtc cttttcttcc ccacctgcct ccacccacca aatagaagag 68400
gtatttcttc ttctttaatg tcattaaggt tatatggata ccattttcta gagaggaaag 68460
aatgatggaa ttgcctagtg tgagtctagc aattatccta acatacacaa atttctcctt 68520
gttctgtgcc aagatactgt atttaatatt taatgaacat taaatattat ttactagtgt 68580
atttaatggc tgaggcaggg ttaaatatgt attattttca tcccagcaga gttgggggag 68640
gtcctagtaa ctatgccatg agctctgtga gggtgaggtg gtgtctttgc ccccgcctcc 68700
ctggcacagt gactggcaca tgattggcat agtgtggaca ttcgtcaagt gaaggaaggc 68760
atcatgagca gatctctggc ctgaatcctt ctgccatcag ctgctcgcca ggtggccctg 68820
gcactgggcc acagggaaac tctccaggct ggtatggttc ctgtctgtgg ctgtcttccc 68880
gggcccatgt taggagactt tcacttccag agccctttcc ctctcagggc cttgcttacc 68940
aagtgactgg ttcccattta ctaggagctc ttaggtcatt gaagatgttg cgtactcccc 69000
ccagtgaggg ctgccttttg atcacagccg ccagaagcct caaggaagga gcagagctgg 69060
aaacagacgc caggccattg cttctgttcc tctggggcag acccagccac ggaagagaca 69120
ttctgggaca agggctgggg tccacctttc aaacgtgtct gcagcaggct ctcagcatgg 69180
actctctgcc tccaaacatc cacctcctca tcggaaaatg gatgggagtg cctgcctgga 69240
gcagctggtg ggagagcgca gcgccagcac gtaggacaca ctcggttcat gggctgatgc 69300
cgttcgcatt gactgcctct tcagctgggt gttgagccac accttggagt caccagtctt 69360
tggagaccaa gtctgctact tttttctcta aagtgacaat cctctgaaac ctccagatca 69420
tcttgaagcc cccgtctgaa agttgcccag agccagtgcc tcacctgctg ttccttgttc 69480
actttttcac gggaggcctt gcagggcttt atgacaagat tttatgggtg gctgcccagc 69540
atcattgtga ctcgtgagac agagagaaac cagttgtaac catgtagaca gtggaagtga 69600
tagggagaaa agaggtgagg ggactcttca atccgaaggg aaatgaagtc taagcaggcg 69660
caccctgcag gttcagtgtc aagcccaggg cctggcccca gggtgtggta tttgttgact 69720
gggtgtgtgg accctgggag aaagtctgag aatgaatgtt cctcttagag gtagagagtg 69780
gaaggtgact ctgtgtgtac ttggaattag tgatttctgt acagatgatt cttttagaat 69840
catcatgagt atttttctct ttcagaccca agaaactcga gagatcttac atttccacta 69900
taccacatgg cctgactttg gagtccctga atcaccagcc tcattcttga actttctttt 69960
caaagtccga gagtcagggt cactcagccc ggagcacggg cccgttgtgg tgcactgcag 70020
tgcaggcatc ggcaggtctg gaaccttctg tctggctgat acctgcctct tgctggtaag 70080
gaggccctcg cgggtgccct ggggagctcc tctacctgct ctgctgtgat gttttttcct 70140
aagtagaaac tgaagcgctc ctcttccaaa atacagagac tcactgtgtt agtctgtttt 70200
tgcgttacta ataaaggcgt acctgagact cggtaatttg taaagaaaag aggtttaact 70260
ggctcccggt tctgcaggct gtacaagcat ggcaccagca tctgctcggc tcctggggag 70320
gcctcaggga gcttccagtc atggtggaag gtgaagggga gcaggagcaa gagatggggg 70380
aggtcccaga ctcttaacca gctctcttgt gaatgcattg cctcagggag ggcaccaagc 70440
ctttcatgag ggacctgtcc ccctgaccca gacacctccc acccagcccc acctccaaca 70500
ctagggatca catttcagca tgagattggg aggggacaga catctaacgg tgttattaac 70560
gttgcccttg agaattggac ctggctgact tatatctcct ctctggcttt cagatggaca 70620
agaggaaaga cccttcttcc gttgatatca agaaagtgct gttagaaatg aggaagtttc 70680
ggatggggct gatccagaca gccgaccagc tgcgcttctc ctacctggct gtgatcgaag 70740
gtgccaaatt catcatgggg gactcttccg tgcaggtcag cattgccttt gtttgaatcc 70800
aggtgtgacc attttaactt ttttgtcttt gaaggaggct gtcagttgta aaagttcaaa 70860
caccgtctgg tgtcagggga aatagctacc cttcatgttt aaaatagcta gaaagttgtc 70920

aaaatgttca ccatgttgca ctttgtgcct ttgaagtgct cacatagaga gcattgatag 70980
gaagacgaga ctttattttc aaaagatttc atcttccaag tacatggctg cagccctgag 71040
aggccgagag cccctcgcca agccgtcacc tctgctcatg caaagggatt tcctgacaaa 71100
ccagccgaag tgaacactaa taggacttcc tcttgctgct ctttcaagga tcagtggaag 71160
gagctttccc acgaggacct ggagccccca cccgagcata tccccccacc tccccggcca 71220
cccaaacgaa tcctggagcc acacaatggg aaatgcaggg agttcttccc aaatcaccag 71280
tgggtgaagg aagagaccca ggaggataaa gactgcccca tcaaggaaga aaaaggaagc 71340
cccttaaatg ccgcacccta cggcatcgaa aggtaatatg attgggtccc agcttgttgg 71400
ggtgagggga aatgactttc tgttctagaa acacacgctg gtactgaaac cctgtggatg 71460
cagcctcctg ttggcaagca gcgcttccgc atccttgggg aacagggcgc gtggaccaca 71520
gccactccac tcctggctgc tggaggtccg gtattgggca cagggtggcc gcaggacatg 71580
agccacttct gtgggcttct agtgccacct tgtggtgctt gttggaatga ggggctcgga 71640
gccaccgagt agggtttttc tgccccccct gacgacagcg ccctccccca ggtttccgga 71700
cagtcctgaa atgtgatgtc caggcttgag tgccctcagt ccccacagtg gtcctttggg 71760
gaatgtaacc ttttttatgt ggtcttgatt aaatcccatt ttacttcctt gcaggttaac 71820
aaccattatt gagtacctat tgatatgtgt ggtgtactga gttaactaga acatgtcccc 71880
tggtctgtgt tctagaccat cttgctggga aaaaggcaga cccaaagcat attttggtgg 71940
gggcccatgg acagtgatgt gatagaggtg tccgctgagg tggtcaggga aggctgcttg 72000
cagtaggtgg ccgtgcacgg aaagtttgca gaatgagcag gtgttagttc cagctggaga 72060
tgactgccgg ctgtgccctt ggtacctgct ttctggaggg aagttttaag acgtgtgcat 72120
acttgaccca gcagttgtat acatggagaa atttactttg cagcaactct caaaacaagc 72180
gtgtaaagat gtgtataggt agttgtgttt gttgtggcat tgtttgtagt agtgaaaaat 72240
tagagacagg ccaatgatat aaccagggac ctgatcaatt atgttctctc ccggtgttgg 72300
gatattctgt agctcttaaa gaatgagatc tgggtgtact gatgtggcca gacattgcaa 72360
ttgcagtaca tgagaaggca aatcatacag tagtgtgtac accagtgagt cctccagcca 72420
gataaatcct cacagtgacc agtcgcccag gcaccttgtg aaccctaccc tgggtgtggg 72480
tgctatctga agtacctggg ggagggggtg acaagtggac ttcaggctga tgtgggccct 72540
ggcctggccc tccctccaag cagagggggc tggctcgctg gaaggttaac atcatccaac 72600
tctgtctaca cgtggcttgt tttttcctag aattcctgcc acaatagcag catccttgcc 72660
attcattttc tccaaagtga gtaacccatc tctgccctct gattcctcag catgagtcaa 72720
gacactgaag ttagaagtcg ggtcgtgggg ggaagtcttc gaggtgccca ggctgcctcc 72780
ccagccaaag gggagccgtc actgcccgag aaggacgagg accatgcact gagttactgg 72840
aagcccttcc tggtcaacat gtgcgtggct acggtcctca cggccggcgc ttacctctgc 72900
tacagggtat gtttccactg acagacgcgc tggcgagatg ctcgtgtgca gagagcactg 72960
gccgctagcc cgatggtagg attcagttct gtggtgcatc tgagccagtc tcagaagaaa 73020
cagatcaaag gtttttaaag tctggaactg tggaagggct aacaagagaa ttaaggatcg 73080
atgcactggg gttttaagga gccctctggt cccaagaata taagagtcta atctcagggc 73140
cttaacctat tcaggagtaa gtagagaaaa tgccaaatac gtctgtttct ctctctcttt 73200
ttttttttat tcctttgttt ttggaaaaaa atagagttac aacacattgt tgtttttaac 73260
ctttataaaa agcagctttt tgttatttct ggaacaaaaa aaaacaaagt aggcacttat 73320
gaaactttct cataccctta ggtgatgtaa tcagccatat aatttatatt tgatttccca 73380
gggaaggaat cccaaacttt tacgaatgta aactcccttg gagaagaggg ttaggacgct 73440
gttgcgctca agcccccctc agctgtgtgc acactgagcc aggacagggt ctttgagctt 73500
tcccactata agaagaacag caacaaaagg ccgtctagaa aaacagaacc tgcctctgct 73560
tctgctcagg gtgtccccgc tgggtttcca ttgtcctttc tccattgctc cctcctgtga 73620
cagccatctt gctcatgtac cagccctcat caccccatcc ccataaatgg gtgtcctcga 73680
ggcctctgcc tgggggtcag aggtcaccac agggtggcca ttggcatgtc aacccgctgt 73740
taattcagag aagtgggctc cacctcattg ggagaagtgc catttcagca gaaattcaca 73800
cgttagacgt gtgttgctgt taagtaaggg gaagagagag gactagcctc agagctctgg 73860
ccatggaaat gacctcctaa gactttttcg tggttttaaa tattttacct ctttccaggt 73920
ggcatctgag tacatcagat ggttttgcaa aatgcaaaca attttttcct tggggatgat 73980
ttttggggag agggggctac tgtaaaaaat aaaaccaaaa ccccctttgc tccctcggag 74040
gttgaagttg ccggggggtg tggccggggt catgcatgag gcgacagctc tgcaggtgcg 74100
ggtctgggct catctgaact gtttggtttc attccagttc ctgttcaaca gcaacacata 74160
gcctgaccct cctccactcc acctccaccc actgtccgcc tctgcccgca gagcccacgc 74220
ccgactagca ggcatgccgc ggtaggtaag ggccgccgga ccgcgtagag agccgggccc 74280
cggacggacg ttggttctgc actaaaaccc atcttccccg gatgtgtgtc tcacccctca 74340
tccttttact ttttgcccct tccactttga gtaccaaatc cacaagccat tttttgagga 74400
gagtgaaaga gagtaccatg ctggcggcgc agagggaagg ggcctacacc cgtcttgggg 74460
ctcgccccac ccagggctcc ctcctggagc atcccaggcg ggcggcacgc caacagcccc 74520
ccccttgaat ctgcagggag caactctcca ctccatattt atttaaacaa ttttttcccc 74580
aaaggcatcc atagtgcact agcattttct tgaaccaata atgtattaaa attttttgat 74640
gtcagccttg catcaagggc tttatcaaaa agtacaataa taaatcctca ggtagtactg 74700
ggaatggaag gctttgccat gggcctgctg cgtcagacca gtactgggaa ggaggacggt 74760
tgtaagcagt tgttatttag tgatattgtg ggtaacgtga gaagatagaa caatgctata 74820

atatataatg aacacgtggg tatttaataa gaaacatgat gtgagattac tttgtcccgc 74880
ttattctcct ccctgttatc tgctagatct agttctcaat cactgctccc ccgtgtgtat 74940
tagaatgcat gtaaggtctt cttgtgtcct gatgaaaaat atgtgcttga aatgagaaac 75000
tttgatctct gcttactaat gtgccccatg tccaagtcca acctgcctgt gcatgacctg 75060
atcattacat ggctgtggtt cctaagcctg ttgctgaagt cattgtcgct cagcaatagg 75120
gtgcagtttt ccaggaatag gcatttgcct aattcctggc atgacactct agtgacttcc 75180
tggtgaggcc cagcctgtcc tggtacagca gggtcttgct gtaactcaga cattccaagg 75240
gtatgggaag ccatattcac acctcacgct ctggacatga tttagggaag cagggacacc 75300
ccccgccccc cacctttggg atcagcctcc gccattccaa gtcaacactc ttcttgagca 75360
gaccgtgatt tggaagagag gcacctgctg gaaaccacac ttcttgaaac agcctgggtg 75420
acggtccttt aggcagcctg ccgccgtctc tgtcccggtt caccttgccg agagaggcgc 75480
gtctgcccca ccctcaaacc ctgtggggcc tgatggtgct cacgactctt cctgcaaagg 75540
gaactgaaga cctccacatt aagtggcttt ttaacatgaa aaacacggca gctgtagctc 75600
ccgagctact ctcttgccag cattttcaca ttttgccttt ctcgtggtag aagccagtac 75660
agagaaattc tgtggtggga acattcgagg tgtcaccctg cagagctatg gtgaggtgtg 75720
gataaggctt aggtgccagg ctgtaagcat tctgagctgg gcttgttgtt tttaagtcct 75780
gtatatgtat gtagtagttt gggtgtgtat atatagtagc atttcaaaat ggacgtactg 75840
gtttaacctc ctatccttgg agagcagctg gctctccacc ttgttacaca ttatgttaga 75900
gaggtagcga gctgctctgc tatatgcctt aagccaatat ttactcatca ggtcattatt 75960
ttttacaatg gccatggaat aaaccatttt tacaaaaata aaaacaaaaa aagcaaggtg 76020
ttttggtata ataccttttc aggtgtgtgt ggatacgtgg ctgcatgacc gggtgggtgg 76080
gggggagtgt ctcagggtct tctgtgacct cacagaactg tcagactgta cagttttcca 76140
acttgccata ttcatgatgg gtttgcattt tagctgcaac aataaaattt ttttctaaag 76200
aacatgaatt tggggtgctt cccatttttt tctttgctta atagagctaa accaggatga 76260
gtaactcctg tttctttcta tccctgctga tgtgaaacag atgttgtcaa tcagctgggg 76320
ttagagtttt ccacttctaa gaattaacct cagcatccct gcattgccag caccctcagg 76380
ctggagcgct ttccttgact gtgagcttgt tgaacacctt aggcctcagc ccatttcctt 76440
cccaaattga cgctttgcct gtgtagggcc ctcagataac ttaacaaact taccagtgtt 76500
gtttgaagaa cagtgttttg agttgtaatc tcaaaaccat atcccttacc caattacctg 76560
taagacacaa tggttaccac atctcagtac gtaaagtcca cttgatatag aattgactta 76620
gaaataagac agattagtat agtttttcat ttgtgtacaa aattaaacaa tgtaaattcc 76680
ccccaaagtg atttttttga ctttttgaag taattttgga cttgcaaaat gttgccaaaa 76740
tagtacgaag agttccccag taccctcgaa gtttcctcga ctgtttcaaa gctggctgca 76800
ggcccaggct catgagactg ggaagaggac aggctgtggt catgtggacc cacaggggcc 76860
tggggctgca gaagtcagtg tggcttccac catttcaggt ataaaaaagg gcatctaagc 76920
tttcaagaag agggaggatg ctctagggca gcggtcccca accttttctg gcaccaggaa 76980
ccggttccat ggaagacaat tttttcacag gcctgggggt ggtgagggat ggttttggga 77040
tagaaacttc cacctcagat catcaggcat cagattctca taaggagctt gcaacctgat 77100
ctcttgcaca cattcagttc acaatagggt tcacgctcct aagagaacct gatgctgcag 77160
ctgatctaac aggagatgga gctcaggtgg tcatgctcag tcgctcgcca ctcacctcct 77220
gccatgcagt ccagttccta acaggcctca gaccagtacc ggtctgtggc ctgggggttg 77280
aggacccctg ctctaggctg gtactgctga tgcttaaaaa gagagggttt gccagaaatc 77340
agatgggaca aaagggcaaa ggccgtgcca cagagtgccc atatagggga gagcacgcct 77400
ggagccttcg agagcatgca gagaagcctg gagactgcat ttaccggagc tgctgcctga 77460
ggccaccctc caagtgtccc cacagcgcac cacaagacca caggagtgac ctcctcactg 77520
gcaggtattt ggggaaacaa ctgctgtcta ctcttttggg taaaaagtga aacaccaata 77580
gtttaattga aatttcagaa aattgaacat atgaacaagg caaataaata ctaagtaagt 77640
taaaaacaca aaatatgtcc aggaagtatc gatgagaatg ttcaagttaa agttctccaa 77700
tgccattgct acagcaacct caaaccctag gttctctctg cactattaac acagacatct 77760
caggacatgg tttgcttttt tttaagactt aaataggaaa ctaatttttc tttctttaaa 77820
gcaattgcgt tcttcagtga actctttctt taggccagtt gatggcttct tagcagttta 77880
ttgacgagat cctagggtag cttccgaagc tgggttgatt gattgcattt gggtgcggat 77940
ggccaaagtg agtggcccta ctgcctgtgc tgctcagggc tcctgggctg atgtggtggc 78000
t 78001<210>
13
<211> 2160
<212> DNA
<213> Mus musculus

<400> 13
ggcgccctgc tctcccggcg gggcggcgga gggggcgggc tggccggcgc acggtgatgt 60
ggcgggactc tttgtgcact gcggcaggat acgcgcttgg gcgtcgggac gcggctgcgc 120
tcagctctct cctctcggaa gctgcagcca tgatggaagt ttgagagttg agccgctgtg 180
aggccaggcc cggcgcaggc gagggagatg agagacggcg gcggccacgg cccagagccc 240
ctctcagcgc ctgtgagcag ccgcgggggc agcgccctcg gggagccggc cgggcggcgg 300

cggcggcagc ggcggcgggc ctcgcctcct cgtcgtctgt tctaaccggg cagcttctga 360
gcagcttcgg agagagacgg tggaagaagc cgtgggctcg agcgggagcc ggcgcaggct 420
cggcggctgc acctcccgct cctggagcgg gggggagaag cggcggcggc ggccgcggct 480
ccggggaggg ggtcggagtc gcctgtcacc attgccaggg ctgggaacgc cggagagttg 540
ctctctcccc ttctcctgcc tccaacacgg cggcggcggc ggcggcacgt ccagggaccc 600
gggccggtgt taagcctccc gtccgccgcc gccgcacccc ccctggcccg ggctccggag 660
gccgccggag gaggcagccg ctgcgaggat tatccgtctt ctccccattc cgctgcctcg 720
gctgccaggc ctctggctgc tgaggagaag caggcccagt ctctgcaacc atccagcagc 780
cgccgcagca gccattaccc ggctgcggtc cagggccaag cggcagcaga gcgaggggca 840
tcagcgaccg ccaagtccag agccatttcc atcctgcaga agaagcctcg ccaccagcag 900
cttctgccat ctctctcctc ctttttcttc agccacaggc tcccagacat gacagccatc 960
atcaaagaga tcgttagcag aaacaaaagg agatatcaag aggatggatt cgacttagac 1020
ttgacctata tttatccaaa tattattgct atgggatttc ctgcagaaag acttgaaggt 1080
gtatacagga acaatattga tgatgtagta aggtttttgg attcaaagca taaaaaccat 1140
tacaagatat acaatctatg tgctgagaga cattatgaca ccgccaaatt taactgcaga 1200
gttgcacagt atccttttga agaccataac ccaccacagc tagaacttat caaacccttc 1260
tgtgaagatc ttgaccaatg gctaagtgaa gatgacaatc atgttgcagc aattcactgt 1320
aaagctggaa agggacggac tggtgtaatg atttgtgcat atttattgca tcggggcaaa 1380
tttttaaagg cacaagaggc cctagatttt tatggggaag taaggaccag agacaaaaag 1440
ggagtcacaa ttcccagtca gaggcgctat gtatattatt atagctacct gctaaaaaat 1500
cacctggatt acagacccgt ggcactgctg tttcacaaga tgatgtttga aactattcca 1560
atgttcagtg gcggaacttg caatcctcag tttgtggtct gccagctaaa ggtgaagata 1620
tattcctcca attcaggacc cacgcggcgg gaggacaagt tcatgtactt tgagttccct 1680
cagccattgc ctgtgtgtgg tgatatcaaa gtagagttct tccacaaaca gaacaagatg 1740
ctcaaaaagg acaaaatgtt tcacttttgg gtaaatacgt tcttcatacc aggaccagag 1800
gaaacctcag aaaaagtgga aaatggaagt ctttgtgatc aggaaatcga tagcatttgc 1860
agtatagagc gtgcagataa tgacaaggag tatcttgtac tcaccctaac aaaaaacgat 1920
cttgacaaag caaacaaaga caaggccaac cgatacttct ctccaaattt taaggtgaaa 1980
ctatacttta caaaaacagt agaggagcca tcaaatccag aggctagcag ttcaacttct 2040
gtgactccag atgttagtga caatgaacct gatcattata gatattctga caccactgac 2100
tctgatccag agaatgaacc ttttgatgaa gatcagcatt cacaaattac aaaagtctga 2160

<210> 14
<211> 5572
<212> DNA
<213> H. sapiens

<400> 14
cctcccctcg cccggcgcgg tcccgtccgc ctctcgctcg cctcccgcct cccctcggtc 60
ttccgaggcg cccgggctcc cggcgcggcg gcggaggggg cgggcaggcc ggcgggcggt 120
gatgtggcgg gactctttat gcgctgcggc aggatacgcg ctcggcgctg ggacgcgact 180
gcgctcagtt ctctcctctc ggaagctgca gccatgatgg aagtttgaga gttgagccgc 240
tgtgaggcga ggccgggctc aggcgaggga gatgagagac ggcggcggcc gcggcccgga 300
gcccctctca gcgcctgtga gcagccgcgg gggcagcgcc ctcggggagc cggccggcct 360
gcggcggcgg cagcggcggc gtttctcgcc tcctcttcgt cttttctaac cgtgcagcct 420
cttcctcggc ttctcctgaa agggaaggtg gaagccgtgg gctcgggcgg gagccggctg 480
aggcgcggcg gcggcggcgg cacctcccgc tcctggagcg ggggggagaa gcggcggcgg 540
cggcggccgc ggcggctgca gctccaggga gggggtctga gtcgcctgtc accatttcca 600
gggctgggaa cgccggagag ttggtctctc cccttctact gcctccaaca cggcggcggc 660
ggcggcggca catccaggga cccgggccgg ttttaaacct cccgtccgcc gccgccgcac 720
cccccgtggc ccgggctccg gaggccgccg gcggaggcag ccgttcggag gattattcgt 780
cttctcccca ttccgctgcc gccgctgcca ggcctctggc tgctgaggag aagcaggccc 840
agtcgctgca accatccagc agccgccgca gcagccatta cccggctgcg gtccagagcc 900
aagcggcggc agagcgaggg gcatcagcta ccgccaagtc cagagccatt tccatcctgc 960
agaagaagcc ccgccaccag cagcttctgc catctctctc ctcctttttc ttcagccaca 1020
ggctcccaga catgacagcc atcatcaaag agatcgttag cagaaacaaa aggagatatc 1080
aagaggatgg attcgactta gacttgacct atatttatcc aaacattatt gctatgggat 1140
ttcctgcaga aagacttgaa ggcgtataca ggaacaatat tgatgatgta gtaaggtttt 1200
tggattcaaa gcataaaaac cattacaaga tatacaatct ttgtgctgaa agacattatg 1260
acaccgccaa atttaattgc agagttgcac aatatccttt tgaagaccat aacccaccac 1320
agctagaact tatcaaaccc ttttgtgaag atcttgacca atggctaagt gaagatgaca 1380
atcatgttgc agcaattcac tgtaaagctg gaaagggacg aactggtgta atgatatgtg 1440
catatttatt acatcggggc aaatttttaa aggcacaaga ggccctagat ttctatgggg 1500
aagtaaggac cagagacaaa aagggagtaa ctattcccag tcagaggcgc tatgtgtatt 1560

attatagcta cctgttaaag aatcatctgg attatagacc agtggcactg ttgtttcaca 1620
agatgatgtt tgaaactatt ccaatgttca gtggcggaac ttgcaatcct cagtttgtgg 1680
tctgccagct aaaggtgaag atatattcct ccaattcagg acccacacga cgggaagaca 1740
agttcatgta ctttgagttc cctcagccgt tacctgtgtg tggtgatatc aaagtagagt 1800
tcttccacaa acagaacaag atgctaaaaa aggacaaaat gtttcacttt tgggtaaata 1860
cattcttcat accaggacca gaggaaacct cagaaaaagt agaaaatgga agtctatgtg 1920
atcaagaaat cgatagcatt tgcagtatag agcgtgcaga taatgacaag gaatatctag 1980
tacttacttt aacaaaaaat gatcttgaca aagcaaataa agacaaagcc aaccgatact 2040
tttctccaaa ttttaaggtg aagctgtact tcacaaaaac agtagaggag ccgtcaaatc 2100
cagaggctag cagttcaact tctgtaacac cagatgttag tgacaatgaa cctgatcatt 2160
atagatattc tgacaccact gactctgatc cagagaatga accttttgat gaagatcagc 2220
atacacaaat tacaaaagtc tgaatttttt tttatcaaga gggataaaac accatgaaaa 2280
taaacttgaa taaactgaaa atggaccttt ttttttttaa tggcaatagg acattgtgtc 2340
agattaccag ttataggaac aattctcttt tcctgaccaa tcttgtttta ccctatacat 2400
ccacagggtt ttgacacttg ttgtccagtt gaaaaaaggt tgtgtagctg tgtcatgtat 2460
ataccttttt gtgtcaaaag gacatttaaa attcaattag gattaataaa gatggcactt 2520
tcccgtttta ttccagtttt ataaaaagtg gagacagact gatgtgtata cgtaggaatt 2580
ttttcctttt gtgttctgtc accaactgaa gtggctaaag agctttgtga tatactggtt 2640
cacatcctac ccctttgcac ttgtggcaac agataagttt gcagttggct aagagaggtt 2700
tccgaagggt tttgctacat tctaatgcat gtattcgggt taggggaatg gagggaatgc 2760
tcagaaagga aataatttta tgctggactc tggaccatat accatctcca gctatttaca 2820
cacacctttc tttagcatgc tacagttatt aatctggaca ttcgaggaat tggccgctgt 2880
cactgcttgt tgtttgcgca ttttttttta aagcatattg gtgctagaaa aggcagctaa 2940
aggaagtgaa tctgtattgg ggtacaggaa tgaaccttct gcaacatctt aagatccaca 3000
aatgaaggga tataaaaata atgtcatagg taagaaacac agcaacaatg acttaaccat 3060
ataaatgtgg aggctatcaa caaagaatgg gcttgaaaca ttataaaaat tgacaatgat 3120
ttattaaata tgttttctca attgtaacga cttctccatc tcctgtgtaa tcaaggccag 3180
tgctaaaatt cagatgctgt tagtacctac atcagtcaac aacttacact tattttacta 3240
gttttcaatc ataatacctg ctgtggatgc ttcatgtgct gcctgcaagc ttcttttttc 3300
tcattaaata taaaatattt tgtaatgctg cacagaaatt ttcaatttga gattctacag 3360
taagcgtttt ttttctttga agatttatga tgcacttatt caatagctgt cagccgttcc 3420
acccttttga ccttacacat tctattacaa tgaattttgc agttttgcac attttttaaa 3480
tgtcattaac tgttagggaa ttttacttga atactgaata catataatgt ttatattaaa 3540
aaggacattt gtgttaaaaa ggaaattaga gttgcagtaa actttcaatg ctgcacacaa 3600
aaaaaagaca tttgattttt cagtagaaat tgtcctacat gtgctttatt gatttgctat 3660
tgaaagaata gggttttttt tttttttttt tttttttttt ttaaatgtgc agtgttgaat 3720
catttcttca tagtgctccc ccgagttggg actagggctt caatttcact tcttaaaaaa 3780
aatcatcata tatttgatat gcccagactg catacgattt taagcggagt acaactacta 3840
ttgtaaagct aatgtgaaga tattattaaa aaggtttttt tttccagaaa tttggtgtct 3900
tcaaattata ccttcacctt gacatttgaa tatccagcca ttttgtttct taatggtata 3960
aaattccatt ttcaataact tattggtgct gaaattgttc actagctgtg gtctgaccta 4020
gttaatttac aaatacagat tgaataggac ctactagagc agcatttata gagtttgatg 4080
gcaaatagat taggcagaac ttcatctaaa atattcttag taaataatgt tgacacgttt 4140
tccatacctt gtcagtttca ttcaacaatt tttaaatttt taacaaagct cttaggattt 4200
acacatttat atttaaacat tgatatatag agtattgatt gattgctcat aagttaaatt 4260
ggtaaagtta gagacaacta ttctaacacc tcaccattga aatttatatg ccaccttgtc 4320
tttcataaaa gctgaaaatt gttacctaaa atgaaaatca acttcatgtt ttgaagatag 4380
ttataaatat tgttctttgt tacaatttcg ggcaccgcat attaaaacgt aactttattg 4440
ttccaatatg taacatggag ggccaggtca taaataatga cattataatg ggcttttgca 4500
ctgttattat ttttcctttg gaatgtgaag gtctgaatga gggttttgat tttgaatgtt 4560
tcaatgtttt tgagaagcct tgcttacatt ttatggtgta gtcattggaa atggaaaaat 4620
ggcattatat atattatata tataaatata tattatacat actctcctta ctttatttca 4680
gttaccatcc ccatagaatt tgacaagaat tgctatgact gaaaggtttt cgagtcctaa 4740
ttaaaacttt atttatggca gtattcataa ttagcctgaa atgcattctg taggtaatct 4800
ctgagtttct ggaatatttt cttagacttt ttggatgtgc agcagcttac atgtctgaag 4860
ttacttgaag gcatcacttt taagaaagct tacagttggg ccctgtacca tcccaagtcc 4920
tttgtagctc ctcttgaaca tgtttgccat acttttaaaa gggtagttga ataaatagca 4980
tcaccattct ttgctgtggc acaggttata aacttaagtg gagtttaccg gcagcatcaa 5040
atgtttcagc tttaaaaaat aaaagtaggg tacaagttta atgtttagtt ctagaaattt 5100
tgtgcaatat gttcataacg atggctgtgg ttgccacaaa gtgcctcgtt tacctttaaa 5160
tactgttaat gtgtcatgca tgcagatgga aggggtggaa ctgtgcacta aagtgggggc 5220
tttaactgta gtatttggca gagttgcctt ctacctgcca gttcaaaagt tcaacctgtt 5280
ttcatataga atatatatac taaaaaattt cagtctgtta aacagcctta ctctgattca 5340
gcctcttcag atactcttgt gctgtgcagc agtggctctg tgtgtaaatg ctatgcactg 5400
aggatacaca aaaataccaa tatgatgtgt acaggataat gcctcatccc aatcagatgt 5460

ccatttgtta ttgtgtttgt taacaaccct ttatctctta gtgttataaa ctccacttaa 5520
aactgattaa agtctcattc ttgtcaaaaa aaaaaaaaaa aaaaaaaaaa aa 5572<210>
15
<211> 103886
<212> DNA
<213> H. sapiens

<400> 15
cccttgcagg gaccgtccct gcatttccct ctacactgag cagcgtggtc acctggtcct 60
tttcacctgt gcacaggtaa cctcagactc gagtcagtga cactgctcaa cgcacccatc 120
tcagctttca tcatcagtcc tccacccccg ccccacaaca gcctaccctg cctccggctg 180
ggtttctggg cagaggccga ggcttagctc gttatcctcg cctcgcgttg ctgcaaaagc 240
cgcagcaagt gcagctgcag gctggcggct gggaaccggc ccgagcaagc cccaggcagc 300
tacactgggc atgctcagta gagcctgcgg cttggggact ctgcgctcgc acccagagct 360
accgctctgc cccctcctac cgccccctgc cctgccctgc cctcccctcg cccggcgcgg 420
tcccgtccgc ctctcgctcg cctcccgcct cccctcggtc ttccgaggcg cccgggctcc 480
cggcgcggcg gcggaggggg cgggcaggcc ggcgggcggt gatgtggcgg gactctttat 540
gcgctgcggc aggatacgcg ctcggcgctg ggacgcgact gcgctcagtt ctctcctctc 600
ggaagctgca gccatgatgg aagtttgaga gttgagccgc tgtgaggcga ggccgggctc 660
aggcgaggga gatgagagac ggcggcggcc gcggcccgga gcccctctca gcgcctgtga 720
gcagccgcgg gggcagcgcc ctcggggagc cggccggcct gcggcggcgg cagcggcggc 780
gtttctcgcc tcctcttcgt cttttctaac cgtgcagcct cttcctcggc ttctcctgaa 840
agggaaggtg gaagccgtgg gctcgggcgg gagccggctg aggcgcggcg gcggcggcgg 900
cacctcccgc tcctggagcg ggggggagaa gcggcggcgg cggcggccgc ggcggctgca 960
gctccaggga gggggtctga gtcgcctgtc accatttcca gggctgggaa cgccggagag 1020
ttggtctctc cccttctact gcctccaaca cggcggcggc ggcggcggca catccaggga 1080
cccgggccgg ttttaaacct cccgtccgcc gccgccgcac cccccgtggc ccgggctccg 1140
gaggccgccg gcggaggcag ccgttcggag gattattcgt cttctcccca ttccgctgcc 1200
gccgctgcca ggcctctggc tgctgaggag aagcaggccc agtcgctgca accatccagc 1260
agccgccgca gcagccatta cccggctgcg gtccagagcc aagcggcggc agagcgaggg 1320
gcatcagcta ccgccaagtc cagagccatt tccatcctgc agaagaagcc ccgccaccag 1380
cagcttctgc catctctctc ctcctttttc ttcagccaca ggctcccaga catgacagcc 1440
atcatcaaag agatcgttag cagaaacaaa aggagatatc aagaggatgg attcgactta 1500
gacttgacct gtatccattt ctgcggctgc tcctctttac ctttctgtca ctctcttaga 1560
acgtgggagt agacggatgc gaaaatgtcc gtagtttggg tgactataac atttaaccct 1620
ggtcaggttg ctaggtcata tattttgtgt ttcctttctg tgtattcaac ctagggtgtg 1680
tttggctaga cggaactctt gcctggttgc aagtgtcaag ccaccgattg ctttcttagg 1740
ctatctatat ggtctcttcc tgagggctat tgtccgttaa tacagaatac agtacactgt 1800
tagtggatta gcgagctcgg taatccggtc tcctaaatga acaaaaaagt agacgctttt 1860
tgaggttgag catatttcga ttaaatcttg gcttaggccc tagatcaagg gtttagatca 1920
gaataaaatg aaaattagtg ttgcacgtac gcatattgca tcagaatctt gcagtgattg 1980
ttttagtttc ctgagttgca ttgatagatt cttttaaaat atgactgatt tgcataactt 2040
tagaagcaga atcattttca gtatatatgg tgcacattga gggcaaaaag tagttttgtt 2100
aatgtttaaa cttaagttac ctacaacttt gaactgtatg tagaagtttt gtagtttgaa 2160
gtcaatagtg ccataatata ccttataagg cgttcttact agatctttgt tatatttacc 2220
tttttctctc cctatggggt gatgtaggat agtgcttgaa atttgcactt cagtagcatt 2280
taatgttcag tgctcttgtc ataaacatag aatggatatt gagtagtttc tgatcccaga 2340
tggtaatgtg taggttcaag ggtattgtgt gtagcaagtg aagattgcag aaataaaact 2400
tcagttcatg cttgaaattt aagtattgtt gtgatgccag aattgctgct caccgttttt 2460
aggtttcagg tcctctgaca ccttttggta tcgttaattt tactgatttg tgtagaatgt 2520
cagttgtatt ttaccagcta atatctagaa atgctggcaa gaggggttta ctccagcttt 2580
agattgtagg tatgttagct tttttcatac agtgtattaa atttactgag tcagcttgct 2640
gaataagaca gaagcccaag aattttaaca gtgtgtagct ttagttgtct aaaagttagg 2700
ccttcgggct tcaaaagtta gtggtcatcg aaaagcatta atctttgcag tttcaggtac 2760
aacacattgg ttttgattag ggatggggat ggggccctct ttttgcagaa tggggaaagt 2820
attgacagga attgagagct attggtaggc cagtgtataa ggtatgtgaa aacagaatta 2880
agttattggt ctgaagtgac tgaagcattt aggctctatc aaggcctaaa atttggtaat 2940
atgagtttgg taatgcgaat tgtggcagtg gacaatattt agttaaaatt atgtaattgc 3000
ataagtacta gcacagtatt tttaataaaa gttatttctt agcaaatgtc agttgcattt 3060
tgtctaaagg tagagtgaca ctacagtgtc tatatgtcct gctaaaaatt gtggggaata 3120
ttttttttta agacagcgtt tatatcggga gaggttttat tccgttggat tatgttagct 3180
gcatataaat gtgcacagtt aattttgccc aagtttttgt tttgaaatga atgtaaaact 3240
tactgaagaa gtagcttccc aaaatttagt tttctgttaa gccaaaaata ttattttaaa 3300
agagtatttg caaattttga agttgacatt aattgagaat gttactaagg ctaaactgga 3360
cccgcttgcc cagaagataa ttatggaaaa attcttttgt gacttccaaa gcagtctact 3420

attagcatga attactgaca gtcatccaaa tatataggaa caaaaaatta aatgtttatg 3480
taactttgaa aaaaaagcct ttgaagaaaa taattgaatg ctgtctggga gacagatttc 3540
tttcagcact taaagtacat aacacactac ttttactttt cccacttgat tttaaattat 3600
cagggttatt aagaccttaa aattatttta ccaggttttt acatgtgagc tgtgacatga 3660
ctggcatttt ctttgatttc agcgtatgtt ggtctctaca catgaaattt gtgtgactta 3720
aaactttctc taaaactgta cttttagtta tgatatgcat agaaagcagt atcaaatatt 3780
gcgtcaaatg actaataaca cttaatttct agagttgtgg ttttattgag ccaaaagttg 3840
atatgaaaaa aagtcagtaa ggaaagtcag tgaagtgctt gcttttttga taattgcact 3900
cccaataatt ttgatattcc aacgtacttg gtttgcttgt tttcacgtta atgttttctg 3960
tttattggag tgggaaggca ttaaaattgt cattgaagac tttgtctttg acatgttgta 4020
gtatttattt cagtaactaa cctgtgaaaa gttaaattcc tttatgaaag tagtgattgg 4080
agtattttta tctgataaag aaagattaat aatgaagcca tttctccgag gaaaattgag 4140
gacaataatt cagttttaaa atattatcgc aaaattaaat tattctaaaa atttgttagt 4200
agggttatat gcttaatatt agtctgaaat atagtgctga atttgagact atagaaaaat 4260
taagtgtatt tagggtatgt ggaaacgtta ggcttctgtt gtatttttat tgtttggtag 4320
atttgcctct tttcaaataa atgttcacag ggaatacttt taacttgtag agagtacagt 4380
gactattgaa gttacctaaa ttacctcaag gtaagtgatt actgaaatta atcatgaggg 4440
ttttaaaaag tattctattc acaacattta ttttacattg ttttgtatct gctaagttat 4500
atttcctgaa aaacatgact ggaccaccta attgctgtat gataacttac aaacttcatt 4560
tttcatagtg cttattcaag tgtaaagcac aactgaaagg agtaatgtac agtttatatg 4620
aggaaaaagg aattttattg ctgccagtgt aaaagtttgc acagcagtat agtcatcaat 4680
gcagatttac attgcttata atatactaag taaatactaa atgattaaag ataataaaat 4740
atggtgaggt ataaccacct tcattttaaa cttagtttta gaagatagta aagaaagatt 4800
cctttattac ctttttagaa ttttattttt aataacatgg gaaaggcaac tggtgatatt 4860
ttaatttttg tatggaacag tgcatctgct ttctcatagc cacataaaat acataaactt 4920
cttagtgtta tgaaatggct tactttttgg aagtgaagaa gtcttcaatt cttattttct 4980
aatgttattt tgaaatttgc ctccatttgc tgtttgttca tttggtgata gcgcaacact 5040
taaaaaaata ttttaagccg cttctgaagt aatcacttca gtgactttta atggaggagt 5100
atttgttatg ggaaattcac ttcacaaagt tttaacatta attcacttga agtaaacgtg 5160
ctatttttaa attttcatct caatctttta agtaagacga aagcttagga aatcactttt 5220
attattgata ttgtgtgtga cttcagagtt tgtaaagaga attgtagaag tgttgcatgc 5280
atatgacaat tttctgctta attgaaatgt gaggcctctg ccatactaca aggatttagc 5340
ttccagaaaa tgtaatatta acatagctta agaaatgtat tttttttttt ctgtagaaac 5400
cgttgggtta aacaaacagt tcagaagttt tattacatgg aacccattag ttcttaatct 5460
tgttaccttt ttcttcattt tttctgttaa acttgatttt cacagtcagc attgaagaat 5520
tcatcttgtg gcctgaattc attaagaaaa gatgttagga tttgttctga agatagtgac 5580
ttaggaaatt tgtgagactg gggtcagtca gttctgtttt acaattgctt tctatttggt 5640
agctttgaaa ttaatttagt tgcttatcag agagaataat gttgaggtta gactaacctt 5700
aaattggtaa ggctttgctg agcaaactga taactgtaag tcttttatag ggtgcattac 5760
tgccacatat acgttcttcc ataggtggtt aaaagtatat tggtctgtgt ttggtgattc 5820
tctttgtaca tattgagtat atgcattcac taatgtaaaa taatttgcca agaaaggtga 5880
aattagtata ttgtacttga ctattcacct ttcccttagt tttttgaatt tttttcattg 5940
gttgcagagg aagtattagc aatttaattc ttttaaaata atttgcactg gaataaataa 6000
gtatcggcaa atataagaag agtaacataa tttaagggtg aattaatttt atttgggaag 6060
ttttagttct gtatagttaa ggcagattct tcatttgcaa cagttgacat tgggacatgt 6120
gtgaacatct tcaaggtatt aggacatctt caaggcatta ctttttggca gtgttgagaa 6180
tttttttttt tttttttttt ttttttgaga tggaatctcg ctctatcgcc cagtctgggg 6240
tgcagtggca ggatctcggc tcactggaac ctctgcctcc caggttcagg cgattctcct 6300
gcctgaacct cccaagcagc tgggattata ggtgcatgcc accacgccca gatagttttt 6360
gtacttttag tagagatggg gttttaccac cttggccagg ctggtctcaa actccagacc 6420
tcaagtgatc cgcctgcctc ggcctcccaa aatgctggga ttacaggcgt gagccactgc 6480
gcctggccag tgttgagaat attgagagat ggatattgta gctgtacctg ccatcaaaag 6540
aattttcttg acctccacat agtgtgaaaa agaagactgt ttacacatta tattttaagt 6600
aattatacac ataattatta tcagtactca ccacttcaaa tatgaacagt gaatctaacc 6660
agtgtttgat ttctctgtgt gtgtatgtgt atacaaagtt agcaaacctt ttatcttaat 6720
atttattaaa aaacgaattt ttgtttcttt aaagaaaaga ctaccttaga gaatattgtt 6780
ctatagtttt taaatatggt cagatctatt ttaaattatg ttaaaatttt gagtattacg 6840
tttatctata cttttaagca tatatacatt gtttcatttt agattttagg gaggcagtgt 6900
gggctctgga gccagactgc ttgttttgta atcctggatc ttccatttag tagctggatg 6960
actttgagta ggttatttag attttctcaa tctattttat ctgtaaaatg gggatgataa 7020
tggaacctac cgcatacgtt tatcttgaat agtaagtgag ataataataa gtaatttcat 7080
ttagcatagt acctgccaca ttgtaaatac ttaaatggta gctactgctc tgaaaaactg 7140
taatttcagg ttatgtatgt agggaaatta tttgtatttt catttatggt gtatgattgt 7200
aactgaattt cctcagtttg ggccatgtta ggattttgtt tcaagttata agtgttttta 7260
aaaataaggg tattccttta ggaagtctgg gtatgacatg tctgtgattt tgctggttca 7320

tcacaaatgg gaaataaatc tctgctaact caaactgttg accaaagtaa aattaattat 7380
gccaatcaaa aactatttgc tttaaaatat aaaaggcaaa aacttcctat tagcataatg 7440
aagtagaatt tttaaacttt gttataatct taaattttct ttagtgttga agataggtca 7500
acttaactat catacatttt tattcacata aagtaaactc tgcctcaaat gtaataaact 7560
taatatgagt tatgtaaact ttggtcaata gaggtatatt ttttagcatt tccttttgaa 7620
aatttcagcc ttttgaggga gtcttgcaac tgaatgtcaa gttacattta ttacaataaa 7680
atggacactt aatataatct gtaatgcatt aacataatat gggaactttt aaagtattca 7740
gtctctgtat tattgagtcc tatttccaca tttggccagg attctcaata tgatttaggc 7800
ccaagacgtg ggaagaaaga agtaaagaac taaaggattt ttttcttcat ttttttaatt 7860
gaatatgggg aaagatggaa taagcttatc tgtccagtaa aggccattat gtgtacatag 7920
ggattattat ttttcccccc cttgggctgt actgatttcc cagatgtacc acagcactct 7980
tagtagtgaa gcacttgact tctagtgagt ggattttttg tgtgtgtgtt ttatattgca 8040
gagtgaatac actctgtctg atactatgtg actttctgat tatgtgattt ttatgcattt 8100
tatgtgtttt gtaaactagc tgtatttttg gtccatgtct aggttgtaga attgaattgt 8160
gcattttggc atctgagcac agctgagttt tctaaatcaa tctctctcct tgcacctagt 8220
ttttgcttta gatcactacc taagacttac tgttgattta atattagagc acttaagcat 8280
agctttgact tttatttcct ttgatttttg tagattttca ggctgaagta caataaggtt 8340
ctctgttctt tactagtaat tgcaaagatt gtattctgtg aattttattt gtttaatact 8400
tttgatcttt tgaagaggat gtaattattt aaggtattat gaaatgcatt gtgatttgaa 8460
ttagatactc tttggagatg gagttttgct gttgttgccc aggctggagt gcaatggtgt 8520
gatctcggct caccacagcc tccgcctcct gggttcaagc aattctcttg ccgcagcctc 8580
ccaagtagct ggaattacag gcatgcgcca ccacgcccgg ctaattttat attttttttt 8640
tcagtagaga tgggggtttt ctccatgttg gtcaggctgg cctcgaactc ttgacctcag 8700
gtaatctgcc tgtctcagcc tgctaaagtg ctgagattac aggcatgagc cactgcgccc 8760
ggcctcagat actcttttaa ttagatgcgt ttaaaaattt aacccaccat tgctggcatg 8820
aatagatgta tttttagagt gattcataaa tatcgtatac atgtttaaag ttacaaactt 8880
tttgcttatt tcaaaatgca ggattctttt ccatttaaaa ttccctctct ttgtgagact 8940
tctttttgag tattctggtt actctaaact gattggagat gaaattagat agaattgaaa 9000
actgtacttt taaaatgaaa ttttggggat gtcattaagc ttgatttttt aggttttttt 9060
tttagtgtgt attataaatt attttacact gattgtcagc gataaaatgg aatgcctggg 9120
attttttaaa atttatttta ttcattttta taaggtaaaa acagtgtttt gctaggctta 9180
atttgaccat gttgtaaaat ttattgtata ccttgaaaga atcatttatg aaagatactg 9240
aattagctaa tatatactct gtcttatgta gtttttgatt aacaatacac tttttaaatc 9300
attagctcat ttgattttgc aaagaagaac aggtaaccta agaggcagac agaacaggca 9360
ttacttttat ttttctttct tttttatttt atttatttat ttatttattt attttttgca 9420
gcttaggaat tgtagctcca gtggaatcag tatcttgtta atggctagtg aaagactgag 9480
tctgaagaag gatgcaggac ttttttggca cttggtgcag tatttttccc attatgttac 9540
atgagtggtt cttaaacttc agtgtgttag aacaacctga agggcttatt aagctatgga 9600
ttgcttactc caccctcaga gtttctgatt cagtaggtct ggattgggac ctgagaattt 9660
ttatttctta gaagttttca agtgatgctg atgctggtgc tctggggatc acactttgag 9720
gaccaccaat gaacattatc tcccaccaag caaaccctta acatgttata ctcctttagg 9780
ttattagaat ttatacatgc attatttcat ttgacctgta aactctaagt aactttgcat 9840
ggaaaatgtt atcctgattt tatagacgag atagtgagtt tagaaaggca gtatggtgga 9900
atggagcata gatttggagt tggctagacc taaagtccag attaaatctc tgctcaaggc 9960
tgggcgtggt ggctcatgcc tgtaatccca gtgctttggg aggccagcgt tggcagattg 10020
cttgagtctg gaagttcgag accagtctgg gcaacatagg cagaccctgt ctctacaaaa 10080
aaaaatacaa aaattagtcg ggtgttatag tgcgcattgg tagtcccagc tactgaggag 10140
gctgaggtgg gatcacctga gactgggact ttgaggctgc attgagctgt gattgggaca 10200
ctgtactcct gcctgggtga cagagtgaga ccctctctca aaaataaata aataaataca 10260
tccccgctca gccacttatc agttacgtag atacactgcc taaccttagt gaaccctgtt 10320
tcgacaactc caaaatggga gtaaaaatcc taaacttgta cagtggtttt ttagttttgt 10380
taaaagtaca ggtgaggttt ttttcagagt attggttgcc atctgagagt gatccccttt 10440
cacctcctct aggactttta gcattttctg gagacatttt ggtggtcaca gctggggtgg 10500
tagagtgtgc tattggctag gggcttgaag ccagtgatgc tgcttaacat cctatatggc 10560
acaagacccc tccccatcaa caaagaatta tctagcccaa aatgctgtgt aaaatgtctg 10620
gtatataata agtataatat ttgatgaaaa tcagtacctt tgcccccagg tgtgatattt 10680
aagaaggtca acttactaaa tcagtgatgg agttagtcct aacatctggg tgttctgact 10740
gctgctaggc cagtattctt tatatgataa taagaacttt gtccacagaa gatatcccta 10800
ataacaaaaa aggtttattt gaagaggact catgtgttct ttggctgatt gtgaaagtgt 10860
tgctttgaac ttctgttaga aaaggttgaa gatgttttcc gtaagtgttt ttaatactgt 10920
acgtagtatt cagaaggatg tttaattttt tttttaattt tgctagtagt ttttaaagta 10980
atcctttttc ctttaattat gtagttgttg aactgttggg agttactttt ctcttactat 11040
tttgttattt aatgtattct ttgaccttat gcttttttat tctaaagctg cttttattat 11100
agtcagatat gatgaagtta aatgtacaat gtaaaattgc aaatttccaa cgagctatac 11160
aaacttaaat atttctaagt aaagaaaata gggctgactc taaggttctt tgatccatgt 11220

gttgcattct tttctaggcc ctaaatttgc tatgccagcc tgttgaatta aagtgcttta 11280
tttatctaaa ttagaaactt gtattaaagt gaagttttag aaaaaaagaa acaaaatcgg 11340
aatggagttt taggttagcc cagagatggg aagatgccaa gaaggtagct ttagtggatt 11400
ctgaattttt tggttttgtt ttgtttttag ggcaggcaaa tgtaattaca aaagggttct 11460
aggaatagat tgctgtgatt ttttttctgt ttgcatgatt ttacagtttg ctttgcctct 11520
cacttttgaa tgcagaataa aatgtcaagg ccttattttt ttttaaattc ttaagaaatt 11580
taagatttga ctgttaattc cttttgaaat atgggatatt ttgagatacc aattatttaa 11640
gacaaatagg actcattgtt acaattcagt tgaataaggc ttatgatgtt tatttcagta 11700
tatgaatgaa aactatgtgc ttattgtact taagaaaatt tcttttatta aaaacatgac 11760
taaagagaat tttaaaaatc acccactgtc ctacttctct aaaacttaat gttttcatat 11820
tagcttccag ttttgttcat atgcatatac tttaaaacct agttcatggt gaacttaaga 11880
gggtgttctt ttttaaaaac aatttccatt gcactttgtc gttgccttaa ttaaatggtg 11940
aaatcatcag aaatatttat tttcctatac ttatacattt attaagcttg tttccatttt 12000
tttattttgt gattttttaa gtggatttaa gataacctaa acattagaga ggattttcat 12060
ggttttgatt catgaaatca taatgttata caaacctaac tgaagtgtta gagccttgaa 12120
gatttttccc ccgaattaca tatagtaact ctacttgtat ttaatactga aagcatattt 12180
tacttattta agtgagacaa agtaaaattt agctgaatac tttagatcta tcatttcctt 12240
ttcctgttgt aagaacatta cattgtgttg aaattaaagt ggatatagaa ggtaattaga 12300
ataaactgcc acatcatttt tatagtaaag tggtaataac actattgctt tctgtttttt 12360
taatcagaag gagtatgggc ttataatgat gttactgttc cctgaagcat attttgaatg 12420
atacggttta tatttgcaca gttgcccagg taatcattgt gatattaatt gatcaatttg 12480
ctatttattt gcgttttaaa tcagtactag tatttgtgct taaaaatttt gcatatgttt 12540
tatcagattt aatttttaag tgtcagatac taaaacaaat aaccttaact ttattaaatt 12600
ataatttttt atcatgaggt ggtattcatt tattcatata gttagaacaa aaaatattta 12660
aaatattgag gtagaaacaa attagtctct ttttaattaa aagccagatt acttgttaga 12720
gtaacatttt cccaaatgag gtaaaattgt tgcgactgtt aaacttaagg aaattttgat 12780
ctaggtgtgg tatatacctt cttgtggggt gctaatgaaa acagggatgg caaaaatatt 12840
ttgtttgtga gtgtatgcat ttatgctttt tgacaaccta agaaacactc ttacatctga 12900
gtatctttca tggactagct gtaggaaatc tatataaaat agcttagtat actgaaagta 12960
tgacatagtt ttacatatct agattgtggt tgtgattata tataatacta taaaatatgc 13020
taacgtgctg cttaataata ctatttggat tttttttaat actgaaaagg tcacacagat 13080
tgtgattatt gtgtagtgtc caagaactaa ggcctaccat ctgttactca aatgtatgaa 13140
aaagttaaga taatttagtg atataagtgg ttttgacacc actgtttttg gaataatcta 13200
attatgattt ttataaagac taatatcaaa ttttaaacgt ttgcaaaaat gaaacctaat 13260
agttatactg ttatttatat ttttctatta caatacagat actggctgag aactaaagat 13320
tgtgtaataa acgcctggcc ttcagtcatt tggttttttt tttccctcga ttgtttggat 13380
agttaactgg acatcatgtt ttaacttgag aaattaagtt atacaagatt ttgatatttt 13440
aaactagttt tcctaactgg ttgagatata taagaattta gtattacagg actcaatcag 13500
ggaactgatt taataagaat ttcttaaaaa tttgtttaaa tatttttcaa gttcttttct 13560
tcatcttcta caacttaatt cttgtctgta tgcaaatgag cttccccatt taaaattttg 13620
ctgttgcatt ttaggccact atagaagttg tttctttaat tttcactcac aagaatttgg 13680
tcttaccaaa ttgtgtaaat ctttaaaatt gtgtatttgg cttaatatta tagaatctga 13740
ttgatttaat ctaccttgtt tcatttagta tgttgacatt ttcttgagaa atttgttatg 13800
ccaaatgatt aacataataa tattttaagt ttagatatga ttttgaattt acattttcaa 13860
atgcaacttt gtgtctgtgg cctttttttt tttttttttt ttacgagaaa catcttgcca 13920
attttcagat taatctgtga ggaaagtaga ttggtttact agactcagtt tgtagacttt 13980
ggtgagaact gaattggagg ctatgaaaaa aatacctttt gggcctttct gaatagacat 14040
atatacataa attatatctc ttacattaag tgaggcacat atgtaggtga gatttttacc 14100
tgaatattaa aagtttaaaa gtcgttacct attctgttta cttaatagta tttaaagggt 14160
gtgagaggtg ttatgtgttt ctgtcccttg tttttattcc tatccctccc atctaactgt 14220
tggtactctt atcttcccag gtattaaact tgtatgtttt aaaagcttat ttacttgttg 14280
aaatggttaa cttaattagt tttttctttg aagtttcagc ctaaatattt tctgtttttt 14340
tatatgtcct ttaaatatga aaattctaca gctaatcata attagtaatt gtactttttc 14400
ccctattaca ataactggtt tcataataaa atggtatccc ttcaataaca agcatttata 14460
gtagtttatt aaaactaagg gtgttatcta ttcaaaccaa gcaatgcaga cttactgttg 14520
actctgttaa tatattttaa aattgcatat tactaaaatt taaaatatga ttttgactag 14580
tattttggtg tatgttattt tagatatttt gattatgcac tacttaagaa tgaattgtca 14640
agtatgatta taaagttgat ataatagtta accttcagtg ggaataggaa tcatttaata 14700
ttgttagata tttgtattat tagaacaatc tcctatgatt cttactaata tagtaatgaa 14760
tgacagacaa tatgttggct ttcatattta aaaattcaca tgcatttcta gtttatgttt 14820
ttcttcgact aaaattctgc agcacttagg caaagctatt tttaccagtt ggaaaaaaag 14880
taagtcattt ccaaccaatt ttcctggctt gtagtataga ataaagagac ttgattttat 14940
tacattaaag ccaaatataa aatgatgcaa tctagcacac acttgtttgg aacttttctc 15000
ttttaaatat tcagattaag aggacgttga aaggtaaatt tttttttttt tttgagacgg 15060
agtctagctc tgtcacccag gctggagtgc actggcacga tttcggctta ctgcaagctc 15120

tgcctcccag gttcatgcca ttctcctgcc tcagcctccc gagtagctgg gactacaggc 15180
gcctaccacg gtggcgcccg gctaattttt tgtattttta gtagagacgg ggtttcaccg 15240
tgttagccag gatggtctcg atctcctgac ctcgtgatct gcctgcctcg gcctcccaaa 15300
gtgctgggat tacaggtaaa ttttgattgt tattagcaac tataaaagtt ttgcagttgg 15360
cttattggaa aaagaaaacc tccttgccgg agacggagac gcatttgtat tagaactttg 15420
ttttctgagt accttaccta tagtaggttt caaatattgg tgaattagtt gatggttagg 15480
tctgcataat tactgcgtat ggaaattctg gaaccctatt ttttcaaaat gcagctaatg 15540
ttgagagaat atgcactaaa tattactaga tctttgtttt tcaagatgct gatatccctt 15600
aacatcttct gcactttacc tgtttgaata tcttttttgc tgtaaaaatt agtggcctta 15660
tgtctttctg cataattata gagtagccaa aacctgtttt aggttaatca cctctggcaa 15720
aataaatgat aaaagcatag cttttgtaag cagaatgata ttacagaagt taacttataa 15780
atctaagtgt attaaagaca cttaggaaat ttatgataat gctgggtcag cattacagtt 15840
ttaacttttt acagtttttc atatgctttt tttgtgattt tgctgtagaa aattaacagt 15900
tggcatttgg cttagttcaa gtataatgct gttgacaagt atatctgaca cgtcattgaa 15960
ctaataatat ttttgaaagc tgataggtaa gttatatcta ttttgtttca ttcgtcatta 16020
gtgatcggtc ttagatgttt ttagcgagag caaaactgta gaggaatgtg tgtctgtgtg 16080
tgtatatgtg tgtgtgtgtg tgtgtatttt aacagcagga gagttctgaa acaggaaacc 16140
agtcttatca tattcatcca gagacctagg aagaaggtaa ttgtttggta tactcgttaa 16200
aaccagttgg ttgggcaact taaattttta gaggatcaca gatgtaggct tgagcagttg 16260
taatagatga tttctttttt tttctttctt ttttcttttt tttttgagat agagtctctc 16320
tctgtcatcc aggctggagt gcggtggcgc gatcttggct tactgcaacc tctgcctccc 16380
aggttcaggc gtttctcctg tctcagcctc ctgagtagct gggattacag gcgcatgctg 16440
ccatgcccgg ctaatttttt gtattttagt agagacgggg tttcaccgtg ttccccaggc 16500
tggtctcgaa ctcctgagct caggcaatct acccacctcg gcctcccaaa gtgctgggat 16560
tacaggcgtg agccaccgtg tctggcgata gattatttca taattaacac ctgctatgaa 16620
gaaaaattga ttaaaatagt tgagaagtct agtacactct cagctaatat actaaattat 16680
actatggatt ttagagtatt gttaacatta tcagtgactt gatatcttcc tgaggttcta 16740
atttgcttaa ctttaaataa ttggggttca gatcaccttg attgttccct taaagattaa 16800
attttgtaaa actgtgtgta attttcctgt atctggtttg gatagcttta aaaatggttc 16860
ttaagtttaa tgagttcaac tgggaaaaaa gttagttcta ttttagatgt tgtgtcactg 16920
gaaattatgt ttccctgttt gttatatgca cattattaca aagttgtaat caatgttttc 16980
atactgttct ctggtctgtt tttttcacaa atacactttt tatttgtcgc caggtactta 17040
tttttaaagc tatagaggta atatttcatc aggtgagggt aactaccatg gtttgtttgc 17100
tatactgtgt tagggttatt ttcgtttttt ttttctttta taaactatag ttgtgaatat 17160
gtttatatag tttacttttg gtttattaga atatattgct agagtgggat tacaggatta 17220
aagagtgtac agtattttag tttttttttt ttttttacaa gttgcagatt tgttgccaaa 17280
tgaacgagtt tgtagtattg ctaacaagga gaagaattac tagcaagtct tgatgttact 17340
tttgaagagt gtgatgattg catttaggaa gatatctaaa cttctgtttc aaagcaaaaa 17400
gtatgtgcaa atttcttact catgacaaat tcatataata taaaaacatg aaagttgtga 17460
ggtcaggttg tttggagaag tagaaaactt cagtagagtt tatagatagg cagtcttcct 17520
ttctggtttg gcactgacag cagattaact agaaagtgtt agaaggaacc taaaatttat 17580
actaaagtca atttaagtta attaatatac cagaattcct tcttttacaa tttatttata 17640
aaaacaccat attgagttgc cttgtaatga gacatttaaa ctaaatttaa ataacagaat 17700
tcatgcacca tctaataaca acccettatt tacaattata gagtcctttg caattttata 17760
gatattttca tgtatcccat ttggtccttg aaacaattaa tgaagaagtt acagcaagtg 17820
gtattatcat tattttacag aagaacaaaa caaaaatata tgtggcccag agattgagtg 17880
atttacctga ggttataggc tttagattgc atagctggaa gtagaacctt gttcttctat 17940
attaaatgac aatattcatt aagtacttag cacaggattt ggtacctagt aaatatttaa 18000
atgttcctgt gttattcctg actattcctt ctttattctt aaaacgccat tttttgagca 18060
ctcttaatat ttatagttca aactttgtac ctatgtacct tttctctttt agaaaataag 18120
atttcaggct gcattaattt gatctgtaca ggaatgatta tatgttttac atattgggac 18180
aaattgctct ttttttatat accttaagct ctagggtaca tgtgcacaac atacagattt 18240
gttacatatg tatacatgtg ccatgttagt gtgctgcacc cattaactca tcacttacat 18300
taggtatatc tcctaatgct atccctcccc cctccccata ccccatgaca ggccctggtg 18360
tgtgatgttc cccaccctgt gtccaagtgt tctcattgtt cagttcccac ctatgagtga 18420
gaacacgcgg tgtttggttt tctgtccttg cgatagtttg ctcagaatga tggtttctag 18480
cttcatccat gtccctacca aggacatgaa gctcatcctt ttttatggct gcatagtatt 18540
ccatggtgtc tatgtgtcac attttcttaa tccagtctat cattgatgga catttgggtt 18600
ggttccaagt ctttgctatt gtgaatagtg ctgcagtaaa catacatgtg catgtgtctt 18660
tatagcagca tgatttatac tcctttgggt atatacccag taatgggatt gctgggtcaa 18720
atggtatttc tagttctaga tcactgagga attgccacac tgacttgaac tagtttacag 18780
tcccaccaac agtgtaaaag tgttcctgtt tctccacatc ctctccagca cctgttgttt 18840
cctgactttt taatgatcac tattctaact ggtgtgagat ggtatctcat tgtggttttg 18900
atttgcattt ctctgatggc cagtgatgat gagcattttt tcatgtgtct gttcgctgca 18960
taaatgtatt cttttgagta gtgtctgttc atatccttcg cccacttttt gatggggttg 19020

tttgattttt tcttggaagt ttgtttaagt tctttgtaga ttctggatat tagccctttg 19080
tcagatgggt agattgtaaa agttttctcc cattctctag gttgcctgtt cactctcatg 19140
gtagtttctt ttgctgtgca gaagctcttt aggacaaatt gttcttaaat aatgaacagt 19200
tggcactttt tcaactggaa aattcaagga actgctcttt ctgctttctg ctcaatatga 19260
atcttcaatt tagaaatgag agtccatcat taacaattca acatagctta ttaataggaa 19320
aaaaaaacct agtaacaaat gtaaaatctt tgattaaatg agaaagtcat agaagttcat 19380
cagatttgta tttaaagcat gatttcatta gaaaagttga taataaggat ttaactgtga 19440
cataattgga aaatacttgt ttaaacttaa aattttgaaa agaaatgtaa atgtgatgta 19500
acttatgaat cagtggttga gtttcttttt tgctcacaag aaccctaact gtgtgttact 19560
tgaaagcact gatggaaatc agggaaaaag ctccagaagt tcctacgaaa taaaattaaa 19620
tgataaagtc ctggtatctg ctaacttgcc ttccattcct gttatctttt cttcttagtc 19680
tgacttcatt aattctttca ccctggctac tggtttagct cagtgtttta tgagccaggc 19740
agcttcagac tttgcttttg atgctctttg ttcattacct ctaaagctgt attatcactt 19800
tcattttatc attaatgttt catgtatatg ttatagtttc atattgttac tgcaactttt 19860
acttagctat aatttaaaaa atatctgtga tctgtggaaa taattattct atggcagaaa 19920
agtagttatt gcattttact ttataagttg tttaaggata agcataccta tatattaagc 19980
actacaaaga aacttttaca atggctttat ttttagcaaa ccatcatagt taaaataaga 20040
tttagtgtac atgtcaggaa cacagtctta tgaaataagg tttagggagc tatttttagt 20100
tactatatcc tacttgaaaa ttgtagttaa atttctagca tataccctat taatttagat 20160
gcaagtacag atttgagata aggtagatac attatttgga tgtcaactcg gaagttgttc 20220
aagaaaagat attttgttat ttagatgtaa cttggaacat atttctagtg tttcaagtca 20280
tgattgtatg cctagaacag gcaataaaaa tttacttagc tggtaaaaca gccacattat 20340
ttcaaatata gtttagttat attatggatt aaattgattt ttgtggacag actttagaac 20400
ttaattgcta ttaattacat tttttctttg ggacggtatt tgttctttgg tgagaaagga 20460
ttcttgtaac acctaaatca agactgtcca aacatggcat atgcagttta ccagtcaaaa 20520
caaactaatc acttaagatc tgtgtatttt gttttatttg aattatacct caattaaaac 20580
aaatttagca tgtttacaca aaggtggggg gaaactgtct aatatatctg agatctgttg 20640
gagctggggt gaccatccaa cttaggcaag atagctagta ccatgtatgc atttctttta 20700
cctttcttat tgtatataca gtaatcactg atattatgag aaaaagaact ttttaataat 20760
tcaggagata ttttatcgct agtatatatt gagtgcttat tatgtgcctt atatactttg 20820
gactcattta atcctcaaca caacaaccct atgtagttca tactagaatt attcccattt 20880
aaagatgggg aaagtgatgt ttagagaggc taagtgactt gcccatggcc aaagtattag 20940
aactgggatt tgaacacagg tagcctgatt cctaaataaa tgaccactaa cattaaaaag 21000
acataaacat agaggtgttt gttgcaatgt tagtcataat acctaaaaat tgtgaactag 21060
ttaaatgtcc aatagtagta aaatggttca ttaaccatgg tacacccatc aatataatgt 21120
tatattgtca tttaaaattg tattcccaag tttcgtatca ggaaaatata ataaattttt 21180
gaaaaatgta atgggatata ttgtatttat aatacagtaa tcactatgta accaacattt 21240
ttatttccat caaaaattag ttataaagaa atagaaatct aagaagttta atagatatta 21300
ccattgaaca ctagatttgt attagtatct tttatattta tctctctcat ttcctatatt 21360
ggccatgttt acttttaaaa tagtaaaagt cataagctct attttaaaaa taaatgttag 21420
tttattaacc caagaataat agctaactat aaattcatat ttgataaaat aaaaagatga 21480
cattcatcat aagggataca tacctgtgct agcatatggc atttttaaaa tcacatgagt 21540
aatttgtaat catcatagaa atattagaaa atacaaataa gcaaacacat actgaaaatt 21600
tagtgttccc aaatctaaaa cagagacact gtttttttct tttgggattg tattttagat 21660
atgttcttaa gttataataa agtaaatact taaaaggcaa attgatacat taaggatttc 21720
aaagagtaaa actttttgtt aagctttgat gttctttaga aagtttagat tattccacaa 21780
gtcactgtcg ttgaaagaaa agtagttaca gtggggtctt atgggataag gcattaccat 21840
ttgttcagtt gagagacagc tatcactatg ttttaagcat tgttcatata ttagctcatt 21900
taatcctcat agcaacctta tatgataggt acctttatta gccccattct gcttaggaag 21960
caacagaaag agtatgtatt ttgcctaagg ttgcacaata agttaagctg gggttccaat 22020
tctagcaagt tggttctaga gtgtatgtta ttaaccatta tgccgtagtg cctgcaagta 22080
gatctctaga tgtcagaaat actcatcttc ctctggttac ctggttgtta taaatcttta 22140
tgcttaatac ttatgtcatt atatgtaaat ttcgtattaa acactcaact tattcagaag 22200
aatatcaggt aagtgtaggt taaggctgtt ttctatcaga aatcattata tgtatatata 22260
ttcctcagtt cttatgttgt ttagtttttc taaaatgtca aattttataa tatatggaga 22320
agtataaatg tatattagaa agattttgtt tatttgtgta atttgtggca taagaaatat 22380
ttgcctcaag atttggtgct tgtttaggta gttgctggca ttacttttgg aaatgtcagt 22440
aaattttcat actgtcttgg aattttttca atttttacat tttattagta aatgtaatta 22500
caggttagta aatcacttat ttgaacctgt ttcctttgaa agttttatat ttttatttgg 22560
aaatagaaaa accttaattt cctctcgttg ggcagtatgg tgtcaaaagc ttgggctttg 22620
gagcttcgta tataatctgg gttcaaatta taacttacta gttactaact tgggcaaatt 22680
acccagtctt tttgactctc aatttctttg tctatgaaat gtaatactat ttaggattgt 22740
taggattaaa tgagaatata tttggcatac tgtctggtac atgatactta acaggtacta 22800
gttgtctaca tctttctaac ttaggatgga tgccgatgtc ttgggtaaca tctcaaactt 22860
tatcagtaag gaaggtgaga atctgaagaa aatgaaacct taaaaagatt gaattcctgg 22920

actccattta aaggagtaaa tagctcacga acaagacttg ctgctctgca aagtcttcca 22980
tgttgatcct ggtctttgac tccttatctg tctgattaaa ttgaattcgc tgccgtggca 23040
tccttaaagc tggaccttac tttgtcagtc ctgccttctc catgttgctt tgtgtgtaag 23100
cttcactgga ctgtttgctt tttgctgatt attttatgta tttccatatg tctactttag 23160
cctttgcttg gaatgttcta actgctcttg tttcttcctc tgtttactgg tttctgactt 23220
aactcttaag gattatctaa tatattacct acttggtgaa ggtttatctg tgtccccaca 23280
gaattaatcc ttccctcttt aactcttaag ctatcttatt ttttatctaa tcgggtcttt 23340
ggcacaatta taggtctgtg tgtctgtctt ccctaataga ataggaaagc cttgaggaca 23400
gtagtctttg cttacttagc cttatattct cagtgcccct tgtgcaaaac gttcaatata 23460
tgtttgaatg attatgtgaa tgaaggaggg ggccagctga atttacttta atgattatgt 23520
aacacccatt tatgtatagt tatagacttg tctgaaatga gttaaatcct ttgcaacgtt 23580
tgctgctatg ctttgaatgt ctcttacaaa actcatgttg aaatttaatt gccattgtaa 23640
tggtattaag agatggaacc tttaagaggt gattagataa tggtatctct atccttatga 23700
atggattaat gctgtttgaa tggtagtgga tgagttatct tgggagtttg gcctcctccg 23760
tctcacatgc ttccttacct tctgccatgt ggtaactcaa cacaaagatc cttgccagat 23820
gctgatgcca tgctcttgga cttcccagtc tccagaaccg tgagccaaat acatttctgt 23880
tcattctaaa ctacctgttt tgtggtattc tagtaaagca acataaaatt tactaagaaa 23940
actggtacca agagtgtgat tgttgccata acaaatacct gaaaatgttc aaatggcttg 24000
ggttctggct agagaaggat ggaagagttt ggatgaacag gctagaaaaa gcctgtattg 24060
ctgagaatag agcattaagg acaattctga tgaggattca gaagaagaga gctgtaggga 24120
aaatctggaa cttcttagag agttgtcatc agttggtaga actataagtg gtaaaggtct 24180
ttctgatgat atctcagaaa tgaagaacaa gatactggac actggagtaa aggccatcct 24240
tgttaaatag ttgcaaagaa cttggcgaaa ttatgttcat atcctaagac tttatggaat 24300
gcagaattta agagtgatga actaggatat gctgcagaag aaataactca gcagcagagc 24360
atttaggtta ctggatggct acttttaacc acttaaacta agctggggga agggaatgac 24420
ttgaagacag aatttataat taaaagagag gcagaatgga aatacttgga aaatttgcag 24480
cctggccata gtaaagaatg caaaaggtat gtttaggaga gcaaaccaag ggtgtggtcc 24540
aggaaccatt tgctgaagag attaatattc ctagaggaga cccaagggct atttatcaag 24600
acagtggaaa aagaccccag aggcatttgg gagatctttg aggctgcctg ccccatcaca 24660
ggcccagagc tctaggaggg cagaatggtt tgtggctcag gtggtccttc acaagcttgc 24720
tgcccagggc tacctcagct ccccatattt caacccagtg ggccttggct gtcctaggtc 24780
tggttcagag gggcccaggt gtggcttagg ctactgctga gtactcaaat ggtaaacctt 24840
ggcagcgtct atatggtgct aattctgcag gctcacagga tgaaagagct gtgggagaca 24900
tggctaccac caccaggatt tcaaaggatg atggggatag tctgggagag acttgccaca 24960
ggcttggagc ctctgaaggg tggaaatgtg ggttggagtc actacagaga gtccctacta 25020
gggcattgca taatggagcc atggcagcag gcccaccacc aaagcttcag aactgtagag 25080
ctacaagtat acagtgccag cctgggagaa cttcaggctt gagaccctaa cctgtgaaag 25140
ctgcatgggc taagtacagc aaagccatgg aggtggggct tcccagggtc tattgggatg 25200
aaacgaatgt atttgtaggg tgagaaggac atgagttttg aggcccaggg gcagaatgct 25260
atggtttggt tgtttccttc aaaactcatg ttgaaacttc attgccatgt ggcattatta 25320
tgaggtggaa cctttcagaa gtggttacat tataagggct ttgccttcat taatggatta 25380
atgccattat tgcaggtgtg ggttagttat ctctggagtt tggccccctt tttctctatc 25440
atgtgctcat gccctctttt gccatgtgat gccttctacc atgttatgat gcatccagaa 25500
gactctcacc acatgcagcc ccttgatctt agacttctca gcttctagaa ctgtgagtga 25560
aataaacttc ttttctttat aaattacccc gtctgtggta ttgtatagca gcagaaaata 25620
gacatcagcc tgaagttccc ccaggctggc attaaatact aattattaat tagtatttat 25680
agtgcaagga taaattcaag tttagccctg gttagaatga ccacatttca agggaggggc 25740
tttgtacttc tgtgcatatc tgtaaggata aaaatcttaa tactattctc actgaaatta 25800
atggtttagg ttaggtaagg ttgttagtgc taataattat ttcttttaat aaaatattct 25860
tagttgcgtt gttcaaaaaa catagatgat ttgaatttat attttttggc caaaatatat 25920
ttataatttt gagtaggaat tccagagtat tggtagctat aaccactttg ggttccctgc 25980
cattgcttct ggtgcctcat tttttctgac gtcttccatt ttcttacatt tgtcttctaa 26040
ggtggagtta agattactca gttaagatta tttcacttta ggcctctgct gtcttctgct 26100
ttttttttta aaatgaatgg atataatatc ccaatacatt ttgataattg aacaacagct 26160
acatttttaa gtgaggctac tttcttctaa ttttttaaat ttatttttct cagtttttaa 26220
aaaaaatgtc agattggcta agagttgggg cagctttctt atgtgagagt agtagatgac 26280
agcaaatatt tgtgatgtta aaatgataat cctaatagtt ttcttttaga atctttataa 26340
taaaaaccct ttgaggctga gggtgaattt gtatgttcct aaagtgacaa aaaatgttct 26400
tggggcatag taatttaaat cttagatgct tttattagta taattttttg gtagaaattt 26460
ggcattaaaa aatgcataca gagctttttc tacatacagg gcaagacagc attttgtcat 26520
ggcaattagt aaatagataa ttataaaaca tctaatttta agcaatttgt tacagaaacg 26580
atacaggtac attgtggcaa aaatagaaga tacaaaacaa gcaaatatga gaaaaaatat 26640
acatgccacc acctatatat gacttttagt actttataat cttttgtcta tatatgtcta 26700
tatggatata tacgtattct ttttatccaa atggtattac attgtacatt ctgttttgaa 26760
acctgccttt tttagtcatt tacatctact tttccatctc agtaactttt catcttgtgt 26820

aatgcccgga tcacattaaa atgtttccaa ttagctcaaa aatgtccttt atggctggtt 26880
tggctaaaac agtatccagg ccagcattgc acctatgaaa ttggctgtta ggaatcttgt 26940
atctttaaaa atcaagggca gcaacccatc ctcccgcttc ccccacctcc caccaccccc 27000
ccaccttttt tttttcttaa agatactggc ttgttgaaga gagaatgggt catgtcctac 27060
aaactgtctg aatttgtcca gtttgctgtc tcatagtgtc atttagcttg ttttatccta 27120
tgtatttcct gcaaattaaa atttgtatct gaatccttgg tggatttgag ttgaagatcc 27180
ttaaccatca tagatgatgc tgtgtccttt atattgcatg tcagaagtta catgatcttt 27240
acttgattca tgatgaggag atggccactg aaattggaca gtgacagtct tttctgccca 27300
ttgttcaatt atattcgtct ctttacatta gaaagtattt tgggtagtag tattttggtg 27360
ctgtaagaaa gttcattttc tcatcaacca ctcacctatt ggtttaacac catttgttga 27420
tctttgagta tatcagtaat ttcatcaggg tttgcaaaat aagactttaa attctattgt 27480
tttacattat taattgatgt tttttgataa ggtagaactt gtgaaatggg actatttgtt 27540
tgtctttaaa tacagtctct ataggaaaga caaaataaat acttaaatct cactctttac 27600
catttttcaa agtgaagaac tattccgtta accacctcaa aagatgataa ataaaaaggg 27660
tatttttagt tgtttcaact tttttttttt ttttgagatg gggtcttact ctgttgccca 27720
ggctggtgtg ctgtggtgca atcatggtac accgaagcct cagtctccct gggctcagat 27780
gattctccca cctcagcctg ggactaaagg tgtacaccac catggccagc caattttttt 27840
gtattttttg tagagatggg gttttgccat attgcccagc ctagcctcaa actcctgggc 27900
tgaaggaatc cacccatctc agcctcccaa agtgctagga ttacagacgt gaccaacaat 27960
atccggcctt aacttttttc ttttgagtgt ctttatagac tcaaggactt ttatttaatt 28020
cagggtgtta gtaccattta aatgttttct ttgatgctca gattatcaca gctagtcatt 28080
tggaccttta taccacctcc tatgtccatt tgatataggc cattaatctc tataagcctt 28140
cctccttctc ttggaatgaa aaggtatcct aggctcacct gtaccttccc tactccagac 28200
ctggcattaa gtctttttcc aaggagtttg gtacctttta gtttattatg atattagaga 28260
tgaaaatctg tgttctagga atgtttatta ctgctagagt gatgttgctt ttaggccatt 28320
tcagagaaaa gacctagaaa acagattttt acaaacatga attcatactg atatttttag 28380
ttttttacat gatttcttga ttttacaata ttatctgctt tcttaactta aaattatgaa 28440
ccttaaagtc attagcataa cttctttgct tatttctaca acataaagaa aatagtcctg 28500
gtgcggtggc tcacactgta atcccagcac tttgggagat tgaggcaggt ggattgcttg 28560
agctcaggag ttcaagacca acctgggcaa catgatgaaa ccttgtcttt acaaaaaatt 28620
agctgggcat ggtggcatgt gcttgtattc ccagctactc aggaggctga ggtgggagga 28680
tcacctgagc ccaggaggtc aaggctctag tgagccatga tcatgccacc acactccagc 28740
ctgggtgaca aagtgagacc ctgtttcagg gggaaaaaaa agataaaata gtttgaggag 28800
gctggatgta gtggctcatg catgtcatcc tggcactttg ggaggtcaag gtgggaggat 28860
ggcttgagcc caggagtttg agaccagcct gtgccacatc atgagacctg gtctctattt 28920
aaaaaaaaaa aaaaaagaaa atagtttgag gatatcaata atgatattac tagaatcagt 28980
aaaactacca aaagaagttt aaagtttctt cctagtgttt ttttgttctt agaatacttc 29040
ctaccaagaa gtgcagtaaa agtgcagtgt ccaaatagcc cttgtaacaa aacctttctc 29100
tttctcctgg gtgccaattt gacatttaat cagttttgtt tctagcagtg ttcaatttat 29160
tagattataa gtcttttttt tctttatatt attctaagat caaaaatata taaagatata 29220
cacaggagtc ctgctgctac ctgttcttgc tatgcttttc cccttttctt ccctttctct 29280
gtgaagcagc catttttatt agtttcttgt ttatcactca tgcatgcata tgtttattga 29340
ggatgttgac attcaagcaa atatatgggt taacattctt tttgtcatcc ctatacgaaa 29400
gatataccca gtatactcta ttgggtgggt ttttttcctt aaaatattca gtagatctct 29460
ccagttagca catagttatc ttatagatag aacatataca tataaccttt tcttaaacta 29520
tgctattaaa ataatagctt tcagtaactt gataattatt tttggattga aaatactact 29580
gaaatcaact caatcatgtg aaagctgcag aaagaaaaag acctagaaaa agggcattgg 29640
attaggtcaa ctttgaattt tatttggaag ataaatgagt ccagaagtga gtgggcagag 29700
attattggag ttggtcttga aatgaggcgt taggcagatt gactgggctg gtgtgaaagg 29760
tctgtcagaa aatcatgaga ttagattgag gtacctcaaa aaatgagagc tggtatgatg 29820
agtgggtaag aatcataaaa gcgtagagtg ttgatgattt ttatagttta taaatggttc 29880
ttgtgtgtag agttttgttt ttatgctagc tatagtctgt aacataattc actataatgg 29940
gcatgctaaa tatccatgac agttgaccct tgaacaacac agagggtagg ggcgcctacc 30000
cctgtgcagt tgaaaattca catgtaactt ttgactcccc aaaacttaat atttagccta 30060
tacttgacta gaagtcttac tgatgacata atgttcgtta atacatattt tatatatgtg 30120
tcagatagca tatttgtata ataaagtaag ctgcaggaaa aatattaaaa tcataaagaa 30180
gagaaaatat acttactatt cattaagtgg aagtggatcc tcataaaggt cttcatcctc 30240
actgccttca ctttgagtag gccgaggagt aggagagaga ggaaaggtca gacttgctgt 30300
ctcatgggtg gcagaggtag aagaaggtcc acatacaagt ggtccgacac agctcaaacc 30360
ggttttgttc attggccaac tgtagtttga ttgaaagtaa taataaatga agtttctgcc 30420
tcagttcagt attatcaagt catagatagc aagggctgga agaaacctta gtagtaatct 30480
ctttgagtct aattatcatg tagaatagga aattgcggtc tagaaaggtt aagtgacttg 30540
tccaaattac acaactagtt agagacatag ccagctctta aatctgactt ccagattttc 30600
actgtgtctt cttttttctg taacgtgttg ccttttttag ccatgaaaaa ttagaagttg 30660
aactcttgtc ttttcaggca ggtgtcaatt ttggggtttt gttttgattt ttggtttttg 30720

acataaagta ctttagttct gtgatgtata aaccgtgagt ttctgttttt ctcatatacc 30780
tgaatactgt ccatgtggaa gttacctttt atctttacca gtattaacac ataaatggtt 30840
atacataaat acattgacca ccttttatta ctccagctat agtggggaaa actttctttt 30900
cataactagc taatgtttta aaaagtattc ttttagtttg attgctgcat atttcagata 30960
tttctttcct taactaaagt actcagatat ttatccaaac attattgcta tgggatttcc 31020
tgcagaaaga cttgaaggcg tatacaggaa caatattgat gatgtagtaa ggtaagaatg 31080
ctttgatttt ctatttcaaa tattgatgtt tatattcatg ttgtgttttc atttagaaaa 31140
gatttctaag ccacagaaaa agatactttg tgatgtaaac tattattgta gtgctctata 31200
atcatttttt ggcttaccgt acctaatgga cttcaggggg atacagttca tttgataaga 31260
actgacctta tacattacat aatcaggtac ttatgtgata tcatttcctg gactccataa 31320
aatgctggtc accaggttta atacctggat tccattacag tgtgattttt gtcttatttc 31380
atagttgggg attaggctta aaatcctaga gtggatttat tcagttaaat ttattcacac 31440
taagatgtag atgactaata ctgtatattt ttatgtagac caaattttaa ggtaccactg 31500
tgcatatgta taccaactac ctgaagaagt atttggttgg tacaagagat atagaaagga 31560
atcgctgggt gtaccaaggc taatcagttt tataattttg cataattttc taactgcgat 31620
tatcatttag tttagaacaa tttatttctc aaggcccatg taaatattat ttttaaaata 31680
tacagtctta agaattcatg gcatatttta tgaaaggagg aattcatgtc tgatgtgcaa 31740
atagtcttaa gatattttct aatttcagag caaaaatata tatgtatgaa taaattaact 31800
gtaaattgtc agtaggaacc ttaagaattc gtggcgtatt ttatgaaagg aattcatgtc 31860
tgatgtgcaa atagtcttaa catatttgct aatttcagag caaaaatata tgtgtattaa 31920
taaatcaact gtaaattgtc agtaagaacc ttaatggctt taaaagttaa aatttcaggt 31980
caagcattgt ggtgtgctcc tgtagtccca gctacttggg aggctgaggt gggaggatca 32040
cttggcttga acccccaggt agagggtaga ggccagtctg ggtaacacag cgagaaccca 32100
tctcttaaaa aaaaagttta agttgtggat tatttccttt acactctttc attagtatct 32160
ttcctggaga ctttcaattt aaatacttgg tgcttatgac aattagatgt taaaatggat 32220
gggaaagtac tttgtaactt ataaagcatt atgcagatgt agactccttt tataatagtt 32280
gtgtaagtat ataagacaac ctacattctt catgagctag ccataagttt tagcaacttg 32340
ctttgaacca cggtagattt acaattttct gtagtattga gttgtgttca tttagaattt 32400
tgtaatattt atattgaaaa tcaaattttt gtacctacaa aaactacaaa aaaatccccc 32460
tagtttttat agtttctatt aaaattatag ctggtacata gggatgccag aaggactgtt 32520
taagaagctg aaaatagaga aatgaattta tcttctcata gttaggcagg gcacagtaga 32580
aggatgctta acattgcaag ctgatgggaa cagcaggttg atatagcttg tgataacact 32640
tctaaagaaa aagcaatgag ccatagaaaa aagaaaaaga tacattttga attaaggaag 32700
atggtgaatc tgggaagtga gcagtacagt caccagacgt gtatcctctc ctatggtaca 32760
gaagtgttta ttgggtctct ttatggcctg catgatatat cccacaagat gacctacttc 32820
acattatttt aattctgtat tcaactaagc actaattcaa cccagccaga ttagtactca 32880
taccaaaaaa gagtgaatac tctgaataga gggcaggttt tctgattatg gtgagaatat 32940
ctttgtggta aattaatctg gtgtgctagt ttttacgttg gtctcttctc agtgtcgtta 33000
gtcactgagg ctgattgatc atcttttagg ttactgataa agttcctgta cagctgattt 33060
tcagacctta gattgcaata acttcaccaa gaaaatactt cattgggaag cattttggtc 33120
cttccatttg attcataact cttaccttta tgcctctgaa ggaaaagatt tatacattca 33180
gcttgtaatt agtaatcaag actgaggttt agtctatcta gcttcacaat ctatctagtt 33240
tgttttgtct agccatatga tttcttcaaa tatgccattt cttaaaaaaa aatgttttat 33300
gtatcccgat taatatttag ccagtggttc ttttagccga tggatcttgt cacctcttat 33360
gatactatta atagcatgtc aacatgaaga attatctgct gaatataata gctatgctgt 33420
ccttgtttcc ttttgtctca ttcttttttg attgggggat aattggccaa taaagctttg 33480
atagcctcta ttgcccaggc ccctcctctt cttttatgag agaaaggatg aacagtgacc 33540
agaaataaag gtattgtttt tttctatcaa ctaaaatgga aataaataat tcctaagtaa 33600
tttgcctgtt aggattaaag tctccaagag aatggctgtg cctagtacct aagtgattaa 33660
tttccttgat tggttcacat tatattgagg atattagtaa tcagtagtga ttcctttttt 33720
ggttcaaaga tgatagtgtc acagtgaaaa atgtttttaa aatttttgta tacttaattt 33780
ttctgttaac gaaagtattt tcagttggat ttttgtttgc cctctctatt agaatgccca 33840
aagaatattt aaaattttcc ttttctctta tactgcatat ttttcctgtg atttttcccc 33900
aaacggaaaa tactctgcag agattagact ttgttattgt tgtactacat cattgctttg 33960
actaaaataa actcagattg caaatacctt caagcttaca ttgctcagta tttttttttt 34020
tttttttttt tgagacggag tctcactctt gtcgcccagg ctggagtgca gtggtgccat 34080
ctcagctcac tgcaacctct gcctcctggg ttcaagcgat tctcctgcct cagcctgcgg 34140
agcagctggg attatagatg cccgccccca cgcccagctg atttttgtat ttgtagtaga 34200
gatggggttt taccttgttg gccagtctgg tctcaaactc ctgacctcgg gtgatccatc 34260
tgtctcggcc tctggaatta caggtgtgag ccgccacgcc tggctaaatt gatcagtatt 34320
atttaacttt gagggatatg atttgttatg gaatgcgaag ttttatactt gaggtactca 34380
gagtcctttt gagacaaata tttaacttct ccttttgagg ttaccgccta cgattgggaa 34440
ttaatgtaaa aaataagcca aaagaaagtg agggaaaagt gaaccaagct gtaatttttt 34500
tactcttttt tattgttgtt gttattgttg ctgtttttta ctatcttgat tgcaacagtt 34560
tggcttatat atatagcatt tggaattgac agtaagaaag ccacatctca tagaagctaa 34620

ctattcccaa attgtttttt tcttcttttc ctcttactac tgctgttttc ctcctttctt 34680
gctgctaagc tcttgtcctg acatgctggt aatatgaaac agtgttttat tcagataatt 34740
gattattctg taatatgtat gttaatcttt tttattacac tttaagtaat agggtacata 34800
tgcacaactt acagattcgt tacatatgta tacatgtgcc gtgttggttt gctgcaccca 34860
ttaactcgtc atttacatta ggtatttctc ctaatgttat ccctctccca accccccacc 34920
ccaggacagg ccccggtgtg tgatgttccc cgccctgtgt ccaagtgttc tcgttgttca 34980
gttgccacct gtgagtgaga acatgcggtg tttggttttc tgtccttgcg atagtttgct 35040
cagaatgatg gtttccagct tcatctatgt ccctacaaag gacgtgaagc tcatcctttt 35100
ttatggctgc atactactcc gtggtgtata tgtgccacat tttcttaatc cagtcagtca 35160
ttgatggaca tttgggttgg ttctaattct ttgctattgt gaatagtgct gcagtaaaca 35220
tacgtatgca tgtgtcttta tagtagcatg atttataatc ctttggatat atacccagta 35280
atggaattgc tgggtcaaat ggtatttcta gttctagatc cctgaggaat tgccacactg 35340
tcttccacaa tggttgaact agtttacagt cccaccaaca gtgtaaaaat gttcctgttc 35400
ctccacatcc tctccagcac ctgttgtttc ctgacttttt aatgatcgcc attctaactg 35460
gtgtgagatg gtatctcatt gtggttttga tttgcatttc tctgatggcc agtgatgatg 35520
agcatttttt catgtgtctg ttggctgcat aaatgtctat aaatgtcttc ttttggaaag 35580
tgtctgttca tatcctttgc ccactttttg atggggttgt ttgatttttt tcctgtaaat 35640
ttgtttaagt tctttgtaga ttctggatat tagccatttg tcagatgggt agattgcaga 35700
aattttcttc cattctatag gttgcctgtc cactctgatg gtagtttctt ttgctgtgca 35760
gaagctcttt agtttaatta gatcccattt gactattttg gcttttgttg ccattgcttt 35820
tggtgtttta gtcatgaagt ccttgcccat gcctatgtcc tgaatggtat tgcctaggtt 35880
tgcttctagg gtttttatgg ttttaggtct acatttaagt ctttaacatt taagtcttta 35940
atccatcttg aattaatttt tgtataaggt gtaaggaaat gatccaattt cagctttcta 36000
catatgacta gccagtttcc ccagcaccat ttattaacta gggaaccctt tccccatttc 36060
ctgtttttgt caggtttgtc aaagatcaga tggttgtaga tgtgtcatgt tatttctgag 36120
ggctctgttc tgttccattg gtctatatct ctgttttggt accagtacca tgctgttttg 36180
gttactgtag ccttgtagta tagtttgaag tcaggtagtg tgatgcctcc agcttttttc 36240
tttctgctta ggattgtctt ggcagtgcgg gctctttttt ggctccatat gaactttaaa 36300
gtagtttttt ccaattctgt gaagaaattt attggtagct tgatggggat ggcattgttt 36360
ctataaatta ccttgggcag tgtggccatt ttcacgatat tgattcttcc tacccatgag 36420
catggaatgt tcttccattt gtttgtgtca tcttttattt cgttgagcag tggtttgtag 36480
ttcttgaaca ggtccttcac atcccttgta agttggattc ctaggtattt tattctcttt 36540
gtagcagttg tgagtgggag ttcactcatg atttggctct ctgtctgtct gttattggtg 36600
tataagaatg cttgtgattt ttgcacattg attttgtatc ctgagacttt gctgaagttg 36660
cttatcagct gaaggagatt ttgggctgag acagtggggc tttctaaata tacaatcatg 36720
tcatctgcaa acagggacaa tttgacttcc tcttttccta attgaatacc ctttatttct 36780
ttctcttgcc tgattgccct ggccagaact tccaacactg tgttgaatag gagtggtgag 36840
agagggcgtc cctgtcttgt gccagttttc aaagggaatg cttccagttt ttgcccattc 36900
agtatgatac tggctgtggg tttgtcataa atagctctta ttattttgag atacgttcca 36960
tcaataccta atttattgag agtttttagc atgaagggct gttgaatttt gtcaaaggcc 37020
ttttctgcat ctattgagat aatcatgtgg ttttttgtct ttggttctct ttatgtgatg 37080
gattatgttt attgatttgc gtatgttgaa ccagccttgc atcacaggga tgaagccaac 37140
ttgatcttgg tggataagct ttttgatgtg ctgctggatt cggtttgcca atattttatt 37200
gaggattttt gcattgatgt tcatcagggg tgttggtcta aaattctctt tttttgttgt 37260
gtctctgcca ggctttggta tcgggatggt gctggcctcc taaaatgagt tagggaggat 37320
tccctctttt tctatgaatt ggaatagttt cagaaggaat ggtaccagct cgtcttttta 37380
cctctggtag aattcggctg tgaatctgtc tggtcctgga cttttttcgg ttggtaggct 37440
attaattatt gcctcaattt cagagcctgt tactggtcta ttcagggatt caacttcttc 37500
ctggtttagt cttgggaggg tgtatatgtc caggaattta tccatttctt ctagattttc 37560
tagtttattt gcatagaggt gtttatagta ttctctgatg gtagtttgta tttctgtggg 37620
atcagtggtg atatcccctt tatcattttt tattgcatct atttgattct tctctctttt 37680
cttccttatt agtcttgcta gcagtctatc aattttgttt tttaaaaaaa ccagctcctg 37740
gattcattga tttttttttt gaagggtttt ttgtgtccta tctccttcaa ttctgctctg 37800
atcttagtta tttcttgcct tctgctagct tttgaatttg tttgctcttg catctctagt 37860
tgttttaatt gtgatattag ggtgttgatt ttagatcttt cctgctttct cttgtgggca 37920
tttagtgcta taaatttccc tgtatacact gctttaaatg tgtcccagag attctggtac 37980
gttgtgtctt tgttctcatt ggtttcaaag aacatcttta tttctgcctt catttgttta 38040
tttacccagt agtcattcag gagcaggttg atcagtttcc atgtagttgt gcagttttga 38100
gtgagtttct taatcctgag ttctaatttg attttactgt ggtctgagag acagtttgtt 38160
gtgattttta ttcttttaca tttgctgagg agtgagtgct ttacttccaa ctatgtggtc 38220
aattttggaa taagtgtgat gtggtgctga taagaatgta tattctgttg atttgggatg 38280
gagagttctg tagatgtcta ttaggtctgc ttggtgcaga gctgagttca aatcctggat 38340
atccttgtta accttctgtc tcgttgatct gtctcatatt gacagtgggg tgttaaaatc 38400
tcccgatatt aactgtgtgg gagtctaagt ctctttgtag gtcactcagg acttgcttta 38460
tgaatctagg tgctcctgta ttgggtgtat atatatttag gatagttagc tcttcttgtt 38520

gaattgatcc ctttaccatt atgtaatgcc cttctttgtc tcttttgatc tttgttggtt 38580
tacagtttgt tttattagag actaggattg caacccctgc tttttcttgc tttccatttg 38640
cttggtagat cttcctccat ccctttattt tgagcctgtg tgtgtgtctg catgtgagat 38700
acatctcctg aatacagcac actgatgggt cttgactctt tatccaattt gccagtcttt 38760
gtcttttaat tggggcattt accccattta catttaaggt taatattgtt atgtgtgaat 38820
ttgatcctgt cattgtgatg ttagctggtt attttgccca ttagttgatg tagtttcttc 38880
ctagcatcaa tggtctttac aatttggcat gtttttgcag tggctgatac cagttgttcc 38940
tttccatgtt tagtgcttcc ttcaggagct cttgtaaggc aggcctggta gtgacaaaat 39000
ctctcagcat ttgcttgtct gtaaaggttt ttatttctcc ttcccttatg aagcttagtt 39060
tggctggata tgaaattctg ggttgaaaat tcttttcttt aagaatgtag actattggcc 39120
cccactctct tctggcttgt agagtttcag cggagagatc tgctgttagt ctgatgggct 39180
tccctttgtg ggtaacccga cctttctctc tggctgccct ttacattttt tcctgcattt 39240
ccaccttggt gaatctaaca attatgtgtc ttggggttgc tcttctctag gagtatcttt 39300
gtggtggtct ctgtattccc tgaatttgaa tgttggcgtg ccttgctatg ttggggaagt 39360
tctcctggat aatatcctga agagtgtttt ccagcttggt tccattctcc ccgtcactgt 39420
caagtacacc aatcaaacgt agatttggtc ttttcacata gtcccatatt tcttggaggc 39480
tttgttcatt tctttttact gttttttctc taaacttctc ttcttgcttc atttcattca 39540
tttgatctgc aattactgat accctttctt ccacttgatc gaatcggctg ctgaagcttg 39600
tgcatgcgtc atgtagttct cgagccatga ttttcagctc catcaggtca tttatggtct 39660
tctgtacact gtttattcta gttagccatt tgtctaatct tttttcaagg tttttagctt 39720
ccttgcgatg ggtttgaaca tcctccttta gctcggagaa gtttgttact actgaccttc 39780
tgaagcctac ttctgtcaac tcgtcaaagt cattcttcat ccagctttgt tccattgctg 39840
gtgaggagct gtgatccttt ggaggagaag aggcactctg ggttttagaa ttttcagctt 39900
ttctgctctg ttttctcccc atctttgtgg ttttatctac ctttggtctt tgattatggt 39960
gacctacaga tggggttttg gtgtggatgt cctttttgtt aatgttgatg ctattccttt 40020
ctgtttgtta gttttccttc taacagtcag gtccctcagc tgtaggtctg ttggagtttg 40080
ctggaggtcc actccagaca ctgtctggat atcaccagtg gaggctgcag aacagcaaat 40140
attgcagaac agcaaatatt gctgccggag ccttcctctg gaagcttcgt cttgggggca 40200
cccggctgta tgaggtgtca gtcggcccct actgggaggt gtctcccagt taggctacaa 40260
gggggtcagg gacccacttg aggaggcagt ctgtccgttc tcagagctca aacactgtgc 40320
tggtagaact actgctctct tcagagctgt cagagaggga tgtttaagtc tgaggaagtt 40380
tctgctgcct tttgttcagc tatgccctgc ctccagaggt ggagtctaca gaggcaggca 40440
ggcctccttg agctgtggtg ggctccaccc agttggagct tcccaaccac tttacctact 40500
caagcctcag caatgatgga cgcccctccc ccagccaggc tgctgccttg aagttcaatt 40560
tggaactgct acgctagcag tgagcaaggc tctgtgggcg taggacctgc tgagccaggc 40620
acgggatata atctcctgtt gtgccatttg ctaagaccgt tggaaaagcg cagtatttgg 40680
gtggcagtgt cccaattttc ccggtatagt gtgtcacagc ttcccttggc ttggaaaggg 40740
acatcccccg accccttgtg cttcctgggt gaggcaatgc cccgccctgc ttcagctcac 40800
cctccgtggg ctgcacccac tttccaacca gtcccagtga gaagaaccag gtacctcagt 40860
tggaaatgca gaaatcacct gtcttccgcg tggatcatgc tgggagctgc agacaggagc 40920
tgttcctatt tgaccatctt ggaatgccac cttttttttt tttttttttt ttttaaggca 40980
gtttcttgct ctgtcaccca ggctggggtg cagaggcatg atcacggctc actgcaacct 41040
ctgccttctg ggctcaagtg atcctcccac ctcagcctcc caagttgctg ggaccacagc 41100
cacgcatcac caggcctggc taatttttgt gttttttgta gagatagggt ttcgctgtgt 41160
ttcccaggct ggtctcaaac tcctgcgctc aagcgatccg cctgcctcag cctcccaaag 41220
tgctgggatt acaggcatga gccactgcac ccggccaata tgtatgttaa tctcatccct 41280
caagctgata ctgaagtttt tcaatttatg ttatttggtg taaatctagg cagtctttaa 41340
caaaattggt gcttcatgtg tttaagaggc ataacttaag aattgtttgt ttcttataaa 41400
tcaggagaat ggaggtttaa tagaggtgaa ctgtctttct cactgcagaa cctttaatat 41460
gccactatgc attgtaaatc tcccaagagt gagattctag tatgatgctt ttcttttcct 41520
tttctgttct ttccctttcc ctctacctcc tttttctttt ctttgttggt ggcatgagtc 41580
ctatattata aggaaatgct tttagagtac agtcttctga tatatagtga tttttgaaaa 41640
agatttattt attgtcttgt tcactgtgag ctttttcccc catgtataag cagctgtgta 41700
atagattcaa gagcaccccc tcgccccttt ttttttgaga cagagtctcg ctcggtcact 41760
caggctggag tacggtggtg ctgtgatcat ggttcacctc gacttctggg ctccagcgat 41820
cctcccacct catcctctca agtagctggg accacaggcg tgtgtcaccg tacatggcta 41880
atttttctat ttttagtaga ggcagagttt cgccatgttt tccaggctgg tctcgaactc 41940
ctgaactcaa gcagtccacc tgtctcagcc tcccaaagtg ctgggattac aggcgtgagc 42000
ctccactccc agtctcaaat attcttttga aatatttgaa atatgttgat ctctcagtct 42060
ttcaacctta gttgtatgtt gattttcaat aaaaaggaag tatttgttgc cctaacatca 42120
gtattggcta ttcagtttaa aaaaggagtt aaagagatgt tatttatagg caggcttcaa 42180
aagaggaaag aatgatcagt ttcattctct gtttctagca tattctgact ccttctctca 42240
tattacctcg tttttcccac atttttcttt taataaagtg aaattcacat aacagctaac 42300
cattttaacc acggaaagtg tacattccgt ggcatttatt accttcacag tgttacctct 42360
acctttatca agtttcaaaa cattttatca ccccaaaaga aagccctgtt cttattgggt 42420

gcctcttgct tttttttttt tttttttttt taaatcttga gacggggtct tggtttgttt 42480
cccaggttgt agtgcaatgg ggcgatctca tctcattgca acctctgcct cccgagttca 42540
agcaattctc ctgcctcagc ctcccgtagc tgggactaca ggcacgcgcc acatgcctgg 42600
ctaatttttg tatttttagt agaaacgtgg tttcaccatg ttggccaggc tagccttgaa 42660
ctcctgacct taggtaatct gcctgccttg gcctcccaaa gtgctgggat tataggcgtg 42720
agccaccgtt ctggccacct cctacttctt tcattagtct taattcctta gtggatttga 42780
cagtgtttat attatctaca ccaatgcatt tttttgtatg ttaataatag gagatattta 42840
ttgggcattt atttacatac ttatttgcat gtgtaactgt tgtatagcca gtttatgtat 42900
ataatcttac taaatctcta cagtaaatct atccccattt catagatggt ttaaaagaag 42960
ttaacttccc caagcattac ttttagtaag tagtgagact gaaggttgag ttctggtctg 43020
tcagattccg aagttgtttc cttaggaacc atattatctt gtgtacaact cttgggctaa 43080
tcttgttaat attctttatt tgacctcaca ctgttgattc ataccatgtt taaaattgaa 43140
atacattacc tatatttaaa aattgagacc tcacataaaa agctatattt ctagctgctt 43200
ttgaaatttc gaaggatctt ccaacacttg gctgacattc ctgcgtgaca aaaatcagct 43260
ggagctgtgt aatgtccgac ctgtctctgt caatgaacag attattcatt gtcatttctc 43320
atctgttttt gagattgtaa ctttgattct gtataactca tagaattagt agttagacct 43380
atatatgtta gttattacat tatttgtata tgtacagact gctttagaca atattgtagt 43440
gttatatgtt aattttatca attaaaatgt gctataggat cattgtagag atcttgtctc 43500
taattaacag gattcataaa gagaaaacaa aggagagaaa catccagatt gaaaagacct 43560
acatagtgcc aagcacaata aaagaaaccc tatactaggc aaattattat gaaatttgtt 43620
ttaggacacc agaaataaag ataatatcta aaaacttttt agaatcgcct aggagggatt 43680
aggaatatga atagcatcta acttgttggc agtagaattg aaggtagaag gtggtaaaac 43740
atcaccttga aagttctgca ttcagcctag aattctgtat ccagttaaac catcaatcaa 43800
gtgtgaaagt agggggaaaa actcaaggac taaaaaaatg tgctcatgaa gaacattttt 43860
tttggacatt acccagaaga tgtggttcaa caaaacaagg gaataaacca agaaaggaaa 43920
taaaagagct tcagtaaaca gagaatccca ctcagaagca acaaagaaga aagtcccagg 43980
atgacagctg tgacacaagc tgaaaagtta ccagttttgc ttggagcagg agattagaac 44040
ttccgggaaa ataatcaaat tgatagatgg atgttgaaat atttggagaa aaatgtaatg 44100
gattcttgca aaactgagca aattagaaaa aggaaacaat tattagcatt ggtggtttga 44160
gttaacccaa aattgtgatg ttgctatttt aggggaatta agataagtga aacacgtatg 44220
gaatactgct agttttgtaa gtctccttta ccatggcagg acatctgtag ttaataaatc 44280
tgtaagaagc agtattaaga ataatatttt aaaataccta attaaatagg aggaaaaaga 44340
atccgagtag ttgagggtga ttgcatctgg ggagaagcag gaataaaggt ttgaagataa 44400
atagggccag agatgagtaa ttttgttact gtgataatat attttatata tatgtgtatg 44460
tgtgtgcgtg tgtatatata tatatgagat atatctcata tatatgagat atatatgata 44520
tatatatgat atatatgata tatatatata aaacatacag ttcagtagca ttaacatcta 44580
gcattcagta catttacatt gttgtgcaat tgtcaccact gtccatctct agaacttttt 44640
cattatccca cactgacata ccacacccat taaataataa ctcctcattg ctcctcctgt 44700
tagtaccaac cattgttcta ctttcatctc tatgtatttg actattctag gtacctcgta 44760
taagcggaat cacgtgatat ctttttgtaa ctggcttatt tcactaacta tcttcgaggt 44820
tcatccatgt tgtagcagtt gttagcattt ccttcctttt aaaaggccga ataatattcc 44880
attgttatgt atataccata ttttgtttat ccatttattc atcaatagac acttttggct 44940
gttgtgaata atgctgctat gaatatgggt atgtaatacc tgtttgagta tctgctttca 45000
tttcttttgg atatgtaccc aaaagtgaga ttgctggatt gaatggtaat tctatattta 45060
aatttttgag aaaccaccat actgtttgat atactggctg cccaatttta cattaccacc 45120
agcaatacac tagggttccg aatttgccac atcctcacca tcgtgttgtt ttgtttatgt 45180
ttttttttaa taatagccat cctaatgggt gtgaagtctc attgtggttt taatttgcat 45240
tttcctaatg atcagcgata ttgaacattt gcacatgctt atttggtcat ttgtatatca 45300
gctttggagc aatgatgtct cttgaagcct tttgcccatt tatgaattga gtagtttggg 45360
atttttaaat tgtgttttag aagttctttg tatactctgg ctgggcacgg tggctcgtgc 45420
ctttgggagg ccgaggcagg tggatcacga ggttaggagt tcaagaccag actggctggt 45480
atagtgaaac cccatctcta ctaaaaatac aaaaattagc tggtgtggtc gggcgtgatg 45540
gtgcacacct gtagtcccag ctgttgggga ggctgaggca ggagacttgc ttgaacccgg 45600
aaggtggggg ggttgcagtg agacaggatt gtgccactgc actcagcctg ggtgacagag 45660
cgagactctg tctcaaaaaa aaagaaaaag aagttctttg tattctctga atattaatcc 45720
cttattggat atgttatttg caaatatttt ctcccataaa gaatgggtta ctttttcact 45780
ctgttgattg tttcctttgc tgtgcaggag ctatttagct tgaaaaaatc caacttgtct 45840
gttttctttt gttgcctgta cctttggtgt cacattcaag aaataattgc caaattcata 45900
ccatgaaact ttcccccatg ttttctcctg aaggttttta tagttttagc tctcacattt 45960
aggtgtttga tccattttga gttaaatttt gtatataatg ttatgtaaga gtccagcttc 46020
acactttggg atgtgaatat ctagttttcc cagcattatt tgttgaaaag agtgtctttt 46080
tccccattga atagtcttgg cactcttgtt gaaaattatt tgacaataga tgcaagggtt 46140
tatttttggg ctctctcgac tattctgtta gactatatgt ttgttttttt atgtcaggac 46200
caccctaatt ttagtactgt agctttatag taaaatttga aaccaggaag tatatgtctt 46260
gtgtatttat ttacttattt tttgaaatag catctggctc tgttgcccag gctggagtgc 46320

agtggcacaa tcttagctca ctgcaacctc cacatctgag gttcaagcaa tcctcccacc 46380
tcagcctcct gagtagctgg gattgtagac acataccacc atgctcagct agtttttgta 46440
tttcttgtag agacagggtt ttgccatatt gcccaggtgg gtctcgaact cctgagccca 46500
agcagtctgc cctcctcagt gtcccaaagt gttgcgatta caggtatgag ccaccgtgca 46560
tggccccaac ttcttatatt tcaagatggt tttggccctt cagggccctt tgtgagtttt 46620
aggatggatt ttttttttaa cttttaagtt taggggtgca tgtgcaggtt tattacatag 46680
gtaaatttgt gtcaaggtgt tctgttgtat agattatttc atcacccagg tattaagcct 46740
agtacccatt agttattttt cctgagctcg cctcctccca cctggatttt tttttttatt 46800
tctaccagaa acattgttgg gattttggta gggattgtat tagtctgtag attgcattga 46860
atagtactga catcttaaca atattaagtc tttaaagcca tgaacaccag atgtctttcc 46920
atttatttac gtattctttc ttttctttca gcaatgtttt gtaattttca gtgtacaagt 46980
attttacctc cttggttaag ttaattccta agtattttat tcattctgat gatcttataa 47040
atctgttttc ttaatttcct ttcctaattg ttcattctta gggtatagaa acacaactga 47100
ttcttcgcac attaaatttg tgccctgctt cttcgcgggt ttgtttattc tttttttgtg 47160
tttgaaatcc ttgaggtttt ctgcatataa gattatatca tctgcaaatg agataatttt 47220
acttgttcct ttccaatttg agatgatttt tattcatttt cttaatgctc tctcatacat 47280
tcaatactat gttgaatgga agtggtgaaa gcaggcatcc tgtcttgttt ctgaccttat 47340
aggaaaagct ttcaattctt tgccattgac tatcatgtta gctatgggat tttttttttc 47400
cccccagata gagtctcgct gtgtcgccca ggctggagtg cagtggtgcg atctcggttc 47460
actgcaccct cctcctcccg ccaggttcaa gtgattctcc tgcttcagcc tcccaagtag 47520
ctgggattac aggtgtccac cactatgccc agctaatttt cgtattttta gtagaaacat 47580
ggtttcacca tgttggccaa gctggtctcg aactcttggc ctcaagtgat tcacctgcct 47640
cggcctccca tagtgctggg attatagtca gccaccatgc ctggccactg tgggattttt 47700
atatatggcc ttcattatgt tgtggtaatt tctttttatt cttagtttat tgagtgtttt 47760
tatcataaaa tcttgttgaa ttttttcaaa tattttttct gtgctagttg agatgaccat 47820
gtgatttgtt ttcttctttc tattaacatg atatattgtt tttcatatat tgagccattt 47880
ttgcatccca ggaataaatt ttacttggtc ttcgtgtata atccatttaa taagctgtgg 47940
aattcagttt gttggttctg tgttgaggac tttatatcaa tgttcctaag ggctactggt 48000
ctatagtttt cttttgtagt ttctttgact ttgctatcag ggcaatgctg gcctcattga 48060
atgtgttagg aagtgtttcc tcatccattt ttggcaaaac tttgggaaaa aacgatgttc 48120
tttaaatgtt tgatagaatt cacagataaa aaaatcacat ctagggcttt tgtctggaat 48180
ttttttattg ttattattga ttcagtcttg ttactagtta taggtctatt cagattttct 48240
ttttgtgtgt gtgattcagt attagtacat tttgtacttc tcgttatttc tccatttaat 48300
ctatattatc taatttgttg gcatacaatt gttcatagta ctgtcttctc ttttttttaa 48360
acttctgtgc agttgatact aatgtcccta cttttatttc agattttagt aatttgaatc 48420
ttctttatct taatacaggt aaagctgggt caattgttaa aattttttca aagaaccagt 48480
ttttggtttc attgattttt ctctgttatt tttctattat ttatatcctc tctaagcttt 48540
gttatttcct tcatcctgct agctttgggt ttattagttt gttctttttc tagttcctta 48600
agatttgaag ttggattatt gatttgagat cgttttcaat taaatgtgta caactacaaa 48660
tttccctctt aggactgctt ttgctgttct gtaatttttg gcatgttgtg tttttttgtt 48720
ttaatttatt tctaagtatt ttctaagttc ccttgtgatt tcttctgcgt ttaaccgtgt 48780
attttttaat ttccacagtt ggtgaatttt ctactttttc ttcagttatt gattttattg 48840
attttcagtg gcatcctgtt gtgatagaag atactttatt tggttccctg tttttttttt 48900
aaaacagagt gttgctctgt cacccaggct ggagtgcagt ggtgcgatct tggctcactg 48960
caacatccac ctcctgggtt cgagcaattc tcctggtctc agcctcccca gtaggtagga 49020
ttacaggcac atgccaccat gcccagctaa tttttgtatt tttagtagag acagggtttc 49080
gccatgttgg ccaggatgat ctcgaactcc tgacttcaag tgatccaccc gccttggcct 49140
cccgaagtgc taggattata ggcgttagcc accttgtctg gcccagatcc tctattttct 49200
tgcctatatt ctgactgggt gtttttgtcc attattgaga gtagggtatc gaagtgtcca 49260
gctgttattt cagaactgtc tgtttacctt caattctgtc aattttttgc ttcatataat 49320
tgggtggtct cttattaggc atgtaaatgt ttttgattat tatatcttct tgctatattg 49380
acacttattg atgtgtaata tcttttttgt ctcaaccttg ttttgattta gtttgtctaa 49440
tattaatgta gctacctgca ctctcatttg gttattactt gtatagaatg tctttttaca 49500
tcccttattt gtgtctttgg atctgaaatg agtctcttgt agacagcata tacaatctct 49560
tgtagattgt gtttttctta tacattctgt caaactctgc cttttgattg aagagtttta 49620
tcatttacaa ttaaagtaat tattgataag gatttaactg ctgccatttt gctgtttatt 49680
ttctatatgc cttacagctt ttttgtccct catttcctgc cttactggca cttgtgttta 49740
gttgatattt tgtggtgaag tgttttaatt tccttgttat ttccttttgt ttatattctg 49800
ctattttctg tgtgtgtgtg tgtgtggttg ccattggggg tcacatttaa tgccctaaag 49860
ttataacact gcaatttcaa tttataccaa tttactttca atagcataca aaaattcagc 49920
tcataagatt cagtccctgc tcccttccag ttattgatgt cataaaatta catttttatg 49980
tattgtgtgt ctaaaagcgt agactaataa ttgtttttta atgcagtagt gtcttaattt 50040
gtggaaaaca aaaagtggag ttgtaaacca atgttacaat aatgctagct ttggtaattg 50100
ctcatgtatt tatcatttct cagatcttta tttcttaata cagcttcaag ttactgtcta 50160
gtctcctttt atttcagcct gaaagactca cttcagcgtt tcttgcagga caggtctggt 50220

gataatgaac tctctcagat tttgttaatc tggaaatgtc ttaatgtctt catttttaag 50280
gacatttttg ctggatatag tattctcagt tgacaggtat ttgtgtttat ttgtttgttt 50340
cctttcagga ttttaaatat atcatcccac tgccttctgt ccttcaagag ttctgatgag 50400
aaatctgctg atattgagga tccctttgta tgttacaagt tgcttctctc attccatgtt 50460
caggattctc ttttttcata gtttgattat aatctatctc agtttttcct acttggatct 50520
ctgagttttt cctacttgga gttaattgag cttcttgaat atttatattc atgtctcatc 50580
aaatttggga agtttttgat taatatttct tcacataatc ttttttgccc cttttctctc 50640
tttttattct gggattccca gagtgtgtat gtgttggtcc acttgatgat ggtgttccac 50700
aggtgtctta ggctcttgtc ttcaattttc cttcagtttt ttttttctgt tcctcagaca 50760
cgtggtattt tcagctgttc tgccttccaa gtttactgat tcttctgcct gcccaaattg 50820
gcttttgaat tcctctagta aatttttatt tcagtttttg tacttttcag ctccagcatt 50880
tattttttga tttttttatg ttttctcttt attgatattt caattttgtt ttttgacatt 50940
atccatatct tcctttagct ttttgagcac ctttcaacat ttgttttaaa gtctatgtct 51000
agtaagtgtc tgccatctga tcttctcagg cacagtttct gttaatttat ttttttcctt 51060
ttggcctata cttaatgttt tgcttggggt agtttttttt tttggtatgc tttgtgattt 51120
ttgttgttgt tgtcaaaaac tggaatttga atcttaaaga gtggtaactt tggaaattag 51180
atttttctct tttctctaag atgtgctatt ttgtttggtt tttttatttg ttgtagagta 51240
tttctatgct gggtgtaatc ttaagatctt ctcggcctgt gtttttccct gggcatgtgt 51300
agtgactttc taaattgccc tatgtatgca gttcttttgc agtagtatcc tccttaaatg 51360
tttggctcct aaaaggcaaa ataaataaat aaataaataa aaattaaaaa ttaaaaataa 51420
atcaaagggg tgaaatagct ctggatcttt aaatccctgg agcaattttt tcagccaatg 51480
gcagttaaat aatgatagtc tgcctctgtg tcacattttg atcagaagca gcaattagca 51540
atcagaacac agattcctga tatttggagg gcaaggtctt tgttgccaac cttgactctt 51600
acaaactgtg tgcagggtgc tctgggaaca tgtgcatggt tgcctgcttt gagagtgggt 51660
gatgggtagc cgcgacggca caaagagctg aaattgactc aaactaactg atttaccatt 51720
caagtctttc cttagaaact gaaaacctga atagactcca gagttccaga atcacagatt 51780
ctgcttacag tcgtctaggt ggggagatgg gttcctggta cttctgattc tgccatcttt 51840
ctttgactaa tttttttttt ttgttcttga gatggagtct tactctgttg cccgcccagg 51900
ctggagtaca gtagcatgac ctcggctcac tgcaacctct gcctcccggg ttcaggcaat 51960
tctcctgcct cagcttccca agtagctgga attacaggcg tgcaccacca tgcctggcta 52020
attttttgta tttttagtag agacagggtt tcaccatgtt ggccaggcta gtctcgaact 52080
cctgacctca ggtgatcaac ccgcctcagc ctcccaaagt gctaggatta caggtgtgag 52140
ccactgcacc cagctcttag acaaattttt tattccaaac tttttttatt ttatcatttg 52200
aaaggtatat gtttattatt ttgtcaaaaa taattttaaa acgtattctt gaagcttatt 52260
tagatctgtt tcataggaac tgtgaagaaa gtaaagaatt taaaaaatga agacagattt 52320
tctcaccctg cttatgggtg cttctcgtgc tagccttttg caagtgtcgg gaagtgtaac 52380
ctgcaggagg catcagggct ttgggcctgc atggtctgag tgctgccctg tgagtttcag 52440
aaggcgcagc aacctgtata cctgaaagcc atctctgctg gggcaggtac ctagtgtccc 52500
cacctacctg ggtcgtagtc aggccctggg caagcctgct atgcttttcc ttccctaatc 52560
cctcaggggt gggatagaga gcacagtggc ctcccaggga ggtagaagct gctccagact 52620
aacaatcaga gctgccagtt cttaatcccc aagaccgcca gacttcacaa agacataccg 52680
aggtctgtgc tgtcagtgcc ccactactac actcccttaa gtagccccac attcttgtgc 52740
ttgtttcttt tttctgctct ctttccttgc ccaggtaaga ggtctgccca taagggatat 52800
tttgcagcat gtgaagcttt ttaaaaagtt aggcttattg aagtataatt tacacacaaa 52860
gtacaaaaaa aaaaagactg tgttctcaaa tctgtgagtc attaatgggt ttagatgttt 52920
atatattgaa attattggaa gtaaggtatg tttatattag aaagatttgt agtctagatt 52980
atccaagttt tgggagtatt acctctctgc ttttgtttat ctactttttt agtctctact 53040
ttccaagtat ctataggcaa attttcccat ttccctttgg aaagtgctgt tttcttgctt 53100
tttttccgcc tttccattgt gtcagactta taaggcaatc agccaactgt gggcatgaaa 53160
tccttgggag gaaagagaag gaagtgggag gggcagccat ggtgaatgtt tccctaagtt 53220
atagtcaagt tctttgagag aacataacct catccccttt ttaaactgtt gtaatacttt 53280
cttttaaata gattgtttat tctcctgcaa gtctcacagt tgttcacagt ggtaggtaag 53340
aaatcataaa gttcaaatat taaagggagc tcacaaaaga gcatggtttc accagccctc 53400
actaaaaaca aaattatggg aaaatgctgt aaaagaaacc agaattcttg gttgcaaatg 53460
atagaaagtg actctgattt acctaatcag aaaggaattt ttaaaaaagt attaggtagg 53520
tggaagctaa gggaagcaag acatggcccc aaggtttcaa caggagcaat ctgcttagga 53580
ctttgctgct tggacacttg gtgtaatagc tgctgccact atgcctcgaa actggtgact 53640
ctgctcatta actcacctcc tctggtgatc tctaggaata atctctgact ctcctgtacc 53700
ttgtcctcac taggattcgg tatccacggc aaaaagatct attaatagtt ggtatcaggc 53760
ctgtacatgt gttaagagaa agatgaggaa agaagtatct gcttctaatc tcttgaaatt 53820
atctccaaat tgaaatggta ttttggttgc ctaacagcct gaagatgaca aatatcccct 53880
acaaatttct cctattttac cctcttccta actatatctg taatttaaag tttcacatat 53940
tcttttgaaa attgttttca ttgtttaccc actttttaag aaaagcaaat gggaacatac 54000
taccactgtt tggccccttt caaaaatttt atatctgagg aatcttccat attgttgtgg 54060
acatctacct acctgattct tttattaact accttttatt tcattttatg atcatgctat 54120

cattaatagg cccctatgat gaatatataa gttgtttcca gttttttttt tgtcattgag 54180
aacagttcac atatgtatct tgttgtcttt tccaagtata tctttaaggc aaattcttag 54240
cagtggagtt gctaggtcca ttgcgtctat ggtttaaact agtgccttca aaaatggtat 54300
tttatatact cactgtttaa gagtgcttct tccttctatc cccactaact ttggcaaact 54360
gaatagtttc aaactttaac tttttatagc ttgttggcaa aaaatggtat cttgttattt 54420
gatcgtgttt ttttaattgt gaggtttagc gactttttga tgtattggtc ttatgtactt 54480
tctggtgtgt gtgtatgtat atactgaccc tattttcaac ttatttttct tttagtttgt 54540
ttgtattttc cttaatgatt ttcaggaaac caattttatt ctttcttcag acagttgtct 54600
aatgttctgc ttctcttgcc atttgaattt tgtgactaca aaattcagat gaaacaataa 54660
tagcataaag aacttggtgg gttatctttt gttttgcatt attgattgtt tattaagaaa 54720
tattctttaa aagtcacctt gcttaaatta gcaagtagga aatgctttca ataaagagaa 54780
ctgtcatgta cccactactc cttacttact gaatcatctt cttttggata gagaagataa 54840
aagtgaaaag ggaatttaag agttcctgcc tttttccttg tctttagcat tatatagctg 54900
tttaatgtgt gggagtctaa tttctttttt ctttcttgag acaaagtctc actctgttgc 54960
ccaggctgga gtgcagtggc acagtcttgg ctcactgcaa cctctgcctc ccaggttcaa 55020
gcaattctcc tgccttagcc tcctgagtag ctgggactac aggcatgtgc caccatgccc 55080
ggctaatttt tgtattttta gtagatatgg gacttcacca tgttggccag gctggtcttg 55140
aactcctgac ctctagtgat ctgcctgctt tggcctccca aagtgctggg attacaggca 55200
tgagccactg cacctggcct aattttttta ttgttctttt tggtgtgaac attctcccct 55260
cctccaagcc ttttgttttt actattttca tgttccttta tatgtgctgc tgttttgttt 55320
catctgtaat tatctctcat cccctttttt ggctattata atatatatat gtacgttttg 55380
aatctgagct ttgaaggtaa attcactgca gctgtgttgg ttgattttag ataatttgtg 55440
tatttcctcc tttgtctttt ttaaactgga gtcatttgta gttgtttata cagaatttta 55500
gtttttaaaa ccacaagtct ttcattatag gttgagttat gaattcatag cctgttattt 55560
aaatgaagct tttgaaatct gttttactga tctgtatcat atctaactac gccagtattt 55620
ccttccttgt ctgacgtgaa ctctaaaatt atgtgaacac tttctccctg tttcctggca 55680
tttccactca aacttgttcc tcattcttag ttagaaatat atccagaatt gtagtttctt 55740
tctaatctaa tgacagaagc aaattaatca agcatggcaa gaatttattg gaaaactgca 55800
tgtagttgaa aatatgttta gtatatattt tgacagctgt gaagtctcta atttttactg 55860
taccttttct ctgttccaat tttatgctct attctaagga tgtacccatt tctactacct 55920
gactagggag catgtgtatt gtatcccagc agattttttt tttcatagat agatatcctt 55980
tagatatctg ttatccagtg taggtagcca ctagccacat gtagctatca ttatgtttaa 56040
atgtaaataa aataaaataa atttactgag ttgtttttgc tagctacatt tcttgtgctc 56100
agtagctaca tgtggcttgt gattactgta ttaagacagc acagatacag aacattttca 56160
ttattgcaaa agttctgtta gacagtgctg ttctatacag tgtcattctg cctctcattc 56220
taaaaagttc taattcctga agttgatgta ctctttctgt tgctgtcctc tagcttaatc 56280
aaaataaatt tgagtctttt taaaggtagg ttgcatttta catactgata tttctaaatc 56340
agaggctatt tatattactt tttttatatt acttttaaaa attagcttta ttggagtata 56400
atttacatgc aataaaatct acccatttta aatgtacggt tcattgactt ttgagaaata 56460
cacacacaca cacacacaca cacacacctt cttgtaaaca cacaccttct tgtaaacaca 56520
acaaccaaga tttagaacac tcgctttatg aaaagtttcc ctcatgccca tttgtagtca 56580
gtccccaaac ctggtttcag gcaatctctg atctgctttc tatatgcttt gcctatacta 56640
ggattacata taaatacagt catatagcat gtattccttt ttgtgtctgg cttctttctt 56700
ttagtataat atttttgaaa tttatccctg ttgttactag tatcaataat ttgttctttt 56760
ttattgctga ctaatattac attgtatgga tatgacattt ctttattagt gtggtgggca 56820
tttgagttgt tttcagtttg ggtctgttat gaacaaagct gctgtaagca ttcatgtgca 56880
agacttttgt ggacatatat ttttgtttct gtttattcaa tacctttgag tagaattgtt 56940
gggtcacatg atgtagatca gttgaacaga gtagattcca gaaaagttca catacacatt 57000
tcttgacaaa ggtgctgaga ttattcatgg gaaaaggata atcttttaaa caaataatac 57060
tggaacaata gagaaaacaa agtgaacctt gacttttatg tcttatcata tacaaaaatt 57120
aatttgaagt ggattgttga cctaaatgta aaagtaaaat ttaaaaatat aaaacttcta 57180
gatgaaaaca taggagaaaa tctctgtgac ttttggttta aagatttctt agacagtaca 57240
tataaaatta actatataag gaaaaaatgg acaaatttga ctttatcaac attaaaaatt 57300
tctgctcatt gaaagactca aaatgaaaag gcaagcagtt ttggagaaaa tatttgcaat 57360
acatatatct gaaaaaggac ttgaatgtat aatatataca taaagatgct cttacaactt 57420
cataatgaga aaataacccc ataaagagaa gggcaggccg ggtgcagtgg ctcatgccta 57480
taatgccagc acttttggag gctgaggtgg gtgaattgct tgagcccagg agtttgagac 57540
cagcctgggc aacatggtga aacccagtct ctacaaaata aaaaaataca agaaattagc 57600
tgggcatgat ggcatgcacc tgtagtccta gctgtttggg aggctgaggt gggaggatag 57660
cttgagcctg ggaggcggag gctgcagtga gctgtgatcg caccgctgca cgcttgcctg 57720
agcaacacag tgagatcctg tctcaaaaca aacaaacaaa aaaaaaaaca aaaaatggaa 57780
acagaaattt tacaaaagaa gatatataga tggccagtag gcatatgaaa agatgtttaa 57840
aatcagtcat cagggaaatg aaaatttaaa cgtaatgaga tagctcatat ttactggaat 57900
ggctcaaaaa gggcttacag gaattggcaa agacatagat taactggaac tcttatgcat 57960
gttggttaga gcacaaaatg atatgatttc ttgggagaaa tatttggcag tttttaagat 58020

tatttttgat agccttctga atttcttagt gagttatagg tcagttctgc cactgtttct 58080
ttcttttctt tctttctttc tttccttcct tcccttcctt cccttccttg cctgtcttgc 58140
ctgccttcct tgccttgcct gccttgcctt cctttcttcc tttcttccct ttcttttctt 58200
tcttttcttt ttttttttaa aggagtctcg ttttgttgcc caggctggag tgcagtggca 58260
cgatcttggc tcactgcaac ctccacctcc cgggttcaag caattctccc tgcctcagct 58320
tccccaatag ctgggattac aggcgcgttc caccatactt ggctaatttt tttaattttg 58380
gcagaggcag ggtttcactg tgttggccag gctagtctcg aacacctgac ctcaagtgat 58440
ctgcccgcct tggcctccca gagtactggg attacaggtg tgagccactg cgcctggcct 58500
ggcactgttt atttcttttc cctccagttt tatacctatt tagagagatt agatttcttt 58560
gagtactagg aatcactatt tttgagcaga attattcaaa actgttatta tttttttcttt 58620
aacttgaggc aatgtaggag aaagcagtac tgtgcaggtg aaagttacaa acaagaacat 58680
tttaaacaag atagttactt tccatgtatt ggatacgtaa cagaattaat tctaataacc 58740
atcctgaaga tggtcaggag gcattagtta agaattgaaa tgtttggagc ttgcctgtgt 58800
tgatgggatt aaggcaggga tgatttatgt gtaaatttat gcgttagtaa cagcagtaac 58860
cgctgtagtt acactagggt tctaagagca aatgttgatt aaacatgaat gtagcaggag 58920
tgataaggtt tggctctgtg tccccaccca aatctcatgt ggagttgtga tcctcagtgt 58980
tggaggaggg gcttggtagg aggtgattgg atcatgggag tggtttgtaa tggttttagc 59040
actatcaccc tagagctgtc tcgcgaaaga gttctcctga gatctgcttg tatataagtg 59100
tgtagcacct cccctctttg ctctctctct tcctcctact cctgccgcgg ggacgtgctt 59160
gctttccctt ggccttctgc catgattgta agtttcctga ggcctctgat taaacctttc 59220
ttcttctaaa agattaccca gtctcaggta gttctttata gcagtgtgag aatggactaa 59280
tacaagggga aatatatatg gttaccaaat agcgaattag ccatgggaaa aagtagcaaa 59340
taaataatta ttttactttt tcagatgcta atttttcttt tcgtttattt taggattggt 59400
gggagctgtc caatgtcctt aggctgtttt ccaaatgaga taccaaaagc tagttctcca 59460
tcgggtttct caggctgcta gaagcattca ttattatggt tgtcattact tcgagttctg 59520
ttgccgctat gcccacagta gtatttgtta cataacaggt gcttgataaa tatttgctaa 59580
atgaattttt ggaaaataca atctgccaca cctttcttct acagtttaca atcttctgtt 59640
gagatcatcc gatagatttt ttttcttaga tattgtactt ttgaggcctc aaattgctgt 59700
cttttgtatt ttctatgtct gcagagactt tccatctttc actcattgta ttcattgttt 59760
tttaacatct ttgtacatat ttatagtaac tgttttaaag tcactgtctg ttaattcaaa 59820
catctggttc atcttggagt ctgattctat tgcctgctct tttttctttg taataggtca 59880
tgtttttctg ctttgcctgt ctagtaaatt ttaatcgtat gttgaaatgt agggagtttg 59940
gattgttact tcctttaagg gtgctgagtt tcattttgtc aggcatttaa attgatagtt 60000
gatttagtat tgtcaggttt ggttctcttt gttaaagcag gcatttttca gatttgtctt 60060
ttgtcctagg gcatggtttt taacttcaag gttgcccttt ccaatgtctc agctaagtat 60120
ctggggtgtt ccatgaggtc tcttccactt tgcctaggcc agaactccag cttctcccag 60180
tattatattt cgttacctct ggcgtcatct ccgttatgct ttcagatcct gcgcatagac 60240
agcccagccc ccagccaagg acctgagatg aaatccatac aaaattctta gtcccttgct 60300
ccacaaactc caacagcctt agcagtctaa tctcttcctg tttacctcag tgaaatctgt 60360
gttccacttg agttccattt ccttctgtat cagagaagag ccaccatgct gaaagcaagg 60420
ggcactatgt ttctttgttc ttaaggatgg tagcctatct gcaacaactg tagtgtgata 60480
taaaaatata taatttatgt tgctgacagt tacaaatact gcttgcagta ctttgtaaca 60540
taatttttca gattcaagtt catatactct ttttttccac atcaccacac acatattttc 60600
agacttcctc ctcatccttc ttcttgccag tagttgtatt ataattcctg ccagtagtta 60660
cattataatt ttggttatat caatattgag tttttatggg attataacta gataaatgcc 60720
attcatagtt aagtgataga gtatattgtg acttttttcc tgcatgtttt attttttctg 60780
gacttcacag ttgtctctct tttttttaaa aaaattagtt ttcaatgttc ttagctttaa 60840
ttcataaact cacccctaat tgtataaatc tctcaacatg tttaagcaca tttggcatta 60900
tatcaatttt atcttttcca ggtgccttct aatctgtccc agtctggact aattgttctt 60960
cctggcttgc tgtatggctg tctactcaag atgtcccttc accatcattc tagggattcc 61020
cttttcctct cttgtgggtt agattcttca gttcttggag actgtcatct tctttcatgg 61080
tttcccactc ttgttttggc ggagcacatc tttagtaact tcctgacaaa gtgtatggtt 61140
tgagatttcg ctgattttaa aatgccctta ttatatagtc acacttgatt tatagtctgt 61200
cttggtatag aattctaggc tgagaagagt tttccctcaa aatcagaagg ttttgcccaa 61260
ttgtttttta gctgctagta ttgctgttaa aaaggataat gtcattttga ttctagattc 61320
ttttatgaaa cctgtttctt ctctggcagc ttttaggatc ttctgtttct ttggtattca 61380
gaaatttcat gagatatgtg tgcttctatt ttggtcttat tttcatctgt tttgccaggt 61440
actcatgcaa ctttccagtt tgaaaactca catccttcac ttttgagtat ttttcttgag 61500
ttatgtcttc ggtttcttct cagtgttctc tgtttctgga actcctaaaa tatatttaac 61560
atcctgaact cttagttttt gtttagtctt ctgattttca tttgtctttt tattctgtat 61620
attctgttaa ttcctcggct tcatggtctt ctagcccttc ttttgcctat cttatggggt 61680
tgaggattaa acagtttata tacctgaggt gcttaggata tgtctgtcat atagtaagtg 61740
cttgtgttag ctgtaattgt tgtttacttt cataactgtc ttgagggaaa ggtctttggt 61800
cttgattctt tgacttcttg gctgtacatg accttggacg agttatgtaa tctctttgag 61860
acctacctcc ctcttctgta tagtgttaat aagctctagc tctcagatgt ttgtgagggt 61920

cgaatggagt atatatgtga aaatgtttaa tacctttgta cagaattaat agttagtacg 61980
tggatctttc aaatatcaaa agttttcagt ttgatgggaa aatgatgtct gaattttcag 62040
ggttattttt aagagtactt gattatgact gtcttgtaaa tctctatgag ctaggtatac 62100
ttgcactaaa tgctaatgct ttttaaagaa gttatgtctt aatattcagt ctcattatgt 62160
taggttgaag atagaagatt atgaaaatat tctctgaaaa gctctggttt tacttcagat 62220
tgtataaatc tgtgtaatgt aataattatt taagaatgac atgattacta ctctaaaccc 62280
atagaagggg tatttgttgg attatttatt ttcacttaaa tggtatttga gattaggaaa 62340
aagaaaatct gtcttttggt ttttcttgat agtattaatg taatttcaaa tgttagctca 62400
tttttgttaa tggtggcttt ttgtttgttt gttttgtttt aaggtttttg gattcaaagc 62460
ataaaaacca ttacaagata tacaatctgt aagtatgttt tcttatttgt atgcttgcaa 62520
atatcttcta aaacaactat taagtgaaag ttatctgctt gttagagtga ggtagagtta 62580
aagatacatt ttaacagaat tgtattccta aaccgattaa gtcaagaagt ccaagagcat 62640
tgttagatca tttagaaagt gtagtgatga ggtaaaacat tgttggcaca gattcatgtt 62700
acttgatctg ctttaaatga cttggcatct agcccatatt tgagcccata accgtgtggt 62760
aatttgaagt gtaattcaca gtagagcttc tgttaaagca ctaatagcat cttccatgga 62820
ggtatacttc agagtgaata taattttgtt tatcctgtgt ctctagagct attgactgaa 62880
aaagctgtta gggcattctc taactgtaca tcacctaagt tatttaaaat tgctgaatta 62940
ggtggcttgt cttgtctagg acagagtttt aaggactgcc cacctgattg atagagctag 63000
ttgaccttat ctttaacttt ttgtttttct tttgactttg ggagtagaga tgtgaaaagg 63060
taaaaaggaa ggaaggaaga gaaaacttaa ctctttttgc ccatgaagac tgtttttcct 63120
tctcaaaata ttgactattt tctgatttgt aaaaatcggc acataaaacg tgttattttt 63180
tacttgactt ttatctttcc catgtgatat ctataaatta tagataggaa aaatttatct 63240
gtaatttagt gatctttcta gtgtgataaa acgtcagaag tactgagagt ggagtggaca 63300
ttgatattgt tactctcagt aagttttcac tgatttttct cagagtcatg aaggaacaaa 63360
cgtttgttaa gtccttatca cttattagat aacacaaaac atgttggggg ggtgtgtaca 63420
gaggtgagta agatgtagct cccattctca agtcgcttac attctaatgt aaaaggtaga 63480
caaagcatta cagaagaagt aactctgcta tagaaggttg caatgaagag aacattggaa 63540
acactaattt taccttataa agaaggtttc ataaaggaag gcaagtttga gctggggtga 63600
aaaggaccag taagggttga ctttcaagcc aaggagagga ggggaagtga tgttacaggc 63660
caaaggaatg gcattgtaag aagcttgttg gcataaaagt gtttagaata tggcagcgaa 63720
ttcattatca tcagattgtg gtgtctgtat gttgggggtg ggagagaatt gtggtggcaa 63780
taggcaacaa gataaaagaa agtaaaaggt gttatggaaa cttaatgggt ccagcttaca 63840
aatgatctat gcatttaggg gtctttctct tttcctgata aacctctcct acaaagagcc 63900
ttgttgcgga taccatagtg tttctttgga ggaaaataaa aactacaaag ctttgtattt 63960
tttgcacaac tggattcaga atataagtaa taaaaaagga caagaacttt caaaagctag 64020
aagccattaa actgagtcac ttcagggtta gactatcaga actggggatt tagaaagtct 64080
cagaatggaa atcgaaggac accaaagaca aattcggcct ttttcaaaat tttattctag 64140
tttaacatat tcaaagaaag ggaaggaaat tcttttcatt cctgtgtgta gtgacttcct 64200
gctttaagaa cttaggactt cagctgtact atcagtattg taggtcactt aacattatta 64260
tggttaaagt tggcattgga gagagcctag gaacctaact gcctgtttgt ttttatattt 64320
ccaaccattg gattcccaag ttaatgaagt ctgtttatta gttgagggta gctcttaatg 64380
catatatttt aatgcccctt ccccacatgg aatcataagc tttcagaact ggagagtacc 64440
tgaaagagat catttagtcc aaccttctca ttttacagat ggggaatctg aggcctagag 64500
aagttaagtg agttgaacaa ggtcacacag gtacatatgg tagccgacca tccactgttt 64560
atgccaatat tccctttacg ttttgctttt ttgcttgttc gttttaacct ctccaaattt 64620
tactgacttc agaagtttct agaactaagt tatagcatgt tttgagttct aatgtcactt 64680
tccgatcttc tttacctttt ttctacctct gtttgtattt ctggttctgg ttaagtgagt 64740
ctggtaagca gcaggtgttc tattttattt cttttatttt taggatagta ttacatgtga 64800
tatatatgtc tttgcaaaca tacataattt gaagatctta aaatatttgc actaggcata 64860
cccacattta atagtatgtt aaatctttta tagcaattat gatatacatg ggtgaagaag 64920
agttcctaat atggcctttc tgattaactg tatctgttta tatctgtgtt ttcttcaggc 64980
attcataaca ttaagcaaat tcaggtgtac tgttacttaa ttgaattaat cagtttgttt 65040
tgtacaagta tattttattt ttgttccttg ttgtataatc tggtaggaat ggggaagggg 65100
agatagtgaa taaagagatg tatacttctt gcctttgagg aatttaagtt ttcactgtat 65160
accaattttt taaaggtatt tactatattt cagtgcatat tttatttgac atactttatc 65220
attttgtggt aaacctttag ctttactaat tttcatctat taagttttct tttgtaagat 65280
ggtgatagct tcatcaaaga gagtaaagaa gagacctgcc tacctagctg attctatggc 65340
aaatctcact tctctggaag cttttcctgt taatcttatt ccttcagttt ctgcctcttg 65400
tttcataaaa actcattctt taaatgctta ttcatttctc ttgtctcata taaaccaata 65460
tgaggtactg gtatcttttg agttttagtt ataaggaagc ataaatggtt aaatttaaat 65520
ggctaaaccc catttgccat ttgtgtatct ttaattttag tttgttgaga gacttatcac 65580
taccaaacca caaagaattt aaaagaaact gtcagtaggt ataggtggaa ggagggcatt 65640
tatcagagat tttaatttaa gaagaaagtc ttcatcctta tcctaccaac ccccattccc 65700
tgagcatatt tatcattact agtcccagca tatttgctcc catatttcct atgcttacct 65760
gtgaagattt tcataacttt ttccttgctt tttactgtca ctgttggttc tgtgatttat 65820

gacagatact gctcttgtag gaatgctggc tttgactgaa atttgttact gcttttgtat 65880
ttaaaacttt tttttattta taagtagaat tatggaacag tagtagaaaa agtttgactt 65940
ttgtaatcag agatactgag cttgagttct ggctctttca tttgtatact gttatttggg 66000
gcaagttttt taatgctctt aagtcttagc tttctcatat ataaaatgga gataataaca 66060
gttatcacgt gattgtgagg atgaaacaaa aaaaagtgga aactctttgt aaggtgtgtt 66120
catctggttg acacttagta gtcattactt ccactttccg tccatatagt cctcttaaca 66180
gtaatatttg agaggcattt ttattaaagc agtcttaagg agtgttcgtc aaaccacatg 66240
ttctgggatc ctgagaaagt aggggaagtt tagagaactg aagctgcaca aaactaatgt 66300
ttattttctg ttgtgttgtc ctgagaccag cttcttagat tgtgtttcct agtcctacat 66360
ctctgattcc ttataaaata ttccattatg aattcttcac tattgacaat ttctcccctt 66420
ttatcttaaa agtaccaaag aaagtgtaaa atgtgactgt cttgtcagtc ctctttttcc 66480
tgtttttcat gtcagtgggt atgaaattac tagcaaggat gcatatatgt gcatatgtca 66540
ttactaaatg cattttcttt ctagaaaaac tcaatatact aaattgtact aaaaaggaaa 66600
agcttgtttt gttttgagtg gtagtatgaa agttgtttta ttttaggtct gaccagttag 66660
aaaccaatgg attgtagttt atttataatt agttaaacct tcatgtgaat ttggttttga 66720
attaccttta aggtagagaa gaaactatat agatgttttt cagggtttct aaatgtacaa 66780
tacaggttca caatcactta tttgaaactc ttggggccaa gtatgtttcc attttcagaa 66840
attttagttt tcaaaaggta gcacagataa atatactttt acataaacac cccagtgggg 66900
tgtgggtcag tacctgaaat gaaatgtttt actcttcgct ctaagtgtat taaatattat 66960
gtacaatctt attacttcag atcaggattt gctgtagttg agtttgccat aaaacttaag 67020
agaaaatttt agatgttttg aacttttggg atattgaaat tgcaggttaa ggagctatgg 67080
acctttattt gttttaaaat gctaagagtt tattttaagt aatttttaaa aaatttgttt 67140
tgcatagtag ttggagttac cagggtactg ctaaccacac tgatatgtaa gatctctttc 67200
tgagcctttt attgtttgta aacatggcct gttaatcatt agaaagccag tacatactaa 67260
catatcactg ctattaagac aaatattagc atactctagt aatgacaagt cagcatttta 67320
ctattctgta ttgattttac ttattctttc attactctca tactgtaatt aaaacttgca 67380
atctgagaga ctgttgaaaa aggtgatcgt tggcttttca acagggagta aggtctggtt 67440
taaaaaaaaa ttagtaagca tttggccaag tagattaaca acattcagtt tttctttact 67500
gtccttatgc ttttactatt tttaacatat atctttttga agaatagttt gagaattatg 67560
tatgcttaac tatgagatac agatactatt gaaactagtc agttgtttat aggtacttgt 67620
aaaattaaaa atatattcca atagcatgca gatttttcat agaggaaatt tgaaagcatg 67680
gaagcacctg aatttacagt actctgtatt agtggcatca caagttttta agcaaatgta 67740
ttagctctaa ttgcatacac ttaatctttt aagctttggt tttattatta taatatgggg 67800
gtgataacag tatctactta atagaattct tgttattaca tgaaataatt aatgttaaac 67860
acagcataat atgtgtcaca ttataaagat tcaggcaatg tttgttagta ttagtacttt 67920
tttttcttcc taagtgcaaa agataacttt atatcacttt taaacttttc ttttagttgt 67980
gctgaaagac attatgacac cgccaaattt aattgcagag gtaggtatga atgtactgta 68040
ctatgttgta taacttaaac ccgatagact gtatcttact gtcataacaa taatgagtca 68100
tccagattat cgagtgagat acatatttaa gaattatctt taaaaatttc aaaaatttta 68160
attttactgt tgtgttttag gaaaaagtat tgcataaagc tattaatatt gtcaggaaga 68220
ctaaagtgca gcatagacta agaattagga aaattcctag actaaaaata gtataaggag 68280
agggtttacc tactatttga ggcagttggt ctaatagtaa gcaatcacag ggagaaagca 68340
gaactactta actcttctgt gttgaggaat gacataaaag gtaggaaagg atataacaaa 68400
tgttgataag aggagtctga tggatgagag gagggaactg ctttaaatga gtttctactt 68460
cagacataag ttaattctca gagcccacaa aaactttcac ttttatttgt gaaatacaac 68520
tcagttctca tggcttaaca ctttaaacca tgagaaaact gaagagttga gaagcttggc 68580
agatgctgct gtgatagtca aaaagaaagt gggtgccatg agctactatt gatgtatttg 68640
ccattgatcc ctcctgaaaa tctagaatgg actttcagac aaatggtttg aaaattctaa 68700
atcactaatg attgagattt agtataggtt tactaagaac gggttttttt tgtttttgtt 68760
tttggtggat ttaggctgtt gcttactaag caaagcaggc tttagttgag gtttatcttg 68820
ctttaaacag atatttaaca gattttcctg gaggtttttg tgtaccactg ggaaaatgaa 68880
gttaggcaga tgactaagtg aaagctgtcc tgctgactcc ttataatgat agtcattgtc 68940
taccagaaga tctctcctgt cacaccaaag gataattgat tatatcctgt accatattat 69000
gagtcacctg attggagata taagacatac ttctcacata tttagatgac acaggttagt 69060
acattgaata tcagccaggg tttttaagga tcttaataga gtggaactaa ggtagaaact 69120
attaagagca attaatagtg atatatctat agtcctgttt ctaaacaagt ttttttaaaa 69180
acctcaactc tgactatagt gaacagagaa gtcttggact cttacaattc atgtgagaag 69240
acctgaaact ttgataacaa ttatatacat tttgtgagta atttctttgg tgtatgcctt 69300
cacatatctc tggtatgtga cctatgctgc agtccattga gcatagattc ccagaatgta 69360
ttctcctgca gaaaatggag gaaaataata cttggcttcc ctaatgatta catgtgtata 69420
caacactaac atttgcaaga ccacctttaa ataacacact tagcattttt attttatgaa 69480
atgtaatatg tagttctttg catagtttat cctattagta atctattctg tctttggaat 69540
atgttttgtg atgatgaaat aaatactata aatagtatta ttccttttgc attgagagtc 69600
ctgacgaaat gtccatgtga cagttcattt tgggtttagc tctacctcta atatgtgacc 69660
tatgctacca gtccgtatag cgtaaattcc cagaatatat cctcctgaat aaaatggggg 69720

aaaataatac ctggcttcct taatgattat atttaagact tatcaagaga ctattttcta 69780
tttaacaatt agaaagttaa gcaatacatt atttttctct ggaatccagt gtttctttta 69840
aatacctgtt aagtttgtat gcaacatttc taaagttacc tacttgttaa ttaaaaattc 69900
aagagttttt ttttcttatt ctgaggttat ctttttacca cagttgcaca atatcctttt 69960
gaagaccata acccaccaca gctagaactt atcaaaccct tttgtgaaga tcttgaccaa 70020
tggctaagtg aagatgacaa tcatgttgca gcaattcact gtaaagctgg aaaggacgga 70080
actggtgtaa tgatatgtgc atatttatta catcggggca aatttttaaa ggcacaagag 70140
gccctagatt tctatgggga agtaaggacc agagacaaaa aggtaagtta ttttttgatg 70200
tttttccttt cctcttcctg gatctgagaa tttattggaa aacagatttt gggtttcttt 70260
ttttccttca gttttattga ggtgtaattg acaagtaaaa attatatata aatacaatgt 70320
ataatatgat gttttgatgt atgtgtatat acattgtgaa atgattacta cagtcaaact 70380
acttaacata ttcatcacct cacataatta ttattctccc cccagggtga aagcatttaa 70440
gatctacaag ctacaatttt caattataca atgttattat taactatagt cactatgctg 70500
tccagtagag cttcagatct tgttcatctt gtgttcctcc ctccccaccc tcagtccctg 70560
gaaaacaggt tttaaagata gttgctaatc cttatttctt ctaaattttt aaatcagttg 70620
ctgcctcaat ttctatatga gaaatgactg attgatttca tttttctgtt cacgctacca 70680
ttttcatatc atactagcac atgttaccca ttaactgtat tgcagatttg gtctcacaaa 70740
attcttctaa aataacattt ttaaaaagca tattaatcaa aaataagctt tatatttctg 70800
aagcttgttt gagcatagaa tgcctttgga taaaatacca ttacctagta aagtgtgaac 70860
ttttataatc cataaaaatt attcttttat aagaatattc ataaatgtag ttagattaat 70920
agaagattct cgattctttg atcagaaaac taaggactat attgaaaaat cagtgacaaa 70980
tttaattctt atagtacatc tgaaagaaaa aagaaaactc ttgggagaac ttttacagtg 71040
atttaatttt gctgttgata tatttctttg ggtggtaagt atggcaaaac atgttaaaat 71100
ttaatgcaaa gagattttgt acatttttcc atctctaaga aggacaaagc ctaagcccct 71160
ccagatagat agaaaaactc atttagagag ttctccttca tgttaatcta atttcttctt 71220
aattcagctg taaaacagaa atagaatgat cgtattaatc atttaaagct gtgtaattgc 71280
atagattcct tgttccttta ccccctctta tatcttgttt cctatccttt gtgacttttt 71340
ttgcattata tataaggatg ccgaaatact gtttattgtt gatagtttac aaaattgaat 71400
cttacattag tgcataattt tggtgaatgt tgaagattat ggtagattgc cttacatttc 71460
tgcatattgt ttgcaccttg gaatgatagc actggcatga attatagagc tgaggatcta 71520
aagattttta ctttgattta tcccattatc atctgcaggg aaacaattgc ttttactgat 71580
taaaaatgca ggctgggcac agcggctcac gcctgtaatc ccagtacttt gggaggccga 71640
ggcgggcgga tcacaaggtc aagagatcga gaccatcctg gccaaccaac atggtgaaac 71700
ctcatctcta ctaaaaatac aaaaattagc tgggtgtggt ggcgcgtgcc tgtaatccca 71760
gctactcagg atgctgaggc aggagaatcg cttgaacccg ggaggtggag gttgcagtga 71820
gccgagactg tgccactgca ctccagcctg gtgatagagg gagactccat ctcaaaaaaa 71880
aaaaaaaatg cagtagcaaa agcgatggta gaaatttaaa acagagttga tgagcagcat 71940
atattttggt agtggaaaaa aaggtaaaaa attttttgta ataaaataga aaaattttgt 72000
aatgtggagg cgcagaacac tagatttaag ccagggggtc ttaaattgtg ttacattcct 72060
tttaaagtct gatggaaggt ataaatgttc tcccctcaaa aaatgtgcat agtgtacata 72120
aaattttgca gtttttatta cattgaaata tattctttta gacagaatgt aaaagaacct 72180
tcatgaaaac tatgtcactt ttttatgcaa aaaccagtgg ctactacatg agagcaatga 72240
ataaatctaa gtggtacaaa ttaaccaaaa ttaagcttta gttctgttca atactaaatt 72300
ttaatgaaaa gactgctatt taacttttaa aataacaagt tgaaactatg ctctttgact 72360
ttgactttgc aacttttata tgatctttga tatccaatca gtgttgactt tggtaaaaag 72420
tgctgaaaat gctattttac aaaagaaaga agagtaaatg gaatctgtag attctattgc 72480
ctgatgaaag tagacgtgtc aagaaataag aattctccaa ggctcttcag ataaattcat 72540
gtttcatcat tttctttgcc ttcaagttac tgagatcatt tttggcaaga tctgtatcat 72600
taatgctgtg ttaggaaaga aaagattatg actccacatt ttactttcaa ggttgaagag 72660
ttaaactgtt taaaaagagt gtatgttatc ctgtaaacag cagtatcagg ctgtagaatt 72720
tgtcttctga aagcagggaa cttatatata gcaaagaact tcatagtgct cccatttctt 72780
gacaaaacct ctcgagaagc tcttgattga aagtcttggc tttcatgaat ctggcagctt 72840
tcacaatagt ggatttttca tgacaaatca tcttacacag ggaattattc aagggttggc 72900
acttgaaaca gtagaatact ttcacaacaa gagataagat ttctttcagg attgatgaca 72960
gtcttgcacc ctagcgcata ctgatgaaga gagcagtggg tgaccatgac atggagagct 73020
tctgtcttta ccagtgcccc aatatcagat gtgttgtctg gcagtaaggt gtactgtctg 73080
cctacagaat actgaggttt cttcaggaga agttttttgg taaagaaact ttaccatttt 73140
gaaagtgtta atgttttctg aagcttccaa aaagattcca aaatgggaat gtttccttga 73200
ttgtgtcacc atgcttgcat ttgatgaaaa cttgtagccg gctatactga gaaatcatat 73260
ctgaagaaag gtggtacttc caatcttttt gtgacctact ttattattgt ttttttaatg 73320
tcagggtttt ttttggaatg gagaaaagta tttgatagag gtattgcaac agtcttattc 73380
ttcttcatgc tacaagtata tttgactctt tctaagatac ttgccttcac tgttcaactg 73440
tgtgactttt tgtttgttta gcattacaat caatatccta gtaggatgat ttaatcaatg 73500
atttttaatt ggaacaaata gtttttgtaa tggtctaggc ttttccaact taactgtgct 73560
ctcacatgtg gtctcttttt ctccctcttt cctccttctt atacactctc acccacacac 73620

atatgcatac ataccctgtc tgatgtatct gcttcttcag aatagttggc tgtgctctgc 73680
tgatgatgag aacttgccat ttaagaagga cttgggatag tccatgtcat catgttcagg 73740
gataaaagta aaacccaagg gcatttaaac tttattgtat tttattttct gtttccagtc 73800
caaattaaat ccaagagaag gctccataat caaaaagtaa ggacatattt taaatttgcc 73860
aatgggaaga tattctagtc attacagtct ggtaatacta tcaattctgt ttctcttcag 73920
aggtgagggg agactatttg atgaaatcgt aagtcctgta gggtgttgtg aaatagggcc 73980
agaatgaaag atagcaagaa tagtgttatg aaaataaaat gcaaagttta taatatcatg 74040
tggtaaaatg taatagtatt tacttcatca gtagaactgc tctagtagct gtatattctc 74100
catccttgca taggttggaa tatcccccaa gtgaaaagag attgatgggc taatagttaa 74160
tagaaaatgg agatctgtac atacagtgtt aagaatgtag atattaaaat tgttatattt 74220
agctgttaca taatattaag actcagagtt aagtaatttc actgaaattg attgcttttt 74280
gtgtcttgga gtcaaaataa ataactgaaa tctactatac ttggctcatg cttaattaat 74340
atacttagac catatttcgg atgaattatt cacagaatct aaaggagtat cctcgtgttc 74400
ttaccttctt tatccctgtg tttatttaaa aaggcaaaaa aaatggagca gatgctgttg 74460
gttgaccata ttttactgaa cagtagcatt tgtgtttagg ttgaaacagc attagaaaac 74520
tagatacgga ttaaagtcag tggtaggttt tttttttttt ttcttccagg aatgtttctt 74580
atagatgatc aaacaggcac aggaagggga agtgttgtga tcaatattat ccagttaata 74640
ttagcattca gaggaaaatt tgagtcctct gatacactgt taaatttctt tctatactat 74700
caagtccaca aatcctggaa ctgcaaaaga attttgagac tgttcaaaat aattaatctc 74760
tgtataggct caggctttcc tgcaaggtta tgaaatgctg ataaaattgg tcttattttg 74820
aaaggctcct cagcttatac ctttccttac aaatgcttcc ttacaaatgc taaagcattt 74880
aatgactcct gacttaaagg gaatttggac agattgaggt tgttggtctt ggaaatataa 74940
tactgcaggc ttctgtaaaa tacttgaaat gtaattgttt taaaactttc aaagatacca 75000
cttgtttgcc tgttggttag aatactggtg aaataatttt taatctttta tgaataacta 75060
atttcatcat aagaaaactt agctaagcat ggtaaagctg ttgttataca actgtggaat 75120
tcttcctgag gagtaactat cttataataa atgtagttga ttatctaaag tagttttatt 75180
cttggaatat ctcataatag gtttattctc ttcttgtcag tatttccttg tagattgagc 75240
ctgtggattt gcatttttgt aattgtgaat caccattata ggagatacat gcattttatc 75300
tacttttcag tttgtatggg gttaacttta ttagaattat ctttaatgtt attttgctta 75360
tatacttaat tttaattata gacaaacatt aagaagctgg agaaaattat gttctagtga 75420
catttatata gaagaagaat cttttttccc cctttctttt ttgaagggag atgaggcagt 75480
catattttgg taaagaattt gtagactttg cagaggtctc ttcaaaataa tctggctcag 75540
agtcttgaca tatcctcagc agacatggtg caaattagat ggcagagtgg tgggtacaag 75600
ttgaccataa aataacgcat taggttagta atgcccaaat aatactttgg gttttcagtg 75660
ttgcagagaa gtcagacaac tgatagttat tataaagaaa aatgttctga gagtgaggta 75720
accgcttaag ggaaggaagc ctccttctgt cttattcact aatttacaag aagataattg 75780
tgttacactt ccttaggagt cattcatttg tatatttgac acttttgctt tatgaacatg 75840
tgaagattat tcaaaagtaa gctgttggtg atttttttct tccaagaaag catgccacag 75900
ggcaacttct agggttggtt ctcatctagt cctgtgctcc acactatctg catctgcact 75960
taagtttcaa tattagataa ctcacatgtt taaactatga agaaagagtt aaaacatcct 76020
gagaatgcta gtaagtatgt atttttgaaa ggacttccaa aatttgagtt taaagaggta 76080
aactcctttt acatgacaaa gttacttaga aacactactg ctgtttccct ctcccttgcc 76140
ttctccctgt cccatgcata cccccagctg tgttccagaa tgatggcaca taaagtaaac 76200
attcatattt atttcccttt ttttgttttt tttttttttt tttttgcttt gtttgttttg 76260
tttttttgtt tgagacagtc tcactcaatc acccaggctg gagtgcagtg gcaacatctc 76320
agctcactgc aacctctacc tcctgagttc aagcgattct cctgcctcag cctcccgagc 76380
agctgggatt acaggcgcct gcccccacgc ctggctaatt tttgtatttt tagtagagat 76440
ggggtttcgc catgttggcc agactggtct tgaactcttg acctcaagtg atccgcccac 76500
ctcggcctcc caaagtgctg agattacagg catgagtcac tgtgcctggc ctcttatttt 76560
ttctttggtt aaacttttag ggaaaaagtt tgagctgctt ttaattttct ttttgttttt 76620
aaataaatta ttaaagtttc tctatgttag gaactcttgt gtacatgagt tcattgagct 76680
tattcttaat aaagacaaat cttctagaaa taatagttgt atctttaaat gatctcaagg 76740
aaaatgtttg gtttctctgg ggaatgaatt ttcatgacct aatcttaaat caggttattt 76800
tttctagcct gtttactaaa tttctacatg ttataaccta atgaaatttt cttacttcct 76860
ctttatttaa aacaaactat aattactgtc tttttaaaaa tcttccaatg tggcgttctt 76920
atttttctta acatttgaat tttcctgggc caaaccatgt tactatgata cacattattt 76980
aaggctgtta tataatacag taaaattgta gaactttcat accttgaagg atcttagcaa 77040
ttatttaatt caaacccatt ctaacataga tgataaaaca gatttgcagg gttgggcacg 77100
gtggctcata cctatattcc cagcactttg ggagactcaa gcgggaagat tgcttgagcc 77160
caggagttca agaccagctt gggcaacata gtgagaggct gtctctacaa aaaaatattt 77220
aaaaaatagc cgggcatggt gtcacgtgcc tgtagttcca gctgcttggg aggctgaggt 77280
gggaggattg ccagagcctg ggaggttgag gctgcagtga gccatgatca caccacagca 77340
ctctagccta gagcctccct gtgtggcagg ctctacactt cagataggca acagatcgag 77400
accttgtttc acaaaacgaa cagatctgca aagatcaacc tgtcctaagt catataatct 77460
ctttgtgtaa taataaaaac cccatcttct aaccttaaac ctggtatttt tttctacgaa 77520

actatgttct gcagtccaaa ttatttttct ttattatttt gaatcctaaa gtagaaatag 77580
aaacttagaa aaataaaaag caactccttt atgacatatg aggacttttt cagttttaaa 77640
taagaaaaac ccaactcaaa gtagcttaaa taaaaggaga cattttttga cttacataac 77700
ctaaaagctc tggcctggat ctaggtgctc aacagatctc aagagtatct ctcttattct 77760
ctctccctcc cttaccctcc tcctcttttc tctcttctgt atatatgtta ttttcaaaca 77820
ggctctcaca agtagtggca agatatagtt cctaatagct tcaggtttgc attctaccta 77880
ctttagcaac tatgatgaga agagagctac aatttgagca aaaatttgta gggtgagttc 77940
tgatttccct ggattgaggc acatgcctat tattcctgaa ccaaatattc tgtccaggga 78000
atggaattct ctggggcatg tatctaaagc tgaagtgtag agcctgccac acagggataa 78060
taatagtttt ccaaagaaaa atcaaggagg ggaaagaatg ttggaaagag aaaaaaatat 78120
cagttgtctg cttcacattt gttctcaata attagtttca tagaagtgaa atactgtgta 78180
acaccctaaa ctttagagat tcttcgtaga caggaaaaat aagaactcaa tgaagttgtg 78240
acttcattca aatcacgtag tttatataca tgctattagt aaaacccagg acagctgagt 78300
acaagtttta cccttatatt cacattgagg tccagatcct ggttttgaat gagataatta 78360
cgtgcagtcg gactgttttc tgatcctaaa aatagagaca ataatatcta tcttgtaaag 78420
ttatggtagt gttaaagata tataaaatgt tggcaagtac cttaatatac aataactact 78480
gctatatgtt gtcattgtaa taataatcat atttcttcct ttgttgaatt gctttcctgt 78540
agtaatctta ttgtgatcat cctgaaacat agatttccga gcttcaagca aacactatta 78600
tgttgaaaaa tctacattat ttctaagttt agcagtgcca gtggaaagtt tattgaaata 78660
gaaaattact tttttaactg aggagtgtag attgtgaatt cgtgattcat cttcttagga 78720
gatgatcgga atattgataa atattgatgc atagaatatg aacaaaacat tacatatctt 78780
gtgctgtgat attaaagtag tattctgttc tggtagtagt atggcagtat tttaggtctg 78840
aaagatgtac ataatctgta ctttgaagtc tgttttttaa gagattaatc acaagagatt 78900
tacataaaac aactaaggtt aaaaataaat ggtggattag agatacatca ggcaaatttc 78960
aatttcaaaa gcagatgaca gaatctcagt atcagggtag catttaaagc aaaaagcatt 79020
aaactggatc aaaaatgtca ttttacatca acacagggta cactccaggt aaagacttaa 79080
cagttatgaa cgaatgtgct aaacatcaaa atgtattaag taaaagctga aagaaatgaa 79140
gaataattgg tagaaaagca atgataggaa tccttaatgt acttcaaaag gcacgaagta 79200
agaaggtaca gtcatgtacc acataatgat gtttgagtca acacctgacc tggtatacga 79260
caatggtcct ataaaattat aatggagcta aaaagcttct atcacctagt gatgttgaag 79320
ccgttgcaat gcaatgtatc actcacatgt ttgtggtaat gctggtgtaa gcaaacttac 79380
tgtactgtca gttgtataaa agcatagcac agttatgttc agcacataat actttataat 79440
gataaacgaa tatgttacta gtttacgtgt ttacagtact attattttag catgtgcttc 79500
tgcttattaa aaaatgttaa ctataaaata atctttaggc agatcctaca ggaggtattc 79560
cagataaagg cattattgtc ataggagatg gcagctccat ggatgttatt gccccttaag 79620
accttccagt gggacaagat gtataggtgg aagacagtga tattgatgat cctgaccttg 79680
tagaggccaa ggctaaagta tgtatttatt agtttttaac aagagtttaa aaagtaaaaa 79740
aaaaaaataa atttaaaaat agaaaaaatc ttaataagga tacaaagaaa aaatgttttt 79800
gtgtagctgt gtaatatgtt tttgttttaa gttaaatgtt acaaaagagt cagagttaaa 79860
tttttttata tttataaagt gaaaaagtta taaaatgcta aggttaattt actgaagaaa 79920
gaaaaaattt ttaaacagat ttagagtagc atgtttataa aatctacagt agtttacagt 79980
aatatcctag gccttcacat taactcacca ctcacccact gactcaccca gagcagcttc 80040
caatcctgta agctccattc gtggtaagtg ccctatacag gtgtacaatt tttatatcct 80100
ttataccatt tttactgaac cttttctgtc ttttagatac acaaataaca ttgtgttaca 80160
gttgcctatg atattgaata cagtaactta ctgtacaagt ttgtagcata ggagctgtat 80220
catatagcct gggtgtgtag tgaactatac catctaggct tgtgtaagta tattttatga 80280
cacagtgatg aaatcatcca gatacagttt ctcttaagca acacataact gtatatatat 80340
atatataggt ggctttaata ataaaataat gaaatatatt tctttttttc tgtcattgac 80400
aaaacaatta ccagtcttac taattagatc aactgcaaaa caaaatctca ctcaaaaata 80460
aaaatatgta agtcccattc cttgattaca attcattaga actggagatt tttaaaaatg 80520
tttaaattta tatggaaata taaaatcatg ttttaaataa tttttgattc aatgaggaaa 80580
tgtaaattta gaactaaaga ttaataacta gagatttata aaggcaaaaa tgttaatgaa 80640
ttaggaaaca gtaaaccatt tgaactaatc taaatactga ttgcatgaaa atgccaataa 80700
aatcaataca tatttttaaa agccagtcaa ggataaaaga aaagagggaa actaaaagtg 80760
agacattaag tatgagaaag gagataaaac taaagtgtgg aggtgatgtt aaaaattata 80820
ttcaggtctg tgctaatcat tttggaaatt tcagtgagaa gtgggcaatt ttcagtgaat 80880
atatcattgt aaatgctttg aggagagaca aaatctgaat agaccctgca ataatgaaaa 80940
tgtctaggag ccctcaagaa atacctggtt taaattgcat ttcaggtggc tggttatcac 81000
accttgaagt atcaagtaat tatgttatct aaattggacc aggtgttaga aaaatatgtg 81060
agacttcaca ggtggctgct tatgtagccc ttatacctta atctgataaa ggtagcatat 81120
atttaaaaag agagaaaacc agaaggtaca atttagggaa aattaaatac ttctatttgg 81180
ccaaagtaag agtacattca agggaattgt gagaggtaag agtggaaaca gaggttggag 81240
ctgtattttt atgctgtgat tgaattacag tgtgtgataa atattgctct tatttgggta 81300
gattaccatt taacattttg aattaattgg taattggatt aatcttaact tttaaaaaac 81360
taatctgaga gtggtataaa ggatagatta cagaatggat aaagggtgat aaatcagttg 81420

gctatggcaa aattgcagga agaaactgaa ataggccaca gaaaactgaa actgacattt 81480
tgagtagtat ttcagaatca agatctggat tttggcaact gaatagatgc gtaggaatca 81540
aagatgagta tactaaatga acttaatcta tgatttgtct gtcattttat tgtgtaccat 81600
tagtgtgtat gcatgtatgt atgtgccagg tagttaatag gctgactgtg tctcttagct 81660
ccactctgct gcctgggcct ttgccatagt gctggagttc cctcacttct cttttctgta 81720
accctattat attactgctg tctctcagct gtgtttcatt cctcaagcag aaagaagatg 81780
ggaggatcat atagtagttg actagaagct gtggagtttg agtgctggga ttatatctag 81840
ttccattact tatgaagatt atatctagtt ccattacttc acctcatcta taaaatggtt 81900
taccaatagt acctacctta catggtttta tgagtattaa attatgtatt tctaaagaca 81960
tttagaacaa tacaaagaat atagtgtggg ctcaataagt gacgatggtg ttagttatta 82020
gaaggccatc gagtgctgga gaaaataatt gaatatcatt gatggaaata aaggaagttt 82080
tccacgttaa aaagcttcgg tttttggaaa tgtgtgtttt cagtatttct gagattacca 82140
ggtagaaatt ccagccagat gttggaattc tgcactggca gttgggaata aagtcatgtt 82200
aaaggagata agttttggag ttattgtgta aaattggtaa agcactggaa tagattggtt 82260
tgacagaggg gaaactcttt ttgagatagg cctatattta gggacaagag aagagacaac 82320
caataagggt tgaaagaaac cttgagagag taagtcctat gacagaagca gggaagtttc 82380
agaatgattg cagaaagatc agccaggctc ttcctgttct ccatcgtggt gcaggatcaa 82440
ggtgaaaagg ataaccccat gcaggaattt tgcatctgca ggctccgctt caacatctgt 82500
ttaggagaaa gtggagacag actgacctga gtaactaagg tcttggaaca gcttacaggt 82560
cagaaccagg tgttttccaa agctagatat actgttagcg cctttggcaa cagaagaaat 82620
gaaaagactg ttgtctgctg cacagttcga ggggccaagg cagacgaaat cctggagaat 82680
gatctaaagg tgcaggagtg tgagttaaga aaaaataact tctcagatac tggaaacttt 82740
ggttttggga cccaggaaca cattgatctg ggtatcagat atgacccaag cattgatgtc 82800
tacagcctgg acttctatgt ggtgctggaa aagccaggtt tcatcattgc aggtaagaag 82860
tgcgggacag gcttcattgg tgccaaatag aatcagcaaa gaggaggcca tgcgctggtt 82920
ccagcagaag tatgatggga tcatccttcc tggcaaataa attctcattt ctacccaaaa 82980
gggtaataaa aagttttcag tgaaatgttt aaaaaaaaaa taaaaaaaag atcagccagc 83040
agccaggatg ggattgtgaa aacagcagga aattagttgt tgacaaagca ttaatgacca 83100
ttaagaaatc agcctcggct gggcatggtg gctcatccct gtaatcgtaa cactttggga 83160
ggccaaggca gatttcttga gtccaggagt tgagaccagc ctaggcaaca tggcaaaacc 83220
ctcgttcctc cttaataaat aaataaatga ataaataaac aagcaagcaa gcagggcttg 83280
gtgttaggcg cctgtactcc tagctactcg ggaggctgag ggggttgaac ctgggaggca 83340
aaggttgcag tgagccaaga ttgcactact gcactccagc ctgggtgaca gagtgagacc 83400
atggcacccc cctccccttc aaaaagaaat cagcctcata atcaatttct ctggattaag 83460
gagtaagagc gtctcaagat ttgctgttta tagagaggga ggctaatagt ttgagagaga 83520
tacagaatct agggagagtg tgggtttttg gtctttcagt tgtttgccag ccttggataa 83580
agaatgaaga ttacttgagc atattattta gagacaagtg gagagaataa aggcacatgc 83640
cagataggag ataattaata aagcacttgt ccaaaataga aacttgttga acaggaagag 83700
acgtcaagta taaggagatt ttaagatggg agaagggaat tttgagtgtt tgtattggat 83760
gacctcaggg ttcccagtaa agcaggagct gaattcatcg aaggtgatgt gttggtcagg 83820
atcaagagag aggttgggag aacaaagtgc taaaatcgtt gtggtcaaga gtttaaaaag 83880
tgtataccag aagagttatt gagtgatagg ggtttgaaat aggcaaagct gtaggaaagg 83940
gggctggaag gaatattagg aggaacacta aatatacttc tgaggtctac ctcctggtct 84000
gtgaacataa aggagctgaa agagtaatgg ctgaagttct ttagtttagc taaagttttt 84060
tagctaaagc tagaattgtt gaaagttgta tttgaggaaa aaaagttaag gatacagttg 84120
accgttcgat aatgtagccc actgttgacc agaagcccta cccacaacat aaacaggcaa 84180
taacacatat tttgtatgtg tattatatag tatattctta acaataaagt aaactagaga 84240
aaagaacatg tatcaagaaa atcataagga agagaaaaca catttacagt actgtactgt 84300
atttattggt accatacatt tatgttgctg tttacaagat gaagcatctg tctgaaatgg 84360
ccagcagcta cagctgtacc tatctactgt acatatcaag caagtcactt tattcttata 84420
atgtctatga cttctttctt tgaaagcgct tccatcatca ctgttggcac ttcatatggg 84480
tctcatggtg ttaaggttta cggcattgca ctagacacaa tgaaaactac acaagagggc 84540
cgggcacggt ggctcacgcc tgtaatccca gcactttggg aggccgaggc gggcggatca 84600
tgaggtcagg agattgagac catcctggct aacacagtga aaccctgtct ctattaaaaa 84660
taaaaaaatt agccaggcat ggtggcacgt gcctgtaatc ccagctaatc gggaggctga 84720
ggcaggagaa tcgctttttc ccagaaggcg taggttgcag tgagccgaga tcgtgccact 84780
gcactccagc ctggatgata gagggagact ctgtcgcaaa aaaaaagaaa agaaaaagaa 84840
aagaaaagaa aaacacacaa gagccgtgag agagatagct tttgattgca atacacaatt 84900
tactggagag atgagctgat catacagaga tgattagtgt cacacagtgt tttaaacaga 84960
ttcttgcaac cctggagttc actgcagtag caacagaagt tagctatgag attttaacag 85020
tagtatagta tgtactacag ttaatattag gtagctatga tttaatgctg catctttgca 85080
tttgtttaca tttatcttga ctacaagtgg tattatgtct ggtcttaagg tttgtgtgca 85140
tatgttttga tgaattttaa ctttttataa taggtttgtg tatattttat ggcagtaaat 85200
gataaaacag actaatctac atatatttta tgtagtcatg acataaacct aactttttct 85260
taactttttg atatttctag tctatgtgtt tcatctgcag gttttttcaa attgttgaaa 85320

tctctgaaaa attttattga aaaaaaatcc atatatgtaa gtggacccac acatttcaaa 85380
cctgtgttca agggtcagct gtgtaaataa ttttcctcaa aattaaagtg gaaaaggaga 85440
gttactacta gtagaaagta gaactgtacc cttgggcagg ggtgtgtgtg tgtagttaaa 85500
gatcaattta acttaaaagg tcttggttag agaataaaaa ctggccctta ttagctttaa 85560
tttacatgaa aaatgaaaaa ttttaggcca ggcacagtgg ctcaggcctg taatccctta 85620
actttgggag accaagggga gtggatcact tgaggtcagg agttcaagac agcctggcca 85680
acatgctgac tcacccttcc ctactgaaaa tacaaaagtt agccaggcgc agtggccatc 85740
cctacagtgc tagctactcg ggaggctgag gcaggagaat tgcttgaacc cgggaggcaa 85800
ggttgcggtg agctgagatc gcaccactgc actccagact gggtgacaga gcgagactcc 85860
atctcaaaaa aaaaaaaaaa aaaaaaagat ttgaaaacag agtatttttt aatctgcaag 85920
agctttacag ccttttattc atatgtataa gcttttaaag atgactaaaa ttttagtgtg 85980
gactttccac tcattggaaa tcctatattg caggtgttaa ttcaatttta gtgagtgtgc 86040
atcatggctc agagagatag gactagaatg aggaggtcac attggagact ctgaaacaga 86100
tacatgtgag cctcccaact ttttaatatt tgttaatcta gaagtgttga attttgggtg 86160
ctgacaaggc agcaggtaga taagaattgc aaagttaaga aaatagactg taatattgat 86220
ggtaaactaa ttgattaaat tttaaaatgt acttttccat gtttttcttt gaattgtcag 86280
taattttgtt tcaactggta ttcatacata gattattcac ccaatgttga caactagtag 86340
atttatatat tttttatgtt gcctatcctt ttttgggtaa ggattaacag aatgtataat 86400
cacctacatt ataggtacac tactaatcac ttgctacttg aaaaaaccta aagctttgaa 86460
atctttttat tattgcacac aaacttatgc caaaaatgga gataaagaga aaaatgtcat 86520
ccactaaacc ccaacaaata atgttgacaa tgtggtctac tcgtagactc gcattgactt 86580
aattttttta aatcttattg catattttga ctagataata aatgcatatg gttaaaaaat 86640
tcacatggtt caaaaaagta cacctcccac tcatcttcca tgtgatattt cctttctgct 86700
tagcaattct gtatttatct tgctaaacat gaatgacagt tgtttgctga aattacatta 86760
aatgtgacgt aataaaatca ttgtaagttt acatttttta actttaataa tttttaatgt 86820
tttaatgaag agtatgaaga gtagtagtac tgctcttcaa agtactacta ctttacctta 86880
ccttttactg ttttgttaag aaaattaggc cgggcgcagt ggctcacgcc tgtaatccca 86940
gcactttggg aggccgagac gggcggatca cgaggtcagg agatcgagac catcctggct 87000
aacacggtga aaccccgtct ctactaaaaa tacaaaaatt agccgggcat ggtggtgcgc 87060
gcctgtagtc ccagctacac gggaggctga ggcaggagaa tggcgtgaac ccgggaggca 87120
gagcttgcag tgagccgaga tcgcaccact gcactccagc ctgggcgaca gagcgaaact 87180
ccgtctcaaa aaaaaaaaaa aaaaaaagaa aaaagaaaat tatatagaaa taaaattcca 87240
gctattccaa aactgcacct tgaatacagg tacagaattg ctaaaaccgt gtaccatttt 87300
gtagttttag catgcttttg tgtaactgca tctggtgttt gatcctcatg agagccctgt 87360
taaggaaggg tacatattat tgtcctcatt ttccttcgaa aacacatcag agtttgtatt 87420
ttgactgtca gcattcaaat acaagtcttt tatttataaa attttggtct ttatactgtg 87480
gctaaaaatc ttaaatcact tgtcatgatt tgaaatggtt tataccgatt ttttttgaca 87540
tttatacaca catacacata tttttaaatt gtctataata aaatcatgct catctttgaa 87600
aaaatattag gagtactaca gtggatacct acatacttgc tattcagcat acctggtttt 87660
ttgtttgttt tttgagacag tcttctctgt ggtccaggtt ggagtgcagt ggcacgatct 87720
cagctcattg caacctccgt ctcccaggct caagtgattg tcctgcctca gcctcccaag 87780
tagctgggac tgcaggtaca catcaccacg cccagctaat tttttgtatt tttggtagag 87840
acggggtttc accatgttgg ccaggctggt ctccaactcc tgacctcaag tgatcagccc 87900
acctcggcct cccagagtgc tgggattaca ggttgtgagc cactgcacct ggcctgtttt 87960
taaattcaca taaatatgtt ttatattttt cattagggag aagaaggttg tgtctacaat 88020
ttttaagaca ttggggagat ttagatgcca gtagtaactt aaaagagaaa taattgcaaa 88080
ttctttttcc tcttgagtat actttcattt aaggtacagt gttctgtaag ttacttttac 88140
cgttaaactt cttaatgttg cttattgttt gtcttacatt tttaggttgg atttttctta 88200
agtcacatgt ctaataaaaa aaacccttaa atacctcatt tattcgtctt cgttagtgaa 88260
tgcattgttg tacatattag atttttctct ttagataact cagcttcccc tattaagtgc 88320
cacatgtatt acaaaatttt atttatgttt tattgtttaa taaactcttg agaactagat 88380
acattttaat catttgtaat acttacattt tctaaaacac ttcatttttc cctggtttct 88440
tcaacaaaga gatgcatgta gtacaaggat agctttacct gtgttagaag attgtttcac 88500
acatttacat caactgcata gtcctgtttt tgttgggccc taatgccagc atcacttttt 88560
gctactgctg tttctgcctt aaaggcaata tgcctctgtc tagtttgctg attctgatac 88620
tctttcccct ggaaagtagg taatcaagtt tgtgaggagc tgtgtgttta aggagtccat 88680
aaatccttgt ggggagccct aggtgtatag agcatagctg tagggcagag gcctttgaca 88740
cttattctgg atatgcagtg gcctttgcct atggggttca tgggtcagag cgctgttgtg 88800
acctttgaat aaatgggttg ttatgataat tgttttaagg gaggagagtt attctgatat 88860
cctttgtatt gatattgctc ttatttatta ttgagctgga tttaagtatt aatcatttaa 88920
ggtcaaattt ctaatgtata tatgttctta aatggctacg acccagttac catagcaatt 88980
tagtgaaata actataatgg aacatttttt ttcaatttgg cttctctttt ttttctgtcc 89040
accagggagt aactattccc agtcagaggc gctatgtgta ttattatagc tacctgttaa 89100
agaatcatct ggattataga ccagtggcac tgttgtttca caagatgatg tttgaaacta 89160
ttccaatgtt cagtggcgga acttgcagta agtgcttgaa attctcatcc ttccatgtat 89220

tggaacagtt ttcttaacca tatctagaag tttacataaa aatttagaaa gaaatttacc 89280
acatttgaaa tttatgcagg agactatatt tctgaagcat ttgaacaaat taattagctt 89340
tgttgttcaa ctcattgggc taaagaagcc aaaagcaatg ggttttaatg tagtcgaagc 89400
caaattatat ttatgaaaga aatattctgt gttataacca ccaaatacag cccaattctg 89460
actagatgat ggaagaacct gtcccatcag aggtccagca tgaggtccag cagaggtcca 89520
ccagaggagt tcagcaattt gctgctctta gggcagggat caattcctta atatcttagg 89580
aagactaggt attgacagta atggtgacaa agcaatgaaa aggaaaggaa gaagtgataa 89640
gacgtggcag caagctgaag tatgatgagt aaagaatagg aatcaaagta tgtggagtgt 89700
tagagaaaac ctggatttag atccagattc tagtcctatc tctgtcatta atctattgcg 89760
taaccctgag catatcatct acctctcttt gagtttgctt gtcaataaaa tgaagagact 89820
ttgaaatctg agacttcctg gataagtact aaatacagat tatgtcactg atgtctgcct 89880
ctatttattt ctccctttta ccctaatctc tataagtcta cctcagtcat cctgatccta 89940
ttctacttct ctgatgttgt tgtcagatag gtgtgatcat cctcatcaga tcttttctgt 90000
attcttagag acagataact ttatcaaaga ccacagattt attagtatag catgttaaag 90060
tcttctaaag agtctcattg atgctctttt catctcagta caatttttaa aactgctgaa 90120
tgcaaggtac tgagctgttg gaagtgactg acagatgaat gtaacagatt catagagaag 90180
gaaaaaggaa gaaaaactca tgctcttcct atagtattga tatcagtgta agagccaaga 90240
gaaaggtata aagtatcatg cagatattaa gggaaagaaa acattcactt tagtaatctt 90300
tcctcatttt ctagtttcct cttatgtact atgatttaat actgtagtaa agttttaata 90360
aaatatgagc tatatgtaat taagtgggag gttgtggggc taggcacgag gctcacacgt 90420
gtaaccccag cactttggga ggctgaggca ggcggatcgc ttgatctcag gagttcgaga 90480
ccagcctgga caacaaggtg aaaccccatc tctactaaaa acacaaaaat tagctgggca 90540
tagtggcaca cacctgtagt cccagcttct tgggaggctg aggcaggaga atcgcttgaa 90600
tccaggaggc agaggttgca gttagccgag atcatgccac tgcactgcag cctggacatc 90660
ggagcaagac tttgtcttag aaataaataa ataaatataa aataaaataa atgggaagtt 90720
gtgtatataa attataaatg ctacattcag aaaagctttt gaaggttgtc agacagtttc 90780
ttaaaggaag ttcaccagtt ctttattgaa cattgaagaa aacatacagt ttagactggc 90840
attaaaactg aaagaagtgg ccagacgcag tggtagacgc agtggttcac gcctgtaatc 90900
ccagcacttt gggaggtcaa ggtggatgga tcacctgagg tcaggagttt gagatcaggc 90960
tggccgacat ggtgaaaccc tgtctctact aaaaatacaa aaattagcca ggcatggtga 91020
tgcgtgcctg tagtcccagc tacttgggag gctgaggcag gagaattgct tgaagccgaa 91080
ggtggaggtt gcagtgagcc gagattgcgt cattgcactc cagccagggc ggtaagagtg 91140
aggctccgtc ttaaaaaaaa aataagtaaa taaattaaaa actactgaaa gaagtattac 91200
aggcaatggg aaatagcttg agtggaagtg cagcagaagg aaaaagctgg acaagaatgt 91260
agtgtcagag aataggtatg gaacgtgtga gtgactgtta gaggatcttg aatggggata 91320
acagacttga tttcataaat actgagatgt catgataata cttgaggact aaaccatgtt 91380
ttaaggacag ttgtatgcaa agttgtaatc gcaagaggaa aaaatagtgg aaaagaaacc 91440
agtaataaaa cttgccttaa tgcaggtatg ctaagacaat caaatgggat ttcattaatt 91500
ttttatttgc catttatagc caaagatttt gtaaaagttt tgagcccagt caggtgaaat 91560
agtctcagaa agaaagaaaa gtgaatctga gacttggaga cattaatgtt gatattttgg 91620
ttttaaacgt gttttaaatc cggtaaaagt gagcttctca catgacaata ttcagtgggt 91680
acttgggagt atgggttcga atctaggtag gagatatatc gatattttgg gcatcatcag 91740
gaaagggaga gtagttaagc ctttcatata aataatggtg tggcgtttgg gcatgggaag 91800
tcttggagga aaggaagaaa aggagagggt gaggactgag ataagaatgg caacttgggt 91860
ttaggaagaa gaagaggaat caatgtagag aacagatagt gctgaaaaat acagcatctt 91920
ctgtagggat tggcagcttt ttcttgattt ttgtcttaat atttctaaga gatggaaaaa 91980
gctactatat tctagacatt taacagggtt aaaaatgtta ctaaaagatg atcaatgtgg 92040
ttttcattca agactataac aatatgtata tatccaagga aatttaattc tgacttaaaa 92100
aaattgtttt gcttgtatag atttagggac acaagtgtaa ttttgttaca tgcatagagt 92160
gtatagtttc aagtcagggc ttttaggttg tccatcatct gaataatata cattgtaccc 92220
attaagtaat ttctcatctt ctactcaccg tttcaagtct ccactattta tcattccatt 92280
ctttacattc tgattttcat ttactaggtg tattagtctg tttttgcatt gctttaaaga 92340
aatacctgag actgagtatt aaacaggttt aacctgtttt ctttatgaat tcttctttaa 92400
ttggctcatg gttctgcacg ctgtacagaa agcatagcag catctgcttc tggggaggcc 92460
tcaggaagcc tccaatcatg gctgaaggca aaaggggagc atggtgagaa tgggagcaag 92520
aaagagaagg agtggtgggg agaaggtgcc acccactttt aaatgccagc tcacttacca 92580
ccaagaggat ggcccaagcc attcatgtgg gatctgcccc catgattcaa gcttcttcca 92640
ccaggcccca cctctagcac tggggattac aattcaacct gagatttggg agggaaagat 92700
atccaaacta tatcactagg tctggatctt gttatttatt ttttggaaca tagtcatata 92760
tatccaagga tatatattgt agaagtccac agaaccatac taatattgga cttctgctta 92820
gttaggtctt atctatctga aacatgatat tcatattgca gagaagatta ttttctttag 92880
tgattgagga aatctttact acttatacat ttttaatata atactataat atttgaagat 92940
gcacatttta gatgtagttt aattgaaacc tggaaatact attaatttgc tttttaaagt 93000
cctaaaatca ggattatcag attctgaatt aatggagttt aaatcaaaaa gattacaagg 93060
cagtttttca gttttattct ggttaatttt atcacagctt tggaatccta ctttgtttat 93120

ttgcttcttg aagttagatt tcccagtgaa atttcagtat cacataaagt cttatgaaat 93180
ggctcattgc actttgaact ttgagtcaag gaagtgaaat ttattgatag attgttggtg 93240
taatatttat cctgtttgtg gtagcttttt tgaataataa gtgtcttaga agaccatgtt 93300
ggagtagcct gcatgctttt atcaaacata ttaattatgt gatggctgat actgctttag 93360
atattacata gaaatagtag taggtgttta ctaaactgga aatttcattt aacttggttt 93420
agctttgcct tgttctcagt cacattgata aaaatgtaag actttgtttt atcttttaga 93480
ataatgacac cttttggtgc tgagaatttt ttgttttata tatatatata tatatacgta 93540
atataaatac aaaatatatt taaatatgta taatatttct catacacttt atgtaacttt 93600
gtgttcctgt ttctctatta tcttggcatg ttttcttcaa atggcacttc ttaacctcct 93660
aaggttaata aatttctttg taatggactt ttgttttcta attcctcagc gtatgacaaa 93720
tgaattatac tttgtcaaat tatttaggta actttcagtt tttgaagtcc tgggatcata 93780
acattcatca gtctttaatt tctgtcatta aggtcattag ctataaatga atttatgagt 93840
agatttaaaa aataaaacat acaatccttc ccttaacaca ctttcccacc atttggttca 93900
actgctagtg taaaagcatg atgaattttg agaagttata ttttaccagt tactttattt 93960
tttaccagtt atttaaaaca gacatgagcc aaagccagaa tacttgttaa tgaaaatgag 94020
gtgttttgga ggaaaggaag gttgtgctgc agtttttact tgaaatctgt tacatttctt 94080
tacagaaatt tcaaatctct tgtttcctgt tatgatggtg gcattatata cctttaaaat 94140
gtgagctata ggaaaatgaa tgatggttaa ttttttaata aatatttaga cttgtgtttt 94200
tgaaattttt tataacattg ttataggttt tatcctcttt ctcttgtgaa catgtagtga 94260
tttgtatttt gtgatctttg ccgcatgcta gagacttaag aatactatag caaatatctg 94320
tcttctttac atttaaaaat ttttcgtgac tactccctgt tgatatctgt cttaaaagtt 94380
acttttgatg tagttcacaa atgtaccaga taattatttc atcgttttta atgcttaaag 94440
tttttatttg tattaggatt tttagtatga ttttaatgtt aaagttttga agttactctg 94500
ccactagaag tctaattttg ggacttacta ttcatgaaat aggaattgac ttttatataa 94560
gtaataggac cttattttga aggttcaaac tggagaaaat cttacattgt ttatattttt 94620
atttcattta tttcagttga tttgcttgag atcaagattg cagatacaga atccatattt 94680
cgtgtatatt gctgatatta atcattaaaa tcgtttttga cagtttgaca gttaaaggca 94740
tttcctgtga aataatactg gtatgtattt aaccatgcag atcctcagtt tgtggtctgc 94800
cagctaaagg tgaagatata ttcctccaat tcaggaccca cacgacggga agacaagttc 94860
atgtactttg agttccctca gccgttacct gtgtgtggtg atatcaaagt agagttcttc 94920
cacaaacaga acaagatgct aaaaaaggtt tgtactttac tttcattggg agaaatatcc 94980
aaaataagga cagattaaaa gctatatttt attttatgac atgtaaggaa ctataatttg 95040
ttttctatta gatctgcagg tgttttgctt actctggcat tggtgagaca ttataagggt 95100
aaataatcct gtttgaagga aaaggcctta tggcattgta acatgagagg aatttttctt 95160
aacaaggatg gttaactgag aagaaattag catgggacca atattttaaa aattttggtc 95220
tataggtaga aatgagatct gttctgtggt cttatgtagt gacacaaacc actttttctc 95280
cattttggct tatgtttctt tttctttcct tttttttttt tttccttttt gttagagaca 95340
gggtcttgtt ctattgccca ggctgagtag ctaagactac aagcatgtgc caccacaccc 95400
agctaatttt ttttattttt atttttgtag ggacagggtc tcactatgtt gcccaggctg 95460
gtctcaaact cctgggcaca agcagtcctc acgctttggc atcccaaaga gttggaatta 95520
caggtgtgcg ccatcatgcc tggccttaac gtttcttaag acttgattat tttctattta 95580
gcttctgtgg atttactgat taatttttta actaggagag aaatcagtat gaagaggaag 95640
taataaagaa tgaaaacatg gtatttaaat gtgcaggttt agaaagttaa tgaagtttga 95700
atttgattga tctgtattta gagaaggcaa cgtcttatta ttttaaaacc aactatccgc 95760
cctgtgcggt ggctcacgcc tgtaattcca gcactttggg aggctgaggt gggcagatca 95820
gctgaggtca ggagttcgag accagcctgg ccaacatggt taaaccccat ctctactaaa 95880
aatacaaaaa aattagccgg gtgtggtggc aggcgcctgt tttcccagct actcaggagg 95940
cttgaggcag gataattgct gaacccgaga ggcggaggtt gcagtaagcc aagaatgcac 96000
cattgtactc cagcctgggc aacaagagtg aaactccatc tcaaaaaaaa agaaaaaaaa 96060
aacaacaact atcttcattt aaaatattaa atgtgaatat ttaaagtgag actaaggtgc 96120
aacattttta gatagtaatg aagaaaagga ctaactttgt agtgttgctg ccttgttaaa 96180
catactagat agcatattgc caatctttaa acattctcaa tgataggatt tatttacttt 96240
ttctgatttt tagcttttct tttgaaagaa aataagagga agtttcattt actgcaaaat 96300
tttaaatgct gctttgatgt atcagtagag atataatttt cctttatcca gaatccaagt 96360
agctggaaaa aaaaatcaaa atatgctgaa cttttttttt tttagccaga aacccatttc 96420
ctatcgtctg tacaaataaa agttaaatat atctcaataa cttagaaaaa ttattttttg 96480
ataatccagg aagtattagc aactgtttta aaattaagat aactagtaag ttttatttag 96540
ctttcaaaaa taggcatcta catcatcatc tctgcatacc tttaggaatt tcctaattct 96600
tatttccctt catctgtact ttaacacatg caaaattgaa ggttagatta aatatttatg 96660
atttatttgt ttatccttga ctacataaat ttccatttta ttgattttcc ctgccttatt 96720
taagaatatg ctatgattaa aacacaaaaa attttagtat aacccatata tatatagaat 96780
tcaccttttt gttatttaaa tattattggc ttattttctt ctaagtaaaa tacaattact 96840
ggctaaaata attgaaataa gcaaaaaaaa aattttaaag accttgtata caagattact 96900
ttgccaggta ctgttaaaag atgcaatgac atttaagacg taacatcctt aaggatctta 96960
ttttctgggg gataaaaaac tttaagataa attagaataa aagatttaaa tggcatttta 97020

aggtaccagg taccagataa gatgtcacaa ggctgtatat cattaattgc caaatgattt 97080
atacaggcca gatttctttg ttggtcaata gaggtttaaa gtgatgaact tctgttgtgt 97140
ttttttatta agaaggtatt atcttattag taagaagtga ttttttttaa gaacaagcat 97200
tttataacat caaaagaaat cagtagtact ctttcctacc ccctcatatt tattctgaaa 97260
gtattcaagc attatattgt catgtaagaa actggagctt ctcatgtttg tattgctgta 97320
gaagtaaaca tgtatttgcc atgcgtcatc agggaagttg cactcaccgt ccaagaactt 97380
ttgttaaagt aaatcttgga ataggtagct catttgaaat gtagaaaaaa ttaaatccat 97440
atctgaattt tgtttatatg tatgtacacg taaactaaaa acgtatttaa agctagtatt 97500
agatgagaaa agaggttttt ttacttaaaa ttttaaggca aaagtagttt atcttagatc 97560
ttgtgagatt gtatttttgg tttaaaattt gagaatttga gtgaagaaaa atcatgtgaa 97620
tgaaaatgca acagataact cagattgcct tataatagtc tttgtgttta cctttattca 97680
gaatatcaaa tgatagttta ttttgttgac tttttgcaaa tgtttaacat aggtgacaga 97740
ttttcttttt taaaaaaata aaacatcatt aattaaatat gtcatttcat ttctttttct 97800
tttctttttt ttttttttta ggacaaaatg tttcactttt gggtaaatac attcttcata 97860
ccaggaccag aggaaacctc agaaaaagta gaaaatggaa gtctatgtga tcaagaaatc 97920
gatagcattt gcagtataga gcgtgcagat aatgacaagg aatatctagt acttacttta 97980
acaaaaaatg atcttgacaa agcaaataaa gacaaagcca accgatactt ttctccaaat 98040
tttaaggtca gttaaattaa acattttgtg ggggttgttg acttgtatgt atgtgatgtg 98100
tgtttaattc taggagtaca gctgatgaag aacttgcttg acaagttttt aacttatgta 98160
ttatttcgaa gcagtgttta cgtagcagta acatgaaagt ttctaataaa atacccaatg 98220
tacacagcgt caaaaaagct gcatttttcc ttttcctaat tcttcgttgt ttgctgaaat 98280
ctggggcaaa ggtgcgggag ggggctaaat gactgggata tgaagtagga atgggagagg 98340
aaagaaatag atgggaactc agtcatttgg gaatgattca tatggaatgt ttttactgct 98400
tccactcctg tctgccttcc aatttattct caatccctca gagtgatctt aaaaatagac 98460
ttgattgtgt cacttctgtt tacactttat aaggaccttg tgtttttttt tttaccatga 98520
cctacaaggc ccagcataat ttagcacagg gctacctcct acatcagcac tagtcacctt 98580
ctctccttgt ttcttgagat tcagtcatac tggtctttct tcagttcttc aaaatgctaa 98640
gcttctgcct cttctagtct ttccagttat tttccttctc cctgtacctt ttcatctcag 98700
ccttttcccc tgaccttcca tagctatctt catatttcca gccttagctt caatctcata 98760
ttctctgaag tcctttgatt gtcctcccgt tattcttttt ttaaaaatcc tatttcctta 98820
tattgtatct tagaattatt tggtttgttt catttttgcc tatgtgtgat atatgtattt 98880
ctacataggt atatatatct acttatagac aagaattctt cagattaaaa aaatctgatt 98940
tgtaaacatt cccaagtggt tgtttaccat ttttttcttc ccccttccta tttcttattc 99000
tacctgattt tcccctgttc attcaccaca ctcgtttctt tctctttttt actctctctt 99060
aatttttcat tcaattttta taacatgtaa taaatctaac tgtagcgtct gagtattaag 99120
aatattgcta gtaatacttc acctgtaatc ccagcacttt gggaggctaa ggcaggcgga 99180
tcacttgagg cccaggagtt taagaccagc cggccaatat ggcgaaaccc tatctgcact 99240
acaaatacaa aaattagctg ggcatggtgt cgcacacctg taatcccagc tacttgggag 99300
gctgaggcac aagaattgct tgagcctgtg agatggaggt tgcagtgagc cgagatcaca 99360
ccagtgcacg tgcacttcag cctgggcaac agagcaagac tctgtcttaa aaaaaaaaaa 99420
aaaaaaaaaa tatatacaca cacacacaca cacacacaca cacacacaca ctattactac 99480
caatatacat acatatatgt atgtatgtat gtatgtatat tggtagtaat agtaatactt 99540
gggcccctgc acgttttaag tgaaaataga tctaatatta aatgtcttta gcccttaaat 99600
tttttttaag tgttcagaag tttcccttta aaaaaatttt taatatataa taattgtaca 99660
tatttatggg atacagagtg atattttcat gtatgcagtg tgtgatgatc aaatcaggat 99720
aattagcata tggatcacct caaacatttg tcatttcttt gtgttaggaa cattcaaaat 99780
tctgtcttct agctatttga aaatatacag taaattattg ttgactagtt acagttctat 99840
agaacactat aatttattcc tcctgtgtgt aattttttat cttttaacca acatctccct 99900
atcctcccct cccactccct ttcccggcct ctaataacca cactcttatg agctcaactt 99960
ttttagcttc catatatgag tgagaacata cggtatttat ctttctgtac ctgacttatt 100020
ttacttaaca tcatgtcctc cgggctagac attctcttta gaatccacag gtttcctttc 100080
ttttctctaa atctgcattt tgctcagcca ttaactttta aaatgtcttt ttccctttag 100140
ttttattgtt ttctatttta atattgcaag atgttttata tttgtgatta caaataaaaa 100200
ctccattatt agtaaacaaa tacaatgtca tatagtagta agtgctataa aaaatagaca 100260
ggatagaaag taatcttggt ttgtatgttt tttgtttttt agcaaagatg attagagaag 100320
gcccaaccaa gcagataaca tttaagcaga ggcctaaatc atataagtga gttatacaaa 100380
tatctgggaa aagagttaag agtacagatg caaaagccct tagacaagag aatgagcttg 100440
gtatatctga agagtggata agtcattttg actgaaacag agtggacaag aaaaccagtc 100500
caagtgtaaa gacactagtg tgtgttcagc ataggaagga tgtaatctga attttgtgtt 100560
taatattccc tgtgttcatg ctttcaaaat acagatgagt gaggaaagta gggagaaggg 100620
gtaataaagg aagctgagag atcagttaag aggtacttga atagtttagt aaagatgaga 100680
gaagatgttt gcttcttgtt gcccctcact gcttagaata gtggcagtga agggtaacaa 100740
gaagctgtca gattaactta aagagtttac tgatgcagtg gatgttggtt gtaagagaag 100800
aattgataat gactcttgga taatagggga gggaggggct gtcaatataa tataatgaag 100860
aagggatttg aagtcatttc tgatttaaat ctcacatcca ctacctactt ttaatagata 100920

tgtagccttt aacaagttcc ctaacctttc tgggccttag ctacctcccc ttggaaatgg 100980
aaatacctaa catgtaaggt tgttttgaca gttattttca ctaggcatgt aaaggcactt 101040
gactctctgt tatagaccac tgtattatgt taatgtccct ctccttcctc cctttaggta 101100
aagtttttag ggctaataaa tcccaaatat caatgttgat cagtagtttg tgtttgtgta 101160
gtgttgttta tatcaaaaac tacattgaag ccgggcacag tggttcacgc ctaaaatcgc 101220
aacactttgg gaggccaagg tgggcctccc accttgaact aaggagtttg agaccagcct 101280
gggcaacatg gtgaaatccc atctctacaa aaaatataaa agctagctgg gtgtggtggc 101340
atgcacctgt agtcctagct acttgggagg ctgaggttga tcctgggagt ttgagcctgc 101400
agtgagctgt gaagatgcca ctgcactcta gtctgggtga cagagcaaga ccctgtctca 101460
aaaacacaca cacacacaca cacacacaca aagaaataca ttgatttttc acataggtag 101520
taagagaaac attctttttg aactcagctg tttgtgaatt gaattttgta attcaaatgc 101580
tatattatgt aaactattga tgactttcaa tctgcattta ttttgtataa ttatttagtt 101640
aatatttgcc acttatattc cttaaaaaat aaaattgagg ttgggcgtgg tggctcacac 101700
ttgtaatccc agcactttgg gaggctgagg caggcagatt gcctgagctc aggagtttga 101760
gatcagcctg ggcaacatca tgaaccccat ttctactaaa atacaaaaaa ttatctgggc 101820
atggtggtgt acacctgtag ccctagctgt ttgggaggct aaggcacgag aattgcttga 101880
acccgggagg cagaggttgc agtgagccaa gatcatgcca ctgcactcca gcttggcaac 101940
agagcaagac tcttgtctcc agaaataaaa ataaataaat tgtattaaca tcctgatagt 102000
ttatctgttt agtacctagc aagaaagaaa atgttgaaca tcttaagaag agggtcattt 102060
aaaaggcctc ttaaagatca tgtttgttac agtgcttaaa aattaatatg ttcatctgca 102120
aaatggaata aaaaatctgt taaaaatata tttcactaaa tagtttaaga tgagtcatat 102180
ttgtgggttt tcattttaaa ttttctttct ctaggtgaag ctgtacttca caaaaacagt 102240
agaggagccg tcaaatccag aggctagcag ttcaacttct gtaacaccag atgttagtga 102300
caatgaacct gatcattata gatattctga caccactgac tctgatccag agaatgaacc 102360
ttttgatgaa gatcagcata cacaaattac aaaagtctga attttttttt atcaagaggg 102420
ataaaacacc atgaaaataa acttgaataa actgaaaatg gacctttttt tttttaatgg 102480
caataggaca ttgtgtcaga ttaccagtta taggaacaat tctcttttcc tgaccaatct 102540
tgttttaccc tatacatcca cagggttttg acacttgttg tccagttgaa aaaaggttgt 102600
gtagctgtgt catgtatata cctttttgtg tcaaaaggac atttaaaatt caattaggat 102660
taataaagat ggcactttcc cgttttattc cagttttata aaaagtggag acagactgat 102720
gtgtatacgt aggaattttt tccttttgtg ttctgtcacc aactgaagtg gctaaagagc 102780
tttgtgatat actggttcac atcctacccc tttgcacttg tggcaacaga taagtttgca 102840
gttggctaag agaggtttcc gaagggtttt gctacattct aatgcatgta ttcgggttag 102900
gggaatggag ggaatgctca gaaaggaaat aattttatgc tggactctgg accatatacc 102960
atctccagct atttacacac acctttcttt agcatgctac agttattaat ctggacattc 103020
gaggaattgg ccgctgtcac tgcttgttgt ttgcgcattt ttttttaaag catattggtg 103080
ctagaaaagg cagctaaagg aagtgaatct gtattggggt acaggaatga accttctgca 103140
acatcttaag atccacaaat gaagggatat aaaaataatg tcataggtaa gaaacacagc 103200
aacaatgact taaccatata aatgtggagg ctatcaacaa agaatgggct tgaaacatta 103260
taaaaattga caatgattta ttaaatatgt tttctcaatt gtaacgactt ctccatctcc 103320
tgtgtaatca aggccagtgc taaaattcag atgctgttag tacctacatc agtcaacaac 103380
ttacacttat tttactagtt ttcaatcata atacctgctg tggatgcttc atgtgctgcc 103440
tgcaagcttc ttttttctca ttaaatataa aatattttgt aatgctgcac agaaatttcc 103500
aatttgagat tctacagtaa gcgttttttt tctttgaaga tttatgatgc acttattcaa 103560
tagctgtcag ccgttccacc cttttgacct tacacattct attacaatga attttgcagt 103620
tttgcacatt ttttaaatgt cattaactgt tagggaattt tacttgaata ctgaatacat 103680
ataatgttta tattaaaaag gacatttgtg ttaaaaagga aattagagtt gcagtaaact 103740
ttcaatgctg cacacaaaaa aaagacattt gatttttcag tagaaattgt cctacatgtg 103800
ctttattgat ttgctattga aagaataggg tttttttttt tttttttttt ttttttttta 103860
aatgtgcagt gttgaatcat ttcttc
103886<210> 16
<211> 16
<212> DNA
<213> Artificial Sequence

<220>
<223> Synthetic Oligonucleotide

<400> 16
ccggaggtgc ttgaat 16

<210> 17
<211> 14
<212> DNA
<213> Artificial Sequence

<220>
<223> Synthetic Oligonucleotide

<400> 17
cggaggtgct tgaa                                                          14

<210> 18
<211> 16
<212> DNA
<213> Artificial Sequence

<220>
<223> Synthetic Oligonucleotide

<400> 18
gaagccatac acctct                                                        16

<210> 19
<211> 14
<212> DNA
<213> Artificial Sequence

<220>
<223> Synthetic Oligonucleotide

<400> 19
aagccataca cctc                                                          14

<210> 20
<211> 16
<212> DNA
<213> Artificial Sequence

<220>
<223> Synthetic Oligonucleotide

<400> 20
gttgaagcca tacacc                                                        16

<210> 21
<211> 14
<212> DNA
<213> Artificial Sequence

<220>
<223> Synthetic Oligonucleotide

<400> 21
ttgaagccat acac                                                          14

<210> 22
<211> 16
<212> DNA
<213> Artificial Sequence

<220>
<223> Synthetic Oligonucleotide

<400> 22
cctcagggtt gaagcc                                                        16

<210> 23
<211> 14
<212> DNA

<213> Artificial Sequence

<220>
<223> Synthetic Oligonucleotide

<400> 23
ctcagggttg aagc                                                         14

<210> 24
<211> 16
<212> DNA
<213> Artificial Sequence

<220>
<223> Synthetic Oligonucleotide

<400> 24
tttgccctca gggttg                                                       16

<210> 25
<211> 14
<212> DNA
<213> Artificial Sequence

<220>
<223> Synthetic Oligonucleotide

<400> 25
ttgccctcag ggtt                                                         14

<210> 26
<211> 16
<212> DNA
<213> Artificial Sequence

<220>
<223> Synthetic Oligonucleotide

<400> 26
agttcttggt tttctt                                                       16

<210> 27
<211> 14
<212> DNA
<213> Artificial Sequence

<220>
<223> Synthetic Oligonucleotide

<400> 27
gttcttggtt ttct                                                         14

<210> 28
<211> 16
<212> DNA
<213> Artificial Sequence

<220>
<223> Synthetic Oligonucleotide

<400> 28
cctcttgatg ttcagg                                                       16

<210> 29
<211> 14

<212> DNA
<213> Artificial Sequence

<220>
<223> Synthetic Oligonucleotide

<400> 29
ctcttgatgt tcag                                                         14

<210> 30
<211> 16
<212> DNA
<213> Artificial Sequence

<220>
<223> Synthetic Oligonucleotide

<400> 30
atgcccctct tgatgt                                                       16

<210> 31
<211> 14
<212> DNA
<213> Artificial Sequence

<220>
<223> Synthetic Oligonucleotide

<400> 31
tgcccctctt gatg                                                         14

<210> 32
<211> 14
<212> DNA
<213> Artificial Sequence

<220>
<223> Synthetic Oligonucleotide

<400> 32
tgccacattg ccct                                                         14

<210> 33
<211> 14
<212> DNA
<213> Artificial Sequence

<220>
<223> Synthetic Oligonucleotide

<400> 33
ttgccacatt gccc                                                         14

<210> 34
<211> 14
<212> DNA
<213> Artificial Sequence

<220>
<223> Synthetic Oligonucleotide

<400> 34
gttgccacat tgcc                                                         14

<210> 35

<211> 14
<212> DNA
<213> Artificial Sequence

<220>
<223> Synthetic Oligonucleotide

<400> 35
tgttgccaca ttgc                                                         14

<210> 36
<211> 14
<212> DNA
<213> Artificial Sequence

<220>
<223> Synthetic Oligonucleotide

<400> 36
ctgttgccac attg                                                         14

<210> 37
<211> 14
<212> DNA
<213> Artificial Sequence

<220>
<223> Synthetic Oligonucleotide

<400> 37
tctgttgcca catt                                                         14

<210> 38
<211> 14
<212> DNA
<213> Artificial Sequence

<220>
<223> Synthetic Oligonucleotide

<400> 38
ttctgttgcc acat                                                         14

<210> 39
<211> 14
<212> DNA
<213> Artificial Sequence

<220>
<223> Synthetic Oligonucleotide

<400> 39
tttctgttgc caca                                                         14

<210> 40
<211> 14
<212> DNA
<213> Artificial Sequence

<220>
<223> Synthetic Oligonucleotide

<400> 40
atttctgttg ccac                                                         14

<210> 41
<211> 16
<212> DNA
<213> Artificial Sequence

<220>
<223> Synthetic Oligonucleotide

<400> 41
gtaggagaaa ggcagg                                                    16

<210> 42
<211> 14
<212> DNA
<213> Artificial Sequence

<220>
<223> Synthetic Oligonucleotide

<400> 42
taggagaaag gcag                                                      14

<210> 43
<211> 16
<212> DNA
<213> Artificial Sequence

<220>
<223> Synthetic Oligonucleotide

<400> 43
ggcttgtaaa gtgatg                                                    16

<210> 44
<211> 14
<212> DNA
<213> Artificial Sequence

<220>
<223> Synthetic Oligonucleotide

<400> 44
gcttgtaaag tgat                                                      14

<210> 45
<211> 16
<212> DNA
<213> Artificial Sequence

<220>
<223> Synthetic Oligonucleotide

<400> 45
ccactggagg atgtga                                                    16

<210> 46
<211> 14
<212> DNA
<213> Artificial Sequence

<220>
<223> Synthetic Oligonucleotide

<400> 46
cactggagga tgtg                                                      14

<210> 47
<211> 16
<212> DNA
<213> Artificial Sequence

<220>
<223> Synthetic Oligonucleotide

<400> 47
tttcagcatg ctttct                                                        16

<210> 48
<211> 14
<212> DNA
<213> Artificial Sequence

<220>
<223> Synthetic Oligonucleotide

<400> 48
ttcagcatgc tttc                                                          14

<210> 49
<211> 16
<212> DNA
<213> Artificial Sequence

<220>
<223> Synthetic Oligonucleotide

<400> 49
catatttgtc acaaac                                                        16

<210> 50
<211> 14
<212> DNA
<213> Artificial Sequence

<220>
<223> Synthetic Oligonucleotide

<400> 50
atatttgtca caaa                                                          14

<210> 51
<211> 16
<212> DNA
<213> Artificial Sequence

<220>
<223> Synthetic Oligonucleotide

<400> 51
atgcccatat ttgtca                                                        16

<210> 52
<211> 14
<212> DNA
<213> Artificial Sequence

<220>
<223> Synthetic Oligonucleotide

<400> 52

tgcccatatt tgtc                                                          14

<210> 53
<211> 16
<212> DNA
<213> Artificial Sequence

<220>
<223> Synthetic Oligonucleotide

<400> 53
ttttggtggt agagac                                                        16

<210> 54
<211> 14
<212> DNA
<213> Artificial Sequence

<220>
<223> Synthetic Oligonucleotide

<400> 54
tttggtggta gaga                                                          14

<210> 55
<211> 14
<212> DNA
<213> Artificial Sequence

<220>
<223> Synthetic Oligonucleotide

<400> 55
tctgcttcgc acct                                                          14

<210> 56
<211> 14
<212> DNA
<213> Artificial Sequence

<220>
<223> Synthetic Oligonucleotide

<400> 56
gtctgcttcg cacc                                                          14

<210> 57
<211> 14
<212> DNA
<213> Artificial Sequence

<220>
<223> Synthetic Oligonucleotide

<400> 57
agtctgcttc gcac                                                          14

<210> 58
<211> 14
<212> DNA
<213> Artificial Sequence

<220>
<223> Synthetic Oligonucleotide

<400> 58
cagtctgctt cgca                                                         14

<210> 59
<211> 14
<212> DNA
<213> Artificial Sequence

<220>
<223> Synthetic Oligonucleotide

<400> 59
tcagtctgct tcgc                                                         14

<210> 60
<211> 14
<212> DNA
<213> Artificial Sequence

<220>
<223> Synthetic Oligonucleotide

<400> 60
ctcagtctgc ttcg                                                         14

<210> 61
<211> 14
<212> DNA
<213> Artificial Sequence

<220>
<223> Synthetic Oligonucleotide

<400> 61
cctcagtctg cttc                                                         14

<210> 62
<211> 14
<212> DNA
<213> Artificial Sequence

<220>
<223> Synthetic Oligonucleotide

<400> 62
gcctcagtct gctt                                                         14

<210> 63
<211> 14
<212> DNA
<213> Artificial Sequence

<220>
<223> Synthetic Oligonucleotide

<400> 63
agcctcagtc tgct                                                         14

<210> 64
<211> 16
<212> DNA
<213> Artificial Sequence

<220>
<223> Synthetic Oligonucleotide

<400> 64
aactctgagg attgtt                                                        16

<210> 65
<211> 14
<212> DNA
<213> Artificial Sequence

<220>
<223> Synthetic Oligonucleotide

<400> 65
actctgagga ttgt                                                          14

<210> 66
<211> 16
<212> DNA
<213> Artificial Sequence

<220>
<223> Synthetic Oligonucleotide

<400> 66
tcattaactc tgagga                                                        16

<210> 67
<211> 14
<212> DNA
<213> Artificial Sequence

<220>
<223> Synthetic Oligonucleotide

<400> 67
cattaactct gagg                                                          14

<210> 68
<211> 16
<212> DNA
<213> Artificial Sequence

<220>
<223> Synthetic Oligonucleotide

<400> 68
attcatcatt aactct                                                        16

<210> 69
<211> 14
<212> DNA
<213> Artificial Sequence

<220>
<223> Synthetic Oligonucleotide

<400> 69
ttcatcatta actc                                                          14

<210> 70
<211> 16
<212> DNA
<213> Artificial Sequence

<220>

<223> Synthetic Oligonucleotide

<400> 70
ttgttctgaa tgtcca                                                        16

<210> 71
<211> 4
<212> DNA
<213> Artificial Sequence

<220>
<223> Synthetic Oligonucleotide

<400> 71
actg                                                                      4

<210> 72
<211> 4
<212> DNA
<213> Artificial Sequence

<220>
<223> Synthetic Oligonucleotide

<400> 72
actg                                                                      4

<210> 73
<211> 14
<212> DNA
<213> Artificial Sequence

<220>
<223> Synthetic Oligonucleotide

<400> 73
tgttctgaat gtcc                                                          14

<210> 74
<211> 16
<212> DNA
<213> Artificial Sequence

<220>
<223> Synthetic Oligonucleotide

<400> 74
cagatgagtc catttg                                                        16

<210> 75
<211> 14
<212> DNA
<213> Artificial Sequence

<220>
<223> Synthetic Oligonucleotide

<400> 75
agatgagtcc attt                                                          14

<210> 76
<211> 16
<212> DNA
<213> Artificial Sequence

<220>
<223> Synthetic Oligonucleotide

<400> 76
atccacaggg aaattg                                                        16

<210> 77
<211> 14
<212> DNA
<213> Artificial Sequence

<220>
<223> Synthetic Oligonucleotide

<400> 77
tccacaggga aatt                                                          14

<210> 78
<211> 16
<212> DNA
<213> Artificial Sequence

<220>
<223> Synthetic Oligonucleotide

<400> 78
cagttgtaca agttgc                                                        16

<210> 79
<211> 14
<212> DNA
<213> Artificial Sequence

<220>
<223> Synthetic Oligonucleotide

<400> 79
agttgtacaa gttg                                                          14

<210> 80
<211> 16
<212> DNA
<213> Artificial Sequence

<220>
<223> Synthetic Oligonucleotide

<400> 80
cacagagtca gccttc                                                        16

<210> 81
<211> 14
<212> DNA
<213> Artificial Sequence

<220>
<223> Synthetic Oligonucleotide

<400> 81
acagagtcag cctt                                                          14

<210> 82
<211> 16
<212> DNA
<213> Artificial Sequence

<220>
<223> Synthetic Oligonucleotide

<400> 82
ggtcaaccac agagtc                                                        16

<210> 83
<211> 14
<212> DNA
<213> Artificial Sequence

<220>
<223> Synthetic Oligonucleotide

<400> 83
gtcaaccaca gagt                                                          14

<210> 84
<211> 14
<212> DNA
<213> Artificial Sequence

<220>
<223> Synthetic Oligonucleotide

<400> 84
cagccacatg cagc                                                          14

<210> 85
<211> 14
<212> DNA
<213> Artificial Sequence

<220>
<223> Synthetic Oligonucleotide

<400> 85
ccagccacat gcag                                                          14

<210> 86
<211> 14
<212> DNA
<213> Artificial Sequence

<220>
<223> Synthetic Oligonucleotide

<400> 86
accagccaca tgca                                                          14

<210> 87
<211> 14
<212> DNA
<213> Artificial Sequence

<220>
<223> Synthetic Oligonucleotide

<400> 87
taccagccac atgc                                                          14

<210> 88
<211> 14
<212> DNA

<213> Artificial Sequence

<220>
<223> Synthetic Oligonucleotide

<400> 88
ttaccagcca catg                                                          14

<210> 89
<211> 14
<212> DNA
<213> Artificial Sequence

<220>
<223> Synthetic Oligonucleotide

<400> 89
gttaccagcc acat                                                          14

<210> 90
<211> 14
<212> DNA
<213> Artificial Sequence

<220>
<223> Synthetic Oligonucleotide

<400> 90
ggttaccagc caca                                                          14

<210> 91
<211> 14
<212> DNA
<213> Artificial Sequence

<220>
<223> Synthetic Oligonucleotide

<400> 91
aggttaccag ccac                                                          14

<210> 92
<211> 14
<212> DNA
<213> Artificial Sequence

<220>
<223> Synthetic Oligonucleotide

<400> 92
taggttacca gcca                                                          14

<210> 93
<211> 16
<212> DNA
<213> Artificial Sequence

<220>
<223> Synthetic Oligonucleotide

<400> 93
aggttctgct ttcaac                                                        16

<210> 94
<211> 14

<212> DNA
<213> Artificial Sequence

<220>
<223> Synthetic Oligonucleotide

<400> 94
ggttctgctt tcaa                                                         14

<210> 95
<211> 16
<212> DNA
<213> Artificial Sequence

<220>
<223> Synthetic Oligonucleotide

<400> 95
tactgatcaa attgta                                                       16

<210> 96
<211> 14
<212> DNA
<213> Artificial Sequence

<220>
<223> Synthetic Oligonucleotide

<400> 96
actgatcaaa ttgt                                                         14

<210> 97
<211> 16
<212> DNA
<213> Artificial Sequence

<220>
<223> Synthetic Oligonucleotide

<400> 97
tttttcttgt atctgg                                                       16

<210> 98
<211> 14
<212> DNA
<213> Artificial Sequence

<220>
<223> Synthetic Oligonucleotide

<400> 98
ttttcttgta tctg                                                         14

<210> 99
<211> 16
<212> DNA
<213> Artificial Sequence

<220>
<223> Synthetic Oligonucleotide

<400> 99
atccattaaa acctgg                                                       16

<210> 100

<211> 14
<212> DNA
<213> Artificial Sequence

<220>
<223> Synthetic Oligonucleotide

<400> 100
tccattaaaa cctg                                                          14

<210> 101
<211> 16
<212> DNA
<213> Artificial Sequence

<220>
<223> Synthetic Oligonucleotide

<400> 101
atattgctct gcaaag                                                        16

<210> 102
<211> 14
<212> DNA
<213> Artificial Sequence

<220>
<223> Synthetic Oligonucleotide

<400> 102
tattgctctg caaa                                                          14

<210> 103
<211> 4
<212> DNA
<213> Artificial Sequence

<220>
<223> Synthetic Oligonucleotide

<400> 103
actg                                                                      4

<210> 104
<211> 14
<212> DNA
<213> Artificial Sequence

<220>
<223> Synthetic Oligonucleotide

<400> 104
atattgctct gcaa                                                          14

<210> 105
<211> 14
<212> DNA
<213> Artificial Sequence

<220>
<223> Synthetic Oligonucleotide

<400> 105
aatattgctc tgca                                                          14

<210> 106
<211> 14
<212> DNA
<213> Artificial Sequence

<220>
<223> Synthetic Oligonucleotide

<400> 106
gaatattgct ctgc                                                          14

<210> 107
<211> 14
<212> DNA
<213> Artificial Sequence

<220>
<223> Synthetic Oligonucleotide

<400> 107
agaatattgc tctg                                                          14

<210> 108
<211> 14
<212> DNA
<213> Artificial Sequence

<220>
<223> Synthetic Oligonucleotide

<400> 108
tagaatattg ctct                                                          14

<210> 109
<211> 14
<212> DNA
<213> Artificial Sequence

<220>
<223> Synthetic Oligonucleotide

<400> 109
atagaatatt gctc                                                          14

<210> 110
<211> 14
<212> DNA
<213> Artificial Sequence

<220>
<223> Synthetic Oligonucleotide

<400> 110
gatagaatat tgct                                                          14

<210> 111
<211> 14
<212> DNA
<213> Artificial Sequence

<220>
<223> Synthetic Oligonucleotide

<400> 111
ggatagaata ttgc                                                          14

<210> 112
<211> 16
<212> DNA
<213> Artificial Sequence

<220>
<223> Synthetic Oligonucleotide

<400> 112
atggaatcct caaatc                                                        16

<210> 113
<211> 14
<212> DNA
<213> Artificial Sequence

<220>
<223> Synthetic Oligonucleotide

<400> 113
tggaatcctc aaat                                                          14

<210> 114
<211> 16
<212> DNA
<213> Artificial Sequence

<220>
<223> Synthetic Oligonucleotide

<400> 114
gaattctggt atgtga                                                        16

<210> 115
<211> 14
<212> DNA
<213> Artificial Sequence

<220>
<223> Synthetic Oligonucleotide

<400> 115
aattctggta tgtg                                                          14

<210> 116
<211> 16
<212> DNA
<213> Artificial Sequence

<220>
<223> Synthetic Oligonucleotide

<400> 116
agctggaatt ctggta                                                        16

<210> 117
<211> 14
<212> DNA
<213> Artificial Sequence

<220>
<223> Synthetic Oligonucleotide

<400> 117

gctggaattc tggt                                                          14

<210> 118
<211> 16
<212> DNA
<213> Artificial Sequence

<220>
<223> Synthetic Oligonucleotide

<400> 118
tgaaaatcaa aattga                                                        16

<210> 119
<211> 14
<212> DNA
<213> Artificial Sequence

<220>
<223> Synthetic Oligonucleotide

<400> 119
gaaaatcaaa attg                                                          14

<210> 120
<211> 16
<212> DNA
<213> Artificial Sequence

<220>
<223> Synthetic Oligonucleotide

<400> 120
aaacagtgca tagtta                                                        16

<210> 121
<211> 14
<212> DNA
<213> Artificial Sequence

<220>
<223> Synthetic Oligonucleotide

<400> 121
aacagtgcat agtt                                                          14

<210> 122
<211> 16
<212> DNA
<213> Artificial Sequence

<220>
<223> Synthetic Oligonucleotide

<400> 122
ttcaggaatt gttaaa                                                        16

<210> 123
<211> 14
<212> DNA
<213> Artificial Sequence

<220>
<223> Synthetic Oligonucleotide

<400> 123
tcaggaattg ttaa                                                          14

<210> 124
<211> 16
<212> DNA
<213> Artificial Sequence

<220>
<223> Synthetic Oligonucleotide

<400> 124
ttttgtttca ttatag                                                        16

<210> 125
<211> 14
<212> DNA
<213> Artificial Sequence

<220>
<223> Synthetic Oligonucleotide

<400> 125
tttgtttcat tata                                                          14

<210> 126
<211> 16
<212> DNA
<213> Artificial Sequence

<220>
<223> Synthetic Oligonucleotide

<400> 126
gatgacactt gattta                                                        16

<210> 127
<211> 14
<212> DNA
<213> Artificial Sequence

<220>
<223> Synthetic Oligonucleotide

<400> 127
atgacacttg attt                                                          14

<210> 128
<211> 16
<212> DNA
<213> Artificial Sequence

<220>
<223> Synthetic Oligonucleotide

<400> 128
gtgtgatgac acttga                                                        16

<210> 129
<211> 14
<212> DNA
<213> Artificial Sequence

<220>
<223> Synthetic Oligonucleotide

<400> 129
tgtgatgaca cttg                                                          14

<210> 130
<211> 16
<212> DNA
<213> Artificial Sequence

<220>
<223> Synthetic Oligonucleotide

<400> 130
tattcagtgt gatgac                                                        16

<210> 131
<211> 14
<212> DNA
<213> Artificial Sequence

<220>
<223> Synthetic Oligonucleotide

<400> 131
attcagtgtg atga                                                          14

<210> 132
<211> 16
<212> DNA
<213> Artificial Sequence

<220>
<223> Synthetic Oligonucleotide

<400> 132
attggtattc agtgtg                                                        16

<210> 133
<211> 14
<212> DNA
<213> Artificial Sequence

<220>
<223> Synthetic Oligonucleotide

<400> 133
ttggtattca gtgt                                                          14

<210> 134
<211> 14
<212> DNA
<213> Artificial Sequence

<220>
<223> Synthetic Oligonucleotide

<400> 134
cctctagctg taag                                                          14

<210> 135
<211> 14
<212> DNA
<213> Artificial Sequence

<220>

<223> Synthetic Oligonucleotide

<400> 135
ccctctagct gtaa                                                         14

<210> 136
<211> 14
<212> DNA
<213> Artificial Sequence

<220>
<223> Synthetic Oligonucleotide

<400> 136
gccctctagc tgta                                                         14

<210> 137
<211> 14
<212> DNA
<213> Artificial Sequence

<220>
<223> Synthetic Oligonucleotide

<400> 137
ggccctctag ctgt                                                         14

<210> 138
<211> 14
<212> DNA
<213> Artificial Sequence

<220>
<223> Synthetic Oligonucleotide

<400> 138
aggccctcta gctg                                                         14

<210> 139
<211> 14
<212> DNA
<213> Artificial Sequence

<220>
<223> Synthetic Oligonucleotide

<400> 139
gaggccctct agct                                                         14

<210> 140
<211> 14
<212> DNA
<213> Artificial Sequence

<220>
<223> Synthetic Oligonucleotide

<400> 140
agaggccctc tagc                                                         14

<210> 141
<211> 14
<212> DNA
<213> Artificial Sequence

<220>
<223> Synthetic Oligonucleotide

<400> 141
aagaggccct ctag                                                         14

<210> 142
<211> 14
<212> DNA
<213> Artificial Sequence

<220>
<223> Synthetic Oligonucleotide

<400> 142
aaagaggccc tcta                                                         14

<210> 143
<211> 16
<212> DNA
<213> Artificial Sequence

<220>
<223> Synthetic Oligonucleotide

<400> 143
gaatggacag gtcaat                                                       16

<210> 144
<211> 14
<212> DNA
<213> Artificial Sequence

<220>
<223> Synthetic Oligonucleotide

<400> 144
aatggacagg tcaa                                                         14

<210> 145
<211> 16
<212> DNA
<213> Artificial Sequence

<220>
<223> Synthetic Oligonucleotide

<400> 145
gttttgaatg gacagg                                                       16

<210> 146
<211> 14
<212> DNA
<213> Artificial Sequence

<220>
<223> Synthetic Oligonucleotide

<400> 146
ttttgaatgg acag                                                         14

<210> 147
<211> 16
<212> DNA
<213> Artificial Sequence

<220>
<223> Synthetic Oligonucleotide

<400> 147
tggtagtttt gaatgg                                                        16

<210> 148
<211> 14
<212> DNA
<213> Artificial Sequence

<220>
<223> Synthetic Oligonucleotide

<400> 148
ggtagttttg aatg                                                          14

<210> 149
<211> 14
<212> DNA
<213> Artificial Sequence

<220>
<223> Synthetic Oligonucleotide

<400> 149
tcactgtatg gttt                                                          14

<210> 150
<211> 14
<212> DNA
<213> Artificial Sequence

<220>
<223> Synthetic Oligonucleotide

<400> 150
ctcactgtat ggtt                                                          14

<210> 151
<211> 14
<212> DNA
<213> Artificial Sequence

<220>
<223> Synthetic Oligonucleotide

<400> 151
gctcactgta tggt                                                          14

<210> 152
<211> 14
<212> DNA
<213> Artificial Sequence

<220>
<223> Synthetic Oligonucleotide

<400> 152
ggctcactgt atgg                                                          14

<210> 153
<211> 14
<212> DNA

<213> Artificial Sequence

<220>
<223> Synthetic Oligonucleotide

<400> 153
tggctcactg tatg                                                         14

<210> 154
<211> 14
<212> DNA
<213> Artificial Sequence

<220>
<223> Synthetic Oligonucleotide

<400> 154
ctggctcact gtat                                                         14

<210> 155
<211> 14
<212> DNA
<213> Artificial Sequence

<220>
<223> Synthetic Oligonucleotide

<400> 155
gctggctcac tgta                                                         14

<210> 156
<211> 14
<212> DNA
<213> Artificial Sequence

<220>
<223> Synthetic Oligonucleotide

<400> 156
ggctggctca ctgt                                                         14

<210> 157
<211> 14
<212> DNA
<213> Artificial Sequence

<220>
<223> Synthetic Oligonucleotide

<400> 157
aggctggctc actg                                                         14

<210> 158
<211> 14
<212> DNA
<213> Artificial Sequence

<220>
<223> Synthetic Oligonucleotide

<400> 158
caggtccagt tcat                                                         14

<210> 159
<211> 14

<212> DNA
<213> Artificial Sequence

<220>
<223> Synthetic Oligonucleotide

<400> 159
gcaggtccag ttca                                                         14

<210> 160
<211> 14
<212> DNA
<213> Artificial Sequence

<220>
<223> Synthetic Oligonucleotide

<400> 160
tgcaggtcca gttc                                                         14

<210> 161
<211> 14
<212> DNA
<213> Artificial Sequence

<220>
<223> Synthetic Oligonucleotide

<400> 161
gtgcaggtcc agtt                                                         14

<210> 162
<211> 14
<212> DNA
<213> Artificial Sequence

<220>
<223> Synthetic Oligonucleotide

<400> 162
ggtgcaggtc cagt                                                         14

<210> 163
<211> 14
<212> DNA
<213> Artificial Sequence

<220>
<223> Synthetic Oligonucleotide

<400> 163
tggtgcaggt ccag                                                         14

<210> 164
<211> 14
<212> DNA
<213> Artificial Sequence

<220>
<223> Synthetic Oligonucleotide

<400> 164
ttggtgcagg tcca                                                         14

<210> 165

<211> 14
<212> DNA
<213> Artificial Sequence

<220>
<223> Synthetic Oligonucleotide

<400> 165
tttggtgcag gtcc                                                          14

<210> 166
<211> 14
<212> DNA
<213> Artificial Sequence

<220>
<223> Synthetic Oligonucleotide

<400> 166
ctttggtgca ggtc                                                          14

<210> 167
<211> 14
<212> DNA
<213> Artificial Sequence

<220>
<223> Synthetic Oligonucleotide

<400> 167
taactcagat cctg                                                          14

<210> 168
<211> 14
<212> DNA
<213> Artificial Sequence

<220>
<223> Synthetic Oligonucleotide

<400> 168
ataactcaga tcct                                                          14

<210> 169
<211> 14
<212> DNA
<213> Artificial Sequence

<220>
<223> Synthetic Oligonucleotide

<400> 169
aataactcag atcc                                                          14

<210> 170
<211> 14
<212> DNA
<213> Artificial Sequence

<220>
<223> Synthetic Oligonucleotide

<400> 170
aaataactca gatc                                                          14

<210> 171
<211> 14
<212> DNA
<213> Artificial Sequence

<220>
<223> Synthetic Oligonucleotide

<400> 171
aaaataactc agat                                                         14

<210> 172
<211> 14
<212> DNA
<213> Artificial Sequence

<220>
<223> Synthetic Oligonucleotide

<400> 172
caaaataact caga                                                         14

<210> 173
<211> 14
<212> DNA
<213> Artificial Sequence

<220>
<223> Synthetic Oligonucleotide

<400> 173
gcaaaataac tcag                                                         14

<210> 174
<211> 14
<212> DNA
<213> Artificial Sequence

<220>
<223> Synthetic Oligonucleotide

<400> 174
agcaaaataa ctca                                                         14

<210> 175
<211> 14
<212> DNA
<213> Artificial Sequence

<220>
<223> Synthetic Oligonucleotide

<400> 175
tagcaaaata actc                                                         14

<210> 176
<211> 14
<212> DNA
<213> Artificial Sequence

<220>
<223> Synthetic Oligonucleotide

<400> 176
cagggcctgg agag                                                         14

<210> 177
<211> 16
<212> DNA
<213> Artificial Sequence

<220>
<223> Synthetic Oligonucleotide

<400> 177
tctgaagtcc atgatc                                                        16

<210> 178
<211> 14
<212> DNA
<213> Artificial Sequence

<220>
<223> Synthetic Oligonucleotide

<400> 178
ctgaagtcca tgat                                                          14

<210> 179
<211> 16
<212> DNA
<213> Artificial Sequence

<220>
<223> Synthetic Oligonucleotide

<400> 179
tgggcatgat tccatt                                                        16

<210> 180
<211> 14
<212> DNA
<213> Artificial Sequence

<220>
<223> Synthetic Oligonucleotide

<400> 180
gggcatgatt ccat                                                          14

<210> 181
<211> 14
<212> DNA
<213> Artificial Sequence

<220>
<223> Synthetic Oligonucleotide

<400> 181
tggagcccac gtgc                                                          14

<210> 182
<211> 16
<212> DNA
<213> Artificial Sequence

<220>
<223> Synthetic Oligonucleotide

<400> 182

ttgaagttga gggctg                                                    16

<210> 183
<211> 14
<212> DNA
<213> Artificial Sequence

<220>
<223> Synthetic Oligonucleotide

<400> 183
tgaagttgag ggct                                                      14

<210> 184
<211> 16
<212> DNA
<213> Artificial Sequence

<220>
<223> Synthetic Oligonucleotide

<400> 184
accagtatta attttg                                                    16

<210> 185
<211> 14
<212> DNA
<213> Artificial Sequence

<220>
<223> Synthetic Oligonucleotide

<400> 185
ccagtattaa tttt                                                      14

<210> 186
<211> 16
<212> DNA
<213> Artificial Sequence

<220>
<223> Synthetic Oligonucleotide

<400> 186
gtgttctttg aagcgg                                                    16

<210> 187
<211> 14
<212> DNA
<213> Artificial Sequence

<220>
<223> Synthetic Oligonucleotide

<400> 187
tgttctttga agcg                                                      14

<210> 188
<211> 14
<212> DNA
<213> Artificial Sequence

<220>
<223> Synthetic Oligonucleotide

<400> 188
agttactttg gtgt                                                         14

<210> 189
<211> 16
<212> DNA
<213> Artificial Sequence

<220>
<223> Synthetic Oligonucleotide

<400> 189
tggtacatgg aagtct                                                       16

<210> 190
<211> 14
<212> DNA
<213> Artificial Sequence

<220>
<223> Synthetic Oligonucleotide

<400> 190
ggtacatgga agtc                                                         14

<210> 191
<211> 4
<212> DNA
<213> Artificial Sequence

<220>
<223> Synthetic Oligonucleotide

<400> 191
actg                                                                     4

<210> 192
<211> 4
<212> DNA
<213> Artificial Sequence

<220>
<223> Synthetic Oligonucleotide

<400> 192
actg                                                                     4

<210> 193
<211> 4
<212> DNA
<213> Artificial Sequence

<220>
<223> Synthetic Oligonucleotide

<400> 193
actg                                                                     4

<210> 194
<211> 4
<212> DNA
<213> Artificial Sequence

<220>
<223> Synthetic Oligonucleotide

<400> 194
actg                                                                    4

<210> 195
<211> 4
<212> DNA
<213> Artificial Sequence

<220>
<223> Synthetic Oligonucleotide

<400> 195
actg                                                                    4

<210> 196
<211> 4
<212> DNA
<213> Artificial Sequence

<220>
<223> Synthetic Oligonucleotide

<400> 196
actg                                                                    4

<210> 197
<211> 4
<212> DNA
<213> Artificial Sequence

<220>
<223> Synthetic Oligonucleotide

<400> 197
actg                                                                    4

<210> 198
<211> 4
<212> DNA
<213> Artificial Sequence

<220>
<223> Synthetic Oligonucleotide

<400> 198
actg                                                                    4

<210> 199
<211> 4
<212> DNA
<213> Artificial Sequence

<220>
<223> Synthetic Oligonucleotide

<400> 199
actg                                                                    4

<210> 200
<211> 14
<212> DNA
<213> Artificial Sequence

<220>

<223> Synthetic Oligonucleotide

<400> 200
tccatgccat atgt                                                         14

<210> 201
<211> 16
<212> DNA
<213> Artificial Sequence

<220>
<223> Synthetic Oligonucleotide

<400> 201
ccctgaagaa gtccat                                                       16

<210> 202
<211> 14
<212> DNA
<213> Artificial Sequence

<220>
<223> Synthetic Oligonucleotide

<400> 202
cctgaagaag tcca                                                         14

<210> 203
<211> 16
<212> DNA
<213> Artificial Sequence

<220>
<223> Synthetic Oligonucleotide

<400> 203
gcccagttcc atgacc                                                       16

<210> 204
<211> 14
<212> DNA
<213> Artificial Sequence

<220>
<223> Synthetic Oligonucleotide

<400> 204
cccagttcca tgac                                                         14

<210> 205
<211> 16
<212> DNA
<213> Artificial Sequence

<220>
<223> Synthetic Oligonucleotide

<400> 205
ttgaggaagc cagatt                                                       16

<210> 206
<211> 14
<212> DNA
<213> Artificial Sequence

<220>
<223> Synthetic Oligonucleotide

<400> 206
tgaggaagcc agat                                                         14

<210> 207
<211> 16
<212> DNA
<213> Artificial Sequence

<220>
<223> Synthetic Oligonucleotide

<400> 207
tggatgcagt aatctc                                                       16

<210> 208
<211> 14
<212> DNA
<213> Artificial Sequence

<220>
<223> Synthetic Oligonucleotide

<400> 208
ggatgcagta atct                                                         14

<210> 209
<211> 16
<212> DNA
<213> Artificial Sequence

<220>
<223> Synthetic Oligonucleotide

<400> 209
tataaagtcc agcatt                                                       16

<210> 210
<211> 14
<212> DNA
<213> Artificial Sequence

<220>
<223> Synthetic Oligonucleotide

<400> 210
ataaagtcca gcat                                                         14

<210> 211
<211> 16
<212> DNA
<213> Artificial Sequence

<220>
<223> Synthetic Oligonucleotide

<400> 211
aagttcctgc ttgaag                                                       16

<210> 212
<211> 14
<212> DNA
<213> Artificial Sequence

<220>
<223> Synthetic Oligonucleotide

<400> 212
agttcctgct tgaa                                                         14

<210> 213
<211> 14
<212> DNA
<213> Artificial Sequence

<220>
<223> Synthetic Oligonucleotide

<400> 213
aatggtgaag tact                                                         14

<210> 214
<211> 12
<212> DNA
<213> Artificial Sequence

<220>
<223> Synthetic Oligonucleotide

<400> 214
tgtcagcagg at                                                           12

<210> 215
<211> 12
<212> DNA
<213> Artificial Sequence

<220>
<223> Synthetic Oligonucleotide

<400> 215
cagcaggaaa ta                                                           12

<210> 216
<211> 12
<212> DNA
<213> Artificial Sequence

<220>
<223> Synthetic Oligonucleotide

<400> 216
ccagcaggaa at                                                           12

<210> 217
<211> 12
<212> DNA
<213> Artificial Sequence

<220>
<223> Synthetic Oligonucleotide

<400> 217
accagcagga aa                                                           12

<210> 218
<211> 12
<212> DNA

<213> Artificial Sequence

<220>
<223> Synthetic Oligonucleotide

<400> 218
gaccagcagg aa                                                          12

<210> 219
<211> 12
<212> DNA
<213> Artificial Sequence

<220>
<223> Synthetic Oligonucleotide

<400> 219
tgaccagcag ga                                                          12

<210> 220
<211> 4
<212> DNA
<213> Artificial Sequence

<220>
<223> Synthetic Oligonucleotide

<400> 220
actg                                                                    4

<210> 221
<211> 12
<212> DNA
<213> Artificial Sequence

<220>
<223> Synthetic Oligonucleotide

<400> 221
atgaccagca gg                                                          12

<210> 222
<211> 12
<212> DNA
<213> Artificial Sequence

<220>
<223> Synthetic Oligonucleotide

<400> 222
aatgaccagc ag                                                          12

<210> 223
<211> 12
<212> DNA
<213> Artificial Sequence

<220>
<223> Synthetic Oligonucleotide

<400> 223
caatgaccag ca                                                          12

<210> 224
<211> 12

<212> DNA
<213> Artificial Sequence

<220>
<223> Synthetic Oligonucleotide

<400> 224
ccaatgacca gc                                                            12

<210> 225
<211> 12
<212> DNA
<213> Artificial Sequence

<220>
<223> Synthetic Oligonucleotide

<400> 225
accacaagcc aa                                                            12

<210> 226
<211> 12
<212> DNA
<213> Artificial Sequence

<220>
<223> Synthetic Oligonucleotide

<400> 226
tagccgccca ca                                                            12

<210> 227
<211> 4
<212> DNA
<213> Artificial Sequence

<220>
<223> Synthetic Oligonucleotide

<400> 227
actg                                                                     4

<210> 228
<211> 12
<212> DNA
<213> Artificial Sequence

<220>
<223> Synthetic Oligonucleotide

<400> 228
ccggccacca ca                                                            12

<210> 229
<211> 12
<212> DNA
<213> Artificial Sequence

<220>
<223> Synthetic Oligonucleotide

<400> 229
accggccacc ac                                                            12

<210> 230

<211> 12
<212> DNA
<213> Artificial Sequence

<220>
<223> Synthetic Oligonucleotide

<400> 230
gatgttgctg gc                                                          12

<210> 231
<211> 4
<212> DNA
<213> Artificial Sequence

<220>
<223> Synthetic Oligonucleotide

<400> 231
actg                                                                    4

<210> 232
<211> 12
<212> DNA
<213> Artificial Sequence

<220>
<223> Synthetic Oligonucleotide

<400> 232
cgatgttgct gg                                                          12

<210> 233
<211> 12
<212> DNA
<213> Artificial Sequence

<220>
<223> Synthetic Oligonucleotide

<400> 233
ccgatgttgc tg                                                          12

<210> 234
<211> 12
<212> DNA
<213> Artificial Sequence

<220>
<223> Synthetic Oligonucleotide

<400> 234
gccgatgttg ct                                                          12

<210> 235
<211> 12
<212> DNA
<213> Artificial Sequence

<220>
<223> Synthetic Oligonucleotide

<400> 235
tgccgatgtt gc                                                          12

<210> 236
<211> 12
<212> DNA
<213> Artificial Sequence

<220>
<223> Synthetic Oligonucleotide

<400> 236
ctgccgatgt tg                                                          12

<210> 237
<211> 12
<212> DNA
<213> Artificial Sequence

<220>
<223> Synthetic Oligonucleotide

<400> 237
caggcccaca aa                                                          12

<210> 238
<211> 12
<212> DNA
<213> Artificial Sequence

<220>
<223> Synthetic Oligonucleotide

<400> 238
ccaggcccac aa                                                          12

<210> 239
<211> 12
<212> DNA
<213> Artificial Sequence

<220>
<223> Synthetic Oligonucleotide

<400> 239
gccaggccca ca                                                          12

<210> 240
<211> 12
<212> DNA
<213> Artificial Sequence

<220>
<223> Synthetic Oligonucleotide

<400> 240
ccaagccact tg                                                          12

<210> 241
<211> 12
<212> DNA
<213> Artificial Sequence

<220>
<223> Synthetic Oligonucleotide

<400> 241
agagcgcatt cc                                                          12

<210> 242
<211> 12
<212> DNA
<213> Artificial Sequence

<220>
<223> Synthetic Oligonucleotide

<400> 242
acaggtagag gc                                                            12

<210> 243
<211> 12
<212> DNA
<213> Artificial Sequence

<220>
<223> Synthetic Oligonucleotide

<400> 243
agatcttggt ga                                                            12

<210> 244
<211> 4
<212> DNA
<213> Artificial Sequence

<220>
<223> Synthetic Oligonucleotide

<400> 244
actg                                                                      4

<210> 245
<211> 13
<212> DNA
<213> Artificial Sequence

<220>
<223> Synthetic Oligonucleotide

<400> 245
tgttccagcc cag                                                           13

<210> 246
<211> 12
<212> DNA
<213> Artificial Sequence

<220>
<223> Synthetic Oligonucleotide

<400> 246
tgttccagcc ca                                                            12

<210> 247
<211> 4
<212> DNA
<213> Artificial Sequence

<220>
<223> Synthetic Oligonucleotide

<400> 247

actg                                                                    4

<210> 248
<211> 4
<212> DNA
<213> Artificial Sequence

<220>
<223> Synthetic Oligonucleotide

<400> 248
actg                                                                    4

<210> 249
<211> 4
<212> DNA
<213> Artificial Sequence

<220>
<223> Synthetic Oligonucleotide

<400> 249
actg                                                                    4

<210> 250
<211> 4
<212> DNA
<213> Artificial Sequence

<220>
<223> Synthetic Oligonucleotide

<400> 250
actg                                                                    4

<210> 251
<211> 12
<212> DNA
<213> Artificial Sequence

<220>
<223> Synthetic Oligonucleotide

<400> 251
atgttccagc cc                                                          12

<210> 252
<211> 12
<212> DNA
<213> Artificial Sequence

<220>
<223> Synthetic Oligonucleotide

<400> 252
tggtgatgcc ca                                                          12

<210> 253
<211> 12
<212> DNA
<213> Artificial Sequence

<220>
<223> Synthetic Oligonucleotide

<400> 253
atggtgatgc cc                                                          12

<210> 254
<211> 12
<212> DNA
<213> Artificial Sequence

<220>
<223> Synthetic Oligonucleotide

<400> 254
catggtgatg cc                                                          12

<210> 255
<211> 4
<212> DNA
<213> Artificial Sequence

<220>
<223> Synthetic Oligonucleotide

<400> 255
actg                                                                    4

<210> 256
<211> 12
<212> DNA
<213> Artificial Sequence

<220>
<223> Synthetic Oligonucleotide

<400> 256
tcatggtgat gc                                                          12

<210> 257
<211> 12
<212> DNA
<213> Artificial Sequence

<220>
<223> Synthetic Oligonucleotide

<400> 257
atcatggtga tg                                                          12

<210> 258
<211> 12
<212> DNA
<213> Artificial Sequence

<220>
<223> Synthetic Oligonucleotide

<400> 258
ccctcctgtc ac                                                          12

<210> 259
<211> 12
<212> DNA
<213> Artificial Sequence

<220>
<223> Synthetic Oligonucleotide

<400> 259
gccctcctgt ca                                                          12

<210> 260
<211> 12
<212> DNA
<213> Artificial Sequence

<220>
<223> Synthetic Oligonucleotide

<400> 260
agccctcctg tc                                                          12

<210> 261
<211> 12
<212> DNA
<213> Artificial Sequence

<220>
<223> Synthetic Oligonucleotide

<400> 261
cagccctcct gt                                                          12

<210> 262
<211> 12
<212> DNA
<213> Artificial Sequence

<220>
<223> Synthetic Oligonucleotide

<400> 262
ccagccctcc tg                                                          12

<210> 263
<211> 12
<212> DNA
<213> Artificial Sequence

<220>
<223> Synthetic Oligonucleotide

<400> 263
gccagccctc ct                                                          12

<210> 264
<211> 12
<212> DNA
<213> Artificial Sequence

<220>
<223> Synthetic Oligonucleotide

<400> 264
gacgaaggtc tg                                                          12

<210> 265
<211> 12
<212> DNA
<213> Artificial Sequence

<220>

<223> Synthetic Oligonucleotide

<400> 265
gtatttgtcg aa                                                          12

<210> 266
<211> 12
<212> DNA
<213> Artificial Sequence

<220>
<223> Synthetic Oligonucleotide

<400> 266
ggacaccgtc ag                                                          12

<210> 267
<211> 12
<212> DNA
<213> Artificial Sequence

<220>
<223> Synthetic Oligonucleotide

<400> 267
cagcttcagg ta                                                          12

<210> 268
<211> 12
<212> DNA
<213> Artificial Sequence

<220>
<223> Synthetic Oligonucleotide

<400> 268
catgaccatg ag                                                          12

<210> 269
<211> 12
<212> DNA
<213> Artificial Sequence

<220>
<223> Synthetic Oligonucleotide

<400> 269
gcatgaccat ga                                                          12

<210> 270
<211> 12
<212> DNA
<213> Artificial Sequence

<220>
<223> Synthetic Oligonucleotide

<400> 270
ggcatgacca tg                                                          12

<210> 271
<211> 12
<212> DNA
<213> Artificial Sequence

<220>
<223> Synthetic Oligonucleotide

<400> 271
tggcatgacc at                                                          12

<210> 272
<211> 12
<212> DNA
<213> Artificial Sequence

<220>
<223> Synthetic Oligonucleotide

<400> 272
ctggcatgac ca                                                          12

<210> 273
<211> 4
<212> DNA
<213> Artificial Sequence

<220>
<223> Synthetic Oligonucleotide

<400> 273
actg                                                                    4

<210> 274
<211> 12
<212> DNA
<213> Artificial Sequence

<220>
<223> Synthetic Oligonucleotide

<400> 274
cctggcatga cc                                                          12

<210> 275
<211> 12
<212> DNA
<213> Artificial Sequence

<220>
<223> Synthetic Oligonucleotide

<400> 275
gcagcccacc tc                                                          12

<210> 276
<211> 12
<212> DNA
<213> Artificial Sequence

<220>
<223> Synthetic Oligonucleotide

<400> 276
agcagcccac ct                                                          12

<210> 277
<211> 12
<212> DNA
<213> Artificial Sequence

<220>
<223> Synthetic Oligonucleotide

<400> 277
gagcagccca cc                                                          12

<210> 278
<211> 12
<212> DNA
<213> Artificial Sequence

<220>
<223> Synthetic Oligonucleotide

<400> 278
catgagcttc ac                                                          12

<210> 279
<211> 12
<212> DNA
<213> Artificial Sequence

<220>
<223> Synthetic Oligonucleotide

<400> 279
gcatgagctt ca                                                          12

<210> 280
<211> 13
<212> DNA
<213> Artificial Sequence

<220>
<223> Synthetic Oligonucleotide

<400> 280
ggcatgagct tca                                                         13

<210> 281
<211> 12
<212> DNA
<213> Artificial Sequence

<220>
<223> Synthetic Oligonucleotide

<400> 281
ggcatgagct tc                                                          12

<210> 282
<211> 4
<212> DNA
<213> Artificial Sequence

<220>
<223> Synthetic Oligonucleotide

<400> 282
actg                                                                    4

<210> 283
<211> 12
<212> DNA

<213> Artificial Sequence

<220>
<223> Synthetic Oligonucleotide

<400> 283
gggcatgagc tt                                                          12

<210> 284
<211> 12
<212> DNA
<213> Artificial Sequence

<220>
<223> Synthetic Oligonucleotide

<400> 284
cagcatgagt cc                                                          12

<210> 285
<211> 12
<212> DNA
<213> Artificial Sequence

<220>
<223> Synthetic Oligonucleotide

<400> 285
ccagcatgag tc                                                          12

<210> 286
<211> 4
<212> DNA
<213> Artificial Sequence

<220>
<223> Synthetic Oligonucleotide

<400> 286
actg                                                                    4

<210> 287
<211> 12
<212> DNA
<213> Artificial Sequence

<220>
<223> Synthetic Oligonucleotide

<400> 287
gccagcatga gt                                                          12

<210> 288
<211> 12
<212> DNA
<213> Artificial Sequence

<220>
<223> Synthetic Oligonucleotide

<400> 288
ccatggtgaa ga                                                          12

<210> 289
<211> 12

<212> DNA
<213> Artificial Sequence

<220>
<223> Synthetic Oligonucleotide

<400> 289
cacagctgcc cg                                                          12

<210> 290
<211> 12
<212> DNA
<213> Artificial Sequence

<220>
<223> Synthetic Oligonucleotide

<400> 290
acacagctgc cc                                                          12

<210> 291
<211> 12
<212> DNA
<213> Artificial Sequence

<220>
<223> Synthetic Oligonucleotide

<400> 291
cacacagctg cc                                                          12

<210> 292
<211> 13
<212> DNA
<213> Artificial Sequence

<220>
<223> Synthetic Oligonucleotide

<400> 292
gcacacagct gcc                                                         13

<210> 293
<211> 12
<212> DNA
<213> Artificial Sequence

<220>
<223> Synthetic Oligonucleotide

<400> 293
gcacacagct gc                                                          12

<210> 294
<211> 4
<212> DNA
<213> Artificial Sequence

<220>
<223> Synthetic Oligonucleotide

<400> 294
actg                                                                    4

<210> 295

<211> 12
<212> DNA
<213> Artificial Sequence

<220>
<223> Synthetic Oligonucleotide

<400> 295
gccggagact ga                                                          12

<210> 296
<211> 12
<212> DNA
<213> Artificial Sequence

<220>
<223> Synthetic Oligonucleotide

<400> 296
attgaggttg ac                                                          12

<210> 297
<211> 12
<212> DNA
<213> Artificial Sequence

<220>
<223> Synthetic Oligonucleotide

<400> 297
cattgaggtt ga                                                          12

<210> 298
<211> 12
<212> DNA
<213> Artificial Sequence

<220>
<223> Synthetic Oligonucleotide

<400> 298
gcattgaggt tg                                                          12

<210> 299
<211> 12
<212> DNA
<213> Artificial Sequence

<220>
<223> Synthetic Oligonucleotide

<400> 299
ggcattgagg tt                                                          12

<210> 300
<211> 12
<212> DNA
<213> Artificial Sequence

<220>
<223> Synthetic Oligonucleotide

<400> 300
gggcattgag gt                                                          12

<210> 301
<211> 4
<212> DNA
<213> Artificial Sequence

<220>
<223> Synthetic Oligonucleotide

<400> 301
actg                                                                    4

<210> 302
<211> 4
<212> DNA
<213> Artificial Sequence

<220>
<223> Synthetic Oligonucleotide

<400> 302
actg                                                                    4

<210> 303
<211> 4
<212> DNA
<213> Artificial Sequence

<220>
<223> Synthetic Oligonucleotide

<400> 303
actg                                                                    4

<210> 304
<211> 4
<212> DNA
<213> Artificial Sequence

<220>
<223> Synthetic Oligonucleotide

<400> 304
actg                                                                    4

<210> 305
<211> 4
<212> DNA
<213> Artificial Sequence

<220>
<223> Synthetic Oligonucleotide

<400> 305
actg                                                                    4

<210> 306
<211> 4
<212> DNA
<213> Artificial Sequence

<220>
<223> Synthetic Oligonucleotide

<400> 306
actg                                                                    4

<210> 307
<211> 4
<212> DNA
<213> Artificial Sequence

<220>
<223> Synthetic Oligonucleotide

<400> 307
actg                                                                    4

<210> 308
<211> 4
<212> DNA
<213> Artificial Sequence

<220>
<223> Synthetic Oligonucleotide

<400> 308
actg                                                                    4

<210> 309
<211> 4
<212> DNA
<213> Artificial Sequence

<220>
<223> Synthetic Oligonucleotide

<400> 309
actg                                                                    4

<210> 310
<211> 4
<212> DNA
<213> Artificial Sequence

<220>
<223> Synthetic Oligonucleotide

<400> 310
actg                                                                    4

<210> 311
<211> 4
<212> DNA
<213> Artificial Sequence

<220>
<223> Synthetic Oligonucleotide

<400> 311
actg                                                                    4

<210> 312
<211> 4
<212> DNA
<213> Artificial Sequence

<220>
<223> Synthetic Oligonucleotide

<400> 312

actg                                                                    4

<210> 313
<211> 4
<212> DNA
<213> Artificial Sequence

<220>
<223> Synthetic Oligonucleotide

<400> 313
actg                                                                    4

<210> 314
<211> 4
<212> DNA
<213> Artificial Sequence

<220>
<223> Synthetic Oligonucleotide

<400> 314
actg                                                                    4

<210> 315
<211> 4
<212> DNA
<213> Artificial Sequence

<220>
<223> Synthetic Oligonucleotide

<400> 315
actg                                                                    4

<210> 316
<211> 4
<212> DNA
<213> Artificial Sequence

<220>
<223> Synthetic Oligonucleotide

<400> 316
actg                                                                    4

<210> 317
<211> 4
<212> DNA
<213> Artificial Sequence

<220>
<223> Synthetic Oligonucleotide

<400> 317
actg                                                                    4

<210> 318
<211> 4
<212> DNA
<213> Artificial Sequence

<220>
<223> Synthetic Oligonucleotide

<400> 318
actg                                                                    4

<210> 319
<211> 4
<212> DNA
<213> Artificial Sequence

<220>
<223> Synthetic Oligonucleotide

<400> 319
actg                                                                    4

<210> 320
<211> 4
<212> DNA
<213> Artificial Sequence

<220>
<223> Synthetic Oligonucleotide

<400> 320
actg                                                                    4

<210> 321
<211> 4
<212> DNA
<213> Artificial Sequence

<220>
<223> Synthetic Oligonucleotide

<400> 321
actg                                                                    4

<210> 322
<211> 4
<212> DNA
<213> Artificial Sequence

<220>
<223> Synthetic Oligonucleotide

<400> 322
actg                                                                    4

<210> 323
<211> 4
<212> DNA
<213> Artificial Sequence

<220>
<223> Synthetic Oligonucleotide

<400> 323
actg                                                                    4

<210> 324
<211> 4
<212> DNA
<213> Artificial Sequence

<220>
<223> Synthetic Oligonucleotide

<400> 324
actg                                                                    4

<210> 325
<211> 4
<212> DNA
<213> Artificial Sequence

<220>
<223> Synthetic Oligonucleotide

<400> 325
actg                                                                    4

<210> 326
<211> 4
<212> DNA
<213> Artificial Sequence

<220>
<223> Synthetic Oligonucleotide

<400> 326
actg                                                                    4

<210> 327
<211> 4
<212> DNA
<213> Artificial Sequence

<220>
<223> Synthetic Oligonucleotide

<400> 327
actg                                                                    4

<210> 328
<211> 4
<212> DNA
<213> Artificial Sequence

<220>
<223> Synthetic Oligonucleotide

<400> 328
actg                                                                    4

<210> 329
<211> 14
<212> DNA
<213> Artificial Sequence

<220>
<223> Synthetic Oligonucleotide

<400> 329
atggggcaac ttca                                                        14

<210> 330
<211> 14
<212> DNA
<213> Artificial Sequence

<220>

<223> Synthetic Oligonucleotide

<400> 330
catggggcaa cttc                                                         14

<210> 331
<211> 14
<212> DNA
<213> Artificial Sequence

<220>
<223> Synthetic Oligonucleotide

<400> 331
acatggggca actt                                                         14

<210> 332
<211> 14
<212> DNA
<213> Artificial Sequence

<220>
<223> Synthetic Oligonucleotide

<400> 332
gggatgctct gggc                                                         14

<210> 333
<211> 14
<212> DNA
<213> Artificial Sequence

<220>
<223> Synthetic Oligonucleotide

<400> 333
cgctccaggt tcca                                                         14

<210> 334
<211> 14
<212> DNA
<213> Artificial Sequence

<220>
<223> Synthetic Oligonucleotide

<400> 334
gatacacctc cacc                                                         14

<210> 335
<211> 14
<212> DNA
<213> Artificial Sequence

<220>
<223> Synthetic Oligonucleotide

<400> 335
agatacacct ccac                                                         14

<210> 336
<211> 14
<212> DNA
<213> Artificial Sequence

<220>
<223> Synthetic Oligonucleotide

<400> 336
gagatacacc tcca                                                         14

<210> 337
<211> 14
<212> DNA
<213> Artificial Sequence

<220>
<223> Synthetic Oligonucleotide

<400> 337
gcctgtctgt ggaa                                                         14

<210> 338
<211> 14
<212> DNA
<213> Artificial Sequence

<220>
<223> Synthetic Oligonucleotide

<400> 338
ctgtcacact tgct                                                         14

<210> 339
<211> 14
<212> DNA
<213> Artificial Sequence

<220>
<223> Synthetic Oligonucleotide

<400> 339
cggccgctga ccac                                                         14

<210> 340
<211> 14
<212> DNA
<213> Artificial Sequence

<220>
<223> Synthetic Oligonucleotide

<400> 340
ccggccgctg acca                                                         14

<210> 341
<211> 14
<212> DNA
<213> Artificial Sequence

<220>
<223> Synthetic Oligonucleotide

<400> 341
cccggccgct gacc                                                         14

<210> 342
<211> 14
<212> DNA
<213> Artificial Sequence

<220>
<223> Synthetic Oligonucleotide

<400> 342
tcccggccgc tgac                                                          14

<210> 343
<211> 14
<212> DNA
<213> Artificial Sequence

<220>
<223> Synthetic Oligonucleotide

<400> 343
atcccggccg ctga                                                          14

<210> 344
<211> 14
<212> DNA
<213> Artificial Sequence

<220>
<223> Synthetic Oligonucleotide

<400> 344
catcccggcc gctg                                                          14

<210> 345
<211> 14
<212> DNA
<213> Artificial Sequence

<220>
<223> Synthetic Oligonucleotide

<400> 345
gcatcccggc cgct                                                          14

<210> 346
<211> 14
<212> DNA
<213> Artificial Sequence

<220>
<223> Synthetic Oligonucleotide

<400> 346
gtgcccttcc cttg                                                          14

<210> 347
<211> 14
<212> DNA
<213> Artificial Sequence

<220>
<223> Synthetic Oligonucleotide

<400> 347
atgagggtgc cgct                                                          14

<210> 348
<211> 14
<212> DNA

<213> Artificial Sequence

<220>
<223> Synthetic Oligonucleotide

<400> 348
tatgagggtg ccgc                                                         14

<210> 349
<211> 14
<212> DNA
<213> Artificial Sequence

<220>
<223> Synthetic Oligonucleotide

<400> 349
ctatgagggt gccg                                                         14

<210> 350
<211> 14
<212> DNA
<213> Artificial Sequence

<220>
<223> Synthetic Oligonucleotide

<400> 350
cctatgaggg tgcc                                                         14

<210> 351
<211> 14
<212> DNA
<213> Artificial Sequence

<220>
<223> Synthetic Oligonucleotide

<400> 351
cgaataaact ccag                                                         14

<210> 352
<211> 14
<212> DNA
<213> Artificial Sequence

<220>
<223> Synthetic Oligonucleotide

<400> 352
ccgaataaac tcca                                                         14

<210> 353
<211> 14
<212> DNA
<213> Artificial Sequence

<220>
<223> Synthetic Oligonucleotide

<400> 353
tccgaataaa ctcc                                                         14

<210> 354
<211> 14

<212> DNA
<213> Artificial Sequence

<220>
<223> Synthetic Oligonucleotide

<400> 354
ttccgaataa actc                                                         14

<210> 355
<211> 14
<212> DNA
<213> Artificial Sequence

<220>
<223> Synthetic Oligonucleotide

<400> 355
gccaggggca gcag                                                         14

<210> 356
<211> 14
<212> DNA
<213> Artificial Sequence

<220>
<223> Synthetic Oligonucleotide

<400> 356
gagtagaggc aggc                                                         14

<210> 357
<211> 14
<212> DNA
<213> Artificial Sequence

<220>
<223> Synthetic Oligonucleotide

<400> 357
ccccaaagtc ccca                                                         14

<210> 358
<211> 14
<212> DNA
<213> Artificial Sequence

<220>
<223> Synthetic Oligonucleotide

<400> 358
tccccaaagt cccc                                                         14

<210> 359
<211> 14
<212> DNA
<213> Artificial Sequence

<220>
<223> Synthetic Oligonucleotide

<400> 359
gtccccaaag tccc                                                         14

<210> 360

<211> 14
<212> DNA
<213> Artificial Sequence

<220>
<223> Synthetic Oligonucleotide

<400> 360
acgtgggcag cagc                                                          14

<210> 361
<211> 14
<212> DNA
<213> Artificial Sequence

<220>
<223> Synthetic Oligonucleotide

<400> 361
cacgtgggca gcag                                                          14

<210> 362
<211> 14
<212> DNA
<213> Artificial Sequence

<220>
<223> Synthetic Oligonucleotide

<400> 362
ccacgtgggc agca                                                          14

<210> 363
<211> 14
<212> DNA
<213> Artificial Sequence

<220>
<223> Synthetic Oligonucleotide

<400> 363
gccacgtggg cagc                                                          14

<210> 364
<211> 14
<212> DNA
<213> Artificial Sequence

<220>
<223> Synthetic Oligonucleotide

<400> 364
cagggaacca ggcc                                                          14

<210> 365
<211> 14
<212> DNA
<213> Artificial Sequence

<220>
<223> Synthetic Oligonucleotide

<400> 365
tcagggaacc aggc                                                          14

<210> 366
<211> 14
<212> DNA
<213> Artificial Sequence

<220>
<223> Synthetic Oligonucleotide

<400> 366
ctcagggaac cagg                                                          14

<210> 367
<211> 14
<212> DNA
<213> Artificial Sequence

<220>
<223> Synthetic Oligonucleotide

<400> 367
cctcagggaa ccag                                                          14

<210> 368
<211> 14
<212> DNA
<213> Artificial Sequence

<220>
<223> Synthetic Oligonucleotide

<400> 368
tcctcaggga acca                                                          14

<210> 369
<211> 14
<212> DNA
<213> Artificial Sequence

<220>
<223> Synthetic Oligonucleotide

<400> 369
gtcctcaggg aacc                                                          14

<210> 370
<211> 14
<212> DNA
<213> Artificial Sequence

<220>
<223> Synthetic Oligonucleotide

<400> 370
ggtcctcagg gaac                                                          14

<210> 371
<211> 14
<212> DNA
<213> Artificial Sequence

<220>
<223> Synthetic Oligonucleotide

<400> 371
tggtcctcag ggaa                                                          14

<210> 372
<211> 14
<212> DNA
<213> Artificial Sequence

<220>
<223> Synthetic Oligonucleotide

<400> 372
ctggtcctca ggga                                                          14

<210> 373
<211> 14
<212> DNA
<213> Artificial Sequence

<220>
<223> Synthetic Oligonucleotide

<400> 373
gctggtcctc aggg                                                          14

<210> 374
<211> 14
<212> DNA
<213> Artificial Sequence

<220>
<223> Synthetic Oligonucleotide

<400> 374
gtgctggggg gcag                                                          14

<210> 375
<211> 14
<212> DNA
<213> Artificial Sequence

<220>
<223> Synthetic Oligonucleotide

<400> 375
ggtgctgggg ggca                                                          14

<210> 376
<211> 14
<212> DNA
<213> Artificial Sequence

<220>
<223> Synthetic Oligonucleotide

<400> 376
gggtgctggg gggc                                                          14

<210> 377
<211> 14
<212> DNA
<213> Artificial Sequence

<220>
<223> Synthetic Oligonucleotide

<400> 377

tgggtgctgg gggg                                                          14

<210> 378
<211> 14
<212> DNA
<213> Artificial Sequence

<220>
<223> Synthetic Oligonucleotide

<400> 378
gagcagctca gcag                                                          14

<210> 379
<211> 14
<212> DNA
<213> Artificial Sequence

<220>
<223> Synthetic Oligonucleotide

<400> 379
ggagcagctc agca                                                          14

<210> 380
<211> 14
<212> DNA
<213> Artificial Sequence

<220>
<223> Synthetic Oligonucleotide

<400> 380
tggagcagct cagc                                                          14

<210> 381
<211> 14
<212> DNA
<213> Artificial Sequence

<220>
<223> Synthetic Oligonucleotide

<400> 381
ctggagcagc tcag                                                          14

<210> 382
<211> 14
<212> DNA
<213> Artificial Sequence

<220>
<223> Synthetic Oligonucleotide

<400> 382
ccctcacccc caaa                                                          14

<210> 383
<211> 14
<212> DNA
<213> Artificial Sequence

<220>
<223> Synthetic Oligonucleotide

<400> 383
accctcaccc ccaa                                                          14

<210> 384
<211> 14
<212> DNA
<213> Artificial Sequence

<220>
<223> Synthetic Oligonucleotide

<400> 384
caccctcacc ccca                                                          14

<210> 385
<211> 14
<212> DNA
<213> Artificial Sequence

<220>
<223> Synthetic Oligonucleotide

<400> 385
acaccctcac cccc                                                          14

<210> 386
<211> 14
<212> DNA
<213> Artificial Sequence

<220>
<223> Synthetic Oligonucleotide

<400> 386
gacaccctca cccc                                                          14

<210> 387
<211> 14
<212> DNA
<213> Artificial Sequence

<220>
<223> Synthetic Oligonucleotide

<400> 387
agacaccctc accc                                                          14

<210> 388
<211> 14
<212> DNA
<213> Artificial Sequence

<220>
<223> Synthetic Oligonucleotide

<400> 388
tagacaccct cacc                                                          14

<210> 389
<211> 14
<212> DNA
<213> Artificial Sequence

<220>
<223> Synthetic Oligonucleotide

<400> 389
tggcagcagg aagc                                                          14

<210> 390
<211> 14
<212> DNA
<213> Artificial Sequence

<220>
<223> Synthetic Oligonucleotide

<400> 390
atggcagcag gaag                                                          14

<210> 391
<211> 14
<212> DNA
<213> Artificial Sequence

<220>
<223> Synthetic Oligonucleotide

<400> 391
catggcagca ggaa                                                          14

<210> 392
<211> 14
<212> DNA
<213> Artificial Sequence

<220>
<223> Synthetic Oligonucleotide

<400> 392
gcatggcagc agga                                                          14

<210> 393
<211> 14
<212> DNA
<213> Artificial Sequence

<220>
<223> Synthetic Oligonucleotide

<400> 393
ggcagcagat ggca                                                          14

<210> 394
<211> 14
<212> DNA
<213> Artificial Sequence

<220>
<223> Synthetic Oligonucleotide

<400> 394
cggcagcaga tggc                                                          14

<210> 395
<211> 14
<212> DNA
<213> Artificial Sequence

<220>

<223> Synthetic Oligonucleotide

<400> 395
ccggcagcag atgg                                                         14

<210> 396
<211> 14
<212> DNA
<213> Artificial Sequence

<220>
<223> Synthetic Oligonucleotide

<400> 396
tccggcagca gatg                                                         14

<210> 397
<211> 14
<212> DNA
<213> Artificial Sequence

<220>
<223> Synthetic Oligonucleotide

<400> 397
ctccggcagc agat                                                         14

<210> 398
<211> 14
<212> DNA
<213> Artificial Sequence

<220>
<223> Synthetic Oligonucleotide

<400> 398
gctccggcag caga                                                         14

<210> 399
<211> 14
<212> DNA
<213> Artificial Sequence

<220>
<223> Synthetic Oligonucleotide

<400> 399
ggctccggca gcag                                                         14

<210> 400
<211> 14
<212> DNA
<213> Artificial Sequence

<220>
<223> Synthetic Oligonucleotide

<400> 400
cggctccggc agca                                                         14

<210> 401
<211> 14
<212> DNA
<213> Artificial Sequence

<220>
<223> Synthetic Oligonucleotide

<400> 401
ccggctccgg cagc                                                          14

<210> 402
<211> 14
<212> DNA
<213> Artificial Sequence

<220>
<223> Synthetic Oligonucleotide

<400> 402
gccggctccg gcag                                                          14

<210> 403
<211> 14
<212> DNA
<213> Artificial Sequence

<220>
<223> Synthetic Oligonucleotide

<400> 403
agttacaaaa gcaa                                                          14

<210> 404
<211> 4
<212> DNA
<213> Artificial Sequence

<220>
<223> Synthetic Oligonucleotide

<400> 404
actg                                                                      4

<210> 405
<211> 4
<212> DNA
<213> Artificial Sequence

<220>
<223> Synthetic Oligonucleotide

<400> 405
actg                                                                      4

<210> 406
<211> 16
<212> DNA
<213> Artificial Sequence

<220>
<223> Synthetic Oligonucleotide

<400> 406
gtcgcccttc agcacg                                                        16

<210> 407
<211> 14
<212> DNA
<213> Artificial Sequence

<220>
<223> Synthetic Oligonucleotide

<400> 407
tcgcccttca gcac                                                          14

<210> 408
<211> 12
<212> DNA
<213> Artificial Sequence

<220>
<223> Synthetic Oligonucleotide

<400> 408
cgcccttcag ca                                                            12

<210> 409
<211> 16
<212> DNA
<213> Artificial Sequence

<220>
<223> Synthetic Oligonucleotide

<400> 409
actctggacc caaacc                                                        16

<210> 410
<211> 14
<212> DNA
<213> Artificial Sequence

<220>
<223> Synthetic Oligonucleotide

<400> 410
ctctggaccc aaac                                                          14

<210> 411
<211> 16
<212> DNA
<213> Artificial Sequence

<220>
<223> Synthetic Oligonucleotide

<400> 411
ccatttcagg agacct                                                        16

<210> 412
<211> 14
<212> DNA
<213> Artificial Sequence

<220>
<223> Synthetic Oligonucleotide

<400> 412
catttcagga gacc                                                          14

<210> 413
<211> 14
<212> DNA

<213> Artificial Sequence

<220>
<223> Synthetic Oligonucleotide

<400> 413
tttgggaggt ggtc                                                         14

<210> 414
<211> 14
<212> DNA
<213> Artificial Sequence

<220>
<223> Synthetic Oligonucleotide

<400> 414
cacaccaggc agag                                                         14

<210> 415
<211> 14
<212> DNA
<213> Artificial Sequence

<220>
<223> Synthetic Oligonucleotide

<400> 415
ctttacagct tcca                                                         14

<210> 416
<211> 14
<212> DNA
<213> Artificial Sequence

<220>
<223> Synthetic Oligonucleotide

<400> 416
cactaccttc cact                                                         14

<210> 417
<211> 14
<212> DNA
<213> Artificial Sequence

<220>
<223> Synthetic Oligonucleotide

<400> 417
aacacacact acct                                                         14

<210> 418
<211> 14
<212> DNA
<213> Artificial Sequence

<220>
<223> Synthetic Oligonucleotide

<400> 418
ctcttcaaaa caca                                                         14

<210> 419
<211> 14

<212> DNA
<213> Artificial Sequence

<220>
<223> Synthetic Oligonucleotide

<400> 419
gtaattgtgc tgtc                                                          14

<210> 420
<211> 14
<212> DNA
<213> Artificial Sequence

<220>
<223> Synthetic Oligonucleotide

<400> 420
tttttcttcg aatt                                                          14

<210> 421
<211> 14
<212> DNA
<213> Artificial Sequence

<220>
<223> Synthetic Oligonucleotide

<400> 421
cattttcgat agcg                                                          14

<210> 422
<211> 14
<212> DNA
<213> Artificial Sequence

<220>
<223> Synthetic Oligonucleotide

<400> 422
accttccagg ttca                                                          14

<210> 423
<211> 14
<212> DNA
<213> Artificial Sequence

<220>
<223> Synthetic Oligonucleotide

<400> 423
ataggaagca taaa                                                          14

<210> 424
<211> 14
<212> DNA
<213> Artificial Sequence

<220>
<223> Synthetic Oligonucleotide

<400> 424
tcttttaaag aaga                                                          14

<210> 425

<211> 14
<212> DNA
<213> Artificial Sequence

<220>
<223> Synthetic Oligonucleotide

<400> 425
aaggatattt taaa                                                         14

<210> 426
<211> 4
<212> DNA
<213> Artificial Sequence

<220>
<223> Synthetic Oligonucleotide

<400> 426
actg                                                                    4

<210> 427
<211> 14
<212> DNA
<213> Artificial Sequence

<220>
<223> Synthetic Oligonucleotide

<400> 427
gaacaaaaat taaa                                                         14

<210> 428
<211> 14
<212> DNA
<213> Artificial Sequence

<220>
<223> Synthetic Oligonucleotide

<400> 428
ttccacagat ctgt                                                         14

<210> 429
<211> 14
<212> DNA
<213> Artificial Sequence

<220>
<223> Synthetic Oligonucleotide

<400> 429
tttataaagt aaag                                                         14

<210> 430
<211> 4
<212> DNA
<213> Artificial Sequence

<220>
<223> Synthetic Oligonucleotide

<400> 430
actg                                                                    4

<210> 431
<211> 4
<212> DNA
<213> Artificial Sequence

<220>
<223> Synthetic Oligonucleotide

<400> 431
actg                                                                    4

<210> 432
<211> 4
<212> DNA
<213> Artificial Sequence

<220>
<223> Synthetic Oligonucleotide

<400> 432
actg                                                                    4

<210> 433
<211> 14
<212> DNA
<213> Artificial Sequence

<220>
<223> Synthetic Oligonucleotide

<400> 433
tactgtgaga aata                                                        14

<210> 434
<211> 4
<212> DNA
<213> Artificial Sequence

<220>
<223> Synthetic Oligonucleotide

<400> 434
actg                                                                    4

<210> 435
<211> 14
<212> DNA
<213> Artificial Sequence

<220>
<223> Synthetic Oligonucleotide

<400> 435
ttccagcttg aaga                                                        14

<210> 436
<211> 14
<212> DNA
<213> Artificial Sequence

<220>
<223> Synthetic Oligonucleotide

<400> 436
gatcagttct catg                                                        14

<210> 437
<211> 4
<212> DNA
<213> Artificial Sequence

<220>
<223> Synthetic Oligonucleotide

<400> 437
actg                                                                    4

<210> 438
<211> 4
<212> DNA
<213> Artificial Sequence

<220>
<223> Synthetic Oligonucleotide

<400> 438
actg                                                                    4

<210> 439
<211> 14
<212> DNA
<213> Artificial Sequence

<220>
<223> Synthetic Oligonucleotide

<400> 439
ttatcaatga tgca                                                        14

<210> 440
<211> 14
<212> DNA
<213> Artificial Sequence

<220>
<223> Synthetic Oligonucleotide

<400> 440
gcatgctgga cagt                                                        14

<210> 441
<211> 4
<212> DNA
<213> Artificial Sequence

<220>
<223> Synthetic Oligonucleotide

<400> 441
actg                                                                    4

<210> 442
<211> 4
<212> DNA
<213> Artificial Sequence

<220>
<223> Synthetic Oligonucleotide

<400> 442

actg                                                                    4

<210> 443
<211> 14
<212> DNA
<213> Artificial Sequence

<220>
<223> Synthetic Oligonucleotide

<400> 443
ttgcacctga acta                                                          14

<210> 444
<211> 14
<212> DNA
<213> Artificial Sequence

<220>
<223> Synthetic Oligonucleotide

<400> 444
cagaatatat ttct                                                          14

<210> 445
<211> 4
<212> DNA
<213> Artificial Sequence

<220>
<223> Synthetic Oligonucleotide

<400> 445
actg                                                                    4

<210> 446
<211> 4
<212> DNA
<213> Artificial Sequence

<220>
<223> Synthetic Oligonucleotide

<400> 446
actg                                                                    4

<210> 447
<211> 4
<212> DNA
<213> Artificial Sequence

<220>
<223> Synthetic Oligonucleotide

<400> 447
actg                                                                    4

<210> 448
<211> 4
<212> DNA
<213> Artificial Sequence

<220>
<223> Synthetic Oligonucleotide

<400> 448
actg                                                                    4

<210> 449
<211> 4
<212> DNA
<213> Artificial Sequence

<220>
<223> Synthetic Oligonucleotide

<400> 449
actg                                                                    4

<210> 450
<211> 14
<212> DNA
<213> Artificial Sequence

<220>
<223> Synthetic Oligonucleotide

<400> 450
ataagagatt aaaa                                                        14

<210> 451
<211> 14
<212> DNA
<213> Artificial Sequence

<220>
<223> Synthetic Oligonucleotide

<400> 451
   tcccccttct catt                                                         14

<210> 452
<211> 14
<212> DNA
<213> Artificial Sequence

<220>
<223> Synthetic Oligonucleotide

<400> 452
gggcattgtt aaaa                                                        14

<210> 453
<211> 4
<212> DNA
<213> Artificial Sequence

<220>
<223> Synthetic Oligonucleotide

<400> 453
actg                                                                    4

<210> 454
<211> 4
<212> DNA
<213> Artificial Sequence

<220>
<223> Synthetic Oligonucleotide

<400> 454
actg                                                                    4

<210> 455
<211> 14
<212> DNA
<213> Artificial Sequence

<220>
<223> Synthetic Oligonucleotide

<400> 455
agaactcaca tctg                                                        14

<210> 456
<211> 14
<212> DNA
<213> Artificial Sequence

<220>
<223> Synthetic Oligonucleotide

<400> 456
gagctggacg gagg                                                        14

<210> 457
<211> 14
<212> DNA
<213> Artificial Sequence

<220>
<223> Synthetic Oligonucleotide

<400> 457
aagcttcatc ggag                                                        14

<210> 458
<211> 14
<212> DNA
<213> Artificial Sequence

<220>
<223> Synthetic Oligonucleotide

<400> 458
ataatggcat cccg                                                        14

<210> 459
<211> 4
<212> DNA
<213> Artificial Sequence

<220>
<223> Synthetic Oligonucleotide

<400> 459
actg                                                                    4

<210> 460
<211> 14
<212> DNA
<213> Artificial Sequence

<220>

<223> Synthetic Oligonucleotide

<400> 460
tgctgtccta taag                                                         14

<210> 461
<211> 14
<212> DNA
<213> Artificial Sequence

<220>
<223> Synthetic Oligonucleotide

<400> 461
cacaaaggta attg                                                         14

<210> 462
<211> 14
<212> DNA
<213> Artificial Sequence

<220>
<223> Synthetic Oligonucleotide

<400> 462
atcatttctt ccag                                                         14

<210> 463
<211> 4
<212> DNA
<213> Artificial Sequence

<220>
<223> Synthetic Oligonucleotide

<400> 463
actg                                                                     4

<210> 464
<211> 4
<212> DNA
<213> Artificial Sequence

<220>
<223> Synthetic Oligonucleotide

<400> 464
actg                                                                     4

<210> 465
<211> 4
<212> DNA
<213> Artificial Sequence

<220>
<223> Synthetic Oligonucleotide

<400> 465
actg                                                                     4

<210> 466
<211> 14
<212> DNA
<213> Artificial Sequence

<220>
<223> Synthetic Oligonucleotide

<400> 466
ccattacctt ccag                                                         14

<210> 467
<211> 14
<212> DNA
<213> Artificial Sequence

<220>
<223> Synthetic Oligonucleotide

<400> 467
gcataaacag ggtt                                                         14

<210> 468
<211> 4
<212> DNA
<213> Artificial Sequence

<220>
<223> Synthetic Oligonucleotide

<400> 468
actg                                                                     4

<210> 469
<211> 4
<212> DNA
<213> Artificial Sequence

<220>
<223> Synthetic Oligonucleotide

<400> 469
actg                                                                     4

<210> 470
<211> 4
<212> DNA
<213> Artificial Sequence

<220>
<223> Synthetic Oligonucleotide

<400> 470
actg                                                                     4

<210> 471
<211> 4
<212> DNA
<213> Artificial Sequence

<220>
<223> Synthetic Oligonucleotide

<400> 471
actg                                                                     4

<210> 472
<211> 14
<212> DNA
<213> Artificial Sequence

<220>
<223> Synthetic Oligonucleotide

<400> 472
tatgaaagga atgt                                                         14

<210> 473
<211> 4
<212> DNA
<213> Artificial Sequence

<220>
<223> Synthetic Oligonucleotide

<400> 473
actg                                                                    4

<210> 474
<211> 14
<212> DNA
<213> Artificial Sequence

<220>
<223> Synthetic Oligonucleotide

<400> 474
ttccttaagc ttcc                                                         14

<210> 475
<211> 4
<212> DNA
<213> Artificial Sequence

<220>
<223> Synthetic Oligonucleotide

<400> 475
actg                                                                    4

<210> 476
<211> 4
<212> DNA
<213> Artificial Sequence

<220>
<223> Synthetic Oligonucleotide

<400> 476
actg                                                                    4

<210> 477
<211> 4
<212> DNA
<213> Artificial Sequence

<220>
<223> Synthetic Oligonucleotide

<400> 477
actg                                                                    4

<210> 478
<211> 4
<212> DNA

<213> Artificial Sequence

<220>
<223> Synthetic Oligonucleotide

<400> 478
actg                                                                    4

<210> 479
<211> 4
<212> DNA
<213> Artificial Sequence

<220>
<223> Synthetic Oligonucleotide

<400> 479
actg                                                                    4

<210> 480
<211> 4
<212> DNA
<213> Artificial Sequence

<220>
<223> Synthetic Oligonucleotide

<400> 480
actg                                                                    4

<210> 481
<211> 4
<212> DNA
<213> Artificial Sequence

<220>
<223> Synthetic Oligonucleotide

<400> 481
actg                                                                    4

<210> 482
<211> 4
<212> DNA
<213> Artificial Sequence

<220>
<223> Synthetic Oligonucleotide

<400> 482
actg                                                                    4

<210> 483
<211> 4
<212> DNA
<213> Artificial Sequence

<220>
<223> Synthetic Oligonucleotide

<400> 483
actg                                                                    4

<210> 484
<211> 4

<212> DNA
<213> Artificial Sequence

<220>
<223> Synthetic Oligonucleotide

<400> 484
actg                                                                    4

<210> 485
<211> 14
<212> DNA
<213> Artificial Sequence

<220>
<223> Synthetic Oligonucleotide

<400> 485
gaacagttaa acat                                                        14

<210> 486
<211> 16
<212> DNA
<213> Artificial Sequence

<220>
<223> Synthetic Oligonucleotide

<400> 486
tagagcttcc acttct                                                      16

<210> 487
<211> 14
<212> DNA
<213> Artificial Sequence

<220>
<223> Synthetic Oligonucleotide

<400> 487
gagcttccac ttct                                                        14

<210> 488
<211> 14
<212> DNA
<213> Artificial Sequence

<220>
<223> Synthetic Oligonucleotide

<400> 488
tgttggccgt ggta                                                        14

<210> 489
<211> 14
<212> DNA
<213> Artificial Sequence

<220>
<223> Synthetic Oligonucleotide

<400> 489
atgttggccg tggt                                                        14

<210> 490

<211> 14
<212> DNA
<213> Artificial Sequence

<220>
<223> Synthetic Oligonucleotide

<400> 490
gatgttggcc gtgg                                                          14

<210> 491
<211> 14
<212> DNA
<213> Artificial Sequence

<220>
<223> Synthetic Oligonucleotide

<400> 491
agatgttggc cgtg                                                          14

<210> 492
<211> 14
<212> DNA
<213> Artificial Sequence

<220>
<223> Synthetic Oligonucleotide

<400> 492
gagatgttgg ccgt                                                          14

<210> 493
<211> 14
<212> DNA
<213> Artificial Sequence

<220>
<223> Synthetic Oligonucleotide

<400> 493
ggagatgttg gccg                                                          14

<210> 494
<211> 14
<212> DNA
<213> Artificial Sequence

<220>
<223> Synthetic Oligonucleotide

<400> 494
aggagatgtt ggcc                                                          14

<210> 495
<211> 14
<212> DNA
<213> Artificial Sequence

<220>
<223> Synthetic Oligonucleotide

<400> 495
caggagatgt tggc                                                          14

<210> 496
<211> 14
<212> DNA
<213> Artificial Sequence

<220>
<223> Synthetic Oligonucleotide

<400> 496
gcaggagatg ttgg                                                          14

<210> 497
<211> 14
<212> DNA
<213> Artificial Sequence

<220>
<223> Synthetic Oligonucleotide

<400> 497
ggcaggagat gttg                                                          14

<210> 498
<211> 14
<212> DNA
<213> Artificial Sequence

<220>
<223> Synthetic Oligonucleotide

<400> 498
gtggtgccaa ggca                                                          14

<210> 499
<211> 14
<212> DNA
<213> Artificial Sequence

<220>
<223> Synthetic Oligonucleotide

<400> 499
tgtggtgcca aggc                                                          14

<210> 500
<211> 14
<212> DNA
<213> Artificial Sequence

<220>
<223> Synthetic Oligonucleotide

<400> 500
ttgtggtgcc aagg                                                          14

<210> 501
<211> 14
<212> DNA
<213> Artificial Sequence

<220>
<223> Synthetic Oligonucleotide

<400> 501
tttgtggtgc caag                                                          14

<210> 502
<211> 14
<212> DNA
<213> Artificial Sequence

<220>
<223> Synthetic Oligonucleotide

<400> 502
ctttgtggtg ccaa                                                         14

<210> 503
<211> 14
<212> DNA
<213> Artificial Sequence

<220>
<223> Synthetic Oligonucleotide

<400> 503
actttgtggt gcca                                                         14

<210> 504
<211> 14
<212> DNA
<213> Artificial Sequence

<220>
<223> Synthetic Oligonucleotide

<400> 504
cactttgtgg tgcc                                                         14

<210> 505
<211> 14
<212> DNA
<213> Artificial Sequence

<220>
<223> Synthetic Oligonucleotide

<400> 505
gcactttgtg gtgc                                                         14

<210> 506
<211> 14
<212> DNA
<213> Artificial Sequence

<220>
<223> Synthetic Oligonucleotide

<400> 506
tgcactttgt ggtg                                                         14

<210> 507
<211> 14
<212> DNA
<213> Artificial Sequence

<220>
<223> Synthetic Oligonucleotide

<400> 507

ttgcactttg tggt                                                          14

<210> 508
<211> 14
<212> DNA
<213> Artificial Sequence

<220>
<223> Synthetic Oligonucleotide

<400> 508
cggtgttgca cttt                                                          14

<210> 509
<211> 14
<212> DNA
<213> Artificial Sequence

<220>
<223> Synthetic Oligonucleotide

<400> 509
gcggtgttgc actt                                                          14

<210> 510
<211> 14
<212> DNA
<213> Artificial Sequence

<220>
<223> Synthetic Oligonucleotide

<400> 510
agcggtgttg cact                                                          14

<210> 511
<211> 14
<212> DNA
<213> Artificial Sequence

<220>
<223> Synthetic Oligonucleotide

<400> 511
aagcggtgtt gcac                                                          14

<210> 512
<211> 14
<212> DNA
<213> Artificial Sequence

<220>
<223> Synthetic Oligonucleotide

<400> 512
gaagcggtgt tgca                                                          14

<210> 513
<211> 14
<212> DNA
<213> Artificial Sequence

<220>
<223> Synthetic Oligonucleotide

<400> 513
cgaagcggtg ttgc                                                          14

<210> 514
<211> 14
<212> DNA
<213> Artificial Sequence

<220>
<223> Synthetic Oligonucleotide

<400> 514
acgaagcggt gttg                                                          14

<210> 515
<211> 14
<212> DNA
<213> Artificial Sequence

<220>
<223> Synthetic Oligonucleotide

<400> 515
cacgaagcgg tgtt                                                          14

<210> 516
<211> 14
<212> DNA
<213> Artificial Sequence

<220>
<223> Synthetic Oligonucleotide

<400> 516
acacgaagcg gtgt                                                          14

<210> 517
<211> 14
<212> DNA
<213> Artificial Sequence

<220>
<223> Synthetic Oligonucleotide

<400> 517
aacacgaagc ggtg                                                          14

<210> 518
<211> 14
<212> DNA
<213> Artificial Sequence

<220>
<223> Synthetic Oligonucleotide

<400> 518
gctgctgtac atct                                                          14

<210> 519
<211> 4
<212> DNA
<213> Artificial Sequence

<220>
<223> Synthetic Oligonucleotide

<400> 519
actg                                                                    4

<210> 520
<211> 14
<212> DNA
<213> Artificial Sequence

<220>
<223> Synthetic Oligonucleotide

<400> 520
agctgctgta catc                                                        14

<210> 521
<211> 14
<212> DNA
<213> Artificial Sequence

<220>
<223> Synthetic Oligonucleotide

<400> 521
aagctgctgt acat                                                        14

<210> 522
<211> 14
<212> DNA
<213> Artificial Sequence

<220>
<223> Synthetic Oligonucleotide

<400> 522
gaagctgctg taca                                                        14

<210> 523
<211> 14
<212> DNA
<213> Artificial Sequence

<220>
<223> Synthetic Oligonucleotide

<400> 523
ggaagctgct gtac                                                        14

<210> 524
<211> 14
<212> DNA
<213> Artificial Sequence

<220>
<223> Synthetic Oligonucleotide

<400> 524
tggaagctgc tgta                                                        14

<210> 525
<211> 14
<212> DNA
<213> Artificial Sequence

<220>

<223> Synthetic Oligonucleotide

<400> 525
ctggaagctg ctgt                                                         14

<210> 526
<211> 14
<212> DNA
<213> Artificial Sequence

<220>
<223> Synthetic Oligonucleotide

<400> 526
cctggaagct gctg                                                         14

<210> 527
<211> 14
<212> DNA
<213> Artificial Sequence

<220>
<223> Synthetic Oligonucleotide

<400> 527
acctggaagc tgct                                                         14

<210> 528
<211> 14
<212> DNA
<213> Artificial Sequence

<220>
<223> Synthetic Oligonucleotide

<400> 528
cacctggaag ctgc                                                         14

<210> 529
<211> 14
<212> DNA
<213> Artificial Sequence

<220>
<223> Synthetic Oligonucleotide

<400> 529
tcacctggaa gctg                                                         14

<210> 530
<211> 14
<212> DNA
<213> Artificial Sequence

<220>
<223> Synthetic Oligonucleotide

<400> 530
atcacctgga agct                                                         14

<210> 531
<211> 14
<212> DNA
<213> Artificial Sequence

<220>
<223> Synthetic Oligonucleotide

<400> 531
catcacctgg aagc                                                         14

<210> 532
<211> 14
<212> DNA
<213> Artificial Sequence

<220>
<223> Synthetic Oligonucleotide

<400> 532
acatcacctg gaag                                                         14

<210> 533
<211> 14
<212> DNA
<213> Artificial Sequence

<220>
<223> Synthetic Oligonucleotide

<400> 533
tacatcacct ggaa                                                         14

<210> 534
<211> 14
<212> DNA
<213> Artificial Sequence

<220>
<223> Synthetic Oligonucleotide

<400> 534
gtacatcacc tgga                                                         14

<210> 535
<211> 14
<212> DNA
<213> Artificial Sequence

<220>
<223> Synthetic Oligonucleotide

<400> 535
tgtacatcac ctgg                                                         14

<210> 536
<211> 14
<212> DNA
<213> Artificial Sequence

<220>
<223> Synthetic Oligonucleotide

<400> 536
gtgtacatca cctg                                                         14

<210> 537
<211> 14
<212> DNA
<213> Artificial Sequence

<220>
<223> Synthetic Oligonucleotide

<400> 537
tagcgggtcc tgag                                                          14

<210> 538
<211> 14
<212> DNA
<213> Artificial Sequence

<220>
<223> Synthetic Oligonucleotide

<400> 538
gtagcgggtc ctga                                                          14

<210> 539
<211> 14
<212> DNA
<213> Artificial Sequence

<220>
<223> Synthetic Oligonucleotide

<400> 539
tgtagcgggt cctg                                                          14

<210> 540
<211> 14
<212> DNA
<213> Artificial Sequence

<220>
<223> Synthetic Oligonucleotide

<400> 540
ctgtagcggg tcct                                                          14

<210> 541
<211> 14
<212> DNA
<213> Artificial Sequence

<220>
<223> Synthetic Oligonucleotide

<400> 541
gctgtagcgg gtcc                                                          14

<210> 542
<211> 14
<212> DNA
<213> Artificial Sequence

<220>
<223> Synthetic Oligonucleotide

<400> 542
ggctgtagcg ggtc                                                          14

<210> 543
<211> 14
<212> DNA

<213> Artificial Sequence

<220>
<223> Synthetic Oligonucleotide

<400> 543
tggctgtagc gggt                                                         14

<210> 544
<211> 14
<212> DNA
<213> Artificial Sequence

<220>
<223> Synthetic Oligonucleotide

<400> 544
ctggctgtag cggg                                                         14

<210> 545
<211> 14
<212> DNA
<213> Artificial Sequence

<220>
<223> Synthetic Oligonucleotide

<400> 545
tctggctgta gcgg                                                         14

<210> 546
<211> 14
<212> DNA
<213> Artificial Sequence

<220>
<223> Synthetic Oligonucleotide

<400> 546
ttctggctgt agcg                                                         14

<210> 547
<211> 14
<212> DNA
<213> Artificial Sequence

<220>
<223> Synthetic Oligonucleotide

<400> 547
tgaacaccgc ggcc                                                         14

<210> 548
<211> 14
<212> DNA
<213> Artificial Sequence

<220>
<223> Synthetic Oligonucleotide

<400> 548
atgaacaccg cggc                                                         14

<210> 549
<211> 14

<212> DNA
<213> Artificial Sequence

<220>
<223> Synthetic Oligonucleotide

<400> 549
catgaacacc gcgg                                                         14

<210> 550
<211> 14
<212> DNA
<213> Artificial Sequence

<220>
<223> Synthetic Oligonucleotide

<400> 550
gcatgaacac cgcg                                                         14

<210> 551
<211> 14
<212> DNA
<213> Artificial Sequence

<220>
<223> Synthetic Oligonucleotide

<400> 551
tgcatgaaca ccgc                                                         14

<210> 552
<211> 14
<212> DNA
<213> Artificial Sequence

<220>
<223> Synthetic Oligonucleotide

<400> 552
ttgcatgaac accg                                                         14

<210> 553
<211> 14
<212> DNA
<213> Artificial Sequence

<220>
<223> Synthetic Oligonucleotide

<400> 553
attgcatgaa cacc                                                         14

<210> 554
<211> 14
<212> DNA
<213> Artificial Sequence

<220>
<223> Synthetic Oligonucleotide

<400> 554
tattgcatga acac                                                         14

<210> 555

<211> 14
<212> DNA
<213> Artificial Sequence

<220>
<223> Synthetic Oligonucleotide

<400> 555
atattgcatg aaca                                                          14

<210> 556
<211> 14
<212> DNA
<213> Artificial Sequence

<220>
<223> Synthetic Oligonucleotide

<400> 556
catattgcat gaac                                                          14

<210> 557
<211> 14
<212> DNA
<213> Artificial Sequence

<220>
<223> Synthetic Oligonucleotide

<400> 557
aggttgtgca ggta                                                          14

<210> 558
<211> 14
<212> DNA
<213> Artificial Sequence

<220>
<223> Synthetic Oligonucleotide

<400> 558
caggttgtgc aggt                                                          14

<210> 559
<211> 14
<212> DNA
<213> Artificial Sequence

<220>
<223> Synthetic Oligonucleotide

<400> 559
gcaggttgtg cagg                                                          14

<210> 560
<211> 14
<212> DNA
<213> Artificial Sequence

<220>
<223> Synthetic Oligonucleotide

<400> 560
agcaggttgt gcag                                                          14

<210> 561
<211> 14
<212> DNA
<213> Artificial Sequence

<220>
<223> Synthetic Oligonucleotide

<400> 561
cagcaggttg tgca                                                         14

<210> 562
<211> 14
<212> DNA
<213> Artificial Sequence

<220>
<223> Synthetic Oligonucleotide

<400> 562
ccagcaggtt gtgc                                                         14

<210> 563
<211> 14
<212> DNA
<213> Artificial Sequence

<220>
<223> Synthetic Oligonucleotide

<400> 563
cccagcaggt tgtg                                                         14

<210> 564
<211> 14
<212> DNA
<213> Artificial Sequence

<220>
<223> Synthetic Oligonucleotide

<400> 564
gcccagcagg ttgt                                                         14

<210> 565
<211> 14
<212> DNA
<213> Artificial Sequence

<220>
<223> Synthetic Oligonucleotide

<400> 565
ggcccagcag gttg                                                         14

<210> 566
<211> 14
<212> DNA
<213> Artificial Sequence

<220>
<223> Synthetic Oligonucleotide

<400> 566
aggcccagca ggtt                                                         14

<210> 567
<211> 16
<212> DNA
<213> Artificial Sequence

<220>
<223> Synthetic Oligonucleotide

<400> 567
tgtcattgct ggtcca                                                       16

<210> 568
<211> 14
<212> DNA
<213> Artificial Sequence

<220>
<223> Synthetic Oligonucleotide

<400> 568
tcattgctgg tcca                                                         14

<210> 569
<211> 16
<212> DNA
<213> Artificial Sequence

<220>
<223> Synthetic Oligonucleotide

<400> 569
tggccagccg gaactt                                                       16

<210> 570
<211> 14
<212> DNA
<213> Artificial Sequence

<220>
<223> Synthetic Oligonucleotide

<400> 570
gccagccgga actt                                                         14

<210> 571
<211> 16
<212> DNA
<213> Artificial Sequence

<220>
<223> Synthetic Oligonucleotide

<400> 571
gggatgaggg tcagcg                                                       16

<210> 572
<211> 14
<212> DNA
<213> Artificial Sequence

<220>
<223> Synthetic Oligonucleotide

<400> 572

gatgagggtc agcg                                                          14

<210> 573
<211> 14
<212> DNA
<213> Artificial Sequence

<220>
<223> Synthetic Oligonucleotide

<400> 573
aaggcaaaga ccac                                                          14

<210> 574
<211> 14
<212> DNA
<213> Artificial Sequence

<220>
<223> Synthetic Oligonucleotide

<400> 574
ggagcgcagg gtgc                                                          14

<210> 575
<211> 14
<212> DNA
<213> Artificial Sequence

<220>
<223> Synthetic Oligonucleotide

<400> 575
tgcacctcct tgtt                                                          14

<210> 576
<211> 14
<212> DNA
<213> Artificial Sequence

<220>
<223> Synthetic Oligonucleotide

<400> 576
cagcagaccc tgga                                                          14

<210> 577
<211> 16
<212> DNA
<213> Artificial Sequence

<220>
<223> Synthetic Oligonucleotide

<400> 577
acatctggca gaggtt                                                        16

<210> 578
<211> 14
<212> DNA
<213> Artificial Sequence

<220>
<223> Synthetic Oligonucleotide

<400> 578
atctggcaga ggtt                                                          14

<210> 579
<211> 16
<212> DNA
<213> Artificial Sequence

<220>
<223> Synthetic Oligonucleotide

<400> 579
caggccagca ggagta                                                        16

<210> 580
<211> 14
<212> DNA
<213> Artificial Sequence

<220>
<223> Synthetic Oligonucleotide

<400> 580
ggccagcagg agta                                                          14

<210> 581
<211> 16
<212> DNA
<213> Artificial Sequence

<220>
<223> Synthetic Oligonucleotide

<400> 581
ctcaaacaaa aagtcc                                                        16

<210> 582
<211> 14
<212> DNA
<213> Artificial Sequence

<220>
<223> Synthetic Oligonucleotide

<400> 582
caaacaaaaa gtcc                                                          14

<210> 583
<211> 16
<212> DNA
<213> Artificial Sequence

<220>
<223> Synthetic Oligonucleotide

<400> 583
gtggtgacat tggtca                                                        16

<210> 584
<211> 14
<212> DNA
<213> Artificial Sequence

<220>
<223> Synthetic Oligonucleotide

<400> 584
ggtgacattg gtca                                                          14

<210> 585
<211> 4
<212> DNA
<213> Artificial Sequence

<220>
<223> Synthetic Oligonucleotide

<400> 585
actg                                                                      4

<210> 586
<211> 4
<212> DNA
<213> Artificial Sequence

<220>
<223> Synthetic Oligonucleotide

<400> 586
actg                                                                      4

<210> 587
<211> 14
<212> DNA
<213> Artificial Sequence

<220>
<223> Synthetic Oligonucleotide

<400> 587
ttggcagtgg tgtt                                                          14

<210> 588
<211> 14
<212> DNA
<213> Artificial Sequence

<220>
<223> Synthetic Oligonucleotide

<400> 588
atgttggcag tggt                                                          14

<210> 589
<211> 16
<212> DNA
<213> Artificial Sequence

<220>
<223> Synthetic Oligonucleotide

<400> 589
aggaaatgtt ggcagt                                                        16

<210> 590
<211> 14
<212> DNA
<213> Artificial Sequence

<220>

<223> Synthetic Oligonucleotide

<400> 590
gaaatgttgg cagt                                                          14

<210> 591
<211> 14
<212> DNA
<213> Artificial Sequence

<220>
<223> Synthetic Oligonucleotide

<400> 591
caggaaatgt tggc                                                          14

<210> 592
<211> 14
<212> DNA
<213> Artificial Sequence

<220>
<223> Synthetic Oligonucleotide

<400> 592
gggcaggaaa tgtt                                                          14

<210> 593
<211> 14
<212> DNA
<213> Artificial Sequence

<220>
<223> Synthetic Oligonucleotide

<400> 593
aaggtaggta ccag                                                          14

<210> 594
<211> 14
<212> DNA
<213> Artificial Sequence

<220>
<223> Synthetic Oligonucleotide

<400> 594
accaaggtag gtac                                                          14

<210> 595
<211> 14
<212> DNA
<213> Artificial Sequence

<220>
<223> Synthetic Oligonucleotide

<400> 595
ctttgtggca ccaa                                                          14

<210> 596
<211> 14
<212> DNA
<213> Artificial Sequence

<220>
<223> Synthetic Oligonucleotide

<400> 596
gcactttgtg gcac                                                          14

<210> 597
<211> 14
<212> DNA
<213> Artificial Sequence

<220>
<223> Synthetic Oligonucleotide

<400> 597
tgcactttgt ggca                                                          14

<210> 598
<211> 14
<212> DNA
<213> Artificial Sequence

<220>
<223> Synthetic Oligonucleotide

<400> 598
gctgcacttt gtgg                                                          14

<210> 599
<211> 14
<212> DNA
<213> Artificial Sequence

<220>
<223> Synthetic Oligonucleotide

<400> 599
ggtgctgcac tttg                                                          14

<210> 600
<211> 14
<212> DNA
<213> Artificial Sequence

<220>
<223> Synthetic Oligonucleotide

<400> 600
ggcggtgctg cact                                                          14

<210> 601
<211> 14
<212> DNA
<213> Artificial Sequence

<220>
<223> Synthetic Oligonucleotide

<400> 601
tgaacactag gcgg                                                          14

<210> 602
<211> 14
<212> DNA
<213> Artificial Sequence

<220>
<223> Synthetic Oligonucleotide

<400> 602
tcttgaacac tagg                                                         14

<210> 603
<211> 16
<212> DNA
<213> Artificial Sequence

<220>
<223> Synthetic Oligonucleotide

<400> 603
cacctcttga acacta                                                       16

<210> 604
<211> 14
<212> DNA
<213> Artificial Sequence

<220>
<223> Synthetic Oligonucleotide

<400> 604
cctcttgaac acta                                                         14

<210> 605
<211> 14
<212> DNA
<213> Artificial Sequence

<220>
<223> Synthetic Oligonucleotide

<400> 605
acctcttgaa cact                                                         14

<210> 606
<211> 14
<212> DNA
<213> Artificial Sequence

<220>
<223> Synthetic Oligonucleotide

<400> 606
cacacctctt gaac                                                         14

<210> 607
<211> 14
<212> DNA
<213> Artificial Sequence

<220>
<223> Synthetic Oligonucleotide

<400> 607
cctcgaaccc actg                                                         14

<210> 608
<211> 16
<212> DNA

<213> Artificial Sequence

<220>
<223> Synthetic Oligonucleotide

<400> 608
cttctggacc tcgatc                                                        16

<210> 609
<211> 14
<212> DNA
<213> Artificial Sequence

<220>
<223> Synthetic Oligonucleotide

<400> 609
tctggacctc gatc                                                          14

<210> 610
<211> 16
<212> DNA
<213> Artificial Sequence

<220>
<223> Synthetic Oligonucleotide

<400> 610
ctgctataca tcttgg                                                        16

<210> 611
<211> 14
<212> DNA
<213> Artificial Sequence

<220>
<223> Synthetic Oligonucleotide

<400> 611
gctatacatc ttgg                                                          14

<210> 612
<211> 16
<212> DNA
<213> Artificial Sequence

<220>
<223> Synthetic Oligonucleotide

<400> 612
cacggtgtac atcacc                                                        16

<210> 613
<211> 14
<212> DNA
<213> Artificial Sequence

<220>
<223> Synthetic Oligonucleotide

<400> 613
cggtgtacat cacc                                                          14

<210> 614
<211> 16

<212> DNA
<213> Artificial Sequence

<220>
<223> Synthetic Oligonucleotide

<400> 614
ggacagactg tagccc                                                       16

<210> 615
<211> 14
<212> DNA
<213> Artificial Sequence

<220>
<223> Synthetic Oligonucleotide

<400> 615
acagactgta gccc                                                         14

<210> 616
<211> 16
<212> DNA
<213> Artificial Sequence

<220>
<223> Synthetic Oligonucleotide

<400> 616
gtcatcgcca atcttc                                                       16

<210> 617
<211> 14
<212> DNA
<213> Artificial Sequence

<220>
<223> Synthetic Oligonucleotide

<400> 617
catcgccaat cttc                                                         14

<210> 618
<211> 16
<212> DNA
<213> Artificial Sequence

<220>
<223> Synthetic Oligonucleotide

<400> 618
tcacactgag gtcatc                                                       16

<210> 619
<211> 14
<212> DNA
<213> Artificial Sequence

<220>
<223> Synthetic Oligonucleotide

<400> 619
acactgaggt catc                                                         14

<210> 620

<211> 14
<212> DNA
<213> Artificial Sequence

<220>
<223> Synthetic Oligonucleotide

<400> 620
cgccccgtca ctga                                                         14

<210> 621
<211> 16
<212> DNA
<213> Artificial Sequence

<220>
<223> Synthetic Oligonucleotide

<400> 621
ccagcaacca gcaata                                                       16

<210> 622
<211> 14
<212> DNA
<213> Artificial Sequence

<220>
<223> Synthetic Oligonucleotide

<400> 622
agcaaccagc aata                                                         14

<210> 623
<211> 16
<212> DNA
<213> Artificial Sequence

<220>
<223> Synthetic Oligonucleotide

<400> 623
caggctgtac aggtac                                                       16

<210> 624
<211> 14
<212> DNA
<213> Artificial Sequence

<220>
<223> Synthetic Oligonucleotide

<400> 624
ggctgtacag gtac                                                         14

<210> 625
<211> 16
<212> DNA
<213> Artificial Sequence

<220>
<223> Synthetic Oligonucleotide

<400> 625
gaagctcctc tcagag                                                       16

<210> 626
<211> 14
<212> DNA
<213> Artificial Sequence

<220>
<223> Synthetic Oligonucleotide

<400> 626
agctcctctc agag                                                         14

<210> 627
<211> 16
<212> DNA
<213> Artificial Sequence

<220>
<223> Synthetic Oligonucleotide

<400> 627
cccagccaat gcccag                                                       16

<210> 628
<211> 14
<212> DNA
<213> Artificial Sequence

<220>
<223> Synthetic Oligonucleotide

<400> 628
cagccaatgc ccag                                                         14

<210> 629
<211> 16
<212> DNA
<213> Artificial Sequence

<220>
<223> Synthetic Oligonucleotide

<400> 629
tcaaacagac acttga                                                       16

<210> 630
<211> 14
<212> DNA
<213> Artificial Sequence

<220>
<223> Synthetic Oligonucleotide

<400> 630
aaacagacac ttga                                                         14

<210> 631
<211> 16
<212> DNA
<213> Artificial Sequence

<220>
<223> Synthetic Oligonucleotide

<400> 631
agcactgaac attctc                                                       16

<210> 632
<211> 14
<212> DNA
<213> Artificial Sequence

<220>
<223> Synthetic Oligonucleotide

<400> 632
cactgaacat tctc                                                          14

<210> 633
<211> 16
<212> DNA
<213> Artificial Sequence

<220>
<223> Synthetic Oligonucleotide

<400> 633
ggatccacca gaatcc                                                        16

<210> 634
<211> 14
<212> DNA
<213> Artificial Sequence

<220>
<223> Synthetic Oligonucleotide

<400> 634
atccaccaga atcc                                                          14

<210> 635
<211> 14
<212> DNA
<213> Artificial Sequence

<220>
<223> Synthetic Oligonucleotide

<400> 635
tgatcagtaa ggcc                                                          14

<210> 636
<211> 16
<212> DNA
<213> Artificial Sequence

<220>
<223> Synthetic Oligonucleotide

<400> 636
ggacaaagat gaaaaa                                                        16

<210> 637
<211> 14
<212> DNA
<213> Artificial Sequence

<220>
<223> Synthetic Oligonucleotide

<400> 637

acaaagatga aaaa                                                          14

<210> 638
<211> 16
<212> DNA
<213> Artificial Sequence

<220>
<223> Synthetic Oligonucleotide

<400> 638
ggcacgcagc ttggcc                                                        16

<210> 639
<211> 14
<212> DNA
<213> Artificial Sequence

<220>
<223> Synthetic Oligonucleotide

<400> 639
cacgcagctt ggcc                                                          14

<210> 640
<211> 16
<212> DNA
<213> Artificial Sequence

<220>
<223> Synthetic Oligonucleotide

<400> 640
tggaccccca gcagag                                                        16

<210> 641
<211> 14
<212> DNA
<213> Artificial Sequence

<220>
<223> Synthetic Oligonucleotide

<400> 641
gacccccagc agag                                                          14

<210> 642
<211> 14
<212> DNA
<213> Artificial Sequence

<220>
<223> Synthetic Oligonucleotide

<400> 642
caaaggcaaa gacc                                                          14

<210> 643
<211> 14
<212> DNA
<213> Artificial Sequence

<220>
<223> Synthetic Oligonucleotide

<400> 643
tcacaaaggc aaag                                                          14

<210> 644
<211> 14
<212> DNA
<213> Artificial Sequence

<220>
<223> Synthetic Oligonucleotide

<400> 644
cagtcacaaa ggca                                                          14

<210> 645
<211> 14
<212> DNA
<213> Artificial Sequence

<220>
<223> Synthetic Oligonucleotide

<400> 645
cgtcagtcac aaag                                                          14

<210> 646
<211> 14
<212> DNA
<213> Artificial Sequence

<220>
<223> Synthetic Oligonucleotide

<400> 646
gctcgtcagt caca                                                          14

<210> 647
<211> 14
<212> DNA
<213> Artificial Sequence

<220>
<223> Synthetic Oligonucleotide

<400> 647
catgctcgtc agtc                                                          14

<210> 648
<211> 14
<212> DNA
<213> Artificial Sequence

<220>
<223> Synthetic Oligonucleotide

<400> 648
gggcatgctc gtca                                                          14

<210> 649
<211> 4
<212> DNA
<213> Artificial Sequence

<220>
<223> Synthetic Oligonucleotide

<400> 649
actg                                                                    4

<210> 650
<211> 4
<212> DNA
<213> Artificial Sequence

<220>
<223> Synthetic Oligonucleotide

<400> 650
actg                                                                    4

<210> 651
<211> 4
<212> DNA
<213> Artificial Sequence

<220>
<223> Synthetic Oligonucleotide

<400> 651
actg                                                                    4

<210> 652
<211> 4
<212> DNA
<213> Artificial Sequence

<220>
<223> Synthetic Oligonucleotide

<400> 652
actg                                                                    4

<210> 653
<211> 4
<212> DNA
<213> Artificial Sequence

<220>
<223> Synthetic Oligonucleotide

<400> 653
actg                                                                    4

<210> 654
<211> 14
<212> DNA
<213> Artificial Sequence

<220>
<223> Synthetic Oligonucleotide

<400> 654
cttgggcatg ctcg                                                        14

<210> 655
<211> 4
<212> DNA
<213> Artificial Sequence

<220>

<223> Synthetic Oligonucleotide

<400> 655
actg                                                                    4

<210> 656
<211> 4
<212> DNA
<213> Artificial Sequence

<220>
<223> Synthetic Oligonucleotide

<400> 656
actg                                                                    4

<210> 657
<211> 14
<212> DNA
<213> Artificial Sequence

<220>
<223> Synthetic Oligonucleotide

<400> 657
tgccttgggc atgc                                                        14

<210> 658
<211> 14
<212> DNA
<213> Artificial Sequence

<220>
<223> Synthetic Oligonucleotide

<400> 658
gggtgccttg ggca                                                        14

<210> 659
<211> 14
<212> DNA
<213> Artificial Sequence

<220>
<223> Synthetic Oligonucleotide

<400> 659
gcagggtgcc ttgg                                                        14

<210> 660
<211> 14
<212> DNA
<213> Artificial Sequence

<220>
<223> Synthetic Oligonucleotide

<400> 660
agcgcagggt gcct                                                        14

<210> 661
<211> 16
<212> DNA
<213> Artificial Sequence

<220>
<223> Synthetic Oligonucleotide

<400> 661
gtggagcgca gggtgc                                                        16

<210> 662
<211> 14
<212> DNA
<213> Artificial Sequence

<220>
<223> Synthetic Oligonucleotide

<400> 662
tggagcgcag ggtg                                                          14

<210> 663
<211> 14
<212> DNA
<213> Artificial Sequence

<220>
<223> Synthetic Oligonucleotide

<400> 663
tggtggagcg cagg                                                          14

<210> 664
<211> 14
<212> DNA
<213> Artificial Sequence

<220>
<223> Synthetic Oligonucleotide

<400> 664
gcttggtgga gcgc                                                          14

<210> 665
<211> 14
<212> DNA
<213> Artificial Sequence

<220>
<223> Synthetic Oligonucleotide

<400> 665
agagcttggt ggag                                                          14

<210> 666
<211> 16
<212> DNA
<213> Artificial Sequence

<220>
<223> Synthetic Oligonucleotide

<400> 666
aaaagagctt ggtgga                                                        16

<210> 667
<211> 14
<212> DNA
<213> Artificial Sequence

<220>
<223> Synthetic Oligonucleotide

<400> 667
aagagcttgg tgga                                                         14

<210> 668
<211> 14
<212> DNA
<213> Artificial Sequence

<220>
<223> Synthetic Oligonucleotide

<400> 668
aaaagagctt ggtg                                                         14

<210> 669
<211> 14
<212> DNA
<213> Artificial Sequence

<220>
<223> Synthetic Oligonucleotide

<400> 669
caaaaaagag cttg                                                         14

<210> 670
<211> 16
<212> DNA
<213> Artificial Sequence

<220>
<223> Synthetic Oligonucleotide

<400> 670
aaggagctga ggaaca                                                       16

<210> 671
<211> 14
<212> DNA
<213> Artificial Sequence

<220>
<223> Synthetic Oligonucleotide

<400> 671
ggagctgagg aaca                                                         14

<210> 672
<211> 16
<212> DNA
<213> Artificial Sequence

<220>
<223> Synthetic Oligonucleotide

<400> 672
gcagaccctg gaagga                                                       16

<210> 673
<211> 14
<212> DNA

<213> Artificial Sequence

<220>
<223> Synthetic Oligonucleotide

<400> 673
agaccctgga agga                                                         14

<210> 674
<211> 16
<212> DNA
<213> Artificial Sequence

<220>
<223> Synthetic Oligonucleotide

<400> 674
accagcagac cctgga                                                       16

<210> 675
<211> 4
<212> DNA
<213> Artificial Sequence

<220>
<223> Synthetic Oligonucleotide

<400> 675
actg                                                                     4

<210> 676
<211> 16
<212> DNA
<213> Artificial Sequence

<220>
<223> Synthetic Oligonucleotide

<400> 676
cagtagagaa cagcca                                                       16

<210> 677
<211> 14
<212> DNA
<213> Artificial Sequence

<220>
<223> Synthetic Oligonucleotide

<400> 677
gtagagaaca gcca                                                         14

<210> 678
<211> 16
<212> DNA
<213> Artificial Sequence

<220>
<223> Synthetic Oligonucleotide

<400> 678
tgttgaggaa acagta                                                       16

<210> 679
<211> 14

<212> DNA
<213> Artificial Sequence

<220>
<223> Synthetic Oligonucleotide

<400> 679
ttgaggaaac agta                                                         14

<210> 680
<211> 16
<212> DNA
<213> Artificial Sequence

<220>
<223> Synthetic Oligonucleotide

<400> 680
cctgcacctc cttgtt                                                       16

<210> 681
<211> 14
<212> DNA
<213> Artificial Sequence

<220>
<223> Synthetic Oligonucleotide

<400> 681
atgagggtct tcat                                                         14

<210> 682
<211> 14
<212> DNA
<213> Artificial Sequence

<220>
<223> Synthetic Oligonucleotide

<400> 682
accccggagt aggc                                                         14

<210> 683
<211> 12
<212> DNA
<213> Artificial Sequence

<220>
<223> Synthetic Oligonucleotide

<400> 683
cccggagtag gc                                                           12

<210> 684
<211> 16
<212> DNA
<213> Artificial Sequence

<220>
<223> Synthetic Oligonucleotide

<400> 684
caggaccccg gagtag                                                       16

<210> 685

<211> 16
<212> DNA
<213> Artificial Sequence

<220>
<223> Synthetic Oligonucleotide

<400> 685
gcaggacccc ggagta                                                        16

<210> 686
<211> 14
<212> DNA
<213> Artificial Sequence

<220>
<223> Synthetic Oligonucleotide

<400> 686
aggaccccgg agta                                                          14

<210> 687
<211> 14
<212> DNA
<213> Artificial Sequence

<220>
<223> Synthetic Oligonucleotide

<400> 687
caggaccccg gagt                                                          14

<210> 688
<211> 12
<212> DNA
<213> Artificial Sequence

<220>
<223> Synthetic Oligonucleotide

<400> 688
cagacccctc gc                                                            12

<210> 689
<211> 12
<212> DNA
<213> Artificial Sequence

<220>
<223> Synthetic Oligonucleotide

<400> 689
agaggatgct gg                                                            12

<210> 690
<211> 16
<212> DNA
<213> Artificial Sequence

<220>
<223> Synthetic Oligonucleotide

<400> 690
gagccaggtg acagag                                                        16

<210> 691
<211> 14
<212> DNA
<213> Artificial Sequence

<220>
<223> Synthetic Oligonucleotide

<400> 691
agccaggtga caga                                                         14

<210> 692
<211> 12
<212> DNA
<213> Artificial Sequence

<220>
<223> Synthetic Oligonucleotide

<400> 692
tgagccaggt ga                                                           12

<210> 693
<211> 14
<212> DNA
<213> Artificial Sequence

<220>
<223> Synthetic Oligonucleotide

<400> 693
ttttccacct tgga                                                         14

<210> 694
<211> 12
<212> DNA
<213> Artificial Sequence

<220>
<223> Synthetic Oligonucleotide

<400> 694
ctgcaggcca ct                                                           12

<210> 695
<211> 16
<212> DNA
<213> Artificial Sequence

<220>
<223> Synthetic Oligonucleotide

<400> 695
tcaccagctg gatggg                                                       16

<210> 696
<211> 16
<212> DNA
<213> Artificial Sequence

<220>
<223> Synthetic Oligonucleotide

<400> 696
ttcaccagct ggatgg                                                       16

<210> 697
<211> 14
<212> DNA
<213> Artificial Sequence

<220>
<223> Synthetic Oligonucleotide

<400> 697
caccagctgg atgg                                                          14

<210> 698
<211> 14
<212> DNA
<213> Artificial Sequence

<220>
<223> Synthetic Oligonucleotide

<400> 698
tcaccagctg gatg                                                          14

<210> 699
<211> 12
<212> DNA
<213> Artificial Sequence

<220>
<223> Synthetic Oligonucleotide

<400> 699
tcaccagctg ga                                                            12

<210> 700
<211> 16
<212> DNA
<213> Artificial Sequence

<220>
<223> Synthetic Oligonucleotide

<400> 700
tgtcttcacc agctgg                                                        16

<210> 701
<211> 14
<212> DNA
<213> Artificial Sequence

<220>
<223> Synthetic Oligonucleotide

<400> 701
gtcttcacca gctg                                                          14

<210> 702
<211> 16
<212> DNA
<213> Artificial Sequence

<220>
<223> Synthetic Oligonucleotide

<400> 702

gtgtgtcttc accagc                                                        16

<210> 703
<211> 14
<212> DNA
<213> Artificial Sequence

<220>
<223> Synthetic Oligonucleotide

<400> 703
tgtgtcttca ccag                                                          14

<210> 704
<211> 16
<212> DNA
<213> Artificial Sequence

<220>
<223> Synthetic Oligonucleotide

<400> 704
ttgtgtgtct tcacca                                                        16

<210> 705
<211> 14
<212> DNA
<213> Artificial Sequence

<220>
<223> Synthetic Oligonucleotide

<400> 705
tgtgtgtctt cacc                                                          14

<210> 706
<211> 16
<212> DNA
<213> Artificial Sequence

<220>
<223> Synthetic Oligonucleotide

<400> 706
aggttgtgtg tcttca                                                        16

<210> 707
<211> 14
<212> DNA
<213> Artificial Sequence

<220>
<223> Synthetic Oligonucleotide

<400> 707
ggttgtgtgt cttc                                                          14

<210> 708
<211> 16
<212> DNA
<213> Artificial Sequence

<220>
<223> Synthetic Oligonucleotide

<400> 708
gcaggttgtg tgtctt                                                        16

<210> 709
<211> 14
<212> DNA
<213> Artificial Sequence

<220>
<223> Synthetic Oligonucleotide

<400> 709
caggttgtgt gtct                                                          14

<210> 710
<211> 16
<212> DNA
<213> Artificial Sequence

<220>
<223> Synthetic Oligonucleotide

<400> 710
tcagcaggtt gtgtgt                                                        16

<210> 711
<211> 14
<212> DNA
<213> Artificial Sequence

<220>
<223> Synthetic Oligonucleotide

<400> 711
cagcaggttg tgtg                                                          14

<210> 712
<211> 16
<212> DNA
<213> Artificial Sequence

<220>
<223> Synthetic Oligonucleotide

<400> 712
ggtcagcagg ttgtgt                                                        16

<210> 713
<211> 16
<212> DNA
<213> Artificial Sequence

<220>
<223> Synthetic Oligonucleotide

<400> 713
tggtcagcag gttgtg                                                        16

<210> 714
<211> 14
<212> DNA
<213> Artificial Sequence

<220>
<223> Synthetic Oligonucleotide

<400> 714
gtcagcaggt tgtg                                                         14

<210> 715
<211> 14
<212> DNA
<213> Artificial Sequence

<220>
<223> Synthetic Oligonucleotide

<400> 715
ggtcagcagg ttgt                                                         14

<210> 716
<211> 16
<212> DNA
<213> Artificial Sequence

<220>
<223> Synthetic Oligonucleotide

<400> 716
ctggtggtca gcaggt                                                       16

<210> 717
<211> 14
<212> DNA
<213> Artificial Sequence

<220>
<223> Synthetic Oligonucleotide

<400> 717
tggtggtcag cagg                                                         14

<210> 718
<211> 12
<212> DNA
<213> Artificial Sequence

<220>
<223> Synthetic Oligonucleotide

<400> 718
ctggtggtca gc                                                           12

<210> 719
<211> 16
<212> DNA
<213> Artificial Sequence

<220>
<223> Synthetic Oligonucleotide

<400> 719
agttcctggt ggtcag                                                       16

<210> 720
<211> 14
<212> DNA
<213> Artificial Sequence

<220>

<223> Synthetic Oligonucleotide

<400> 720
gttcctggtg gtca                                                         14

<210> 721
<211> 16
<212> DNA
<213> Artificial Sequence

<220>
<223> Synthetic Oligonucleotide

<400> 721
tatagttcct ggtggt                                                       16

<210> 722
<211> 14
<212> DNA
<213> Artificial Sequence

<220>
<223> Synthetic Oligonucleotide

<400> 722
atagttcctg gtgg                                                         14

<210> 723
<211> 16
<212> DNA
<213> Artificial Sequence

<220>
<223> Synthetic Oligonucleotide

<400> 723
gatatagttc ctggtg                                                       16

<210> 724
<211> 14
<212> DNA
<213> Artificial Sequence

<220>
<223> Synthetic Oligonucleotide

<400> 724
atatagttcc tggt                                                         14

<210> 725
<211> 16
<212> DNA
<213> Artificial Sequence

<220>
<223> Synthetic Oligonucleotide

<400> 725
ccaaagatat agttcc                                                       16

<210> 726
<211> 16
<212> DNA
<213> Artificial Sequence

<220>
<223> Synthetic Oligonucleotide

<400> 726
tccaaagata tagttc                                                    16

<210> 727
<211> 14
<212> DNA
<213> Artificial Sequence

<220>
<223> Synthetic Oligonucleotide

<400> 727
caaagatata gttc                                                      14

<210> 728
<211> 14
<212> DNA
<213> Artificial Sequence

<220>
<223> Synthetic Oligonucleotide

<400> 728
ccaaagatat agtt                                                      14

<210> 729
<211> 12
<212> DNA
<213> Artificial Sequence

<220>
<223> Synthetic Oligonucleotide

<400> 729
ccaggcccat ga                                                        12

<210> 730
<211> 16
<212> DNA
<213> Artificial Sequence

<220>
<223> Synthetic Oligonucleotide

<400> 730
ggcacccagg cccatg                                                    16

<210> 731
<211> 14
<212> DNA
<213> Artificial Sequence

<220>
<223> Synthetic Oligonucleotide

<400> 731
gcacccaggc ccat                                                      14

<210> 732
<211> 16
<212> DNA
<213> Artificial Sequence

<220>
<223> Synthetic Oligonucleotide

<400> 732
cagaaggcac ccaggc                                                    16

<210> 733
<211> 14
<212> DNA
<213> Artificial Sequence

<220>
<223> Synthetic Oligonucleotide

<400> 733
agaaggcacc cagg                                                      14

<210> 734
<211> 12
<212> DNA
<213> Artificial Sequence

<220>
<223> Synthetic Oligonucleotide

<400> 734
ccagacatca gg                                                        12

<210> 735
<211> 12
<212> DNA
<213> Artificial Sequence

<220>
<223> Synthetic Oligonucleotide

<400> 735
gacagggcag at                                                        12

<210> 736
<211> 12
<212> DNA
<213> Artificial Sequence

<220>
<223> Synthetic Oligonucleotide

<400> 736
tgacagggca ga                                                        12

<210> 737
<211> 12
<212> DNA
<213> Artificial Sequence

<220>
<223> Synthetic Oligonucleotide

<400> 737
ccactcccat tc                                                        12

<210> 738
<211> 16
<212> DNA

<213> Artificial Sequence

<220>
<223> Synthetic Oligonucleotide

<400> 738
gccaggcatg gagctc                                                        16

<210> 739
<211> 14
<212> DNA
<213> Artificial Sequence

<220>
<223> Synthetic Oligonucleotide

<400> 739
ccaggcatgg agct                                                          14

<210> 740
<211> 12
<212> DNA
<213> Artificial Sequence

<220>
<223> Synthetic Oligonucleotide

<400> 740
ccaggcatgg ag                                                            12

<210> 741
<211> 12
<212> DNA
<213> Artificial Sequence

<220>
<223> Synthetic Oligonucleotide

<400> 741
cagggtgact gc                                                            12

<210> 742
<211> 16
<212> DNA
<213> Artificial Sequence

<220>
<223> Synthetic Oligonucleotide

<400> 742
gttccgcagg gtgact                                                        16

<210> 743
<211> 14
<212> DNA
<213> Artificial Sequence

<220>
<223> Synthetic Oligonucleotide

<400> 743
ttccgcaggg tgac                                                          14

<210> 744
<211> 16

<212> DNA
<213> Artificial Sequence

<220>
<223> Synthetic Oligonucleotide

<400> 744
gcggttccgc agggtg                                                        16

<210> 745
<211> 14
<212> DNA
<213> Artificial Sequence

<220>
<223> Synthetic Oligonucleotide

<400> 745
cggttccgca gggt                                                          14

<210> 746
<211> 16
<212> DNA
<213> Artificial Sequence

<220>
<223> Synthetic Oligonucleotide

<400> 746
tcggccccag gagccc                                                        16

<210> 747
<211> 14
<212> DNA
<213> Artificial Sequence

<220>
<223> Synthetic Oligonucleotide

<400> 747
cggccccagg agcc                                                          14

<210> 748
<211> 16
<212> DNA
<213> Artificial Sequence

<220>
<223> Synthetic Oligonucleotide

<400> 748
ccatcggccc caggag                                                        16

<210> 749
<211> 14
<212> DNA
<213> Artificial Sequence

<220>
<223> Synthetic Oligonucleotide

<400> 749
catcggcccc agga                                                          14

<210> 750

<211> 16
<212> DNA
<213> Artificial Sequence

<220>
<223> Synthetic Oligonucleotide

<400> 750
gacccatcgg ccccag                                                       16

<210> 751
<211> 14
<212> DNA
<213> Artificial Sequence

<220>
<223> Synthetic Oligonucleotide

<400> 751
acccatcggc ccca                                                         14

<210> 752
<211> 16
<212> DNA
<213> Artificial Sequence

<220>
<223> Synthetic Oligonucleotide

<400> 752
ttctggaccc atcggc                                                       16

<210> 753
<211> 12
<212> DNA
<213> Artificial Sequence

<220>
<223> Synthetic Oligonucleotide

<400> 753
ggacccatcg gc                                                           12

<210> 754
<211> 14
<212> DNA
<213> Artificial Sequence

<220>
<223> Synthetic Oligonucleotide

<400> 754
tctggaccca tcgg                                                         14

<210> 755
<211> 16
<212> DNA
<213> Artificial Sequence

<220>
<223> Synthetic Oligonucleotide

<400> 755
caccagcccc caggtg                                                       16

<210> 756
<211> 14
<212> DNA
<213> Artificial Sequence

<220>
<223> Synthetic Oligonucleotide

<400> 756
accagccccc aggt                                                         14

<210> 757
<211> 16
<212> DNA
<213> Artificial Sequence

<220>
<223> Synthetic Oligonucleotide

<400> 757
tagggcacca gccccc                                                       16

<210> 758
<211> 14
<212> DNA
<213> Artificial Sequence

<220>
<223> Synthetic Oligonucleotide

<400> 758
agggcaccag cccc                                                         14

<210> 759
<211> 16
<212> DNA
<213> Artificial Sequence

<220>
<223> Synthetic Oligonucleotide

<400> 759
tggagtaggg caccag                                                       16

<210> 760
<211> 14
<212> DNA
<213> Artificial Sequence

<220>
<223> Synthetic Oligonucleotide

<400> 760
ggagtagggc acca                                                         14

<210> 761
<211> 12
<212> DNA
<213> Artificial Sequence

<220>
<223> Synthetic Oligonucleotide

<400> 761
cttggagtag gg                                                           12

<210> 762
<211> 16
<212> DNA
<213> Artificial Sequence

<220>
<223> Synthetic Oligonucleotide

<400> 762
tgatgggctt ggagta                                                        16

<210> 763
<211> 14
<212> DNA
<213> Artificial Sequence

<220>
<223> Synthetic Oligonucleotide

<400> 763
gatgggcttg gagt                                                          14

<210> 764
<211> 16
<212> DNA
<213> Artificial Sequence

<220>
<223> Synthetic Oligonucleotide

<400> 764
tgtggtacag gtcgat                                                        16

<210> 765
<211> 14
<212> DNA
<213> Artificial Sequence

<220>
<223> Synthetic Oligonucleotide

<400> 765
gtggtacagg tcga                                                          14

<210> 766
<211> 12
<212> DNA
<213> Artificial Sequence

<220>
<223> Synthetic Oligonucleotide

<400> 766
ggtacaggtc ga                                                            12

<210> 767
<211> 16
<212> DNA
<213> Artificial Sequence

<220>
<223> Synthetic Oligonucleotide

<400> 767

ggtgtggtac aggtcg                                                        16

<210> 768
<211> 14
<212> DNA
<213> Artificial Sequence

<220>
<223> Synthetic Oligonucleotide

<400> 768
gtgtggtaca ggtc                                                          14

<210> 769
<211> 16
<212> DNA
<213> Artificial Sequence

<220>
<223> Synthetic Oligonucleotide

<400> 769
catggtgtgg tacagg                                                        16

<210> 770
<211> 14
<212> DNA
<213> Artificial Sequence

<220>
<223> Synthetic Oligonucleotide

<400> 770
atggtgtggt acag                                                          14

<210> 771
<211> 16
<212> DNA
<213> Artificial Sequence

<220>
<223> Synthetic Oligonucleotide

<400> 771
tacatggtgt ggtaca                                                        16

<210> 772
<211> 14
<212> DNA
<213> Artificial Sequence

<220>
<223> Synthetic Oligonucleotide

<400> 772
acatggtgtg gtac                                                          14

<210> 773
<211> 16
<212> DNA
<213> Artificial Sequence

<220>
<223> Synthetic Oligonucleotide

<400> 773
atgtacatgg tgtggt                                                        16

<210> 774
<211> 14
<212> DNA
<213> Artificial Sequence

<220>
<223> Synthetic Oligonucleotide

<400> 774
tgtacatggt gtgg                                                          14

<210> 775
<211> 12
<212> DNA
<213> Artificial Sequence

<220>
<223> Synthetic Oligonucleotide

<400> 775
gcctccatgt ac                                                            12

<210> 776
<211> 12
<212> DNA
<213> Artificial Sequence

<220>
<223> Synthetic Oligonucleotide

<400> 776
agcttcacca gg                                                            12

<210> 777
<211> 12
<212> DNA
<213> Artificial Sequence

<220>
<223> Synthetic Oligonucleotide

<400> 777
gttcacctcc ag                                                            12

<210> 778
<211> 16
<212> DNA
<213> Artificial Sequence

<220>
<223> Synthetic Oligonucleotide

<400> 778
catgccccag ccgccg                                                        16

<210> 779
<211> 14
<212> DNA
<213> Artificial Sequence

<220>
<223> Synthetic Oligonucleotide

<400> 779
atgccccagc cgcc                                                         14

<210> 780
<211> 16
<212> DNA
<213> Artificial Sequence

<220>
<223> Synthetic Oligonucleotide

<400> 780
gatgagggtc ttcatg                                                       16

<210> 781
<211> 16
<212> DNA
<213> Artificial Sequence

<220>
<223> Synthetic Oligonucleotide

<400> 781
gaccccggag taggca                                                       16

<210> 782
<211> 16
<212> DNA
<213> Artificial Sequence

<220>
<223> Synthetic Oligonucleotide

<400> 782
atgctggagc cagtgc                                                       16

<210> 783
<211> 14
<212> DNA
<213> Artificial Sequence

<220>
<223> Synthetic Oligonucleotide

<400> 783
tgctggagcc agtg                                                         14

<210> 784
<211> 14
<212> DNA
<213> Artificial Sequence

<220>
<223> Synthetic Oligonucleotide

<400> 784
gtcttggagg gccg                                                         14

<210> 785
<211> 14
<212> DNA
<213> Artificial Sequence

<220>

<223> Synthetic Oligonucleotide

<400> 785
cccaggtgtc agag                                                          14

<210> 786
<211> 16
<212> DNA
<213> Artificial Sequence

<220>
<223> Synthetic Oligonucleotide

<400> 786
ttttccacct tggatc                                                        16

<210> 787
<211> 14
<212> DNA
<213> Artificial Sequence

<220>
<223> Synthetic Oligonucleotide

<400> 787
tttccacctt ggat                                                          14

<210> 788
<211> 16
<212> DNA
<213> Artificial Sequence

<220>
<223> Synthetic Oligonucleotide

<400> 788
tttttccacc ttggat                                                        16

<210> 789
<211> 16
<212> DNA
<213> Artificial Sequence

<220>
<223> Synthetic Oligonucleotide

<400> 789
aggtgttttt ccacct                                                        16

<210> 790
<211> 14
<212> DNA
<213> Artificial Sequence

<220>
<223> Synthetic Oligonucleotide

<400> 790
ggtgtttttc cacc                                                          14

<210> 791
<211> 16
<212> DNA
<213> Artificial Sequence

<220>
<223> Synthetic Oligonucleotide

<400> 791
ccaggaagga taggac                                                    16

<210> 792
<211> 14
<212> DNA
<213> Artificial Sequence

<220>
<223> Synthetic Oligonucleotide

<400> 792
caggaaggat agga                                                      14

<210> 793
<211> 16
<212> DNA
<213> Artificial Sequence

<220>
<223> Synthetic Oligonucleotide

<400> 793
tgacactgca ggccac                                                    16

<210> 794
<211> 14
<212> DNA
<213> Artificial Sequence

<220>
<223> Synthetic Oligonucleotide

<400> 794
gacactgcag gcca                                                      14

<210> 795
<211> 16
<212> DNA
<213> Artificial Sequence

<220>
<223> Synthetic Oligonucleotide

<400> 795
acatgaggat gacact                                                    16

<210> 796
<211> 14
<212> DNA
<213> Artificial Sequence

<220>
<223> Synthetic Oligonucleotide

<400> 796
catgaggatg acac                                                      14

<210> 797
<211> 16
<212> DNA
<213> Artificial Sequence

<220>
<223> Synthetic Oligonucleotide

<400> 797
gcagaaggtg tacatg                                                        16

<210> 798
<211> 14
<212> DNA
<213> Artificial Sequence

<220>
<223> Synthetic Oligonucleotide

<400> 798
cagaaggtgt acat                                                          14

<210> 799
<211> 16
<212> DNA
<213> Artificial Sequence

<220>
<223> Synthetic Oligonucleotide

<400> 799
gcagtcagtg cagaag                                                        16

<210> 800
<211> 14
<212> DNA
<213> Artificial Sequence

<220>
<223> Synthetic Oligonucleotide

<400> 800
cagtcagtgc agaa                                                          14

<210> 801
<211> 16
<212> DNA
<213> Artificial Sequence

<220>
<223> Synthetic Oligonucleotide

<400> 801
acacggccca gtttcg                                                        16

<210> 802
<211> 14
<212> DNA
<213> Artificial Sequence

<220>
<223> Synthetic Oligonucleotide

<400> 802
cacggcccag tttc                                                          14

<210> 803
<211> 16
<212> DNA

<213> Artificial Sequence

<220>
<223> Synthetic Oligonucleotide

<400> 803
ggcccatgat gccatg                                                        16

<210> 804
<211> 14
<212> DNA
<213> Artificial Sequence

<220>
<223> Synthetic Oligonucleotide

<400> 804
gcccatgatg ccat                                                          14

<210> 805
<211> 16
<212> DNA
<213> Artificial Sequence

<220>
<223> Synthetic Oligonucleotide

<400> 805
tacagaaggc acccag                                                        16

<210> 806
<211> 14
<212> DNA
<213> Artificial Sequence

<220>
<223> Synthetic Oligonucleotide

<400> 806
acagaaggca ccca                                                          14

<210> 807
<211> 16
<212> DNA
<213> Artificial Sequence

<220>
<223> Synthetic Oligonucleotide

<400> 807
gaagttgcca gccaat                                                        16

<210> 808
<211> 14
<212> DNA
<213> Artificial Sequence

<220>
<223> Synthetic Oligonucleotide

<400> 808
aagttgccag ccaa                                                          14

<210> 809
<211> 14

<212> DNA
<213> Artificial Sequence

<220>
<223> Synthetic Oligonucleotide

<400> 809
cagggcagat cctt                                                          14

<210> 810
<211> 16
<212> DNA
<213> Artificial Sequence

<220>
<223> Synthetic Oligonucleotide

<400> 810
caagtagtct atggtg                                                        16

<210> 811
<211> 14
<212> DNA
<213> Artificial Sequence

<220>
<223> Synthetic Oligonucleotide

<400> 811
aagtagtcta tggt                                                          14

<210> 812
<211> 16
<212> DNA
<213> Artificial Sequence

<220>
<223> Synthetic Oligonucleotide

<400> 812
tggaaagcaa gtagtc                                                        16

<210> 813
<211> 14
<212> DNA
<213> Artificial Sequence

<220>
<223> Synthetic Oligonucleotide

<400> 813
ggaaagcaag tagt                                                          14

<210> 814
<211> 16
<212> DNA
<213> Artificial Sequence

<220>
<223> Synthetic Oligonucleotide

<400> 814
ggccagcttt acaaag                                                        16

<210> 815

<211> 14
<212> DNA
<213> Artificial Sequence

<220>
<223> Synthetic Oligonucleotide

<400> 815
gccagcttta caaa                                                         14

<210> 816
<211> 14
<212> DNA
<213> Artificial Sequence

<220>
<223> Synthetic Oligonucleotide

<400> 816
cgcagggcca gctt                                                         14

<210> 817
<211> 16
<212> DNA
<213> Artificial Sequence

<220>
<223> Synthetic Oligonucleotide

<400> 817
aaaggaatag gtggga                                                       16

<210> 818
<211> 14
<212> DNA
<213> Artificial Sequence

<220>
<223> Synthetic Oligonucleotide

<400> 818
aaggaatagg tggg                                                         14

<210> 819
<211> 16
<212> DNA
<213> Artificial Sequence

<220>
<223> Synthetic Oligonucleotide

<400> 819
gcgaaaccaa tatact                                                       16

<210> 820
<211> 14
<212> DNA
<213> Artificial Sequence

<220>
<223> Synthetic Oligonucleotide

<400> 820
cgaaaccaat atac                                                         14

<210> 821
<211> 16
<212> DNA
<213> Artificial Sequence

<220>
<223> Synthetic Oligonucleotide

<400> 821
ccccaggtgt cagagg                                                        16

<210> 822
<211> 4
<212> DNA
<213> Artificial Sequence

<220>
<223> Synthetic Oligonucleotide

<400> 822
actg                                                                      4

<210> 823
<211> 16
<212> DNA
<213> Artificial Sequence

<220>
<223> Synthetic Oligonucleotide

<400> 823
gattgtcaaa gagctt                                                        16

<210> 824
<211> 14
<212> DNA
<213> Artificial Sequence

<220>
<223> Synthetic Oligonucleotide

<400> 824
attgtcaaag agct                                                          14

<210> 825
<211> 16
<212> DNA
<213> Artificial Sequence

<220>
<223> Synthetic Oligonucleotide

<400> 825
ttggtcttgt gattgt                                                        16

<210> 826
<211> 14
<212> DNA
<213> Artificial Sequence

<220>
<223> Synthetic Oligonucleotide

<400> 826
tggtcttgtg attg                                                          14

<210> 827
<211> 16
<212> DNA
<213> Artificial Sequence

<220>
<223> Synthetic Oligonucleotide

<400> 827
aaggccgaat ttggtc                                                        16

<210> 828
<211> 14
<212> DNA
<213> Artificial Sequence

<220>
<223> Synthetic Oligonucleotide

<400> 828
aggccgaatt tggt                                                          14

<210> 829
<211> 12
<212> DNA
<213> Artificial Sequence

<220>
<223> Synthetic Oligonucleotide

<400> 829
aatccgactg tg                                                            12

<210> 830
<211> 12
<212> DNA
<213> Artificial Sequence

<220>
<223> Synthetic Oligonucleotide

<400> 830
aatttaatcc ga                                                            12

<210> 831
<211> 14
<212> DNA
<213> Artificial Sequence

<220>
<223> Synthetic Oligonucleotide

<400> 831
accttcgatc acag                                                          14

<210> 832
<211> 12
<212> DNA
<213> Artificial Sequence

<220>
<223> Synthetic Oligonucleotide

<400> 832

actgatcctg ca                                                            12

<210> 833
<211> 12
<212> DNA
<213> Artificial Sequence

<220>
<223> Synthetic Oligonucleotide

<400> 833
actgtggtca aa                                                            12

<210> 834
<211> 12
<212> DNA
<213> Artificial Sequence

<220>
<223> Synthetic Oligonucleotide

<400> 834
agccgcccag tt                                                            12

<210> 835
<211> 12
<212> DNA
<213> Artificial Sequence

<220>
<223> Synthetic Oligonucleotide

<400> 835
agccttgtcg at                                                            12

<210> 836
<211> 12
<212> DNA
<213> Artificial Sequence

<220>
<223> Synthetic Oligonucleotide

<400> 836
agttcccagc ct                                                            12

<210> 837
<211> 12
<212> DNA
<213> Artificial Sequence

<220>
<223> Synthetic Oligonucleotide

<400> 837
atccgactgt gg                                                            12

<210> 838
<211> 12
<212> DNA
<213> Artificial Sequence

<220>
<223> Synthetic Oligonucleotide

<400> 838
atcctgcact ga                                                          12

<210> 839
<211> 12
<212> DNA
<213> Artificial Sequence

<220>
<223> Synthetic Oligonucleotide

<400> 839
atttaatccg ac                                                          12

<210> 840
<211> 12
<212> DNA
<213> Artificial Sequence

<220>
<223> Synthetic Oligonucleotide

<400> 840
caatttaatc cg                                                          12

<210> 841
<211> 12
<212> DNA
<213> Artificial Sequence

<220>
<223> Synthetic Oligonucleotide

<400> 841
cactgacgag tc                                                          12

<210> 842
<211> 12
<212> DNA
<213> Artificial Sequence

<220>
<223> Synthetic Oligonucleotide

<400> 842
cactgatcct gc                                                          12

<210> 843
<211> 12
<212> DNA
<213> Artificial Sequence

<220>
<223> Synthetic Oligonucleotide

<400> 843
cagccttgtc ga                                                          12

<210> 844
<211> 12
<212> DNA
<213> Artificial Sequence

<220>
<223> Synthetic Oligonucleotide

<400> 844
cagttcccag cc                                                          12

<210> 845
<211> 12
<212> DNA
<213> Artificial Sequence

<220>
<223> Synthetic Oligonucleotide

<400> 845
ccactgatcc tg                                                          12

<210> 846
<211> 12
<212> DNA
<213> Artificial Sequence

<220>
<223> Synthetic Oligonucleotide

<400> 846
ccagccttgt cg                                                          12

<210> 847
<211> 12
<212> DNA
<213> Artificial Sequence

<220>
<223> Synthetic Oligonucleotide

<400> 847
ccagttccca gc                                                          12

<210> 848
<211> 12
<212> DNA
<213> Artificial Sequence

<220>
<223> Synthetic Oligonucleotide

<400> 848
cccagccttg tc                                                          12

<210> 849
<211> 12
<212> DNA
<213> Artificial Sequence

<220>
<223> Synthetic Oligonucleotide

<400> 849
cccagttccc ag                                                          12

<210> 850
<211> 12
<212> DNA
<213> Artificial Sequence

<220>

<223> Synthetic Oligonucleotide

<400> 850
ccgcccagtt cc                                                          12

<210> 851
<211> 12
<212> DNA
<213> Artificial Sequence

<220>
<223> Synthetic Oligonucleotide

<400> 851
cctgcactga cg                                                          12

<210> 852
<211> 14
<212> DNA
<213> Artificial Sequence

<220>
<223> Synthetic Oligonucleotide

<400> 852
ccttggaatg tctg                                                        14

<210> 853
<211> 12
<212> DNA
<213> Artificial Sequence

<220>
<223> Synthetic Oligonucleotide

<400> 853
ccttgtcgat ct                                                          12

<210> 854
<211> 12
<212> DNA
<213> Artificial Sequence

<220>
<223> Synthetic Oligonucleotide

<400> 854
cgactgtggt ca                                                          12

<210> 855
<211> 12
<212> DNA
<213> Artificial Sequence

<220>
<223> Synthetic Oligonucleotide

<400> 855
cgcccagttc cc                                                          12

<210> 856
<211> 12
<212> DNA
<213> Artificial Sequence

<220>
<223> Synthetic Oligonucleotide

<400> 856
ctgatcctgc ac                                                          12

<210> 857
<211> 12
<212> DNA
<213> Artificial Sequence

<220>
<223> Synthetic Oligonucleotide

<400> 857
ctgcactgac ga                                                          12

<210> 858
<211> 12
<212> DNA
<213> Artificial Sequence

<220>
<223> Synthetic Oligonucleotide

<400> 858
ctgtggtcaa aa                                                          12

<210> 859
<211> 12
<212> DNA
<213> Artificial Sequence

<220>
<223> Synthetic Oligonucleotide

<400> 859
cttgtcgatc tc                                                          12

<210> 860
<211> 12
<212> DNA
<213> Artificial Sequence

<220>
<223> Synthetic Oligonucleotide

<400> 860
gatcctgcac tg                                                          12

<210> 861
<211> 12
<212> DNA
<213> Artificial Sequence

<220>
<223> Synthetic Oligonucleotide

<400> 861
gcaatttaat cc                                                          12

<210> 862
<211> 12
<212> DNA
<213> Artificial Sequence

<220>
<223> Synthetic Oligonucleotide

<400> 862
gcactgacga gt                                                          12

<210> 863
<211> 12
<212> DNA
<213> Artificial Sequence

<220>
<223> Synthetic Oligonucleotide

<400> 863
gcccagttcc ca                                                          12

<210> 864
<211> 12
<212> DNA
<213> Artificial Sequence

<220>
<223> Synthetic Oligonucleotide

<400> 864
gccgcccagt tc                                                          12

<210> 865
<211> 12
<212> DNA
<213> Artificial Sequence

<220>
<223> Synthetic Oligonucleotide

<400> 865
gccttgtcga tc                                                          12

<210> 866
<211> 12
<212> DNA
<213> Artificial Sequence

<220>
<223> Synthetic Oligonucleotide

<400> 866
ggtcaaaagg gc                                                          12

<210> 867
<211> 14
<212> DNA
<213> Artificial Sequence

<220>
<223> Synthetic Oligonucleotide

<400> 867
ggtcatgcac aggc                                                        14

<210> 868
<211> 12
<212> DNA

<213> Artificial Sequence

<220>
<223> Synthetic Oligonucleotide

<400> 868
gtcgatctcc tc                                                          12

<210> 869
<211> 12
<212> DNA
<213> Artificial Sequence

<220>
<223> Synthetic Oligonucleotide

<400> 869
gtggtcaaaa gg                                                          12

<210> 870
<211> 12
<212> DNA
<213> Artificial Sequence

<220>
<223> Synthetic Oligonucleotide

<400> 870
gttcccagcc tt                                                          12

<210> 871
<211> 12
<212> DNA
<213> Artificial Sequence

<220>
<223> Synthetic Oligonucleotide

<400> 871
taatccgact gt                                                          12

<210> 872
<211> 14
<212> DNA
<213> Artificial Sequence

<220>
<223> Synthetic Oligonucleotide

<400> 872
tattccatgg ccat                                                        14

<210> 873
<211> 14
<212> DNA
<213> Artificial Sequence

<220>
<223> Synthetic Oligonucleotide

<400> 873
tcaggtcatg caca                                                        14

<210> 874
<211> 12

<212> DNA
<213> Artificial Sequence

<220>
<223> Synthetic Oligonucleotide

<400> 874
tcccagcctt gt                                                          12

<210> 875
<211> 12
<212> DNA
<213> Artificial Sequence

<220>
<223> Synthetic Oligonucleotide

<400> 875
tccgactgtg gt                                                          12

<210> 876
<211> 12
<212> DNA
<213> Artificial Sequence

<220>
<223> Synthetic Oligonucleotide

<400> 876
tcctgcactg ac                                                          12

<210> 877
<211> 12
<212> DNA
<213> Artificial Sequence

<220>
<223> Synthetic Oligonucleotide

<400> 877
tcgatctcct cg                                                          12

<210> 878
<211> 12
<212> DNA
<213> Artificial Sequence

<220>
<223> Synthetic Oligonucleotide

<400> 878
tgatcctgca ct                                                          12

<210> 879
<211> 12
<212> DNA
<213> Artificial Sequence

<220>
<223> Synthetic Oligonucleotide

<400> 879
tgcaatttaa tc                                                          12

<210> 880

<211> 12
<212> DNA
<213> Artificial Sequence

<220>
<223> Synthetic Oligonucleotide

<400> 880
tgcactgacg ag                                                          12

<210> 881
<211> 12
<212> DNA
<213> Artificial Sequence

<220>
<223> Synthetic Oligonucleotide

<400> 881
tggtcaaaag gg                                                          12

<210> 882
<211> 12
<212> DNA
<213> Artificial Sequence

<220>
<223> Synthetic Oligonucleotide

<400> 882
tgtcgatctc ct                                                          12

<210> 883
<211> 12
<212> DNA
<213> Artificial Sequence

<220>
<223> Synthetic Oligonucleotide

<400> 883
tgtggtcaaa ag                                                          12

<210> 884
<211> 12
<212> DNA
<213> Artificial Sequence

<220>
<223> Synthetic Oligonucleotide

<400> 884
ttaatccgac tg                                                          12

<210> 885
<211> 12
<212> DNA
<213> Artificial Sequence

<220>
<223> Synthetic Oligonucleotide

<400> 885
ttcccagcct tg                                                          12

<210> 886
<211> 12
<212> DNA
<213> Artificial Sequence

<220>
<223> Synthetic Oligonucleotide

<400> 886
ttgtcgatct cc                                                          12

<210> 887
<211> 12
<212> DNA
<213> Artificial Sequence

<220>
<223> Synthetic Oligonucleotide

<400> 887
tttaatccga ct                                                          12

<210> 888
<211> 12
<212> DNA
<213> Artificial Sequence

<220>
<223> Synthetic Oligonucleotide

<400> 888
gactgtggtc aa                                                          12

<210> 889
<211> 12
<212> DNA
<213> Artificial Sequence

<220>
<223> Synthetic Oligonucleotide

<400> 889
ccgactgtgg tc                                                          12

<210> 890
<211> 14
<212> DNA
<213> Artificial Sequence

<220>
<223> Synthetic Oligonucleotide

<400> 890
cccagtgggt ttga                                                        14

<210> 891
<211> 12
<212> DNA
<213> Artificial Sequence

<220>
<223> Synthetic Oligonucleotide

<400> 891
ttcttgatgt cc                                                          12

<210> 892
<211> 12
<212> DNA
<213> Artificial Sequence

<220>
<223> Synthetic Oligonucleotide

<400> 892
gactccaaag tc                                                          12

<210> 893
<211> 16
<212> DNA
<213> Artificial Sequence

<220>
<223> Synthetic Oligonucleotide

<400> 893
tcctgcactg acgagt                                                      16

<210> 894
<211> 16
<212> DNA
<213> Artificial Sequence

<220>
<223> Synthetic Oligonucleotide

<400> 894
cactgatcct gcactg                                                      16

<210> 895
<211> 16
<212> DNA
<213> Artificial Sequence

<220>
<223> Synthetic Oligonucleotide

<400> 895
cttccactga tcctta                                                      16

<210> 896
<211> 16
<212> DNA
<213> Artificial Sequence

<220>
<223> Synthetic Oligonucleotide

<400> 896
gaaagctcct tccact                                                      16

<210> 897
<211> 14
<212> DNA
<213> Artificial Sequence

<220>
<223> Synthetic Oligonucleotide

<400> 897

ggcagtcttt atcc                                                          14

<210> 898
<211> 12
<212> DNA
<213> Artificial Sequence

<220>
<223> Synthetic Oligonucleotide

<400> 898
ctggtaaata gc                                                            12

<210> 899
<211> 14
<212> DNA
<213> Artificial Sequence

<220>
<223> Synthetic Oligonucleotide

<400> 899
ctacctgagg attt                                                          14

<210> 900
<211> 14
<212> DNA
<213> Artificial Sequence

<220>
<223> Synthetic Oligonucleotide

<400> 900
cccagtacta cctg                                                          14

<210> 901
<211> 12
<212> DNA
<213> Artificial Sequence

<220>
<223> Synthetic Oligonucleotide

<400> 901
tgttccctct ac                                                            12

<210> 902
<211> 14
<212> DNA
<213> Artificial Sequence

<220>
<223> Synthetic Oligonucleotide

<400> 902
tattcctgga aaac                                                          14

<210> 903
<211> 14
<212> DNA
<213> Artificial Sequence

<220>
<223> Synthetic Oligonucleotide

<400> 903
caagaagtgt ggtt                                                         14

<210> 904
<211> 14
<212> DNA
<213> Artificial Sequence

<220>
<223> Synthetic Oligonucleotide

<400> 904
gtgaaaatgc tggc                                                         14

<210> 905
<211> 14
<212> DNA
<213> Artificial Sequence

<220>
<223> Synthetic Oligonucleotide

<400> 905
aggaggttaa acca                                                         14

<210> 906
<211> 12
<212> DNA
<213> Artificial Sequence

<220>
<223> Synthetic Oligonucleotide

<400> 906
tggatgattg gc                                                           12

<210> 907
<211> 12
<212> DNA
<213> Artificial Sequence

<220>
<223> Synthetic Oligonucleotide

<400> 907
caaaaggatc cc                                                           12

<210> 908
<211> 12
<212> DNA
<213> Artificial Sequence

<220>
<223> Synthetic Oligonucleotide

<400> 908
atgtcaaccg gc                                                           12

<210> 909
<211> 12
<212> DNA
<213> Artificial Sequence

<220>
<223> Synthetic Oligonucleotide

<400> 909
tcagccagac ag                                                          12

<210> 910
<211> 14
<212> DNA
<213> Artificial Sequence

<220>
<223> Synthetic Oligonucleotide

<400> 910
taagtgtccc tttg                                                        14

<210> 911
<211> 14
<212> DNA
<213> Artificial Sequence

<220>
<223> Synthetic Oligonucleotide

<400> 911
ctaaatttag ttca                                                        14

<210> 912
<211> 14
<212> DNA
<213> Artificial Sequence

<220>
<223> Synthetic Oligonucleotide

<400> 912
gactacattt taca                                                        14

<210> 913
<211> 14
<212> DNA
<213> Artificial Sequence

<220>
<223> Synthetic Oligonucleotide

<400> 913
cagtaaggaa tttt                                                        14

<210> 914
<211> 14
<212> DNA
<213> Artificial Sequence

<220>
<223> Synthetic Oligonucleotide

<400> 914
tgtgtccctc agtc                                                        14

<210> 915
<211> 12
<212> DNA
<213> Artificial Sequence

<220>

<223> Synthetic Oligonucleotide

<400> 915
ccgttggacc cc                                                          12

<210> 916
<211> 14
<212> DNA
<213> Artificial Sequence

<220>
<223> Synthetic Oligonucleotide

<400> 916
gacagcttct ataa                                                        14

<210> 917
<211> 14
<212> DNA
<213> Artificial Sequence

<220>
<223> Synthetic Oligonucleotide

<400> 917
agtgactgac caca                                                        14

<210> 918
<211> 14
<212> DNA
<213> Artificial Sequence

<220>
<223> Synthetic Oligonucleotide

<400> 918
gtagcataga gcct                                                        14

<210> 919
<211> 14
<212> DNA
<213> Artificial Sequence

<220>
<223> Synthetic Oligonucleotide

<400> 919
cagatcttgt caag                                                        14

<210> 920
<211> 12
<212> DNA
<213> Artificial Sequence

<220>
<223> Synthetic Oligonucleotide

<400> 920
gtaagaggca gg                                                          12

<210> 921
<211> 12
<212> DNA
<213> Artificial Sequence

<220>
<223> Synthetic Oligonucleotide

<400> 921
ccatggcggg ac                                                          12

<210> 922
<211> 12
<212> DNA
<213> Artificial Sequence

<220>
<223> Synthetic Oligonucleotide

<400> 922
gcttacgatt gt                                                          12

<210> 923
<211> 14
<212> DNA
<213> Artificial Sequence

<220>
<223> Synthetic Oligonucleotide

<400> 923
ccagcacact ggaa                                                        14

<210> 924
<211> 14
<212> DNA
<213> Artificial Sequence

<220>
<223> Synthetic Oligonucleotide

<400> 924
gtcagtccca gcta                                                        14

<210> 925
<211> 14
<212> DNA
<213> Artificial Sequence

<220>
<223> Synthetic Oligonucleotide

<400> 925
ttagtatgac agct                                                        14

<210> 926
<211> 14
<212> DNA
<213> Artificial Sequence

<220>
<223> Synthetic Oligonucleotide

<400> 926
tcagtgtagg aaga                                                        14

<210> 927
<211> 14
<212> DNA
<213> Artificial Sequence

<220>
<223> Synthetic Oligonucleotide

<400> 927
tgaatataca gatg                                                         14

<210> 928
<211> 12
<212> DNA
<213> Artificial Sequence

<220>
<223> Synthetic Oligonucleotide

<400> 928
cccgccacca cc                                                           12

<210> 929
<211> 12
<212> DNA
<213> Artificial Sequence

<220>
<223> Synthetic Oligonucleotide

<400> 929
ttgttccctc ta                                                           12

<210> 930
<211> 14
<212> DNA
<213> Artificial Sequence

<220>
<223> Synthetic Oligonucleotide

<400> 930
tctacctgag tcca                                                         14

<210> 931
<211> 14
<212> DNA
<213> Artificial Sequence

<220>
<223> Synthetic Oligonucleotide

<400> 931
aacatcaagc ttga                                                         14

<210> 932
<211> 14
<212> DNA
<213> Artificial Sequence

<220>
<223> Synthetic Oligonucleotide

<400> 932
gatcaccttc agag                                                         14

<210> 933
<211> 14
<212> DNA

<213> Artificial Sequence

<220>
<223> Synthetic Oligonucleotide

<400> 933
tgaacacatc acta                                                          14

<210> 934
<211> 12
<212> DNA
<213> Artificial Sequence

<220>
<223> Synthetic Oligonucleotide

<400> 934
tttcctcttg tc                                                            12

<210> 935
<211> 14
<212> DNA
<213> Artificial Sequence

<220>
<223> Synthetic Oligonucleotide

<400> 935
taattgatgt caat                                                          14

<210> 936
<211> 14
<212> DNA
<213> Artificial Sequence

<220>
<223> Synthetic Oligonucleotide

<400> 936
agtaattgat gtca                                                          14

<210> 937
<211> 14
<212> DNA
<213> Artificial Sequence

<220>
<223> Synthetic Oligonucleotide

<400> 937
acagtaattg atgt                                                          14

<210> 938
<211> 14
<212> DNA
<213> Artificial Sequence

<220>
<223> Synthetic Oligonucleotide

<400> 938
ttacagtaat tgat                                                          14

<210> 939
<211> 14

<212> DNA
<213> Artificial Sequence

<220>
<223> Synthetic Oligonucleotide

<400> 939
acttacagta attg                                                         14

<210> 940
<211> 14
<212> DNA
<213> Artificial Sequence

<220>
<223> Synthetic Oligonucleotide

<400> 940
agacttacag taat                                                         14

<210> 941
<211> 14
<212> DNA
<213> Artificial Sequence

<220>
<223> Synthetic Oligonucleotide

<400> 941
tcagacttac agta                                                         14

<210> 942
<211> 14
<212> DNA
<213> Artificial Sequence

<220>
<223> Synthetic Oligonucleotide

<400> 942
aatcagactt acag                                                         14

<210> 943
<211> 14
<212> DNA
<213> Artificial Sequence

<220>
<223> Synthetic Oligonucleotide

<400> 943
tgaatcagac ttac                                                         14

<210> 944
<211> 14
<212> DNA
<213> Artificial Sequence

<220>
<223> Synthetic Oligonucleotide

<400> 944
aatgaatcag actt                                                         14

<210> 945

<211> 14
<212> DNA
<213> Artificial Sequence

<220>
<223> Synthetic Oligonucleotide

<400> 945
tgcaggatgt tgag                                                         14

<210> 946
<211> 14
<212> DNA
<213> Artificial Sequence

<220>
<223> Synthetic Oligonucleotide

<400> 946
ctggtcagca ttga                                                         14

<210> 947
<211> 14
<212> DNA
<213> Artificial Sequence

<220>
<223> Synthetic Oligonucleotide

<400> 947
caaagtccct tagc                                                         14

<210> 948
<211> 14
<212> DNA
<213> Artificial Sequence

<220>
<223> Synthetic Oligonucleotide

<400> 948
atttctctta cagg                                                         14

<210> 949
<211> 12
<212> DNA
<213> Artificial Sequence

<220>
<223> Synthetic Oligonucleotide

<400> 949
ttaacgagcc tt                                                           12

<210> 950
<211> 12
<212> DNA
<213> Artificial Sequence

<220>
<223> Synthetic Oligonucleotide

<400> 950
cgacacggga ac                                                           12

<210> 951
<211> 12
<212> DNA
<213> Artificial Sequence

<220>
<223> Synthetic Oligonucleotide

<400> 951
caagtaggat gt                                                          12

<210> 952
<211> 14
<212> DNA
<213> Artificial Sequence

<220>
<223> Synthetic Oligonucleotide

<400> 952
gttccctttg cagg                                                        14

<210> 953
<211> 14
<212> DNA
<213> Artificial Sequence

<220>
<223> Synthetic Oligonucleotide

<400> 953
attagccata tctc                                                        14

<210> 954
<211> 14
<212> DNA
<213> Artificial Sequence

<220>
<223> Synthetic Oligonucleotide

<400> 954
agcattcagc agtg                                                        14

<210> 955
<211> 12
<212> DNA
<213> Artificial Sequence

<220>
<223> Synthetic Oligonucleotide

<400> 955
ttccctctac ac                                                          12

<210> 956
<211> 12
<212> DNA
<213> Artificial Sequence

<220>
<223> Synthetic Oligonucleotide

<400> 956
tccctctaca cc                                                          12

<210> 957
<211> 14
<212> DNA
<213> Artificial Sequence

<220>
<223> Synthetic Oligonucleotide

<400> 957
ctacaggaca atac                                                          14

<210> 958
<211> 14
<212> DNA
<213> Artificial Sequence

<220>
<223> Synthetic Oligonucleotide

<400> 958
aactgggtta agta                                                          14

<210> 959
<211> 14
<212> DNA
<213> Artificial Sequence

<220>
<223> Synthetic Oligonucleotide

<400> 959
tgaacacgct atcc                                                          14

<210> 960
<211> 14
<212> DNA
<213> Artificial Sequence

<220>
<223> Synthetic Oligonucleotide

<400> 960
tttccacttg ggtg                                                          14

<210> 961
<211> 12
<212> DNA
<213> Artificial Sequence

<220>
<223> Synthetic Oligonucleotide

<400> 961
tctacaccag gt                                                            12

<210> 962
<211> 14
<212> DNA
<213> Artificial Sequence

<220>
<223> Synthetic Oligonucleotide

<400> 962

gaaagctcct tcca                                                          14

<210> 963
<211> 12
<212> DNA
<213> Artificial Sequence

<220>
<223> Synthetic Oligonucleotide

<400> 963
aggtaggaga ag                                                            12

<210> 964
<211> 12
<212> DNA
<213> Artificial Sequence

<220>
<223> Synthetic Oligonucleotide

<400> 964
acccagtcag gg                                                            12

<210> 965
<211> 12
<212> DNA
<213> Artificial Sequence

<220>
<223> Synthetic Oligonucleotide

<400> 965
atgtcattaa ac                                                            12

<210> 966
<211> 12
<212> DNA
<213> Artificial Sequence

<220>
<223> Synthetic Oligonucleotide

<400> 966
gaggtgggaa aa                                                            12

<210> 967
<211> 14
<212> DNA
<213> Artificial Sequence

<220>
<223> Synthetic Oligonucleotide

<400> 967
tttcctagga ggtg                                                          14

<210> 968
<211> 12
<212> DNA
<213> Artificial Sequence

<220>
<223> Synthetic Oligonucleotide

<400> 968
gttcttagga ag                                                          12

<210> 969
<211> 12
<212> DNA
<213> Artificial Sequence

<220>
<223> Synthetic Oligonucleotide

<400> 969
ctctacacca gg                                                          12

<210> 970
<211> 14
<212> DNA
<213> Artificial Sequence

<220>
<223> Synthetic Oligonucleotide

<400> 970
tgtttttaca caga                                                        14

<210> 971
<211> 12
<212> DNA
<213> Artificial Sequence

<220>
<223> Synthetic Oligonucleotide

<400> 971
tggcttcatg tc                                                          12

<210> 972
<211> 14
<212> DNA
<213> Artificial Sequence

<220>
<223> Synthetic Oligonucleotide

<400> 972
ttgttcttag gaag                                                        14

<210> 973
<211> 12
<212> DNA
<213> Artificial Sequence

<220>
<223> Synthetic Oligonucleotide

<400> 973
tacaccaggt ca                                                          12

<210> 974
<211> 14
<212> DNA
<213> Artificial Sequence

<220>
<223> Synthetic Oligonucleotide

<400> 974
tgagctacag tagg                                                          14

<210> 975
<211> 12
<212> DNA
<213> Artificial Sequence

<220>
<223> Synthetic Oligonucleotide

<400> 975
ggctgacatt ca                                                            12

<210> 976
<211> 14
<212> DNA
<213> Artificial Sequence

<220>
<223> Synthetic Oligonucleotide

<400> 976
tggtcaactg aaag                                                          14

<210> 977
<211> 12
<212> DNA
<213> Artificial Sequence

<220>
<223> Synthetic Oligonucleotide

<400> 977
tagtcattat ct                                                            12

<210> 978
<211> 12
<212> DNA
<213> Artificial Sequence

<220>
<223> Synthetic Oligonucleotide

<400> 978
ccatttttat ca                                                            12

<210> 979
<211> 14
<212> DNA
<213> Artificial Sequence

<220>
<223> Synthetic Oligonucleotide

<400> 979
gggcttcttc catt                                                          14

<210> 980
<211> 14
<212> DNA
<213> Artificial Sequence

<220>

<223> Synthetic Oligonucleotide

<400> 980
ggtgtggata acag                                                          14

<210> 981
<211> 14
<212> DNA
<213> Artificial Sequence

<220>
<223> Synthetic Oligonucleotide

<400> 981
tcataactat taag                                                          14

<210> 982
<211> 12
<212> DNA
<213> Artificial Sequence

<220>
<223> Synthetic Oligonucleotide

<400> 982
cctctacacc ag                                                            12

<210> 983
<211> 14
<212> DNA
<213> Artificial Sequence

<220>
<223> Synthetic Oligonucleotide

<400> 983
cagcttaggc agag                                                          14

<210> 984
<211> 12
<212> DNA
<213> Artificial Sequence

<220>
<223> Synthetic Oligonucleotide

<400> 984
tcattcccca ct                                                            12

<210> 985
<211> 12
<212> DNA
<213> Artificial Sequence

<220>
<223> Synthetic Oligonucleotide

<400> 985
ctcccacacc at                                                            12

<210> 986
<211> 12
<212> DNA
<213> Artificial Sequence

<220>
<223> Synthetic Oligonucleotide

<400> 986
ttctgctccc ac                                                          12

<210> 987
<211> 12
<212> DNA
<213> Artificial Sequence

<220>
<223> Synthetic Oligonucleotide

<400> 987
cagagaaggt ct                                                          12

<210> 988
<211> 12
<212> DNA
<213> Artificial Sequence

<220>
<223> Synthetic Oligonucleotide

<400> 988
gtatgcactg ct                                                          12

<210> 989
<211> 14
<212> DNA
<213> Artificial Sequence

<220>
<223> Synthetic Oligonucleotide

<400> 989
caatgaagca cagg                                                        14

<210> 990
<211> 12
<212> DNA
<213> Artificial Sequence

<220>
<223> Synthetic Oligonucleotide

<400> 990
tcccaaacaa at                                                          12

<210> 991
<211> 14
<212> DNA
<213> Artificial Sequence

<220>
<223> Synthetic Oligonucleotide

<400> 991
attcttaaca caga                                                        14

<210> 992
<211> 12
<212> DNA
<213> Artificial Sequence

<220>
<223> Synthetic Oligonucleotide

<400> 992
cgggtactat gg                                                            12

<210> 993
<211> 12
<212> DNA
<213> Artificial Sequence

<220>
<223> Synthetic Oligonucleotide

<400> 993
ctacaccagg tc                                                            12

<210> 994
<211> 12
<212> DNA
<213> Artificial Sequence

<220>
<223> Synthetic Oligonucleotide

<400> 994
tatagctcct ct                                                            12

<210> 995
<211> 14
<212> DNA
<213> Artificial Sequence

<220>
<223> Synthetic Oligonucleotide

<400> 995
catttagggt ctaa                                                          14

<210> 996
<211> 12
<212> DNA
<213> Artificial Sequence

<220>
<223> Synthetic Oligonucleotide

<400> 996
atcttcagag at                                                            12

<210> 997
<211> 12
<212> DNA
<213> Artificial Sequence

<220>
<223> Synthetic Oligonucleotide

<400> 997
ttgatatagt ca                                                            12

<210> 998
<211> 12
<212> DNA

<213> Artificial Sequence

<220>
<223> Synthetic Oligonucleotide

<400> 998
taatatgact tg                                                          12

<210> 999
<211> 14
<212> DNA
<213> Artificial Sequence

<220>
<223> Synthetic Oligonucleotide

<400> 999
agccgcctga agtg                                                        14

<210> 1000
<211> 12
<212> DNA
<213> Artificial Sequence

<220>
<223> Synthetic Oligonucleotide

<400> 1000
ccccagcagc gg                                                          12

<210> 1001
<211> 12
<212> DNA
<213> Artificial Sequence

<220>
<223> Synthetic Oligonucleotide

<400> 1001
tccttccact ga                                                          12

<210> 1002
<211> 12
<212> DNA
<213> Artificial Sequence

<220>
<223> Synthetic Oligonucleotide

<400> 1002
   cggtttttgt tc                                                           12

<210> 1003
<211> 14
<212> DNA
<213> Artificial Sequence

<220>
<223> Synthetic Oligonucleotide

<400> 1003
taaatcctct agca                                                        14

<210> 1004
<211> 12

<212> DNA
<213> Artificial Sequence

<220>
<223> Synthetic Oligonucleotide

<400> 1004
gttccctcta ca                                                          12

<210> 1005
<211> 12
<212> DNA
<213> Artificial Sequence

<220>
<223> Synthetic Oligonucleotide

<400> 1005
acaccatctc cc                                                          12

<210> 1006
<211> 12
<212> DNA
<213> Artificial Sequence

<220>
<223> Synthetic Oligonucleotide

<400> 1006
gctccttcca ct                                                          12

<210> 1007
<211> 14
<212> DNA
<213> Artificial Sequence

<220>
<223> Synthetic Oligonucleotide

<400> 1007
cactgatcct gcac                                                        14

<210> 1008
<211> 12
<212> DNA
<213> Artificial Sequence

<220>
<223> Synthetic Oligonucleotide

<400> 1008
tcggactttg aa                                                          12

<210> 1009
<211> 14
<212> DNA
<213> Artificial Sequence

<220>
<223> Synthetic Oligonucleotide

<400> 1009
tcggactttg aaaa                                                        14

<210> 1010

<211> 14
<212> DNA
<213> Artificial Sequence

<220>
<223> Synthetic Oligonucleotide

<400> 1010
atcagccaga caga                                                          14

<210> 1011
<211> 14
<212> DNA
<213> Artificial Sequence

<220>
<223> Synthetic Oligonucleotide

<400> 1011
catcagcaag aggc                                                          14

<210> 1012
<211> 12
<212> DNA
<213> Artificial Sequence

<220>
<223> Synthetic Oligonucleotide

<400> 1012
ttgttcttag ga                                                            12

<210> 1013
<211> 12
<212> DNA
<213> Artificial Sequence

<220>
<223> Synthetic Oligonucleotide

<400> 1013
gccagacaga ag                                                            12

<210> 1014
<211> 12
<212> DNA
<213> Artificial Sequence

<220>
<223> Synthetic Oligonucleotide

<400> 1014
ttgtcgatct gc                                                            12

<210> 1015
<211> 14
<212> DNA
<213> Artificial Sequence

<220>
<223> Synthetic Oligonucleotide

<400> 1015
ggagaagcgc agct                                                          14

<210> 1016
<211> 12
<212> DNA
<213> Artificial Sequence

<220>
<223> Synthetic Oligonucleotide

<400> 1016
ggagaagcgc ag                                                            12

<210> 1017
<211> 14
<212> DNA
<213> Artificial Sequence

<220>
<223> Synthetic Oligonucleotide

<400> 1017
ctgcactgac gagt                                                          14

<210> 1018
<211> 12
<212> DNA
<213> Artificial Sequence

<220>
<223> Synthetic Oligonucleotide

<400> 1018
ctgcaacatg at                                                            12

<210> 1019
<211> 14
<212> DNA
<213> Artificial Sequence

<220>
<223> Synthetic Oligonucleotide

<400> 1019
ctgatcctta gaag                                                          14

<210> 1020
<211> 16
<212> DNA
<213> Artificial Sequence

<220>
<223> Synthetic Oligonucleotide

<400> 1020
ccttccactg atcctg                                                        16

<210> 1021
<211> 12
<212> DNA
<213> Artificial Sequence

<220>
<223> Synthetic Oligonucleotide

<400> 1021
ctccttccac tg                                                            12

<210> 1022
<211> 14
<212> DNA
<213> Artificial Sequence

<220>
<223> Synthetic Oligonucleotide

<400> 1022
tgcagccatg tact                                                          14

<210> 1023
<211> 12
<212> DNA
<213> Artificial Sequence

<220>
<223> Synthetic Oligonucleotide

<400> 1023
cgtttgggtg gc                                                            12

<210> 1024
<211> 14
<212> DNA
<213> Artificial Sequence

<220>
<223> Synthetic Oligonucleotide

<400> 1024
tccactgatc ctgc                                                          14

<210> 1025
<211> 14
<212> DNA
<213> Artificial Sequence

<220>
<223> Synthetic Oligonucleotide

<400> 1025
ttccactgat cctg                                                          14

<210> 1026
<211> 12
<212> DNA
<213> Artificial Sequence

<220>
<223> Synthetic Oligonucleotide

<400> 1026
tccactgatc ct                                                            12

<210> 1027
<211> 14
<212> DNA
<213> Artificial Sequence

<220>
<223> Synthetic Oligonucleotide

<400> 1027

cttccactga tcct                                                          14

<210> 1028
<211> 16
<212> DNA
<213> Artificial Sequence

<220>
<223> Synthetic Oligonucleotide

<400> 1028
tccttccact gatcct                                                        16

<210> 1029
<211> 12
<212> DNA
<213> Artificial Sequence

<220>
<223> Synthetic Oligonucleotide

<400> 1029
ttccactgat cc                                                            12

<210> 1030
<211> 16
<212> DNA
<213> Artificial Sequence

<220>
<223> Synthetic Oligonucleotide

<400> 1030
ctccttccac tgatcc                                                        16

<210> 1031
<211> 14
<212> DNA
<213> Artificial Sequence

<220>
<223> Synthetic Oligonucleotide

<400> 1031
tccttccact gatc                                                          14

<210> 1032
<211> 16
<212> DNA
<213> Artificial Sequence

<220>
<223> Synthetic Oligonucleotide

<400> 1032
gctccttcca ctgatc                                                        16

<210> 1033
<211> 14
<212> DNA
<213> Artificial Sequence

<220>
<223> Synthetic Oligonucleotide

<400> 1033
gctccttcca ctga                                                          14

<210> 1034
<211> 16
<212> DNA
<213> Artificial Sequence

<220>
<223> Synthetic Oligonucleotide

<400> 1034
aagctccttc cactga                                                        16

<210> 1035
<211> 14
<212> DNA
<213> Artificial Sequence

<220>
<223> Synthetic Oligonucleotide

<400> 1035
aagctccttc cact                                                          14

<210> 1036
<211> 12
<212> DNA
<213> Artificial Sequence

<220>
<223> Synthetic Oligonucleotide

<400> 1036
agctccttcc ac                                                            12

<210> 1037
<211> 16
<212> DNA
<213> Artificial Sequence

<220>
<223> Synthetic Oligonucleotide

<400> 1037
gggaaagctc cttcca                                                        16

<210> 1038
<211> 14
<212> DNA
<213> Artificial Sequence

<220>
<223> Synthetic Oligonucleotide

<400> 1038
ttgcaatgtc tggc                                                          14

<210> 1039
<211> 14
<212> DNA
<213> Artificial Sequence

<220>
<223> Synthetic Oligonucleotide

<400> 1039
gatttatctg gctg                                                          14

<210> 1040
<211> 12
<212> DNA
<213> Artificial Sequence

<220>
<223> Synthetic Oligonucleotide

<400> 1040
aagggccctg gg                                                            12

<210> 1041
<211> 12
<212> DNA
<213> Artificial Sequence

<220>
<223> Synthetic Oligonucleotide

<400> 1041
aacttcagtg tc                                                            12

<210> 1042
<211> 12
<212> DNA
<213> Artificial Sequence

<220>
<223> Synthetic Oligonucleotide

<400> 1042
agggcttcca gt                                                            12

<210> 1043
<211> 12
<212> DNA
<213> Artificial Sequence

<220>
<223> Synthetic Oligonucleotide

<400> 1043
aggaagggct tc                                                            12

<210> 1044
<211> 12
<212> DNA
<213> Artificial Sequence

<220>
<223> Synthetic Oligonucleotide

<400> 1044
ccttccactg at                                                            12

<210> 1045
<211> 14
<212> DNA
<213> Artificial Sequence

<220>

<223> Synthetic Oligonucleotide

<400> 1045
ggaaacatac cctg                                                         14

<210> 1046
<211> 14
<212> DNA
<213> Artificial Sequence

<220>
<223> Synthetic Oligonucleotide

<400> 1046
ccttccactg atcc                                                         14

<210> 1047
<211> 12
<212> DNA
<213> Artificial Sequence

<220>
<223> Synthetic Oligonucleotide

<400> 1047
cttccactga tc                                                           12

<210> 1048
<211> 14
<212> DNA
<213> Artificial Sequence

<220>
<223> Synthetic Oligonucleotide

<400> 1048
gaataggtta aggc                                                         14

<210> 1049
<211> 14
<212> DNA
<213> Artificial Sequence

<220>
<223> Synthetic Oligonucleotide

<400> 1049
aacaatgtgt tgta                                                         14

<210> 1050
<211> 12
<212> DNA
<213> Artificial Sequence

<220>
<223> Synthetic Oligonucleotide

<400> 1050
ccctctacac ca                                                           12

<210> 1051
<211> 14
<212> DNA
<213> Artificial Sequence

<220>
<223> Synthetic Oligonucleotide

<400> 1051
gcacacagct gagg                                                         14

<210> 1052
<211> 14
<212> DNA
<213> Artificial Sequence

<220>
<223> Synthetic Oligonucleotide

<400> 1052
gtgcacacag ctga                                                         14

<210> 1053
<211> 14
<212> DNA
<213> Artificial Sequence

<220>
<223> Synthetic Oligonucleotide

<400> 1053
cagtgtgcac acag                                                         14

<210> 1054
<211> 14
<212> DNA
<213> Artificial Sequence

<220>
<223> Synthetic Oligonucleotide

<400> 1054
ctcagtgtgc acac                                                         14

<210> 1055
<211> 12
<212> DNA
<213> Artificial Sequence

<220>
<223> Synthetic Oligonucleotide

<400> 1055
cacccactgg tg                                                           12

<210> 1056
<211> 14
<212> DNA
<213> Artificial Sequence

<220>
<223> Synthetic Oligonucleotide

<400> 1056
catttccatg gcca                                                         14

<210> 1057
<211> 14
<212> DNA
<213> Artificial Sequence

<220>
<223> Synthetic Oligonucleotide

<400> 1057
accaaacagt tcag                                                          14

<210> 1058
<211> 12
<212> DNA
<213> Artificial Sequence

<220>
<223> Synthetic Oligonucleotide

<400> 1058
ttgaccagga ag                                                            12

<210> 1059
<211> 12
<212> DNA
<213> Artificial Sequence

<220>
<223> Synthetic Oligonucleotide

<400> 1059
caggccatgt gg                                                            12

<210> 1060
<211> 14
<212> DNA
<213> Artificial Sequence

<220>
<223> Synthetic Oligonucleotide

<400> 1060
agtcaggcca tgtg                                                          14

<210> 1061
<211> 12
<212> DNA
<213> Artificial Sequence

<220>
<223> Synthetic Oligonucleotide

<400> 1061
gcgcgagccc ga                                                            12

<210> 1062
<211> 12
<212> DNA
<213> Artificial Sequence

<220>
<223> Synthetic Oligonucleotide

<400> 1062
tgtgaggctc ca                                                            12

<210> 1063
<211> 12
<212> DNA

<213> Artificial Sequence

<220>
<223> Synthetic Oligonucleotide

<400> 1063
ccctgaaggt tc                                                          12

<210> 1064
<211> 14
<212> DNA
<213> Artificial Sequence

<220>
<223> Synthetic Oligonucleotide

<400> 1064
tttgataaag ccct                                                        14

<210> 1065
<211> 14
<212> DNA
<213> Artificial Sequence

<220>
<223> Synthetic Oligonucleotide

<400> 1065
caagaagacc ttac                                                        14

<210> 1066
<211> 14
<212> DNA
<213> Artificial Sequence

<220>
<223> Synthetic Oligonucleotide

<400> 1066
tgcacaggca ggtt                                                        14

<210> 1067
<211> 12
<212> DNA
<213> Artificial Sequence

<220>
<223> Synthetic Oligonucleotide

<400> 1067
acagccaggt ag                                                          12

<210> 1068
<211> 14
<212> DNA
<213> Artificial Sequence

<220>
<223> Synthetic Oligonucleotide

<400> 1068
tggaaaactg cacc                                                        14

<210> 1069
<211> 12

<212> DNA
<213> Artificial Sequence

<220>
<223> Synthetic Oligonucleotide

<400> 1069
tgggtggccg gg                                                          12

<210> 1070
<211> 12
<212> DNA
<213> Artificial Sequence

<220>
<223> Synthetic Oligonucleotide

<400> 1070
gggcttcttc ca                                                          12

<210> 1071
<211> 12
<212> DNA
<213> Artificial Sequence

<220>
<223> Synthetic Oligonucleotide

<400> 1071
gctgacatct cg                                                          12

<210> 1072
<211> 14
<212> DNA
<213> Artificial Sequence

<220>
<223> Synthetic Oligonucleotide

<400> 1072
cagcctggca ccta                                                        14

<210> 1073
<211> 12
<212> DNA
<213> Artificial Sequence

<220>
<223> Synthetic Oligonucleotide

<400> 1073
taaaaacaac aa                                                          12

<210> 1074
<211> 12
<212> DNA
<213> Artificial Sequence

<220>
<223> Synthetic Oligonucleotide

<400> 1074
tttataaaac tg                                                          12

<210> 1075

<211> 4
<212> DNA
<213> Artificial Sequence

<220>
<223> Synthetic Oligonucleotide

<400> 1075
actg                                                                    4

<210> 1076
<211> 14
<212> DNA
<213> Artificial Sequence

<220>
<223> Synthetic Oligonucleotide

<400> 1076
agtaaatatt ggct                                                         14

<210> 1077
<211> 12
<212> DNA
<213> Artificial Sequence

<220>
<223> Synthetic Oligonucleotide

<400> 1077
gttgagcatg ac                                                           12

<210> 1078
<211> 12
<212> DNA
<213> Artificial Sequence

<220>
<223> Synthetic Oligonucleotide

<400> 1078
gtgcgctccc at                                                           12

<210> 1079
<211> 14
<212> DNA
<213> Artificial Sequence

<220>
<223> Synthetic Oligonucleotide

<400> 1079
tcctgcactg acga                                                         14

<210> 1080
<211> 16
<212> DNA
<213> Artificial Sequence

<220>
<223> Synthetic Oligonucleotide

<400> 1080
gatcctgcac tgacga                                                       16

<210> 1081
<211> 14
<212> DNA
<213> Artificial Sequence

<220>
<223> Synthetic Oligonucleotide

<400> 1081
gatcctgcac tgac                                                          14

<210> 1082
<211> 16
<212> DNA
<213> Artificial Sequence

<220>
<223> Synthetic Oligonucleotide

<400> 1082
ctgatcctgc actgac                                                        16

<210> 1083
<211> 16
<212> DNA
<213> Artificial Sequence

<220>
<223> Synthetic Oligonucleotide

<400> 1083
tccactgatc ctgcac                                                        16

<210> 1084
<211> 14
<212> DNA
<213> Artificial Sequence

<220>
<223> Synthetic Oligonucleotide

<400> 1084
cgcgagatat ctaa                                                          14

<210> 1085
<211> 14
<212> DNA
<213> Artificial Sequence

<220>
<223> Synthetic Oligonucleotide

<400> 1085
cgcacctggt aaat                                                          14

<210> 1086
<211> 14
<212> DNA
<213> Artificial Sequence

<220>
<223> Synthetic Oligonucleotide

<400> 1086
gttcaagcgg ccta                                                          14

<210> 1087
<211> 14
<212> DNA
<213> Artificial Sequence

<220>
<223> Synthetic Oligonucleotide

<400> 1087
ccctttacac aagt                                                         14

<210> 1088
<211> 14
<212> DNA
<213> Artificial Sequence

<220>
<223> Synthetic Oligonucleotide

<400> 1088
ctcaaaatag attt                                                         14

<210> 1089
<211> 14
<212> DNA
<213> Artificial Sequence

<220>
<223> Synthetic Oligonucleotide

<400> 1089
cacatgagct attc                                                         14

<210> 1090
<211> 14
<212> DNA
<213> Artificial Sequence

<220>
<223> Synthetic Oligonucleotide

<400> 1090
gtgaagtgag tcat                                                         14

<210> 1091
<211> 14
<212> DNA
<213> Artificial Sequence

<220>
<223> Synthetic Oligonucleotide

<400> 1091
ggtcactcaa gatg                                                         14

<210> 1092
<211> 14
<212> DNA
<213> Artificial Sequence

<220>
<223> Synthetic Oligonucleotide

<400> 1092

ggactcactc agca                                                          14

<210> 1093
<211> 14
<212> DNA
<213> Artificial Sequence

<220>
<223> Synthetic Oligonucleotide

<400> 1093
tcagggctac tcat                                                          14

<210> 1094
<211> 14
<212> DNA
<213> Artificial Sequence

<220>
<223> Synthetic Oligonucleotide

<400> 1094
cagcactaga ttca                                                          14

<210> 1095
<211> 14
<212> DNA
<213> Artificial Sequence

<220>
<223> Synthetic Oligonucleotide

<400> 1095
tagcttaatg taac                                                          14

<210> 1096
<211> 14
<212> DNA
<213> Artificial Sequence

<220>
<223> Synthetic Oligonucleotide

<400> 1096
ttgtgacatc tagg                                                          14

<210> 1097
<211> 14
<212> DNA
<213> Artificial Sequence

<220>
<223> Synthetic Oligonucleotide

<400> 1097
gtgcctagca caga                                                          14

<210> 1098
<211> 12
<212> DNA
<213> Artificial Sequence

<220>
<223> Synthetic Oligonucleotide

<400> 1098
cagcctacca gt                                                          12

<210> 1099
<211> 14
<212> DNA
<213> Artificial Sequence

<220>
<223> Synthetic Oligonucleotide

<400> 1099
acatgtcagt aatt                                                        14

<210> 1100
<211> 14
<212> DNA
<213> Artificial Sequence

<220>
<223> Synthetic Oligonucleotide

<400> 1100
ctcatggaca caaa                                                        14

<210> 1101
<211> 14
<212> DNA
<213> Artificial Sequence

<220>
<223> Synthetic Oligonucleotide

<400> 1101
ggaagttttc aagt                                                        14

<210> 1102
<211> 14
<212> DNA
<213> Artificial Sequence

<220>
<223> Synthetic Oligonucleotide

<400> 1102
gaatctggag gtaa                                                        14

<210> 1103
<211> 14
<212> DNA
<213> Artificial Sequence

<220>
<223> Synthetic Oligonucleotide

<400> 1103
agccccttgg ccgt                                                        14

<210> 1104
<211> 14
<212> DNA
<213> Artificial Sequence

<220>
<223> Synthetic Oligonucleotide

<400> 1104
gaatacttca aatc                                                          14

<210> 1105
<211> 14
<212> DNA
<213> Artificial Sequence

<220>
<223> Synthetic Oligonucleotide

<400> 1105
ctgatcctgc actg                                                          14

<210> 1106
<211> 14
<212> DNA
<213> Artificial Sequence

<220>
<223> Synthetic Oligonucleotide

<400> 1106
tttgaggagc tatt                                                          14

<210> 1107
<211> 12
<212> DNA
<213> Artificial Sequence

<220>
<223> Synthetic Oligonucleotide

<400> 1107
ttgttgttcc ct                                                            12

<210> 1108
<211> 12
<212> DNA
<213> Artificial Sequence

<220>
<223> Synthetic Oligonucleotide

<400> 1108
gagttgttgt tc                                                            12

<210> 1109
<211> 12
<212> DNA
<213> Artificial Sequence

<220>
<223> Synthetic Oligonucleotide

<400> 1109
cgagttgttg tt                                                            12

<210> 1110
<211> 12
<212> DNA
<213> Artificial Sequence

<220>

<223> Synthetic Oligonucleotide

<400> 1110
gcgctaggcc gc                                                            12

<210> 1111
<211> 12
<212> DNA
<213> Artificial Sequence

<220>
<223> Synthetic Oligonucleotide

<400> 1111
gttgttgttc cc                                                            12

<210> 1112
<211> 12
<212> DNA
<213> Artificial Sequence

<220>
<223> Synthetic Oligonucleotide

<400> 1112
agttgttgtt cc                                                            12

<210> 1113
<211> 12
<212> DNA
<213> Artificial Sequence

<220>
<223> Synthetic Oligonucleotide

<400> 1113
ctcaccttca tg                                                            12

<210> 1114
<211> 14
<212> DNA
<213> Artificial Sequence

<220>
<223> Synthetic Oligonucleotide

<400> 1114
cttagaaggc agca                                                          14

<210> 1115
<211> 12
<212> DNA
<213> Artificial Sequence

<220>
<223> Synthetic Oligonucleotide

<400> 1115
gttgttccct ct                                                            12

<210> 1116
<211> 12
<212> DNA
<213> Artificial Sequence

<220>
<223> Synthetic Oligonucleotide

<400> 1116
ccaactccaa ct                                                          12

<210> 1117
<211> 12
<212> DNA
<213> Artificial Sequence

<220>
<223> Synthetic Oligonucleotide

<400> 1117
gatctctcga gt                                                          12

<210> 1118
<211> 12
<212> DNA
<213> Artificial Sequence

<220>
<223> Synthetic Oligonucleotide

<400> 1118
aatgcaggat ct                                                          12

<210> 1119
<211> 12
<212> DNA
<213> Artificial Sequence

<220>
<223> Synthetic Oligonucleotide

<400> 1119
ctcggacttt ga                                                          12

<210> 1120
<211> 12
<212> DNA
<213> Artificial Sequence

<220>
<223> Synthetic Oligonucleotide

<400> 1120
tgactctcgg ac                                                          12

<210> 1121
<211> 12
<212> DNA
<213> Artificial Sequence

<220>
<223> Synthetic Oligonucleotide

<400> 1121
cttgtccatc ag                                                          12

<210> 1122
<211> 12
<212> DNA
<213> Artificial Sequence

<220>
<223> Synthetic Oligonucleotide

<400> 1122
gggtctttcc tc                                                          12

<210> 1123
<211> 16
<212> DNA
<213> Artificial Sequence

<220>
<223> Synthetic Oligonucleotide

<400> 1123
cactgatcct tagaag                                                      16

<210> 1124
<211> 14
<212> DNA
<213> Artificial Sequence

<220>
<223> Synthetic Oligonucleotide

<400> 1124
cactgatcct taga                                                        14

<210> 1125
<211> 16
<212> DNA
<213> Artificial Sequence

<220>
<223> Synthetic Oligonucleotide

<400> 1125
tccactgatc cttaga                                                      16

<210> 1126
<211> 16
<212> DNA
<213> Artificial Sequence

<220>
<223> Synthetic Oligonucleotide

<400> 1126
cttccactga tcctgc                                                      16

<210> 1127
<211> 14
<212> DNA
<213> Artificial Sequence

<220>
<223> Synthetic Oligonucleotide

<400> 1127
tccactgatc ctta                                                        14

<210> 1128
<211> 12
<212> DNA

<213> Artificial Sequence

<220>
<223> Synthetic Oligonucleotide

<400> 1128
cagtggacca ca                                                          12

<210> 1129
<211> 12
<212> DNA
<213> Artificial Sequence

<220>
<223> Synthetic Oligonucleotide

<400> 1129
tgcgagttgt tg                                                          12

<210> 1130
<211> 12
<212> DNA
<213> Artificial Sequence

<220>
<223> Synthetic Oligonucleotide

<400> 1130
aaagtcaggc ca                                                          12

<210> 1131
<211> 14
<212> DNA
<213> Artificial Sequence

<220>
<223> Synthetic Oligonucleotide

<400> 1131
taacttcagt gtct                                                        14

<210> 1132
<211> 14
<212> DNA
<213> Artificial Sequence

<220>
<223> Synthetic Oligonucleotide

<400> 1132
gaagggcttc cagt                                                        14

<210> 1133
<211> 14
<212> DNA
<213> Artificial Sequence

<220>
<223> Synthetic Oligonucleotide

<400> 1133
tgaccaggaa gggc                                                        14

<210> 1134
<211> 14

<212> DNA
<213> Artificial Sequence

<220>
<223> Synthetic Oligonucleotide

<400> 1134
aggccctgag atta                                                         14

<210> 1135
<211> 14
<212> DNA
<213> Artificial Sequence

<220>
<223> Synthetic Oligonucleotide

<400> 1135
ggttaaggcc ctga                                                         14

<210> 1136
<211> 14
<212> DNA
<213> Artificial Sequence

<220>
<223> Synthetic Oligonucleotide

<400> 1136
ggaatgtctg agtt                                                         14

<210> 1137
<211> 14
<212> DNA
<213> Artificial Sequence

<220>
<223> Synthetic Oligonucleotide

<400> 1137
taatgacctg atga                                                         14

<210> 1138
<211> 12
<212> DNA
<213> Artificial Sequence

<220>
<223> Synthetic Oligonucleotide

<400> 1138
tgaagttaat tc                                                           12

<210> 1139
<211> 12
<212> DNA
<213> Artificial Sequence

<220>
<223> Synthetic Oligonucleotide

<400> 1139
gaaattgagg aa                                                           12

<210> 1140

<211> 12
<212> DNA
<213> Artificial Sequence

<220>
<223> Synthetic Oligonucleotide

<400> 1140
gtattcaagt aa                                                          12

<210> 1141
<211> 12
<212> DNA
<213> Artificial Sequence

<220>
<223> Synthetic Oligonucleotide

<400> 1141
acaattatgg ca                                                          12

<210> 1142
<211> 12
<212> DNA
<213> Artificial Sequence

<220>
<223> Synthetic Oligonucleotide

<400> 1142
cagtttattc aa                                                          12

<210> 1143
<211> 12
<212> DNA
<213> Artificial Sequence

<220>
<223> Synthetic Oligonucleotide

<400> 1143
gaagctgctg gt                                                          12

<210> 1144
<211> 12
<212> DNA
<213> Artificial Sequence

<220>
<223> Synthetic Oligonucleotide

<400> 1144
gtgaaacatt tt                                                          12

<210> 1145
<211> 14
<212> DNA
<213> Artificial Sequence

<220>
<223> Synthetic Oligonucleotide

<400> 1145
aaaagtgaaa catt                                                        14

<210> 1146
<211> 12
<212> DNA
<213> Artificial Sequence

<220>
<223> Synthetic Oligonucleotide

<400> 1146
ttcttaaagg tg                                                          12

<210> 1147
<211> 12
<212> DNA
<213> Artificial Sequence

<220>
<223> Synthetic Oligonucleotide

<400> 1147
gcacaaattt tc                                                          12

<210> 1148
<211> 12
<212> DNA
<213> Artificial Sequence

<220>
<223> Synthetic Oligonucleotide

<400> 1148
gtttagtgca ca                                                          12

<210> 1149
<211> 12
<212> DNA
<213> Artificial Sequence

<220>
<223> Synthetic Oligonucleotide

<400> 1149
tctggatcag ag                                                          12

<210> 1150
<211> 12
<212> DNA
<213> Artificial Sequence

<220>
<223> Synthetic Oligonucleotide

<400> 1150
tgctgcacat cc                                                          12

<210> 1151
<211> 12
<212> DNA
<213> Artificial Sequence

<220>
<223> Synthetic Oligonucleotide

<400> 1151
tctgactggg aa                                                          12

<210> 1152
<211> 12
<212> DNA
<213> Artificial Sequence

<220>
<223> Synthetic Oligonucleotide

<400> 1152
acaaagagtc cc                                                          12

<210> 1153
<211> 12
<212> DNA
<213> Artificial Sequence

<220>
<223> Synthetic Oligonucleotide

<400> 1153
agagagctga gc                                                          12

<210> 1154
<211> 12
<212> DNA
<213> Artificial Sequence

<220>
<223> Synthetic Oligonucleotide

<400> 1154
tggcctcaca gc                                                          12

<210> 1155
<211> 12
<212> DNA
<213> Artificial Sequence

<220>
<223> Synthetic Oligonucleotide

<400> 1155
ggaccgcagc cg                                                          12

<210> 1156
<211> 12
<212> DNA
<213> Artificial Sequence

<220>
<223> Synthetic Oligonucleotide

<400> 1156
gttagaacag ac                                                          12

<210> 1157
<211> 12
<212> DNA
<213> Artificial Sequence

<220>
<223> Synthetic Oligonucleotide

<400> 1157

cctgaaactg ca                                                          12

<210> 1158
<211> 12
<212> DNA
<213> Artificial Sequence

<220>
<223> Synthetic Oligonucleotide

<400> 1158
tgctcacagg cg                                                          12

<210> 1159
<211> 12
<212> DNA
<213> Artificial Sequence

<220>
<223> Synthetic Oligonucleotide

<400> 1159
agagagcaac tc                                                          12

<210> 1160
<211> 12
<212> DNA
<213> Artificial Sequence

<220>
<223> Synthetic Oligonucleotide

<400> 1160
atggctgctg cg                                                          12

<210> 1161
<211> 12
<212> DNA
<213> Artificial Sequence

<220>
<223> Synthetic Oligonucleotide

<400> 1161
catggctgca gc                                                          12

<210> 1162
<211> 14
<212> DNA
<213> Artificial Sequence

<220>
<223> Synthetic Oligonucleotide

<400> 1162
cttggccctg gacc                                                        14

<210> 1163
<211> 13
<212> DNA
<213> Artificial Sequence

<220>
<223> Synthetic Oligonucleotide

<400> 1163
gctagcctct gga                                                          13

<210> 1164
<211> 12
<212> DNA
<213> Artificial Sequence

<220>
<223> Synthetic Oligonucleotide

<400> 1164
tggccctgga cc                                                           12

<210> 1165
<211> 12
<212> DNA
<213> Artificial Sequence

<220>
<223> Synthetic Oligonucleotide

<400> 1165
tttctgcagg aa                                                           12

<210> 1166
<211> 14
<212> DNA
<213> Artificial Sequence

<220>
<223> Synthetic Oligonucleotide

<400> 1166
tacaggtcaa gtct                                                         14

<210> 1167
<211> 12
<212> DNA
<213> Artificial Sequence

<220>
<223> Synthetic Oligonucleotide

<400> 1167
acaggtcaag tc                                                           12

<210> 1168
<211> 14
<212> DNA
<213> Artificial Sequence

<220>
<223> Synthetic Oligonucleotide

<400> 1168
ttggataaat atct                                                         14

<210> 1169
<211> 12
<212> DNA
<213> Artificial Sequence

<220>
<223> Synthetic Oligonucleotide

<400> 1169
tggataaata tc                                                          12

<210> 1170
<211> 12
<212> DNA
<213> Artificial Sequence

<220>
<223> Synthetic Oligonucleotide

<400> 1170
ttctgcagga tg                                                          12

<210> 1171
<211> 12
<212> DNA
<213> Artificial Sequence

<220>
<223> Synthetic Oligonucleotide

<400> 1171
caccttcaag tc                                                          12

<210> 1172
<211> 12
<212> DNA
<213> Artificial Sequence

<220>
<223> Synthetic Oligonucleotide

<400> 1172
catagattgt at                                                          12

<210> 1173
<211> 12
<212> DNA
<213> Artificial Sequence

<220>
<223> Synthetic Oligonucleotide

<400> 1173
gtcagaatat ct                                                          12

<210> 1174
<211> 12
<212> DNA
<213> Artificial Sequence

<220>
<223> Synthetic Oligonucleotide

<400> 1174
tctgcagtta aa                                                          12

<210> 1175
<211> 12
<212> DNA
<213> Artificial Sequence

<220>

<223> Synthetic Oligonucleotide

<400> 1175
atcttgtgaa ac                                                          12

<210> 1176
<211> 12
<212> DNA
<213> Artificial Sequence

<220>
<223> Synthetic Oligonucleotide

<400> 1176
tcatcaaaag gt                                                          12

<210> 1177
<211> 12
<212> DNA
<213> Artificial Sequence

<220>
<223> Synthetic Oligonucleotide

<400> 1177
ctttgtcaag at                                                          12

<210> 1178
<211> 12
<212> DNA
<213> Artificial Sequence

<220>
<223> Synthetic Oligonucleotide

<400> 1178
tatgcacaaa tc                                                          12

<210> 1179
<211> 12
<212> DNA
<213> Artificial Sequence

<220>
<223> Synthetic Oligonucleotide

<400> 1179
taatccaggt ga                                                          12

<210> 1180
<211> 12
<212> DNA
<213> Artificial Sequence

<220>
<223> Synthetic Oligonucleotide

<400> 1180
cacacaggca at                                                          12

<210> 1181
<211> 12
<212> DNA
<213> Artificial Sequence

<220>
<223> Synthetic Oligonucleotide

<400> 1181
ttgagcatct tg                                                          12

<210> 1182
<211> 14
<212> DNA
<213> Artificial Sequence

<220>
<223> Synthetic Oligonucleotide

<400> 1182
cattttgtcc tttt                                                        14

<210> 1183
<211> 12
<212> DNA
<213> Artificial Sequence

<220>
<223> Synthetic Oligonucleotide

<400> 1183
gatttcctga tc                                                          12

<210> 1184
<211> 15
<212> DNA
<213> Artificial Sequence

<220>
<223> Synthetic Oligonucleotide

<400> 1184
ctggatttga tggct                                                       15

<210> 1185
<211> 15
<212> DNA
<213> Artificial Sequence

<220>
<223> Synthetic Oligonucleotide

<400> 1185
tctggatttg atggc                                                       15

<210> 1186
<211> 12
<212> DNA
<213> Artificial Sequence

<220>
<223> Synthetic Oligonucleotide

<400> 1186
tggacttggc gg                                                          12

<210> 1187
<211> 15
<212> DNA
<213> Artificial Sequence

<220>
<223> Synthetic Oligonucleotide

<400> 1187
ctctggattt gatgg                                                        15

<210> 1188
<211> 15
<212> DNA
<213> Artificial Sequence

<220>
<223> Synthetic Oligonucleotide

<400> 1188
cctctggatt tgatg                                                        15

<210> 1189
<211> 15
<212> DNA
<213> Artificial Sequence

<220>
<223> Synthetic Oligonucleotide

<400> 1189
gcctctggat ttgat                                                        15

<210> 1190
<211> 16
<212> DNA
<213> Artificial Sequence

<220>
<223> Synthetic Oligonucleotide

<400> 1190
tttagcctct ggattt                                                       16

<210> 1191
<211> 16
<212> DNA
<213> Artificial Sequence

<220>
<223> Synthetic Oligonucleotide

<400> 1191
tctagcctct ggattt                                                       16

<210> 1192
<211> 12
<212> DNA
<213> Artificial Sequence

<220>
<223> Synthetic Oligonucleotide

<400> 1192
agtagttgta ct                                                           12

<210> 1193
<211> 12
<212> DNA

<213> Artificial Sequence

<220>
<223> Synthetic Oligonucleotide

<400> 1193
tctggagtca ca                                                          12

<210> 1194
<211> 12
<212> DNA
<213> Artificial Sequence

<220>
<223> Synthetic Oligonucleotide

<400> 1194
gtcataatgt ct                                                          12

<210> 1195
<211> 14
<212> DNA
<213> Artificial Sequence

<220>
<223> Synthetic Oligonucleotide

<400> 1195
gtgtcataat gtct                                                        14

<210> 1196
<211> 12
<212> DNA
<213> Artificial Sequence

<220>
<223> Synthetic Oligonucleotide

<400> 1196
cttcaaaagg at                                                          12

<210> 1197
<211> 12
<212> DNA
<213> Artificial Sequence

<220>
<223> Synthetic Oligonucleotide

<400> 1197
gtcttcaaaa gg                                                          12

<210> 1198
<211> 12
<212> DNA
<213> Artificial Sequence

<220>
<223> Synthetic Oligonucleotide

<400> 1198
atcctcttga ta                                                          12

<210> 1199
<211> 12

<212> DNA
<213> Artificial Sequence

<220>
<223> Synthetic Oligonucleotide

<400> 1199
gtgcctttaa aa                                                            12

<210> 1200
<211> 12
<212> DNA
<213> Artificial Sequence

<220>
<223> Synthetic Oligonucleotide

<400> 1200
tgtcataatg tc                                                            12

<210> 1201
<211> 12
<212> DNA
<213> Artificial Sequence

<220>
<223> Synthetic Oligonucleotide

<400> 1201
caatctgaca ca                                                            12

<210> 1202
<211> 12
<212> DNA
<213> Artificial Sequence

<220>
<223> Synthetic Oligonucleotide

<400> 1202
aatattgttc ct                                                            12

<210> 1203
<211> 12
<212> DNA
<213> Artificial Sequence

<220>
<223> Synthetic Oligonucleotide

<400> 1203
gtcaggagaa ga                                                            12

<210> 1204
<211> 12
<212> DNA
<213> Artificial Sequence

<220>
<223> Synthetic Oligonucleotide

<400> 1204
tgtcaaaacc ac                                                            12

<210> 1205

<211> 12
<212> DNA
<213> Artificial Sequence

<220>
<223> Synthetic Oligonucleotide

<400> 1205
gtcctattgc ca                                                          12

<210> 1206
<211> 12
<212> DNA
<213> Artificial Sequence

<220>
<223> Synthetic Oligonucleotide

<400> 1206
aggtatatac at                                                          12

<210> 1207
<211> 12
<212> DNA
<213> Artificial Sequence

<220>
<223> Synthetic Oligonucleotide

<400> 1207
cagctggtga ca                                                          12

<210> 1208
<211> 12
<212> DNA
<213> Artificial Sequence

<220>
<223> Synthetic Oligonucleotide

<400> 1208
ttcacttagc ca                                                          12

<210> 1209
<211> 12
<212> DNA
<213> Artificial Sequence

<220>
<223> Synthetic Oligonucleotide

<400> 1209
aaccctgcag aa                                                          12

<210> 1210
<211> 12
<212> DNA
<213> Artificial Sequence

<220>
<223> Synthetic Oligonucleotide

<400> 1210
tgtcaaaacc ct                                                          12

<210> 1211
<211> 12
<212> DNA
<213> Artificial Sequence

<220>
<223> Synthetic Oligonucleotide

<400> 1211
gtgtcaaaac cc                                                          12

<210> 1212
<211> 14
<212> DNA
<213> Artificial Sequence

<220>
<223> Synthetic Oligonucleotide

<400> 1212
atggtccaga gccc                                                        14

<210> 1213
<211> 12
<212> DNA
<213> Artificial Sequence

<220>
<223> Synthetic Oligonucleotide

<400> 1213
tggtccagag cc                                                          12

<210> 1214
<211> 12
<212> DNA
<213> Artificial Sequence

<220>
<223> Synthetic Oligonucleotide

<400> 1214
tgggttatgg tc                                                          12

<210> 1215
<211> 12
<212> DNA
<213> Artificial Sequence

<220>
<223> Synthetic Oligonucleotide

<400> 1215
caaagaatgg tg                                                          12

<210> 1216
<211> 12
<212> DNA
<213> Artificial Sequence

<220>
<223> Synthetic Oligonucleotide

<400> 1216
gtttagcatg ct                                                          12

<210> 1217
<211> 12
<212> DNA
<213> Artificial Sequence

<220>
<223> Synthetic Oligonucleotide

<400> 1217
tgcctttaaa aa                                                          12

<210> 1218
<211> 12
<212> DNA
<213> Artificial Sequence

<220>
<223> Synthetic Oligonucleotide

<400> 1218
accaatatgc tc                                                          12

<210> 1219
<211> 12
<212> DNA
<213> Artificial Sequence

<220>
<223> Synthetic Oligonucleotide

<400> 1219
caccaataag tt                                                          12

<210> 1220
<211> 12
<212> DNA
<213> Artificial Sequence

<220>
<223> Synthetic Oligonucleotide

<400> 1220
cctttagctg gc                                                          12

<210> 1221
<211> 12
<212> DNA
<213> Artificial Sequence

<220>
<223> Synthetic Oligonucleotide

<400> 1221
gttgaaagcc tc                                                          12

<210> 1222
<211> 12
<212> DNA
<213> Artificial Sequence

<220>
<223> Synthetic Oligonucleotide

<400> 1222

tttgatgatg gc                                                          12

<210> 1223
<211> 12
<212> DNA
<213> Artificial Sequence

<220>
<223> Synthetic Oligonucleotide

<400> 1223
tcattgtcaa at                                                          12

<210> 1224
<211> 12
<212> DNA
<213> Artificial Sequence

<220>
<223> Synthetic Oligonucleotide

<400> 1224
ttacaactga ga                                                          12

<210> 1225
<211> 12
<212> DNA
<213> Artificial Sequence

<220>
<223> Synthetic Oligonucleotide

<400> 1225
agatggagca gt                                                          12

<210> 1226
<211> 14
<212> DNA
<213> Artificial Sequence

<220>
<223> Synthetic Oligonucleotide

<400> 1226
cttagcactg gcct                                                        14

<210> 1227
<211> 12
<212> DNA
<213> Artificial Sequence

<220>
<223> Synthetic Oligonucleotide

<400> 1227
agagtatctg aa                                                          12

<210> 1228
<211> 12
<212> DNA
<213> Artificial Sequence

<220>
<223> Synthetic Oligonucleotide

<400> 1228
actgatgtag gg                                                            12

<210> 1229
<211> 12
<212> DNA
<213> Artificial Sequence

<220>
<223> Synthetic Oligonucleotide

<400> 1229
ccacagtagg ta                                                            12

<210> 1230
<211> 14
<212> DNA
<213> Artificial Sequence

<220>
<223> Synthetic Oligonucleotide

<400> 1230
ctggcagcca gcac                                                          14

<210> 1231
<211> 12
<212> DNA
<213> Artificial Sequence

<220>
<223> Synthetic Oligonucleotide

<400> 1231
tggcagccag ca                                                            12

<210> 1232
<211> 12
<212> DNA
<213> Artificial Sequence

<220>
<223> Synthetic Oligonucleotide

<400> 1232
gcatcatcaa tc                                                            12

<210> 1233
<211> 12
<212> DNA
<213> Artificial Sequence

<220>
<223> Synthetic Oligonucleotide

<400> 1233
aaatcattgt ca                                                            12

<210> 1234
<211> 12
<212> DNA
<213> Artificial Sequence

<220>
<223> Synthetic Oligonucleotide

<400> 1234
gcaaaactca tt                                                          12

<210> 1235
<211> 14
<212> DNA
<213> Artificial Sequence

<220>
<223> Synthetic Oligonucleotide

<400> 1235
tgtgcaactc tgca                                                        14

<210> 1236
<211> 12
<212> DNA
<213> Artificial Sequence

<220>
<223> Synthetic Oligonucleotide

<400> 1236
gtgcaactct gc                                                          12

<210> 1237
<211> 12
<212> DNA
<213> Artificial Sequence

<220>
<223> Synthetic Oligonucleotide

<400> 1237
aatattcatg ta                                                          12

<210> 1238
<211> 12
<212> DNA
<213> Artificial Sequence

<220>
<223> Synthetic Oligonucleotide

<400> 1238
ggattgcaag tt                                                          12

<210> 1239
<211> 12
<212> DNA
<213> Artificial Sequence

<220>
<223> Synthetic Oligonucleotide

<400> 1239
ataaagcatc tg                                                          12

<210> 1240
<211> 12
<212> DNA
<213> Artificial Sequence

<220>

<223> Synthetic Oligonucleotide

<400> 1240
ccactgaaca tt                                                          12

<210> 1241
<211> 12
<212> DNA
<213> Artificial Sequence

<220>
<223> Synthetic Oligonucleotide

<400> 1241
gaagccctaa tc                                                          12

<210> 1242
<211> 12
<212> DNA
<213> Artificial Sequence

<220>
<223> Synthetic Oligonucleotide

<400> 1242
taaattgtat gc                                                          12

<210> 1243
<211> 12
<212> DNA
<213> Artificial Sequence

<220>
<223> Synthetic Oligonucleotide

<400> 1243
aagatcttca ca                                                          12

<210> 1244
<211> 12
<212> DNA
<213> Artificial Sequence

<220>
<223> Synthetic Oligonucleotide

<400> 1244
caagatcttc ac                                                          12

<210> 1245
<211> 12
<212> DNA
<213> Artificial Sequence

<220>
<223> Synthetic Oligonucleotide

<400> 1245
atgtcaaggt ca                                                          12

<210> 1246
<211> 12
<212> DNA
<213> Artificial Sequence

<220>
<223> Synthetic Oligonucleotide

<400> 1246
ataatggctg ga                                                          12

<210> 1247
<211> 14
<212> DNA
<213> Artificial Sequence

<220>
<223> Synthetic Oligonucleotide

<400> 1247
agcaccaata tgct                                                        14

<210> 1248
<211> 12
<212> DNA
<213> Artificial Sequence

<220>
<223> Synthetic Oligonucleotide

<400> 1248
gcaccaatat gc                                                          12

<210> 1249
<211> 12
<212> DNA
<213> Artificial Sequence

<220>
<223> Synthetic Oligonucleotide

<400> 1249
tggcagacca ca                                                          12

<210> 1250
<211> 12
<212> DNA
<213> Artificial Sequence

<220>
<223> Synthetic Oligonucleotide

<400> 1250
tcctatgcaa tc                                                          12

<210> 1251
<211> 12
<212> DNA
<213> Artificial Sequence

<220>
<223> Synthetic Oligonucleotide

<400> 1251
tataaatgct tc                                                          12

<210> 1252
<211> 12
<212> DNA
<213> Artificial Sequence

<220>
<223> Synthetic Oligonucleotide

<400> 1252
gtcaaattct at                                                          12

<210> 1253
<211> 12
<212> DNA
<213> Artificial Sequence

<220>
<223> Synthetic Oligonucleotide

<400> 1253
ttcaggctaa tc                                                          12

<210> 1254
<211> 12
<212> DNA
<213> Artificial Sequence

<220>
<223> Synthetic Oligonucleotide

<400> 1254
ctgatgaggt at                                                          12

<210> 1255
<211> 12
<212> DNA
<213> Artificial Sequence

<220>
<223> Synthetic Oligonucleotide

<400> 1255
ggtgtcagaa ta                                                          12

<210> 1256
<211> 12
<212> DNA
<213> Artificial Sequence

<220>
<223> Synthetic Oligonucleotide

<400> 1256
tttgaaagac ac                                                          12

<210> 1257
<211> 4
<212> DNA
<213> Artificial Sequence

<220>
<223> Synthetic Oligonucleotide

<400> 1257
actg                                                                    4

<210> 1258
<211> 12
<212> DNA

<213> Artificial Sequence

<220>
<223> Synthetic Oligonucleotide

<400> 1258
tgcaacagcc ca                                                          12

<210> 1259
<211> 14
<212> DNA
<213> Artificial Sequence

<220>
<223> Synthetic Oligonucleotide

<400> 1259
aaagagtccc gcca                                                        14

<210> 1260
<211> 12
<212> DNA
<213> Artificial Sequence

<220>
<223> Synthetic Oligonucleotide

<400> 1260
aagagtcccg cc                                                          12

<210> 1261
<211> 14
<212> DNA
<213> Artificial Sequence

<220>
<223> Synthetic Oligonucleotide

<400> 1261
tccgagagga gaga                                                        14

<210> 1262
<211> 12
<212> DNA
<213> Artificial Sequence

<220>
<223> Synthetic Oligonucleotide

<400> 1262
ccgagaggag ag                                                          12

<210> 1263
<211> 14
<212> DNA
<213> Artificial Sequence

<220>
<223> Synthetic Oligonucleotide

<400> 1263
tcatggctgc agct                                                        14

<210> 1264
<211> 14

<212> DNA
<213> Artificial Sequence

<220>
<223> Synthetic Oligonucleotide

<400> 1264
acagcggctc aact                                                         14

<210> 1265
<211> 12
<212> DNA
<213> Artificial Sequence

<220>
<223> Synthetic Oligonucleotide

<400> 1265
cagcggctca ac                                                           12

<210> 1266
<211> 14
<212> DNA
<213> Artificial Sequence

<220>
<223> Synthetic Oligonucleotide

<400> 1266
ggtgacaggc gact                                                         14

<210> 1267
<211> 12
<212> DNA
<213> Artificial Sequence

<220>
<223> Synthetic Oligonucleotide

<400> 1267
gtgacaggcg ac                                                           12

<210> 1268
<211> 12
<212> DNA
<213> Artificial Sequence

<220>
<223> Synthetic Oligonucleotide

<400> 1268
atggtgacag gc                                                           12

<210> 1269
<211> 12
<212> DNA
<213> Artificial Sequence

<220>
<223> Synthetic Oligonucleotide

<400> 1269
agaggcctgg ca                                                           12

<210> 1270

<211> 12
<212> DNA
<213> Artificial Sequence

<220>
<223> Synthetic Oligonucleotide

<400> 1270
ctggatggtt gc                                                          12

<210> 1271
<211> 14
<212> DNA
<213> Artificial Sequence

<220>
<223> Synthetic Oligonucleotide

<400> 1271
aatggctgct gcgg                                                        14

<210> 1272
<211> 12
<212> DNA
<213> Artificial Sequence

<220>
<223> Synthetic Oligonucleotide

<400> 1272
ccgggtaatg gc                                                          12

<210> 1273
<211> 14
<212> DNA
<213> Artificial Sequence

<220>
<223> Synthetic Oligonucleotide

<400> 1273
tggaccgcag ccgg                                                        14

<210> 1274
<211> 14
<212> DNA
<213> Artificial Sequence

<220>
<223> Synthetic Oligonucleotide

<400> 1274
tgatgcccct cgct                                                        14

<210> 1275
<211> 12
<212> DNA
<213> Artificial Sequence

<220>
<223> Synthetic Oligonucleotide

<400> 1275
gatgcccctc gc                                                          12

<210> 1276
<211> 14
<212> DNA
<213> Artificial Sequence

<220>
<223> Synthetic Oligonucleotide

<400> 1276
ctggacttgg cggt                                                         14

<210> 1277
<211> 14
<212> DNA
<213> Artificial Sequence

<220>
<223> Synthetic Oligonucleotide

<400> 1277
ggaaatggct ctgg                                                         14

<210> 1278
<211> 12
<212> DNA
<213> Artificial Sequence

<220>
<223> Synthetic Oligonucleotide

<400> 1278
gaaatggctc tg                                                           12

<210> 1279
<211> 14
<212> DNA
<213> Artificial Sequence

<220>
<223> Synthetic Oligonucleotide

<400> 1279
agaagctgct ggtg                                                         14

<210> 1280
<211> 12
<212> DNA
<213> Artificial Sequence

<220>
<223> Synthetic Oligonucleotide

<400> 1280
gatggcagaa gc                                                           12

<210> 1281
<211> 14
<212> DNA
<213> Artificial Sequence

<220>
<223> Synthetic Oligonucleotide

<400> 1281
agagagatgg caga                                                         14

<210> 1282
<211> 12
<212> DNA
<213> Artificial Sequence

<220>
<223> Synthetic Oligonucleotide

<400> 1282
gagagatggc ag                                                          12

<210> 1283
<211> 14
<212> DNA
<213> Artificial Sequence

<220>
<223> Synthetic Oligonucleotide

<400> 1283
gtggctgaag aaaa                                                        14

<210> 1284
<211> 12
<212> DNA
<213> Artificial Sequence

<220>
<223> Synthetic Oligonucleotide

<400> 1284
tggctgaaga aa                                                          12

<210> 1285
<211> 14
<212> DNA
<213> Artificial Sequence

<220>
<223> Synthetic Oligonucleotide

<400> 1285
atgtctggga gcct                                                        14

<210> 1286
<211> 12
<212> DNA
<213> Artificial Sequence

<220>
<223> Synthetic Oligonucleotide

<400> 1286
tgtctgggag cc                                                          12

<210> 1287
<211> 14
<212> DNA
<213> Artificial Sequence

<220>
<223> Synthetic Oligonucleotide

<400> 1287

atgatggctg tcat                                                          14

<210> 1288
<211> 12
<212> DNA
<213> Artificial Sequence

<220>
<223> Synthetic Oligonucleotide

<400> 1288
tgatgatggc tg                                                            12

<210> 1289
<211> 14
<212> DNA
<213> Artificial Sequence

<220>
<223> Synthetic Oligonucleotide

<400> 1289
ctttgatgat ggct                                                          14

<210> 1290
<211> 14
<212> DNA
<213> Artificial Sequence

<220>
<223> Synthetic Oligonucleotide

<400> 1290
acgatctctt tgat                                                          14

<210> 1291
<211> 14
<212> DNA
<213> Artificial Sequence

<220>
<223> Synthetic Oligonucleotide

<400> 1291
tgtttctgct aacg                                                          14

<210> 1292
<211> 12
<212> DNA
<213> Artificial Sequence

<220>
<223> Synthetic Oligonucleotide

<400> 1292
gtttctgcta ac                                                            12

<210> 1293
<211> 14
<212> DNA
<213> Artificial Sequence

<220>
<223> Synthetic Oligonucleotide

<400> 1293
tttgtttctg ctaa                                                          14

<210> 1294
<211> 14
<212> DNA
<213> Artificial Sequence

<220>
<223> Synthetic Oligonucleotide

<400> 1294
cttgatatct cctt                                                          14

<210> 1295
<211> 14
<212> DNA
<213> Artificial Sequence

<220>
<223> Synthetic Oligonucleotide

<400> 1295
catcctcttg atat                                                          14

<210> 1296
<211> 14
<212> DNA
<213> Artificial Sequence

<220>
<223> Synthetic Oligonucleotide

<400> 1296
aatccatcct cttg                                                          14

<210> 1297
<211> 12
<212> DNA
<213> Artificial Sequence

<220>
<223> Synthetic Oligonucleotide

<400> 1297
gaatccatcc tc                                                            12

<210> 1298
<211> 14
<212> DNA
<213> Artificial Sequence

<220>
<223> Synthetic Oligonucleotide

<400> 1298
gtctaagtcg aatc                                                          14

<210> 1299
<211> 12
<212> DNA
<213> Artificial Sequence

<220>
<223> Synthetic Oligonucleotide

<400> 1299
caagtctaag tc                                                          12

<210> 1300
<211> 12
<212> DNA
<213> Artificial Sequence

<220>
<223> Synthetic Oligonucleotide

<400> 1300
aggtcaagtc ta                                                          12

<210> 1301
<211> 14
<212> DNA
<213> Artificial Sequence

<220>
<223> Synthetic Oligonucleotide

<400> 1301
cgcagaaatg gata                                                        14

<210> 1302
<211> 12
<212> DNA
<213> Artificial Sequence

<220>
<223> Synthetic Oligonucleotide

<400> 1302
gcagaaatgg at                                                          12

<210> 1303
<211> 14
<212> DNA
<213> Artificial Sequence

<220>
<223> Synthetic Oligonucleotide

<400> 1303
ttcgcatccg tcta                                                        14

<210> 1304
<211> 12
<212> DNA
<213> Artificial Sequence

<220>
<223> Synthetic Oligonucleotide

<400> 1304
tcgcatccgt ct                                                          12

<210> 1305
<211> 14
<212> DNA
<213> Artificial Sequence

<220>

<223> Synthetic Oligonucleotide

<400> 1305
ccctaggttg aata                                                          14

<210> 1306
<211> 12
<212> DNA
<213> Artificial Sequence

<220>
<223> Synthetic Oligonucleotide

<400> 1306
cctaggttga at                                                            12

<210> 1307
<211> 14
<212> DNA
<213> Artificial Sequence

<220>
<223> Synthetic Oligonucleotide

<400> 1307
gttatgcaaa tcag                                                          14

<210> 1308
<211> 12
<212> DNA
<213> Artificial Sequence

<220>
<223> Synthetic Oligonucleotide

<400> 1308
ttatgcaaat ca                                                            12

<210> 1309
<211> 14
<212> DNA
<213> Artificial Sequence

<220>
<223> Synthetic Oligonucleotide

<400> 1309
tgactcagta aatt                                                          14

<210> 1310
<211> 12
<212> DNA
<213> Artificial Sequence

<220>
<223> Synthetic Oligonucleotide

<400> 1310
gactcagtaa at                                                            12

<210> 1311
<211> 14
<212> DNA
<213> Artificial Sequence

<220>
<223> Synthetic Oligonucleotide

<400> 1311
ttaaaattct tggg                                                         14

<210> 1312
<211> 12
<212> DNA
<213> Artificial Sequence

<220>
<223> Synthetic Oligonucleotide

<400> 1312
taaaattctt gg                                                           12

<210> 1313
<211> 14
<212> DNA
<213> Artificial Sequence

<220>
<223> Synthetic Oligonucleotide

<400> 1313
cctaactttt agac                                                         14

<210> 1314
<211> 12
<212> DNA
<213> Artificial Sequence

<220>
<223> Synthetic Oligonucleotide

<400> 1314
ctaactttta ga                                                           12

<210> 1315
<211> 14
<212> DNA
<213> Artificial Sequence

<220>
<223> Synthetic Oligonucleotide

<400> 1315
acctgaaact gcaa                                                         14

<210> 1316
<211> 14
<212> DNA
<213> Artificial Sequence

<220>
<223> Synthetic Oligonucleotide

<400> 1316
gtgtcaaaac cact                                                         14

<210> 1317
<211> 14
<212> DNA
<213> Artificial Sequence

<220>
<223> Synthetic Oligonucleotide

<400> 1317
cctattccca ctga                                                          14

<210> 1318
<211> 12
<212> DNA
<213> Artificial Sequence

<220>
<223> Synthetic Oligonucleotide

<400> 1318
ctattcccac tg                                                            12

<210> 1319
<211> 14
<212> DNA
<213> Artificial Sequence

<220>
<223> Synthetic Oligonucleotide

<400> 1319
cccatagcaa taat                                                          14

<210> 1320
<211> 12
<212> DNA
<213> Artificial Sequence

<220>
<223> Synthetic Oligonucleotide

<400> 1320
ccatagcaat aa                                                            12

<210> 1321
<211> 12
<212> DNA
<213> Artificial Sequence

<220>
<223> Synthetic Oligonucleotide

<400> 1321
atcccatagc aa                                                            12

<210> 1322
<211> 14
<212> DNA
<213> Artificial Sequence

<220>
<223> Synthetic Oligonucleotide

<400> 1322
tctgcaggaa atcc                                                          14

<210> 1323
<211> 12
<212> DNA

<213> Artificial Sequence

<220>
<223> Synthetic Oligonucleotide

<400> 1323
ctgcaggaaa tc                                                          12

<210> 1324
<211> 14
<212> DNA
<213> Artificial Sequence

<220>
<223> Synthetic Oligonucleotide

<400> 1324
ttctgcagga aatc                                                        14

<210> 1325
<211> 14
<212> DNA
<213> Artificial Sequence

<220>
<223> Synthetic Oligonucleotide

<400> 1325
ccttcaagtc tttc                                                        14

<210> 1326
<211> 14
<212> DNA
<213> Artificial Sequence

<220>
<223> Synthetic Oligonucleotide

<400> 1326
ttgttcctgt atac                                                        14

<210> 1327
<211> 14
<212> DNA
<213> Artificial Sequence

<220>
<223> Synthetic Oligonucleotide

<400> 1327
caatattgtt cctg                                                        14

<210> 1328
<211> 12
<212> DNA
<213> Artificial Sequence

<220>
<223> Synthetic Oligonucleotide

<400> 1328
caatattgtt cc                                                          12

<210> 1329
<211> 14

<212> DNA
<213> Artificial Sequence

<220>
<223> Synthetic Oligonucleotide

<400> 1329
acatcatcaa tatt                                                         14

<210> 1330
<211> 14
<212> DNA
<213> Artificial Sequence

<220>
<223> Synthetic Oligonucleotide

<400> 1330
ctttgaatcc aaaa                                                         14

<210> 1331
<211> 12
<212> DNA
<213> Artificial Sequence

<220>
<223> Synthetic Oligonucleotide

<400> 1331
tttgaatcca aa                                                           12

<210> 1332
<211> 14
<212> DNA
<213> Artificial Sequence

<220>
<223> Synthetic Oligonucleotide

<400> 1332
tatgctttga atcc                                                         14

<210> 1333
<211> 14
<212> DNA
<213> Artificial Sequence

<220>
<223> Synthetic Oligonucleotide

<400> 1333
ttgtaatggt tttt                                                         14

<210> 1334
<211> 12
<212> DNA
<213> Artificial Sequence

<220>
<223> Synthetic Oligonucleotide

<400> 1334
atcttgtaat gg                                                           12

<210> 1335

<211> 14
<212> DNA
<213> Artificial Sequence

<220>
<223> Synthetic Oligonucleotide

<400> 1335
agattgtata tctt                                                         14

<210> 1336
<211> 14
<212> DNA
<213> Artificial Sequence

<220>
<223> Synthetic Oligonucleotide

<400> 1336
cggtgtcata atgt                                                         14

<210> 1337
<211> 14
<212> DNA
<213> Artificial Sequence

<220>
<223> Synthetic Oligonucleotide

<400> 1337
aaatttggcg gtgt                                                         14

<210> 1338
<211> 14
<212> DNA
<213> Artificial Sequence

<220>
<223> Synthetic Oligonucleotide

<400> 1338
ttaaatttgg cggt                                                         14

<210> 1339
<211> 12
<212> DNA
<213> Artificial Sequence

<220>
<223> Synthetic Oligonucleotide

<400> 1339
taaatttggc gg                                                           12

<210> 1340
<211> 14
<212> DNA
<213> Artificial Sequence

<220>
<223> Synthetic Oligonucleotide

<400> 1340
tcttcaaaag gata                                                         14

<210> 1341
<211> 12
<212> DNA
<213> Artificial Sequence

<220>
<223> Synthetic Oligonucleotide

<400> 1341
tggtcttcaa aa                                                          12

<210> 1342
<211> 14
<212> DNA
<213> Artificial Sequence

<220>
<223> Synthetic Oligonucleotide

<400> 1342
gtgggttatg gtct                                                        14

<210> 1343
<211> 14
<212> DNA
<213> Artificial Sequence

<220>
<223> Synthetic Oligonucleotide

<400> 1343
aagttctagc tgtg                                                        14

<210> 1344
<211> 12
<212> DNA
<213> Artificial Sequence

<220>
<223> Synthetic Oligonucleotide

<400> 1344
ataagttcta gc                                                          12

<210> 1345
<211> 14
<212> DNA
<213> Artificial Sequence

<220>
<223> Synthetic Oligonucleotide

<400> 1345
aagggtttga taag                                                        14

<210> 1346
<211> 14
<212> DNA
<213> Artificial Sequence

<220>
<223> Synthetic Oligonucleotide

<400> 1346
tcaagatctt caca                                                        14

<210> 1347
<211> 14
<212> DNA
<213> Artificial Sequence

<220>
<223> Synthetic Oligonucleotide

<400> 1347
agccattggt caag                                                         14

<210> 1348
<211> 14
<212> DNA
<213> Artificial Sequence

<220>
<223> Synthetic Oligonucleotide

<400> 1348
tcttcactta gcca                                                         14

<210> 1349
<211> 14
<212> DNA
<213> Artificial Sequence

<220>
<223> Synthetic Oligonucleotide

<400> 1349
tgctgcaaca tgat                                                         14

<210> 1350
<211> 12
<212> DNA
<213> Artificial Sequence

<220>
<223> Synthetic Oligonucleotide

<400> 1350
gctgcaacat ga                                                           12

<210> 1351
<211> 14
<212> DNA
<213> Artificial Sequence

<220>
<223> Synthetic Oligonucleotide

<400> 1351
ttacagtgaa ttgc                                                         14

<210> 1352
<211> 12
<212> DNA
<213> Artificial Sequence

<220>
<223> Synthetic Oligonucleotide

<400> 1352

ccagctttac ag                                                          12

<210> 1353
<211> 12
<212> DNA
<213> Artificial Sequence

<220>
<223> Synthetic Oligonucleotide

<400> 1353
cattacacca gt                                                          12

<210> 1354
<211> 14
<212> DNA
<213> Artificial Sequence

<220>
<223> Synthetic Oligonucleotide

<400> 1354
atcattacac cagt                                                        14

<210> 1355
<211> 12
<212> DNA
<213> Artificial Sequence

<220>
<223> Synthetic Oligonucleotide

<400> 1355
tcattacacc ag                                                          12

<210> 1356
<211> 14
<212> DNA
<213> Artificial Sequence

<220>
<223> Synthetic Oligonucleotide

<400> 1356
aataaatatg caca                                                        14

<210> 1357
<211> 14
<212> DNA
<213> Artificial Sequence

<220>
<223> Synthetic Oligonucleotide

<400> 1357
tgtgccttta aaaa                                                        14

<210> 1358
<211> 14
<212> DNA
<213> Artificial Sequence

<220>
<223> Synthetic Oligonucleotide

<400> 1358
ttgtgccttt aaaa                                                         14

<210> 1359
<211> 14
<212> DNA
<213> Artificial Sequence

<220>
<223> Synthetic Oligonucleotide

<400> 1359
gggcctcttg tgcc                                                         14

<210> 1360
<211> 14
<212> DNA
<213> Artificial Sequence

<220>
<223> Synthetic Oligonucleotide

<400> 1360
ccttacttcc ccat                                                         14

<210> 1361
<211> 14
<212> DNA
<213> Artificial Sequence

<220>
<223> Synthetic Oligonucleotide

<400> 1361
ctctggtcct tact                                                         14

<210> 1362
<211> 16
<212> DNA
<213> Artificial Sequence

<220>
<223> Synthetic Oligonucleotide

<400> 1362
gtctctggtc cttact                                                       16

<210> 1363
<211> 14
<212> DNA
<213> Artificial Sequence

<220>
<223> Synthetic Oligonucleotide

<400> 1363
tctctggtcc ttac                                                         14

<210> 1364
<211> 14
<212> DNA
<213> Artificial Sequence

<220>
<223> Synthetic Oligonucleotide

<400> 1364
cctctgactg ggaa                                                         14

<210> 1365
<211> 14
<212> DNA
<213> Artificial Sequence

<220>
<223> Synthetic Oligonucleotide

<400> 1365
catagcgcct ctga                                                         14

<210> 1366
<211> 14
<212> DNA
<213> Artificial Sequence

<220>
<223> Synthetic Oligonucleotide

<400> 1366
caggtagcta taat                                                         14

<210> 1367
<211> 12
<212> DNA
<213> Artificial Sequence

<220>
<223> Synthetic Oligonucleotide

<400> 1367
caggtagcta ta                                                           12

<210> 1368
<211> 14
<212> DNA
<213> Artificial Sequence

<220>
<223> Synthetic Oligonucleotide

<400> 1368
tcatcttgtg aaac                                                         14

<210> 1369
<211> 12
<212> DNA
<213> Artificial Sequence

<220>
<223> Synthetic Oligonucleotide

<400> 1369
catcttgtga aa                                                           12

<210> 1370
<211> 14
<212> DNA
<213> Artificial Sequence

<220>

<223> Synthetic Oligonucleotide

<400> 1370
gtttcaaaca tcat                                                         14

<210> 1371
<211> 14
<212> DNA
<213> Artificial Sequence

<220>
<223> Synthetic Oligonucleotide

<400> 1371
tagtttcaaa catc                                                         14

<210> 1372
<211> 12
<212> DNA
<213> Artificial Sequence

<220>
<223> Synthetic Oligonucleotide

<400> 1372
agtttcaaac at                                                           12

<210> 1373
<211> 12
<212> DNA
<213> Artificial Sequence

<220>
<223> Synthetic Oligonucleotide

<400> 1373
gaatagtttc aa                                                           12

<210> 1374
<211> 14
<212> DNA
<213> Artificial Sequence

<220>
<223> Synthetic Oligonucleotide

<400> 1374
cattggaata gttt                                                         14

<210> 1375
<211> 14
<212> DNA
<213> Artificial Sequence

<220>
<223> Synthetic Oligonucleotide

<400> 1375
cactgaacat tgga                                                         14

<210> 1376
<211> 12
<212> DNA
<213> Artificial Sequence

<220>
<223> Synthetic Oligonucleotide

<400> 1376
actgaacatt gg                                                          12

<210> 1377
<211> 14
<212> DNA
<213> Artificial Sequence

<220>
<223> Synthetic Oligonucleotide

<400> 1377
ccgccactga acat                                                        14

<210> 1378
<211> 14
<212> DNA
<213> Artificial Sequence

<220>
<223> Synthetic Oligonucleotide

<400> 1378
cagaccacaa actg                                                        14

<210> 1379
<211> 12
<212> DNA
<213> Artificial Sequence

<220>
<223> Synthetic Oligonucleotide

<400> 1379
agaccacaaa ct                                                          12

<210> 1380
<211> 14
<212> DNA
<213> Artificial Sequence

<220>
<223> Synthetic Oligonucleotide

<400> 1380
ctggcagacc acaa                                                        14

<210> 1381
<211> 14
<212> DNA
<213> Artificial Sequence

<220>
<223> Synthetic Oligonucleotide

<400> 1381
agctggcaga ccac                                                        14

<210> 1382
<211> 14
<212> DNA
<213> Artificial Sequence

<220>
<223> Synthetic Oligonucleotide

<400> 1382
acctttagct ggca                                                         14

<210> 1383
<211> 14
<212> DNA
<213> Artificial Sequence

<220>
<223> Synthetic Oligonucleotide

<400> 1383
tcttcacctt tagc                                                         14

<210> 1384
<211> 12
<212> DNA
<213> Artificial Sequence

<220>
<223> Synthetic Oligonucleotide

<400> 1384
tcaaagtaca tg                                                           12

<210> 1385
<211> 14
<212> DNA
<213> Artificial Sequence

<220>
<223> Synthetic Oligonucleotide

<400> 1385
gaactcaaag taca                                                         14

<210> 1386
<211> 12
<212> DNA
<213> Artificial Sequence

<220>
<223> Synthetic Oligonucleotide

<400> 1386
ggaactcaaa gt                                                           12

<210> 1387
<211> 14
<212> DNA
<213> Artificial Sequence

<220>
<223> Synthetic Oligonucleotide

<400> 1387
agggaactca aagt                                                         14

<210> 1388
<211> 12
<212> DNA

<213> Artificial Sequence

<220>
<223> Synthetic Oligonucleotide

<400> 1388
gggaactcaa ag                                                          12

<210> 1389
<211> 14
<212> DNA
<213> Artificial Sequence

<220>
<223> Synthetic Oligonucleotide

<400> 1389
gctgagggaa ctca                                                        14

<210> 1390
<211> 14
<212> DNA
<213> Artificial Sequence

<220>
<223> Synthetic Oligonucleotide

<400> 1390
tcaccacaca cagg                                                        14

<210> 1391
<211> 14
<212> DNA
<213> Artificial Sequence

<220>
<223> Synthetic Oligonucleotide

<400> 1391
gaactctact ttga                                                        14

<210> 1392
<211> 12
<212> DNA
<213> Artificial Sequence

<220>
<223> Synthetic Oligonucleotide

<400> 1392
gaagaactct ac                                                          12

<210> 1393
<211> 14
<212> DNA
<213> Artificial Sequence

<220>
<223> Synthetic Oligonucleotide

<400> 1393
gtggaagaac tcta                                                        14

<210> 1394
<211> 12

<212> DNA
<213> Artificial Sequence

<220>
<223> Synthetic Oligonucleotide

<400> 1394
tggaagaact ct                                                          12

<210> 1395
<211> 14
<212> DNA
<213> Artificial Sequence

<220>
<223> Synthetic Oligonucleotide

<400> 1395
tgtttgtgga agaa                                                        14

<210> 1396
<211> 14
<212> DNA
<213> Artificial Sequence

<220>
<223> Synthetic Oligonucleotide

<400> 1396
catcttgttc tgtt                                                        14

<210> 1397
<211> 14
<212> DNA
<213> Artificial Sequence

<220>
<223> Synthetic Oligonucleotide

<400> 1397
agtgaaacat tttg                                                        14

<210> 1398
<211> 12
<212> DNA
<213> Artificial Sequence

<220>
<223> Synthetic Oligonucleotide

<400> 1398
ttacccaaaa gt                                                          12

<210> 1399
<211> 14
<212> DNA
<213> Artificial Sequence

<220>
<223> Synthetic Oligonucleotide

<400> 1399
tatttaccca aaag                                                        14

<210> 1400

<211> 14
<212> DNA
<213> Artificial Sequence

<220>
<223> Synthetic Oligonucleotide

<400> 1400
gtatttaccc aaaa                                                          14

<210> 1401
<211> 12
<212> DNA
<213> Artificial Sequence

<220>
<223> Synthetic Oligonucleotide

<400> 1401
tatttaccca aa                                                            12

<210> 1402
<211> 14
<212> DNA
<213> Artificial Sequence

<220>
<223> Synthetic Oligonucleotide

<400> 1402
tcctggtatg aaga                                                          14

<210> 1403
<211> 12
<212> DNA
<213> Artificial Sequence

<220>
<223> Synthetic Oligonucleotide

<400> 1403
cctggtatga ag                                                            12

<210> 1404
<211> 12
<212> DNA
<213> Artificial Sequence

<220>
<223> Synthetic Oligonucleotide

<400> 1404
ggtcctggta tg                                                            12

<210> 1405
<211> 14
<212> DNA
<213> Artificial Sequence

<220>
<223> Synthetic Oligonucleotide

<400> 1405
ttcctctggt cctg                                                          14

<210> 1406
<211> 14
<212> DNA
<213> Artificial Sequence

<220>
<223> Synthetic Oligonucleotide

<400> 1406
aggtttcctc tggt                                                          14

<210> 1407
<211> 12
<212> DNA
<213> Artificial Sequence

<220>
<223> Synthetic Oligonucleotide

<400> 1407
ggtttcctct gg                                                            12

<210> 1408
<211> 14
<212> DNA
<213> Artificial Sequence

<220>
<223> Synthetic Oligonucleotide

<400> 1408
ttttctgagg tttc                                                          14

<210> 1409
<211> 14
<212> DNA
<213> Artificial Sequence

<220>
<223> Synthetic Oligonucleotide

<400> 1409
agacttccat tttc                                                          14

<210> 1410
<211> 12
<212> DNA
<213> Artificial Sequence

<220>
<223> Synthetic Oligonucleotide

<400> 1410
agacttccat tt                                                            12

<210> 1411
<211> 14
<212> DNA
<213> Artificial Sequence

<220>
<223> Synthetic Oligonucleotide

<400> 1411
caaatgctat cgat                                                          14

<210> 1412
<211> 14
<212> DNA
<213> Artificial Sequence

<220>
<223> Synthetic Oligonucleotide

<400> 1412
gcacgctcta tact                                                          14

<210> 1413
<211> 12
<212> DNA
<213> Artificial Sequence

<220>
<223> Synthetic Oligonucleotide

<400> 1413
cacgctctat ac                                                            12

<210> 1414
<211> 14
<212> DNA
<213> Artificial Sequence

<220>
<223> Synthetic Oligonucleotide

<400> 1414
tccttgtcat tatc                                                          14

<210> 1415
<211> 14
<212> DNA
<213> Artificial Sequence

<220>
<223> Synthetic Oligonucleotide

<400> 1415
gctttgtcaa gatc                                                          14

<210> 1416
<211> 14
<212> DNA
<213> Artificial Sequence

<220>
<223> Synthetic Oligonucleotide

<400> 1416
tgctttgtca agat                                                          14

<210> 1417
<211> 14
<212> DNA
<213> Artificial Sequence

<220>
<223> Synthetic Oligonucleotide

<400> 1417

aagtatcggt tggc                                                          14

<210> 1418
<211> 12
<212> DNA
<213> Artificial Sequence

<220>
<223> Synthetic Oligonucleotide

<400> 1418
agtatcggtt gg                                                            12

<210> 1419
<211> 14
<212> DNA
<213> Artificial Sequence

<220>
<223> Synthetic Oligonucleotide

<400> 1419
ttaaaatttg gaga                                                          14

<210> 1420
<211> 14
<212> DNA
<213> Artificial Sequence

<220>
<223> Synthetic Oligonucleotide

<400> 1420
ccttaaaatt tgga                                                          14

<210> 1421
<211> 12
<212> DNA
<213> Artificial Sequence

<220>
<223> Synthetic Oligonucleotide

<400> 1421
cttaaaattt gg                                                            12

<210> 1422
<211> 14
<212> DNA
<213> Artificial Sequence

<220>
<223> Synthetic Oligonucleotide

<400> 1422
tctactgttt ttgt                                                          14

<210> 1423
<211> 14
<212> DNA
<213> Artificial Sequence

<220>
<223> Synthetic Oligonucleotide

<400> 1423
ggctcctcta ctgt                                                          14

<210> 1424
<211> 15
<212> DNA
<213> Artificial Sequence

<220>
<223> Synthetic Oligonucleotide

<400> 1424
agcctctgga tttga                                                         15

<210> 1425
<211> 14
<212> DNA
<213> Artificial Sequence

<220>
<223> Synthetic Oligonucleotide

<400> 1425
agcctctgga tttg                                                          14

<210> 1426
<211> 15
<212> DNA
<213> Artificial Sequence

<220>
<223> Synthetic Oligonucleotide

<400> 1426
tagcctctgg atttg                                                         15

<210> 1427
<211> 14
<212> DNA
<213> Artificial Sequence

<220>
<223> Synthetic Oligonucleotide

<400> 1427
tagcctctgg attt                                                          14

<210> 1428
<211> 16
<212> DNA
<213> Artificial Sequence

<220>
<223> Synthetic Oligonucleotide

<400> 1428
gctagcctct ggattt                                                        16

<210> 1429
<211> 15
<212> DNA
<213> Artificial Sequence

<220>
<223> Synthetic Oligonucleotide

<400> 1429
ctagcctctg gattt                                                        15

<210> 1430
<211> 15
<212> DNA
<213> Artificial Sequence

<220>
<223> Synthetic Oligonucleotide

<400> 1430
gctagcctct ggatt                                                        15

<210> 1431
<211> 14
<212> DNA
<213> Artificial Sequence

<220>
<223> Synthetic Oligonucleotide

<400> 1431
ctagcctctg gatt                                                         14

<210> 1432
<211> 13
<212> DNA
<213> Artificial Sequence

<220>
<223> Synthetic Oligonucleotide

<400> 1432
tagcctctgg att                                                          13

<210> 1433
<211> 15
<212> DNA
<213> Artificial Sequence

<220>
<223> Synthetic Oligonucleotide

<400> 1433
tgctagcctc tggat                                                        15

<210> 1434
<211> 12
<212> DNA
<213> Artificial Sequence

<220>
<223> Synthetic Oligonucleotide

<400> 1434
tagcctctgg at                                                           12

<210> 1435
<211> 15
<212> DNA
<213> Artificial Sequence

<220>

<223> Synthetic Oligonucleotide

<400> 1435
ctgctagcct ctgga                                                        15

<210> 1436
<211> 15
<212> DNA
<213> Artificial Sequence

<220>
<223> Synthetic Oligonucleotide

<400> 1436
actgctagcc tctgg                                                        15

<210> 1437
<211> 15
<212> DNA
<213> Artificial Sequence

<220>
<223> Synthetic Oligonucleotide

<400> 1437
aactgctagc ctctg                                                        15

<210> 1438
<211> 15
<212> DNA
<213> Artificial Sequence

<220>
<223> Synthetic Oligonucleotide

<400> 1438
gaactgctag cctct                                                        15

<210> 1439
<211> 15
<212> DNA
<213> Artificial Sequence

<220>
<223> Synthetic Oligonucleotide

<400> 1439
tgaactgcta gcctc                                                        15

<210> 1440
<211> 15
<212> DNA
<213> Artificial Sequence

<220>
<223> Synthetic Oligonucleotide

<400> 1440
ttgaactgct agcct                                                        15

<210> 1441
<211> 14
<212> DNA
<213> Artificial Sequence

<220>
<223> Synthetic Oligonucleotide

<400> 1441
tgaactgcta gcct                                                          14

<210> 1442
<211> 14
<212> DNA
<213> Artificial Sequence

<220>
<223> Synthetic Oligonucleotide

<400> 1442
agttgaactg ctag                                                          14

<210> 1443
<211> 12
<212> DNA
<213> Artificial Sequence

<220>
<223> Synthetic Oligonucleotide

<400> 1443
gttgaactgc ta                                                            12

<210> 1444
<211> 12
<212> DNA
<213> Artificial Sequence

<220>
<223> Synthetic Oligonucleotide

<400> 1444
gaagttgaac tg                                                            12

<210> 1445
<211> 14
<212> DNA
<213> Artificial Sequence

<220>
<223> Synthetic Oligonucleotide

<400> 1445
acagaagttg aact                                                          14

<210> 1446
<211> 14
<212> DNA
<213> Artificial Sequence

<220>
<223> Synthetic Oligonucleotide

<400> 1446
tcattgtcac taac                                                          14

<210> 1447
<211> 12
<212> DNA
<213> Artificial Sequence

<220>
<223> Synthetic Oligonucleotide

<400> 1447
cattgtcact aa                                                          12

<210> 1448
<211> 12
<212> DNA
<213> Artificial Sequence

<220>
<223> Synthetic Oligonucleotide

<400> 1448
tcaggttcat tg                                                          12

<210> 1449
<211> 14
<212> DNA
<213> Artificial Sequence

<220>
<223> Synthetic Oligonucleotide

<400> 1449
atgatcaggt tcat                                                        14

<210> 1450
<211> 14
<212> DNA
<213> Artificial Sequence

<220>
<223> Synthetic Oligonucleotide

<400> 1450
tataatgatc aggt                                                        14

<210> 1451
<211> 12
<212> DNA
<213> Artificial Sequence

<220>
<223> Synthetic Oligonucleotide

<400> 1451
ataatgatca gg                                                          12

<210> 1452
<211> 14
<212> DNA
<213> Artificial Sequence

<220>
<223> Synthetic Oligonucleotide

<400> 1452
tggtgtcaga atat                                                        14

<210> 1453
<211> 14
<212> DNA

<213> Artificial Sequence

<220>
<223> Synthetic Oligonucleotide

<400> 1453
tcagtggtgt caga                                                          14

<210> 1454
<211> 14
<212> DNA
<213> Artificial Sequence

<220>
<223> Synthetic Oligonucleotide

<400> 1454
ctctggatca gagt                                                          14

<210> 1455
<211> 14
<212> DNA
<213> Artificial Sequence

<220>
<223> Synthetic Oligonucleotide

<400> 1455
aaggttcatt ctct                                                          14

<210> 1456
<211> 14
<212> DNA
<213> Artificial Sequence

<220>
<223> Synthetic Oligonucleotide

<400> 1456
ttcatcaaaa ggtt                                                          14

<210> 1457
<211> 14
<212> DNA
<213> Artificial Sequence

<220>
<223> Synthetic Oligonucleotide

<400> 1457
cttcatcaaa aggt                                                          14

<210> 1458
<211> 12
<212> DNA
<213> Artificial Sequence

<220>
<223> Synthetic Oligonucleotide

<400> 1458
cttcatcaaa ag                                                            12

<210> 1459
<211> 14

<212> DNA
<213> Artificial Sequence

<220>
<223> Synthetic Oligonucleotide

<400> 1459
atgctgatct tcat                                                         14

<210> 1460
<211> 14
<212> DNA
<213> Artificial Sequence

<220>
<223> Synthetic Oligonucleotide

<400> 1460
ttttgtaatt tgtg                                                         14

<210> 1461
<211> 14
<212> DNA
<213> Artificial Sequence

<220>
<223> Synthetic Oligonucleotide

<400> 1461
tcagactttt gtaa                                                         14

<210> 1462
<211> 14
<212> DNA
<213> Artificial Sequence

<220>
<223> Synthetic Oligonucleotide

<400> 1462
tgtcctattg ccat                                                         14

<210> 1463
<211> 14
<212> DNA
<213> Artificial Sequence

<220>
<223> Synthetic Oligonucleotide

<400> 1463
tctgacacaa tgtc                                                         14

<210> 1464
<211> 12
<212> DNA
<213> Artificial Sequence

<220>
<223> Synthetic Oligonucleotide

<400> 1464
ctgacacaat gt                                                           12

<210> 1465

<211> 14
<212> DNA
<213> Artificial Sequence

<220>
<223> Synthetic Oligonucleotide

<400> 1465
tgttcctata actg                                                         14

<210> 1466
<211> 12
<212> DNA
<213> Artificial Sequence

<220>
<223> Synthetic Oligonucleotide

<400> 1466
gttcctataa ct                                                           12

<210> 1467
<211> 14
<212> DNA
<213> Artificial Sequence

<220>
<223> Synthetic Oligonucleotide

<400> 1467
aagattggtc agga                                                         14

<210> 1468
<211> 12
<212> DNA
<213> Artificial Sequence

<220>
<223> Synthetic Oligonucleotide

<400> 1468
agattggtca gg                                                           12

<210> 1469
<211> 14
<212> DNA
<213> Artificial Sequence

<220>
<223> Synthetic Oligonucleotide

<400> 1469
gtgtcaaaac cctg                                                         14

<210> 1470
<211> 12
<212> DNA
<213> Artificial Sequence

<220>
<223> Synthetic Oligonucleotide

<400> 1470
agctacacaa cc                                                           12

<210> 1471
<211> 14
<212> DNA
<213> Artificial Sequence

<220>
<223> Synthetic Oligonucleotide

<400> 1471
cacagctaca caac                                                         14

<210> 1472
<211> 12
<212> DNA
<213> Artificial Sequence

<220>
<223> Synthetic Oligonucleotide

<400> 1472
acagctacac aa                                                           12

<210> 1473
<211> 14
<212> DNA
<213> Artificial Sequence

<220>
<223> Synthetic Oligonucleotide

<400> 1473
tatatacatg acac                                                         14

<210> 1474
<211> 12
<212> DNA
<213> Artificial Sequence

<220>
<223> Synthetic Oligonucleotide

<400> 1474
atatacatga ca                                                           12

<210> 1475
<211> 14
<212> DNA
<213> Artificial Sequence

<220>
<223> Synthetic Oligonucleotide

<400> 1475
aattttaaat gtcc                                                         14

<210> 1476
<211> 12
<212> DNA
<213> Artificial Sequence

<220>
<223> Synthetic Oligonucleotide

<400> 1476
attttaaatg tc                                                           12

<210> 1477
<211> 12
<212> DNA
<213> Artificial Sequence

<220>
<223> Synthetic Oligonucleotide

<400> 1477
tcctaattga at                                                          12

<210> 1478
<211> 12
<212> DNA
<213> Artificial Sequence

<220>
<223> Synthetic Oligonucleotide

<400> 1478
aaagtgccat ct                                                          12

<210> 1479
<211> 14
<212> DNA
<213> Artificial Sequence

<220>
<223> Synthetic Oligonucleotide

<400> 1479
tttataaaac tgga                                                        14

<210> 1480
<211> 12
<212> DNA
<213> Artificial Sequence

<220>
<223> Synthetic Oligonucleotide

<400> 1480
ttataaaact gg                                                          12

<210> 1481
<211> 14
<212> DNA
<213> Artificial Sequence

<220>
<223> Synthetic Oligonucleotide

<400> 1481
tgcaaactta tctg                                                        14

<210> 1482
<211> 12
<212> DNA
<213> Artificial Sequence

<220>
<223> Synthetic Oligonucleotide

<400> 1482

gcaaacttat ct                                                            12

<210> 1483
<211> 12
<212> DNA
<213> Artificial Sequence

<220>
<223> Synthetic Oligonucleotide

<400> 1483
agccaactgc aa                                                            12

<210> 1484
<211> 14
<212> DNA
<213> Artificial Sequence

<220>
<223> Synthetic Oligonucleotide

<400> 1484
cttagccaac tgca                                                          14

<210> 1485
<211> 12
<212> DNA
<213> Artificial Sequence

<220>
<223> Synthetic Oligonucleotide

<400> 1485
ttagccaact gc                                                            12

<210> 1486
<211> 14
<212> DNA
<213> Artificial Sequence

<220>
<223> Synthetic Oligonucleotide

<400> 1486
taaatcattg tcaa                                                          14

<210> 1487
<211> 14
<212> DNA
<213> Artificial Sequence

<220>
<223> Synthetic Oligonucleotide

<400> 1487
gcactggcct tgat                                                          14

<210> 1488
<211> 12
<212> DNA
<213> Artificial Sequence

<220>
<223> Synthetic Oligonucleotide

<400> 1488
cactggcctt ga                                                            12

<210> 1489
<211> 12
<212> DNA
<213> Artificial Sequence

<220>
<223> Synthetic Oligonucleotide

<400> 1489
ttagcactgg cc                                                            12

<210> 1490
<211> 12
<212> DNA
<213> Artificial Sequence

<220>
<223> Synthetic Oligonucleotide

<400> 1490
tgtgtaaggt ca                                                            12

<210> 1491
<211> 12
<212> DNA
<213> Artificial Sequence

<220>
<223> Synthetic Oligonucleotide

<400> 1491
gttaatgaca tt                                                            12

<210> 1492
<211> 12
<212> DNA
<213> Artificial Sequence

<220>
<223> Synthetic Oligonucleotide

<400> 1492
gacaatttct ac                                                            12

<210> 1493
<211> 12
<212> DNA
<213> Artificial Sequence

<220>
<223> Synthetic Oligonucleotide

<400> 1493
aacactgcac at                                                            12

<210> 1494
<211> 14
<212> DNA
<213> Artificial Sequence

<220>
<223> Synthetic Oligonucleotide

<400> 1494
taggtcaagt ctaa                                                          14

<210> 1495
<211> 14
<212> DNA
<213> Artificial Sequence

<220>
<223> Synthetic Oligonucleotide

<400> 1495
tataggtcaa gtct                                                          14

<210> 1496
<211> 14
<212> DNA
<213> Artificial Sequence

<220>
<223> Synthetic Oligonucleotide

<400> 1496
tttggataaa tata                                                          14

<210> 1497
<211> 14
<212> DNA
<213> Artificial Sequence

<220>
<223> Synthetic Oligonucleotide

<400> 1497
tgggacttca cacc                                                          14

<210> 1498
<211> 12
<212> DNA
<213> Artificial Sequence

<220>
<223> Synthetic Oligonucleotide

<400> 1498
accacagcta gt                                                            12

<210> 1499
<211> 12
<212> DNA
<213> Artificial Sequence

<220>
<223> Synthetic Oligonucleotide

<400> 1499
gggacttcac ac                                                            12

<210> 1500
<211> 14
<212> DNA
<213> Artificial Sequence

<220>

<223> Synthetic Oligonucleotide

<400> 1500
ccaaaaacct tact                                                          14

<210> 1501
<211> 12
<212> DNA
<213> Artificial Sequence

<220>
<223> Synthetic Oligonucleotide

<400> 1501
gtggcaacca ca                                                            12

<210> 1502
<211> 12
<212> DNA
<213> Artificial Sequence

<220>
<223> Synthetic Oligonucleotide

<400> 1502
aaggcttaaa gt                                                            12

<210> 1503
<211> 12
<212> DNA
<213> Artificial Sequence

<220>
<223> Synthetic Oligonucleotide

<400> 1503
aggacttggg at                                                            12

<210> 1504
<211> 12
<212> DNA
<213> Artificial Sequence

<220>
<223> Synthetic Oligonucleotide

<400> 1504
gggaatcaga gc                                                            12

<210> 1505
<211> 12
<212> DNA
<213> Artificial Sequence

<220>
<223> Synthetic Oligonucleotide

<400> 1505
atttgatgct gc                                                            12

<210> 1506
<211> 12
<212> DNA
<213> Artificial Sequence

<220>
<223> Synthetic Oligonucleotide

<400> 1506
ttccaatgac ta                                                          12

<210> 1507
<211> 12
<212> DNA
<213> Artificial Sequence

<220>
<223> Synthetic Oligonucleotide

<400> 1507
atcaacagct gc                                                          12

<210> 1508
<211> 14
<212> DNA
<213> Artificial Sequence

<220>
<223> Synthetic Oligonucleotide

<400> 1508
gattgcaagt tccg                                                        14

<210> 1509
<211> 12
<212> DNA
<213> Artificial Sequence

<220>
<223> Synthetic Oligonucleotide

<400> 1509
tgcttggaag ga                                                          12

<210> 1510
<211> 14
<212> DNA
<213> Artificial Sequence

<220>
<223> Synthetic Oligonucleotide

<400> 1510
ctgctgcaca tcca                                                        14

<210> 1511
<211> 12
<212> DNA
<213> Artificial Sequence

<220>
<223> Synthetic Oligonucleotide

<400> 1511
cacattaaca gt                                                          12

<210> 1512
<211> 12
<212> DNA
<213> Artificial Sequence

<220>
<223> Synthetic Oligonucleotide

<400> 1512
agttgaaatt tc                                                          12

<210> 1513
<211> 12
<212> DNA
<213> Artificial Sequence

<220>
<223> Synthetic Oligonucleotide

<400> 1513
accctcattc ag                                                          12

<210> 1514
<211> 12
<212> DNA
<213> Artificial Sequence

<220>
<223> Synthetic Oligonucleotide

<400> 1514
agaatgagac tt                                                          12

<210> 1515
<211> 12
<212> DNA
<213> Artificial Sequence

<220>
<223> Synthetic Oligonucleotide

<400> 1515
gtaaatccta ag                                                          12

<210> 1516
<211> 12
<212> DNA
<213> Artificial Sequence

<220>
<223> Synthetic Oligonucleotide

<400> 1516
taaatatagg tc                                                          12

<210> 1517
<211> 12
<212> DNA
<213> Artificial Sequence

<220>
<223> Synthetic Oligonucleotide

<400> 1517
aatgccttat ta                                                          12

<210> 1518
<211> 14
<212> DNA

<213> Artificial Sequence

<220>
<223> Synthetic Oligonucleotide

<400> 1518
caccttaaaa tttg                                                         14

<210> 1519
<211> 12
<212> DNA
<213> Artificial Sequence

<220>
<223> Synthetic Oligonucleotide

<400> 1519
gtgtacagat tt                                                           12

<210> 1520
<211> 12
<212> DNA
<213> Artificial Sequence

<220>
<223> Synthetic Oligonucleotide

<400> 1520
ctttcagtca ta                                                           12

<210> 1521
<211> 12
<212> DNA
<213> Artificial Sequence

<220>
<223> Synthetic Oligonucleotide

<400> 1521
gggcatatca aa                                                           12

<210> 1522
<211> 12
<212> DNA
<213> Artificial Sequence

<220>
<223> Synthetic Oligonucleotide

<400> 1522
tgaggcatta tc                                                           12

<210> 1523
<211> 4
<212> DNA
<213> Artificial Sequence

<220>
<223> Synthetic Oligonucleotide

<400> 1523
actg                                                                    4

<210> 1524
<211> 19

<212> DNA
<213> Artificial Sequence

<220>
<223> Primer

<400> 1524
cgtgggctcc agcattcta                                                    19

<210> 1525
<211> 21
<212> DNA
<213> Artificial Sequence

<220>
<223> Primer

<400> 1525
agtcatttct gcctttgcgt c                                                 21

<210> 1526
<211> 22
<212> DNA
<213> Artificial Sequence

<220>
<223> Probe

<400> 1526
ccaatggtcg ggcactgctc aa                                                22

<210> 1527
<211> 30
<212> DNA
<213> Artificial Sequence

<220>
<223> Primer

<400> 1527
gaaaatagac ttcctgaata actatgcatt                                        30

<210> 1528
<211> 18
<212> DNA
<213> Artificial Sequence

<220>
<223> Primer

<400> 1528
actcgcttgc cagcttgc                                                     18

<210> 1529
<211> 23
<212> DNA
<213> Artificial Sequence

<220>
<223> Probe

<400> 1529
tttctgagtc cccgtgccca aca                                               23

<210> 1530

<211> 20
<212> DNA
<213> Artificial Sequence

<220>
<223> Primer

<400> 1530
tgagccttgc caccttctct                                                    20

<210> 1531
<211> 15
<212> DNA
<213> Artificial Sequence

<220>
<223> Primer

<400> 1531
gcgcacccca gccaa                                                         15

<210> 1532
<211> 29
<212> DNA
<213> Artificial Sequence

<220>
<223> Probe

<400> 1532
agaggagctt cttttccctc tacctgggc                                          29

<210> 1533
<211> 20
<212> DNA
<213> Artificial Sequence

<220>
<223> Primer

<400> 1533
atttcctgcc cctggtacct                                                    20

<210> 1534
<211> 17
<212> DNA
<213> Artificial Sequence

<220>
<223> Primer

<400> 1534
cgggcccaca cctcttg                                                       17

<210> 1535
<211> 26
<212> DNA
<213> Artificial Sequence

<220>
<223> Probe

<400> 1535
ccacaaagtg cagcaccgcc tagtgt                                             26

<210> 1536
<211> 19
<212> DNA
<213> Artificial Sequence

<220>
<223> Primer

<400> 1536
gccacaggct cccagacat                                                    19

<210> 1537
<211> 25
<212> DNA
<213> Artificial Sequence

<220>
<223> Primer

<400> 1537
tccatcctct tgatatctcc ttttg                                             25

<210> 1538
<211> 31
<212> DNA
<213> Artificial Sequence

<220>
<223> Probe

<400> 1538
acagccatca tcaaagagat cgttagcaga a                                      31

<210> 1539
<211> 24
<212> DNA
<213> Artificial Sequence

<220>
<223> Primer

<400> 1539
atgacaatca tgttgcagca attc                                              24

<210> 1540
<211> 25
<212> DNA
<213> Artificial Sequence

<220>
<223> Primer

<400> 1540
cgatgcaata aatatgcaca aatca                                             25

<210> 1541
<211> 28
<212> DNA
<213> Artificial Sequence

<220>
<223> Probe

<400> 1541
ctgtaaagct ggaaagggac ggactggt                                          28

<210> 1542
<211> 14
<212> DNA
<213> Artificial Sequence

<220>
<223> Synthetic Oligonucleotide

<400> 1542
gtgtgcacac agct                                                         14

<210> 1543
<211> 12
<212> DNA
<213> Artificial Sequence

<220>
<223> Synthetic Oligonucleotide

<400> 1543
cagcctgggc ac                                                           12

<210> 1544
<211> 14
<212> DNA
<213> Artificial Sequence

<220>
<223> Synthetic Oligonucleotide

<400> 1544
tgctcgaact cctt                                                         14

<210> 1545
<211> 14
<212> DNA
<213> Artificial Sequence

<220>
<223> Synthetic Oligonucleotide

<400> 1545
gaagtcactg gctt                                                         14

<210> 1546
<211> 14
<212> DNA
<213> Artificial Sequence

<220>
<223> Synthetic Oligonucleotide

<400> 1546
gggaagtcac tggc                                                         14

<210> 1547
<211> 14
<212> DNA
<213> Artificial Sequence

<220>
<223> Synthetic Oligonucleotide

<400> 1547

gttaggcaaa gggc                                                          14

<210> 1548
<211> 14
<212> DNA
<213> Artificial Sequence

<220>
<223> Synthetic Oligonucleotide

<400> 1548
gggctgagtg accc                                                          14

<210> 1549
<211> 14
<212> DNA
<213> Artificial Sequence

<220>
<223> Synthetic Oligonucleotide

<400> 1549
atgctagtgc acta                                                          14

<210> 1550
<211> 14
<212> DNA
<213> Artificial Sequence

<220>
<223> Synthetic Oligonucleotide

<400> 1550
agctcgctac ctct                                                          14

<210> 1551
<211> 14
<212> DNA
<213> Artificial Sequence

<220>
<223> Synthetic Oligonucleotide

<400> 1551
gaggtatccc atct                                                          14

<210> 1552
<211> 14
<212> DNA
<213> Artificial Sequence

<220>
<223> Synthetic Oligonucleotide

<400> 1552
ggcaacttca acct                                                          14

<210> 1553
<211> 20
<212> DNA
<213> Artificial Sequence

<220>
<223> Synthetic Oligonucleotide

<400> 1553
gaagtagcca ccaactgtgc                                                    20

<210> 1554
<211> 12
<212> DNA
<213> Artificial Sequence

<220>
<223> Synthetic Oligonucleotide

<400> 1554
tagccaccaa ct                                                            12

<210> 1555
<211> 4
<212> DNA
<213> Artificial Sequence

<220>
<223> Synthetic Oligonucleotide

<400> 1555
actg                                                                      4

<210> 1556
<211> 4
<212> DNA
<213> Artificial Sequence

<220>
<223> Synthetic Oligonucleotide

<400> 1556
actg                                                                      4

<210> 1557
<211> 10
<212> DNA
<213> Artificial Sequence

<220>
<223> Synthetic Oligonucleotide

<400> 1557
agccaccaac                                                               10

<210> 1558
<211> 8
<212> DNA
<213> Artificial Sequence

<220>
<223> Synthetic Oligonucleotide

<400> 1558
gccaccaa                                                                  8

<210> 1559
<211> 13
<212> DNA
<213> Artificial Sequence

<220>
<223> Synthetic Oligonucleotide

<400> 1559
tagccaccaa ctg                                                          13

<210> 1560
<211> 12
<212> DNA
<213> Artificial Sequence

<220>
<223> Synthetic Oligonucleotide

<400> 1560
taccgaacac ct                                                           12

<210> 1561
<211> 20
<212> DNA
<213> Artificial Sequence

<220>
<223> Synthetic Oligonucleotide

<400> 1561
gtccctgaag atgtcaatgc                                                   20

<210> 1562
<211> 20
<212> DNA
<213> Artificial Sequence

<220>
<223> Synthetic Oligonucleotide

<400> 1562
tgcactttgt ggtaccaagg                                                   20

<210> 1563
<211> 14
<212> DNA
<213> Artificial Sequence

<220>
<223> Synthetic Oligonucleotide

<400> 1563
gcttctccat cata                                                         14

<210> 1564
<211> 14
<212> DNA
<213> Artificial Sequence

<220>
<223> Synthetic Oligonucleotide

<400> 1564
tgtcttgctg cttt                                                         14

<210> 1565
<211> 4
<212> DNA
<213> Artificial Sequence

<220>

<223> Synthetic Oligonucleotide

<400> 1565
actg                                                                    4

<210> 1566
<211> 4
<212> DNA
<213> Artificial Sequence

<220>
<223> Synthetic Oligonucleotide

<400> 1566
actg                                                                    4

<210> 1567
<211> 4
<212> DNA
<213> Artificial Sequence

<220>
<223> Synthetic Oligonucleotide

<400> 1567
actg                                                                    4

<210> 1568
<211> 20
<212> DNA
<213> Artificial Sequence

<220>
<223> Synthetic Oligonucleotide

<400> 1568
atggcttctc catcatatcc                                                  20

<210> 1569
<211> 20
<212> DNA
<213> Artificial Sequence

<220>
<223> Synthetic Oligonucleotide

<400> 1569
cttgggcatg ctcgtcagtc                                                  20

<210> 1570
<211> 20
<212> DNA
<213> Artificial Sequence

<220>
<223> Synthetic Oligonucleotide

<400> 1570
ccttccctga aggttcctcc                                                  20

<210> 1571
<211> 20
<212> DNA
<213> Artificial Sequence

<220>
<223> Synthetic Oligonucleotide

<400> 1571
ctgctagcct ctggatttga                                                   20

<210> 1572
<211> 4
<212> DNA
<213> Artificial Sequence

<220>
<223> Synthetic Oligonucleotide

<400> 1572
actg                                                                     4

<210> 1573
<211> 14
<212> DNA
<213> Artificial Sequence

<220>
<223> Synthetic Oligonucleotide

<400> 1573
gaatatctat aatg                                                         14

<210> 1574
<211> 12
<212> DNA
<213> Artificial Sequence

<220>
<223> Synthetic Oligonucleotide

<400> 1574
gtaagcaagg ct                                                           12

<210> 1575
<211> 2254
<212> DNA
<213> Rattus norvegicus

<400> 1575
agagaatgga gggacacgta gaggaaggct ctgaacttgg ggagcagaag gtcctgattg 60
ataatcctgc tgacattctg gtcattgccg cgtatttcct gctggtcatt ggtgttggct 120
tgtggtctat gttcagaacc aatagaggca cagttggtgg ctacttcttg gcaggacgga 180
gcatggtgtg gtggccggtt ggagcctctc tgttcgccag caacatcggc agcggtcatt 240
ttgtgggcct ggcggggact ggtgcagcaa gtggcttggc cgtggctgga tttgagtgga 300
atgcgctctt tgtggtgttg ctcctcggct ggctcttcgt gcctgtgtat ctgaccgccg 360
gagtgattac catgcctcag tacctccgca agcgctttgg tgggcgccgt attcgcctct 420
acctgtccgt gctctcgctt tttttgtaca ttttcaccaa gatctcggtg gatatgttct 480
ccggagcagt attcattcaa caggccctgg gctggaacat ttacgcttct gtcatcgcac 540
tcttgggcat caccatgatt tatactgtga caggagggct ggcggcactg atgtacacag 600
acactgtgca gaccttcgtc attcttgccg gggccttcat cctcactggt tatgctttcc 660
atgaagtggg cgggtactca ggtctcttcg acaaatacct gggagcagtg acttcactga 720
cggtgtccaa ggatccagct gttggcaaca tctccagcac ctgctatcag ccgaggcccg 780
actcctatca cctgctgcgt gaccctgtga caggagggct gccgtggcct gcgctgcttc 840
tggggcttac cattgtctca ggctggcact ggtgcagtga ccaggtaatt gtgcagcgct 900
gcctggctgg aaagaatctg actcacatca aggctggatg catcctgtgt ggttacctga 960
agctgatgcc catgttcctc atggtcatgc caggcatgat tagccgcatt ctttacccag 1020
atgaggtggc ctgtgtggta cctgaggtgt gtaagcgggt gtgtggcact gaggtgggct 1080
gctctaacat cgcctaccca cggcttgttg tgaagctcat gcccaatggt ctgcgtggac 1140
tcatgctggc agtcatgctg gccgccctca tgtcttctct agcgtccatc tttaacagca 1200

gcagcacact cttcaccatg gatatctaca cacgcctgcg gcctcgggcg ggtgataggg 1260
agctgctgct ggttggaagg ctttgggtgg tgttcattgt ggcagtgtcc gtggcttggc 1320
tgccagtggt gcaggcagca cagggcggcc agctcttcga ttacattcag tctgtctcca 1380
gctacctggc gcctccagtg tccgctgtct ttgtgctggc actctttgtg ccccgtgtta 1440
atgagaaggg tgctttctgg ggactcattg ggggcctgct gatgggccta gctcgtctca 1500
tacccgagtt cttctttggc acgggcagct gtgtgcgacc ctctgcatgc ccagccatct 1560
tctgccgggt acactacctc tacttcgcca tcattctctt cttctgctcc ggcttcctca 1620
cactcgcaat ctcccggtgc accgcaccca tccctcagaa gcatctccac cgcctggttt 1680
tcagtctccg gcacagcaag gaggagcggg aggatctgga tgctgaagag ttagagggtc 1740
cagcccctcc tcctgtgcag aacgggtgcc aggaatgtgc aatggggatt gaagaggtcc 1800
agtccccagc tccaggcctg ctccgccagt gcttgctctg gttctgtggg atgagcaaga 1860
gtgggtcagg gagtcctcca cccactaccg aggaggtggc tgcaaccacc aggcggctag 1920
aggacatcag tgaggatccc agctgggccc gagtagtcaa cctcaatgcc ctgctcatga 1980
tgactgtggc tgtgttcctc tggggctttt atgcataaag tcaagtgtgt tggttaccat 2040
gagttgcaac cagggcatgt ctgagcctca tgaaaagtga gggtgaagaa cttgtagtgg 2100
atcccagaaa agggcaaaaa tacggcagga aggaactggt tcccttcctc tttacctggg 2160
gtccagtcca tttgattggt tgtcacttcc cacaagatgg tggccaattt gtcatagatg 2220
tttgcctatg caaaaataaa gctgcccttc taac 2254

<210> 1576
<211> 3300
<212> DNA
<213> Mus musculus

<400> 1576
tacatcaatg gaatttaaat agagtcgtca tataataagg gaaacaatgt cccaactgga 60
catcacatgg taccaaacaa aagacccagt accaggaatg agttaccatc ttatgaagtc 120
attaaccata gagagctaat ggtagcctca atcattacaa aagctgttgc caaggttact 180
gggtgctctc ataccctgat ggtaagaccc tatggctgaa gatggaactt attgatatcc 240
ttgaacattg agaaattgag ctgtgtgact agaagcttca ccccatcgac gatggtcaca 300
gtgctgcaaa gtgctatgtt caggaggaag gtatccagcc gcctccctca gccacactct 360
gagactcaca ccagagacct gctcaaaggg catgcccact ggcccacaaa tattatggga 420
gcaactgacc acttttgggg ggtgtattta aggtccactt catgaaatgg gacccatccc 480
tgacactgct aaaatgcctg agaacctgaa actagataga gcaagggctc taggggaaag 540
ctcactccag ttattctaag ggcacagggt tatgacgcct aatgacatat cgctggctac 600
atcccttggc cagcacatca ctgaaacctc accagaggag cttcttggag tagaaggtga 660
ttaacagaac tgtccgtgac tggatgactt gcagagagtg agagacttgg gagcactcag 720
acttcaatgg gatgcttgta tctcacccct ctgctaaaga tgcaggttct atacaggagg 780
tgcaaagatt gtaagagtca gagttggagg ttgtcttcag gaaagcagag ttttgcagac 840
acaacaaagc tgatgaaaat ctgaactcac agacagtgat agcatgcaca agacagatat 900
ctgaactcac agaccgtgat aacatggcac aaagacctgc acaaagttcg aaccaaataa 960
aatctgagca tggaaaatga ggtgtgggca caaagtccca ctcctaagta agaaactact 1020
tgcagttgac agctactagg agagagaaat gggtttcttt caatggagag acactgggtg 1080
tatcaactgc accccaaggc aggccacacg ctcaggaaga gttggccaac acaaaacaca 1140
ctccgtgttt ggttttctgt ttgcttgggt ttgtttttgt gcttttattc ttccttcctt 1200
tctttctttg tttctttgtt tctctctctc tttctctctt tttctctctc cctcctcctc 1260
ttcttctttc ttctttttct tcttcttctt cttcttcttc tttttcttct tcttcttctt 1320
ctttttcttc ttcttcttct tcttcttctt cttcttcttc ttcttcttct tcttcttctt 1380
cttcttcttc ttcttcttct tctgatcaga gaggggaggg gatctgggaa aagtttgggg 1440
agaggaaaga atttgcccaa aatatattgc ataaaaactt tttaaaaata aatttaaaac 1500
aatttttagg atagagcaaa gagaaagtag aaaatatttg ggttgggaag ggcaggagaa 1560
tgagggaaat gtgatttttt tctccctagg ttttgtatgg cagaagtcag ggcatggcat 1620
gcgtgacagt caggctgagg aacatgtatg tcctgctgac tgtcaggggg tgctatggag 1680
gacttgtgcg gaggacactg tcagagttgg attcggacct tcctaatcaa agttagaggg 1740
tgtattttca gagaacgcag gaaggaactt tgcttggaac actgggtata ggatggatcc 1800
taaacccagg aaggagtgct cttgaattcc aaatggtcca gcgccccagg accagccttc 1860
ggccttgata gatcctgatt cagataaata aagctggaga aggaggctga gacctggggg 1920
acttgtcggg tcagtgctcc tgaggtaacc attaatcctt cccccagggg aatccaggga 1980
ctagcccctt gagggacaga tggtggagag aatggagcaa cacgtagagg caggctctga 2040
acttggggag cagaaggtcc tgattgataa tcctgctgac attctggtta tcgctgccta 2100
tttcctgctg gtcattggtg ttggcttgtg ggtgagacat tgaggggggt tggataggga 2160
aatgcttctg ggcttgaggg gtaaagattt agggagacct cagagaggag tgggagaaaa 2220
gggtgcttgg atataatgag ggagaaacct agatttagta ggcaagccaa ttttaattct 2280
ttgtcttcgt accttctgga ttgtgcaaaa gagactgggg gtatcaatag gttttttttt 2340
aattcaagtg ttctaacaag tgctctaaga gatgtatcag ttcccacgtc tgtattatgg 2400

ctgagcagca gcctatattt aaggtcacca ggcaagttag gctgaatcta ggcatatcta 2460
ggttccagta gttgcgctag gattagggcc tgggttgttc tgagtgtcgg ggaaggttgg 2520
gggtaaggag gtgcagtctg gggagtccag ggctggttaa tcttcagcct gaaacaaggc 2580
tgaggaatgt gttgaggaag ctaaggaagt ccaaagatgt gccccaatcc cagtttcccc 2640
ccacttctgt ttcccagtct atgttcagaa ccaatagagg cacagttggt ggctacttcc 2700
tggcaggacg gaacatggtg tggtggccgg tgagaggggc tgggggatgg gatgggaggg 2760
accctggagg agcaccctac ctcaccccca tttctggcca cccaggttgg agcctctctg 2820
ttcgccagca acatcggcag cggtcatttt gtgggcctgg cagggactgg tgcagcaagt 2880
ggcttggcgg tggctggatt tgagtggaat gtgaggtctt cttttctata ataatccacc 2940
ccagtggaaa ctcctggaaa gatcacaact tggggaagct cctggcgtgg ggatatgaac 3000
aagttgggac agcacagttt gctgagggtg ggtgggtgtt aggttgggct gggagcaagg 3060
atcgcgtgct agcttacagc agtagggttc aggcaggtcc tgctcctgac tttctgtata 3120
cgccagcatc agtcctcgtg ggccttgacc actctgaaat ggggccggca cctcactgat 3180
gaagtacctg tctccatgtt ctttgatgct gtagtctgtg agcaactcta ggctgaactg 3240
aagttctttg caaaaacaag gatgggttgg aaagatgagg gtttgagcct tcctgtaaga 3300

## Description

### BACKGROUND

Targeting disease-causing gene sequences was first suggested nearly 40 years ago (Belikova et al., Tet. Lett., 1967, 37, 3557-3562), and antisense activity was demonstrated in cell culture a decade later (Zamecnik et al., Proc. Natl. Acad. Sci. U.S.A., 1978, 75, 280-284). One advantage of antisense technology in the treatment of a disease or condition that stems from a disease-causing gene is that it is a direct genetic approach that has the ability to modulate expression of specific disease-causing genes.

Generally, the principle behind antisense technology is that an antisense compound hybridizes to a target nucleic acid and effects modulation of gene expression activity or function, such as transcription, translation or splicing. The modulation of gene expression can be achieved by, for example, target degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi is a form of antisense-mediated gene silencing involving the introduction of dsRNA leading to the sequence-specific reduction of targeted endogenous mRNA levels. Sequence-specificity makes antisense compounds extremely attractive as tools for target validation and gene functionalization, as well as research tools for identifying and characterizing nucleases and as therapeutics to selectively modulate the expression of genes involved in the pathogenesis of any one of a variety of diseases.

Antisense technology is an effective means for reducing the expression of one or more specific gene products and can therefore prove to be uniquely useful in a number of therapeutic, diagnostic, and research applications. Chemically modified nucleosides are routinely used for incorporation into antisense compounds to enhance one or more properties, such as nuclease resistance, pharmacokinetics or affinity for a target RNA.

Despite the expansion of knowledge since the discovery of antisense technology, there remains an unmet need for antisense compounds with greater efficacy, reduced toxicity and lower cost. Until the present disclosure, high-affinity modifications have not been employed in the design of short antisense compounds for reducing target RNA *in vivo.* This is because of concerns regarding the degree of target specificity that a sequence 15 nucleotides or shorter would have when employed to reduce target in a living system. Previous studies have described that greater specificity, and therefore greater potential for potency, is achieved by antisense compounds between 16 and 20 nucleobases in length.

The present disclosure describes incorporation of chemically-modified high-affinity nucleotides into antisense compounds allows for short antisense compounds about 8-16 nucleobases in length useful in the reduction of target RNAs in animals with increased potency and improved therapeutic index. Thus, provided herein are short antisense compounds comprising high-affinity nucleotide modifications useful for reducing a target RNA *in vivo.* Such short antisense compounds are effective at lower doses than previously described antisense compounds, allowing for a reduction in toxicity and cost of treatment.

### SUMMARY

Disclosed herein are short antisense compounds and methods of using said compounds to reduce target RNA expression in cells or tissues. In certain embodiments, provided herein is a method of reducing expression of a target in an animal, comprising administering to the animal a short antisense compound targeted to a nucleic acid of such target. In certain embodiments, shorts antisense compounds are oligonucleotide compounds. In certain embodiments short antisense oligonucleotides are about 8 to 16, preferably 9 to 15, more preferably 9 to 14, more preferably 10 to 14 nucleotides in length and comprises a gap region flanked on each side by a wing, wherein each wing independently consists of 1 to 3 nucleotides. Preferred motifs include but are not limited to wing - deoxy gap - wing motifs selected from 3-10-3, 2-10-3, 2-10-2, 1-10-1, 2-8-2, 1-8-1, 3-6-3 or 1-6-1. In a preferred embodiment, the short antisense oligonucleotide comprise at least one high-affinity modification. In a further embodiment, the high-affinity modification includes chemically-modified high-affinity nucleotides. In a preferred embodiment, each wing independently consists of 1 to 3 high-affinity modified nucleotides. In one embodiment the high affinity modified nucleotides are sugar-modified nucleotides.

In certain embodiments short antisense compounds exhibit greater uptake in the gut as compared to antisense compounds of greater length. Thus, also provided herein are methods of reducing a target in an animal, comprising orally administering the short antisense compounds of the present invention.

In certain embodiments, short antisense compounds are targeted to a nucleic acid encoding a protein selected from ApoB, SGLT2, PCSK9, SOD1, CRP, GCCR, GCGR, DGAT2, PTP1B and PTEN.

Further provided are methods of treating a metabolic disorder in an animal, comprising administering to an animal in need of such therapy a short antisense compound targeted to a nucleic acid involved in regulating glucose metabolism or clearance, lipid metabolism, cholesterol metabolism, or insulin signaling.

Also provided are methods of increasing insulin sensitivity, decreasing blood glucose or decreasing HbA_{1c} in an animal, comprising administering to said animal a short antisense compound targeted to a nucleic acid encoding a target involved in regulating glucose metabolism or clearance, lipid metabolism, cholesterol metabolism, or insulin signaling.

Further provided are methods of decreasing total serum cholesterol, serum LDL, serum VLDL, serum HDL, serum triglycerides, serum apolipoprotein(a) or free fatty acids in an animal, comprising administering to said animal a short antisense compound targeted to a nucleic acid encoding a target that is involved in regulating glucose metabolism or clearance, lipid metabolism, cholesterol metabolism, or insulin signaling, wherein said short antisense compound is 8 to 16 nucleotides in length and comprises a gap region flanked on each side by a wing, wherein each wing independently consists of 1 to 3 high-affinity modified nucleotides.

Certain targets involved in regulating glucose metabolism or clearance, lipid metabolism, cholesterol metabolism, or insulin signaling include, but are not limited to, GCGR and ApoB-100. Thus, provided are short antisense compounds targeting nucleic acids encoding GCGR and ApoB-100 and methods of reducing expression of said targets and/or target nucleic acids in animal. In addition, provided is the use of short antisense compounds targeting nucleic acids encoding GCGR, and ApoB-100 for the treatment of a metabolic or cardiovascular disease or condition.

In certain embodiments, short antisense compounds further comprise a conjugate group. Conjugate groups include, but are not limited to, C₁₆ and cholesterol.

In certain embodiments short antisense compounds comprise at least one modified nucleobase, internucleoside linkage or sugar moiety. In certain embodiments, such modified internucleoside linkage is a phosphorothioate internucleoside linkage. In certain embodiments, each internucleoside linkage is a phosphorothioate internucleoside linkage.

In certain embodiments, short antisense compounds comprise at least one high affinity modification. In certain such embodiments, the high-affinity modification is a chemically-modified high-affinity nucleotide. In certain embodiments, chemically-modified high affinity nucleotides are sugar-modified nucleotides. In certain embodiments, at least one of the sugar-modified nucleotides comprises a bridge between the 4' and the 2' position of the sugar. Each of the sugar-modified nucleotides is, independently, in the β-D or α-L sugar conformation. In certain embodiments, each of said high-affinity modified nucleotides confers a ΔTₘ of at least 1 to 4 degrees per nucleotide. In certain embodiments, each of said sugar-modified nucleotides comprises a 2'-substituent group that is other than H or OH. Such sugar-modified nucleotides include those having a 4' to 2' bridged bicyclic sugar moiety. In certain embodiments, each of the 2'-substituent groups is, independently, alkoxy, substituted alkoxy, or halogen. In certain embodiments, each of the 2'-substituent groups is OCH₂CH₂OCH₃ (2'-MOE).

In certain embodiments, short antisense compounds have one or more sugar-modified nucleotides comprising a bridge between the 4' and 2' position of the sugar, wherein each of said bridges independently comprises from 2 to 4 linked groups independently selected from -[C(R₁)(R₂)]ₙ-, -C(R₁)=C(R₂)-, -C(R₁)=N, -C(=NR₁)-, -C(=O)-, -C(=S)-, -O-, -Si(R₁)₂-, -S(=O)ₓ- and -N(R₁)-;
wherein
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂ The-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one aspect, each of said bridges is, independently, -[C(R₁)(R₂)]ₙ-, -[C(R₁)(R₂)]ₙ-O-, -C(R₁R₂)-N(R₁)-O- or -C(R₁R₂)-O-N(R₁)-. In another aspect, each of said bridges is, independently, 4'-(CH₂)₃-2', 4'-(CH₂)₂-2', 4'-CH₂-O-2', 4'-(CH₂)₂-O-2', 4'-CH₂-O-N(R₁)-2' and 4'-CH₂-N(R₁)-O-2'- wherein each R₁ is, independently, H, a protecting group or C₁-C₁₂ alkyl.

In certain embodiments, provided herein are short antisense compounds useful in the reduction of targets and/or target RNAs associated with disease states in animals. In certain embodiments, provided are methods of using the short antisense compounds for reducing expression of a target RNA in an animal. In certain embodiments, provided herein is the use of a short antisense compound in the preparation of a medicament for the treatment of a metabolic disorder in an animal. In certain embodiments, provided herein is the use of a short antisense compound in the preparation of a medicament for increasing insulin sensitivity, decreasing blood glucose or decreasing HbA_{1c} in an animal. Also provided is the use of a short antisense compound in the preparation of a medicament for decreasing total serum cholesterol, serum LDL, serum VLDL, serum HDL, serum triglycerides, serum apolipoprotein(a) or free fatty acids in an animal.

In certain embodiments, short antisense compounds provided herein exhibit equal or increased potency with regard to target RNA knockdown as compared to longer parent antisense oligonucleotide at least 20 nucleotides in length. In certain embodiments, short antisense compounds exhibit a faster onset of action (target RNA reduction) as compared to the parent antisense oligonucleotide. In certain embodiments, increased potency is in the kidney. In certain embodiments, target RNA is predominately expressed in the kidney. In certain embodiments, increased potency is in the liver. In certain embodiments, target RNA is predominately expressed in the liver.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated by reference in their entirety for any purpose. US patent application serial nos 10/712,795 and 10/200,710 are hereby expressly incorporated by reference in their entirety for any purpose.

### A. Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, chemical analysis, pharmaceutical preparation, formulation and delivery, and treatment of subjects. Certain such techniques and procedures may be found for example in "Carbohydrate Modifications in Antisense Research" Edited by Sangvi and Cook, American Chemical Society , Washington D.C., 1994; and "Remington's Pharmaceutical Sciences," Mack Publishing Co., Easton, Pa., 18th edition, 1990; and which is hereby incorporated by reference for any purpose. Where permitted, all patents, applications, published applications and other publications and sequences from GenBank and other data bases referred to throughout in the disclosure herein are incorporated by reference in their entirety.

Unless otherwise indicated, the following terms have the following meanings:

As used herein, the term "nucleoside" means a glycosylamine comprising a nucleobase and a sugar. Nucleosides includes, but are not limited to, naturally occurring nucleosides, abasic nucleosides, modified nucleosides, and nucleosides having mimetic bases and/or sugar groups.

As used herein, the term "nucleotide" refers to a glycosomine comprising a nucleobase and a sugur having a phosphate group covalently linked to the sugar. Nucleotides may be modified with any of a variety of substituents.

As used herein, the term "nucleobase" refers to the base portion of a nucleoside or nucleotide. A nucleobase may comprise any atom or group of atoms capable of hydrogen bonding to a base of another nucleic acid.

As used herein, the term "heterocyclic base moiety" refers to a nucleobase comprising a heterocycle.

As used herein, the term "deoxyribonucleotide" means a nucleotide having a hydrogen at the 2' position of the sugar portion of the nucleotide. Deoxyribonucleotides may be modified with any of a variety of substituents.

As used herein, the term "ribonucleotide" means a nucleotide having a hydroxy at the 2' position of the sugar portion of the nucleotide. Ribonucleotides may be modified with any of a variety of substituents.

As used herein, the term "oligomeric compound" refers to a polymeric structure comprising two or more sub-structures and capable of hybridizing to a region of a nucleic acid molecule. In certain embodiments, oligomeric compounds are oligonucleosides. In certain embodiments, oligomeric compounds are oligonucleotides. In certain embodiments, oligomeric compounds are antisense compounds. In certain embodiments, oligomeric compounds are antisense oligonucleotides. In certain embodiments, oligomeric compounds are short antisense compounds. In certain embodiments, oligomeric compounds are short antisense oligonucleotides. In certain embodiments, oligomeric compounds are chimeric oligonucleotides.

As used herein, the term "monomer" refers to a single unit of an oligomer. Monomers include, but are not limited to, nucleosides and nucleotides, whether naturally occuring or modified.

As used herein "oligonucleoside" refers to an oligonucleotide in which the internucleoside linkages do not contain a phosphorus atom.

As used herein, the term "oligonucleotide" refers to an oligomeric compound comprising a plurality of linked nucleotides. In certain embodiment, one or more nucleotides of an oligonucleotide is modified. In certain embodiments, an oligonucleotide comprises ribonucleic acid (RNA) or deoxyribonucleic acid (DNA). In certain embodiments, oligonucleotides are composed of naturally-and/or non-naturally-occurring nucleobases, sugars and covalent internucleotide linkages, and may further include non-nucleic acid conjugates.

As used herein "internucleotide linkage" refers to a covalent linkage between adjacent nucleotides.

As used herein, the term "monomeric linkage" refers to a covalent linkage between two monmers. Monomeric linkages include, but are not limited to internucleotide linkages and internucleoside linkages.

As used herein "naturally occuring internucleotide linkage" refers to a 3' to 5' phosphodiester linkage.

As used herein, the term "antisense compound" refers to an oligomeric compound that is at least partially complementary to a target nucleic acid molecule to which it hybridizes. In certain embodiments, an antisense compound modulates (increases or decreases) expression of a target nucleic acid. Antisense compounds include, but are not limited to, compounds that are oligonucleotides, oligonucleosides, oligonucleotide analogs, oligonucleotide mimetics, and chimeric combinations of these. Consequently, while all antisense compounds are oligomeric compounds, not all oligomeric compounds are antisense compounds.

As used herein, the term "antisense oligonucleotide" refers to an antisense compound that is an oligonucleotide.

As used herein, the term "parent antisense oligonucleotide" refers to an oligonucleotide 20 nucleotides in length having a deoxy gap region having ten 2'-deoxyribonucleotides, flanked by a first and a second wing region each having five 2'-O-(2-methoxyethyl) ribonucleotides (a 5-10-5 MOE gapmer) and comprising the sequence of the corresponding short antisense compound to which it is a parent.

As used herein, the term "short antisense compound" refers to an antisense compound about 8, 9, 10, 11, 12, 13, 14, 15 or 16 monomers in length. In certain embodiments, a short antisense compound has at least one high-affinity modification.

As used herein, the term "short antisense oligonucleotide" or refers to an antisense oligonucleotide about 8, 9, 10, 11, 12, 13, 14, 15 or 16 nucleotides in length. In certain embodiments, a short antisense oligonucleotide has at least one high-affinity modification.

As used herein, the term "short gapmer" refers to a short antisense oligonucleotide having a first and a second wing region each independently 1 to 3 nucleotides in length and a gap region 2 to 14 nucleobase in length.

As used herein, the term "motif" refers to the pattern of unmodified and modified nucleotides in a short antisense compound

As used herein, the term "chimeric antisense oligomer" refers to an antisense oligomeric compound, having at least one sugar, nucleobase or internucleoside linkage that is differentially modified as compared to at least on other sugar, nucleobase or internucleoside linkage within the same antisense oligomeric compound. The remainder of the sugars, nucleobases and internucleoside linkages can be independently modified or unmodified, the same or different.

As used herein, the term "chimeric antisense oligonucleotide" refers to an antisense oligonucleotide, having at least one sugar, nucleobase or internucleoside linkage that is differentially modified as compared to at least on other sugar, nucleobase or internucleoside linkage within the same antisense oligonucleotide. The remainder of the sugars, nucleobases and internucleoside linkages can be independently modified or unmodified, the same or different.

As used herein, the term "mixed-backbone antisense oligonucleotide" refers to an antisense oligonucleotide wherein at least one internucleoside linkage of the antisense oligonucleotide is different from at least one other internucleotide linkage of the antisense oligonucleotide.

As used herein, the term "target" refers to a protein, the modulation of which is desired.

As used herein, the term "target gene" refers to a gene encoding a target.

As used herein, the terms "target nucleic acid" and "nucleic acid molecule encoding a target" refer to any nucleic acid molecule the expression or activity of which is capable of being modulated by an antisense compound. Target nucleic acids include, but are not limited to, RNA (including, but not limited to pre-mRNA and mRNA or portions thereof) transcribed from DNA encoding a target, and also cDNA derived from such RNA, and miRNA. For example, the target nucleic acid can be a cellular gene (or mRNA transcribed from the gene) whose expression is associated with a particular disorder or disease state, or a nucleic acid molecule from an infectious agent.

As used herein, the term "targeting" or "targeted to" refers to the association of an antisense compound to a particular target nucleic acid molecule or a particular region of nucleotides within a target nucleic acid molecule.

As used herein, the term "5' target site" refers to the nucleotide of a target nucleic acid which is complementary to the 5'-most nucleotide of a particular antisense compound.

As used herein, the term "3' target site" refers to the nucleotide of a target nucleic acid which is complementary to the 3'-most nucleotide of a particular antisense compound.

As used herein, the term "target region," refers to a portion of a target nucleic acid to which one or more antisense compounds is complementary.

As used herein, the term "target segment" refers to a smaller or sub-portions of a region within a target nucleic acid.

As used herein, the term "nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

As used herein, the term "non-complementary nucleobase" refers to a pair of nucleobases that do not form hydrogen bonds with one another or otherwise support hybridization.

As used herein, the term "complementary" refers to the capacity of an oligomeric compound to hybridize to another oligomeric compound or nucleic acid through nucleobase complementarity. In certain embodiments, an antisense compound and its target are complementary to each other when a sufficient number of corresponding positions in each molecule are occupied by nucleobases that can bond with each other to allow stable association between the antisense compound and the target. One skilled in the art recognizes that the inclusion of mismatches is possible without eliminating the ability of the oligomeric compounds to remain in association. Therefore, described herein are antisense compounds that may comprise up to about 20% nucleotides that are mismatched (i.e., are not nucleobase complementary to the corresponding nucleotides of the target). Preferably the antisense compounds contain no more than about 15%, more preferably not more than about 10%, most preferably not more than 5% or no mismatches. The remaining nucleotides are nucleobase complementary or otherwise do not disrupt hybridization (e.g., universal bases). One of ordinary skill in the art would recognize the compounds provided herein are at least 80%, at least 85%, at least 90%, at least 95%, at least 96%, at least 97%, at least 98%, at least 99% or 100% complementary to a target nucleic acid.

As used herein, the term "mismatch" refers to a non-complementary nucleobase within a complementary oligomeric compound.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymidine and uracil which pair through the formation of hydrogen bonds. The natural base guanine is nucleobase complementary to the natural bases cytosine and 5-methyl cytosine. Hybridization can occur under varying circumstances.

As used herein, the term "specifically hybridizes" refers to the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "designing" or "designed to" refer to the process of designing an oligomeric compound that specifically hybridizes with a selected nucleic acid molecule.

As used herein, the term "modulation" refers to a perturbation of function or activity when compared to the level of the function or activity prior to modulation. For example, modulation includes the change, either an increase (stimulation or induction) or a decrease (inhibition or reduction) in gene expression. As further example, modulation of expression can include perturbing splice site selection of pre-mRNA processing.

As used herein, the term "expression" refers to all the functions and steps by which a gene's coded information is converted into structures present and operating in a cell. Such structures include, but are not limited to the products of transcription and translation.

As used herein, "variant" refers to an alternative RNA transcript that can be produced from the same genomic region of DNA. Variants include, but are not limited to "pre-mRNA variants" which are transcripts produced from the same genomic DNA that differ from other transcripts produced from the same genomic DNA in either their start or stop position and contain both intronic and exonic sequence. Variants also include, but are not limited to, those with alternate splice junctions, or alternate initiation and termination codons.

As used herein, "high-affinity modified monomer" refers to a monomer having at least one modified nucleobase, internucleoside linkage or sugar moiety, when compared to naturally occurring monomers, such that the modification increases the affinity of an antisense compound comprising the high-affinity modified monomer to its target nucleic acid. High-affinity modifications include, but are not limited to, monomers (e.g., nucleosides and nucleotides) comprising 2'-modifed sugars.

As used herein, the term "2'-modified" or "2'-substituted" means a sugar comprising substituent at the 2' position other than H or OH. 2'-modified monomers, include, but are not limited to, BNA's and monomers (e.g., nucleosides and nucleotides) with 2'- substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. In certain embodiments, short antisense compounds comprise a 2'modified monomer that does not have the formula 2'-O(CH₂)ₙH, wherein n is one to six. In certain embodiments, short antisense compounds comprise a 2'modified monomer that does not have the formula 2'-OCH₃. In certain embodiments, short antisense compounds comprise a 2'modified monomer that does not have the formula or, in the alternative, 2'-O(CH₂)₂OCH₃.

As used herein, the term "bicyclic nucleic acid" or "BNA" or "bicyclic nucleoside" or "bicyclic nucleotide" refers to a nucleoside or nucleotide wherein the furanose portion of the nucleoside includes a bridge connecting two carbon atoms on the furanose ring, thereby forming a bicyclic ring system.

As used herein, unless otherwise indicated, the term "methyleneoxy BNA" alone refers to β-D-methyleneoxy BNA.

As used herein, the term "MOE" refers to a 2'-methoxyethyl substituent.

As used herein, the term "gapmer" refers to a chimeric oligomeric compound comprising a central region (a "gap") and a region on either side of the central region (the "wings"), wherein the gap comprises at least one modification that is different from that of each wing. Such modifications include nucleobase, monomeric linkage, and sugar modifications as well as the absence of modification (unmodified). Thus, in certain embodiments, the nucleotide linkages in each of the wings are different than the nucleotide linkages in the gap. In certain embodiments, each wing comprises nucleotides with high affinity modifications and the gap comprises nucleotides that do not comprise that modification. In certain embodiments the nucleotides in the gap and the nucleotides in the wings all comprise high affinity modifications, but the high affinity modifications in the gap are different than the high affinity modifications in the wings. In certain embodiments, the modifications in the wings are the same as one another. In certain embodiments, the modifications in the wings are different from each other. In certain embodiments, nucleotides in the gap are unmodified and nucleotides in the wings are modified. In certain embodiments, the modification(s) in each wing are the same. In certain embodiments, the modification(s) in one wing are different from the modification(s) in the other wing. In certain embodiments, short antisense compounds are gapmers having 2'-deoxynucleotides in the gap and nucleotides with high-affinity modifications in the wing.

As used herein, the term "prodrug" refers to a therapeutic agent that is prepared in an inactive form that is converted to an active form (i.e., drug) within the body or cells thereof by the action of endogenous enzymes or other chemicals and/or conditions.

As used herein, the term "pharmaceutically acceptable salts" refers to salts of active compounds that retain the desired biological activity of the active compound and do not impart undesired toxicological effects thereto.

As used herein, the term "cap structure" or "terminal cap moiety" refers to chemical modifications, which have been incorporated at either terminus of an antisense compound.

As used herein, the term "prevention" refers to delaying or forestalling the onset or development of a condition or disease for a period of time from hours to days, preferably weeks to months.

As used herein, the term "amelioration" refers to a lessening of at least one indicator of the severity of a condition or disease. The severity of indicators may be determined by subjective or objective measures which are known to those skilled in the art.

As used herein, the term "treatment" refers to administering a composition of the invention to effect an alteration or improvement of the disease or condition. Prevention, amelioration, and/or treatment may require administration of multiple doses at regular intervals, or prior to onset of the disease or condition to alter the course of the disease or condition. Moreover, a single agent may be used in a single individual for each prevention, amelioration, and treatment of a condition or disease sequentially, or concurrently.

As used herein, the term "pharmaceutical agent" refers to a substance provides a therapeutic benefit when administered to a subject.

As used herein, the term "therapeutically effective amount" refers to an amount of a pharmaceutical agent that provides a therapeutic benefit to an animal.

As used herein, "administering" means providing a pharmaceutical agent to an animal, and includes, but is not limited to administering by a medical professional and self-administering.

As used herein, the term "co-administration" refers to administration of two or more pharmaceutical agents to an animal. The two or more pharmaceutical agents may be in a single pharmaceutical composition, or may be in separate pharmaceutical compositions. Each of the two or more pharmaceutical agents may be administered through the same or different routes of administration. Co-administration encompasses administration in parallel or sequentially.

As used herein, the term "pharmaceutical composition" refers to a mixture of substances suitable for administering to an individual. For example, a pharmaceutical composition may comprise an antisense oligonucleotide and a sterile aqueous solution.

As used herein, the term "individual" refers to a human or non-human animal selected for treatment or therapy.

As used herein, the term "animal" refers to a human or non-human animal, including, but not limited to, mice, rats, rabbits, dogs, cats, pigs, and non-human primates, including, but not limited to, monkeys and chimpanzees.

As used herein, the term "subject" refers to an animal, including, but not limited to a human, to whom a pharmaceutical composition is administered.

As used herein, the term "duration" refers to the period of time during which an activity or event continues. In certain embodiments, the duration of treatment is the period of time during which doses of a pharmaceutical agent are administered.

As used herein, the term "parenteral administration," refers to administration through injection or infusion. Parenteral administration includes, but is not limited to, subcutaneous administration, intravenous administration, or intramuscular administration.

As used herein, the term "subcutaneous administration" refers to administration just below the skin. "Intravenous administration" means administration into a vein.

As used herein, the term "dose" refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual.

As used herein, the term "dosage unit" refers to a form in which a pharmaceutical agent is provided. In certain embodiments, a dosage unit is a vial comprising lyophilized antisense oligonucleotide. In certain embodiments, a dosage unit is a vial comprising reconstituted antisense oligonucleotide.

As used herein, the term "pharmaceutical agent" refers to a substance provides a therapeutic benefit when administered to an individual. For example, in certain embodiments, an antisense oligonucleotide is a pharmaceutical agent.

As used herein, the term "active pharmaceutical ingredient" refers to the substance in a pharmaceutical composition that provides a desired effect.

As used herein, the term "therapeutically effective amount" refers to an amount of a pharmaceutical agent that provides a therapeutic benefit to an individual. In certain embodiments, a therapeutically effective amount of an antisense compound is the amount that needs to be administered to result in an observable benefit.

As used herein, the term "hypercholesterolemia" refers to a condition characterized by elevated serum cholesterol.

As used herein, the term "hyperlipidemia" refers to a condition characterized by elevated serum lipids.

As used herein, the term "hypertriglyceridemia" refers to a condition characterized by elevated triglyceride levels.

As used herein, the term "non-familial hypercholesterolemia" refers to a condition characterized by elevated cholesterol that is not the result of a single inherited gene mutation.

As used herein, the term "polygenic hypercholesterolemia" refers to a condition characterized by elevated cholesterol that results from the influence of a variety of genetic factors. In certain embodiments, polygenic hypercholesterolemia may be exacerbated by dietary intake of lipids.

As used herein, the term "familial hypercholesterolemia (FH)" refers to an autosomal dominant metabolic disorder characterized by a mutation in the LDL-receptor (LDL-R) gene, markedly elevated LDL-C and premature onset of atherosclerosis. A diagnosis of familial hypercholesterolemia is made when a individual meets one or more of the following criteria: genetic testing confirming 2 mutated LDL-receptor genes; genetic testing confirming one mutated LDL-receptor gene; document history of untreated serum LDL-cholesterol greater than 500 mg/dL; tendinous and/or cutaneous xanthoma prior to age 10 years; or, both parents have documented elevated serum LDL-cholesterol prior to lipid-lowering therapy consistent with heterozygous familial hypercholesterolemia.

As used herein, the term "homozygous familial hypercholesterolemia" or "HoFH" refers to a condition characterized by a mutation in both maternal and paternal LDL-R genes.

As used herein, the term "heterozygous familial hypercholesterolemia" or "HeFH" refers to a condition characterized by a mutation in either the maternal or paternal LDL-R gene.

As used herein, the term "mixed dyslipidemia" refers to a condition characterized by elevated serum cholesterol and elevated serum triglycerides.

As used herein, the term "diabetic dyslipidemia" or "Type II diabetes with dyslipidemia" refers to a condition characterized by Type II diabetes, reduced HDL-C, elevated serum triglycerides, and elevated small, dense LDL particles.

As used herein, the term "CHD risk equivalents," refers to indicators of clinical atherosclerotic disease that confer a high risk for coronary heart disease. For example, in certain embodiments, CHD risk equivalents include, without limitation, clinical coronary heart disease, symptomatic carotid artery disease, peripheral arterial disease, and/or abdominal aortic aneurysm.

As used herein, the term "non-alcoholic fatty liver disease (NAFLD)" refers to a condition characterized by fatty inflammation of the liver that is not due to excessive alcohol use (for example, alcohol consumption of over 20 g/day). In certain embodiments, NAFLD is related to insulin resistance and the metabolic syndrome.

As used herein, the term "non-alcoholic steatohepatitis (NASH)" refers to a condition characterized by inflammation and the accumulation of fat and fibrous tissue in the liver, that is not due to excessive alcohol use. NASH is an extreme form of NAFLD.

As used herein, the term "major risk factors" refers to factors that contribute to a high risk for a particular disease or condition. In certain embodiments, major risk factors for coronary heart disease include, without limitation, cigarette smoking, hypertension, low HDL-C, family history of coronary heart disease, and age.

As used herein, the term "CHD risk factors" refers to CHD risk equivalents and major risk factors.

As used herein, the term "coronary heart disease (CHD)" refers to a narrowing of the small blood vessels that supply blood and oxygen to the heart, which is often a result of atherosclerosis.

As used herein, the term "reduced coronary heart disease risk" refers to a reduction in the likelihood that a individual will develop coronary heart disease. In certain embodiments, a reduction in coronary heart disease risk is measured by an improvement in one or more CHD risk factors, for example, a decrease in LDL-C levels.

As used herein, the term "atherosclerosis" refers to a hardening of the arteries affecting large and medium-sized arteries and is characterized by the presence of fatty deposits. The fatty deposits are called "atheromas" or "plaques," which consist mainly of cholesterol and other fats, calcium and scar tissue, and damage the lining of arteries.

As used herein, the term "history of coronary heart disease" refers to the occurrence of clinically evident coronary heart disease in the medical history of a individual or a individual's family member.

As used herein, the term "Early onset coronary heart disease" refers to a diagnosis of coronary heart disease prior to age 50.

As used herein, the term "statin intolerant individual" refers to a individual who as a result of statin therapy experiences one or more of creatine kinase increases, liver function test abnormalities, muscle aches, or central nervous system side effects.

As used herein, the term "efficacy" refers to the ability to produce a desired effect. For example, efficacy of a lipid-lowering therapy may be reduction in the concentration of one or more of LDL-C, VLDL-C, IDL-C, non-HDL-C, ApoB, lipoprotein(a), or triglycerides.

As used herein, the term "acceptable safety profile" refers to a pattern of side effects that is within clinically acceptable limits.

As used herein, the term "side effects" refers to physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies. For example, increased aminotransferase levels in serum may indicate liver toxicity or liver function abnormality. For example, increased bilirubin may indicate liver toxicity or liver function abnormality.

As used herein, the term "injection site reaction" refers to inflammation or abnormal redness of skin at a site of injection in an individual.

As used herein, the term "individual compliance" refers to adherence to a recommended or prescribed therapy by an individual.

As used herein, the term "lipid-lowering therapy" refers to a therapeutic regimen provided to a individual to reduce one or more lipids in a individual. In certain embodiments, a lipid-lowering therapy is provide to reduce one or more of ApoB, total cholesterol, LDL-C, VLDL-C, IDL-C, non-HDL-C, triglycerides, small dense LDL particles, and Lp(a) in an individual.

As used herein, the term "lipid-lowering agent" refers to a pharmaceutical agent provided to a individual to achieve a lowering of lipids in the individual. For example, in certain embodiments, a lipid-lowering agent is provided to an individual to reduce one or more of ApoB, LDL-C, total cholesterol, and triglycerides.

As used herein, the term "LDL-C target" refers to an LDL-C level that is desired following lipid-lowering therapy.

As used herein, the term "comply" refers to the adherence with a recommended therapy by an individual.

As used herein, the term "recommended therapy" refers to a therapeutic regimen recommended by a medical professional for the treatment, amelioration, or prevention of a disease.

As used herein, the term "low LDL-receptor activity" refers to LDL-receptor activity that is not sufficiently high to maintain clinically acceptable levels of LDL-C in the bloodstream.

As used herein, the term "cardiovascular outcome" refers to the occurrence of major adverse cardiovascular events.

As used herein, the term "improved cardiovascular outcome" refers to a reduction in the occurrence of major adverse cardiovascular events, or the risk thereof. Examples of major adverse cardiovascular events include, without limitation, death, reinfarction, stroke, cardiogenic shock, pulmonary edema, cardiac arrest, and atrial dysrhythmia.

As used herein, the term "surrogate markers of cardiovascular outcome" refers to indirect indicators of cardiovascular events, or the risk thereof. For example, surrogate markers of cardiovascular outcome include carotid intimal media thickness (CIMT). Another example of a surrogate marker of cardiovascular outcome includes atheroma size. Atheroma size may be determined by intravascular ultrasound (IVUS).

As used herein, the term "increased HDL-C" refers to an increase in serum HDL-C in an individual over time.

As used herein, the term "lipid-lowering" refers to a reduction in one or more serum lipids in an individual over time.

As used herein, the term "metabolic disorder" refers to a condition characterized by an alteration or disturbance in metabolic function. "Metabolic" and "metabolism" are terms well know in the art and generally include the whole range of biochemical processes that occur within a living organism. Metabolic disorders include, but are not limited to, hyperglycemia, prediabetes, diabetes (type I and type II), obesity, insulin resistance and metabolic syndrome.

As used herein, the term "metabolic syndrome" refers to a clustering of lipid and non-lipid cardiovascular risk factors of metabolic origin. It has been closely linked to the generalized metabolic disorder known as insulin resistance. The National Cholesterol Education Program (NCEP) Adult Treatment Panel III (ATPIII) established criteria for diagnosis of metabolic syndrome when three or more of five risk determinants are present. The five risk determinants are abdominal obesity defined as waist circumference of greater than 102 cm for men or greater than 88cm for women, triglyceride levels greater than or equal to 150 mg/dL, HDL cholesterol levels of less than 40 mg/dL for men and less than 50 mg/dL for women, blood pressure greater than or equal to 130/85 mm Hg and fasting glucose levels greater than or equal to 110 mg/dL. These determinants can be readily measured in clinical practice (JAMA, 2001, 285: 2486-2497).

The term "alkyl," as used herein, refers to a saturated straight or branched hydrocarbon radical containing up to twenty four carbon atoms. Examples of alkyl groups include, but are not limited to, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred. The term "lower alkyl" as used herein includes from 1 to about 6 carbon atoms. Alkyl groups as used herein may optionally include one or more further substituent groups.

The term "alkenyl," as used herein, refers to a straight or branched hydrocarbon chain radical containing up to twenty four carbon atoms and having at least one carbon-carbon double bond. Examples of alkenyl groups include, but are not limited to, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

The term "alkynyl," as used herein, refers to a straight or branched hydrocarbon radical containing up to twenty four carbon atoms and having at least one carbon-carbon triple bond. Examples of alkynyl groups include, but are not limited to, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substitutent groups.

The term "aminoalkyl" as used herein, refers to an amino substituted alkyl radical. This term is meant to include C₁-C₁₂ alkyl groups having an amino substituent at any position and wherein the alkyl group attaches the aminoalkyl group to the parent molecule. The alkyl and/or amino portions of the aminoalkyl group can be further substituted with substituent groups.

The term "aliphatic," as used herein, refers to a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation polyalkoxys, such as polyalkylene glycols, polyamines, and polyimines. Aliphatic groups as used herein may optionally include further substitutent groups.

The term "alicyclic" or "alicyclyl" refers to a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substitutent groups.

The term "alkoxy," as used herein, refers to a radical formed between an alkyl group and an oxygen atom wherein the oxygen atom is used to attach the alkoxy group to a parent molecule. Examples of alkoxy groups include, but are not limited to, methoxy, ethoxy, propoxy, isopropoxy, n-butoxy, sec-butoxy, tert-butoxy, n-pentoxy, neopentoxy, n-hexoxy and the like. Alkoxy groups as used herein may optionally include further substitutent groups.

The terms "halo" and "halogen," as used herein, refer to an atom selected from fluorine, chlorine, bromine and iodine.

The terms "aryl" and "aromatic," as used herein, refer to a mono- or polycyclic carbocyclic ring system radicals having one or more aromatic rings. Examples of aryl groups include, but are not limited to, phenyl, naphthyl, tetrahydronaphthyl, indanyl, idenyl and the like. Preferred aryl ring systems have from about 5 to about 20 carbon atoms in one or more rings. Aryl groups as used herein may optionally include further substitutent groups.

The terms "aralkyl" and "arylalkyl," as used herein, refer to a radical formed between an alkyl group and an aryl group wherein the alkyl group is used to attach the aralkyl group to a parent molecule. Examples include, but are not limited to, benzyl, phenethyl and the like. Aralkyl groups as used herein may optionally include further substitutent groups attached to the alkyl, the aryl or both groups that form the radical group.

The term "heterocyclic radical" as used herein, refers to a radical mono-, or poly-cyclic ring system that includes at least one heteroatom and is unsaturated, partially saturated or fully saturated, thereby including heteroaryl groups. Heterocyclic is also meant to include fused ring systems wherein one or more of the fused rings contain at least one heteroatom and the other rings can contain one or more heteroatoms or optionally contain no heteroatoms. A heterocyclic group typically includes at least one atom selected from sulfur, nitrogen or oxygen. Examples of heterocyclic groups include, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, quinoxalinyl, pyridazinonyl, tetrahydrofuryl and the like. Heterocyclic groups as used herein may optionally include further substitutent groups.

The terms "heteroaryl," and "heteroaromatic," as used herein, refer to a radical comprising a mono- or poly-cyclic aromatic ring, ring system or fused ring system wherein at least one of the rings is aromatic and includes one or more heteroatom. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include, but are not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like. Heteroaryl radicals can be attached to a parent molecule directly or through a linking moiety such as an aliphatic group or hetero atom. Heteroaryl groups as used herein may optionally include further substitutent groups.

The term "heteroarylalkyl," as used herein, refers to a heteroaryl group as previously defined having an alky radical that can attach the heteroarylalkyl group to a parent molecule. Examples include, but are not limited to, pyridinylmethyl, pyrimidinylethyl, napthyridinylpropyl and the like. Heteroarylalkyl groups as used herein may optionally include further substitutent groups on one or both of the heteroaryl or alkyl portions.

The term "mono or poly cyclic structure" as used in the present invention includes all ring systems that are single or polycyclic having rings that are fused or linked and is meant to be inclusive of single and mixed ring systems individually selected from aliphatic, alicyclic, aryl, heteroaryl, aralkyl, arylalkyl, heterocyclic, heteroaryl, heteroaromatic, heteroarylalkyl. Such mono and poly cyclic structures can contain rings that are uniform or have varying degrees of saturation including fully saturated, partially saturated or fully unsaturated. Each ring can comprise ring atoms selected from C, N, O and S to give rise to heterocyclic rings as well as rings comprising only C ring atoms which can be present in a mixed motif such as for example benzimidazole wherein one ring has only carbon ring atoms and the fused ring has two nitrogen atoms. The mono or poly cyclic structures can be further substituted with substituent groups such as for example phthalimide which has two =O groups attached to one of the rings. In another aspect, mono or poly cyclic structures can be attached to a parent molecule directly through a ring atom, through a substituent group or a bifunctional linking moiety.

The term "acyl," as used herein, refers to a radical formed by removal of a hydroxyl group from an organic acid an d has the general formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substitutent groups.

The term "hydrocarbyl includes groups comprising C, O and H. Included are straight, branched and cyclic groups having any degree of saturation. Such hydrocarbyl groups can include one or more heteroatoms selected from N, O and S and can be further mono or poly substituted with one or more substituent groups.

The terms "substituent" and "substituent group," as used herein, include groups that are typically added to other groups or parent compounds to enhance desired properties or give desired effects. Substituent groups can be protected or unprotected and can be added to one available site or to many available sites in a parent compound. Substituent groups may also be further substituted with other substituent groups and may be attached directly or via a linking group such as an alkyl or hydrocarbyl group to a parent compound. Such groups include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxo (-O-Rₐₐ), aryl, aralkyl, heterocyclic, heteroaryl, heteroarylalkyl, amino (-NR_{bb}R_{cc}), imino(=NR_{bb}), amido (-C(O)NR_{bb}R_{cc}or -N(R_{bb})C(O)Rₐₐ), azido (-N₃), nitro (-NO₂) cyano (-CN), carbamido (-OC(O)NR_{bb}R_{cc} or -N(R_{bb})C(O)ORₐₐ), ureido (-N(R_{bb})C(O)NR_{bb}R_{cc}), thioureido (-N(R_{bb})C(S)NR_{bb}R_{cc}), guanidinyl (-N(R_{bb})C(=NR_{b}b)NR_{bb}R_{cc}), amidinyl (-C(=NR_{bb})NR_{bb}R_{cc} or -N(R_{bb})C(NR_{bb})Rₐₐ), thiol (-SR_{bb}), sulfinyl (-S(O)R_{bb}), sulfonyl (-S(O)₂R_{bb} sulfonamidyl (-S(O)₂NR_{bb}R_{cc} or -N(R_{bb})S(O)₂R_{bb}) and conjugate groups. Wherein each Rₐₐ, R_{bb} and R_{cc} is, independently, H, an optionally linked chemical functional group or a further substituent group with a preferred list including without limitation H, alkyl, alkenyl, alkynyl, aliphatic, alkoxy, acyl, aryl, aralkyl, heteroaryl, alicyclic, heterocyclic and heteroarylalkyl.

### B. Certain Oligomeric Compounds

In certain embodiments, it is desirable to chemically modify oligomeric compounds, compared to naturally occuring oligomers, such as DNA or RNA. Certain such modifications alter the activity of the oligomeric compound. Certain such chemical modifications can alter activity by, for example: increasing affinity of an antisense compound for its target nucleic acid, increasing its resistance to one or more nucleases, and/or altering the pharmacokinetics or tissue distribution of the oligomeric compound. In certain instances, the use of chemistries that increase the affinity of an oligomeric compound for its target can allow for the use of shorter oligomeric compounds.

### 1. Certain monomers

In certain embodiment, oligomeric compounds comprise one or more modified monomer. In certain such embodiments, oligomeric compounds comprise one or more high affinity monomer. In certain embodiments, such high-affinity monomer is selected from monomers (e.g., nucleosides and nucleotides) comprising 2'-modifed sugars, including, but not limited to: BNA's and monomers (e.g., nucleosides and nucleotides) with 2'- substituents such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl.

In certain embodiments, the oligomeric compounds including, but no limited to short antisense compounds of the present invention, comprise one or more high affinity monomers provided that the oligomeric compound does not comprise a nucleotide comprising a 2'-O(CH₂)ₙH, wherein n is one to six.

In certain embodiments, the oligomeric compounds including, but no limited to short antisense compounds of the present invention, comprise one or more high affinity monomer provided that the oligomeric compound does not comprise a nucleotide comprising a 2'-OCH₃ or a 2'-O(CH₂)₂OCH₃.

In certain embodiments, the oligomeric compounds including, but no limited to short antisense compounds of the present invention, comprise one or more high affinity monomer provided that the oligomeric compound does not comprise a α-L-Methyleneoxy (4'-CH₂-O-2') BNA.

In certain embodiments, the oligomeric compounds including, but no limited to short antisense compounds of the present invention, comprise one or more high affinity monomer provided that the oligomeric compound does not comprise a β-D-Methyleneoxy (4'-CH₂-O-2') BNA.

In certain embodiments, the oligomeric compounds including, but no limited to short antisense compounds of the present invention, comprise one or more high affinity monomer provided that the oligomeric compound does not comprise a α-L-Methyleneoxy (4'-CH₂-O-2') BNA or a β-D-Methyleneoxy (4'-CH₂-O-2') BNA.

### a. Certain Nucleobases

The naturally occurring base portion of a nucleoside is typically a heterocyclic base. The two most common classes of such heterocyclic bases are the purines and the pyrimidines. For those nucleosides that include a pentofuranosyl sugar, a phosphate group can be linked to the 2', 3' or 5' hydroxyl moiety of the sugar. In forming oligonucleotides, those phosphate groups covalently link adjacent nucleosides to one another to form a linear polymeric compound. Within oligonucleotides, the phosphate groups are commonly referred to as forming the internucleotide backbone of the oligonucleotide. The naturally occurring linkage or backbone of RNA and of DNA is a 3' to 5' phosphodiester linkage.

In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable with the compounds described herein. In certain embodiments, a modified nucleobase is a nucleobase that is fairly similar in structure to the parent nucleobase, such as for example a 7-deaza purine, a 5-methyl cytosine, or a G-clamp. In certain embodiments, nucleobase mimetic include more complicated structures, such as for example a tricyclic phenoxazine nucleobase mimetic. Methods for preparation of the above noted modified nucleobases are well known to those skilled in the art.

### b. Certain sugars

Oligomeric compounds provided herein may comprise one or more monomer, including a nucleoside or nucleotide, having a modified sugar moiety. For example, the furanosyl sugar ring of a nucleoside can be modified in a number of ways including, but not limited to, addition of a substituent group, bridging of two non-geminal ring atoms to form a bicyclic nucleic acid (BNA).

In certain embodiments, oligomeric compounds comprise one or more monomers that is a BNA. In certain such embodiments, BNA s include, but are not limited to, (A) α-L-Methyleneoxy (4'-CH₂-O-2') BNA , (B) β-D-Methyleneoxy (4'-CH₂-O-2') BNA , (C) Ethyleneoxy (4'-(CH₂)₂-O-2') BNA , (D) Aminooxy (4'-CH₂-O-N(R)-2') BNA and (E) Oxyamino (4'-CH₂-N(R)-O-2') BNA, as depicted in Figure 1.

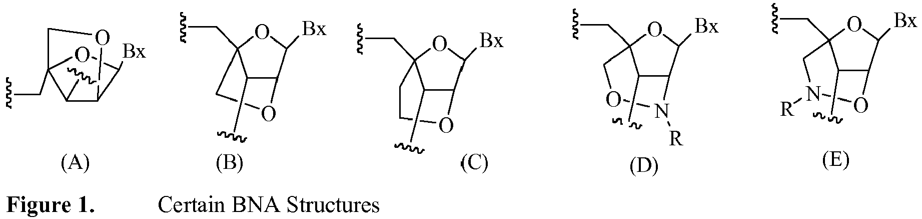

In certain embodimnents, BNA compounds include, but are not limited to, compounds having at least one bridge between the 4' and the 2' position of the sugar wherein each of the bridges independently comprises 1 or from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
wherein:
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one embodiment, each of the bridges of the BNA compounds is, independently, -[C(R₁)(R₂)]ₙ-, -[C(R₁)(R₂)]ₙ-O-, -C(R₁R₂)-N(R₁)-O- or -C(R₁R₂)-O-N(R₁)-. In another embodiment, each of said bridges is, independently, 4'-CH₂-2', 4'-(CH₂)₂-2', 4'-(CH₂)₃-2', 4'-CH₂-O-2', 4'-(CH₂)₂-O-2', 4'-CH₂-ON(R₁)-2' and 4'-CH₂-N(R₁)-O-2'- wherein each R₁ is, independently, H, a protecting group or C₁-C₁₂ alkyl.

Certain BNA's have been prepared and disclosed in the patent literature as well as in scientific literature (Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; WO 94/14226; WO 2005/021570; Singh et al., J. Org. Chem., 1998, 63, 10035-10039; Examples of issued US patents and published applications that disclose BNA s include, for example, U.S. Patent Nos. 7,053,207; 6,268,490; 6,770,748; 6,794,499; 7,034,133; and 6,525,191; and U.S. Pre-Grant Publication Nos. 2004-0171570; 2004-0219565; 2004-0014959; 2003-0207841; 2004-0143114; and 20030082807.

Also provided herein are BNAs in which the 2'-hydroxyl group of the ribosyl sugar ring is linked to the 4' carbon atom of the sugar ring thereby forming a methyleneoxy (4'-CH₂-O-2') linkage to form the bicyclic sugar moiety (reviewed in Elayadi et al., Curr. Opinion Invens. Drugs, 2001, 2, 558-561; Braasch et al., Chem. Biol., 2001, 8 1-7; and Orum et al., Curr. Opinion Mol. Ther., 2001, 3, 239-243; see also U.S. Patents: 6,268,490 and 6,670,461). The linkage can be a methylene (-CH₂-) group bridging the 2' oxygen atom and the 4' carbon atom, for which the term methyleneoxy (4'-CH₂-O-2') BNA is used for the bicyclic moiety; in the case of an ethylene group in this position, the term ethyleneoxy (4'-CH₂CH₂-O-2') BNA is used (Singh et al., Chem. Commun., 1998, 4, 455-456: Morita et al., Bioorganic Medicinal Chemistry, 2003, 11, 2211-2226). Methyleneoxy (4'-CH₂-O-2') BNA and other bicyclic sugar analogs display very high duplex thermal stabilities with complementary DNA and RNA (Tm = +3 to +10° C), stability towards 3'-exonucleolytic degradation and good solubility properties. Potent and nontoxic antisense oligonucleotides compriseing BNAs have been described (Wahlestedt et al., Proc. Natl. Acad. Sci. U. S. A., 2000, 97, 5633-5638).

An isomer of methyleneoxy (4'-CH₂-O-2') BNA that has also been discussed is alpha-L-methyleneoxy (4'-CH₂-O-2') BNA which has been shown to have superior stability against a 3'-exonuclease. The alpha-L- methyleneoxy (4'-CH₂-O-2') BNA's were incorporated into antisense gapmers and chimeras that showed potent antisense activity (Frieden et al., Nucleic Acids Research, 2003, 21, 6365-6372).

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA, phosphorothioate- methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs compriseing oligodeoxyribonucleotide duplexes as substrates for nucleic acid polymerases has also been described (Wengel et al., WO 99/14226 ). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-Amino- and 2'-methylamino-BNA's have been prepared and the thermal stability of their duplexes with complementary RNA and DNA strands has been previously reported.

Modified sugar moieties are well known and can be used to alter, typically increase, the affinity of the antisense compound for its target and/or increase nuclease resistance. A representative list of preferred modified sugars includes but is not limited to bicyclic modified sugars (BNA's), including methyleneoxy (4'-CH₂-O-2') BNA and ethyleneoxy (4'-(CH₂)₂-O-2' bridge) BNA ; substituted sugars, especially 2'-substituted sugars having a 2'-F, 2'-OCH₃ or a 2'-O(CH₂)₂-OCH₃ substituent group; and 4'-thio modified sugars. Sugars can also be replaced with sugar mimetic groups among others. Methods for the preparations of modified sugars are well known to those skilled in the art. Some representative patents and publications that teach the preparation of such modified sugars include, but are not limited to, U.S. Patents: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; 5,700,920; 6,531,584; and 6,600,032; and WO 2005/121371.

In certain embodiments, BNA's include bicyclic nucleoside having the formula: 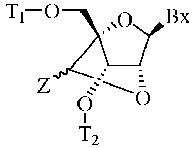 wherein:
Bx is a heterocyclic base moiety;
T 1 is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂ NJ₃C(=X)NJ₁J₂ and CN, wherein each J₁, J₂ and J₃ is, independently, H or C₁-C₆ alkyl, and X is O, S or NJ₁.

In certain such embodiments, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H, C₁-C₆ alkyl, or substituted C₁-C₆ alkyl and X is O or NJ₁.

In certain embodiments, the Z group is C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J2 or CN; wherein each J₁, J₂ and J₃ is, independently, H or C₁-C₆ alkyl, and X is O, S or NJ₁. In another embodiment, the Z group is C₁-C₆ alkyl substituted with one or more X^{x}, wherein each X^{x} is independently halo (e.g., fluoro), hydroxyl, alkoxy (e.g., CH₃O-), substituted alkoxy or azido.

In certain embodiments, the Z group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, the Z group is -CH₂X^{x}, wherein X^{x} is halo (e.g., fluoro), hydroxyl, alkoxy (e.g., CH₃O-) or azido.

In certain such embodiments, the Z group is in the (R)-configuration:

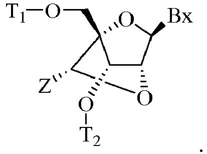

In certain such embodiments, the Z group is in the (S)-configuration:

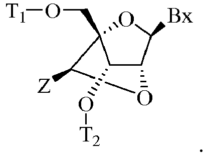

In certain embodiments, each T₁ and T₂ is a hydroxyl protecting group. A preferred list of hydroxyl protecting groups includes benzyl, benzoyl, 2,6-dichlorobenzyl, t-butyldimethylsilyl, t-butyldiphenylsilyl, mesylate, tosylate, dimethoxytrityl (DMT), 9-phenylxanthine-9-yl (Pixyl) and 9-(p-methoxyphenyl)xanthine-9-yl (MOX). In certain embodiments, T₁ is a hydroxyl protecting group selected from acetyl, benzyl, t-butyldimethylsilyl, t-butyldiphenylsilyl and dimethoxytrityl wherein a more preferred hydroxyl protecting group is T₁ is 4,4'-dimethoxytrityl.

In certain embodiments, T₂ is a reactive phosphorus group wherein preferred reactive phosphorus groups include diisopropylcyanoethoxy phosphoramidite and H-phosphonate. In certain embodiments T₁ is 4,4'-dimethoxytrityl and T₂ is diisopropylcyanoethoxy phosphoramidite.

In certain embodiments, oligomeric compounds have at least one monomer of the formula: 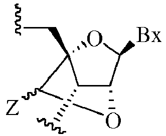 or of the formula: 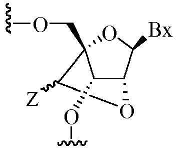 or of the formula: 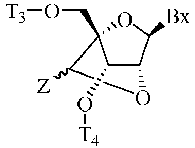 wherein
Bx is a heterocyclic base moiety;
T₃ is H, a hydroxyl protecting group, a linked conjugate group or an internucleoside linking group attached to a nucleoside, a nucleotide, an oligonucleoside, an oligonucleotide, a monomeric subunit or an oligomeric compound;
T₄ is H, a hydroxyl protecting group, a linked conjugate group or an internucleoside linking group attached to a nucleoside, a nucleotide, an oligonucleoside, an oligonucleotide, a monomeric subunit or an oligomeric compound;
wherein at least one of T₃ and T₄ is an internucleoside linking group attached to a nucleoside, a nucleotide, an oligonucleoside, an oligonucleotide, a monomeric subunit or an oligomeric compound; and
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In one embodiment, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H or C₁-C₆ alkyl, and X is O or NJ₁.

In certain such embodiments, at least one Z is C₁-C₆ alkyl or substituted C₁-C₆ alkyl. In certain embodiments, each Z is, independently, C₁-C₆ alkyl or substituted C₁-C₆alkyl. In certain embodiments, at least one Z is C₁-C₆ alkyl. In certain embodiments, each Z is, independently, C₁-C₆ alkyl. In certain embodiments, at least one Z is methyl. In certain embodiments, each Z is methyl. In certain embodiments, at least one Z is ethyl. In certain embodiments, each Z is ethyl. In certain embodiments, at least one Z is substituted C₁-C₆ alkyl. In certain embodiments, each Z is, independently, substituted C₁-C₆ alkyl. In certain embodiments, at least one Z is substituted methyl. In certain embodiments, each Z is substituted methyl. In certain embodiments, at least one Z is substituted ethyl. In certain embodiments, each Z is substituted ethyl.

In certain embodiments, at least one substituent group is C₁-C₆ alkoxy (e.g., at least one Z is C₁-C₆ alkyl substituted with one or more C₁-C₆ alkoxy). In another embodiment, each substituent group is, independently, C₁-C₆alkoxy (e.g., each Z is, independently, C₁-C₆alkyl substituted with one or more C₁-C₆ alkoxy).

In certain embodiments, at least one C₁-C₆ alkoxy substituent group is CH₃O- (e.g., at least one Z is CH₃OCH₂-). In another embodiment, each C₁-C₆ alkoxy substituent group is CH₃O- (e.g., each Z is CH₃OCH₂-).

In certain embodiments, at least one substituent group is halogen (e.g., at least one Z is C₁-C₆ alkyl substituted with one or more halogen). In certain embodiments, each substituent group is, independently, halogen (e.g., each Z is, independently, C₁-C₆ alkyl substituted with one or more halogen). In certain embodiments, at least one halogen substituent group is fluoro (e.g., at least one Z is CH₂FCH₂-, CHF₂CH₂- or CF₃CH₂-)- In certain embodiments, each halo substituent group is fluoro (e.g., each Z is, independently, CH₂FCH₂-, CHF₂CH₂- or CF₃CH₂-).

In certain embodiments, at least one substituent group is hydroxyl (e.g., at least one Z is C₁-C₆ alkyl substituted with one or more hydroxyl). In certain embodiments, each substituent group is, independently, hydroxyl (e.g., each Z is, independently, C₁-C₆ alkyl substituted with one or more hydroxyl). In certain embodiments, at least one Z is HOCH₂-. In another embodiment, each Z is HOCH₂-.

In certain embodiments, at least one Z is CH₃-, CH₃CH₂-, CH₂OCH₃-, CH₂F- or HOCH₂-. In certain embodiments, each Z is, independently, CH₃-, CH₃CH₂-, CH₂OCH₃-, CH₂F- or HOCH₂-.

In certain embodiments, at least one Z group is C₁-C₆ alkyl substituted with one or more X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, at least one Z group is C₁-C₆ alkyl substituted with one or more X^{x}, wherein each X^{x} is, independently, halo (e.g., fluoro), hydroxyl, alkoxy (e.g., CH₃O-) or azido.

In certain embodiments, each Z group is, independently, C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, C₁-C₆ alkyl substituted with one or more X^{x}, wherein each X^{x} is independently halo (e.g., fluoro), hydroxyl, alkoxy (e.g., CH₃O-) or azido.

In certain embodiments, at least one Z group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁ In certain embodiments, at least one Z group is -CH₂X^{x}, wherein X^{x} is halo (e.g., fluoro), hydroxyl, alkoxy (e.g., CH₃O-) or azido.

In certain embodiments, each Z group is, independently, -CH₂X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, -CH₂X^{x}, wherein each X^{x} is, independently, halo (e.g., fluoro), hydroxyl, alkoxy (e.g., CH₃O-) or azido.

In certain embodiments, at least one Z is CH₃-. In another embodiment, each Z is, CH₃-.

In certain embodiments, the Z group of at least one monomer is in the (R)- configuration represented by the formula: 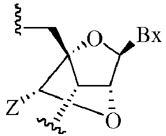 or the formula: 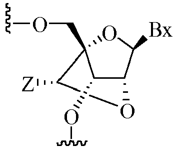 or the formula:

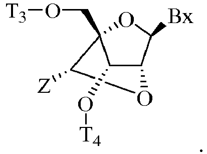

In certain embodiments, the Z group of each monomer of the formula is in the (R)- configuration.

In certain embodiments, the Z group of at least one monomer is in the (S)- configuration represented by the formula: 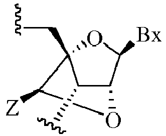 or the formula: 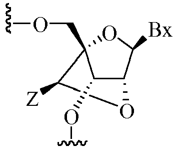 or the formula:

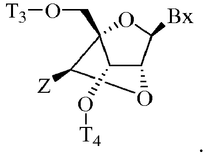

In certain embodiments, the Z group of each monomer of the formula is in the (S)- configuration.

In certain embodiments, T₃ is H or a hydroxyl protecting group. In certain embodiments, T₄ is H or a hydroxyl protecting group. In a further embodiment T₃ is an internucleoside linking group attached to a nucleoside, a nucleotide or a monomeric subunit. In certain embodiments, T₄ is an internucleoside linking group attached to a nucleoside, a nucleotide or a monomeric subunit. In certain embodiments,T₃ is an internucleoside linking group attached to an oligonucleoside or an oligonucleotide. In certain embodiments, T₄ is an internucleoside linking group attached to an oligonucleoside or an oligonucleotide. In certain embodiments, T₃ is an internucleoside linking group attached to an oligomeric compound. In certain embodiments, T₄ is an internucleoside linking group attached to an oligomeric compound. In In certain embodiments, at least one of T₃ and T₄ comprises an internucleoside linking group selected from phosphodiester or phosphorothioate.

In certain embodiments, oligomeric compounds have at least one region of at least two contiguous monomers of the formula: 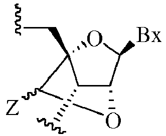 or of the formula: 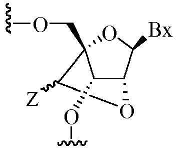 or of the formula:
to

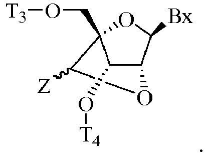

In certain embodiments, the oligomeric compound comprises at least two regions of at least two contiguous monomers of the above formula. In certain embodiments, the oligomeric compound comprises a gapped oligomeric compound. In certain embodiments, the oligmeric compound comprises at least one region of from about 8 to about 14 contiguous β-D-2'-deoxyribofuranosyl nucleosides. In certain embodiments, the oligomeric compound comprises at least one region of from about 9 to about 12 contiguous β-D-2'-deoxyribofuranosyl nucleosides.

In certain embodiments, monmers include sugar mimetics. In certain such embodiments, a mimetic is used in place of the sugar or sugar-internucleoside linkage combination, and the nucleobase is maintained for hybridization to a selected target. Representative examples of a sugar mimetics include, but are not limited to, cyclohexenyl or morpholino. Representative examples of a mimetic for a sugar-internucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14, incorporated herein by reference). Methods of synthesis of sugar, nucleoside and nucleobase mimetics are well known to those skilled in the art.

### 3. Monomeric Linkages

Described herein are linking groups that link monomers (including, but not limited to, modified and unmodified nucleosides and nucleotides) together, thereby forming an oligomeric compound. The two main classes of linking groups are defined by the presence or absence of a phosphorus atom. Representative phosphorus containing linkages include, but are not limited to, phosphodiesters (P=O), phosphotriesters, methylphosphonates, phosphoramidate, and phosphorothioates (P=S). Representative non-phosphorus containing linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)2-O-); and N,N'-dimethylhydrazine (-CH₂-N(CH₃)-N(CH₃)-)₋ Oligomeric compounds having non-phosphorus linking groups are referred to as oligonucleosides. Modified linkages, compared to natural phosphodiester linkages, can be used to alter, typically increase, nuclease resistance of the oligomeric compound. In certain embodiments, linkages having a chiral atom can be prepared a racemic mixtures, as separate enantomers. Representative chiral linkages include, but are not limited to, alkylphosphonates and phosphorothioates. Methods of preparation of phosphorous-containing and non-phosphorous-containing linkages are well known to those skilled in the art.

The oligomeric compounds described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids et al. Included in the antisense compounds provided herein are all such possible isomers, as well as their racemic and optically pure forms.

### 4. Oligomeric Compounds

In certain embodiments, provided herein are oligomeric compounds having reactive phosphorus groups useful for forming linkages including for example phosphodiester and phosphorothioate internucleoside linkages. Methods of preparation and/or purification of precursors or oligomeric compounds are not a limitation of the compositions or methods provided herein. Methods for synthesis and purification of oligomeric compounds including DNA, RNA, oligonucleotides, oligonucleosides, and antisense compounds are well known to those skilled in the art.

Generally, oligomeric compounds comprise a plurality of monomeric subunits linked together by linking groups. Nonlimiting examples of oligomeric compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound.

In certain embodiments, the present invention provides chimeric oligomeric compounds. In certain such embodiments, chimeric oligomeric compounds are chimeric oligonucleotides. In certain such embodiments, the chimeric oligonucleotides comprise differently modified nucleotides. In certain embodiments, chimeric oligonucleotides are mixed-backbone antisense oligonucleotides.

In general a chimeric oligomeric compound will have modified nucleosides that can be in isolated positions or grouped together in regions that will define a particular motif. Any combination of modifications and/or mimetic groups can comprise a chimeric oligomeric compound as described herein.

In certain embodiments, chimeric oligomeric compounds typically comprise at least one region modified so as to confer increased resistance to nuclease degradation, increased cellular uptake, and/or increased binding affinity for the target nucleic acid. In certain embodiments, an additional region of the oligomeric compound may serve as a substrate for enzymes capable of cleaving RNA:DNA or RNA:RNA hybrids. By way of example, RNase H is a cellular endonuclease that cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter oligomeric compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

In certain embodiments, chimeric oligomeric compounds are gapmers. In certain embodiments, chimeric compounds are short antisense compounds. In certain embodiments, short antisense compounds are gapmers. In certain such embodiments, a mixed-backbone antisense oligomer has one type of internucleotide linkages in one or both wings and a different type of internucleotide linkages in the gap. In certain such embodiments, the mixed-backbone antisense oligonucleotide has phosphodiester linkages in the wings and phosphorothioate linkages in the gap. In certain embodiments in which the internucleotide linkages in a wing is different from the internucleotide linkages in the gap, the internucleotide linkage bridging that wing and the gap is the same as the internucleotide linkage in the wing. In certain embodiments in which the internucleotide linkages in a wing is different from the internucleotide linkages in the gap, the internucleotide linkage bridging that wing and the gap is the same as the internucleotide linkage in the gap.

### C. Certain Short Antisense Compounds

Disclosed herein are short antisense compounds 8 to 16, preferably 9 to 15, more preferably 9 to 14, more preferably 10 to 14 nucleotides in length. In certain embodiments, short antisense compounds are 9 to 14 nucleotides in length. In certain embodiments, short antisense compounds are 10 to 14 nucleotides in length. In certain embodiments, such short antisense compounds are short antisense oligonucleotides.

In certain embodiments, short antisense compounds comprise one or more chemical modifications. In certain such embodiments, short antisense compounds comprise at least one modified nucleotide. In certain embodiments short antisense compounds comprise at least two or more modified nucleotides. In certain embodiments, short antisense compounds comprise at least one modified internucleotide linkage. In certain embodiments, short antisense compounds are mixed-backbone oligonucleotides. In certain embodiments, short antisense compounds are chimeric oligonucleotides. In certain embodiments, short antisense oligonucleotides are uniformly modified. In certain embodiments, short antisense oligonucleotides comprise modifications independently selected at each nucleobase and at each linkage.

In certain embodiments, short antisense compounds are short gapmers. In certain such embodiments, short gapmers comprise at least one high affinity modification in one or more wings of the compound. In certain embodiments, short antisense compounds comprise 1 to 3 high-affinity modifications in each wing. In certain embodiments, high affinity modifications of the short antisense compounds allow for a target affinity similar to, or even greater than, the target affinity of longer antisense compounds. In certain embodiments, the high-affinity modified nucleotides are sugar modified nucleotides. Such sugar modified nucleotides include those comprising a bridge between the 4' and 2' position of the sugar. Exemplary high affinity sugar modifications include, but are not limited to, BNA s and other 2'-modifications such as 2'-MOE. In an alternate embodiment of the invention, the high affinity modification is not a 2'-O-(CH₂)ₙH (n=1-6) sugar-modified nucleotide. In an additional alternate embodiment, the high affinity modified nucleotide is not a 2'-OCH₃ or a 2'-OCH₂CH₂OCH₃ nucleotide. In certain embodiments, the high-affinity modified nucleotides confer a ΔTₘ of at least 1, at least 1.5, at least 2, at least 2.5, at least 3.0, at least 3.5 or at least 4.0 degrees per nucleotide. Some high-affinity nucleotide modifications are known in the art to increase toxicity. As shown herein, short antisense compounds having a limited number (generally 2 to 6) of high affinity modifications exhibit little to no increase in toxicity but retain or increase affinity for the target RNA, while also significantly reducing expression of the RNA target. Short antisense compounds of the invention may optionally comprise a conjugate group, such as, for example, cholesterol or C_{16.}

### 1. Certain Wings

In certain embodiments, the short antisense compounds comprise a 5' wing and/or a 3' wing. In such embodiments, the features of the 3' wing and the features of the 5' wing are selected independently. Thus, in such embodiments, the number of monomers in the 5' wing and the number of monomers (length) in the 3' wing may be the same or may be different; the modifications, if any, in the 5' wing may be the same as the modifications, if any, in the 3' wing or such modifications, if any, may be different; and the monomeric linkages in the 5' wing and the monomeric linkages in the 3' wing may be the same or they may be different.

In certain embodiments a wing comprises one, two or three monomers (i.e. has a length of 1, 2, or 3). In certain embodiments, the monomers of a wing are modified. In certain such embodiments, the monomers of the wing are modified to increase affinity of the antisense compound for its target nucleic acid. In certain embodiments, the monomers of a wing are nucleosides or nucleotides. In certain such embodiments, the nucleosides or nucleotides of the wing comprise a 2' modification. In certain such embodiments, the monomers (nucleosides or nucleotides) of the wing are BNA's. In certain such embodiments, the monomers of the wing are selected from α-L-Methyleneoxy (4'-CH₂-O-2') BNA , β-D-Methyleneoxy (4'-CH₂-O-2') BNA , Ethyleneoxy (4'-(CH₂)₂-O-2') BNA , Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, - OCF₃, O-(CH₂)₂-O-CH₃, 2'-O(CH₂)₂SCH₃, O-(CH₂)₂-O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, the monomeric linkages in a wing are naturally occurring internucleotide linkages. In certain embodiments, the monomeric linkages in a wing are non-naturally occurring internucleotide or internucleoside linkages. In certain such embodiments, the monomeric linkages in the wing are more resistant to one or more nucleases than naturally occurring internucleotide linkages. In certain such embodiments, the monomeric linkages in the wing are phosphorothioate linkages (P=S). In certain embodiments where a wing has more than one monomeric linkage, the monomeric linkages are the same as one another. In certain embodiments where a wing has more than one monomers linkage, the monomers linkages are different from each other.

One of ordinary skill in the art will recognize that the features and modifications discussed above may be used in any combination to prepare a wing. The table below provides non-limiting examples showing how one might prepare a wing by selecting a certain number of monomers, monomeric modifications (if any), and monomeric linkages both within the wing.

| Length | Monomer type/ modifications | monomeric linkages within wing |
|---|---|---|
| 1 | 2' MOE | None |
| 1 | BNA | None |
| 1 | Methyleneoxy BNA | None |
| 1 | ENA | None |
| 2 | 2' MOE | P=S |
| 2 | BNA | P=S |
| 2 | Methyleneoxy BNA | P=S |
| 2 | ENA | P=S |
| 2 | 2' MOE | P=O |
| 2 | BNA | P=O |
| 2 | Methyleneoxy BNA | P=O |
| 2 | ENA | P=O |
| 3 | 2' MOE | P=S |
| 3 | BNA | P=S |
| 3 | Methyleneoxy BNA | P=S |
| 3 | ENA | P=S |
| 3 | 2' MOE | P=O |
| 3 | BNA | P=O |
| 3 | Methyleneoxy BNA | P=O |
| 3 | ENA | P=O |

In certain embodiments in which a wing comprises two, three or four monomers, those two, three or four monomers all comprise the same modifications, if any. In certain embodiments in which a wing comprises two, three or four monomers, one or more of those two, three or four nucleobases comprises one or more modifications that is different from one or more of the modifications of one or more of the remaining monomers.

### 2. Certain Gaps

In certain embodiments, the short antisense compounds comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, the monomeric linkages in the gap are naturally occurring internucleotide linkages. In certain embodiments, the monomeric linkages in the gap are non-naturally occurring linkages. In certain such embodiments, the monomeric linkages in the gap are more resistant to one or more nuclease than naturally occurring internucleotide linkages. In certain such embodiments, the monomeric linkages in the gap are phosphorothioate linkages (P=S). In certain embodiments, the monomeric linkages in the gap are all the same as one another. In certain embodiments, the monomeric linkages within the gap are not all the same.

One of ordinary skill in the art will recognize that the features and modifications discussed above may be used in any combination to prepare a gap. The table below provides non-limiting examples showing how one might prepare a gap by selecting a certain number of monomers, monomeric modifications (if any), and monomeric linkages within the gap region.

| Length | Monomer type/ modifications | Monomeric linkages within gap |
|---|---|---|
| 5 | DNA | P=S |
| 6 | DNA | P=S |
| 7 | DNA | P=S |
| 8 | DNA | P=S |
| 9 | DNA | P=S |
| 10 | DNA | P=S |
| 11 | DNA | P=S |
| 12 | DNA | P=S |
| 13 | DNA | P=S |
| 14 | DNA | P=S |
| 6 | DNA | P=O |
| 7 | DNA | P=O |
| 8 | DNA | P=O |
| 9 | DNA | P=O |
| 10 | DNA | P=O |
| 11 | DNA | P=O |
| 12 | DNA | P=O |
| 8 | RNA | P=S |
| 9 | RNA | P=S |
| 10 | RNA | P=S |
| 11 | RNA | P=S |
| 12 | RNA | P=S |

### 3. Certain Gapped Antisense Oligomeric Compounds

One of ordinary skill in the art will recognize that the wings and the gaps discussed above may be selected and then combined in a variety of combinations to generate gapped oligomeric compounds, including, but not limited to, gapped antisense oligomeric compounds, and gapped antisense oligonucleotides. The features (length, modifications, linkages) of the 5' wing and the 3' wing may be selected independently of one another. The features of the gap include at least one difference in modification compared to the features of the 5' wing and at least one difference compared to the features of the 3' wing (i.e., there must be at least one difference in modification between neighboring regions to distinguish those neighboring regions from one another). The features of the gap may otherwise be selected independently.

In certain embodiments, the monomeric linkages within a wing and the monomeric linkages within the gap are the same. In certain embodiments, the monomeric linkages within a wing and the monomeric linkages within the gap are different. In certain such embodiments, the monomeric linkage bridging the wing and the gap are the same as the monomeric linkages in the wing. In certain embodiments, the monomeric linkage bridging the wing and the gap are the same as the monomeric linkages in the gap. In certain embodiments, short antisense compounds have uniform linkages throughout the compound. In certain such embodiments, all of the linkages are phosphorothioate (P=S) linkages.

One of ordinary skill in the art will recognize that the 3' wings, 5' wings, gaps, and linkages discussed above may be used in any combination to prepare a gapmer. The table below provides non-limiting examples showing how one might prepare a gapmer by selecting a certain 5' wing, a gap, a 3' wing and certain linkages bridging the gap and each wing.

| 5' Wing | | | 5'Bridge | Gap | | | 3' Bridge | 3' Wing | | |
|---|---|---|---|---|---|---|---|---|---|---|
| Length | Monomer | Link | Link | Length | Monomer | Link | Link | Length | Monomer | Link |
| 2 | MOE | P=S | P=S | 6 | DNA | P=S | P=S | 2 | MOE | P=S |
| 2 | BNA | P=S | P=O | 8 | DNA | P= O | P=S | 3 | BNA | P=S |
| 1 | MOE | Non e | P=S | 10 | DNA | P=S | P=S | 1 | MOE | P=S |
| 2 | MOE | P=S | P=S | 8 | RNA | P=S | P=S | 2 | MOE | P=S |
| 3 | Methyleneo xy BNA | P=S | P=S | 8 | RNA | P=S | P=S | 3 | MOE | P=S |
| 3 | DNA | P=O | P=O | 10 | RNA | P=S | P=O | 3 | 2'OH | P= O |
| 2 | 2-F | P=S | P=S | 5 | RNA | P=S | P=S | 2 | 2'-F | P=S |
| 1 | MOE | P=O | P=S | 5 | DNA | P= O | P=S | 4 | MOE | P=S |

In certain embodiments, the oligomeric compounds disclosed herein may comprise from about 8 to about 16, preferably 9 to 15, more preferably 9 to 14, more preferably 10 to 14 monomers (i.e. from about 8 to about 16 linked monomers). One of ordinary skill in the art will appreciate that this comprehends antisense compounds of 8, 9, 10, 11, 12, 13, 14, 15 or 16 nucleobases. In certain embodiments, oligomeric compounds are antisense compounds.

In certain embodiments, short antisense compounds are 8 nucleobases in length.

In certain embodiments, short antisense compounds are 9 nucleobases in length.

In certain embodiments, short antisense compounds are 10 nucleobases in length.

In certain embodiments, short antisense compounds are 11 nucleobases in length.

In certain embodiments, short antisense compounds are 12 nucleobases in length.

In certain embodiments, short antisense compounds are 13 nucleobases in length.

In certain embodiments, short antisense compounds are 14 nucleobases in length.

In certain embodiments, short antisense compounds are 15 nucleobases in length.

In certain embodiments, short antisense compounds are 16 nucleobases in length.

In certain embodiments, short antisense compounds are 8 monomers in length. In certain embodiments, short antisense compounds are 9 monomers in length. In certain embodiments, short antisense compounds are 10 monomers in length. In certain embodiments, short antisense compounds are 11 monomers in length. In certain embodiments, short antisense compounds are monomers in length. In certain embodiments, short antisense compounds are 13 monomers in length. In certain embodiments, short antisense compounds are 14 monomers in length. In certain embodiments, short antisense compounds are 15 monomers in length. In certain embodiments, short antisense compounds are 16 monomers in length. In certain embodiments, short antisense compounds comprise 9 to 15 monomers. In certain embodiments, short antisense compounds comprise 10 to 15 monomers. In certain embodiments, short antisense compounds comprise 12 to 14 monomers. In certain embodiments, short antisense compounds comprise 12 to 14 nucleotides or nucleosides.

One having skill in the art and informed by the short antisense compounds illustrated herein will be able, without undue experimentation, to identify further short antisense compounds.

In certain embodiments, short antisense compounds comprise a gap flanked by more than one wing on either or both sides. Thus, in certain embodiments, a short antisense compound comprises two or more 5' wings and two or more 3' wings. In certain embodiments, a short antisense compound comprises one 5' wing and two or more 3' wings. In certain embodiments, a short antisense compound comprises one 3' wing and two or more 5' wings. Certain such embodiments comprise, for example, the following regions: a first 5' wing - a bridge - a second 5' wing - a bridge - a gap - a bridge - a second 3' wing - a bridge - a first 3 'wing. In such embodiments, each region has at least one difference in modification when compared to its neighboring region. Thus, in such embodiments, the second 5' wing and the second 3' wing each independently comprises one or more differences in modification compared to the gap and compared to the first 5' wing and the first 3' wing. In such embodiments, the modifications of the first 3' wing and first 5' wing may either or both be the same or different from the modifications of the gap, if any.

### 4. Certain Conjugate Groups

In one aspect, oligomeric compounds are modified by covalent attachment of one or more conjugate groups. In general, conjugate groups modify one or more properties of the attached oligomeric compound including but not limited to pharmacodynamic, pharmacokinetic, binding, absorption, cellular distribution, cellular uptake, charge and clearance. Conjugate groups are routinely used in the chemical arts and are linked directly or via an optional linking moiety or linking group to a parent compound such as an oligomeric compound. A preferred list of conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes.

Preferred conjugate groups amenable to the present invention include lipid moieties such as a cholesterol moiety (Letsinger et al., Proc. Natl. Acad. Sci. USA, 1989, 86, 6553); cholic acid (Manoharan et al., Bioorg. Med. Chem. Lett., 1994, 4, 1053); a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765); a thiocholesterol (Oberhauser et al., Nucl. Acids Res., 1992, 20, 533); an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49); a phospholipid, e.g., dihexadecyl-rac-glycerol or triethylammonium-1,2-di-O-hexadecyl-rac-glycero-3-H-phosphonate (Manoharan et al., Tetrahedron Lett., 1995, 36, 3651; Shea et al., Nucl. Acids Res., 1990, 18, 3777); a polyamine or a polyethylene glycol chain (Manoharan et al., Nucleosides & Nucleotides, 1995, 14, 969); adamantane acetic acid (Manoharan et al., Tetrahedron Lett., 1995, 36, 3651); a palmityl moiety (Mishra et al., Biochim. Biophys. Acta, 1995, 1264, 229); or an octadecylamine or hexylamino-carbonyloxycholesterol moiety (Crooke et al., J. Pharmacol. Exp. Ther., 1996, 277, 923).

Linking groups or bifunctional linking moieties such as those known in the art are amenable to the compounds provided herein. Linking groups are useful for attachment of chemical functional groups, conjugate groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

### 5. Synthesis. Purification and Analysis

Oligomerization of modified and unmodified nucleosides and nucleotides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Oligomeric compounds provided herein can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

Methods of purification and analysis of oligomeric compounds are known to those skilled in the art. Analysis methods include capillary electrophoresis (CE) and electrospray-mass spectroscopy. Such synthesis and analysis methods can be performed in multi-well plates. The method of the invention is not limited by the method of oligomer purification.

### D. Antisense

Antisense mechanisms are all those involving the hybridization of a compound with target nucleic acid, wherein the outcome or effect of the hybridization is either target degradation or target occupancy with concomitant stalling of the cellular machinery involving, for example, transcription or splicing.

One type of antisense mechanism involving target degradation includes an RNase H. RNase H is a cellular endonuclease which cleaves the RNA strand of an RNA:DNA duplex. It is known in the art that single-stranded antisense compounds which are "DNA-like" elicit RNAse H activity in mammalian cells. Activation of RNase H, therefore, results in cleavage of the RNA target, thereby greatly enhancing the efficiency of DNA-like oligonucleotide-mediated inhibition of gene expression.

In certain embodiments, chemically-modified antisense compounds have a higher affinity for target RNAs than does non-modified DNA. In certain such embodiments, that higher affinity in turn provides increased potency allowing for the administration of lower doses of such compounds, reduced potential for toxicity and improvement in therapeutic index and decreased overall cost of therapy.

The present disclosure demonstrates that the incorporation of chemically-modified high-affinity nucleotides and nucleosides into antisense compounds allows for the design of short antisense compounds 8-16 nucleobases in length useful for the reduction of target RNAs and/or target proteins in cells, tissues, and animals, including, but not limited to, humans with increased potency and improved therapeutic index. Thus, in certain embodiments, provided herein are short antisense compounds comprising high-affinity nucleotide modifications useful for reducing a target RNA *in vivo.* Certain such short antisense compounds are effective at lower doses than previously described antisense compounds, allowing for a reduction in toxicity and cost of treatment. In addition, certain short antisense compounds have greater potential for oral dosing.

To address the need for more potent antisense compounds, provided herein are short antisense compounds (8-16, preferably 9 to 15, more preferably 9 to 14, more preferably 10 to 14 nucleotides in length) with increased activity *in vivo* relative to longer compounds. Certain short antisense compounds are gapmer compounds comprising high-affinity chemically-modified nucleotides on the 3' and 5' ends (wings) of the compound. In certain embodiments, the addition of high-affinity modified nucleotides allows antisense compounds to be active against, and specific for, their intended target RNA *in vivo* despite being shorter in length. Contemplated herein are short antisense compounds wherein each of the wings independently comprises 1 to 3 high-affinity modified nucleotides. In certain embodiments, the high-affinity modifications are sugar modifications. High-affinity modified nucleotides include, but are not limited to, BNA s or other 2'-modified nucleotides, such as 2'-MOE nucleotides. Also contemplated are short antisense compounds having at least one modified internucleotide linkage, such as a phosphorothioate internucleotide linkage. In certain embodiments, the short antisense compounds of the present invention can have all phosphorothioate internucleoside linkages. The short antisense compounds optionally comprise a conjugate group. As shown herein, short antisense compounds have greater affinity for target RNA than they have for DNA and are significantly more potent *in vivo* as shown by reduction of target mRNA as well as by amelioration of a variety of disease indications.

As used herein, an RNA which is involved in regulating glucose metabolism or clearance, lipid metabolism, cholesterol metabolism or insulin metabolism is any RNA involved in the biochemical pathways that regulate these processes. Such RNAs are well known in the art. Examples of target genes include, but are not limited to, ApoB-100 (also known as APOB; Ag(x) antigen; apoB-48; apolipoprotein B; apolipoprotein B-100; apolipoprotein B-48) and GCGR (also known as glucagon receptor; GR), CRP, DGAT2, GCCR, PCSK9, PTEN, PTP1B, SGLT2, and SOD1.

### 1. Modulation of Target Expression

In certain embodiments, a target is identified and antisense oligonucleotides are designed to modulate that target or its expression. In certain embodiments, designing an oligomeric compound to a target nucleic acid molecule can be a multistep process. Typically the process begins with the identification of a target protein, the activity of which is to be modulated, and then identifying the nucleic acid the expression of which yields the target protein. In certain embodiments, designing of an antisense compound results in an antisense compound that is hybridizable to the targeted nucleic acid molecule. In certain embodiments, the antisense compound is an antisense oligonucleotide or antisense oligonucleoside. In certain embodiments, an antisense compound and a target nucleic acid are complementary to one another. In certain such embodiments, an antisense compound is perfectly complementary to a target nucleic acid. In certain embodiments, an antisense compound includes one mismatch. In certain embodiments, an antisense compound includes two mismatches. In certain embodiments, an antisense compound includes three or more mismatches.

Modulation of expression of a target nucleic acid can be achieved through alteration of any number of nucleic acid functions. In certain embodiments, the functions of RNA to be modulated include, but are not limited to, translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. Thus, in one embodiment modulation of expression can mean increase or decrease in target RNA or protein levels. In another embodiment modulation of expression can mean an increase or decrease of one or more RNA splice products, or a change in the ratio of two or more splice products.

In certain embodiments, expression of a target gene is modulated using an oligomeric compound comprising from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of a target gene using one or more antisense compounds of 8, 9, 10, 11, 12, 13, 14, 15 or 16 nucleobases.

In certain embodiments, methods of modulating a target gene comprises use of a short antisense compound that is 8 nucleobases in length. In certain embodiments, methods of modulating a target gene comprises use of a short antisense compound that is 9 nucleobases in length. In certain embodiments, methods of modulating a target gene comprises use of a short antisense compound that is 8 nucleobases in length. In certain embodiments, methods of modulating a target gene comprises use of a short antisense compound that is 10 nucleobases in length. In certain embodiments, methods of modulating a target gene comprises use of a short antisense compound that is 10 nucleobases in length. In certain embodiments, methods of modulating a target gene comprises use of a short antisense compound that is 11 nucleobases in length. In certain embodiments, methods of modulating a target gene comprises use of a short antisense compound that is 12 nucleobases in length. In certain embodiments, methods of modulating a target gene comprises use of a short antisense compound that is 13 nucleobases in length. In certain embodiments, methods of modulating a target gene comprises use of a short antisense compound that is 14 nucleobases in length. In certain embodiments, methods of modulating a target gene comprises use of a short antisense compound that is 15 nucleobases in length. In certain embodiments, methods of modulating a target gene comprises use of a short antisense compound that is 16 nucleobases in length.

In certain embodiments, methods of modulating expression of a target gene comprises use of a short antisense compound comprising 9 to 15 monomers. In certain embodiments, methods of modulating expression of a target gene comprises use of a short antisense compound comprising 10 to 15 monomers. In certain embodiments, methods of modulating expression of a target gene comprises use of a short antisense compound comprising 12 to 14 monomers. In certain embodiments, methods of modulating expression of a target gene comprises use of a short antisense compound comprising 12 or 14 nucleotides or nucleosides.

### 2. Hybridization

In certain embodiments, antisense compounds specifically hybridize when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

As used herein, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

### 3. Complementarity

It is understood in the art that incorporation of nucleotide affinity modifications may allow for a greater number of mismatches compared to an unmodified compound. Similarly, certain oligonucleotide sequences may be more tolerant to mismatches than other oligonucleotide sequences. One of ordinary skill in the art is capable of determining an appropriate number of mismatches between oligonucleotides, or between an oligonucleotide and a target nucleic acid, such as by determining melting temperature (Tₘ). Tₘ or ΔTₘ can be calculated by techniques that are familiar to one of ordinary skill in the art. For example, techniques described in Freier et al. (Nucleic Acids Research, 1997, 25, 22: 4429-4443) allow one of ordinary skill in the art to evaluate nucleotide modifications for their ability to increase the melting temperature of an RNA:DNA duplex.

### 4. Identity

Antisense compounds, or a portion thereof, may have a defined percent identity to a SEQ ID NO, or a compound having a specific Isis number. As used herein, a sequence is identical to the sequence disclosed herein if it has the same nucleobase pairing ability. For example, an RNA which contains uracil in place of thymidine in the disclosed sequences of the compounds described herein would be considered identical as they both pair with adenine. This identity may be over the entire length of the oligomeric compound, or in a portion of the antisense compound (e.g., nucleobases 1-20 of a 27-mer may be compared to a 20-mer to determine percent identity of the oligomeric compound to the SEQ ID NO. It is understood by those skilled in the art that an antisense compound need not have an identical sequence to those described herein to function similarly to the antisense compound described herein. Shortened versions of antisense compounds taught herein, or non-identical versions of the antisense compounds taught herein, are also provided herein. Non-identical versions are those wherein each base does not have the same pairing activity as the antisense compounds disclosed herein. Bases do not have the same pairing activity by being shorter or having at least one abasic site. Alternatively, a non-identical version can include at least one base replaced with a different base with different pairing activity (e.g., G can be replaced by C, A, or T). Percent identity is calculated according to the number of bases that have identical base pairing corresponding to the SEQ ID NO or antisense compound to which it is being compared. The non-identical bases may be adjacent to each other, dispersed through out the oligonucleotide, or both.

For example, a 16-mer having the same sequence as nucleobases 2-17 of a 20-mer is 80% identical to the 20-mer. Alternatively, a 20-mer containing four nucleobases not identical to the 20-mer is also 80% identical to the 20-mer. A 14-mer having the same sequence as nucleobases 1-14 of an 18-mer is 78% identical to the 18-mer. Such calculations are well within the ability of those skilled in the art.

The percent identity is based on the percent of nucleobases in the original sequence present in a portion of the modified sequence. Therefore, a 30 nucleobase antisense compound comprising the full sequence of the complement of a 20 nucleobase active target segment would have a portion of 100% identity with the complement of the 20 nucleobase active target segment, while further comprising an additional 10 nucleobase portion. In the context of the instant description, the complement of an active target segment may constitute a single portion. In preferred embodiments, the oligonucleotides provided herein are at least 80%, at least 85%, at least 90%, at least 95%, at least 96%, at least 97%, at least 98%, at least 99% or 100% identical to at least a portion of the complement of the active target segments presented herein.

### E. Target Nucleic Acids, Regions and Segments

In certain embodiments, short antisense compounds may be designed to target any target nucleic acid. In certain embodiments, the target nucleic acid encodes a target that is clinically relevant. In such embodiments, modulation of the target nucleic acid results in clinical benefit. Certain target nucleic acids include, but are not limited to, the target nucleic acids illustrated in Table 1.

In certain embodiments, a target nucleic acid is a nucleic acid molecule encoding ApoB. Nucleic acid molecules that encode ApoB include, without limitation, SEQ ID NO: 1 and SEQ ID NO: 2.

In certain embodiments, a target nucleic acid is a nucleic acid molecule encoding SGLT2. Nucleic acid molecules that encode SGLT2 include, without limitation, SEQ ID NO: 3.

In certain embodiments, a target nucleic acid is a nucleic acid molecule encoding PCSK9. Nucleic acid molecules that encode PCSK9 include, without limitation, SEQ ID NO: 4.

In certain embodiments, a target nucleic acid is a nucleic acid molecule encoding SOD1. Nucleic acid molecules that encode SOD1 include, without limitation, SEQ ID NO: 5.

In certain embodiments, a target nucleic acid is a nucleic acid molecule encoding CRP. Nucleic acid molecules that encode CRP include, without limitation, SEQ ID NO: 6.

In certain embodiments, a target nucleic acid is a nucleic acid molecule encoding GCCR. Nucleic acid molecules that encode GCCR include, without limitation, SEQ ID NO: 7 and SEQ ID NO: 8.

In certain embodiments, a target nucleic acid is a nucleic acid molecule encoding GCGR. Nucleic acid molecules that encode GCGR include, without limitation, SEQ ID NO: 9.

In certain embodiments, a target nucleic acid is a nucleic acid molecule encoding DGAT2. Nucleic acid molecules that encode DGAT2 include, without limitation, SEQ ID NO: 10.

In certain embodiments, a target nucleic acid is a nucleic acid molecule encoding PTP1B. Nucleic acid molecules that encode PTP1B include, without limitation, SEQ ID NO: 11 and SEQ ID NO: 12.

In certain embodiments, a target nucleic acid is a nucleic acid molecule encoding PTEN. Nucleic acid molecules that encode PTEN include, without limitation, SEQ ID NO: 14 or SEQ ID NO: 15.

**Table 1: Certain Target Nucleic Acids**

| **Target** | **Species** | **GENBANK® Accession Number** | **SEQ ID NO** |
|---|---|---|---|
| ApoB | Human | NM_000384.1 | 1 |
| ApoB | Mouse | XM_137955.5 | 2 |
| SGLT2 | Human | NM_003041.1 | 3 |
| PCSK9 | Human | NM_174936.2 | 4 |
| SOD1 | Human | X02317.1 | 5 |
| CRP | Human | NM_000567.1 | 6 |
| GCCR | Mouse | BC031885.1 | 7 |
| GCCR | Human | Nucleotides 1 to 10600 of AC012634 | 8 |
| GCGR | Human | NM_000160.1 | 9 |
| DGAT2 | Human | NM_032564.2 | 10 |
| PTP1B | Human | NM_002827.2 | 11 |
| PTP1B | Human | Nucleotides 1417800 to 1425600 of NT_011362.9 | 12 |
| PTEN | Mouse | U92437.1 | 13 |
| PTEN | Human | NM_000314.4 | 14 |
| PTEN | Human | Nucleotides 8063255 to 8167140 of NT_033890.3 | 15 |

The targeting process usually includes determination of at least one target region, segment, or site within the target nucleic acid for the antisense interaction to occur such that the desired effect will result.

In certain embodiments, the 5'-most nucleotide of a target region is the 5' target site of a short antisense compound and the 3'-most nucleotide of a target region is the 3' target site of the same short antisense compound. In certain embodiments, the 5'-most nucleotide of a target region is the 5' target site of a short antisense compound and the 3'-most nucleotide of a target region is the 3' target site of a different short antisense compound. In certain embodiments, a target region comprises a nucleotide sequence within 10, 15, or 20 nucleotides of a 5' target site or a 3' target site.

In certain embodiments, a target region is a structurally defined region of the nucleic acid. For example, in certain such embodiments, a target region may encompass a 3' UTR, a 5' UTR, an exon, an intron, a coding region, a translation initiation region, translation termination region, or other defined nucleic acid region.

The locations on the target nucleic acid defined by having one or more active short antisense compounds targeted thereto are referred to as "active target segments." In certain embodiments, the target nucleic acid having one or more active short antisense compounds targeted thereto is a target RNA. When an active target segment is defined by multiple short antisense compounds, the compounds are preferably separated by no more than about 10 nucleotides on the target sequence, more preferably no more than about 5 nucleotides on the target sequence, even more preferably the short antisense compounds are contiguous, most preferably the short antisense compounds are overlapping. There may be substantial variation in activity (e.g., as defined by percent inhibition) of the short antisense compounds within an active target segment. Active short antisense compounds are those that modulate the expression of their target nucleic acid, including but not limited to a target RNA. Active short antisense compounds inhibit expression of their target RNA at least 10%, preferably 20%. In a preferred embodiment, at least about 50%, preferably about 70% of the short antisense compounds targeted to the active target segment modulate expression of their target RNA at least 40%. In a more preferred embodiment, the level of inhibition required to define an active short antisense compound is defined based on the results from the screen used to define the active target segments.

A suitable target segment is at least about an 8-nucleobase portion of a target region to which an active short antisense compound is targeted. Target segments can include DNA or RNA sequences that comprise at least the 8 consecutive nucleobases from the 5'-terminus of one of the illustrative target segments (the remaining nucleobases being a consecutive stretch of the same DNA or RNA beginning immediately upstream of the 5'-terminus of the target segment and continuing until the DNA or RNA comprises about 8 to about 16 nucleobases). Target segments are also represented by DNA or RNA sequences that comprise at least the 8 consecutive nucleobases from the 3'-terminus of one of the illustrative target segments (the remaining nucleobases being a consecutive stretch of the same DNA or RNA beginning immediately downstream of the 3'-terminus of the target segment and continuing until the DNA or RNA comprises about 8 to about 16 nucleobases). It is also understood that antisense target segments may be represented by DNA or RNA sequences that comprise at least 8 consecutive nucleobases from an internal portion of the sequence of an illustrative target segment, and may extend in either or both directions until the short antisense compound comprises about 8 to about 16 nucleobases. One having skill in the art armed with the target segments illustrated herein will be able, without undue experimentation, to identify further target segments.

Once one or more target regions, segments or sites have been identified, short antisense compounds are chosen which are sufficiently complementary to the target, i.e., hybridize sufficiently well and with sufficient specificity, to give the desired effect.

The short antisense compounds may also be targeted to regions of the target nucleobase sequence comprising any consecutive nucleobases 8 to 16 nucleobases in length along the target nucleic acid molecule.

Target segments 8-16 nucleobases in length comprising a stretch of at least eight (8) consecutive nucleobases selected from within the illustrative target segments are considered to be suitable for targeting as well. Thus, the short antisense compounds may also encompass 8-16 nucleobases within those segments identified herein as beginning at a particular 5' target site. Any segment of 8, 9, 10, 11, or more preferably 12, 13, 14, 15 or 16 contiguous nucleobases in a 50, preferably 25, more preferably 16 nucleobase perimeter around these regions are also considered to be suitable for targeting.

In a further embodiment, the "suitable target segments" identified herein may be employed in a screen for additional short antisense compounds that modulate the expression of a target nucleic acid. "Modulators" are those compounds that decrease or increase the expression of a target nucleic acid and which comprise at least an 8-nucleobase portion which is complementary to a target segment. The screening method comprises the steps of contacting a target segment of a nucleic acid with one or more candidate modulators, and selecting for one or more candidate modulators which decrease or increase the expression of a target nucleic acid. Once it is shown that the candidate modulator or modulators are capable of modulating (e.g. either decreasing or increasing) the expression of a target nucleic acid, the modulator may then be employed in further investigative studies of the function of the target, or for use as a research, diagnostic, or therapeutic agent in accordance with the present invention.

For all short antisense compounds discussed herein, sequence, monomer, monomeric modification, and monomeric linkage may each be selected independently. In certain embodiments, short antisense compounds are described by a motif. In such embodiments, any motif may be used with any sequence, whether or not the sequence and/or the motif is specifically disclosed herein. In certain embodiments, short antisense compounds comprise modifications that are not amenable to description by motif (for example, short antisense compounds comprising several different modifications and/or linkages at various positions throughout the compound). Such combinations may be incorporated for any sequence, whether or not it is disclosed herein. The sequence listing accompanying this filing provides certain nucleic acid sequences independent of chemical modification. Though that listing identifies each sequence as either "RNA" or "DNA" as required, in reality, those sequences may be modified with any combination of chemical modifications and/or motifs.

In certain embodiments, short antisense compounds comprise at least one high-affinity modified monomer. In certain embodiments, provided are short antisense compounds targeted to nucleic acid molecules encoding targets including, but not limited to, ApoB-100 (also known as APOB; Ag(x) antigen; apoB-48; apolipoprotein B; apolipoprotein B-100; apolipoprotein B-48), GCGR (also known as glucagon receptor; GR), CRP, DGAT2, GCCR, PCSK9, PTEN, PTP1B, SGLT2, and SOD1. In certain such embodiments, such short antisense compounds are targeted to a nucleic acid molecule encoding any of those targets.

### F. Certain Targets

In certain embodiments, short antisense compounds may be designed to modulate any target. In certain embodiments, the target is clinically relevant. In such embodiments, modulation of the target results in clinical benefit. Certain targets are preferentially expressed in the kidney. Certain targets are preferentially expressed in the liver. Certain targets are associated with a metabolic disorder. Certain targets are associated to a cardiovascular disorder. In certain embodiments, a target is selected from: ApoB, SGLT2, PCSK9, SOD1, CRP, GCCR, GCGR, DGAT2, PTP1B, and PTEN. In certain embodiments, a target is selected from: ApoB, SGLT2, PCSK9, SOD1, CRP, GCCR, GCGR, DGAT2, and PTP1B. In certain embodiments, a target is any protein other than SGLT2.

In certain embodiments, short antisense compounds exhibit liver and kidney-specific target RNA reduction *in vivo.* Such property renders those short antisense compounds particularly useful for inhibition of many target RNAs involved in metabolic and cardiovascular diseases. Thus, provided herein are methods of treating cardiovascular or metabolic disorders by contacting said kidney or liver tissues with short antisense compounds targeted to RNAs associated with said disorders. Thus, also provided are methods for ameliorating any of a variety of metabolic or cardiovascular disease indications with the short antisense compounds of the present invention.

### 7. Glucagon Receptor (GCGR)

The maintenance of normal glycemia is a carefully regulated metabolic event. Glucagon, the 29-amino acid peptide responsible for maintaining blood glucose levels in the postabsorbative state, increases glucose release from the liver by activating hepatic glycogenolysis, gluconeogenesis, stimulating lipolysis in adipose tissue, and stimulating insulin secretion. During high blood glucose levels, insulin reverses the glucagon-mediated enhancement of glycogenolysis and gluconeogenesis. In patients with diabetes, insulin is either not available or not fully effective. While treatment for diabetes has traditionally focused on increasing insulin levels, antagonism of glucagon function has been considered as an alternative therapy. As glucagon exerts its physiological effects by signaling through the glucagon receptor, the glucagon receptor has been proposed as a potential therapeutic target for diabetes (Madsen et al., Curr. Pharm. Des., 1999, 5, 683-691).

Glucagon receptor is belongs to the superfamily of G-protein-coupled receptors having seven transmembrane domains. It is also a member of the smaller sub-family of homologous receptors which bind peptides that are structurally similar to glucagon. The gene encoding human glucagon receptor was cloned in 1994 and analysis of the genomic sequence revealed multiple introns and an 82% identity to the rat glucagon receptor gene (Lok et al., Gene, 1994, 140, 203-209.; MacNeil et al., Biochem. Biophys. Res. Commun., 1994, 198, 328-334). Cloning of the rat glucagon receptor gene also led to the description of multiple alternative splice variants (Maget et al., FEBS Lett., 1994, 351, 271-275). The human glucagon receptor gene is localized to chromosome 17q25 (Menzel et al., Genomics, 1994, 20, 327-328). A missense mutation of Gly to Ser at codon 40 in the glucagon receptor gene leads to a 3-fold lower affinity for glucagon (Fujisawa et al., Diabetologia, 1995, 38, 983-985) and this mutation has been linked to several disease states, including non-insulin-dependent diabetes mellitus (Fujisawa et al., Diabetologia, 1995, 38, 983-985), hypertension (Chambers and Morris, Nat. Genet., 1996, 12, 122), and central adiposity (Siani et al., Obes. Res., 2001, 9, 722-726).

### Definitions

"Glucagon receptor" is the gene product or protein of which expression is to be modulated by administration of a short antisense compound. Glucagon receptor is generally referred to as GCGR but may also be referred to as GR, GGR, MGC138246, MGC93090.

"GCGR nucleic acid" means any nucleic acid encoding GCGR. For example, in certain embodiments, a GCGR nucleic acid includes, without limitation, a GCGR sequence encoding GCGR, an RNA sequence transcribed from DNA encoding GCGR, and an mRNA sequence encoding GCGR. "GCGR mRNA" means an mRNA encoding a GCGR protein.

### Therapeutic Indications

Antisense technology is an effective means for reducing glucagon receptor (GCGR) expression and has proven to be uniquely useful in a number of therapeutic, diagnostic, and research applications. As such, in certain embodiments, the present invention provides short antisense compounds targeted to a nucleic acid encoding glucagon receptor, and which modulate the expression of glucagon receptor. Further provided herein are short antisense compounds capable of inhibiting GCGR expression. Also provided herein are methods of treating an individual comprising administering one or more pharmaceutical compositions comprising a short antisense compound targeted to a GCGR nucleic acid. In certain embodiments, because short antisense compounds targeted to a GCGR nucleic acid inhibit GCGR expression, provided herein are methods of treating a subject having a disease or condition associated with GCGR activity by administering one or more pharmaceutical compositions comprising a short antisense compound targeted to a GCGR nucleic acid. For example, provided herein are methods of treating a subject having high blood glucose, hyperglycemia, prediabetes, diabetes, Type 2 diabetes, metabolic syndrome, obesity and/or insulin resistance.

Also contemplated herein are pharmaceutical composition comprising one or more short antisense compounds targeted to GCGR and optionally a pharmaceutically acceptable carrier, diluent, enhancer or excipient. Certain compounds of the invention can also be used in the manufacture of a medicament for the treatment of diseases and disorders related to glucagon effects mediated by GCGR.

Certain embodiments of the present invention include methods of reducing the expression of GCGR in tissues or cells comprising contacting said cells or tissues with a short antisense compound targeted to a nucleic acid encoding GCGR or pharmaceutical composition comprising such a short antisense compound. In certain such embodiments, the invention provides methods of decreasing blood glucose levels, blood triglyceride levels, or blood cholesterol levels in a subject comprising administering to the subject a short antisense compound or a pharmaceutical composition. Blood levels may be plasma levels or serum levels. Also contemplated are methods of improving insulin sensitivity, methods of increasing GLP-1 levels and methods of inhibiting hepatic glucose output in an animal comprising administering to said animal an antisense oligonucleotide or a pharmaceutical composition of the invention. An improvement in insulin sensitivity may be indicated by a reduction in circulating insulin levels.

In certain embodiments, the invention provides methods of treating a subject having a disease or condition associated with glucagon activity via GCGR comprising administering to the subject a therapeutically or prophylactically effective amount of a short antisense compound or a pharmaceutical composition. In certain embodiments, such disease or condition may be a metabolic disease or condition. In certain embodiments, the metabolic disease or condition is diabetes, hyperglycemia, hyperlipidemia, metabolic syndrome X, obesity, primary hyperglucagonemia, insulin deficiency, or insulin resistance. In some embodiments, the diabetes is Type 2 diabetes. In some embodiments the obesity is diet-induced. In some embodiments, hyperlipidemia is associated with elevated blood lipid levels. Lipids include cholesterol and triglycerides. In one embodiment, the condition is liver steatosis. In some embodiments, the steatosis is steatohepatitis or non-alcoholic steatohepatitis.

In certain embodiments, the invention provides methods of preventing or delaying the onset of elevated blood glucose levels in an animal as well as methods of preserving beta-cell function in an animal using the oligomeric compounds delineated herein.

Certain short antisense compounds targeted to GCGR can be used to modulate the expression of GCGR in a subject in need thereof, such as an animal, including, but not limited to, a human In certain embodiments, such methods comprise the step of administering to said animal an effective amount of a short antisense compound that reduces expression of GCGR RNA. In certain embodiments, short antisense compounds effectively reduce the levels or function of GCGR RNA. Because reduction in GCGR mRNA levels can lead to alteration in GCGR protein products of expression as well, such resultant alterations can also be measured. Certain antisense compounds that effectively reduce the levels or function of GCGR RNA or protein products of expression is considered an active antisense compound. In certain embodiments, short antisense compounds reduce the expression of GCGR causing a reduction of RNA by at least 10%, by at least 20%, by at least 25%, by at least 30%, by at least 40%, by at least 50%, by at least 60%, by at least 70%, by at least 75%, by at least 80%, by at least 85%, by at least 90%, by at least 95%, by at least 98%, by at least 99%, or by 100%.

Further provided are methods of screening for modulators of glucagon receptor and methods of modulating the expression of glucagon receptor in cells, tissues or animals comprising contacting said cells, tissues or animals with one or more short antisense compounds targeted to GCGR or with compositions comprising such compounds. Methods of treating an animal, particularly a human, suspected of having or being prone to a disease or condition associated with expression of glucagon receptor are also set forth herein. Certain such methods comprise administering a therapeutically or prophylactically effective amount of one or more of the compounds or compositions of the invention to the person in need of treatment.

The reduction of the expression of glucagon receptor may be measured, for example, in blood, plasma, serum, adipose tissue, liver or any other body fluid, tissue or organ of the animal. Preferably, the cells contained within said fluids, tissues or organs being analyzed contain a nucleic acid molecule encoding glucagon receptor protein and/or the glucagon receptor protein itself.

Pharmaceutical and other compositions comprising short antisense compounds are also provided. In certain embodiments short antisense compounds targeted to a nucleic acid encoding GCGR are utilized in pharmaceutical compositions by adding an effective amount of a compound to a suitable pharmaceutically acceptable diluent or carrier.

The short antisense compounds targeting a GCGR nucleic acid may have any one or more properties or characteristics of the short antisense compounds generally described herein. In certain embodiments, short antisense compounds targeting a GCGR nucleic acid have a motif (wing - deoxy gap -wing) selected from 1-12-1, 1-1-10-2, 2-10-1-1, 3-10-3, 2-10-3, 2-10-2, 1-10-1,1-10-2, 3-8-3, 2-8-2, 1-8-1, 3-6-3 or 1-6-1. In certain embodiments, short antisense compounds targeting a GCGR nucleic acid have a motif (wing - deoxy gap -wing) selected from 1-12-1, 2-10-2, 3-10-3, 3-8-3, 1-1-10-2.

### Certain Short Antisense Compounds Targeted to a GCGR nucleic acid

In certain embodiments, short antisense compounds are targeted to a GCGR nucleic acid having the sequence GENBANK® Accession No. NM_000160.1, incorporated herein as SEQ ID NO: 9. In certain such embodiments, a short antisense compound targeted to SEQ ID NO: 9 is at least 90% complementary to SEQ ID NO: 9. In certain such embodiments, a short antisense compound targeted to SEQ ID NO: 9 is at least 95% complementary to SEQ ID NO: 9. In certain such embodiments, a short antisense compound targeted to SEQ ID NO: 9 is 100% complementary to SEQ ID NO: 9. In certain embodiments, a short antisense compound targeted to SEQ ID NO: 9 includes a nucleotide sequence selected from the nucleotide sequences set forth in Tables 12 and 13.

The nucleotide sequences set forth in each SEQ ID NO in Tables 12 and 13 are independent of any modification to a sugar moiety, an internucleoside linkage, or a nucleobase. As such, short antisense compounds defined by a SEQ ID NO may comprise, independently, one or more modifications to a sugar moiety, an internucleoside linkage, or a nucleobase. Short antisense compounds described by Isis Number (Isis NO.) indicate a combination of nucleobase sequence and one or more modifications to a sugar moiety, an internucleoside linkage, or a nucleobase.

In certain embodiments, short antisense compounds targeted to a GCCR nucleic acid comprise a gapmer motif. In certain embodiments, a short antisense compound targeted to a GCCR nucleic acid comprises a 3-10-3 gapmer motif. In certain embodiments, short antisense compounds targeted to a GCCR nucleic acid comprise a gapmer motif. In certain embodiments, a short antisense compound targeted to a GCCR nucleic acid comprises a 3-8-3 gapmer motif. In certain embodiments, short antisense compounds targeted to a GCCR nucleic acid comprise a gapmer motif. In certain embodiments, a short antisense compound targeted to a GCCR nucleic acid comprises a 2-10-2 gapmer motif.

Tables 12 and 13 illustrate examples of short antisense compounds targeted to SEQ ID NO: 9. Table 12 illustrates short antisense compounds that are 100% complementary to SEQ ID NO: 9. Table 13 illustrates short antisense compounds that have one or two mismatches with respect to SEQ ID NO: 9. The column labeled 'gapmer motif indicates the wing-gap-wing motif of each short antisense compounds. The gap segment comprises 2'-deoxynucleotides and each nucleotide of each wing segment comprises a 2'-modified sugar. The particular 2'-modified sugar is also indicated in the 'gapmer motif column. For example, '2-10-2 MOE' means a 2-10-2 gapmer motif, where a gap segment of ten 2'-deoxynucleotides is flanked by wing segments of two nucleotides, where the nucleotides of the wing segments are 2'-MOE nucleotides. Internucleoside linkages are phosphorothioate. The short antisense compounds comprise 5-methylcytidine in place of unmodified cytosine, unless "unmodified cytosine" is listed in the gapmer motif column, in which case the indicated cytosines are unmodified cytosines. For example, "5-mC in gap only" indicates that the gap segment has 5-methylcytosines, while the wing segments have unmodified cytosines.

**Table 12: Short Antisense Compounds targeted to SEQ ID NO: 9**

| **ISIS NO.** | **5' Target Site** | **3' Target Site** | **Sequence (5'-3')** | **Gapmer Motif** | **SEQ ID NO** |
|---|---|---|---|---|---|
| 338463 | 378 | 393 | TAGAGCTTCCACTTCT | 3-10-3 MOE | 486 |
| 338534 | 378 | 391 | GAGCTTCCACTTCT | 3-8-3 MOE | 487 |
| 327130 | 499 | 512 | TGTTGGCCGTGGTA | 3-8-3 MOE | 488 |
| 327131 | 500 | 513 | ATGTTGGCCGTGGT | 3-8-3 MOE | 489 |
| 327132 | 501 | 514 | GATGTTGGCCGTGG | 3-8-3 MOE | 490 |
| 327133 | 502 | 515 | AGATGTTGGCCGTG | 3-8-3 MOE | 491 |
| 327134 | 503 | 516 | GAGATGTTGGCCGT | 3-8-3 MOE | 492 |
| 327135 | 504 | 517 | GGAGATGTTGGCCG | 3-8-3 MOE | 493 |
| 327136 | 505 | 518 | AGGAGATGTTGGCC | 3-8-3 MOE | 494 |
| 327137 | 506 | 519 | CAGGAGATGTTGGC | 3-8-3 MOE | 495 |
| 327138 | 507 | 520 | GCAGGAGATGTTGG | 3-8-3 MOE | 496 |
| 327139 | 508 | 521 | GGCAGGAGATGTTG | 3-8-3 MOE | 497 |
| 327140 | 531 | 544 | GTGGTGCCAAGGCA | 3-8-3 MOE | 498 |
| 327141 | 532 | 545 | TGTGGTGCCAAGGC | 3-8-3 MOE | 499 |
| 327142 | 533 | 546 | TTGTGGTGCCAAGG | 3-8-3 MOE | 500 |
| 327143 | 534 | 547 | TTTGTGGTGCCAAG | 3-8-3 MOE | 501 |
| 327144 | 535 | 548 | CTTTGTGGTGCCAA | 3-8-3 MOE | 502 |
| 327145 | 536 | 549 | ACTTTGTGGTGCCA | 3-8-3 MOE | 503 |
| 327146 | 537 | 550 | CACTTTGTGGTGCC | 3-8-3 MOE | 504 |
| 327147 | 538 | 551 | GCACTTTGTGGTGC | 3-8-3 MOE | 505 |
| 327148 | 539 | 552 | TGCACTTTGTGGTG | 3-8-3 MOE | 506 |
| 327149 | 540 | 553 | TTGCACTTTGTGGT | 3-8-3 MOE | 507 |
| 327150 | 545 | 558 | CGGTGTTGCACTTT | 3-8-3 MOE | 508 |
| 327151 | 546 | 559 | GCGGTGTTGCACTT | 3-8-3 MOE | 509 |
| 327152 | 547 | 560 | AGCGGTGTTGCACT | 3-8-3 MOE | 510 |
| 327153 | 548 | 561 | AAGCGGTGTTGCAC | 3-8-3 MOE | 511 |
| 327154 | 549 | 562 | GAAGCGGTGTTGCA | 3-8-3 MOE | 512 |
| 327155 | 550 | 563 | CGAAGCGGTGTTGC | 3-8-3 MOE | 513 |
| 327156 | 551 | 564 | ACGAAGCGGTGTTG | 3-8-3 MOE | 514 |
| 327157 | 552 | 565 | CACGAAGCGGTGTT | 3-8-3 MOE | 515 |
| 327158 | 553 | 566 | ACACGAAGCGGTGT | 3-8-3 MOE | 516 |
| 327159 | 554 | 567 | AACACGAAGCGGTG | 3-8-3 MOE | 517 |
| 345897 | 684 | 697 | GCTGCTGTACATCT | 2-10-2 MOE | 518 |
| 327160 | 684 | 697 | GCTGCTGTACATCT | 3-8-3 MOE | 518 |
| 327161 | 685 | 698 | AGCTGCTGTACATC | 3-8-3 MOE | 520 |
| 327162 | 686 | 699 | AAGCTGCTGTACAT | 3-8-3 MOE | 521 |
| 327163 | 687 | 700 | GAAGCTGCTGTACA | 3-8-3 MOE | 522 |
| 327164 | 688 | 701 | GGAAGCTGCTGTAC | 3-8-3 MOE | 523 |
| 327165 | 689 | 702 | TGGAAGCTGCTGTA | 3-8-3 MOE | 524 |
| 327166 | 690 | 703 | CTGGAAGCTGCTGT | 3-8-3 MOE | 525 |
| 327167 | 691 | 704 | CCTGGAAGCTGCTG | 3-8-3 MOE | 526 |
| 327168 | 692 | 705 | ACCTGGAAGCTGCT | 3-8-3 MOE | 527 |
| 327169 | 693 | 706 | CACCTGGAAGCTGC | 3-8-3 MOE | 528 |
| 327170 | 694 | 707 | TCACCTGGAAGCTG | 3-8-3 MOE | 529 |
| 327171 | 695 | 708 | ATCACCTGGAAGCT | 3-8-3 MOE | 530 |
| 327172 | 696 | 709 | CATCACCTGGAAGC | 3-8-3 MOE | 531 |
| 327173 | 697 | 710 | ACATCACCTGGAAG | 3-8-3 MOE | 532 |
| 327174 | 698 | 711 | TACATCACCTGGAA | 3-8-3 MOE | 533 |
| 327175 | 699 | 712 | GTACATCACCTGGA | 3-8-3 MOE | 534 |
| 327176 | 700 | 713 | TGTACATCACCTGG | 3-8-3 MOE | 535 |
| 327177 | 701 | 714 | GTGTACATCACCTG | 3-8-3 MOE | 536 |
| 327178 | 869 | 882 | TAGCGGGTCCTGAG | 3-8-3 MOE | 537 |
| 327179 | 870 | 883 | GTAGCGGGTCCTGA | 3-8-3 MOE | 538 |
| 327180 | 871 | 884 | TGTAGCGGGTCCTG | 3-8-3 MOE | 539 |
| 327181 | 872 | 885 | CTGTAGCGGGTCCT | 3-8-3 MOE | 540 |
| 327182 | 873 | 886 | GCTGTAGCGGGTCC | 3-8-3 MOE | 541 |
| 327183 | 874 | 887 | GGCTGTAGCGGGTC | 3-8-3 MOE | 542 |
| 327184 | 875 | 888 | TGGCTGTAGCGGGT | 3-8-3 MOE | 543 |
| 327185 | 876 | 889 | CTGGCTGTAGCGGG | 3-8-3 MOE | 544 |
| 327186 | 877 | 890 | TCTGGCTGTAGCGG | 3-8-3 MOE | 545 |
| 327187 | 878 | 891 | TTCTGGCTGTAGCG | 3-8-3 MOE | 546 |
| 327188 | 955 | 968 | TGAACACCGCGGCC | 3-8-3 MOE | 547 |
| 327189 | 956 | 969 | ATGAACACCGCGGC | 3-8-3 MOE | 548 |
| 327190 | 957 | 970 | CATGAACACCGCGG | 3-8-3 MOE | 549 |
| 327191 | 958 | 971 | GCATGAACACCGCG | 3-8-3 MOE | 550 |
| 327192 | 959 | 972 | TGCATGAACACCGC | 3-8-3 MOE | 551 |
| 327193 | 960 | 973 | TTGCATGAACACCG | 3-8-3 MOE | 552 |
| 327194 | 961 | 974 | ATTGCATGAACACC | 3-8-3 MOE | 553 |
| 327195 | 962 | 975 | TATTGCATGAACAC | 3-8-3 MOE | 554 |
| 327196 | 963 | 976 | ATATTGCATGAACA | 3-8-3 MOE | 555 |
| 327197 | 964 | 977 | CATATTGCATGAAC | 3-8-3 MOE | 556 |
| 327198 | 1019 | 1032 | AGGTTGTGCAGGTA | 3-8-3 MOE | 557 |
| 327199 | 1020 | 1033 | CAGGTTGTGCAGGT | 3-8-3 MOE | 558 |
| 327200 | 1021 | 1034 | GCAGGTTGTGCAGG | 3-8-3 MOE | 559 |
| 327201 | 1022 | 1035 | AGCAGGTTGTGCAG | 3-8-3 MOE | 560 |
| 327202 | 1023 | 1036 | CAGCAGGTTGTGCA | 3-8-3 MOE | 561 |
| 327203 | 1024 | 1037 | CCAGCAGGTTGTGC | 3-8-3 MOE | 562 |
| 327204 | 1025 | 1038 | CCCAGCAGGTTGTG | 3-8-3 MOE | 563 |
| 327205 | 1026 | 1039 | GCCCAGCAGGTTGT | 3-8-3 MOE | 564 |
| 327206 | 1027 | 1040 | GGCCCAGCAGGTTG | 3-8-3 MOE | 565 |
| 327207 | 1028 | 1041 | AGGCCCAGCAGGTT | 3-8-3 MOE | 566 |
| 338491 | 1160 | 1175 | TGTCATTGCTGGTCCA | 3-10-3 MOE | 567 |
| 338562 | 1160 | 1173 | TCATTGCTGGTCCA | 3-8-3 MOE | 568 |
| 338498 | 1307 | 1322 | TGGCCAGCCGGAACTT | 3-10-3 MOE | 569 |
| 338569 | 1307 | 1320 | GCCAGCCGGAACTT | 3-8-3 MOE | 570 |
| 338499 | 1329 | 1344 | GGGATGAGGGTCAGCG | 3-10-3 MOE | 571 |
| 338570 | 1329 | 1342 | GATGAGGGTCAGCG | 3-8-3 MOE | 572 |
| 385067 | 1364 | 1377 | AAGGCAAAGACCAC | 3-8-3 MOE | 573 |
| 338573 | 1401 | 1414 | GGAGCGCAGGGTGC | 3-8-3 MOE | 574 |
| 338580 | 1487 | 1500 | TGCACCTCCTTGTT | 3-8-3 MOE | 575 |

**Table 13: Short antisense compounds targeted to SEQ ID NO: 1 and having 1 or 2 mismatches**

| **ISIS NO.** | **5' Target Site** | **3' Target Site** | **Sequence (5'-3')** | **Gapmer Motif** | **SEQ ID NO** |
|---|---|---|---|---|---|
| 338577 | 158 | 171 | CAGCAGACCCTGGA | 3-8-3 MOE | 576 |
| 338458 | 237 | 252 | ACATCTGGCAGAGGTT | 3-10-3 MOE | 577 |
| 338529 | 237 | 250 | ATCTGGCAGAGGTT | 3-8-3 MOE | 578 |
| 338466 | 318 | 333 | CAGGCCAGCAGGAGTA | 3-10-3 MOE | 579 |
| 338537 | 318 | 331 | GGCCAGCAGGAGTA | 3-8-3 MOE | 580 |
| 338533 | 364 | 377 | CAAACAAAAAGTCC | 3-8-3 MOE | 582 |
| 338462 | 364 | 379 | CTCAAACAAAAAGTCC | 3-10-3 MOE | 581 |
| 338535 | 397 | 410 | GGTGACATTGGTCA | 3-8-3 MOE | 584 |
| 338464 | 397 | 412 | GTGGTGACATTGGTCA | 3-10-3 MOE | 583 |
| 338466 | 470 | 485 | CAGGCCAGCAGGAGTA | 3-10-3 MOE | 579 |
| 338537 | 470 | 483 | GGCCAGCAGGAGTA | 3-8-3 MOE | 580 |
| 385048 | 497 | 510 | TTGGCAGTGGTGTT | 3-8-3 MOE | 587 |
| 385049 | 500 | 513 | ATGTTGGCAGTGGT | 3-8-3 MOE | 588 |
| 338467 | 503 | 518 | AGGAAATGTTGGCAGT | 3-10-3 MOE | 589 |
| 338538 | 503 | 516 | GAAATGTTGGCAGT | 3-8-3 MOE | 590 |
| 385050 | 506 | 519 | CAGGAAATGTTGGC | 3-8-3 MOE | 591 |
| 385051 | 509 | 522 | GGGCAGGAAATGTT | 3-8-3 MOE | 592 |
| 385052 | 523 | 536 | AAGGTAGGTACCAG | 3-8-3 MOE | 593 |
| 385053 | 526 | 539 | ACCAAGGTAGGTAC | 3-8-3 MOE | 594 |
| 385056 | 535 | 548 | CTTTGTGGCACCAA | 3-8-3 MOE | 595 |
| 385057 | 538 | 551 | GCACTTTGTGGCAC | 3-8-3 MOE | 596 |
| 338539 | 539 | 552 | TGCACTTTGTGGCA | 3-8-3 MOE | 597 |
| 385058 | 541 | 554 | GCTGCACTTTGTGG | 3-8-3 MOE | 598 |
| 385059 | 544 | 557 | GGTGCTGCACTTTG | 3-8-3 MOE | 599 |
| 385060 | 547 | 560 | GGCGGTGCTGCACT | 3-8-3 MOE | 600 |
| 385063 | 556 | 569 | TGAACACTAGGCGG | 3-8-3 MOE | 601 |
| 385064 | 559 | 572 | TCTTGAACACTAGG | 3-8-3 MOE | 602 |
| 338469 | 561 | 576 | CACCTCTTGAACACTA | 3-10-3 MOE | 603 |
| 338540 | 561 | 574 | CCTCTTGAACACTA | 3-8-3 MOE | 604 |
| 385065 | 562 | 575 | ACCTCTTGAACACT | 3-8-3 MOE | 605 |
| 385066 | 565 | 578 | CACACCTCTTGAAC | 3-8-3 MOE | 606 |
| 338541 | 590 | 603 | CCTCGAACCCACTG | 3-8-3 MOE | 607 |
| 338473 | 658 | 673 | CTTCTGGACCTCGATC | 3-10-3 MOE | 608 |
| 338544 | 658 | 671 | TCTGGACCTCGATC | 3-8-3 MOE | 609 |
| 338474 | 681 | 696 | CTGCTATACATCTTGG | 3-10-3 MOE | 610 |
| 338545 | 681 | 694 | GCTATACATCTTGG | 3-8-3 MOE | 611 |
| 338475 | 703 | 718 | CACGGTGTACATCACC | 3-10-3 MOE | 612 |
| 338546 | 703 | 716 | CGGTGTACATCACC | 3-8-3 MOE | 613 |
| 338547 | 718 | 731 | ACAGACTGTAGCCC | 3-8-3 MOE | 615 |
| 338476 | 718 | 733 | GGACAGACTGTAGCCC | 3-10-3 MOE | 614 |
| 338550 | 889 | 902 | CATCGCCAATCTTC | 3-8-3 MOE | 617 |
| 338479 | 889 | 904 | GTCATCGCCAATCTTC | 3-10-3 MOE | 616 |
| 338551 | 899 | 912 | ACACTGAGGTCATC | 3-8-3 MOE | 619 |
| 338480 | 899 | 914 | TCACACTGAGGTCATC | 3-10-3 MOE | 618 |
| 338552 | 924 | 937 | CGCCCCGTCACTGA | 3-8-3 MOE | 620 |
| 338555 | 992 | 1005 | AGCAACCAGCAATA | 3-8-3 MOE | 622 |
| 338484 | 992 | 1007 | CCAGCAACCAGCAATA | 3-10-3 MOE | 621 |
| 338485 | 1018 | 1033 | CAGGCTGTACAGGTAC | 3-10-3 MOE | 623 |
| 338556 | 1018 | 1031 | GGCTGTACAGGTAC | 3-8-3 MOE | 624 |
| 338558 | 1051 | 1064 | AGCTCCTCTCAGAG | 3-8-3 MOE | 626 |
| 338487 | 1051 | 1066 | GAAGCTCCTCTCAGAG | 3-10-3 MOE | 625 |
| 338559 | 1079 | 1092 | CAGCCAATGCCCAG | 3-8-3 MOE | 628 |
| 338488 | 1079 | 1094 | CCCAGCCAATGCCCAG | 3-10-3 MOE | 627 |
| 338560 | 1131 | 1144 | AAACAGACACTTGA | 3-8-3 MOE | 630 |
| 338489 | 1131 | 1146 | TCAAACAGACACTTGA | 3-10-3 MOE | 629 |
| 338490 | 1145 | 1160 | AGCACTGAACATTCTC | 3-10-3 MOE | 631 |
| 338561 | 1145 | 1158 | CACTGAACATTCTC | 3-8-3 MOE | 632 |
| 338563 | 1181 | 1194 | ATCCACCAGAATCC | 3-8-3 MOE | 634 |
| 338492 | 1181 | 1196 | GGATCCACCAGAATCC | 3-10-3 MOE | 633 |
| 338564 | 1216 | 1229 | TGATCAGTAAGGCC | 3-8-3 MOE | 635 |
| 338565 | 1232 | 1245 | ACAAAGATGAAAAA | 3-8-3 MOE | 637 |
| 338494 | 1232 | 1247 | GGACAAAGATGAAAAA | 3-10-3 MOE | 636 |
| 338566 | 1267 | 1280 | CACGCAGCTTGGCC | 3-8-3 MOE | 639 |
| 338495 | 1267 | 1282 | GGCACGCAGCTTGGCC | 3-10-3 MOE | 638 |
| 338571 | 1344 | 1357 | GACCCCCAGCAGAG | 3-8-3 MOE | 641 |
| 338500 | 1344 | 1359 | TGGACCCCCAGCAGAG | 3-10-3 MOE | 640 |
| 385068 | 1366 | 1379 | CAAAGGCAAAGACC | 3-8-3 MOE | 642 |
| 385069 | 1369 | 1382 | TCACAAAGGCAAAG | 3-8-3 MOE | 643 |
| 385070 | 1372 | 1385 | CAGTCACAAAGGCA | 3-8-3 MOE | 644 |
| 385071 | 1375 | 1388 | CGTCAGTCACAAAG | 3-8-3 MOE | 645 |
| 385072 | 1378 | 1391 | GCTCGTCAGTCACA | 3-8-3 MOE | 646 |
| 385073 | 1381 | 1394 | CATGCTCGTCAGTC | 3-8-3 MOE | 647 |
| 386608 | 1384 | 1397 | GGGCATGCTCGTCA | 1-12-1 MOE | 648 |
| 386593 | 1384 | 1397 | GGGCATGCTCGTCA | 2-10-2 MOE | 648 |
| 396146 | 1384 | 1397 | GGGCATGCTCGTCA | 2-10-2 MOE | 648 |
| 338572 | 1384 | 1397 | GGGCATGCTCGTCA | 3-8-3 MOE | 648 |
| 396149 | 1384 | 1397 | GGGCATGCTCGTCA | 1-1-10-2 2'-(butylacetamido)-palmitamide/OMe/ OMe | 648 |
| 386627 | 1384 | 1397 | GGGCATGCTCGTCA | 2-10-2 Methyleneoxy BNA | 648 |
| 386610 | 1387 | 1400 | CTTGGGCATGCTCG | 1-12-1 MOE | 654 |
| 386595 | 1387 | 1400 | CTTGGGCATGCTCG | 2-10-2 MOE | 654 |
| 385074 | 1387 | 1400 | CTTGGGCATGCTCG | 3-8-3 MOE | 654 |
| 385075 | 1390 | 1403 | TGCCTTGGGCATGC | 3-8-3 MOE | 657 |
| 385076 | 1393 | 1406 | GGGTGCCTTGGGCA | 3-8-3 MOE | 648 |
| 385077 | 1396 | 1409 | GCAGGGTGCCTTGG | 3-8-3 MOE | 659 |
| 385078 | 1399 | 1412 | AGCGCAGGGTGCCT | 3-8-3 MOE | 660 |
| 338502 | 1401 | 1416 | GTGGAGCGCAGGGTGC | 3-10-3 MOE | 661 |
| 385079 | 1402 | 1415 | TGGAGCGCAGGGTG | 3-8-3 MOE | 662 |
| 385080 | 1405 | 1418 | TGGTGGAGCGCAGG | 3-8-3 MOE | 663 |
| 385081 | 1408 | 1421 | GCTTGGTGGAGCGC | 3-8-3 MOE | 664 |
| 385082 | 1411 | 1424 | AGAGCTTGGTGGAG | 3-8-3 MOE | 665 |
| 338503 | 1412 | 1427 | AAAAGAGCTTGGTGGA | 3-10-3 MOE | 666 |
| 338574 | 1412 | 1425 | AAGAGCTTGGTGGA | 3-8-3 MOE | 667 |
| 385083 | 1414 | 1427 | AAAAGAGCTTGGTG | 3-8-3 MOE | 668 |
| 385084 | 1417 | 1430 | CAAAAAAGAGCTTG | 3-8-3 MOE | 669 |
| 338504 | 1434 | 1449 | AAGGAGCTGAGGAACA | 3-10-3 MOE | 670 |
| 338575 | 1434 | 1447 | GGAGCTGAGGAACA | 3-8-3 MOE | 671 |
| 327167 | 1441 | 1454 | CCTGGAAGCTGCTG | 3-8-3 MOE | 526 |
| 338576 | 1445 | 1458 | AGACCCTGGAAGGA | 3-8-3 MOE | 673 |
| 338505 | 1445 | 1460 | GCAGACCCTGGAAGGA | 3-10-3 MOE | 672 |
| 338506 | 1449 | 1464 | ACCAGCAGACCCTGGA | 3-10-3 MOE | 674 |
| 338577 | 1449 | 1462 | CAGCAGACCCTGGA | 3-8-3 MOE | 576 |
| 338507 | 1464 | 1479 | CAGTAGAGAACAGCCA | 3-10-3 MOE | 676 |
| 338578 | 1464 | 1477 | GTAGAGAACAGCCA | 3-8-3 MOE | 677 |
| 338508 | 1475 | 1490 | TGTTGAGGAAACAGTA | 3-10-3 MOE | 678 |
| 338579 | 1475 | 1488 | TTGAGGAAACAGTA | 3-8-3 MOE | 679 |
| 338509 | 1487 | 1502 | CCTGCACCTCCTTGTT | 3-10-3 MOE | 680 |
| 338580 | 1610 | 1623 | TGCACCTCCTTGTT | 3-8-3 MOE | 575 |

In certain embodiments, a target region is nucleotides 378-391 of SEQ ID NO: 9. In certain embodiments, a short antisense compound is targeted to nucleotides 378-391 of SEQ ID NO: 9. In certain such embodiments, a short antisense compound targeted to nucleotides 378-391 comprises a nucleotide sequence selected from SEQ ID NO 486 or 487. In certain such embodiments, a short antisense compound targeted to nucleotides 378-391 of SEQ ID NO: 9 is selected from Isis No 338463 or 338534.

In certain embodiments, a target region is nucleotides 499-521 of SEQ ID NO: 9. In certain embodiments, a short antisense compound is targeted to nucleotides 499-521 of SEQ ID NO: 9. In certain such embodiments, a short antisense compound targeted to nucleotides 499-521 comprises a nucleotide sequence selected from SEQ ID NO 488, 489, 490, 491, 492, 493, 494, 495, 496, or 497. In certain such embodiments, a short antisense compound targeted to nucleotides 499-521 of SEQ ID NO: 9 is selected from Isis No 327130, 327131, 327132, 327133, 327134, 327135, 327136, 327137, 327138, or 327139.

In certain embodiments, a target region is nucleotides 531-553 of SEQ ID NO: 9. In certain embodiments, a short antisense compound is targeted to nucleotides 531-553 of SEQ ID NO: 9. In certain such embodiments, a short antisense compound targeted to nucleotides 531-553 comprises a nucleotide sequence selected from SEQ ID NO 498, 499, 500, 501, 502, 503, 504, 505, 506, or 507. In certain such embodiments, a short antisense compound targeted to nucleotides 531-553 of SEQ ID NO: 9 is selected from Isis No 327140, 327141, 327142, 327143, 327144, 327145, 327146, 327147, 327148, or 327149.

In certain embodiments, a target region is nucleotides 545-567 of SEQ ID NO: 9. In certain embodiments, a short antisense compound is targeted to nucleotides 545-567 of SEQ ID NO: 9. In certain such embodiments, a short antisense compound targeted to nucleotides 545-567 comprises a nucleotide sequence selected from SEQ ID NO 508, 509, 510, 511, 512, 513, 514, 515, 516, or 517. In certain such embodiments, a short antisense compound targeted to nucleotides 545-567 of SEQ ID NO: 9 is selected from Isis No 327150, 327151, 327152, 327153, 327154, 327155, 327156, 327157, 327158, or 327159.

In certain embodiments, a target region is nucleotides 531-567 of SEQ ID NO: 9. In certain embodiments, a short antisense compound is targeted to nucleotides 531-567 of SEQ ID NO: 9. In certain such embodiments, a short antisense compound targeted to nucleotides 531-567 comprises a nucleotide sequence selected from SEQ ID NO 498, 499, 500, 501, 502, 503, 504, 505, 506, 507, 508, 509, 510, 511, 512, 513, 514, 515, 516, or 517. In certain such embodiments, a short antisense compound targeted to nucleotides 531-567 of SEQ ID NO: 9 is selected from Isis No 327140, 327141, 327142, 327143, 327144, 327145, 327146, 327147, 327148, 327149, 327150, 327151, 327152, 327153, 327154, 327155, 327156, 327157, 327158, or 327159.

In certain embodiments, a target region is nucleotides 684-714 of SEQ ID NO: 9. In certain embodiments, a short antisense compound is targeted to nucleotides 684-714 of SEQ ID NO: 9. In certain such embodiments, a short antisense compound targeted to nucleotides 684-714 comprises a nucleotide sequence selected from SEQ ID NO 518, 520, 521, 522, 523, 524, 525, 526, 527, 528, 529, 530, 531, 532, 533, 534, 535, or 536. In certain such embodiments, a short antisense compound targeted to nucleotides 684-714 of SEQ ID NO: 9 is selected from Isis No 345897, 327160, 327161, 327162, 327163, 327164, 327165, 327166, 327167, 327168, 327169, 327170, 327171, 327172, 327173, 327174, 327175, 327176, or 327177.

In certain embodiments, a target region is nucleotides 869-891 of SEQ ID NO: 9. In certain embodiments, a short antisense compound is targeted to nucleotides 869-891 of SEQ ID NO: 9. In certain such embodiments, a short antisense compound targeted to nucleotides 869-891 comprises a nucleotide sequence selected from SEQ ID NO 537, 538, 539, 540, 541, 542, 543, 544, 545, or 546. In certain such embodiments, a short antisense compound targeted to nucleotides 869-891 of SEQ ID NO: 9 is selected from Isis No 327178, 327179, 327180, 327181, 327182, 327183, 327184, 327185, 327186, or 327187.

In certain embodiments, a target region is nucleotides 955-977 of SEQ ID NO: 9. In certain embodiments, a short antisense compound is targeted to nucleotides 955-977 of SEQ ID NO: 9. In certain such embodiments, a short antisense compound targeted to nucleotides 955-977 comprises a nucleotide sequence selected from SEQ ID NO 547, 548, 549, 550, 551, 552, 553, 554, 555, or 556. In certain such embodiments, a short antisense compound targeted to nucleotides 955-977 of SEQ ID NO: 9 is selected from Isis No 327188, 327189, 327190, 327191, 327192, 327193, 327194, 327195, 327196, or 327197.

In certain embodiments, a target region is nucleotides 1019-1041 of SEQ ID NO: 9. In certain embodiments, a short antisense compound is targeted to nucleotides 1019-1041 of SEQ ID NO: 9. In certain such embodiments, a short antisense compound targeted to nucleotides 1019-1041 comprises a nucleotide sequence selected from SEQ ID NO 557, 558, 559, 560, 561, 562, 563, 564, 565, or 566. In certain such embodiments, a short antisense compound targeted to nucleotides 1019-1041 of SEQ ID NO: 9 is selected from Isis No 327198, 327199, 327200, 327201, 327202, 327203, 327204, 327205, 327206, or 327207.

In certain embodiments, a target region is nucleotides 1160-1175 of SEQ ID NO: 9. In certain embodiments, a short antisense compound is targeted to nucleotides 1160-1175 of SEQ ID NO: 9. In certain such embodiments, a short antisense compound targeted to nucleotides 1160-1175 comprises a nucleotide sequence selected from SEQ ID NO 567 or 568. In certain such embodiments, a short antisense compound targeted to nucleotides 1160-1175 of SEQ ID NO: 9 is selected from Isis No 338491 or 338562.

In certain embodiments, a target region is nucleotides 1307-1377 of SEQ ID NO: 9. In certain embodiments, a short antisense compound is targeted to nucleotides 1307-1377 of SEQ ID NO: 9. In certain such embodiments, a short antisense compound targeted to nucleotides 1307-1377 comprises a nucleotide sequence selected from SEQ ID NO 569, 570, 571, 572, or 573. In certain such embodiments, a short antisense compound targeted to nucleotides 1307-1377 of SEQ ID NO: 9 is selected from Isis No 338498, 338569, 338499, 338570, or 385067.

In certain embodiments, a target region is nucleotides 1307-1414 of SEQ ID NO: 9. In certain embodiments, a short antisense compound is targeted to nucleotides 1307-1414 of SEQ ID NO: 9. In certain such embodiments, a short antisense compound targeted to nucleotides 1307-1414 comprises a nucleotide sequence selected from SEQ ID NO 569, 570, 571, 572, 573, or 574. In certain such embodiments, a short antisense compound targeted to nucleotides 1307-1414 of SEQ ID NO: 9 is selected from Isis No 338498, 338569, 338499, 338570, 385067, or 338573.

In certain embodiments, short antisense compounds targeted to a GCGR nucleic acid are 8 to 16, preferably 9 to 15, more preferably 9 to 14, more preferably 10 to 14 nucleotides in length. In certain embodiments, short antisense compounds targeted to a GCGR nucleic acid are 9 to 14 nucleotides in length. In certain embodiments, short antisense compounds targeted to a GCGR nucleic acid are 10 to 14 nucleotides in length. In certain embodiments, such short antisense compounds are short antisense oligonucleotides.

In certain embodiments, short antisense compounds targeted to a GCGR nucleic acid are short gapmers. In certain such embodiments, short gapmers targeted to a GCGR nucleic acid comprise at least one high affinity modification in one or more wings of the compound. In certain embodiments, short antisense compounds targeted to a GCGR nucleic acid comprise 1 to 3 high-affinity modifications in each wing. In certain such embodiments, the nucleosides or nucleotides of the wing comprise a 2' modification. In certain such embodiments, the monomers of the wing are BNA's. In certain such embodiments, the monomers of the wing are selected from α-L-Methyleneoxy (4'-CHD₂-O-2') BNA , β-D-Methyleneoxy (4'-CH₂-O-2') BNA , Ethyleneoxy (4'-(CH₂)₂-O-2') BNA , Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, - OCF₃, O-(CH₂)₂-O-CH₃, 2'-O(CH₂)₂SCH₃, O-(CH₂)₂-O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, short antisense compounds targeted to a GCGR nucleic acid comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, short antisense compounds targeted to a GCGR nucleic acid have uniform monomeric linkages. In certain such embodiments, those linkages are all phosphorothioate linkages. In certain embodiments, the linkages are all phosphodiester linkages. In certain embodiments, short antisense compounds targeted to a GCGR nucleic acid have mixed backbones.

In certain embodiments, short antisense compounds targeted to a GCGR nucleic acid are 8 monomers in length. In certain embodiments, short antisense compounds targeted to a GCGR nucleic acid are 9 monomers in length. In certain embodiments, short antisense compounds targeted to a GCGR nucleic acid are 10 monomers in length. In certain embodiments, short antisense compounds targeted to a GCGR nucleic acid are 11 monomers in length. In certain embodiments, short antisense compounds targeted to a GCGR nucleic acid are monomers in length. In certain embodiments, short antisense compounds targeted to a GCGR nucleic acid are 13 monomers in length. In certain embodiments, short antisense compounds targeted to a GCGR nucleic acid are 14 monomers in length. In certain embodiments, short antisense compounds targeted to a GCGR nucleic acid are 15 monomers in length. In certain embodiments, short antisense compounds targeted to a GCGR nucleic acid are 16 monomers in length. In certain embodiments, short antisense compounds targeted to a GCGR nucleic acid comprise 9 to 15 monomers. In certain embodiments, short antisense compounds targeted to a GCGR nucleic acid comprise 10 to 15 monomers. In certain embodiments, short antisense compounds targeted to a GCGR nucleic acid comprise 12 to 14 monomers. In certain embodiments, short antisense compounds targeted to a GCGR nucleic acid comprise 12 to 14 nucleotides or nucleosides.

In certain embodiments, the invention provides methods of modulating expression of GCGR. In certain embodiments, such methods comprise use of one or more short antisense compound targeted to a GCGR nucleic acid, wherein the short antisense compound targeted to a GCGR nucleic acid is from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of GCGR using one or more short antisense compounds targeted to a GCGR nucleic acid of 8, 9, 10, 11, 12, 13, 14, 15 or 16 monomers.

In certain embodiments, methods of modulating GCGR comprise use of a short antisense compound targeted to a GCGR nucleic acid that is 8 monomers in length. In certain embodiments, methods of modulating GCGR comprise use of a short antisense compound targeted to a GCGR nucleic acid that is 9 monomers in length. In certain embodiments, methods of modulating GCGR comprise use of a short antisense compound targeted to a GCGR nucleic acid that is 10 monomers in length. In certain embodiments, methods of modulating GCGR comprise use of a short antisense compound targeted to a GCGR nucleic acid that is 11 monomers in length. In certain embodiments, methods of modulating GCGR comprise use of a short antisense compound targeted to a GCGR nucleic acid that is 12 monomers in length. In certain embodiments, methods of modulating GCGR comprise use of a short antisense compound targeted to a GCGR nucleic acid that is 13 monomers in length. In certain embodiments, methods of modulating GCGR comprise use of a short antisense compound targeted to a GCGR nucleic acid that is 14 monomers in length. In certain embodiments, methods of modulating GCGR comprise use of a short antisense compound targeted to a GCGR nucleic acid that is 15 monomers in length. In certain embodiments, methods of modulating GCGR comprise use of a short antisense compound targeted to a GCGR nucleic acid that is 16 monomers in length.

In certain embodiments, methods of modulating expression of GCGR comprise use of a short antisense compound targeted to a GCGR nucleic acid comprising 9 to 15 monomers. In certain embodiments, methods of modulating expression of GCGR comprise use of a short antisense compound targeted to a GCGR nucleic acid comprising 10 to 15 monomers. In certain embodiments, methods of modulating expression of GCGR comprise use of a short antisense compound targeted to a GCGR nucleic acid comprising 12 to 14 monomers. In certain embodiments, methods of modulating expression of GCGR comprise use of a short antisense compound targeted to a GCGR nucleic acid comprising 12 or 14 nucleotides or nucleosides.

### Salts, prodrugs and bioequivalents

The antisense compounds provided herein comprise any pharmaceutically acceptable salts, esters, or salts of such esters, or any other functional chemical equivalent which, upon administration to an animal including a human, is capable of providing (directly or indirectly) the biologically active metabolite or residue thereof. Accordingly, for example, the disclosure is also drawn to prodrugs and pharmaceutically acceptable salts of the antisense compounds, pharmaceutically acceptable salts of such prodrugs, and other bioequivalents.

The term "prodrug" indicates a therapeutic agent that is prepared in an inactive or less active form that is converted to an active form (i.e., drug) within the body or cells thereof by the action of endogenous enzymes, chemicals, and/or conditions. In particular, prodrug versions of the oligonucleotides are prepared as SATE ((S-acetyl-2-thioethyl) phosphate) derivatives according to the methods disclosed in WO 93/24510 or WO 94/26764. Prodrugs can also include antisense compounds wherein one or both ends comprise nucleobases that are cleaved (e.g., by incorporating phosphodiester backbone linkages at the ends) to produce the active compound. In certain embodiments, one or more non-drug moieties is cleaved from a prodrug to yield the active form. In certain such embodiments, such non-drug moieties is not a nucleotide or oligonucleotide.

The term "pharmaceutically acceptable salts" refers to physiologically and pharmaceutically acceptable salts of the compounds described herein: i.e., salts that retain the desired biological activity of the parent compound and do not impart undesired toxicological effects thereto. Sodium salts of antisense oligonucleotides are useful and are well accepted for therapeutic administration to humans.

In certain embodiments, salts, including, but not limited to sodium salts, of double stranded nucleic acids (including but not limited to dsRNA compounds) are also provided.

### G. Certain Pharmaceutical Compositions

In certain embodiments, pharmaceutical compositions of the present invention comprise one or more short antisense compound and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition of the present invention is prepared using known techniques, including, but not limited to mixing, dissolving, granulating, dragee-making, levigating, emulsifying, encapsulating, entrapping or tabletting processes.

In certain embodiments, a pharmaceutical composition of the present invention is a liquid (e.g., a suspension, elixir and/or solution). In certain of such embodiments, a liquid pharmaceutical composition is prepared using ingredients known in the art, including, but not limited to, water, glycols, oils, alcohols, flavoring agents, preservatives, and coloring agents.

In certain embodiments, a pharmaceutical composition of the present invention is a solid (e.g., a powder, tablet, and/or capsule). In certain of such embodiments, a solid pharmaceutical composition comprising one or more oligonucleotides is prepared using ingredients known in the art, including, but not limited to, starches, sugars, diluents, granulating agents, lubricants, binders, and disintegrating agents.

In certain embodiments, a pharmaceutical composition of the present invention is formulated as a depot preparation. Certain such depot preparations are typically longer acting than non-depot preparations. In certain embodiments, such preparations are administered by implantation (for example subcutaneously or intramuscularly) or by intramuscular injection. In certain embodiments, depot preparations are prepared using suitable polymeric or hydrophobic materials (for example an emulsion in an acceptable oil) or ion exchange resins, or as sparingly soluble derivatives, for example, as a sparingly soluble salt.

In certain embodiments, a pharmaceutical composition of the present invention comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition of the present invention comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition of the present invention comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80.TM., and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80.TM.; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition of the present invention comprises a sustained-release system. A non-limiting example of such a sustained-release system is a semi-permeable matrix of solid hydrophobic polymers. In certain embodiments, sustained-release systems may, depending on their chemical nature, release pharmaceutical agents over a period of hours, days, weeks or months.

In certain embodiments, a pharmaceutical composition of the present invention is prepared for oral administration. In certain of such embodiments, a pharmaceutical composition is formulated by combining one or more oligonucleotides with one or more pharmaceutically acceptable carriers. Certain of such carriers enable pharmaceutical compositions to be formulated as tablets, pills, dragees, capsules, liquids, gels, syrups, slurries, suspensions and the like, for oral ingestion by a subject. In certain embodiments, pharmaceutical compositions for oral use are obtained by mixing oligonucleotide and one or more solid excipient. Suitable excipients include, but are not limited to, fillers, such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). In certain embodiments, such a mixture is optionally ground and auxiliaries are optionally added. In certain embodiments, pharmaceutical compositions are formed to obtain tablets or dragee cores. In certain embodiments, disintegrating agents (e.g., cross-linked polyvinyl pyrrolidone, agar, or alginic acid or a salt thereof, such as sodium alginate) are added.

In certain embodiments, dragee cores are provided with coatings. In certain such embodiments, concentrated sugar solutions may be used, which may optionally comprise gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to tablets or dragee coatings.

In certain embodiments, pharmaceutical compositions for oral administration are push-fit capsules made of gelatin. Certain of such push-fit capsules comprise one or more pharmaceutical agents of the present invention in admixture with one or more filler such as lactose, binders such as starches, and/or lubricants such as talc or magnesium stearate and, optionally, stabilizers. In certain embodiments, pharmaceutical compositions for oral administration are soft, sealed capsules made of gelatin and a plasticizer, such as glycerol or sorbitol. In certain soft capsules, one or more pharmaceutical agents of the present invention are be dissolved or suspended in suitable liquids, such as fatty oils, liquid paraffin, or liquid polyethylene glycols. In addition, stabilizers may be added.

In certain embodiments, pharmaceutical compositions are prepared for buccal administration. Certain of such pharmaceutical compositions are tablets or lozenges formulated in conventional manner.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may comprise formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may comprise substances that increase the viscosity of the suspension, such as sodium carboxymethyl cellulose, sorbitol, or dextran. Optionally, such suspensions may also comprise suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition is prepared for administration by inhalation. Certain of such pharmaceutical compositions for inhalation are prepared in the form of an aerosol spray in a pressurized pack or a nebulizer. Certain of such pharmaceutical compositions comprise a propellant, e.g., dichlorodifluoromethane, trichlorofluoromethane, dichlorotetrafluoroethane, carbon dioxide or other suitable gas. In certain embodiments using a pressurized aerosol, the dosage unit may be determined with a valve that delivers a metered amount. In certain embodiments, capsules and cartridges for use in an inhaler or insufflator may be formulated. Certain of such formulations comprise a powder mixture of a pharmaceutical agent of the invention and a suitable powder base such as lactose or starch.

In certain embodiments, a pharmaceutical composition is prepared for rectal administration, such as a suppositories or retention enema. Certain of such pharmaceutical compositions comprise known ingredients, such as cocoa butter and/or other glycerides.

In certain embodiments, a pharmaceutical composition is prepared for topical administration. Certain of such pharmaceutical compositions comprise bland moisturizing bases, such as ointments or creams. Exemplary suitable ointment bases include, but are not limited to, petrolatum, petrolatum plus volatile silicones, lanolin and water in oil emulsions such as Eucerin.TM., available from Beiersdorf (Cincinnati, Ohio). Exemplary suitable cream bases include, but are not limited to, Nivea.TM. Cream, available from Beiersdorf (Cincinnati, Ohio), cold cream (USP), Purpose Cream.TM., available from Johnson & Johnson (New Brunswick, N.J.), hydrophilic ointment (USP) and Lubriderm.TM., available from Pfizer (Morris Plains, N.J.).

In certain embodiments, a pharmaceutical composition of the present invention comprises an oligonucleotide in a therapeutically effective amount. In certain embodiments, the therapeutically effective amount is sufficient to prevent, alleviate or ameliorate symptoms of a disease or to prolong the survival of the subject being treated. Determination of a therapeutically effective amount is well within the capability of those skilled in the art.

In certain embodiments, one or more short antisense compound of the present invention is formulated as a prodrug. In certain embodiments, upon in vivo administration, a prodrug is chemically converted to the biologically, pharmaceutically or therapeutically more active form of the short antisense compound. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, a prodrug is produced by modifying a pharmaceutically active compound such that the active compound will be regenerated upon in vivo administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see, e.g., Nogrady (1985) Medicinal Chemistry A Biochemical Approach, Oxford University Press, New York, pages 388-392).

In certain embodiments, a pharmaceutical composition comprising one or more pharmaceutical agents of the present invention is useful for treating a conditions or disorders in a mammalian, and particularly in a human, subject. Suitable administration routes include, but are not limited to, oral, rectal, transmucosal, intestinal, enteral, topical, suppository, through inhalation, intrathecal, intraventricular, intraperitoneal, intranasal, intraocular and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., in the renal or cardiac area).

In certain embodiments, short antisense compounds, compared to their parent oligonucleotides, make them particularly suited to oral administration. In certain embodiments, short antisense compounds are better suited for oral administration than their parent oligonucleotides because they have increased potency compared to those parent oligonucleotides. In certain embodiments, short antisense compounds are better suited for oral administration than their parent oligonucleotides because they have better stability, availability or solubility properties compared to those parent oligonucleotides.

In a further aspect, a pharmaceutical agent is sterile lyophilized oligonucleotide that is reconstituted with a suitable diluent, e.g., sterile water for injection. The reconstituted product is administered as a subcutaneous injection or as an intravenous infusion after dilution into saline. The lyophilized drug product consists of the oligonucleotide which has been prepared in water for injection, adjusted to pH 7.0-9.0 with acid or base during preparation, and then lyophilized. The lyophilized oligonucleotide may be 25-800 mg of the oligonucleotide. It is understood that this encompasses 25, 50, 75, 100, 125, 150, 175, 200, 225, 250, 275, 300, 325, 350, 375, 425, 450, 475, 500, 525, 550, 575, 600, 625, 650, 675, 700, 725, 750, 775, and 800 mg of lyophilized oligonucleotide. The lyophilized drug product may be packaged in a 2 mL Type I, clear glass vial (ammonium sulfate-treated), stoppered with a bromobutyl rubber closure and sealed with an aluminum FLIP-OFF® overseal.

The compositions of the present invention may additionally comprise other adjunct components conventionally found in pharmaceutical compositions, at their art-established usage levels. Thus, for example, the compositions may comprise additional, compatible, pharmaceutically-active materials such as, for example, antipruritics, astringents, local anesthetics or anti-inflammatory agents, or may comprise additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation.

The antisense compounds provided herein may also be admixed, encapsulated, conjugated or otherwise associated with other molecules, molecule structures or mixtures of compounds.

Also described herein are pharmaceutical compositions and formulations which include the antisense compounds provided herein. The pharmaceutical compositions may be administered in a number of ways depending upon whether local or systemic treatment is desired and upon the area to be treated. In a preferred embodiment, administration is topical to the surface of the respiratory tract, particularly pulmonary, e.g., by nebulization, inhalation, or insufflation of powders or aerosols, by mouth and/or nose.

The pharmaceutical formulations described herein, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers, finely divided solid carriers, or both, and then, if necessary, shaping the product (e.g., into a specific particle size for delivery). In a preferred embodiment, the pharmaceutical formulations are prepared for pulmonary administration in an appropriate solvent, e.g., water or normal saline, possibly in a sterile formulation, with carriers or other agents to allow for the formation of droplets of the desired diameter for delivery using inhalers, nasal delivery devices, nebulizers, and other devices for pulmonary delivery. Alternatively, the pharmaceutical formulations may be formulated as dry powders for use in dry powder inhalers.

A "pharmaceutical carrier" or "excipient" can be a pharmaceutically acceptable solvent, suspending agent or any other pharmacologically inert vehicle for delivering one or more nucleic acids to an individual and are known in the art. The excipient may be liquid or solid and is selected, with the planned manner of administration in mind, so as to provide for the desired bulk, consistency, etc., when combined with a nucleic acid and the other components of a given pharmaceutical composition.

### H. Certain Therapeutic Uses

In certain embodiments, antisense compounds are used to modulate the expression of a target gene in an animal, such as a human. In certain embodiments, such compounds can be used to treat metabolic disorders or modulate one or more disease indications. For example, the methods comprise the step of administering to said animal in need of therapy for a disease or condition associated with a target gene an effective amount of an antisense compound that modulates expression of the target gene. Antisense compounds provided herein which effectively modulate expression of a target RNA or protein products of expression are considered active antisense compounds. Active antisense compounds also include compounds which effectively modulate one or more of a number of disease indications, including metabolic and cardiovascular disease indications, examples of which are described below.

Modulation of expression of a target gene can be measured in a bodily fluid, which may or may not contain cells; tissue; or organ of the animal. Methods of obtaining samples for analysis, such as body fluids (e.g., sputum, serum, urine), tissues (e.g., biopsy), or organs, and methods of preparation of the samples to allow for analysis are well known to those skilled in the art. Methods for analysis of RNA and protein levels are discussed above and are well known to those skilled in the art. The effects of treatment can be assessed by measuring biomarkers, or disease indications, associated with the target gene expression in the aforementioned fluids, tissues or organs, collected from an animal contacted with one or more compounds described herein, by routine clinical methods known in the art. These biomarkers include but are not limited to: liver transaminases, bilirubin, albumin, blood urea nitrogen, creatine and other markers of kidney and liver function; interleukins, tumor necrosis factors, intracellular adhesion molecules, C-reactive protein, chemokines, cytokines, and other markers of inflammation.

The antisense compounds provided herein can be utilized in pharmaceutical compositions by adding an effective amount of a compound to a suitable pharmaceutically acceptable diluent or carrier. Acceptable carriers and diluents are well known to those skilled in the art. Selection of a diluent or carrier is based on a number of factors, including, but not limited to, the solubility of the compound and the route of administration. Such considerations are well understood by those skilled in the art. In one aspect, the antisense compounds described herein inhibit expression of a target gene. The compounds can also be used in the manufacture of a medicament for the treatment of diseases and disorders related to a target gene.

Methods whereby bodily fluids, organs or tissues are contacted with an effective amount of one or more of the antisense compounds or compositions provided herein are also contemplated. Bodily fluids, organs or tissues can be contacted with one or more of the compounds resulting in modulation of target gene expression in the cells of bodily fluids, organs or tissues. An effective amount can be determined by monitoring the modulatory effect of the antisense compound or compounds or compositions on target nucleic acids or their products by methods routine to the skilled artisan.

### Co-administration

In certain embodiments, two or more antisense compounds are co-administered. In certain embodiments, pharmaceutical compositions include one or more antisense compounds, particularly oligonucleotides, targeted to a first nucleic acid and one or more antisense compounds targeted to a second nucleic acid target. One or more of those antisense compounds may be a short antisense compound. In certain embodiments, pharmaceutical compositions include two or more antisense compounds targeted to different regions of the same nucleic acid target. One or more of such antisense compounds may be a short antisense compound. Two or more combined compounds may be used together or sequentially.

In certain embodiments, one or more pharmaceutical compositions are co-administered with one or more other pharmaceutical agents. In certain embodiments, such one or more other pharmaceutical agents are designed to treat the same disease or condition as the one or more pharmaceutical compositions of the present invention. In certain embodiments, such one or more other pharmaceutical agents are designed to treat a different disease or condition as the one or more pharmaceutical compositions of the present invention. In certain embodiments, such one or more other pharmaceutical agents are designed to treat an undesired effect of one or more pharmaceutical compositions of the present invention. In certain embodiments, one or more pharmaceutical compositions of the present invention are co-administered with another pharmaceutical agent to treat an undesired effect of that other pharmaceutical agent. In certain embodiments, one or more pharmaceutical compositions of the present invention and one or more other pharmaceutical agents are administered at the same time. In certain embodiments, one or more pharmaceutical compositions of the present invention and one or more other pharmaceutical agents are administered at different times. In certain embodiments, one or more pharmaceutical compositions of the present invention and one or more other pharmaceutical agents are prepared together in a single formulation. In certain embodiments, one or more pharmaceutical compositions of the present invention and one or more other pharmaceutical agents are prepared separately.

In certain embodiments, pharmaceutical agents that may be co-administered with a pharmaceutical composition of the present invention include lipid-lowering agents. In certain such embodiments, pharmaceutical agents that may be co-administered with a pharmaceutical composition of the present invention include, but are not limited to atorvastatin, simvastatin, rosuvastatin, and ezetimibe. In certain such embodiments, the lipid-lowering agent is administered prior to administration of a pharmaceutical composition of the present invention. In certain such embodiments, the lipid-lowering agent is administered following administration of a pharmaceutical composition of the present invention. In certain such embodiments the lipid-lowering agent is administered at the same time as a pharmaceutical composition of the present invention. In certain such embodiments the dose of a co-administered lipid-lowering agent is the same as the dose that would be administered if the lipid-lowering agent was administered alone. In certain such embodiments the dose of a co-administered lipid-lowering agent is lower than the dose that would be administered if the lipid-lowering agent was administered alone. In certain such embodiments the dose of a co-administered lipid-lowering agent is greater than the dose that would be administered if the lipid-lowering agent was administered alone.

In certain embodiments, a co-administered lipid-lowering agent is a HMG-CoA reductase inhibitor. In certain such embodiments the HMG-CoA reductase inhibitor is a statin. In certain such embodiments the statin is selected from atorvastatin, simvastatin, pravastatin, fluvastatin, and rosuvastatin. In certain embodiments, a co-administered lipid-lowering agent is a cholesterol absorption inhibitor. In certain such embodiments, cholesterol absorption inhibitor is ezetimibe. In certain embodiments, a co-administered lipid-lowering agent is a co-formulated HMG-CoA reductase inhibitor and cholesterol absorption inhibitor. In certain such embodiments the co-formulated lipid-lowering agent is ezetimibe/simvastatin. In certain embodiments, a co-administered lipid-lowering agent is a microsomal triglyceride transfer protein inhibitor.

In certain embodiments, a co-administered pharmaceutical agent is a bile acid sequestrant. In certain such embodiments, the bile acid sequestrant is selected from cholestyramine, colestipol, and colesevelam.

In certain embodiments, a co-administered pharmaceutical agent is a nicotinic acid. In certain such embodiments, the nicotinic acid is selected from immediate release nicotinic acid, extended release nicotinic acid, and sustained release nicotinic acid.

In certain embodiments, a co-administered pharmaceutical agent is a fibric acid. In certain such embodiments, a fibric acid is selected from gemfibrozil, fenofibrate, clofibrate, bezafibrate, and ciprofibrate.

Further examples of pharmaceutical agents that may be co-administered with a pharmaceutical composition of the present invention include, but are not limited to, corticosteroids, including but not limited to prednisone; immunoglobulins, including, but not limited to intravenous immunoglobulin (IVIg); analgesics (e.g., acetaminophen); anti-inflammatory agents, including, but not limited to non-steroidal anti-inflammatory drugs (e.g., ibuprofen, COX-1 inhibitors, and COX-2, inhibitors); salicylates; antibiotics; antivirals; antifungal agents; antidiabetic agents (e.g., biguanides, glucosidase inhibitors, insulins, sulfonylureas, and thiazolidenediones); adrenergic modifiers; diuretics; hormones (e.g., anabolic steroids, androgen, estrogen, calcitonin, progestin, somatostan, and thyroid hormones); immunomodulators; muscle relaxants; antihistamines; osteoporosis agents (e.g., biphosphonates, calcitonin, and estrogens); prostaglandins, antineoplastic agents; psychotherapeutic agents; sedatives; poison oak or poison sumac products; antibodies; and vaccines.

In certain embodiments, the pharmaceutical compositions of the present invention may be administered in conjuction with a lipid-lowering therapy. In certain such embodiments, a lipid-lowering therapy is therapeutic lifestyle change. In certain such embodiments, a lipid-lowering therapy is LDL apheresis.

### I. Kits, Research Reagents and Diagnostics

The antisense compounds provided herein can be utilized for diagnostics, and as research reagents and kits. Furthermore, antisense compounds, which are able to inhibit gene expression or modulate gene expression with specificity, are often used by those of ordinary skill to elucidate the function of particular genes or to distinguish between functions of various members of a biological pathway.

For use in kits and diagnostics, the antisense compounds described herein, either alone or in combination with other compounds or therapeutics, can be used as tools in differential and/or combinatorial analyses to elucidate expression patterns of a portion or the entire complement of genes expressed within cells and tissues. Methods of gene expression analysis are well known to those skilled in the art.

### J. Certain Advantages of Short Antisense Compounds

In certain embodiments, short antisense compounds have advantages when compared to their parent oligonucleotides. For example, in certain embodiments, short antisense compounds have greater affinity for a target nucleic acid than their parent oligonucleotide. In certain embodiments, short antisense compounds have greater potency in vitro than their parent oligonucleotide. In certain such embodiments, that increased in vitro potency is not entirely explained by increased affinity. In certain embodiments, such increased in vitro potency may be attributable to increased ability of short antisense compounds to penetrate cells and/or increased ability to access target nucleic acids in a cell. In certain embodiments, short antisense compounds have greater potency in vivo than their parent oligonucleotides. In certain embodiments, such greater in vivo potency is not attributable to increased in vitro potency or increased affinity. In certain embodiments, short antisense compounds have even greater in vivo potency compared to their parent oligonucleotides than would be predicted based on in vitro potencies or on affinities. In certain embodiments, such increased in vivo potency may be attributable to increased bioavailability, better penetration into the cell, better access to target nucleic acid once in the cell, or other factors.

In certain embodiments, one would expect short antisense compounds to be less specific for their target nucleic acid compared to their parent oligonucleotides. In certain such embodiments, one would expect increased side-effects, including potential for toxic effects, from short antisense compounds. In certain embodiments, such additional side-effects are not observed. In certain embodiments, non-target nucleic acids to which a particular short antisense compound may bind are not available to the short antisense compound. In such embodiments, side-effects, including toxicity, are less problematic than would be predicted.

In certain embodiments, because they are smaller, short antisense compounds are less likely to bind proteins. In certain such embodiments, such less binding of proteins results in lower toxicity, since protein binding may have undesired consequences. In certain embodiments, such less binding of proteins results in greater potency, since it leaves more antisense compound available for therapeutic effect. In certain embodiments, less binding of proteins results in decreased drug-drug interaction toxicity.

### Nonlimiting disclosure and incorporation by reference

While certain compounds, compositions and methods described herein have been described with specificity in accordance with certain embodiments, the following examples serve only to illustrate the compounds described herein and are not intended to limit the same. Each of the references, GenBank accession numbers, and the like recited in the present application is incorporated herein by reference in its entirety.

### Example 1

### Cell culture and treatment with short antisense compounds

The effect of short antisense compounds on target nucleic acid expression can be tested in any one of a number of cultured or primary cell lines. Cells lines can be obtained from publicly available sources, such as the American Type Culture Collection (Manassas, VA). Cells are cultured according to methods well known to those of ordinary skill in the art.

When cells reached appropriate confluency, they were treated with oligonucleotide using LIPOFECTIN® as described. When cells reached 65-75% confluency, they were treated with oligonucleotide. Oligonucleotide was mixed with LIPOFECTIN® Invitrogen Life Technologies, Carlsbad, CA) in Opti-MEM®-1 reduced serum medium (Invitrogen Life Technologies, Carlsbad, CA) to achieve the desired concentration of oligonucleotide and a LIPOFECTIN® concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM®-1 and then treated with 130 µL of the transfection mixture. Cells grown in 24-well plates or other standard tissue culture plates were treated similarly, using appropriate volumes of medium and oligonucleotide. Cells were treated and data were obtained in duplicate or triplicate. After approximately 4-7 hours of treatment at 37°C, the medium containing the transfection mixture was replaced with fresh culture medium. Cells were harvested 16-24 hours after oligonucleotide treatment.

Control oligonucleotides are used to determine the optimal oligomeric compound concentration for a particular cell line. Furthermore, when oligomeric compounds are tested in oligomeric compound screening experiments or phenotypic assays, control oligonucleotides are tested in parallel.

The concentration of oligonucleotide used varies from cell line to cell line. To determine the optimal oligonucleotide concentration for a particular cell line, the cells are treated with a positive control oligonucleotide at a range of concentrations. The concentration of positive control oligonucleotide that results in 80% inhibition of the target mRNA is then utilized as the screening concentration for new oligonucleotides in subsequent experiments for that cell line. If 80% inhibition is not achieved, the lowest concentration of positive control oligonucleotide that results in 60% inhibition of the target mRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 nM to 40 nM when the antisense oligonucleotide is transfected by electroporation.

### Example 2: Real-time Quantitative PCR Analysis of Target mRNA Levels

Quantitation of target mRNA levels was accomplished by real-time quantitative PCR using the ABI PRISM® 7600, 7700, or 7900 Sequence Detection System (PE-Applied Biosystems, Foster City, CA) according to manufacturer's instructions.

Prior to quantitative PCR analysis, primer-probe sets specific to the target gene being measured were evaluated for their ability to be "multiplexed" with a GAPDH amplification reaction. After isolation the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MaCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR were normalized using either the expression level of GAPDH, a gene whose expression is constant, or by quantifying total RNA using RiboGreen® (Molecular Probes, Inc. Eugene, OR). GAPDH expression was quantified by RT, real-time PCR, by being run simultaneously with the target, multiplexing, or separately. Total RNA was quantified using RiboGreen® RNA quantification reagent (Molecular Probes, Inc. Eugene, OR).

170 µL of RiboGreen® working reagent (RiboGreen® reagent diluted 1:350 in 10mM Tris-HCl, 1 mM EDTA, pH 7.5) was pipetted into a 96-well plate containing 30 µL purified cellular RNA. The plate was read in a CytoFluor® 4000 (PE Applied Biosystems) with excitation at 485nm and emission at 530nm.

The GAPDH PCR probes have JOE covalently linked to the 5' end and TAMRA or MGB covalently linked to the 3' end, where JOE is the fluorescent reporter dye and TAMRA or MGB is the quencher dye. In some cell types, primers and probe designed to a GAPDH sequence from a different species are used to measure GAPDH expression. For example, a human GAPDH primer and probe set is used to measure GAPDH expression in monkey-derived cells and cell lines.

Probes and primers for use in real-time PCR were designed to hybridize to target nucleic acids using routine methods. For example, PrimerExpress® (Applied Biosystems, Foster City, CA) software is routinely used to design probes and primers for use in real-time PCR. Examples of primer and probe sequences and the target nucleic acids to which they hybridize are presented in Table 24. The target-specific PCR probes have FAM covalently linked to the 5' end and TAMRA or MGB covalently linked to the 3' end, where FAM is the fluorescent dye and TAMRA or MGB is the quencher dye.

**Table 24**

| **Target-specific primers and probes for use in real-time PCR** | | | | |
|---|---|---|---|---|
| **Target Name** | **Species** | **Sequence Description** | **Sequence (5' to 3')** | **SEQ ID NO** |
| ApoB | Mouse | Forward Primer | CGTGGGCTCCAGCATTCTA | 1524 |
| ApoB | Mouse | Reverse Primer | AGTCATTTCTGCCTTTGCGTC | 1525 |
| ApoB | Mouse | Probe | CCAATGGTCGGGCACTGCTCAA | 1526 |
| ApoB | Mouse | Forward Primer | GAAAATAGACTTCCTGAATAACTATGCATT | 1527 |
| ApoB | Mouse | Reverse Primer | ACTCGCTTGCCAGCTTGC | 1528 |
| ApoB | Mouse | Probe | TTTCTGAGTCCCCGTGCCCAACA | 1529 |
| GCGR | Mouse | Forward Primer | TGAGCCTTGCCACCTTCTCT | 1530 |
| GCGR | Mouse | Reverse Primer | GCGCACCCCAGCCAA | 1531 |
| GCGR | Mouse | Probe | AGAGGAGCTTCTTTTCCCTCTACCTGGGC | 1532 |
| GCGR | Mouse | Forward Primer | ATTTCCTGCCCCTGGTACCT | 1533 |
| GCGR | Mouse | Reverse Primer | CGGGCCCACACCTCTTG | 1534 |
| GCGR | Mouse | Probe | CCACAAAGTGCAGCACCGCCTAGTGT | 1535 |
| PTEN | Mouse | Forward Primer | GCCACAGGCTCCCAGACAT | 1536 |
| PTEN | Mouse | Reverse Primer | TCCATCCTCTTGATATCTCCTTTTG | 1537 |
| PTEN | Mouse | Probe | ACAGCCATCATCAAAGAGATCGTTAGCAGAA | 1538 |
| PTEN | Mouse | Forward Primer | ATGACAATCATGTTGCAGCAATTC | 1539 |
| PTEN | Mouse | Reverse Primer | CGATGCAATAAATATGCACAAATCA | 1540 |
| PTEN | Mouse | Probe | CTGTAAAGCTGGAAAGGGACGGACTGGT | 1541 |

### Example 3: Short Antisense Compounds Targeted to an ApoB nucleic acid and having 2'-MOE or methyleneoxy (4'-CH₂-O-2') BNA modifications

Six-week old male Balb/c mice (Jackson Laboratory, Bar Harbor, ME) were injected intraperitoneally (i.p.) with antisense compounds targeted to ApoB, at a frequency of twice per week for three weeks. Antisense compound doses included 2.4, 1.2, 0.6, 0.3 and 0.15 µmol/kg. For antisense compounds 14 nucleotides in length, these doses equate to approximately 12, 6, 3, 1.5 or 0.75 mg/kg, respectively. Shown in Table 25 are the sequences and motifs of the antisense compounds used in this study. The antisense compounds are either 20 or 14 nucleotides in length and have a central "gap" region consisting of ten 2'-deoxynucleotides flanked by wings having 2'-O-methoxyethyl (2'-MOE) or BNA modified "wings." For example, the 2-10-2 MOE gapmer motif indicates an antisense compound with a gap of ten nucleotides flanked by 2 nucleotide wings with 2'-MOE modifications. Bolded residues indicate 2'-O-methoxyethyl moieties and italicized residues indicate methyleneoxy (4'-CH₂₋O-2') BNAs. The internucleoside linkages of each compound are phosphorothioate throughout. All cytosine residues of ISIS 147764 and ISIS 372938 are replaced by 5-methyl cytosines. For ISIS 387462, only the cytosine residue in the wing of the compound is replaced by 5-methyl cytosine. ApoB antisense compounds are targeted to publicly available ApoB-100 sequences, including Genbank Accession No. XM_137955.5 (SEQ ID NO: 2).

**Table 25: Antisense Compounds Targeted to an ApoB nucleic acid**

| **ISIS NO** | **Target SEQ ID NO** | **5' Target Site** | **Sequence (5'-3')** | **Gapmer Motif** | **SEQ ID NO** |
|---|---|---|---|---|---|
| 147764 | 2 | 8865 | **GTCCC**TGAAGATGTC**AATGC** | 5-10-5 MOE | 1561 |
| 372938 | 2 | 8235 | **GG**TACATGGAAG**TC** | 2-10-2 MOE | 190 |
| 387462 | 2 | 8235 | *GG*TACATGGAAG*TC* | 2-10-2 methyleneoxy (4'-CH₂-O-2') BNA | 190 |

Forty-eight hours following the final injection, mice were sacrificed to evaluate transaminases (Table 26); liver and kidney weight (Table 27); triglyceride, LDL, HDL and free fatty acid levels (Table 28); target mRNA level in liver (Table 29); target protein level in plasma; and oligonucleotide tissue concentration (Table 30). These endpoints were determined using methods described herein and well known to those of ordinary skill in the art.

**Table 26: ALT and AST Levels (IU/L)**

| **ISIS NO** | **Dose µmol/kg** | **ALT** | **AST** |
|---|---|---|---|
| Saline | N/A | 27.8 | 46.3 |
| 147764 | 2.4 | 29.5 | 64.0 |
| 372938 | 2.4 | 26.0 | 49.0 |
| 372938 | 1.2 | 24.8 | 49.5 |
| 372938 | 0.6 | 28.0 | 79.3 |
| 372938 | 0.3 | 28.3 | 60.0 |
| 372938 | 0.15 | 28.3 | 50.3 |
| 387462 | 2.4 | 41.3 | 84.0 |
| 387462 | 1.2 | 35.3 | 63.5 |
| 387462 | 0.6 | 32.0 | 77.3 |
| 387462 | 0.3 | 27.8 | 55.0 |
| 387462 | 0.15 | 29.3 | 68.3 |

**Table 27: Liver and Kidney Weight (% of saline control)**

| **ISIS NO** | **Dose** µ**mol/kg** | **Liver** | **Kidney** |
|---|---|---|---|
| Saline | N/A | 100 | 100 |
| 147764 | 2.4 | 102 | 105 |
| 372938 | 2.4 | 100 | 100 |
| 372938 | 1.2 | 90 | 101 |
| 372938 | 0.6 | 96 | 112 |
| 372938 | 0.3 | 91 | 107 |
| 372938 | 0.15 | 96 | 98 |
| 387462 | 2.4 | 116 | 90 |
| 387462 | 1.2 | 113 | 90 |
| 387462 | 0.6 | 106 | 97 |
| 387462 | 0.3 | 101 | 126 |
| 387462 | 0.15 | 95 | 100 |

Total body weight and food consumption did not differ significantly between saline-treated or oligonucleotide-treated animals. Glucose levels also were similar among all treatment groups.

**Table 28: Triglyceride (TRIG), Total Cholesterol (CHOL), HDL, LDL and Free Fatty Acid (FFA) Levels**

| **ISIS NO** | **Dose** µ**mol/kg** | **TRIG (mg/dL)** | **CHOL (mg/dL)** | **HDL (mg/dL)** | **LDL (mg/dL)** | **FFA (mg/dL)** |
|---|---|---|---|---|---|---|
| Saline | N/A | 167 | 107 | 81.8 | 11.0 | 1.76 |
| 147764 | 2.4 | 167 | 107 | 81.3 | 10.3 | 1.29 |
| 372938 | 2.4 | 153 | 104 | 79.0 | 10.3 | 1.28 |
| 372938 | 1.2 | 136 | 101 | 77.8 | 9.5 | 1.70 |
| 372938 | 0.6 | 184 | 110 | 83.3 | 10.8 | 1.66 |
| 372938 | 0.3 | 138 | 109 | 84.3 | 11.0 | 1.53 |
| 372938 | 0.15 | 151 | 106 | 82.8 | 10.8 | 1.57 |
| 387462 | 2.4 | 49 | 14 | 9.0 | 1.5 | 0.74 |
| 387462 | 1.2 | 71 | 23 | 16.5 | 2.0 | 0.76 |
| 387462 | 0.6 | 150 | 55 | 39.3 | 3.7 | 1.43 |
| 387462 | 0.3 | 136 | 92 | 72.8 | 7.5 | 1.14 |
| 387462 | 0.15 | 163 | 104 | 81.5 | 9.3 | 1.47 |

**Table 29: % ApoB mRNA Level (relative to saline control)**

| **ISIS NO** | **2.4** µ**mol/kg** | **1.2** µ**mol/kg** | **0.6** µ**mol/kg** | **0.3** µ**mol/kg** | **0.15** µ**mol/kg** |
|---|---|---|---|---|---|
| 147764 | 57.7 | ND | ND | ND | ND |
| 372938 | 77.0 | 90.0 | 87.3 | 92.6 | 93.1 |
| 387462 | 1.5 | 8.5 | 27.4 | 58.9 | 75.8 |

Treatment with ISIS 387462 resulted in a significant and dose-dependent decrease in triglycerides, total cholesterol, HDL, LDL and free fatty acids. In accordance with these phenotypic findings, treatment with ISIS 387462 also led to a dose-dependent reduction in ApoB mRNA (Table 29) and protein (not shown) levels in mouse plasma. To determine whether the observed increase in efficiency with the methyleneoxy (4'-CHD₂-O-2') BNA gapmer is due to an increase in oligonucleotide accumulation, full-length and total oligonucleotide concentration in the liver and kidney were determined.

**Table 30: Full-length and Total Antisense Compound Tissue Concentration (µM) Relative to ApoB mRNA level (% of saline control)**

| **ISIS NO** | **Dose** µ**mol/kg** | **Kidney Full-Length** | **Liver Full-Length** | **Kidney Total** | **Liver Total** | **ApoB mRNA** |
|---|---|---|---|---|---|---|
| 147764 | 2.4 | 28.6 | 22.9 | 33.5 | 31.3 | 58 |
| 372938 | 2.4 | 32.0 | 5.49 | 34.0 | 7.76 | 77 |
| 387462 | 2.4 | 37.2 | 5.69 | 38.9 | 7.31 | 1.5 |
| 387462 | 1.2 | 29.8 | 3.71 | 31.3 | 4.91 | 8.5 |
| 387462 | 0.6 | 18.9 | 1.97 | 20.0 | 2.57 | 27 |
| 387462 | 0.3 | 9.11 | 0.73 | 9.49 | 0.78 | 59 |
| 387462 | 0.15 | 6.97 | 0.19 | 7.43 | 0.24 | 76 |

Levels of the 2-10-2 methyleneoxy (4'-CH2-O-2') BNA gapmer were similar to the 5-10-5 and 2-10-2 MOE gapmers in the kidney, but significantly reduced in the liver. The EC₅₀ for ISIS 387462 in the liver was determined by comparing oligonucleotide concentration in the liver to inhibition of ApoB mRNA. The approximate EC₅₀ for ISIS 387462 is 1 µM. In contrast, an effective 5-10-5 MOE gapmer compound typically has an EC₅₀ of approximately 15 µM in the liver.

Taken together, these results demonstrate that the ApoB short gapmer having methyleneoxy (4'-CH₂-O-2') in the wings is a potent inhibitor of target mRNA expression and can effectively lower triglycerides, cholesterol and free fatty acids. The potency of the short antisense compound does not appear to be a result of increased tissue accumulation since similar levels of the compound were observed in kidney and reduced levels were found in the liver, relative to the 5-10-5 MOE gapmer. In addition, the methyleneoxy (4'-CH₂-O-2') BNA gapmer exhibited little to no adverse side effects.

### Example 4: Short Antisense Compounds Targeted to a GCGR nucleic acid and having 2'-MOE Modifications

Eight-week old male C57/BL6 mice (Jackson Laboratory, Bar Harbor, ME) were administered a single dose of GCGR oligonucleotide by intraperitoneal injection at a concentration of 6.25, 12.5, 25 or 50 mg. Each dose group consisted of four animals. Shown in Table 31 are the sequences, motifs and conjugates of the GCGR antisense compounds used in this study. Bolded residues indicate 2'-O-methoxyethyl (2'-MOE) moieties. All compounds comprise phosphorothioate internucleoside linkages throughout and each cytosine is replaced with 5-methylcytosine. ISIS 386626, ISIS 386627 and ISIS 386628 further comprise a C₁₆ conjugate group attached to the 2'-O position of the sugar via a diamide linkage (2'-OCH₂C(=O)N(H)(CH₂)₄N(H)C(=O)-(CH₂)₁₅CH₃). GCGR antisense compounds target published GCGR sequences, including Genbank® Accession No. BC031885.1 (SEQ ID NO: 7).

**Table 31: Short antisense compounds targeted to a GCGR nucleic acid**

| **ISIS NO** | **Target SEQ ID NO** | **5' Target Site** | **Sequence (5'-3')** | **Gapmer Motif** | **Conjugate** | **SEQ ID NO** |
|---|---|---|---|---|---|---|
| 148364 | 7 | 393 | **TGCAC**TTTGTGGTAC**CAAGG** | 5-10-5 MOE | None | 1562 |
| 386626 | 7 | 1768 | G_{C16}**C**TTCTCCATCA**TA** | 2-10-2 MOE | C16 | 1563 |
| 386627 | 7 | 1244 | G_{C16}**G**GCATGCTCGT**CA** | 2-10-2 MOE | C16 | 653 |
| 386593 | 7 | 1244 | **GG**GCATGCTCGT**CA** | 2-10-2 MOE | None | 649 |
| 386628 | 7 | 1680 | T_{C16}**G**TCTTGCTGCT**TT** | 2-10-2 MOE | C16 | 1564 |
| 386594 | 7 | 1680 | **TG**TCTTGCTGCT**TT** | 2-10-2 MOE | None | 1565 |

Mice were sacrificed 48 hours following injection to determine serum transaminase levels (Table 32); liver, white adipose tissue (WAT), spleen and kidney weight (Table 33); cholesterol, triglyceride and glucose levels (Table 34); GCGR mRNA levels (Tables 35-41); and full-length and total oligonucleotide concentration in liver and kidney (Table 42). Endpoints were assessed using methods described herein and well known to those of ordinary skill in the art. Data is included from a pre-treatment bleed (Pre-Bleed) and post-treatment bleed (Post-Bleed).

**Table 32: ALT & AST Levels (IU/L)**

| **ISIS NO** | **Dose (mg/kg)** | **ALT Pre-Bleed** | **ALT Post-Bleed** | **AST Pre-Bleed** | **AST Post-Bleed** |
|---|---|---|---|---|---|
| Saline | N/A | 36 | 51 | 55 | 85 |
| 148364 | 50 | 24 | 40 | 40 | 115 |
| 148364 | 25 | 26 | 35 | 42 | 87 |
| 148364 | 12.5 | 23 | 32 | 44 | 69 |
| 148364 | 6.25 | 28 | 34 | 47 | 76 |
| 386626 | 50 | 28 | 40 | 48 | 120 |
| 386626 | 25 | 30 | 36 | 44 | 92 |
| 386626 | 12.5 | 28 | 34 | 44 | 90 |
| 386626 | 6.25 | 26 | 42 | 46 | 69 |
| 386627 | 50 | 27 | 457 | 42 | 451 |
| 386627 | 25 | 29 | 97 | 45 | 142 |
| 386627 | 12.5 | 29 | 62 | 46 | 81 |
| 386627 | 6.25 | 23 | 87 | 38 | 96 |
| 386593 | 50 | 23 | 33 | 46 | 58 |
| 386593 | 25 | 25 | 32 | 41 | 95 |
| 386593 | 12.5 | 26 | 33 | 43 | 74 |
| 386593 | 6.25 | 28 | 31 | 43 | 53 |
| 386628 | 50 | 28 | 68 | 44 | 76 |
| 386628 | 25 | 24 | 32 | 40 | 57 |
| 386628 | 12.5 | 28 | 35 | 42 | 75 |
| 386628 | 6.25 | 22 | 29 | 40 | 59 |
| 386594 | 50 | 29 | 34 | 46 | 92 |
| 386594 | 25 | 27 | 31 | 47 | 82 |
| 386594 | 12.5 | 28 | 33 | 45 | 74 |
| 386594 | 6.25 | 23 | 48 | 42 | 67 |

**Table 33: Organ Weights (% saline control)**

| **ISIS NO** | **Dose (mg/kg)** | **Liver** | **WAT** | **Kidney** | **Spleen** |
|---|---|---|---|---|---|
| Saline | N/A | 100 | 100 | 100 | 100 |
| 148364 | 50 | 103 | 80 | 108 | 123 |
| 148364 | 25 | 103 | 75 | 112 | 115 |
| 148364 | 12.5 | 100 | 84 | 108 | 96 |
| 148364 | 6.25 | 101 | 89 | 104 | 113 |
| 386626 | 50 | 112 | 77 | 104 | 130 |
| 386626 | 25 | 109 | 97 | 103 | 120 |
| 386626 | 12.5 | 96 | 73 | 97 | 114 |
| 386626 | 6.25 | 100 | 90 | 100 | 95 |
| 386627 | 50 | 90 | 113 | 102 | 165 |
| 386627 | 25 | 99 | 87 | 99 | 143 |
| 386627 | 12.5 | 109 | 93 | 102 | 136 |
| 386627 | 6.25 | 103 | 96 | 102 | 131 |
| 386593 | 50 | 96 | 98 | 102 | 118 |
| 386593 | 25 | 83 | 94 | 100 | 104 |
| 386593 | 12.5 | 99 | 82 | 101 | 129 |
| 386593 | 6.25 | 96 | 77 | 98 | 144 |
| 386628 | 50 | 104 | 100 | 99 | 126 |
| 386628 | 25 | 102 | 97 | 109 | 113 |
| 386628 | 12.5 | 101 | 111 | 99 | 114 |
| 386628 | 6.25 | 98 | 106 | 102 | 151 |
| 386594 | 50 | 90 | 80 | 99 | 131 |
| 386594 | 25 | 93 | 76 | 99 | 128 |
| 386594 | 12.5 | 94 | 98 | 100 | 113 |
| 386594 | 6.25 | 102 | 85 | 101 | 119 |

Overall, the GCGR antisense compounds exhibited little to no adverse side effects.

**Table 34: Triglyceride (TRIG), Cholesterol (CHOL) and Glucose Levels (IU/L)**

| **ISIS NO** | **Dose (mg/kg)** | **TRIG Pre-Bleed** | **TRIG Post-Bleed** | **CHOL Pre-Bleed** | **CHOL Post-Bleed** | **Glucose Pre-Bleed** | **Glucose Post-Bleed** |
|---|---|---|---|---|---|---|---|
| Saline | N/A | 132 | 181 | 91 | 96 | 208 | 285 |
| 148364 | 50 | 110 | 177 | 81 | 94 | 207 | 228 |
| 148364 | 25 | 115 | 200 | 83 | 96 | 219 | 239 |
| 148364 | 12.5 | 106 | 179 | 85 | 89 | 198 | 256 |
| 148364 | 6.25 | 86 | 162 | 86 | 89 | 226 | 215 |
| 386626 | 50 | 87 | 163 | 79 | 57 | 239 | 179 |
| 386626 | 25 | 100 | 187 | 87 | 72 | 235 | 186 |
| 386626 | 12.5 | 100 | 148 | 82 | 76 | 232 | 185 |
| 386626 | 6.25 | 86 | 162 | 85 | 90 | 222 | 221 |
| 386627 | 50 | 106 | 120 | 83 | 126 | 227 | 150 |
| 386627 | 25 | 101 | 148 | 90 | 115 | 218 | 203 |
| 386627 | 12.5 | 99 | 203 | 86 | 98 | 237 | 219 |
| 386627 | 6.25 | 111 | 165 | 88 | 104 | 238 | 228 |
| 386593 | 50 | 130 | 128 | 100 | 95 | 244 | 213 |
| 386593 | 25 | 119 | 135 | 83 | 77 | 206 | 208 |
| 386593 | 12.5 | 122 | 128 | 83 | 79 | 222 | 233 |
| 386593 | 6.25 | 120 | 138 | 84 | 78 | 214 | 219 |
| 386628 | 50 | 102 | 98 | 88 | 95 | 209 | 232 |
| 386628 | 25 | 102 | 129 | 84 | 85 | 210 | 223 |
| 386628 | 12.5 | 90 | 123 | 90 | 94 | 231 | 240 |
| 386628 | 6.25 | 117 | 121 | 83 | 85 | 228 | 229 |
| 386594 | 50 | 93 | 99 | 84 | 85 | 203 | 274 |
| 386594 | 25 | 106 | 94 | 90 | 86 | 219 | 272 |
| 386594 | 12.5 | 118 | 133 | 85 | 95 | 200 | 292 |
| 386594 | 6.25 | 112 | 146 | 78 | 94 | 222 | 275 |

GCGR 2-10-2 MOE gapmers exhibited a trend toward lower post-bleed triglyceride levels, relative to the 5-10-5 MOE gapmer, with ISIS 386628 and ISIS 386594 having the greatest dose-dependent effect. Glucose levels also were decreased in a dose-dependent manner following treatment with ISIS 386626 and ISIS 386627. Treatment with ISIS 386628, ISIS 386593 and ISIS 386594 also generally led to a decrease in post-bleed glucose levels. Cholesterol levels did not appear to significantly differ among treatment groups.

To determine whether the phenotypic changes shown above correlated with a decrease in GCGR mRNA, treated animals were evaluated for levels of target mRNA in liver by real time PCR according to methods described herein. Tables 35 to 41 show results from direct comparisons of the antisense compounds targeting GCGR nucleic acid for their effect on target expression. Results are expressed as percent of saline control.

**Table 35: GCGR mRNA levels following treatment with ISIS 148364 & ISIS 386626**

| **ISIS NO** | **50 mg/kg** | **25 mg/kg** | **12.5 mg/kg** | **6.25 mg/kg** |
|---|---|---|---|---|
| 148364 | 36 | 79 | 87 | 62 |
| 386626 | 0 | 8 | 3 | 7 |

**Table 36: GCGR mRNA levels following treatment with ISIS 148364 & ISIS 386627**

| **ISIS NO** | **50 mg/kg** | **25 mg/kg** | **12.5 mg/kg** | **6.25 mg/kg** |
|---|---|---|---|---|
| 148364 | 63 | 87 | 105 | 86 |
| 386627 | 3 | 30 | 57 | 74 |

**Table 37: GCGR mRNA levels following treatment with ISIS 148364 & ISIS 386593**

| **ISIS NO** | **50 mg/kg** | **25 mg/kg** | **12.5 mg/kg** | **6.25 mg/kg** |
|---|---|---|---|---|
| 148364 | 56 | 74 | 105 | 86 |
| 386593 | 9 | 38 | 74 | 90 |

**Table 38: GCGR mRNA levels following treatment with ISIS 148364 & ISIS 386628**

| **ISIS NO** | **50 mg/kg** | **25 mg/kg** | **12.5 mg/kg** | **6.25 mg/kg** |
|---|---|---|---|---|
| 148364 | 42 | 77 | 98 | 101 |
| 386628 | 2 | 18 | 53 | 77 |

**Table 39: GCGR mRNA levels following treatment with ISIS 148364 & ISIS 386594**

| **ISIS NO** | **50 mg/kg** | **25 mg/kg** | **12.5 mg/kg** | **6.25 mg/kg** |
|---|---|---|---|---|
| 148364 | 59 | 98 | 102 | 96 |
| 386594 | 25 | 47 | 50 | 96 |

**Table 40: GCGR mRNA levels following treatment with ISIS 386627 & ISIS 386593**

| **ISIS NO** | **50 mg/kg** | **25 mg/kg** | **12.5 mg/kg** | **6.25 mg/kg** |
|---|---|---|---|---|
| 386627 | 5 | 40 | 58 | 42 |
| 386593 | 10 | 29 | 34 | 71 |

**Table 41: GCGR mRNA levels following treatment with ISIS 386628 & ISIS 386594**

| **ISIS NO** | **50 mg/kg** | **25 mg/kg** | **12.5 mg/kg** | **6.25 mg/kg** |
|---|---|---|---|---|
| 386628 | 4 | 13 | 38 | 97 |
| 386594 | 19 | 50 | 56 | 99 |

Treatment with the 2-10-2 MOE gapmers led to a significant dose-dependent decrease in GCGR mRNA expression. ISIS 386626 exhibited the greatest decrease in target mRNA. To determine whether the observed increase in efficiency with the short antisense compounds is due to an increase in antisense compound accumulation, full-length and total antisense compound concentration in the liver and kidney were determined.

**Table 42: Total and Full-length Antisense Compound Concentrations in Liver and Kidney (µg/g)**

| **ISIS NO** | **Total Kidney** | **Total Liver** | **Full-length Kidney** | **Full-length Liver** |
|---|---|---|---|---|
| 148364 | 90 | 54 | 58 | 46 |
| 386626 | 757 | 274 | 355 | 125 |
| 386593 | 91 | 12 | 77 | 12 |
| 386628 | 496 | 286 | 305 | 202 |

The results shown in Table 42 demonstrate that short antisense compounds comprising a C₁₆ conjugate exhibit a significant increase in antisense compound accumulation in both liver and kidney. However, ISIS 386593, which was effective at reducing target mRNA, triglycerides and glucose levels, accumulates to a level similar to the 5-10-5 MOE gapmer in liver and to a lower level in kidney. These results suggest that while conjugation with C₁₆ can increase liver and kidney antisense compound concentration, it does not entirely account for the effectiveness of the short antisense compounds.

Taken together, these results demonstrate that GCGR short antisense compounds are capable of significantly inhibiting target mRNA expression while also lowering triglyceride and glucose levels. In addition, with the exception of ISIS 386627, the short MOE gapmers exhibited little to no toxic effects.

### Example 5: Short antisense compounds targeting to a GCGR nucleic acid and having 2'-MOE and Methyleneoxy (4'-CH₂-O-2') BNA modifications

Eight-week old male C57/BL6 mice (Jackson Laboratory, Bar Harbor, ME) were administered a single dose of GCGR antisense compound by intraperitonel (i.p.) injection at a concentration of 10, 3.2, 1, and 0.32 µmol.kg. Each dose group consisted of four animals. Shown in Table 43 are the sequences, motifs and conjugates of the GCGR antisense compounds used in this study. Bolded residues indicate 2'-O-methoxyethyl (2'-MOE) modifications and the italicized residues indicate methyleneoxy (4'-CH₂-O-2') BNA modifications. All antisense compounds comprise phosphorothioate internucleoside linkages throughout and each cytosine is replaced with 5-methylcytosine. GCGR antisense compounds target published GCGR nucleic acids, including Genbank Accession No. BC031885.1 (SEQ ID NO: 7).

**Table 43: Antisense Compounds targeted to a GCGR nucleic acid**

| **ISIS NO** | **Target SEQ ID NO** | **5' Target Site** | **Sequence (5'-3')** | **Gapmer Motif** | **SEQ ID NO** |
|---|---|---|---|---|---|
| 148364 | 7 | 393 | **TGCAC**TTTGTGGTAC**CAAGG** | 5-10-5 MOE | 1562 |
| 396144 | 7 | 1768 | **GC**TTCTCCATCA**TA** | 2-10-2 MOE | 1566 |
| 396148 | 7 | 1768 | *GC*TTCTCCATCAT*A* | 2-10-2 Methyleneoxy (4'-CH₂-O-2') BNA | 1567 |
| 396145 | 7 | 1765 | **ATGGC**TTCTCCATCA**TATCC** | 5-10-5 MOE | 1568 |
| 396146 | 7 | 1244 | **GG**GCATGCTCGT**CA** | 2-10-2 MOE | 650 |
| 396149 | 7 | 1244 | *GG*GCATGCTCGT*CA* | 2-10-2 Methyleneoxy (4'-CH₂-O-2') BNA | 652 |
| 396147 | 7 | 1241 | **CTTG**GGCATGCTCGT**CAGTC** | 5-10-5 MOE | 1569 |

To determine whether the phenotypic changes shown above correlated with a decrease in GCGR mRNA, treated animals were evaluated for levels of target mRNA in liver by RT, real time PCR according to methods described herein. Table 44 show results from direct comparisons of the antisense compounds targeting GCGR nucleic acid for their effect on target expression. Results are expressed as percent of saline control.

**TABLE 44: GCGR mRNA levels**

| **ISIS NO.** | **0.32** µ**mol/kg** | **1** µ**mol/kg** | **3.2** µ**mol/kg** | **10** µ**mol/kg** |
|---|---|---|---|---|
| 148364 | 105 | 106 | 73 | 38 |
| 396144 | 122 | 117 | 40 | 35 |
| 396148 | 20 | 6 | 2 | 1 |
| 396145 | nd | Nd | 33 | 8 |
| 396146 | 98 | 135 | 95 | 35 |
| 396149 | 91 | 41 | 30 | 7 |
| 396147 | nd | Nd | 68 | 28 |

As shown in Table 44, each short antisense compound having methyleneoxy (4'-CH₂-O-2') BNA modifications demonstrated a dose-dependent reduction in GCGR mRNA levels. Furthermore, the short antisense compounds were more effective at target reduction than the 5-10-5 MOE gapmer. Each short antisense compound comprising methyleneoxy (4'-CH₂-O-2') BNA in the wings resulted in a significant reduction in GCGR protein relative to both saline control and ISIS 148364 treatment. Next, estimated ED₅₀ concentrations for each antisense were calculated using Graphpad Prism; ED₅₀ is the dose at which 50% mRNA reduction is observed. The results are shown below in Table 45.

**Table 45: Estimated ED₅₀ Concentration**

| **Gapmer Motif** | **ISIS NO** | **ED₅₀ (**µ**mole/kg)** | **ED₅₀ (mg/kg)** |
|---|---|---|---|
| 5-10-5 MOE | 148364 | 7 | 50.6 |
| 2-10-2 MOE | 396144 | 4 | 18.1 |
| 2-10-2 methyleneoxy BNA | 396148 | 0.1 | 0.4 |
| 5-10-5 MOE | 396145 | 2.1 | 9.3 |
| 2-10-2 MOE | 396146 | 8.3 | 40 |
| 2-10-2 methylenexy BNA | 396149 | 1.1 | 5 |
| 5-10-5 MOE | 396147 | 5.2 | 37.5 |

### Example 6: Short Antisense Compounds Targeting a PTEN nucleic acid and having methyleneoxy (4'-CH₂-O-2') BNA Modifications

Six-week old male Balb/c mice (Jackson Laboratory, Bar Harbor, ME) were administered a single i.p. injection of PTEN antisense compound at a dose of 8 µmol/kg. Each dose group consisted of four animals. Shown in Table 46 are the sequences and motifs of the PTEN antisense compounds used in this study. Bolded residues indicate 2'-O-methoxyethyl moieties (2'-MOE) and italicized residues indicate Methyleneoxy BNA nucleotides. Each antisense compound comprises phosphorothioate linkages throughout. In addition, the cytosine residues in the gap of ISIS 384073 and in the wings of ISIS 392056, ISIS 392057, ISIS 392061 and ISIS 392063 are replaced with 5-methylcytosines. Antisense compounds target published PTEN nucleic acids, including Genbank Accession No. U92437.1 (SEQ ID NO: 13).

**Table 46: Antisense Compounds targeted to a PTEN nucleic acid**

| **ISIS NO** | **Target SEQ ID NO** | **5' Target Site** | **Sequence (5'-3')** | **Gapmer Motif** | **SEQ ID NO** |
|---|---|---|---|---|---|
| 141923 | Control | N/A | **CCTTC**CCTGAAGGTT**CCTCC** | 5-10-5 MOE | 1570 |
| 116847 | 29 | 2011 | **TCAAA**TCCAGAGGCT**AGCAG** | 5-10-5 MOE | 1571 |
| 384073 | 29 | 2013 | *AAA*TCCAGAGGCT*AGC* | 3-10-3 methyleneoxy (4'-CH₂-O-2') BNA | 1428 |
| 391172 | 29 | 2013 | *AA*ATCCAGAGGCT*AG* | 2-10-3 methyleneoxy (4'-CH₂-O-2') BNA | 1429 |
| 392056 | 29 | 140 | *AG*CTGCAGCCAT*GA* | 2-10-2 methyleneoxy (4'-CH₂-O-2') BNA | 1263 |
| 392057 | 29 | 807 | *GG*TCCAGGGCCA*AG* | 2-10-2 methyleneoxy (4'-CH₂-O-2') BNA | 1162 |
| 392061 | 29 | 2014 | *AA*TCCAGAGGCT*AG* | 2-10-2 methyleneoxy (4'-CH₂-O-2') BNA | 1431 |
| 392063 | 29 | 3099 | *AG*GCCAGTGCTA*AG* | 2-10-2 methyleneoxy (4'-CH₂-O-2') BNA | 1226 |

Mice were sacrificed 72 hours following injection to determine serum transaminase levels (Table 47); liver and spleen weights (Table 47); and PTEN mRNA levels in liver, kidney and fat (Table 48), according to procedures described herein and well know to one of ordinary skill in the art.

**Table 47: Transaminase Levels and Organ Weights**

| **ISIS NO** | **AST (IU/L)** | **ALT (IU/L)** | **Liver Weight % Saline** | **Spleen Weight % Saline** |
|---|---|---|---|---|
| Saline | 98.5 | 37.5 | 100 | 100 |
| 141923 | 89.5 | 34.8 | 101 | 108 |
| 116847 | 59.8 | 29.5 | 109 | 108 |
| 384073 | 57.8 | 29.3 | 115 | 111 |
| 391172 | 48.5 | 32.8 | 120 | 112 |
| 392056 | 516 | 892 | 125 | 167 |
| 392057 | 63.8 | 34.5 | 125 | 101 |
| 392061 | 189 | 42.0 | 123 | 111 |
| 392063 | 67.3 | 21.8 | 127 | 134 |

Overall, the short antisense compounds with methyleneoxy (4'-CH₂-O-2') BNA modifications exhibited little to no adverse effects. In addition, total body weight did not significantly differ between treatment groups.

**Table 48: %PTEN mRNA levels in Liver, Kidney and Fat**

| **ISIS NO** | **Liver** | **Kidney** | **Fat** |
|---|---|---|---|
| Saline | 100 | 100 | 100 |
| 141923 | 102 | 133 | 118 |
| 116847 | 37 | 96 | 85 |
| 384073 | 24 | 74 | 77 |
| 391172 | 18 | 63 | 101 |
| 392056 | 27 | 88 | 74 |
| 392057 | 33 | 79 | 96 |
| 392061 | 24 | 61 | 85 |
| 392063 | 6.5 | 52 | 72 |

As shown in Table 48, each antisense compound targeted to a PTEN nucleic acid led to a significant reduction in target mRNA levels in liver as compared with saline treated and control treated animals. The antisense compounds had various effects on target mRNA levels in kidney and fat.

### Example 7: Short Antisense Compounds Targeting a PTEN nucleic acid and having BNA Modifications

Six-week old male Balb/c mice (Jackson Laboratory, Bar Harbor, ME) were administered a single intraperitoneal (i.p.) injection of antisense compound targeted a PTEN nucleic acid at a dose of 8, 4, 2 or 1 µmol/kg. Each dose group consisted of four animals. Shown in Table 49 are the sequence, wing chemistry and motif of each antisense compound used in this study. Bold residues indicate 2'-MOE modified nucleotides, italicized letters indicate methyleneoxy (4'-CH₂-O-2') BNA modifications. All antisense compounds comprise phosphorothioate linkages at each position. Each cytosine of ISIS 116847 and the cytosine residues in the methyleneoxy (4'-CH₂-O-2') BNA wings of ISIS 392063 are replaced with 5-methylcytosines, while the thymidine residues in the methyleneoxy (4'-CH₂-O-2') BNA wings of ISIS 392745 are replaced with 5-methyl thymidines. Antisense compounds target published PTEN nucleic acids, including Genbank Accession No. U92437.1 (SEQ ID NO: 13).

**Table 49: Antisense Compounds Targeted to a PTEN Nucleic Acid**

| **ISIS NO** | **Target SEQ ID NO** | **Target Site** | **Sequence (5'-3')** | **Gapmer Motif** | **SEQ ID NO** |
|---|---|---|---|---|---|
| 116847 | 13 | 2011 | **TCAAA**TCCAGAGGCT**AGCAG** | 5-10-5 MOE | 1571 |
| 392063 | 13 | 3099 | *CT*TAGCACTGGC*CT* | 2-10-2 Methyleneoxy BNA | 1226 |
| 392745 | 13 | 3099 | *CT*TAGCACTGGC*CT* | 2-10-2 methyleneoxy BNA | 1226 |

Mice were sacrificed 72 hours following injection to determine serum transaminase levels (Table 50); liver, kidney and spleen weights (Table 50); PTEN mRNA levels in liver (Table 51); and estimated ED₅₀ oligonucleotide concentration (Table 52). These endpoints were measured using methods described herein and well known to those of ordinary skill in the art.

**Table 50: AST, ALT and Bilirubin Levels and Organ Weights**

| **ISIS NO** | **Dose** µ**mol/kg** | **AST (IU/L)** | **ALT (IU/L)** | **Bilirubin (mg/dL)** | **Liver Weight % Saline** | **Kidney Weight % Saline** | **Spleen Weight % Saline** |
|---|---|---|---|---|---|---|---|
| Saline | N/A | 64.0 | 31.8 | 0.15 | 100 | 100 | 100 |
| 116847 | 8 | 73.0 | 32.0 | 0.1 | 114 | 92 | 106 |
| 392063 | 8 | 50.3 | 17.3 | 0.1 | 115 | 98 | 115 |
| 392063 | 4 | 100.8 | 31.3 | 0.15 | 122 | 94 | 116 |
| 392063 | 2 | 60.5 | 32.8 | 0.1 | 112 | 99 | 106 |
| 392063 | 1 | 57.5 | 29.3 | 0.1 | 104 | 95 | 107 |
| 392745 | 8 | 75.5 | 23.5 | 0.13 | 125 | 99 | 100 |
| 392745 | 4 | 77.0 | 29.3 | 0.13 | 121 | 100 | 96 |
| 392745 | 2 | 69.0 | 32.0 | 0.13 | 110 | 98 | 103 |
| 392745 | 1 | 52.0 | 27.3 | 0.1 | 109 | 97 | 104 |

Overall, the PTEN antisense compounds did not show significant signs of toxicity. Kidney, liver and spleen weights were all within normal ranges. Total body weight did not significantly differ between treatment groups.

**Table 51: % PTEN mRNA levels in Liver (relative to saline control)**

| **ISIS NO** | **8** µ**mol/kg** | **4** µ**mol/kg** | **2** µ**mol/kg** | **1** µ**mol/kg** |
|---|---|---|---|---|
| 116847 | 36 | ND | ND | ND |
| 392063 | 7.4 | 16 | 32 | 60 |
| 392745 | 5.2 | 11 | 31 | 60 |

As shown in Table 51, each short antisense compound having methyleneoxy (4'-CH₂-O-2') BNA modifications demonstrated a dose-dependent reduction in PTEN mRNA levels. Furthermore, the short antisense compounds were more effective at target reduction than the 5-10-5 MOE gapmer. Levels of PTEN protein in liver were also determined following administration of each antisense compound at a dose of 8 µmol/kg. Each short antisense compound comprising methyleneoxy (4'-CH₂-O-2') BNA in the wings resulted in a significant reduction in PTEN protein relative to both saline control and ISIS 116847 treatment. Next, estimated ED₅₀ concentrations for each oligonucleotide were calculated using Graphpad Prism. The results are shown below in Table 52.

**Table 52: Estimated ED₅₀ Concentration**

| **Wing Chemistry** | **ISIS NO** | **ED₅₀ (**µ**mole/kg)** | **ED₅₀ (mg/kg)** |
|---|---|---|---|
| MOE (with 5-MeC) | 116847 | 6.3 | 45.2 |
| methyleneoxy BNA (with 5-MeC) | 392063 | 1.3 | 5.8 |
| methyleneoxy BNA | 392745 | 1.2 | 5.6 |

To further investigate different types of bicyclic nucleic acid compounds, an additional set of short antisense compounds targeting a PTEN nucleic acid was designed and tested. Six-week old male Balb/c mice (Jackson Laboratory, Bar Harbor, ME) were administered a single intraperitoneal (i.p.) injection of antisense compound at a dose of 8, 4, 2 or 1 µmol/kg. Each dose group consisted of four animals. Shown in Table 53 are the sequence, wing chemistry and motif of each antisense compound used in this study. All antisense compounds comprise phosphorothioate linkages at each position. The cytosine residues in the methyleneoxy (4'-CH₂-O-2') BNA wings of ISIS 392063 are replaced with 5-methylcytosines. The antisense compound target published PTEN nucleic acids, including Genbank Accession No. U92437.1 (SEQ ID NO: 13).

**Table 53: Antisense Compounds Targeting a PTEN Nucleic Acid**

| **ISIS NO** | **Target SEQ ID NO** | **5' Target Site** | **Sequence (5'-3')** | **Gapmer Motif** | **SEQ ID NO** |
|---|---|---|---|---|---|
| 392063 | 29 | 3099 | *CT*TAGCACTGGC*CT* | 2-10-2 Methyleneoxy BNA | 1226 |
| 396564 | 29 | 3099 | CTTAGCACTGGCCT | 2-10-2 Oxyamino (4'-CH₂-N(R)-O-2') BNA | 1226 |
| 396006 | 29 | 3099 | CTTAGCACTGGCCT | 2-10-2 α-L-Methyleneoxy BNA | 1226 |

Mice were sacrificed 72 hours following injection to determine serum transaminase levels (Table 54); liver and spleen weights (Table 54); and PTEN mRNA levels in liver (Table 55), according to methods described herein and well known to those of ordinary skill in the art.

**Table 54: AST and ALT Levels and Organ Weights**

| **ISIS NO** | **Dose** µ**mol/kg** | **AST (IU/L)** | **ALT (IU/L)** | **Liver Weight** | **Spleen Weight** |
|---|---|---|---|---|---|
| Saline | N/A | 71 | 33 | 100 | 100 |
| 392063 | 8 | 97 | 38 | 118 | 103 |
| 392063 | 4 | 179 | 36 | 115 | 107 |
| 392063 | 2 | 67 | 32 | 109 | 116 |
| 392063 | 1 | 68 | 27 | 102 | 105 |
| 396564 | 8 | 67 | 25 | 100 | 104 |
| 396564 | 4 | 96 | 30 | 102 | 106 |
| 396564 | 2 | 68 | 27 | 100 | 119 |
| 396564 | 1 | 79 | 39 | 97 | 109 |
| 396006 | 8 | 56 | 28 | 110 | 104 |
| 396006 | 2 | 139 | 36 | 97 | 105 |

**Table 55: % PTEN mRNA levels in Liver (relative to saline control)**

| **ISIS NO** | **8** µ**mol/kg** | **4** µ**mol/kg** | **2** µ**mol/kg** | **1** µ**mol/kg** |
|---|---|---|---|---|
| 392063 | 6.9 | 18 | 39 | 71 |
| 396564 | 86 | 97 | 100 | 96 |
| 396006 | 6.5 | ND | ND | 70 |

As shown above, short antisense compounds having α-L-methyleneoxy (4'-CH₂-O-2') BNA modifications led to a dose-dependent reduction in target mRNA levels. Treatment with the short antisense compound having oxyamino BNA modifications led to a modest reduction in target expression.

### Example 8. Single Dose Administration Dose Response Study with Short Antisense Compounds Targeting ApoB and PTEN nucleic acids

Six-week old male Balb/c mice (Jackson Laboratory, Bar Harbor, ME) were administered a single intraperitoneal (i.p.) injection of antisense compound at a dose of 8, 4, 2 or 1 µmol/kg. Each dose group consisted of four animals. Shown in Table 56 are the sequence, wing chemistry and motif of each antisense compound used in this study. Italicized residues indicate methyleneoxy (4'-CH₂-O-2') BNA modifications, underlined residues indicate *N*-methyl-oxyamino (4'-CH₂-N(CH₃)-O-2') BNA modifications, and boxed residues indicate α-L-methyleneoxy (4'-CH₂-O-2') BNA modifications. All antisense compounds comprise phosphorothioate linkages at each position. Each cytosine of ISIS 116847 and the cytosine residues in the methyleneoxy (4'-CH₂-O-2') BNA wings of ISIS 392063 are replaced with 5-methylcytosines, while the thymidine residues in the methyleneoxy (4'-CH₂-O-2') BNA wings of ISIS 392745 are replaced with 5-methyl thymidines. PTEN antisense compounds target published PTEN nucleic acid, including Genbank Accession No. U92437.1 (SEQ ID NO: 13). ApoB antisense compounds target published ApoB nucleic acid, including Genbank Accession No. XM_137955.5 (SEQ ID NO: 2).

**Table 56: Short Antisense Compounds Targeted to ApoB and PTEN Nucleic Acids**

| **ISIS NO** | **Target** | **Target Seq ID** | **5' Target Site** | **SEQUENCE** | **Gapmer** | **SEQ ID NO** |
|---|---|---|---|---|---|---|
| 387462 | ApoB | 19 | 8235 | *GG*TACATGGAAG*TC* | 2-10-2 Methyleneoxy BNA | 193 |
| 392063 | PTEN | 29 | 3099 | *CT*TAGCACTGGCCT | 2-10-2 Methyleneoxy BNA | 1226 |
| 396565 | PTEN | 29 | 3099 | CUTAGCACTGGCCU | 2-10-2 N-Me-oxyamino BNA | 1226 |
| 396006 | PTEN | 29 | 3099 | CUTAGCACTGGCCU | 2-10-2 α-L-methyleneoxy BNA | 1226 |

**Table 57: %ApoB and PTEN mRNA Reduction (relative to saline control)**

| **ISIS NO** | **Dose (**µ**mol/kg)** | **%ApoB mRNA Reduction (relative to saline)** | **%PTEN mRNA Reduction (relative to saline)** |
|---|---|---|---|
| 387462 | 8 | 0.62 | 92.8 |
| | 4 | 6.55 | 103 |
| | 2 | 18.6 | 105 |
| | 1 | 42.0 | 98.0 |
| 392063 | 8 | 126 | 6.79 |
| | 4 | 111 | 18.1 |
| | 2 | 112 | 42.4 |
| | 1 | 114 | 62.3 |
| 396565 | 8 | 116 | 23.8 |
| | 4 | 1.04 | 46.6 |
| | 2 | 94.4 | 76.1 |
| | 1 | 115 | 89.5 |
| 396006 | 8 | 94.3 | 62.9 |
| | 4 | 101 | 18.2 |
| | 2 | 79.7 | 52.4 |
| | 1 | 111 | 82.4 |

As shown in Table 57, each short antisense compound having Methyleneoxy BNA modifications demonstrated a dose-dependent reduction in target mRNA levels. Notably, the short antisense compound with N-methyl-oxyamino BNA wings (ISIS 396565) also demonstrated dose-dependent reduction in PTEN expression similar to both the β-D-methyleneoxy BNA and α-L-methyleneoxy BNA short antisense compounds. Next, estimated ED₅₀ concentrations for each antisense were calculated using Graphpad Prism. The results are shown below in Table 58.

**Table 58: Estimated ED₅₀ Concentrations**

| **Wing Chemistry** | **ISIS NO** | **ED₅₀ (**µ**mole/kg)** | **ED₅₀ (mg/kg)** |
|---|---|---|---|
| Methyleneoxy BNA | 387462 | 0.8 | 3.9 |
| Methyleneoxy BNA | 392063 | 1.5 | 7 |
| N-Me-oxyamino BNA | 396565 | 3.8 | 17.4 |
| α-L-methyleneoxy BNA | 396006 | 2.1 | 9.3 |

### EXAMPLE 9: Administration of a Parent and Parent Mixed Backbone Antisense Compound Targeting SGLT-2 mRNA.

ISIS 257016 was administered to *db*/*db* mice (Charles River Laboratories, Wilmington, MA) intraperitoneally at a dose of 1, 7.5, 14 or 17 mg/kg twice a week. Control groups included a group receiving saline on the same dosing schedule and a group receiving ISIS 145733. ISIS 257016 and ISIS 145733 both comprise the sequence GAAGTAGCCACCAACTGTGC (SEQ ID NO: 1572) further comprising a central "gap" region consisting of ten 2'-deoxynucleotides, which is flanked on both sides (5' and 3' directions) by five-nucleotide "wings". The wings are composed of 2'-methoxyethyl (2'-MOE) nucleotides. All cytidine residues are 5-methylcytidines. The internucleoside (backbone) linkages are phosphorothioate (P=S) throughout the oligonucleotide for ISIS 145733; however ISIS 257016 has a mixed backbone. The internucleoside linkages for ISIS 257016 are phosphodiester (P=O) in the wings and phosphorothioate in the gap. Forty-eight hours following administration of the last dose the mice were sacrificed and kidney tissue was analyzed for SGLT-2 mRNA levels. The results are shown below in Table 59.

**Table 59: Antisense inhibition of SGLT2 mRNA expression in vivo by 5-10-5 MOE gapmers**

| | **% change in SGLT2 expression relative to saline** | |
|---|---|---|
| **Dose of oligonucleotide nmol/kg** | **ISIS 145733** | **ISIS 257016** |
| **17** | -37.5 | -76 |
| **14** | -31.25 | -74 |
| **7.5** | -12.5 | -62.5 |
| **1** | +3 | -44 |

Both ISIS 257016 and ISIS 145733 markedly reduced SGLT-2 levels compared to saline control. (mRNA levels determined using RT, real-time PCR as described above) However, ISIS 257016 has been shown to be about 20-50 times more potent for reducing SGLT-2 mRNA compared to ISIS 145733. An associated reduction in plasma glucose levels was seen for the treatment groups (661 ±14 for the saline group compared to 470 ±23 for the group receiving ISIS 257016). Accumulation of ISIS 257016 and ISIS 145733 in the kidney was similar over the dose range, however little of the full length 257016 antisense was detected in the kidney which supports the theory that a degradation product is responsible for the increased activity. Also the onset of action following a single dose of 25 mg/kg correlated to a time pint were little intact 257016 antisense compound was left.

Similar studies were performed in lean mice, *ob*/*ob* mice and in ZDF rats (Charles Rivers Laboratories) using ISIS 257016, ISIS 145733 or saline in a similar same dosing schedule as described above. The sequence of the binding site for ISIS 145733 and ISIS 257016 is conserved between mouse and rat (see Table 60). Reduction of SGLT-2 mRNA in the kidney was similar to that seen above. In a study utilizing rats, at a dose of 10 mg/kg given two times a week for two weeks, ISIS 145733 was shown to reduce SGLT-2 mRNA levels by about 40% whereas the reduction achieved with ISIS 257016 was greater than 80%. ISIS 257016 reduces SGLT2 expression maximally at a low dose of 12.5 mg/kg. Additional studies at lower dosing ranges show significant reduction of SGLT2 mRNA levels with the mixed backbone antisense compound at doses less than 1 mg/kg/wk.

### EXAMPLE 10: Administration of a Parent and Short Antisense Compound Targeting SGLT-2 mRNA

Pharmacokinetic studies indicated that ISIS 257016 was acting as a prodrug that was metabolized to a 12 nucleobase pharmacophore. In a next study, ZDF rats were dosed intraperitoneally twice per week with 1.5 mg/kg of either ISIS 257016 or ISIS 370717, or with saline at a similar dosing schedule. ISIS 370717 is a 12 nucleobase antisense compound targeted to SGLT-2 nucleic acid comprising the sequence TAGCCACCAACT (SEQ ID NO: 154) and further comprising central "gap" region consisting of ten 2'-deoxynucleotides, which is flanked on both sides (5' and 3' directions) by one-nucleotide "wings". The wings are composed of 2'-methoxyethyl (2'-MOE) nucleotides. All cytidine residues are 5-methylcytidines. The internucleoside (backbone) linkages are phosphorothioate (P=S) throughout the oligonucleotide.

Following five weeks of dosing the animals were sacrificed and kidney tissue was analyzed for SGLT-2 mRNA levels. The pharmacological activity of ISIS 257016 and ISIS 370717 were similar, however, the 12 nucleotide antisense compound displayed a faster onset of action. ISIS 370717 displayed nearly 80% inhibition of SGLT2 expression in kidney on day two after a single dose of 2.8 umoles/kg whereas ISIS 257016 displayed only about 25% inhibition on day 2 after the same single dose administration. The date support that ISIS 257016 is a prodrug having a 12 nucleotide pharmacophore.

### EXAMPLE 11: Potency and Bioavailability of a Short Antisense Compound

The improved potency displayed by ISIS 370717 and the improved oral bioavailability for these short antisense compounds makes these compounds useful for oral administration. Normal rats received ISIS 370717, ISIS 145733 or saline at 100 mg/kg twice per week via intrajejunal administration. About 48 hours following the last dose, the animals were sacrificed and kidney tissue was analyzed for antisense compound concentration and SGLT-2 mRNA levels. There was a significantly higher accumulation of ISIS 370717 in the kidney tissue (approximately 500 micro grams per gram of tissue) compared to the controls. Moreover, SGLT-2 mRNA was reduced by more than 80% over the controls.

### EXAMPLE 12: Wing, Gap and Total Length Variations Around a 12 nucleotide short antisense compound

ISIS 370717 1-10-1 MOE gapmer was used as a template to make sequence related oligos with varying motifs. These variations are provided in Table 60. The antisense compounds were designed to target different regions of the mouse or rat SGLT2 nucleic acid, using published sequences (GenBank accession number U29881.1, incorporated herein as SEQ ID NO: 1575, and GenBank accession number AJ292928.1, incorporated herein as SEQ ID NO: 1576, respectively).

**Table 60: Short Antisense compounds targeting SGLT2 nucleic acids**

| **ISIS NO** | **5' Target Site on mouse SEQ ID NO: 1575** | **5' Target Site on rat SEQ ID NO: 1576** | **Gapmer Motif** | **Sequence (5'-3')** | **SEQ ID NO** |
|---|---|---|---|---|---|
| 257016 | 2680 | 148 | 5-10-5 MOE | GAAGTAGCCACCAACTGTGC | 1553 |
| 370717 | 2684 | 152 | 1-10-1 MOE | TAGCCACCAACT | 1554 |
| 386169 | 2684 | 152 | 2-8-2 MOE | TAGCCACCAACT | 1555 |
| 386176 | 2685 | 153 | 1-8-1 | AGCCACCAAC | 1556 |
| | | | MOE | | |
| 386196 | 2684 | 152 | 3-6-3 MOE | TAGCCACCAACT | 1557 |

The antisense compounds were analyzed for their effect on mouse SGLT2 mRNA levels. Data are ranges taken from three experiments in which mice were dosed twice per week for three weeks with 2.5, 0.5 or 0.1 umol/kg of the above MOE gapmers given by intraperitoneal injection. Mice were sacrificed 48 hours following last administration and evaluated for SGLT2 levels in kidney. SGLT2 mRNA levels were determined by RT, real-time PCR as described by other examples herein. PCR results were normalized to an internal ISIS control. The results are shown below in Table 61.

**Table 61: Antisense inhibition of SGLT2 in vivo by 1-10-1 and 1-10-2 MOE gapmers**

| | **% change in SGLT2 expression relative to saline** | | | | |
|---|---|---|---|---|---|
| **Dose of oligonucleotide umol/kg** | **ISIS 370717** | **ISIS 386169** | **ISIS 386176** | **ISIS 386196** | **ISIS 386197** |
| **2.5** | -82 | -85 | -80 | -50 | -20 |
| **0.5** | -70 | -80 | -68 | -30 | -15 |
| **0.1** | -55 | -70 | -65 | -35 | -20 |

These results illustrate that all the various motifs tested inhibit the expression of SGLT2 *in vivo* in a dose-dependent manner. The 1-10-1, 2-8-2 and 1-8-1 gapmers were found to be particularly potent.

### EXAMPLE 13: Antisense Inhibition of Rat SGLT-2 by 1-10-1 and 1-10-2 MOE Gapmers

1-10-1 and 1-10-2 MOE gapmer antisense compounds, provided in Table 62, were designed to target different regions of the mouse or rat SGLT2 RNA. All short antisense compounds in Table 62 are chimeric oligonucleotides ("gapmers") either 12 or 13 nucleotides in length, composed of a central "gap" segment consisting of ten 2'-deoxynucleotides, which are flanked on the 5' side by a one-nucleoside "wing" and on the 3' side by a two or one-nucleotide "wing". The wings are composed of 2'-methoxyethyl (2'-MOE) nucleotides. The internucleoside (backbone) linkages are phosphorothioate (P=S) throughout the oligonucleotide. All cytidine residues are 5-methylcytidines.

**Table 62: Antisense compounds targeting SGLT2 nucleic acid**

| **ISIS NO** | **5' Target Site on SEQ ID NO: XXX (mouse)** | **5' Target Site on SEQ ID NO: XXX (rat)** | **Gapmer Motif** | **Sequence (5'-3')** | **SEQ ID NO** |
|---|---|---|---|---|---|
| 370717 | 2684 | 152 | 1-10-1 MOE | TAGCCACCAACT | 1554 |
| 382675 | 2683 | 151 | 1-10-1 MOE | TAGCCACCAACTG | 1559 |
| 379692 | | 508 | 1-10-1 MOE | TGTTCCAGCCCA | 246 |
| 382676 | | 507 | 1-10-2 MOE | TGTTCCAGCCCAG | 246 |
| 379699 | | 1112 | 1-10-2 MOE | GGCATGAGCTTC | 281 |
| 382677 | | 1111 | 1-10-2 MOE | GGCATGAGCTTCA | 281 |
| 382677 | | 958 | 1-10-2 MOE | GGCATGAGCTTCA | 281 |

The short antisense compounds were analyzed for their effect on rat SGLT2 mRNA levels. Data are ranges taken from three experiments in which Male Sprague-Dawley rats (170-200g) were dosed twice per week for three weeks with 450, 150 or 50 nmol/kg of either a 1-10-1 or 1-10-2 MOE gapmer given by intraperitoneal injection. Rats were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 63.

**Table 63: Antisense inhibition of SGLT2 mRNA in vivo by 1-10-1 and 1-10-2 MOE gapmers**

| | **% change in SGLT2 expression relative to saline** | | | | | |
|---|---|---|---|---|---|---|
| **Dose of oligonucleotide nmol/kg** | **ISIS 370717 1-10-1** | **ISIS 382675 1-10-2** | **ISIS 379692 1-10-1** | **ISIS 382676 1-10-2** | **ISIS 379699 1-10-1** | **ISIS 382677 1-10-2** |
| **450** | -70 | -80 | -90 | -85 | -83 | -75 |
| **150** | -70 | -65 | -85 | -80 | -75 | -60 |
| **50** | -55 | -50 | -80 | -65 | -60 | -40 |

These results illustrate that both the 1-10-1 and 1-10-2 MOE gapmers reduce SGLT2 mRNA *in vivo* in a dose-dependent manner.

Rats were further evaluated for total body weight, liver, spleen and kidney weight. Significant changes in spleen, liver or body weight can indicate that a particular compound causes toxic effects. All changes were within the margin of error of the experiment. No significant changes in body weight were observed during the treatment or at study termination. No significant changes in liver or spleen weights were observed.

Toxic effects of short antisense compounds administered *in vivo* can also be assessed by measuring the levels of enzymes and proteins associated with disease or injury of the liver or kidney. Elevations in the levels of the serum transaminases aspartate aminotransferase (AST) and alanine aminotransferase (ALT) are often indicators of liver disease or injury. Serum total bilirubin is an indicator of liver and biliary function, and albumin and blood urea nitrogen (BUN) are indicators of renal function. Glucose and triglyceride levels are sometimes altered due to toxicity of a treatment. Serum glucose also depends in part upon the activity of SGLT2. The levels of ALT, AST, total bilirubin, albumin, BUN, glucose and triglyceride were measured in rats treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function. Triglyceride and glucose levels were not significantly elevated relative to saline-treated animals.

### EXAMPLE 14: Antisense Inhibition of Mouse and Rat SGLT2 by 1-10-1 MOE Gapmers

1-10-1 MOE gapmer antisense compounds designed to target different regions of mouse SGLT2 mRNA are shown in Table 64.

**Table 64: Composition of Antisense Compounds Targeting SGLT2 mRNA**

| **ISIS NO** | 5' Target Site on SEQ ID NO: XXX (mouse) | 5' Target Site on SEQ ID NO: XXX (rat) | **Motif** | **Sequence (5'-3')** | **SEQ ID NO** |
|---|---|---|---|---|---|
| 370717 | 2684 | 152 | 1-10-1 MOE | TAGCCACCAACT | 1554 |
| 379692 | | 508 | 1-10-1 MOE | TGTTCCAGCCCA | 246 |
| 379699 | | 1112 | 1-10-1 MOE | GGCATGAGCTTC | 281 |
| 379702 | | 1525 | 1-10-1 MOE | GCACACAGCTGC | 293 |
| 381408 | 3034** | | 1-10-1 MOE | TACCGAACACCT | 1560 |

| | | | | | |
|---|---|---|---|---|---|
| ** indicates 3 mismatches to a target sequence | | | | | |

The short antisense compounds were analyzed for their effect on mouse SGLT2 mRNA levels. Data was taken from three experiments in which Male 6-week old Balb/c mice were dosed twice per week for two weeks with 450, 150, or 50 nmol/kg of one of the above 1-10-1 MOE gapmers given by intraperitoneal injection. Mice were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 65.

**Table 65: Antisense inhibition of SGLT2 mRNA in vivo by 1-10-1 MOE gapmers**

| | **% change in SGLT2 expression relative to saline** | | | | |
|---|---|---|---|---|---|
| **Dose of oligonucleotide nmol/kg** | **ISIS 370717** | **ISIS 379692** | **ISIS 379699** | **ISIS 379702** | **ISIS 381408** |
| 450 | -65 | -80 | -80 | -75 | - |
| 150 | -55 | -70 | -62.5 | -72.5 | - |
| 50 | -47.5 | -52.5 | -42.5 | -52.5 | - |

These results illustrate that all the 1-10-1 MOE gapmers except, ISIS 381408, inhibit the expression of SGLT2 in vivo in a dose-dependent manner in mouse. Activity of ISIS 381408 has been shown in Rat studies (See Table 65).

### Evaluation of 1-10-1 Gapmers in Rat

The effect of the above 1-10-1 gapmers (see Table 64 above) on rat SGLT2 mRNA levels. Data are taken from four experiments in which male Sprague-Dawley rats (170-200g) were dosed twice per week for three weeks with 250 nmol/kg given by intraperitoneal injection. Rats were sacrificed 48 hours following last administration and evaluated for SGLT2 mRNA levels in kidney. Target levels were determined by RT, real-time PCR as described by other examples herein. PCR results were normalized to an internal ISIS control. The results are shown below in Table 66.

**Table 66: Antisense inhibition of SGLT2 mRNA in vivo by 1-10-1 MOE gapmers**

| | **% change in SGLT2 expression relative to saline** | | | | |
|---|---|---|---|---|---|
| **Dose of oligonucleotide nmol/kg** | **ISIS 370717** | **ISIS 379692** | **ISIS 379699** | **ISIS 379702** | **ISIS 381408** |
| 250 | -70 | -85 | -75 | -25 | -5 |

These results illustrate that all the 1-10-1 MOE gapmers inhibit the expression of SGLT2 in *in vivo* rat studies.

### EXAMPLE 15: Antisense Inhibition of Mouse and Rat SGLT2 Expression by Additional 1-10-1 and 2-8-2 MOE Gapmers

1-10-1 and 2-8-2 MOE gapmer short antisense compounds were designed to target different regions of the mouse SGLT2 RNA but have complementarity across species. The short antisense compounds are shown in Table 67. All short antisense compounds in Table 67 are gapmers 12 nucleotides in length, composed of a central "gap" segment consisting of 2'-deoxynucleotides, which are flanked on both sides (5' and 3' directions) by wing segments having 2'-modifications. The wings are composed of 2'-methoxyethyl (2'-MOE) nucleotides. The internucleoside (backbone) linkages are phosphorothioate (P=S) throughout the oligonucleotide. All cytidine residues are 5-methylcytidines.

**Table 67: Short Antisense Compounds Targeting SGLT2 nucleic acid**

| **ISIS NO** | 5' Target Site (rat) | Target SEQ ID (rat) | **Gapmer Motif** | **Sequence (5'-3')** | **SEQ ID NO** |
|---|---|---|---|---|---|
| 379692 | 508 | | 1-10-1 MOE | TGTTCCAGCCCA | 246 |
| 388625 | 508 | | 1-10-1 MOE | TGTTCCAGCCCA | 246 |
| 379699 | 1112 | | 1-10-1 MOE | GGCATGAGCTTC | 281 |
| 388626 | 1112 | | 2-8-2 MOE | GGCATGAGCTTC | 281 |
| 379702 | 1525 | | 2-8-2 MOE | GCACACAGCTGC | 293 |
| 388627 | 1525 | | 2-8-2 MOE | GCACACAGCTGC | 293 |

The short antisense compounds were analyzed for their effect on mouse SGLT2 mRNA levels *in vivo.* Data was taken from three experiments in which male 6-week old Balb/c mice were dosed twice per week for three weeks with 0.5, 0.1, or 0.02 umol/kg of either a 1-10-1 or 2-8-2 MOE gapmer given by intraperitoneal injection. Mice were sacrificed 48 hours following last administration and evaluated for SGLT2 levels in kidney. Target levels were determined by RT, real-time PCR as described by other examples herein. PCR results were normalized to an internal ISIS control. The results are shown below in Table 68.

**Table 68: Antisense inhibition of SGLT2 mRNA in vivo by 1-10-1 and 2-8-2 MOE gapmers**

| | **% change in SGLT2 expression relative to saline** | | | | | |
|---|---|---|---|---|---|---|
| **Dose of oligonucleotide umol/kg** | **ISIS 379692 1-10-1** | **ISIS 388625 2-8-2** | **ISIS 379699 1-10-1** | **ISIS 388626 2-8-2** | **ISIS 379702 1-10-1** | **ISIS 388627 2-8-2** |
| 0.5 | -85 | -90 | -75 | -80 | -70 | -65 |
| 0.1 | -75 | -88 | -60 | -60 | -65 | -50 |
| 0.02 | -55 | -65 | -30 | -45 | -40 | -38 |

These results illustrate that both the 1-10-1 and 2-8-2 MOE gapmers inhibit the expression of SGLT2 in vivo in a dose-dependent manner.

Mice were further evaluated for total body weight, liver, spleen and kidney weight. All changes were within the margin of error of the experiment. No significant changes in body weight were observed during the treatment or at study termination. No significant changes in liver or spleen weights were observed.

The levels of ALT, AST, BUN, transaminases, plasma creatinine, glucose and triglyceride were measured in mice treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function. Triglyceride and glucose levels were not significantly elevated relative to saline-treated animals.

### Evaluation of ISIS 3796921-10-1 MOE Gapmer, ISIS 392170 1-10-1 Methyleneoxy BNA Gapmer, ISIS 388625 2-8-2 MOE Gapmer and ISIS 392173 2-8-2 Methyleneoxy BNA Gapmer in Mice

The effect of ISIS 379692 1-10-1 MOE gapmer and ISIS 388625 2-8-2 MOE gapmer are compared with the effect of ISIS 392170 1-10-1 Methyleneoxy BNA Gapmer and ISIS 392173 2-8-2 Methyleneoxy BNA Gapmer (see Table 69) on mouse SGLT2 mRNA levels *in vivo.* Data are taken from three experiments in which male 6-week old Balb/c mice were dosed twice per week for three weeks with 5, 25 and 125 nmol/kg of either the ISIS 379692 1-10-1 MOE gapmer or the ISIS 388625 2-8-2 MOE gapmer given by intraperitoneal injection. Mice were sacrificed 48 hours following last administration and evaluated for SGLT2 mRNA levels in kidney. Target levels were determined by RT, real-time PCR as described by other examples herein. PCR results were normalized to an internal ISIS control. The data are expressed as percent change ("+" indicates an increase, "-" indicates a decrease) relative to saline treated animals and are illustrated in Table 69.

**Table 69: Antisense inhibition of SGLT2 mRNA in vivo by a 1-10-1 and a 2-8-2 MOE gapmer**

| **Dose of oligonucleotide nmol/kg** | **ISIS 379692 1-10-1 MOE** | **ISIS 392170 1-10-1 Methyleneoxy BNA** | **ISIS 388625 2-8-2 MOE** | **ISIS 392173 2-8-2 Methyleneo xy BNA** |
|---|---|---|---|---|
| 125 | -58 | -69 | -70 | -75 |
| 25 | -46 | -54 | -47 | -57 |
| 5 | -7 | -23 | -18 | -44 |

These results illustrate that both the 1-10-1 and 2-8-2 MOE gapmer inhibit the expression of SGLT2 *in vivo* at the highest three dosing ranges in a dose-dependent manner. The results also illustrate that the Methyleneoxy BNA constructs are more potent then the MOE constructs. No significant changes in body weight were observed during the treatment or at study termination. No significant changes in liver or spleen weights were observed. The toxicity parameters including levels of ALT, AST, BUN, and creatinine were within normal ranges and are not significantly changed relative to saline-treated animals, demonstrating that the compounds do not significantly affect renal or hepatic function.

### Evaluation of ISIS 379692 1-10-1 MOE Gapmer and ISIS 388625 2-8-2 MOE Gapmer in Rat

The effect of ISIS 379692 1-10-1 MOE gapmer and ISIS 388625 MOE 2-8-2 gapmer (see Table 70) on rat SGLT2 mRNA levels *in vivo.* Data are taken from four experiments in which male Sprague-Dawley rats (170-200g) were dosed twice per week for three weeks with 200, 50, 12.5, or 3.125 nmol/kg of either the ISIS 379692 1-10-1 MOE gapmer or the ISIS 388625 2-8-2 MOE gapmer given by intraperitoneal injection. Rats were sacrificed 48 hours following last administration and evaluated for SGLT2 levels in kidney. Target levels were determined by RT, real-time PCR as described by other examples herein. PCR results were normalized to an internal ISIS control. The results are shown below in Table 70.

**Table 70: Antisense inhibition of SGLT2 mRNA in vivo by a 1-10-1 and a 2-8-2 MOE gapmer**

| | **% change in SGLT2 expression relative to saline** | |
|---|---|---|
| **Dose of oligonucleotide umol/kg** | **ISIS 379692 1-10-1** | **ISIS 388625 2-8-2** |
| 200 | -80 | -80 |
| 50 | -65 | -65 |
| 12.5 | -15 | -15 |
| 3.125 | +30 | +25 |

These results illustrate that both the 1-10-1 and 2-8-2 MOE gapmer inhibit the expression of SGLT2 in vivo at the highest three dosing ranges in a dose-dependent manner.

Rats were further evaluated for total body weight, liver, spleen and kidney weight. All changes were within the margin of error of the experiment. No significant changes in body weight were observed during the treatment or at study termination. No significant changes in liver or spleen weights were observed.

The levels of ALT, AST, BUN, cholesterol, plasma creatinine and triglycerides were measured in rats treated with the short antisense compounds. The levels of routine clinical indicators of liver and kidney injury and disease were within normal ranges and are not significantly changed relative to saline-treated animals, demonstrating that the short antisense compounds do not significantly affect renal or hepatic function.

### EXAMPLE 16: Antisense Inhibition of SGLT2 Expression in ZDF rat

ISIS 388625, 388626 and control oligo ISIS 388628 were analyzed for their effect on ZDF rat plasma glucose levels and HbA1c. The leptin receptor deficient Zucker diabetic fatty (ZDF) rat is a useful model for the investigation of type 2 diabetes. Diabetes develops spontaneously in these male rats at ages 8-10 weeks, and is associated with hyperphagia, polyuria, polydipsia, and impaired weight gain, symptoms which parallel the clinical symptoms of diabetes (Phillips MS, et al., 1996, Nat Genet 13, 18-19). Six week old ZDF rats were injected intraperitoneally with short antisense compound at a dose of 40 0nM/kg once a week for twelve weeks. Data are illustrated in Tables 71 and 72.

**Table 71: Plasma glucose**

| ISIS NO. | Seq ID NO | Sequence (5'-3') | Motif | Plasma glucose levels recorded on specific dates (mg/dl) | | | |
|---|---|---|---|---|---|---|---|
| | | | | Day 10 | Day 40 | Day 55 | Day 66 |
| PBS | | n/a | n/a | 450.7 | 478.5 | 392.8 | 526.2 |
| 388625 | 246 | TGTTCCAGCCCA | 2-8-2 MOE | 435.5 | 278.7 | 213.8 | 325.5 |
| 388626 | 281 | GGCATGAGCTTC | 2-8-2 MOE | 434.7 | 300.5 | 219.8 | 379.8 |
| 388628 | 226 | TAGCCGCCCACA | 2-8-2 MOE | 436.0 | 502.0 | 411.2 | 668.8 |

**Table 72: HbA1c Status**

| ISIS NO. | Seq ID NO | Sequence (5'-3') | Motif | Percentage HbA1c on specific dates (%) p < 0.001 | | |
|---|---|---|---|---|---|---|
| | | | | Day 40 | Day 55 | Day 68 |
| PBS | | n/a | n/a | 8.0 | 8.9 | 10.0 |
| 388625 | 246 | TGTTCCAGCCCA | 2-8-2 MOE | 6.5 | 5.8 | 4.3 |
| 388626 | 281 | GGCATGAGCTTC | 2-8-2 MOE | 6.6 | 5.9 | 4.0 |
| 388628 | 226 | TAGCCGCCCACA | 2-8-2 MOE | 8.0 | 9.1 | 7.8 |

ISIS 388625 and 388626 significantly reduced plasma glucose levels and HbA1C compared to PBS and control treated animals.

### EXAMPLE 17: Antisense Inhibition of SGLT2 Expression in Dog Kidney (ISIS 388625)

ISIS 388625 is a 2-8-2 MOE Gapmer with sequence TGTTCCAGCCCA (SEQ ID NO: 246) (e.g. see Table 71). The effect of ISIS 388625 on dog SGLT2 mRNA levels. Data are taken from two dosing groups in which a total of nine male beagle dogs were dosed with either one or ten mg/kg/week of ISIS 388625 or saline given by subcutaneous injection twice weekly. On day 46 of the study all dogs were sacrificed and evaluated for SGLT2 levels in kidney. Target levels were determined by quantitative RT, real-time PCR as described by other examples herein. PCR results were normalized to an internal ISIS control. The results are shown below in Table 73.

**Table 73: Antisense inhibition of SGLT2 mRNA in vivo by ISIS 388625**

| | **% change in SGLT2 expression Relative to saline** |
|---|---|
| **Dose of oligonucleotide mg/kg/wk** | **ISIS 388625** |
| 1 | -85 |
| 10 | -95 |

These results illustrate that greater than 80% reduction of SGLT2 mRNA can be achieved at a 1 mg/kg/wk dose of ISIS 388625. Even greater reduction can be achieved at slightly higher doses. Administration of ISIS 388625 in dog was also shown to improve glucose tolerance. Peak plasma glucose levels were decreased by over 50% on average and the subsequent drop in glucose was lessened compared to saline controls in a standard glucose tolerance test. Urinary glucose excretion was also increased.

### EXAMPLE 18: In vivo testing of short antisense compounds targeted to SGLT2 nucleic acid

Twenty 1-10-1 MOE gapmers that are complementary to human/monkey/mouse/rat SGLT2 were designed, synthesized and tested *in vivo* for suppression of SGLT2 mRNA levels in kidney. Target sites for mouse and rat are indicated in Table 74. Target sites for human are indicated in Tables 4 and 5. Data are averages from two experiments in which male 6-week old Balb/c mice were administered intraperitoneal injections of 350 nmol/kg of oligonucleotide, twice per week, over a period of two weeks (a total of four injections). Mice were sacrificed 48 hours following the last administration and evaluated for SGLT2 mRNA levels in kidney. SGLT2 mRNA levels were determined by quantitative real-time PCR analysis according to standard procedures, using two different PCR primer probe sets, primer probe set (PPS) 534 and PPS 553. SGLT2 mRNA levels were normalized to cyclophilin mRNA levels, which were also measured by quantitative real-time PCR. The results are shown below in Table 74.

**Table 74: Antisense inhibition of SGLT2 in vivo**

| **ISIS NO** | **5' Target Site on SEQ ID NO: XYX (mouse)** | **5' Target Site on SEQ ID NO: XXX (rat)** | **Sequence (5'-3')** | **Motif** | **PPS 534 % Saline** | **PPS 553 % Saline** | **SEQ ID NO** |
|---|---|---|---|---|---|---|---|
| PBS | | | N/A | | --- | --- | |
| 370717 | 2684 | 152 | TAGCCACCAACT | 1-10-1 MOE | -84.4 | -84.3 | 1554 |
| 379684 | 2070 | 64 | TGTCAGCAGGAT | 1-10-1 MOE | -45.0 | -43.2 | 214 |
| 379685 | 2103 | 97 | TGACCAGCAGGA | 1-10-1 MOE | -10.3 | -20.5 | 219 |
| 379686 | 2121* | 115 | ACCACAAGCCAA | 1-10-1 MOE | -71.9 | -75.1 | 225 |
| 379687 | 2824 | 216 | GATGTTGCTGGC | 1-10-1 MOE | -47.1 | -52.1 | 230 |
| 379688 | 2876 | 268 | CCAAGCCACTTG | 1-10-1 MOE | -62.6 | -70.4 | 240 |
| 379689 | | 298 | AGAGCGCATTCC | 1-10-1 MOE | -17.5 | -30.4 | 241 |
| 379690 | | 415 | ACAGGTAGAGGC | 1-10-1 MOE | -18.9 | -22.5 | 242 |
| 379691 | | 454 | AGATCTTGGTGA | 1-10-1 MOE | -35.0 | -48.6 | 243 |
| 379692 | | 508 | TGTTCCAGCCCA | 1-10-1 MOE | -88.1 | -88.5 | 246 |
| 379693 | | 546 | CATGGTGATGCC | 1-10-1 MOE | -51.6 | -59.9 | 254 |
| 379694 | | 609 | GACGAAGGTCTG | 1-10-1 MOE | -42.1 | -54.4 | 264 |
| 379695 | | 717 | GGACACCGTCAG | 1-10-1 MOE | -52.5 | -64.1 | 266 |
| 379696 | | 954 | CAGCTTCAGGTA | 1-10-1 MOE | -24.6 | -36.2 | 267 |
| 379697 | | 982 | CTGGCATGACCA | 1-10-1 MOE | -32.0 | -46.3 | 272 |
| 379698 | | 1071 | GCAGCCCACCTC | 1-10-1 MOE | -11.8 | -27.0 | 275 |
| 379699 | | 1112 | GGCATGAGCTTC | 1-10-1 MOE | -83.5 | -85.8 | 281 |
| 379700 | | 1138 | CCAGCATGAGTC | 1-10-1 MOE | -2.8 | -16.4 | 285 |
| 379701 | | 1210 | CCATGGTGAAGA | 1-10-1 MOE | -0.3 | -11.9 | 288 |
| 379702 | | 1525 | GCACACAGCTGC | 1-10-1 MOE | -87.8 | -89.5 | 293 |
| 379703 | | 1681 | GCCGGAGACTGA | 1-10-1 MOE | -44.2 | -45.9 | 295 |

| | | | | | | | |
|---|---|---|---|---|---|---|---|
| * indicates 1 or 2 mismatches to a target sequence | | | | | | | |

### Example 19: Antisense inhibition of human PCSK9 in Hep3B cells

Short antisense compounds targeted to a PCSK9 nucleic acid were tested for their effects on PCSK9 mRNA *in vitro.* The short antisense compounds are presented in Table 6. The Isis No, gapmer motif and SEQ ID NO of each short antisense compound are shown again in Table 75. Cultured Hep3B cells were treated with 100 nM of short antisense compound. 5-10-5 MOE gapmers targeted to a PCSK9 nucleic acid were used as positive controls. After the treatment period, RNA was isolated from the cells and PCSK9 mRNA levels were measured by quantitative real-time PCR, as described herein. PCSK9 mRNA levels were adjusted according to total RNA content as measured by RIBOGREEN®. Results are presented in Table 75 as percent inhibition of PCSK9 (%Inhib), relative to untreated control cells. In the "% Inhib" column, a "0" indicates that no reduction of PCSK9 mRNA was observed with that particular short antisense compound.

**Table 75: Antisense inhibition of PCSK9 by short antisense compounds**

| **ISIS No.** | **SEQ ID NO** | **5' Target Site on SEQ ID NO: 4** | **3' Target Site on SEQ ID NO: 4** | **Gapmer Motif** | **% Inhibition Range** | **% Inhib** |
|---|---|---|---|---|---|---|
| 400297 | 329 | 695 | 708 | 2-10-2 MOE | | 0 |
| 400298 | 330 | 696 | 709 | 2-10-2 MOE | | 0 |
| 400299 | 331 | 697 | 710 | 2-10-2 MOE | | 0 |
| 400300 | 332 | 742 | 755 | 2-10-2 MOE | | 9 |
| 400301 | 333 | 757 | 770 | 2-10-2 MOE | 20-30% | 27 |
| 400302 | 334 | 828 | 841 | 2-10-2 MOE | | 0 |
| 400303 | 335 | 829 | 842 | 2-10-2 MOE | | 0 |
| 400304 | 336 | 830 | 843 | 2-10-2 MOE | 10-20% | 11 |
| 400305 | 337 | 937 | 950 | 2-10-2 MOE | 30-40% | 38 |
| 400306 | 338 | 952 | 965 | 2-10-2 MOE | 40-50% | 40 |
| 400307 | 339 | 988 | 1001 | 2-10-2 MOE | 70-80% | 76 |
| 400308 | 340 | 989 | 1002 | 2-10-2 MOE | 50-60% | 55 |
| 400309 | 341 | 990 | 1003 | 2-10-2 MOE | 40-50% | 44 |
| 400310 | 342 | 991 | 1004 | 2-10-2 MOE | | 8 |
| 400311 | 343 | 992 | 1005 | 2-10-2 MOE | 10-20% | 18 |
| 400312 | 344 | 993 | 1006 | 2-10-2 MOE | 20-30% | 28 |
| 400313 | 345 | 994 | 1007 | 2-10-2 MOE | 10-20% | 10 |
| 400314 | 346 | 1057 | 1070 | 2-10-2 MOE | 20-30% | 26 |
| 400315 | 347 | 1075 | 1088 | 2-10-2 MOE | | 0 |
| 400316 | 348 | 1076 | 1089 | 2-10-2 MOE | | 8 |
| 400317 | 349 | 1077 | 1090 | 2-10-2 MOE | | 7 |
| 400318 | 350 | 1078 | 1091 | 2-10-2 MOE | 20-30% | 26 |
| 400319 | 351 | 1093 | 1106 | 2-10-2 MOE | | 0 |
| 400320 | 352 | 1094 | 1107 | 2-10-2 MOE | | 0 |
| 400321 | 353 | 1095 | 1108 | 2-10-2 MOE | | 0 |
| 400322 | 354 | 1096 | 1109 | 2-10-2 MOE | | 0 |
| 400323 | 355 | 1147 | 1160 | 2-10-2 MOE | | 0 |
| 400324 | 356 | 1255 | 1268 | 2-10-2 MOE | | 7 |
| 400325 | 357 | 1334 | 1347 | 2-10-2 MOE | | 4 |
| 400326 | 358 | 1335 | 1348 | 2-10-2 MOE | | 0 |
| 400327 | 359 | 1336 | 1349 | 2-10-2 MOE | 30-40% | 36 |
| 400328 | 360 | 1453 | 1466 | 2-10-2 MOE | 10-20% | 13 |
| 400329 | 361 | 1454 | 1467 | 2-10-2 MOE | 10-20% | 14 |
| 400330 | 362 | 1455 | 1468 | 2-10-2 MOE | 40-50% | 43 |
| 400331 | 363 | 1456 | 1469 | 2-10-2 MOE | 30-40% | 35 |
| 400332 | 364 | 1569 | 1582 | 2-10-2 MOE | | 0 |
| 400333 | 365 | 1570 | 1583 | 2-10-2 MOE | | 0 |
| 400334 | 366 | 1571 | 1584 | 2-10-2 MOE | | 0 |
| 400335 | 367 | 1572 | 1585 | 2-10-2 MOE | | 0 |
| 400336 | 368 | 1573 | 1586 | 2-10-2 MOE | | 4 |
| 400337 | 369 | 1574 | 1587 | 2-10-2 MOE | | 0 |
| 400338 | 370 | 1575 | 1588 | 2-10-2 MOE | | 9 |
| 400339 | 371 | 1576 | 1589 | 2-10-2 MOE | | 0 |
| 400340 | 372 | 1577 | 1590 | 2-10-2 MOE | | 0 |
| 400341 | 373 | 1578 | 1591 | 2-10-2 MOE | | 0 |
| 400342 | 374 | 1621 | 1634 | 2-10-2 MOE | | 0 |
| 400343 | 375 | 1622 | 1635 | 2-10-2 MOE | | 0 |
| 400344 | 376 | 1623 | 1636 | 2-10-2 MOE | | 0 |
| 400345 | 377 | 1624 | 1637 | 2-10-2 MOE | | 0 |
| 400346 | 378 | 1738 | 1751 | 2-10-2 MOE | | 5 |
| 400347 | 379 | 1739 | 1752 | 2-10-2 MOE | | 0 |
| 400348 | 380 | 1740 | 1753 | 2-10-2 MOE | | 0 |
| 400349 | 381 | 1741 | 1754 | 2-10-2 MOE | 10-20% | 13 |
| 400350 | 382 | 1834 | 1847 | 2-10-2 MOE | 10-20% | 15 |
| 400351 | 383 | 1835 | 1848 | 2-10-2 MOE | 10-20% | 14 |
| 400352 | 384 | 1836 | 1849 | 2-10-2 MOE | 20-30% | 29 |
| 400353 | 385 | 1837 | 1850 | 2-10-2 MOE | 10-20% | 19 |
| 400354 | 386 | 1838 | 1851 | 2-10-2 MOE | 10-20% | 19 |
| 400355 | 387 | 1839 | 1852 | 2-10-2 MOE | | 0 |
| 400356 | 388 | 1840 | 1853 | 2-10-2 MOE | | 0 |
| 400357 | 389 | 2083 | 2096 | 2-10-2 MOE | | 0 |
| 400358 | 390 | 2084 | 2097 | 2-10-2 MOE | 10-20% | 12 |
| 400359 | 391 | 2085 | 2098 | 2-10-2 MOE | | 0 |
| 400360 | 392 | 2086 | 2099 | 2-10-2 MOE | 30-40% | 38 |
| 400361 | 393 | 2316 | 2329 | 2-10-2 MOE | | 2 |
| 400362 | 394 | 2317 | 2330 | 2-10-2 MOE | 10-20% | 16 |
| 400363 | 395 | 2318 | 2331 | 2-10-2 MOE | | 8 |
| 400364 | 396 | 2319 | 2332 | 2-10-2 MOE | | 0 |
| 400365 | 397 | 2320 | 2333 | 2-10-2 MOE | 20-30% | 25 |
| 400366 | 398 | 2321 | 2334 | 2-10-2 MOE | 10-20% | 15 |
| 400367 | 399 | 2322 | 2335 | 2-10-2 MOE | 10-20% | 12 |
| 400368 | 400 | 2323 | 2336 | 2-10-2 MOE | 10-20% | 11 |
| 400369 | 401 | 2324 | 2337 | 2-10-2 MOE | | 0 |
| 400370 | 402 | 2325 | 2338 | 2-10-2 MOE | 10-20% | 13 |
| 400371 | 403 | 3543 | 3556 | 2-10-2 MOE | | 0 |

As illustrated in Table 75, short antisense compounds targeted to a PCSK9 nucleic acid, having a 2-10-2 MOE gapmer motif, reduced PCSK9 mRNA in cultured cells.

Short antisense compounds targeted to a PCSK9 nucleic acid were tested in a dose response experiment Hep3B cells. Cells were treated as described herein with nM concentrations of short antisense compound as indicated in Tables 76. After the treatment period, RNA was isolated from the cells and PCSK9 mRNA levels were measured by quantitative real-time PCR, as described herein. PCSK9 mRNA levels were normalized to cyclophilin mRNA levels, as measured by real-time PCR using a cyclophilin-specific primer probe set. Results are presented as percent inhibition of PCSK9, relative to untreated control cells. Also shown is the EC₅₀ (concentration at which 50% reduction of mRNA is observed) for each short antisense compound tested in the dose response experiment, as calculated using Graphpad Prism. As illustrated in the following table, PCSK9 mRNA levels were reduced in a dose-dependent manner.

**Table 76: Dose-dependent antisense inhibition of PCSK9 by short antisense compounds**

| | % Inhibition | | | | | |
|---|---|---|---|---|---|---|
| | 160 nM | 80 nM | 40 nM | 20 nM | 10 nM | 5 nM |
| 5-10-5 | 95 | 96 | 85 | 78 | 58 | 38 |
| 400307 | 93 | 92 | 56 | 45 | 39 | 35 |
| 400308 | 86 | 77 | 40 | 26 | 10 | 31 |
| 400309 | 78 | 72 | 12 | 38 | 23 | 49 |
| 400327 | 55 | 43 | 49 | 23 | 37 | 5 |
| 400330 | 71 | 82 | 69 | 40 | 32 | 8 |
| 400331 | 82 | 75 | 63 | 47 | 40 | 29 |
| 400352 | 64 | 63 | 44 | 40 | 16 | 7 |
| 400353 | 48 | 54 | 43 | 23 | 27 | 15 |

### Example 20: Antisense inhibition of PCSK9 by short antisense compounds comprising BNAs

Short antisense compounds targeted to a PCSK9 nucleic acid were tested in dose response experiments, in both mouse and human cultured cells. The compounds tested included ISIS 403739 and ISIS 403740. ISIS 403739 is a short antisense compound consisting of the nucleotide sequence of SEQ ID NO: 404 and having a 2-10-2 gapmer motif, where the nucleotides in the wings comprise (6'S)-6'methyl BNA. ISIS 403740 is a short antisense compound consisting of the nucleotide sequence of SEQ ID NO: 405 and having a 2-10-2 gapmer motif, where the nucleotides in the wings comprise (6'S)-6'methyl BNA. Also tested was a 5-10-5 MOE gapmer targeted to a PCSK9 nucleic acid.

Mouse hepatocytes were plated and treated as described herein with nM concentrations of short antisense compound as indicated in Table 77. After the treatment period, RNA was isolated from the cells and PCSK9 mRNA levels were measured by quantitative real-time PCR, as described herein. PCSK9 mRNA levels were normalized to cyclophilin mRNA levels, as measured by real-time PCR using a cyclophilin-specific primer probe set. Results are presented as percent inhibition of PCSK9, relative to untreated control cells. Where present, "0" indicates no observed reduction in PCSK9 mRNA. ISIS 403739 exhibited dose-dependent reduction of mouse PCSK9 mRNA at the doses of 30 nM and higher. ISIS 403740 exhibited reduction of mouse PCSK9 mRNA at the two highest doses of short antisense compound.

**Table 77: Antisense inhibition of mouse PCSK9 by short antisense compounds comprising BNAs**

| | % Inhibition | | | | | | |
|---|---|---|---|---|---|---|---|
| | 3.75 nM | 7.5 nM | 15 nM | 30 nM | 60 nM | 120 nM | 240 nM |
| 5-10-5 | 10 | 15 | 21 | 18 | 44 | 43 | 77 |
| 403739 | 40 | 19 | 29 | 29 | 32 | 49 | 57 |
| 403740 | 3 | 0 | 29 | 13 | 0 | 40 | 33 |

Human Hep3B cells were treated with nM concentrations of short antisense compound as described herein. After the treatment period, RNA was isolated from the cells and PCSK9 mRNA levels were measured by quantitative real-time PCR, as described herein. PCSK9 mRNA levels were normalized to cyclophilin mRNA levels, as measured by real-time PCR using a cyclophilin-specific primer probe set. Results are presented as percent inhibition of PCSK9, relative to untreated control cells. The data are shown in Table 78 and demonstrate a dose-dependent reduction in human PCSK9 mRNA following treatment with ISIS 403740. ISIS 403739 exhibited dose-dependent reduction at higher doses.

**Table 78: Antisense inhibition of mouse PCSK9 by short antisense compounds comprising BNAs**

| | % Inhibition | | | | | | |
|---|---|---|---|---|---|---|---|
| | 2.5 nM | 5 nM | 10 nM | 20 nM | 40 nM | 80 nM | 160 nM |
| 5-10-5 | 7 | 2 | 21 | 33 | 30 | 59 | 71 |
| 403739 | 10 | 5 | 7 | 6 | 25 | 52 | 65 |
| 403740 | 6 | 12 | 16 | 29 | 45 | 48 | 59 |

### Example 21: Antisense inhibition of GCGR in HepG2 cells

Short antisense compounds targeted to a GCGR nucleic acid were tested for their effects on GCGR mRNA *in vitro.*

### HepG2 Cells

Cultured HepG2 cells at a density of 10000 cells per well in a 96-well plate were treated as described herein with 25, 50, 100 or 200 nM of antisense oligonucleotide. After the treatment period, RNA was isolated from the cells and GCGR mRNA levels were measured by quantitative real-time PCR, as described herein. GCGR mRNA levels were adjusted according to total RNA content as measured by RIBOGREEN®. Results are presented as percent reduction in GCGR mRNA, relative to untreated control cells.

Table 79 presents data following treatment with the indicated doses of ISIS 327161, a 3-10-3 MOE gapmer. ISIS 327161 reduced GCGR mRNA in a dose-dependent manner.

**Table 79: Antisense inhibition of GCGR in HepG2 cells by a short antisense compound**

| **ISIS NO.** | **Seq ID NO** | **Sequence (5'-3')** | **Gapmer Motif** | **25 nM** | **50 nM** | **100 nM** | **200 nM** |
|---|---|---|---|---|---|---|---|
| 327161 | 520 | AGCTGCTGTACATC | 3-8-3 MOE | -36 | -30 | -33 | -64 |

### Monkey hepatocytes

Additional short antisense compounds targeted to a GCGR nucleic acid were tested for their effects on monkey GCGR mRNA *in vitro.* Cultured primary monkey hepatocytes were treated as described herein with 25, 50, 100 or 200 nM of short antisense compound. After the treatment period, RNA was isolated from the cells and GCGR mRNA levels were measured by quantitative real-time PCR, as described herein. GCGR mRNA levels were adjusted according to total RNA content as measured by RIBOGREEN®. Results are presented in Table 80 as percent reduction in GCGR mRNA, relative to untreated control cells.

**Table 80: Antisense inhibition of GCGR in primary monkey hepatocytes by short antisense compounds**

| **ISIS NO.** | **Seq ID NO** | **Sequence (5'-3')** | **Gapmer Motif** | **25 nM** | **50 nM** | **100 nM** | **200 nM** |
|---|---|---|---|---|---|---|---|
| 327131 | 489 | ATGTTGGCCGTGGT | 3-8-3 MOE | 0 | -8 | -36 | -36 |
| 327161 | 520 | AGCTGCTGTACATC | 3-8-3 MOE | -19 | -33 | -55 | -54 |

### Example 22: Antisense inhibition of DGAT2 by short antisense compounds

Short antisense compounds targeted to a DGAT2 nucleic acid were tested for their effects on DGAT2 mRNA *in vitro.* Cultured A10 cells in a 96-well plate were treated with 75 nM of short antisense compound. After a treatment period of approximately 24 hours, RNA was isolated from the cells and DGAT2 mRNA levels were measured by quantitative real-time PCR, as described herein. DGAT2 mRNA levels were adjusted according to total RNA content as measured by RIBOGREEN®. Results are presented as percent inhibition of DGAT2, relative to untreated control cells in Table 81.

**Table 81: Antisense inhibition of DGAT2 in A10 cells**

| **ISIS NO.** | **Seq ID NO** | **Sequence (5'-3')** | **Gapmer Motif** | **% Control** |
|---|---|---|---|---|
| 372491 | 795 | ACATGAGGATGACACT | 3-10-3 MOE | 80 |
| 372500 | 702 | GTGTGTCTTCACCAGC | 3-10-3 MOE | 16 |
| 372501 | 704 | TTGTGTGTCTTCACCA | 3-10-3 MOE | 28 |
| 372503 | 708 | GCAGGTTGTGTGTCTT | 3-10-3 MOE | 35 |
| 372508 | 719 | AGTTCCTGGTGGTCAG | 3-10-3 MOE | 35 |
| 372516 | 805 | TACAGAAGGCACCCAG | 3-10-3 MOE | 27 |
| 372524 | 738 | GCCAGGCATGGAGCTC | 3-10-3 MOE | 21 |
| 372530 | 746 | TCGGCCCCAGGAGCCC | 3-10-3 MOE | 35 |
| 372546 | 825 | TTGGTCTTGTGATTGT | 3-10-3 MOE | 34 |
| 372563 | 691 | AGCCAGGTGACAGA | 2-10-2 MOE | 48 |
| 372569 | 796 | CATGAGGATGACAC | 2-10-2 MOE | 104 |
| 372578 | 703 | TGTGTCTTCACCAG | 2-10-2 MOE | 59 |
| 372580 | 707 | GGTTGTGTGTCTTC | 2-10-2 MOE | 48 |
| 372586 | 720 | GTTCCTGGTGGTCA | 2-10-2 MOE | 40 |
| 372594 | 806 | ACAGAAGGCACCCA | 2-10-2 MOE | 77 |
| 372602 | 739 | CCAGGCATGGAGCT | 2-10-2 MOE | 39 |
| 372618 | 765 | GTGGTACAGGTCGA | 2-10-2 MOE | 29 |
| 372624 | 826 | TGGTCTTGTGATTG | 2-10-2 MOE | 56 |

Additional short antisense compounds targeted to DGAT2 mRNA were tested *in vitro* in a dose-response experiment. A10 cells were prepared as described above and treated with 6.25, 12.5, 25.0, 50.0, 100.0, and 200.0 nM short antisense compounds to determine if DGAT2 inhibition occurs in a dose-dependent manner. The data demonstrate that each of the short antisense compounds presented in Table 82 reduces rat DGAT2 mRNA in a dose-dependent manner. Results are presented as percent inhibition, relative to untreated control cells. A "0" indicates that DGAT2 mRNA was not reduced.

**Table 82: Dose-Dependent Inhibition of DGAT2 in A10 cells**

| **ISIS NO.** | **Seq ID NO** | **Sequence (5'-3')** | **Gapmer Motif** | **6.25 nM** | **12.5 nM** | **25.0 nM** | **50.0 nM** | **100.0 nM** | **200.0 nM** |
|---|---|---|---|---|---|---|---|---|---|
| 372562 | 784 | GTCTTGGAGGGCCG | 2-10-2 MOE | 0 | 0 | 0 | 36 | 48 | 75 |
| 372568 | 794 | GACACTGCAGGCCA | 2-10-2 MOE | 0 | 0 | 15 | 26 | 72 | 69 |
| 372586 | 720 | GTTCCTGGTGGTCA | 2-10-2 MOE | 19 | 0 | 7 | 22 | 45 | 77 |
| 372602 | 739 | CCAGGCATGGAGCT | 2-10-2 MOE | 0 | 0 | 0 | 18 | 47 | 76 |
| 372618 | 765 | GTGGTACAGGTCGA | 2-10-2 MOE | 0 | 5 | 0 | 27 | 65 | 80 |

Additional short antisense compounds targeted to DGAT2 mRNA were tested *in vitro.* A10 cells were prepared as described above and treated with 0.62, 1.85, 5.56, 16.67, 50.0, and 150.0 nM short antisense compounds to determine if DGAT2 inhibition occurs in a dose-dependent manner. DGAT2 mRNA was measured using quantitative real-time PCR, as described herein. The data demonstrate that each of the short antisense compounds presented in Table 83 below inhibit rat DGAT2 mRNA in a dose-dependent manner. Results are presented as percent inhibition of rat DGAT2, relative to untreated control cells. Where present, "0" indicates that no reduction in DGAT2 mRNA was observed.

**Table 83: Dose-Dependent Inhibition of DGAT2 in A10 cells**

| **ISIS NO.** | **Seq ID NO** | **Sequence (5'-3')** | **Gapm er Motif** | **0.62 nM** | **1.85 nM** | **5.56 nM** | **16.67 nM** | **50 nM** | **150 nM** |
|---|---|---|---|---|---|---|---|---|---|
| 372500 | 702 | GTGTGTCTTCACCAGC | 3-10-3 MOE | 0 | 0 | 0 | 18 | 64 | 88 |
| 372501 | 704 | TTGTGTGTCTTCACCA | 3-10-3 MOE | 1 | 5 | 10 | 11 | 25 | 68 |
| 372503 | 708 | GCAGGTTGTGTGTCTT | 3-10-3 MOE | 7 | 10 | 4 | 25 | 54 | 80 |
| 372508 | 719 | AGTTCCTGGTGGTCAG | 3-10-3 MOE | 0 | 0 | 6 | 14 | 39 | 71 |
| 372516 | 805 | TACAGAAGGCACCCAG | 3-10-3 MOE | 1 | 10 | 0 | 4 | 35 | 81 |
| 372524 | 738 | GCCAGGCATGGAGCTC | 3-10-3 MOE | 7 | 0 | 5 | 30 | 68 | 91 |
| 372530 | 746 | TCGGCCCCAGGAGCCC | 3-10-3 MOE | 0 | 2 | 0 | 10 | 38 | 78 |
| 372546 | 825 | TTGGTCTTGTGATTGT | 3-10-3 MOE | 0 | 2 | 11 | 4 | 48 | 78 |
| 372563 | 691 | AGCCAGGTGACAGA | 2-10-2 MOE | 0 | 0 | 0 | 1 | 4 | 46 |
| 372578 | 703 | TGTGTCTTCACCAG | 2-10-2 MOE | 0 | 0 | 0 | 2 | 7 | 42 |
| 372580 | 707 | GGTTGTGTGTCTTC | 2-10-2 MOE | 0 | 5 | 5 | 3 | 16 | 42 |
| 372586 | 720 | GTTCCTGGTGGTCA | 2-10-2 MOE | 0 | 0 | 0 | 0 | 7 | 55 |
| 372594 | 806 | ACAGAAGGCACCCA | 2-10-2 MOE | 0 | 0 | 0 | 0 | 2 | 15 |
| 372602 | 739 | CCAGGCATGGAGCT | 2-10-2 MOE | 0 | 0 | 10 | 0 | 19 | 51 |
| 372618 | 765 | GTGGTACAGGTCGA | 2-10-2 MOE | 0 | 0 | 0 | 0 | 30 | 60 |
| 372624 | 826 | TGGTCTTGTGATTG | 2-10-2 MOE | 0 | 0 | 0 | 1 | 16 | 38 |

### Example 23: Antisense inhibition of human PTP1B in HuVEC cells

Short antisense compounds targeted to a PTP1B nucleic acid were tested for their effects on PTP1B mRNA *in vitro.* Cultured HuVEC cells at a density of 5000 cells per well in a 96-well plate were treated as described herein with 3 nM of short antisense compound. After the treatment period, RNA was isolated from the cells and PTP1B mRNA levels were measured by quantitative real-time PCR, as described herein. PTP1B mRNA levels were adjusted according to total RNA content as measured by RIBOGREEN®. Results are presented as percent inhibition of PTP1B (% Inhib), relative to untreated control cells. The data demonstrated that short antisense compounds targeted to a PTP1B nucleic acid and having a 2-10-2 gapmer motif can inhibit PTP1B in HuVEC cells in Table 84.

**Table 84: Antisense inhibition of PTP1B in HuVEC cells by short antisense compounds**

| ISIS NO. | SEQ ID NO | Gapmer Motif | %Inhib |
|---|---|---|---|
| 399301 | 1542 | 2-10-2 OMe | 55 |
| 404137 | 1053 | 2-10-2 MOE | 76 |
| 404138 | 1054 | 2-10-2 MOE | 76 |
| 404139 | 1052 | 2-10-2 MOE | 80 |
| 404140 | 1051 | 2-10-2 MOE | 73 |

### Example 24: Antisense inhibition of human PTP1B in HepG2 cells

Short antisense compounds targeted to a PTP1B nucleic acid were tested for their effects on PTP1B mRNA *in vitro.* Cultured HepG2 cells at a density of 10000 cells per well in a 96-well plate were treated with 25 nM of antisense oligonucleotide. After the treatment period, RNA was isolated from the cells and PTP1B mRNA levels were measured by quantitative real-time PCR, as described herein. PTP1B mRNA levels were adjusted according to total RNA content as measured by RIBOGREEN®. Results are presented as percent inhibition (% Inhib) of PTP1B, relative to untreated control cells. The data demonstrated that short antisense compounds targeted to a PTP1B nucleic acid and having a 2-10-2 gapmer motif can inhibit PTP1B in HepG2 cells in Table 85.

**Table 85: Antisense inhibition of PTP1B in HepG2 cells by short antisense compounds**

| ISIS NO. | SEQ ID NO | Gapmer Motif | %Inhib |
|---|---|---|---|
| 399301 | 1542 | 2-10-2 OMe | 43 |
| 404137 | 1053 | 2-10-2 MOE | 71 |
| 404138 | 1054 | 2-10-2 MOE | 86 |
| 404139 | 1052 | 2-10-2 MOE | 45 |
| 404140 | 1051 | 2-10-2 MOE | 93 |

### Example 25: Antisense inhibition of PTP1B in HuVEC cells: Dose response experiment

Human vascular endothelial (HuVEC) cells were plated at a density of 5000 cells per well and treated as described herein with nM concentrations of short antisense compound as indicated in Table 86. After the treatment period, RNA was isolated from the cells and PTP1B mRNA levels were measured by quantitative real-time PCR, as described herein. PTP1B mRNA levels were adjusted according to total RNA content as measured by RIBOGREEN®. Two different human PTP1B primer probe sets were used to measure mRNA levels. Results with Primer Probe Set (PPS) 198 are shown in Table 86, and results with Primer Probe Set (PPS) 3000 are shown in Table 87. Results are presented as percent inhibition of PTP1B mRNA expression relative to untreated control cells. Where present, "0" indicates that no PTP1B mRNA reduction was observed. As illustrated in Tables 86 and 87, PTP1B mRNA levels were reduced in a dose-dependent manner.

**Table 86: Dose Response for Human PTP1B in HuVEC cells, using PPS 198**

| | | | **% Inhibition** | | | |
|---|---|---|---|---|---|---|
| **ISIS NO.** | **Seq ID NO** | **Gapmer Motif** | **1.11 nM** | **3.33 nM** | **10.0 nM** | **30.0 nM** |
| 398105 | 1066 | 2-10-2 MOE | 0 | 25 | 79 | 90 |
| 398112 | 1072 | 2-10-2 MOE | 1 | 10 | 73 | 93 |
| 398120 | 1086 | 2-10-2 MOE | 0 | 31 | 80 | 96 |
| 399096 | 1544 | 2-10-2 MOE | 3 | 30 | 78 | 96 |
| 399102 | 1545 | 2-10-2 MOE | 0 | 15 | 62 | 88 |
| 399113 | 1547 | 2-10-2 MOE | 0 | 31 | 72 | 90 |
| 399132 | 1548 | 2-10-2 MOE | 0 | 32 | 75 | 95 |
| 399173 | 1549 | 2-10-2 MOE | 0 | 24 | 63 | 89 |
| 399208 | 1550 | 2-10-2 MOE | 0 | 37 | 86 | 93 |
| 399276 | 1551 | 2-10-2 MOE | 0 | 8 | 61 | 89 |
| 399301 | 1542 | 2-10-2 MOE | 8 | 63 | 91 | 97 |
| 399315 | 1552 | 2-10-2 MOE | 0 | 20 | 68 | 88 |
| 398173 | 1543 | 1-10-1 MOE | 0 | 4 | 80 | 97 |

**Table 87: Dose Response for Human PTP1B in HuVEC cells, using PPS 3000**

| | | | **% Inhibition** | | | |
|---|---|---|---|---|---|---|
| **ISIS NO.** | **Seq ID NO** | **Gapmer Motif** | **1.11 nM** | **3.33 nM** | **10.0 nM** | **30.0 nM** |
| 398105 | 1066 | 2-10-2 MOE | 0 | 35 | 79 | 93 |
| 398112 | 1072 | 2-10-2 MOE | 0 | 26 | 77 | 94 |
| 398120 | 1086 | 2-10-2 MOE | 0 | 35 | 79 | 93 |
| 399096 | 1544 | 2-10-2 MOE | 0 | 23 | 75 | 94 |
| 399102 | 1545 | 2-10-2 MOE | 0 | 9 | 60 | 87 |
| 399113 | 1547 | 2-10-2 MOE | 0 | 9 | 65 | 90 |
| 399132 | 1548 | 2-10-2 MOE | 0 | 26 | 76 | 91 |
| 399173 | 1549 | 2-10-2 MOE | 0 | 11 | 59 | 92 |
| 399208 | 1550 | 2-10-2 MOE | 0 | 47 | 85 | 96 |
| 399276 | 1551 | 2-10-2 MOE | 0 | 14 | 64 | 86 |
| 399301 | 1542 | 2-10-2 MOE | 16 | 65 | 93 | 99 |
| 399315 | 1552 | 2-10-2 MOE | 0 | 25 | 71 | 93 |
| 398173 | 1543 | 1-10-1 MOE | 0 | 18 | 80 | 90 |

### Example 26: Antisense inhibition of ApoB by short antisense compounds

The short antisense compounds shown in Table 88 were tested for their effects in vivo. Six-week old male Balb/c mice (Jackson Laboratory, Bar Harbor, ME) were administered intraperitoneal doses of 3.2, 1, 0.32, or .1 umol/kg, twice per week for three weeks. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 89 as percent inhibition relative to ApoB mRNA levels in saline-treated control animals.

**Table 88: Short Antisense Compounds Targeting an ApoB nucleic acid**

| **ISIS NO** | **Sequence (5'-3')** | **Gapmer Motif** | **SEQ ID NO** |
|---|---|---|---|
| 387462 | GGTACATGGAAGTC | 2-10-2 Methyleneoxy BNA | 190 |
| 398296 | GGTACATGGAAGTC | 2-10-2 6'-(S)-methyl Methyleneoxy BNA | 190 |

**Table 89: Antisense inhibition of ApoB by Short Antisense Compounds Comprising BNA**

| Isis No | Dose (umol/kg) | % Inhib |
|---|---|---|
| 379818 | 1 | 56 |
| 387462 | 0.1 | 33 |
| | 0.32 | 57 |
| | 1 | 93 |
| | 3.2 | 99 |
| 398296 | 0.1 | 17 |
| | 0.32 | 35 |
| | 1 | 80 |
| | 3.2 | 98 |

Table 89 shows that ApoB mRNA levels were reduced in a dose-dependent manner following treatment with short antisense compounds having a 2-10-2 gapmer motif and BNA modifications in the wings. At the 1 umol/kg dose, ApoB inhibition by the short antisense compounds was greater than observed with a 5-10-5 MOE gapmer at an equivalent dose. Cholesterol was reduced at the 1 and 3.2 umol/kg doses of short antisense compound.

The short antisense compounds exhibited little to no adverse side effects, as judged by organ and body weights, serum transaminases, bilirubin, blood urea nitrogen, and creatinine.

### Example 27: Antisense inhibition of PTEN by short antisense compounds

The short antisense compounds shown in Table 90 were tested for their effects in vivo. Six-week old male Balb/c mice (Jackson Laboratory, Bar Harbor, ME) were administered intraperitoneal doses of 3.2, 1, 0.32, or .1 umol/kg, twice per week for three weeks. A 5-10-5 MOE gapmer was used for a control treatment. Mice were sacrificed approximately 48 hours following the final dose. Liver tissue was collected for RNA isolation, and blood was collected for serum chemistry analyses. PTEN mRNA levels were measured by real-time PCR as described herein. PTEN mRNA levels were normalized to RNA levels as determined by RIBOGREEN, and are presented in Table 91 as percent inhibition relative to PTEN mRNA levels in saline-treated control animals.

**Table 90: Short Antisense Compounds targeted to a PTEN nucleic acid**

| **ISIS NO** | **Sequence (5'-3')** | **Gapmer Motif** | **SEQ ID NO** |
|---|---|---|---|
| 392063 | AGGCCAGTGCTAAG | 2-10-2 Methyleneoxy BNA | 1226 |
| 392749 | AGGCCAGTGCTAAG | 2-10-2 (6'S)-6'-methyl Methyleneoxy BNA | 1226 |
| 396006 | AGGCCAGTGCTAAG | 2-10-2 alpha-L-methyleneoxy BNA | 1226 |

**Table 91: Antisense inhibition of PTEN by short antisense compounds comprising BNA modifications**

| Isis No | Dose (umol/kg) | % Inhib |
|---|---|---|
| 116847 | 1 | 47 |
| 392063 | 0.1 | 26 |
| | 0.32 | 43 |
| | 1 | 74 |
| | 3.2 | 96 |
| 392749 | 0.1 | 17 |
| | 0.32 | 34 |
| | 1 | 64 |
| | 3.2 | 96 |
| 396006 | 0.1 | 20 |
| | 0.32 | 32 |
| | 1 | 67 |
| | 3.2 | 88 |

Table 91 shows that PTEN mRNA levels were reduced in a dose-dependent manner following treatment with short antisense compounds having a 2-10-2 gapmer motif and BNA modifications in the wings. At the 1 umol/kg dose, PTEN inhibition by the short antisense compounds was greater than observed with a 5-10-5 MOE gapmer at an equivalent dose.

With the exception of the highest dose of ISIS 392063, no significant increases in serum transaminases were observed. Overall, the short antisense compounds exhibited little to no adverse side effects.

### Example 28: Single dose administration of short antisense compounds comprising BNA modifications

Six-week old male Balb/c mice (Jackson Laboratory, Bar Harbor, ME) were administered a single intraperitoneal injection of short antisense compound at a dose of 8, 4, 2 or 1 µmol/kg. The short antisense compounds tested were ISIS 387462 and ISIS 398296. Each dose group consisted of four animals. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 92 as percent inhibition relative to ApoB mRNA levels in saline-treated control animals.

**Table 92: Antisense inhibition of ApoB by Short Antisense Compounds Comprising BNA**

| Isis No | Dose (umol/kg) | % Inhib |
|---|---|---|
| 379818 | 8 | 77 |
| 387462 | 8 | 99 |
| | 4 | 93 |
| | 2 | 81 |
| | 1 | 58 |
| 398296 | 8 | 97 |
| | 4 | 81 |
| | 2 | 54 |
| | 1 | 19 |

Table 92 shows that ApoB mRNA levels were reduced in a dose-dependent manner following a single administration of short antisense compounds having a 2-10-2 gapmer motif and BNA modifications in the wings. At the 8 umol/kg dose, ApoB inhibition by the short antisense compounds was greater than observed with a 5-10-5 MOE gapmer at an equivalent dose. The ED₅₀ of ISIS 387462 was 3.9 mg/kg, and the ED₅₀ of ISIS 398296 was 8.7 mg/kg. Cholesterol was also reduced in a dose-dependent manner. Triglycerides were reduced at the highest dose.

The short antisense compounds exhibited little to no adverse side effects, as judged by organ and body weights, serum transaminases, bilirubin, blood urea nitrogen, and creatinine.

In a similar single dose administration study, ISIS 392748, having SEQ ID NO: 1226, a 2-10-2 gapmer motif, where the nucleotides of the wings comprise (6'R)-6'-methyl methyleneoxy BNA modifications, reduced PTEN mRNA in a dose-dependent manner. Additionally, ISIS 392749, having SEQ ID NO: 1226, a 2-10-2 gapmer motif, where the nucleotides of the wings comprise (6'S)-6'-methyl methyleneoxy BNA modifications, reduced PTEN mRNA in a dose-dependent manner. A short antisense compound having 2-10-2 gapmer motifs, the sequence of SEQ ID NO: 1226, and 6-(S)-CH2-O-CH3-BNA modifications also reduced PTEN mRNA in a similar *in vivo* study. A short antisense compound having 2-10-2 gapmer motifs, the sequence of SEQ ID NO: 1226, and 6-(R)-CH2-O-CH3-BNA modifications also reduced PTEN mRNA in a similar *in vivo* study.

### Example 29: Single dose administration of short antisense compounds comprising BNA modifications

Six-week old male Balb/c mice (Jackson Laboratory, Bar Harbor, ME) were administered a single intraperitoneal injection of antisense compound at a dose of 8, 4, 2 or 1 µmol/kg. Each dose group consisted of four animals. The compounds tested were ISIS 392063, ISIS 392749, and ISIS 366006. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 93 as percent inhibition relative to ApoB mRNA levels in saline-treated control animals.

**Table 93: Antisense inhibition of PTEN by short antisense compounds comprising BNA modifications**

| Isis No | Dose (umol/kg) | % Inhib |
|---|---|---|
| 116847 | 8 | 62 |
| 392063 | 8 | 92 |
| | 4 | 82 |
| | 2 | 58 |
| | 1 | 38 |
| 396565 | 8 | 76 |
| | 4 | 38 |
| | 2 | 24 |
| | 1 | 11 |
| 396006 | 8 | 94 |
| | 4 | 82 |
| | 2 | 48 |
| | 1 | 18 |

Table 93 shows that PTEN mRNA levels were reduced in a dose-dependent manner following treatment with short antisense compounds having a 2-10-2 gapmer motif and BNA modifications in the wings. At the 8 umol/kg dose, PTEN inhibition by the short antisense compounds was greater than observed with a 5-10-5 MOE gapmer at an equivalent dose. The estimated ED_{50S} were 7 mg/kg for ISIS 392063, 17.4 mg/kg for ISIS 396565, and 9.3 mg/kg for ISIS 396006.

With the exception of the highest dose of ISIS 392063, no significant increases in serum transaminases were observed. Overall, the short antisense compounds exhibited little to no adverse side effects.

### Example 30: Antisense inhibition of ApoB by short antisense compounds comprising palmitic acid conjugates

Six-week old male Balb/c mice (Jackson Laboratory, Bar Harbor, ME) were administered a single intraperitoneal injection of antisense compound at a dose of 2.5, 1.0. 0.4, and 0.16 umol/kg. Each dose group consisted of four animals. The compounds tested are shown in Table 94. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 95 as percent inhibition relative to ApoB mRNA levels in saline-treated control animals.

**Table 94: Short antisense compounds comprising palmitic conjugates**

| **ISIS NO** | **Sequence (5'-3')** | **Gapmer Motif** | **SEQ ID NO** |
|---|---|---|---|
| 387462 | GGTACATGGAAGTC | 2-10-2 Methyleneoxy BNA | 190 |
| 391871 | GGTACATGGAAGTC | 1-1-10-2 2'-(butylacetomido)-palmitamide/MOE/MOE Unmodified cytosines in gap (i.e., 2-10-2 MOE with 2'-(butylacetomido)-palmitamide substituted at 5' nucleotide | 190 |
| 391872 | GGTACATGGAAGTC | 1-1-10-2 2'-(butylacetomido)-palmitamide Methyleneoxy BNA/Methyleneoxy BNA Unmodified cytosines in gap (i.e., 2-10-2 methyleneoxy BNA with 2'-(butylacetomido)-palmitamide substituted at 5' nucleotide) | 190 |

**Table 95: Antisense inhibition by short antisense compounds comprising palmitic acid conjugates**

| Isis No | Dose (umol/kg) | % Inhib |
|---|---|---|
| 5-10-5 | 2.5 | 54 |
| 387462 | 2.5 | 99 |
| | 1.0 | 91 |
| | 0.4 | 65 |
| | 0.16 | 16 |
| 391871 | 2.5 | 49 |
| | 1.0 | 18 |
| | 0.4 | 5 |
| | 0.16 | 0 |
| 391872 | 2.5 | 99 |
| | 1.0 | 92 |
| | 0.4 | 50 |
| | 0.16 | 18 |

Table 95 shows that ApoB mRNA levels were reduced in a dose-dependent manner following treatment with short antisense compounds having a palmitic acid (C16) conjugate. At the 2.5 umol/kg dose, ApoB inhibition by the short antisense compounds was greater than observed with a 5-10-5 MOE gapmer at an equivalent dose. In this study, the estimated ED_{50S} were 1.5 mg/kg for ISIS 387462, 13.1 mg/kg for ISIS 391871, and 1.9 mg/kg for ISIS 391872. The estimated ED₅₀ for the 5-10-5 MOE gapmer was 17.4 mg/kg. Triglycerides were reduced at the 2.5 and 1.0 mg/kg doses of ISIS 387462 and ISIS 391872. ISIS 387462 and ISIS 391872 markedly reduced total cholesterol, HDL-C and LDL-C in a dose-dependent manner; reduction in LDL-C was so marked that it fell below the limit of detection. Overall, the short antisense compounds exhibited little to no adverse effects.

### Example 31: Antisense inhibition of PCSK9 in vivo by short antisense compounds comprising BNA modifications

Six-week old male Balb/c mice (Jackson Laboratory, Bar Harbor, ME) were administered a single intraperitoneal injection of antisense compound at a dose of 15, 4.7, 1.5 and .47 umol/kg of ISIS 403739 or 403740. Each dose group consisted of four animals. A 5-10-5 MOE gapmer was used for a control treatment. Mice were sacrificed approximately 72 hours following the final dose. Liver tissue was collected for RNA isolation, and blood was collected for serum chemistry analyses. PCSK9 mRNA levels were measured by real-time PCR as described herein. PCSK9 mRNA levels were normalized to cyclophilin mRNA levels as determined by real-time PCR. ISIS 403739 reduced PCSK9 mRNA by approximately 70%, relative to saline controls. ISIS 403740 reduced PCSK9 by approximately 13% relative to saline controls, however, the reduction was not statistically significant. The lower doses did not significantly reduce PCSK9 mRNA. Overall, the short antisense compounds exhibited little to no adverse side effects.

### EMBODIMENTS OF THE INVENTION

1. A short antisense compound 8 to 16 monomers in length, comprising a 2'-deoxyribonucleotide gap region flanked on each side by a wing, wherein each wing independently comprises 1 to 3 high-affinity modified monomers and wherein the short antisense compound is targeted to a nucleotide encoding GCCR.
2. The short antisense compound of embodiment 1, wherein said high-affinity modified monomers are sugar-modified nucleotides.
3. The short antisense compound of embodiment 2, wherein at least one of the sugar-modified nucleotides comprises a bridge between the 4' and the 2' position of the sugar.
4. The short antisense compound of embodiment 2, wherein each of said high-affinity modified nucleotides confers a ΔTₘ of 1 to 4 degrees per nucleotide.
5. The short antisense compound of embodiment 2, wherein each of said sugar-modified nucleotides comprises a 2'-substituent group that is other than H or OH.
6. The short antisense compound of embodiment 5, wherein at least one of said sugar-modified nucleotides is a 4' to 2' bridged bicyclic nucleotide.
7. The short antisense compound of embodiment 5, wherein each of the 2'-substituent groups is, independently, alkoxy, substituted alkoxy, or halogen.
8. The short antisense compound of embodiment 7, wherein each of the 2'-substituent groups is OCH₂CH₂OCH₃.
9. The short antisense compound embodiment 3, wherein the conformation of each of said sugar-modified nucleotides is, independently, β-D or α-L.
10. The short antisense compound embodiment 5, wherein each of said bridges independently comprises 1 or from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
   wherein
   x is 0, 1, or 2;
   n is 1, 2, 3, or 4;
   each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
   each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.
11. The short antisense compound of embodiment 10, wherein each of said bridges is, independently, 4'-CH₂-2', 4'-(CH₂)₂-2', 4'-CH₂-O-2', 4'-(CH₂)₂-O-2', 4'-CH₂-O-N(R₁)-2' and 4'-CH₂-N(R₁)-O-2'-wherein each R₁ is, independently, H, a protecting group or C₁-C₁₂ alkyl.
12. The short antisense compound of embodiment 1, wherein each of the high-affinity modified monomer is independently selected from bicyclic nucleotides or other 2'-modified nucleotides.
13. The short antisense compound of embodiment 12, wherein the 2'-modified nucleotides are selected from halogen, allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ) or O-CH₂-C(=O)-N(Rₘ)(Rₙ), where each Rₘ and Rₙ is, independently, H or substituted or unsubstituted C₁-C₁₀ alkyl.
14. The short antisense compound of embodimentembodiment 13, wherein the 2'-modified nucleotide is a 2'-OCH₂CH₂OCH₃ nucleotide.
15. The short antisense compound of embodiment 1, wherein at least one monomoeric linkage is a modified monomeric linkage.
16. The antisense compound of embodiment 15, wherein the modified monomeric linkage is a phosphorothioate linkage.
17. The short antisense compound of embodiment 1, wherein each monomeric linkage is a phosphorothioate internucleoside linkage.
18. The short antisense compound of any of embodiments 1-17, that is 8-15 monomers in length.
19. The short antisense compound of embodiment 18 that is 9-15 monomers in length.
20. The short antisense compound of embodiment 18 that is 10-15 monomers in length.
21. The short antisense compound of embodiment 18 that is 9-14 monomers in length.
22. The short antisense compound of embodiment 18 that is 10-14 monomers in length.
23. The short antisense compound of embodiment 18 that is 9-13 monomers in length.
24. The short antisense compound of embodiment 18 that is 10-13 monomers in length.
25. The short antisense compound of embodiment 18 that is 9-12 monomers in length.
26. The short antisense compound of embodiment 18 that is 10-12 monomers in length.
27. The short antisense compound of embodiment 18 that is 9-11 monomers in length.
28. The short antisense compound of embodiment 18 that is 10-11 monomers in length.
29. The short antisense compound of embodiment 18 that is 8 monomers in length.
30. The short antisense compound of embodiment 18 that is 9 monomers in length.
31. The short antisense compound of embodiment 18 that is 10 monomers in length.
32. The short antisense compound of embodiment 18 that is 11 monomers in length.
33. The short antisense compound of embodiment 18 that is 12 monomers in length.
34. The short antisense compound of embodiment 18 that is 13 monomers in length.
35. The short antisense compound of embodiment 18 that is 14 monomers in length.
36. The short antisense compound of embodiment 18 that is 15 monomers in length.
37. The short antisense compound of embodiment 18 that is 16 monomers in length.

## Claims

1. A short antisense compound 8 to 16 nucleotides in length, comprising a gap region flanked on each side by a wing, wherein each wing independently consists of 1 to 3 high-affinity modified nucleotides, and wherein the short antisense compound is targeted to a nucleic acid encoding GCGR.

2. The short antisense compound of claim 1, wherein said high-affinity modified nucleotides are sugar-modified nucleotides.

3. The short antisense compound of claim 2, wherein at least one of the sugar-modified nucleotides comprises a bridge between the 4' and the 2' positions of the sugar.

4. The short antisense compound of claim 2, wherein each of said sugar-modified nucleotides comprises a 2'-substituent group that is other than H or OH.

5. The short antisense compound of claim 4, wherein at least one of said sugar-modified nucleotides is a 4' to 2' bridged bicyclic nucleotide.

6. The short antisense compound of claim 4, wherein each of the 2'-substituent groups is, independently, alkoxy, substituted alkoxy, or halogen.

7. The short antisense compound of claim 6, wherein each of the 2'-substituent groups is OCH₂CH₂OCH₃ (2'-MOE).

8. The short antisense compound claim 4, wherein each of said bridges independently comprises 1 or from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
wherein
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

9. The short antisense compound of claim 5, wherein each of said bridges is, independently, 4'-CH(CH₃)-O-2', 4'-CH₂-2', 4'-(CH₂)₂-2', 4'-CH₂-O-2', 4'-(CH₂)₂-O-2', 4'-CH₂-O-N(R₁)-2' and 4'-CH₂-N(R₁)-O-2'- wherein each R₁ is, independently, H, a protecting group or C₁-C₁₂ alkyl.

10. The short antisense compound of claim 1, wherein each of the high-affinity modified nucleotides is independently selected from bicyclic nucleotides or other 2'-modified nucleotides.

11. The short antisense compound of claim 10, wherein the 2'-modified nucleotides are selected fromallyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ) and O-CH₂-C(=O)-N(Rₘ)(Rₙ), where each Rₘ and Rₙ is, independently, H or substituted or unsubstituted C₁-C₁₀ alkyl.

12. The short antisense compound of any preceding claim, wherein at least one monomeric linkage is a modified monomeric linkage.

13. The antisense compound of claim 12, wherein the modified monomeric linkage is a phosphorothioate linkage.

14. A pharmaceutical composition comprising the antisense compound of any preceding claim and a suitable pharmaceutically acceptable diluent or carrier.

15. An antisense compound according to any of claims 1-13 or a pharmaceutical composition according to claim 14, for use in therapy.
